# EUROPEAN PATENT SPECIFICATION

(11) **EP 3 405 492 B1**
(45) Date of publication and mention of the grant of the patent: **21.10.2020**
(21) Application number: 17702163.1
(22) Date of filing: 20.01.2017
(51) Int. Cl.: C07K 16/28

(54) **MULTISPECIFIC MOLECULES TARGETING CLL-1**
GEGEN CLL-1 GERICHTETE MULTISPEZIFISCHE MOLEKÜLE
MOLÉCULES MULTISPÉCIFIQUES CIBLANT CLL-1

(30) Priority: 21.01.2016 WO PCT/CN2016/071599
(43) Date of publication of application: 28.11.2018
(73) Proprietor: Novartis AG, 4056 Basel (CH)
(72) Inventor: EBERSBACH, Hilmar, 4002 Basel (CH); DALEY, Michael, Cambridge, Massachusetts 02139 (US); JASCUR, Julia, 4002 Basel (CH); WU, Qilong, Shanghai 201203 (CN); YANG, Qiumei, Shanghai 201203 (CN); ZHANG, Jiquan, Shanghai 201203 (CN)
(74) Representative: Bucher, Tamaris Clare
(86) International application number: PCT/IB2017/050318
(87) International publication number: WO 2017/125897

(56) References cited:
- WO-A1-2014/051433
- WO-A1-2014/153002
- WO-A1-2016/014535
- WO-A1-2016/040868
- WO-A1-2016/205200
- LU H ET AL: "Targeting human C-type lectin-like molecule-1 (CLL1) with a bispecific antibody for immunotherapy of acute myeloid leukemia", ANGEWANDTE CHEMIE INTERNATIONAL EDITION, WILEY-VCH VERLAG GMBH, vol. 53, no. 37, 8 September 2014 (2014-09-08), pages 9841-9845, XP002752433, ISSN: 1433-7851, DOI: 10.1002/ANIE.201405353 [retrieved on 2014-07-23]
- "Press Release Merus Presents Preclinical Data on its Novel Bispecific Antibody MCLA---117 at EHA 2013 --- Clinical Candidate Designed for the Treatment of Acute Myeloid Leukemia (AML) ---", , 17 June 2013 (2013-06-17), XP55094670, Retrieved from the Internet: URL:http://www.merus.nl/uploads/docs/Merus PR EHA 17 June 2013 final clean.pdf [retrieved on 2014-01-02]
- M. F. Fey ET AL: "Merus Press release", , 7 January 2013 (2013-01-07), XP55094673, DOI: 10.1093/annonc/mdq179 Retrieved from the Internet: URL:http://www.merus.nl/uploads/images/arc hive/20130107_Merus PR first candidate_FINAL.pdf [retrieved on 2014-01-02]
- Www Posterpresentations ET AL: "RESEARCH POSTER PRESENTATION DESIGN 2011 Introduction MCLA-117 induces T cell-mediated target cell lysis MCLA-117-induces T cell proliferation", , 1 January 2011 (2011-01-01), XP055360870, Retrieved from the Internet: URL:http://www.merus.nl/wordpress/wp-conte nt/uploads/2015/10/MCLA-117-Merus-SITC2016 .pdf [retrieved on 2017-03-31]
- Van Loo: "Preclinical Evaluation of MCLA117, a CLEC12AxCD3 Bispecific Antibody Efficiently Targeting a Novel Leukemic Stem Cell Associated Antigen in AML | Blood Journal", , 1 January 2015 (2015-01-01), XP055360857, Retrieved from the Internet: URL:http://www.bloodjournal.org/content/12 6/23/325?sso-checked=true [retrieved on 2017-03-31]
- WIERSMA VALERIE R ET AL: "C-type lectin-like molecule-1 (CLL1)-targeted TRAIL augments the tumoricidal activity of granulocytes and potentiates therapeutic antibody-dependent cell-mediated cytotoxicity", MABS, LANDES BIOSCIENCE, US, vol. 7, no. 2, 1 March 2015 (2015-03-01), pages 321-330, XP009187814, ISSN: 1942-0862, DOI: 10.1080/19420862.2015.1007811
- X. ZHAO ET AL: "Targeting C-type lectin-like molecule-1 for antibody-mediated immunotherapy in acute myeloid leukemia", HAEMATOLOGICA, vol. 95, no. 1, 31 July 2009 (2009-07-31), pages 71-78, XP55094666, ISSN: 0390-6078, DOI: 10.3324/haematol.2009.009811
- STEVEN R. LEONG ET AL: "An anti-CD3/anti-CLL-1 bispecific antibody for the treatment of acute myeloid leukemia", BLOOD, vol. 129, no. 5, 1 December 2016 (2016-12-01), pages 609-618, XP055360863, & 53RD ANNUAL MEETING AND EXPOSITION OF THE AMERICAN-SOCIETY-OF-HEMATOLOGY (ASH); SAN DIEGO, CA, USA; DECEMBER 10 -13, 2011 ISSN: 0006-4971, DOI: 10.1182/blood-2016-08-735365

### SEQUENCE LISTING

<110> NOVARTIS AG
<120> MULTISPECIFIC MOLECULES TARGETING CLL-1
<130> PAT057213-WO-PCT02
<140> PCT/IB2017/050318
   <141> 2017-01-20
<150> PCT/CN2016/071599
   <151> 2016-01-21
<160> 1911
<170> PatentIn version 3.5
<210> 1
   <211> 21
   <212> PRT
   <213> Artificial Sequence
<220>
   <221> source
   <223> /note="Description of Artificial Sequence: Synthetic peptide"
<400> 1
   Met Ala Leu Pro Val Thr Ala Leu Leu Leu Pro Leu Ala Leu Leu Leu
   1               5                   10                  15
   His Ala Ala Arg Pro
               20
<210> 2
   <211> 6
   <212> PRT
   <213> Artificial Sequence
<220>
   <221> source
   <223> /note="Description of Artificial Sequence: Synthetic 6xHis tag"
<400> 2
   His His His His His His
   1               5
<210> 3
<400> 3
   000
<210> 4
<400> 4
   000
<210> 5
<400> 5
   000
<210> 6
<400> 6
   000
<210> 7
<400> 7
   000
<210> 8
<400> 8
   000
<210> 9
<400> 9
   000
<210> 10
<400> 10
   000
<210> 11
<400> 11
   000
<210> 12
   <211> 63
   <212> DNA
   <213> Artificial Sequence
<220>
   <221> source
   <223> /note="Description of Artificial Sequence: Synthetic oligonucleotide"
<400> 12
   atggccctgc ctgtgacagc cctgctgctg cctctggctc tgctgctgca tgccgctaga 60
   ccc 63
<210> 13
<400> 13
   000
<210> 14
<400> 14
   000
<210> 15
<400> 15
   000
<210> 16
<400> 16
   000
<210> 17
<400> 17
   000
<210> 18
<400> 18
   000
<210> 19
<400> 19
   000
<210> 20
<400> 20
   000
<210> 21
<400> 21
   000
<210> 22
<400> 22
   000
<210> 23
<400> 23
   000
<210> 24
<400> 24
   000
<210> 25
   <211> 5
   <212> PRT
   <213> Artificial Sequence
<220>
   <221> source
   <223> /note="Description of Artificial Sequence: Synthetic peptide"
<400> 25
   Gly Gly Gly Gly Ser
   1               5
<210> 26
   <211> 30
   <212> PRT
   <213> Artificial Sequence
<220>
   <221> source
   <223> /note="Description of Artificial Sequence: Synthetic polypeptide"
<220>
   <221> MISC_FEATURE
   <222> (1)..(30)
   <223> /note="This sequence may encompass 1-6 'Gly Gly Gly Gly Ser' repeating units"
<220>
   <221> source
   <223> /note="See specification as filed for detailed description of substitutions and preferred embodiments"
<400> 26
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser Gly Gly Gly Gly Ser
               20                  25                  30
<210> 27
   <211> 20
   <212> PRT
   <213> Artificial Sequence
<220>
   <221> source
   <223> /note="Description of Artificial Sequence: Synthetic peptide"
<400> 27 1 5 10 15
Gly Gly Gly Ser 20
<210> 28
   <211> 15
   <212> PRT
   <213> Artificial Sequence
<220>
   <221> source
   <223> /note="Description of Artificial Sequence: Synthetic peptide"
<400> 28
   Gly Gly Gly Gly Ser Gly Gly Gly Gly Ser Gly Gly Gly Gly Ser
   1               5                   10                  15
<210> 29
   <211> 4
   <212> PRT
   <213> Artificial Sequence
<220>
   <221> source
   <223> /note="Description of Artificial Sequence: Synthetic peptide"
<400> 29
   Gly Gly Gly Ser
   1
<210> 30
   <211> 5000
   <212> DNA
   <213> Artificial Sequence
<220>
   <221> source
   <223> /note="Description of Artificial Sequence: Synthetic polynucleotide"
<220>
   <221> misc_feature
   <222> (1)..(5000)
   <223> /note="This sequence may encompass 50-5000 nucleotides"
<400> 30
   aaaaaaaaaa aaaaaaaaaa aaaaaaaaaa aaaaaaaaaa aaaaaaaaaa aaaaaaaaaa      60
   aaaaaaaaaa aaaaaaaaaa aaaaaaaaaa aaaaaaaaaa aaaaaaaaaa aaaaaaaaaa     120
   aaaaaaaaaa aaaaaaaaaa aaaaaaaaaa aaaaaaaaaa aaaaaaaaaa aaaaaaaaaa     180
   aaaaaaaaaa aaaaaaaaaa aaaaaaaaaa aaaaaaaaaa aaaaaaaaaa aaaaaaaaaa     240
   aaaaaaaaaa aaaaaaaaaa aaaaaaaaaa aaaaaaaaaa aaaaaaaaaa aaaaaaaaaa     300
   aaaaaaaaaa aaaaaaaaaa aaaaaaaaaa aaaaaaaaaa aaaaaaaaaa aaaaaaaaaa 360
   aaaaaaaaaa aaaaaaaaaa aaaaaaaaaa aaaaaaaaaa aaaaaaaaaa aaaaaaaaaa 420
   aaaaaaaaaa aaaaaaaaaa aaaaaaaaaa aaaaaaaaaa aaaaaaaaaa aaaaaaaaaa 480
   aaaaaaaaaa aaaaaaaaaa aaaaaaaaaa aaaaaaaaaa aaaaaaaaaa aaaaaaaaaa 540
   aaaaaaaaaa aaaaaaaaaa aaaaaaaaaa aaaaaaaaaa aaaaaaaaaa aaaaaaaaaa 600
   aaaaaaaaaa aaaaaaaaaa aaaaaaaaaa aaaaaaaaaa aaaaaaaaaa aaaaaaaaaa 660
   aaaaaaaaaa aaaaaaaaaa aaaaaaaaaa aaaaaaaaaa aaaaaaaaaa aaaaaaaaaa 720
   aaaaaaaaaa aaaaaaaaaa aaaaaaaaaa aaaaaaaaaa aaaaaaaaaa aaaaaaaaaa 780
   aaaaaaaaaa aaaaaaaaaa aaaaaaaaaa aaaaaaaaaa aaaaaaaaaa aaaaaaaaaa 840
   aaaaaaaaaa aaaaaaaaaa aaaaaaaaaa aaaaaaaaaa aaaaaaaaaa aaaaaaaaaa 900
   aaaaaaaaaa aaaaaaaaaa aaaaaaaaaa aaaaaaaaaa aaaaaaaaaa aaaaaaaaaa 960
   aaaaaaaaaa aaaaaaaaaa aaaaaaaaaa aaaaaaaaaa aaaaaaaaaa aaaaaaaaaa 1020
   aaaaaaaaaa aaaaaaaaaa aaaaaaaaaa aaaaaaaaaa aaaaaaaaaa aaaaaaaaaa 1080
   aaaaaaaaaa aaaaaaaaaa aaaaaaaaaa aaaaaaaaaa aaaaaaaaaa aaaaaaaaaa 1140
   aaaaaaaaaa aaaaaaaaaa aaaaaaaaaa aaaaaaaaaa aaaaaaaaaa aaaaaaaaaa 1200
   aaaaaaaaaa aaaaaaaaaa aaaaaaaaaa aaaaaaaaaa aaaaaaaaaa aaaaaaaaaa 1260
   aaaaaaaaaa aaaaaaaaaa aaaaaaaaaa aaaaaaaaaa aaaaaaaaaa aaaaaaaaaa 1320
   aaaaaaaaaa aaaaaaaaaa aaaaaaaaaa aaaaaaaaaa aaaaaaaaaa aaaaaaaaaa 1380
   aaaaaaaaaa aaaaaaaaaa aaaaaaaaaa aaaaaaaaaa aaaaaaaaaa aaaaaaaaaa 1440
   aaaaaaaaaa aaaaaaaaaa aaaaaaaaaa aaaaaaaaaa aaaaaaaaaa aaaaaaaaaa 1500
   aaaaaaaaaa aaaaaaaaaa aaaaaaaaaa aaaaaaaaaa aaaaaaaaaa aaaaaaaaaa 1560
   aaaaaaaaaa aaaaaaaaaa aaaaaaaaaa aaaaaaaaaa aaaaaaaaaa aaaaaaaaaa 1620
   aaaaaaaaaa aaaaaaaaaa aaaaaaaaaa aaaaaaaaaa aaaaaaaaaa aaaaaaaaaa 1680
   aaaaaaaaaa aaaaaaaaaa aaaaaaaaaa aaaaaaaaaa aaaaaaaaaa aaaaaaaaaa 1740
   aaaaaaaaaa aaaaaaaaaa aaaaaaaaaa aaaaaaaaaa aaaaaaaaaa aaaaaaaaaa 1800
   aaaaaaaaaa aaaaaaaaaa aaaaaaaaaa aaaaaaaaaa aaaaaaaaaa aaaaaaaaaa 1860
   aaaaaaaaaa aaaaaaaaaa aaaaaaaaaa aaaaaaaaaa aaaaaaaaaa aaaaaaaaaa 1920
   aaaaaaaaaa aaaaaaaaaa aaaaaaaaaa aaaaaaaaaa aaaaaaaaaa aaaaaaaaaa 1980
   aaaaaaaaaa aaaaaaaaaa aaaaaaaaaa aaaaaaaaaa aaaaaaaaaa aaaaaaaaaa 2040
   aaaaaaaaaa aaaaaaaaaa aaaaaaaaaa aaaaaaaaaa aaaaaaaaaa aaaaaaaaaa 2100
   aaaaaaaaaa aaaaaaaaaa aaaaaaaaaa aaaaaaaaaa aaaaaaaaaa aaaaaaaaaa 2160
   aaaaaaaaaa aaaaaaaaaa aaaaaaaaaa aaaaaaaaaa aaaaaaaaaa aaaaaaaaaa 2220
   aaaaaaaaaa aaaaaaaaaa aaaaaaaaaa aaaaaaaaaa aaaaaaaaaa aaaaaaaaaa 2280
   aaaaaaaaaa aaaaaaaaaa aaaaaaaaaa aaaaaaaaaa aaaaaaaaaa aaaaaaaaaa 2340
   aaaaaaaaaa aaaaaaaaaa aaaaaaaaaa aaaaaaaaaa aaaaaaaaaa aaaaaaaaaa 2400
   aaaaaaaaaa aaaaaaaaaa aaaaaaaaaa aaaaaaaaaa aaaaaaaaaa aaaaaaaaaa 2460
   aaaaaaaaaa aaaaaaaaaa aaaaaaaaaa aaaaaaaaaa aaaaaaaaaa aaaaaaaaaa 2520
   aaaaaaaaaa aaaaaaaaaa aaaaaaaaaa aaaaaaaaaa aaaaaaaaaa aaaaaaaaaa 2580
   aaaaaaaaaa aaaaaaaaaa aaaaaaaaaa aaaaaaaaaa aaaaaaaaaa aaaaaaaaaa 2640
   aaaaaaaaaa aaaaaaaaaa aaaaaaaaaa aaaaaaaaaa aaaaaaaaaa aaaaaaaaaa 2700
   aaaaaaaaaa aaaaaaaaaa aaaaaaaaaa aaaaaaaaaa aaaaaaaaaa aaaaaaaaaa 2760
   aaaaaaaaaa aaaaaaaaaa aaaaaaaaaa aaaaaaaaaa aaaaaaaaaa aaaaaaaaaa 2820
   aaaaaaaaaa aaaaaaaaaa aaaaaaaaaa aaaaaaaaaa aaaaaaaaaa aaaaaaaaaa 2880
   aaaaaaaaaa aaaaaaaaaa aaaaaaaaaa aaaaaaaaaa aaaaaaaaaa aaaaaaaaaa 2940
   aaaaaaaaaa aaaaaaaaaa aaaaaaaaaa aaaaaaaaaa aaaaaaaaaa aaaaaaaaaa 3000
   aaaaaaaaaa aaaaaaaaaa aaaaaaaaaa aaaaaaaaaa aaaaaaaaaa aaaaaaaaaa 3060
   aaaaaaaaaa aaaaaaaaaa aaaaaaaaaa aaaaaaaaaa aaaaaaaaaa aaaaaaaaaa 3120
   aaaaaaaaaa aaaaaaaaaa aaaaaaaaaa aaaaaaaaaa aaaaaaaaaa aaaaaaaaaa 3180
   aaaaaaaaaa aaaaaaaaaa aaaaaaaaaa aaaaaaaaaa aaaaaaaaaa aaaaaaaaaa 3240
   aaaaaaaaaa aaaaaaaaaa aaaaaaaaaa aaaaaaaaaa aaaaaaaaaa aaaaaaaaaa 3300
   aaaaaaaaaa aaaaaaaaaa aaaaaaaaaa aaaaaaaaaa aaaaaaaaaa aaaaaaaaaa 3360
   aaaaaaaaaa aaaaaaaaaa aaaaaaaaaa aaaaaaaaaa aaaaaaaaaa aaaaaaaaaa 3420
   aaaaaaaaaa aaaaaaaaaa aaaaaaaaaa aaaaaaaaaa aaaaaaaaaa aaaaaaaaaa 3480
   aaaaaaaaaa aaaaaaaaaa aaaaaaaaaa aaaaaaaaaa aaaaaaaaaa aaaaaaaaaa 3540
   aaaaaaaaaa aaaaaaaaaa aaaaaaaaaa aaaaaaaaaa aaaaaaaaaa aaaaaaaaaa 3600
   aaaaaaaaaa aaaaaaaaaa aaaaaaaaaa aaaaaaaaaa aaaaaaaaaa aaaaaaaaaa 3660
   aaaaaaaaaa aaaaaaaaaa aaaaaaaaaa aaaaaaaaaa aaaaaaaaaa aaaaaaaaaa 3720
   aaaaaaaaaa aaaaaaaaaa aaaaaaaaaa aaaaaaaaaa aaaaaaaaaa aaaaaaaaaa 3780
   aaaaaaaaaa aaaaaaaaaa aaaaaaaaaa aaaaaaaaaa aaaaaaaaaa aaaaaaaaaa 3840
   aaaaaaaaaa aaaaaaaaaa aaaaaaaaaa aaaaaaaaaa aaaaaaaaaa aaaaaaaaaa 3900
   aaaaaaaaaa aaaaaaaaaa aaaaaaaaaa aaaaaaaaaa aaaaaaaaaa aaaaaaaaaa 3960
   aaaaaaaaaa aaaaaaaaaa aaaaaaaaaa aaaaaaaaaa aaaaaaaaaa aaaaaaaaaa 4020
   aaaaaaaaaa aaaaaaaaaa aaaaaaaaaa aaaaaaaaaa aaaaaaaaaa aaaaaaaaaa 4080
   aaaaaaaaaa aaaaaaaaaa aaaaaaaaaa aaaaaaaaaa aaaaaaaaaa aaaaaaaaaa 4140
   aaaaaaaaaa aaaaaaaaaa aaaaaaaaaa aaaaaaaaaa aaaaaaaaaa aaaaaaaaaa 4200
   aaaaaaaaaa aaaaaaaaaa aaaaaaaaaa aaaaaaaaaa aaaaaaaaaa aaaaaaaaaa 4260
   aaaaaaaaaa aaaaaaaaaa aaaaaaaaaa aaaaaaaaaa aaaaaaaaaa aaaaaaaaaa 4320
   aaaaaaaaaa aaaaaaaaaa aaaaaaaaaa aaaaaaaaaa aaaaaaaaaa aaaaaaaaaa 4380
   aaaaaaaaaa aaaaaaaaaa aaaaaaaaaa aaaaaaaaaa aaaaaaaaaa aaaaaaaaaa 4440
   aaaaaaaaaa aaaaaaaaaa aaaaaaaaaa aaaaaaaaaa aaaaaaaaaa aaaaaaaaaa 4500
   aaaaaaaaaa aaaaaaaaaa aaaaaaaaaa aaaaaaaaaa aaaaaaaaaa aaaaaaaaaa 4560
   aaaaaaaaaa aaaaaaaaaa aaaaaaaaaa aaaaaaaaaa aaaaaaaaaa aaaaaaaaaa 4620
   aaaaaaaaaa aaaaaaaaaa aaaaaaaaaa aaaaaaaaaa aaaaaaaaaa aaaaaaaaaa 4680
   aaaaaaaaaa aaaaaaaaaa aaaaaaaaaa aaaaaaaaaa aaaaaaaaaa aaaaaaaaaa 4740
   aaaaaaaaaa aaaaaaaaaa aaaaaaaaaa aaaaaaaaaa aaaaaaaaaa aaaaaaaaaa 4800
   aaaaaaaaaa aaaaaaaaaa aaaaaaaaaa aaaaaaaaaa aaaaaaaaaa aaaaaaaaaa 4860
   aaaaaaaaaa aaaaaaaaaa aaaaaaaaaa aaaaaaaaaa aaaaaaaaaa aaaaaaaaaa 4920
   aaaaaaaaaa aaaaaaaaaa aaaaaaaaaa aaaaaaaaaa aaaaaaaaaa aaaaaaaaaa 4980
   aaaaaaaaaa aaaaaaaaaa 5000
<210> 31
   <211> 5000
   <212> DNA
   <213> Artificial Sequence
<220>
   <221> source
   <223> /note="Description of Artificial Sequence: Synthetic polynucleotide"
<220>
   <221> misc_feature
   <222> (1)..(5000)
   <223> /note="This sequence may encompass 50-5000 nucleotides"
<400> 31
   tttttttttt tttttttttt tttttttttt tttttttttt tttttttttt tttttttttt 60
   tttttttttt tttttttttt tttttttttt tttttttttt tttttttttt tttttttttt 120
   tttttttttt tttttttttt tttttttttt tttttttttt tttttttttt tttttttttt 180
   tttttttttt tttttttttt tttttttttt tttttttttt tttttttttt tttttttttt 240
   tttttttttt tttttttttt tttttttttt tttttttttt tttttttttt tttttttttt 300
   tttttttttt tttttttttt tttttttttt tttttttttt tttttttttt tttttttttt 360
   tttttttttt tttttttttt tttttttttt tttttttttt tttttttttt tttttttttt 420
   tttttttttt tttttttttt tttttttttt tttttttttt tttttttttt tttttttttt 480
   tttttttttt tttttttttt tttttttttt tttttttttt tttttttttt tttttttttt 540
   tttttttttt tttttttttt tttttttttt tttttttttt tttttttttt tttttttttt 600
   tttttttttt tttttttttt tttttttttt tttttttttt tttttttttt tttttttttt 660
   tttttttttt tttttttttt tttttttttt tttttttttt tttttttttt tttttttttt 720
   tttttttttt tttttttttt tttttttttt tttttttttt tttttttttt tttttttttt 780
   tttttttttt tttttttttt tttttttttt tttttttttt tttttttttt tttttttttt 840
   tttttttttt tttttttttt tttttttttt tttttttttt tttttttttt tttttttttt 900
   tttttttttt tttttttttt tttttttttt tttttttttt tttttttttt tttttttttt 960
   tttttttttt tttttttttt tttttttttt tttttttttt tttttttttt tttttttttt 1020
   tttttttttt tttttttttt tttttttttt tttttttttt tttttttttt tttttttttt 1080
   tttttttttt tttttttttt tttttttttt tttttttttt tttttttttt tttttttttt 1140
   tttttttttt tttttttttt tttttttttt tttttttttt tttttttttt tttttttttt 1200
   tttttttttt tttttttttt tttttttttt tttttttttt tttttttttt tttttttttt 1260
   tttttttttt tttttttttt tttttttttt tttttttttt tttttttttt tttttttttt 1320
   tttttttttt tttttttttt tttttttttt tttttttttt tttttttttt tttttttttt 1380
   tttttttttt tttttttttt tttttttttt tttttttttt tttttttttt tttttttttt 1440
   tttttttttt tttttttttt tttttttttt tttttttttt tttttttttt tttttttttt 1500
   tttttttttt tttttttttt tttttttttt tttttttttt tttttttttt tttttttttt 1560
   tttttttttt tttttttttt tttttttttt tttttttttt tttttttttt tttttttttt 1620
   tttttttttt tttttttttt tttttttttt tttttttttt tttttttttt tttttttttt 1680
   tttttttttt tttttttttt tttttttttt tttttttttt tttttttttt tttttttttt 1740
   tttttttttt tttttttttt tttttttttt tttttttttt tttttttttt tttttttttt 1800
   tttttttttt tttttttttt tttttttttt tttttttttt tttttttttt tttttttttt 1860
   tttttttttt tttttttttt tttttttttt tttttttttt tttttttttt tttttttttt 1920
   tttttttttt tttttttttt tttttttttt tttttttttt tttttttttt tttttttttt 1980
   tttttttttt tttttttttt tttttttttt tttttttttt tttttttttt tttttttttt 2040
   tttttttttt tttttttttt tttttttttt tttttttttt tttttttttt tttttttttt 2100
   tttttttttt tttttttttt tttttttttt tttttttttt tttttttttt tttttttttt 2160
   tttttttttt tttttttttt tttttttttt tttttttttt tttttttttt tttttttttt 2220
   tttttttttt tttttttttt tttttttttt tttttttttt tttttttttt tttttttttt 2280
   tttttttttt tttttttttt tttttttttt tttttttttt tttttttttt tttttttttt 2340
   tttttttttt tttttttttt tttttttttt tttttttttt tttttttttt tttttttttt 2400
   tttttttttt tttttttttt tttttttttt tttttttttt tttttttttt tttttttttt 2460
   tttttttttt tttttttttt tttttttttt tttttttttt tttttttttt tttttttttt 2520
   tttttttttt tttttttttt tttttttttt tttttttttt tttttttttt tttttttttt 2580
   tttttttttt tttttttttt tttttttttt tttttttttt tttttttttt tttttttttt 2640
   tttttttttt tttttttttt tttttttttt tttttttttt tttttttttt tttttttttt 2700
   tttttttttt tttttttttt tttttttttt tttttttttt tttttttttt tttttttttt 2760
   tttttttttt tttttttttt tttttttttt tttttttttt tttttttttt tttttttttt 2820
   tttttttttt tttttttttt tttttttttt tttttttttt tttttttttt tttttttttt 2880
   tttttttttt tttttttttt tttttttttt tttttttttt tttttttttt tttttttttt 2940
   tttttttttt tttttttttt tttttttttt tttttttttt tttttttttt tttttttttt 3000
   tttttttttt tttttttttt tttttttttt tttttttttt tttttttttt tttttttttt 3060
   tttttttttt tttttttttt tttttttttt tttttttttt tttttttttt tttttttttt 3120
   tttttttttt tttttttttt tttttttttt tttttttttt tttttttttt tttttttttt 3180
   tttttttttt tttttttttt tttttttttt tttttttttt tttttttttt tttttttttt 3240
   tttttttttt tttttttttt tttttttttt tttttttttt tttttttttt tttttttttt 3300
   tttttttttt tttttttttt tttttttttt tttttttttt tttttttttt tttttttttt 3360
   tttttttttt tttttttttt tttttttttt tttttttttt tttttttttt tttttttttt 3420
   tttttttttt tttttttttt tttttttttt tttttttttt tttttttttt tttttttttt 3480
   tttttttttt tttttttttt tttttttttt tttttttttt tttttttttt tttttttttt 3540
   tttttttttt tttttttttt tttttttttt tttttttttt tttttttttt tttttttttt 3600
   tttttttttt tttttttttt tttttttttt tttttttttt tttttttttt tttttttttt 3660
   tttttttttt tttttttttt tttttttttt tttttttttt tttttttttt tttttttttt 3720
   tttttttttt tttttttttt tttttttttt tttttttttt tttttttttt tttttttttt 3780
   tttttttttt tttttttttt tttttttttt tttttttttt tttttttttt tttttttttt 3840
   tttttttttt tttttttttt tttttttttt tttttttttt tttttttttt tttttttttt 3900
   tttttttttt tttttttttt tttttttttt tttttttttt tttttttttt tttttttttt 3960
   tttttttttt tttttttttt tttttttttt tttttttttt tttttttttt tttttttttt 4020
   tttttttttt tttttttttt tttttttttt tttttttttt tttttttttt tttttttttt 4080
   tttttttttt tttttttttt tttttttttt tttttttttt tttttttttt tttttttttt 4140
   tttttttttt tttttttttt tttttttttt tttttttttt tttttttttt tttttttttt 4200
   tttttttttt tttttttttt tttttttttt tttttttttt tttttttttt tttttttttt 4260
   tttttttttt tttttttttt tttttttttt tttttttttt tttttttttt tttttttttt 4320
   tttttttttt tttttttttt tttttttttt tttttttttt tttttttttt tttttttttt 4380
   tttttttttt tttttttttt tttttttttt tttttttttt tttttttttt tttttttttt 4440
   tttttttttt tttttttttt tttttttttt tttttttttt tttttttttt tttttttttt 4500
   tttttttttt tttttttttt tttttttttt tttttttttt tttttttttt tttttttttt 4560
   tttttttttt tttttttttt tttttttttt tttttttttt tttttttttt tttttttttt 4620
   tttttttttt tttttttttt tttttttttt tttttttttt tttttttttt tttttttttt 4680
   tttttttttt tttttttttt tttttttttt tttttttttt tttttttttt tttttttttt 4740
   tttttttttt tttttttttt tttttttttt tttttttttt tttttttttt tttttttttt 4800
   tttttttttt tttttttttt tttttttttt tttttttttt tttttttttt tttttttttt 4860
   tttttttttt tttttttttt tttttttttt tttttttttt tttttttttt tttttttttt 4920
   tttttttttt tttttttttt tttttttttt tttttttttt tttttttttt tttttttttt 4980
   tttttttttt tttttttttt 5000
<210> 32
<400> 32
   000
<210> 33
   <211> 5000
   <212> DNA
   <213> Artificial Sequence
<220>
   <221> source
   <223> /note="Description of Artificial Sequence: Synthetic polynucleotide"
<220>
   <221> misc_feature
   <222> (1)..(5000)
   <223> /note="This sequence may encompass 100-5000 nucleotides"
<400> 33
   aaaaaaaaaa aaaaaaaaaa aaaaaaaaaa aaaaaaaaaa aaaaaaaaaa aaaaaaaaaa 60
   aaaaaaaaaa aaaaaaaaaa aaaaaaaaaa aaaaaaaaaa aaaaaaaaaa aaaaaaaaaa 120
   aaaaaaaaaa aaaaaaaaaa aaaaaaaaaa aaaaaaaaaa aaaaaaaaaa aaaaaaaaaa 180
   aaaaaaaaaa aaaaaaaaaa aaaaaaaaaa aaaaaaaaaa aaaaaaaaaa aaaaaaaaaa 240
   aaaaaaaaaa aaaaaaaaaa aaaaaaaaaa aaaaaaaaaa aaaaaaaaaa aaaaaaaaaa 300
   aaaaaaaaaa aaaaaaaaaa aaaaaaaaaa aaaaaaaaaa aaaaaaaaaa aaaaaaaaaa      360
   aaaaaaaaaa aaaaaaaaaa aaaaaaaaaa aaaaaaaaaa aaaaaaaaaa aaaaaaaaaa      420
   aaaaaaaaaa aaaaaaaaaa aaaaaaaaaa aaaaaaaaaa aaaaaaaaaa aaaaaaaaaa      480
   aaaaaaaaaa aaaaaaaaaa aaaaaaaaaa aaaaaaaaaa aaaaaaaaaa aaaaaaaaaa      540
   aaaaaaaaaa aaaaaaaaaa aaaaaaaaaa aaaaaaaaaa aaaaaaaaaa aaaaaaaaaa      600
   aaaaaaaaaa aaaaaaaaaa aaaaaaaaaa aaaaaaaaaa aaaaaaaaaa aaaaaaaaaa      660
   aaaaaaaaaa aaaaaaaaaa aaaaaaaaaa aaaaaaaaaa aaaaaaaaaa aaaaaaaaaa      720
   aaaaaaaaaa aaaaaaaaaa aaaaaaaaaa aaaaaaaaaa aaaaaaaaaa aaaaaaaaaa      780
   aaaaaaaaaa aaaaaaaaaa aaaaaaaaaa aaaaaaaaaa aaaaaaaaaa aaaaaaaaaa      840
   aaaaaaaaaa aaaaaaaaaa aaaaaaaaaa aaaaaaaaaa aaaaaaaaaa aaaaaaaaaa      900
   aaaaaaaaaa aaaaaaaaaa aaaaaaaaaa aaaaaaaaaa aaaaaaaaaa aaaaaaaaaa      960
   aaaaaaaaaa aaaaaaaaaa aaaaaaaaaa aaaaaaaaaa aaaaaaaaaa aaaaaaaaaa     1020
   aaaaaaaaaa aaaaaaaaaa aaaaaaaaaa aaaaaaaaaa aaaaaaaaaa aaaaaaaaaa     1080
   aaaaaaaaaa aaaaaaaaaa aaaaaaaaaa aaaaaaaaaa aaaaaaaaaa aaaaaaaaaa     1140
   aaaaaaaaaa aaaaaaaaaa aaaaaaaaaa aaaaaaaaaa aaaaaaaaaa aaaaaaaaaa     1200
   aaaaaaaaaa aaaaaaaaaa aaaaaaaaaa aaaaaaaaaa aaaaaaaaaa aaaaaaaaaa     1260
   aaaaaaaaaa aaaaaaaaaa aaaaaaaaaa aaaaaaaaaa aaaaaaaaaa aaaaaaaaaa     1320
   aaaaaaaaaa aaaaaaaaaa aaaaaaaaaa aaaaaaaaaa aaaaaaaaaa aaaaaaaaaa     1380
   aaaaaaaaaa aaaaaaaaaa aaaaaaaaaa aaaaaaaaaa aaaaaaaaaa aaaaaaaaaa     1440
   aaaaaaaaaa aaaaaaaaaa aaaaaaaaaa aaaaaaaaaa aaaaaaaaaa aaaaaaaaaa     1500
   aaaaaaaaaa aaaaaaaaaa aaaaaaaaaa aaaaaaaaaa aaaaaaaaaa aaaaaaaaaa     1560
   aaaaaaaaaa aaaaaaaaaa aaaaaaaaaa aaaaaaaaaa aaaaaaaaaa aaaaaaaaaa     1620
   aaaaaaaaaa aaaaaaaaaa aaaaaaaaaa aaaaaaaaaa aaaaaaaaaa aaaaaaaaaa     1680
   aaaaaaaaaa aaaaaaaaaa aaaaaaaaaa aaaaaaaaaa aaaaaaaaaa aaaaaaaaaa     1740
   aaaaaaaaaa aaaaaaaaaa aaaaaaaaaa aaaaaaaaaa aaaaaaaaaa aaaaaaaaaa     1800
   aaaaaaaaaa aaaaaaaaaa aaaaaaaaaa aaaaaaaaaa aaaaaaaaaa aaaaaaaaaa     1860
   aaaaaaaaaa aaaaaaaaaa aaaaaaaaaa aaaaaaaaaa aaaaaaaaaa aaaaaaaaaa     1920
   aaaaaaaaaa aaaaaaaaaa aaaaaaaaaa aaaaaaaaaa aaaaaaaaaa aaaaaaaaaa     1980
   aaaaaaaaaa aaaaaaaaaa aaaaaaaaaa aaaaaaaaaa aaaaaaaaaa aaaaaaaaaa     2040
   aaaaaaaaaa aaaaaaaaaa aaaaaaaaaa aaaaaaaaaa aaaaaaaaaa aaaaaaaaaa     2100
   aaaaaaaaaa aaaaaaaaaa aaaaaaaaaa aaaaaaaaaa aaaaaaaaaa aaaaaaaaaa     2160
   aaaaaaaaaa aaaaaaaaaa aaaaaaaaaa aaaaaaaaaa aaaaaaaaaa aaaaaaaaaa 2220
   aaaaaaaaaa aaaaaaaaaa aaaaaaaaaa aaaaaaaaaa aaaaaaaaaa aaaaaaaaaa 2280
   aaaaaaaaaa aaaaaaaaaa aaaaaaaaaa aaaaaaaaaa aaaaaaaaaa aaaaaaaaaa 2340
   aaaaaaaaaa aaaaaaaaaa aaaaaaaaaa aaaaaaaaaa aaaaaaaaaa aaaaaaaaaa 2400
   aaaaaaaaaa aaaaaaaaaa aaaaaaaaaa aaaaaaaaaa aaaaaaaaaa aaaaaaaaaa 2460
   aaaaaaaaaa aaaaaaaaaa aaaaaaaaaa aaaaaaaaaa aaaaaaaaaa aaaaaaaaaa 2520
   aaaaaaaaaa aaaaaaaaaa aaaaaaaaaa aaaaaaaaaa aaaaaaaaaa aaaaaaaaaa 2580
   aaaaaaaaaa aaaaaaaaaa aaaaaaaaaa aaaaaaaaaa aaaaaaaaaa aaaaaaaaaa 2640
   aaaaaaaaaa aaaaaaaaaa aaaaaaaaaa aaaaaaaaaa aaaaaaaaaa aaaaaaaaaa 2700
   aaaaaaaaaa aaaaaaaaaa aaaaaaaaaa aaaaaaaaaa aaaaaaaaaa aaaaaaaaaa 2760
   aaaaaaaaaa aaaaaaaaaa aaaaaaaaaa aaaaaaaaaa aaaaaaaaaa aaaaaaaaaa 2820
   aaaaaaaaaa aaaaaaaaaa aaaaaaaaaa aaaaaaaaaa aaaaaaaaaa aaaaaaaaaa 2880
   aaaaaaaaaa aaaaaaaaaa aaaaaaaaaa aaaaaaaaaa aaaaaaaaaa aaaaaaaaaa 2940
   aaaaaaaaaa aaaaaaaaaa aaaaaaaaaa aaaaaaaaaa aaaaaaaaaa aaaaaaaaaa 3000
   aaaaaaaaaa aaaaaaaaaa aaaaaaaaaa aaaaaaaaaa aaaaaaaaaa aaaaaaaaaa 3060
   aaaaaaaaaa aaaaaaaaaa aaaaaaaaaa aaaaaaaaaa aaaaaaaaaa aaaaaaaaaa 3120
   aaaaaaaaaa aaaaaaaaaa aaaaaaaaaa aaaaaaaaaa aaaaaaaaaa aaaaaaaaaa 3180
   aaaaaaaaaa aaaaaaaaaa aaaaaaaaaa aaaaaaaaaa aaaaaaaaaa aaaaaaaaaa 3240
   aaaaaaaaaa aaaaaaaaaa aaaaaaaaaa aaaaaaaaaa aaaaaaaaaa aaaaaaaaaa 3300
   aaaaaaaaaa aaaaaaaaaa aaaaaaaaaa aaaaaaaaaa aaaaaaaaaa aaaaaaaaaa 3360
   aaaaaaaaaa aaaaaaaaaa aaaaaaaaaa aaaaaaaaaa aaaaaaaaaa aaaaaaaaaa 3420
   aaaaaaaaaa aaaaaaaaaa aaaaaaaaaa aaaaaaaaaa aaaaaaaaaa aaaaaaaaaa 3480
   aaaaaaaaaa aaaaaaaaaa aaaaaaaaaa aaaaaaaaaa aaaaaaaaaa aaaaaaaaaa 3540
   aaaaaaaaaa aaaaaaaaaa aaaaaaaaaa aaaaaaaaaa aaaaaaaaaa aaaaaaaaaa 3600
   aaaaaaaaaa aaaaaaaaaa aaaaaaaaaa aaaaaaaaaa aaaaaaaaaa aaaaaaaaaa 3660
   aaaaaaaaaa aaaaaaaaaa aaaaaaaaaa aaaaaaaaaa aaaaaaaaaa aaaaaaaaaa 3720
   aaaaaaaaaa aaaaaaaaaa aaaaaaaaaa aaaaaaaaaa aaaaaaaaaa aaaaaaaaaa 3780
   aaaaaaaaaa aaaaaaaaaa aaaaaaaaaa aaaaaaaaaa aaaaaaaaaa aaaaaaaaaa 3840
   aaaaaaaaaa aaaaaaaaaa aaaaaaaaaa aaaaaaaaaa aaaaaaaaaa aaaaaaaaaa 3900
   aaaaaaaaaa aaaaaaaaaa aaaaaaaaaa aaaaaaaaaa aaaaaaaaaa aaaaaaaaaa 3960
   aaaaaaaaaa aaaaaaaaaa aaaaaaaaaa aaaaaaaaaa aaaaaaaaaa aaaaaaaaaa 4020
   aaaaaaaaaa aaaaaaaaaa aaaaaaaaaa aaaaaaaaaa aaaaaaaaaa aaaaaaaaaa 4080
   aaaaaaaaaa aaaaaaaaaa aaaaaaaaaa aaaaaaaaaa aaaaaaaaaa aaaaaaaaaa 4140
   aaaaaaaaaa aaaaaaaaaa aaaaaaaaaa aaaaaaaaaa aaaaaaaaaa aaaaaaaaaa 4200
   aaaaaaaaaa aaaaaaaaaa aaaaaaaaaa aaaaaaaaaa aaaaaaaaaa aaaaaaaaaa 4260
   aaaaaaaaaa aaaaaaaaaa aaaaaaaaaa aaaaaaaaaa aaaaaaaaaa aaaaaaaaaa 4320
   aaaaaaaaaa aaaaaaaaaa aaaaaaaaaa aaaaaaaaaa aaaaaaaaaa aaaaaaaaaa 4380
   aaaaaaaaaa aaaaaaaaaa aaaaaaaaaa aaaaaaaaaa aaaaaaaaaa aaaaaaaaaa 4440
   aaaaaaaaaa aaaaaaaaaa aaaaaaaaaa aaaaaaaaaa aaaaaaaaaa aaaaaaaaaa 4500
   aaaaaaaaaa aaaaaaaaaa aaaaaaaaaa aaaaaaaaaa aaaaaaaaaa aaaaaaaaaa 4560
   aaaaaaaaaa aaaaaaaaaa aaaaaaaaaa aaaaaaaaaa aaaaaaaaaa aaaaaaaaaa 4620
   aaaaaaaaaa aaaaaaaaaa aaaaaaaaaa aaaaaaaaaa aaaaaaaaaa aaaaaaaaaa 4680
   aaaaaaaaaa aaaaaaaaaa aaaaaaaaaa aaaaaaaaaa aaaaaaaaaa aaaaaaaaaa 4740
   aaaaaaaaaa aaaaaaaaaa aaaaaaaaaa aaaaaaaaaa aaaaaaaaaa aaaaaaaaaa 4800
   aaaaaaaaaa aaaaaaaaaa aaaaaaaaaa aaaaaaaaaa aaaaaaaaaa aaaaaaaaaa 4860
   aaaaaaaaaa aaaaaaaaaa aaaaaaaaaa aaaaaaaaaa aaaaaaaaaa aaaaaaaaaa 4920
   aaaaaaaaaa aaaaaaaaaa aaaaaaaaaa aaaaaaaaaa aaaaaaaaaa aaaaaaaaaa 4980
   aaaaaaaaaa aaaaaaaaaa 5000
<210> 34
<400> 34
   000
<210> 35
   <211> 2000
   <212> DNA
   <213> Artificial Sequence
<220>
   <221> source
   <223> /note="Description of Artificial Sequence: Synthetic polynucleotide"
<220>
   <221> misc_feature
   <222> (1)..(2000)
   <223> /note="This sequence may encompass 50-2000 nucleotides"
<400> 35
   aaaaaaaaaa aaaaaaaaaa aaaaaaaaaa aaaaaaaaaa aaaaaaaaaa aaaaaaaaaa 60
   aaaaaaaaaa aaaaaaaaaa aaaaaaaaaa aaaaaaaaaa aaaaaaaaaa aaaaaaaaaa 120
   aaaaaaaaaa aaaaaaaaaa aaaaaaaaaa aaaaaaaaaa aaaaaaaaaa aaaaaaaaaa 180
   aaaaaaaaaa aaaaaaaaaa aaaaaaaaaa aaaaaaaaaa aaaaaaaaaa aaaaaaaaaa      240
   aaaaaaaaaa aaaaaaaaaa aaaaaaaaaa aaaaaaaaaa aaaaaaaaaa aaaaaaaaaa      300
   aaaaaaaaaa aaaaaaaaaa aaaaaaaaaa aaaaaaaaaa aaaaaaaaaa aaaaaaaaaa      360
   aaaaaaaaaa aaaaaaaaaa aaaaaaaaaa aaaaaaaaaa aaaaaaaaaa aaaaaaaaaa      420
   aaaaaaaaaa aaaaaaaaaa aaaaaaaaaa aaaaaaaaaa aaaaaaaaaa aaaaaaaaaa      480
   aaaaaaaaaa aaaaaaaaaa aaaaaaaaaa aaaaaaaaaa aaaaaaaaaa aaaaaaaaaa      540
   aaaaaaaaaa aaaaaaaaaa aaaaaaaaaa aaaaaaaaaa aaaaaaaaaa aaaaaaaaaa      600
   aaaaaaaaaa aaaaaaaaaa aaaaaaaaaa aaaaaaaaaa aaaaaaaaaa aaaaaaaaaa      660
   aaaaaaaaaa aaaaaaaaaa aaaaaaaaaa aaaaaaaaaa aaaaaaaaaa aaaaaaaaaa      720
   aaaaaaaaaa aaaaaaaaaa aaaaaaaaaa aaaaaaaaaa aaaaaaaaaa aaaaaaaaaa      780
   aaaaaaaaaa aaaaaaaaaa aaaaaaaaaa aaaaaaaaaa aaaaaaaaaa aaaaaaaaaa      840
   aaaaaaaaaa aaaaaaaaaa aaaaaaaaaa aaaaaaaaaa aaaaaaaaaa aaaaaaaaaa      900
   aaaaaaaaaa aaaaaaaaaa aaaaaaaaaa aaaaaaaaaa aaaaaaaaaa aaaaaaaaaa      960
   aaaaaaaaaa aaaaaaaaaa aaaaaaaaaa aaaaaaaaaa aaaaaaaaaa aaaaaaaaaa     1020
   aaaaaaaaaa aaaaaaaaaa aaaaaaaaaa aaaaaaaaaa aaaaaaaaaa aaaaaaaaaa     1080
   aaaaaaaaaa aaaaaaaaaa aaaaaaaaaa aaaaaaaaaa aaaaaaaaaa aaaaaaaaaa     1140
   aaaaaaaaaa aaaaaaaaaa aaaaaaaaaa aaaaaaaaaa aaaaaaaaaa aaaaaaaaaa     1200
   aaaaaaaaaa aaaaaaaaaa aaaaaaaaaa aaaaaaaaaa aaaaaaaaaa aaaaaaaaaa     1260
   aaaaaaaaaa aaaaaaaaaa aaaaaaaaaa aaaaaaaaaa aaaaaaaaaa aaaaaaaaaa     1320
   aaaaaaaaaa aaaaaaaaaa aaaaaaaaaa aaaaaaaaaa aaaaaaaaaa aaaaaaaaaa     1380
   aaaaaaaaaa aaaaaaaaaa aaaaaaaaaa aaaaaaaaaa aaaaaaaaaa aaaaaaaaaa     1440
   aaaaaaaaaa aaaaaaaaaa aaaaaaaaaa aaaaaaaaaa aaaaaaaaaa aaaaaaaaaa     1500
   aaaaaaaaaa aaaaaaaaaa aaaaaaaaaa aaaaaaaaaa aaaaaaaaaa aaaaaaaaaa     1560
   aaaaaaaaaa aaaaaaaaaa aaaaaaaaaa aaaaaaaaaa aaaaaaaaaa aaaaaaaaaa     1620
   aaaaaaaaaa aaaaaaaaaa aaaaaaaaaa aaaaaaaaaa aaaaaaaaaa aaaaaaaaaa     1680
   aaaaaaaaaa aaaaaaaaaa aaaaaaaaaa aaaaaaaaaa aaaaaaaaaa aaaaaaaaaa     1740
   aaaaaaaaaa aaaaaaaaaa aaaaaaaaaa aaaaaaaaaa aaaaaaaaaa aaaaaaaaaa     1800
   aaaaaaaaaa aaaaaaaaaa aaaaaaaaaa aaaaaaaaaa aaaaaaaaaa aaaaaaaaaa     1860
   aaaaaaaaaa aaaaaaaaaa aaaaaaaaaa aaaaaaaaaa aaaaaaaaaa aaaaaaaaaa     1920
   aaaaaaaaaa aaaaaaaaaa aaaaaaaaaa aaaaaaaaaa aaaaaaaaaa aaaaaaaaaa     1980
   aaaaaaaaaa aaaaaaaaaa                                                 2000
<210> 36
<400> 36
   000
<210> 37
<400> 37
   000
<210> 38
   <211> 40
   <212> PRT
   <213> Artificial Sequence
<220>
   <221> source
   <223> /note="Description of Artificial Sequence: Synthetic polypeptide"
<220>
   <221> MISC_FEATURE
   <222> (1)..(40)
   <223> /note="This sequence may encompass 1-10 'Gly Gly Gly Ser' repeating units"
<400> 38
   Gly Gly Gly Ser Gly Gly Gly Ser Gly Gly Gly Ser Gly Gly Gly Ser
   1               5                   10                  15
   Gly Gly Gly Ser Gly Gly Gly Ser Gly Gly Gly Ser Gly Gly Gly Ser
               20                  25                  30
   Gly Gly Gly Ser Gly Gly Gly Ser
           35                  40
<210> 39
   <211> 247
   <212> PRT
   <213> Artificial Sequence
<220>
   <221> source
   <223> /note="Description of Artificial Sequence: Synthetic polypeptide"
<400> 39
   Glu Val Gln Leu Gln Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Gly Ile Ile Pro Ile Phe Gly Thr Ala Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asp Leu Glu Met Ala Thr Ile Met Gly Gly Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Gln Ser Ala Leu Thr Gln Pro Ala Ser
       130                 135                 140
   Val Ser Gly Ser Pro Gly Gln Ser Ile Thr Ile Ser Cys Thr Gly Thr
   145                 150                 155                 160
   Ser Ser Asp Val Gly Gly Tyr Asn Tyr Val Ser Trp Tyr Gln Gln His
                   165                 170                 175
   Pro Gly Lys Ala Pro Lys Leu Met Ile Tyr Asp Val Ser Asn Arg Pro
               180                 185                 190
   Ser Gly Val Ser Asn Arg Phe Ser Gly Ser Lys Ser Gly Asn Thr Ala
           195                 200                 205
   Ser Leu Thr Ile Ser Gly Leu Gln Ala Glu Asp Glu Ala Asp Tyr Tyr
       210                 215                 220
   Cys Ser Ser Tyr Thr Ser Ser Ser Thr Leu Asp Val Val Phe Gly Gly
   225                 230                 235                 240
   Gly Thr Lys Leu Thr Val Leu
                   245
<210> 40
   <211> 245
   <212> PRT
   <213> Artificial Sequence
<220>
   <221> source
   <223> /note="Description of Artificial Sequence: Synthetic polypeptide"
<400> 40
   Glu Val Gln Leu Val Glu Ser Gly Gly Gly Val Val Gln Pro Gly Gly
   1 5 10 15
   Ser Leu Arg Leu Ser Cys Ala Ala Ser Gly Phe Thr Phe Asp Asp Tyr
   20 25 30
   Ala Met His Trp Val Arg Gln Ala Pro Gly Lys Gly Leu Glu Trp Val
   35 40 45
   Ser Leu Ile Ser Gly Asp Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Val Glu Asp Thr Ala Val Tyr Tyr Cys
   85 90 95
   Ala Arg Val Phe Asp Ser Tyr Tyr Met Asp Val Trp Gly Lys Gly Thr
   100 105 110
   Thr Val Thr Val Ser Ser Gly Gly Gly Gly Ser Gly Gly Gly Gly Ser
   115 120 125
   Gly Ser Gly Gly Ser Glu Ile Val Leu Thr Gln Ser Pro Leu Ser Leu
   130 135 140
   Pro Val Thr Pro Gly Gln Pro Ala Ser Ile Ser Cys Arg Ser Ser Gln
   145 150 155 160
   Ser Leu Val Tyr Thr Asp Gly Asn Thr Tyr Leu Asn Trp Phe Gln Gln
   165 170 175
   Arg Pro Gly Gln Ser Pro Arg Arg Leu Ile Tyr Lys Val Ser Asn Arg
   180 185 190
   Asp Ser Gly Val Pro Asp Arg Phe Ser Gly Ser Gly Ser Asp Thr Asp
   195 200 205
   Phe Thr Leu Lys Ile Ser Arg Val Glu Ala Glu Asp Val Gly Ile Tyr
   210 215 220
   Tyr Cys Met Gln Gly Thr His Trp Ser Phe Thr Phe Gly Gln Gly Thr
   225 230 235 240
   Arg Leu Glu Ile Lys
   245
<210> 41
   <211> 247
   <212> PRT
   <213> Artificial Sequence
<220>
   <221> source
   <223> /note="Description of Artificial Sequence: Synthetic polypeptide"
<400> 41
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Gly Trp Ile Arg Gln Pro Pro Gly Lys Gly Leu Glu
           35                  40                  45
   Trp Ile Gly Ser Ile Tyr Tyr Ser Gly Ser Thr Tyr Tyr Asn Pro Ser
       50                  55                  60
   Leu Lys Ser Arg Val Ser Ile Ser Val Asp Thr Ser Lys Asn Gln Phe
   65                  70                  75                  80
   Ser Leu Lys Leu Lys Tyr Val Thr Ala Ala Asp Thr Ala Val Tyr Tyr
                   85                  90                  95
   Cys Ala Thr Pro Gly Thr Tyr Tyr Asp Phe Leu Ser Gly Tyr Tyr Pro
               100                 105                 110
   Phe Tyr Trp Gly Gln Gly Thr Leu Val Thr Val Ser Ser Gly Gly Gly
           115                 120                 125
   Gly Ser Gly Gly Gly Gly Ser Gly Gly Gly Gly Ser Asp Ile Val Met
       130                 135                 140
   Thr Gln Ser Pro Ser Ser Leu Ser Ala Ser Val Gly Asp Arg Val Thr
   145                 150                 155                 160
   Ile Thr Cys Arg Ala Ser Gln Gly Ile Ser Ser Tyr Leu Ala Trp Tyr
                   165                 170                 175
   Gln Gln Lys Pro Gly Lys Ala Pro Lys Leu Leu Ile Tyr Ala Ala Ser
               180                 185                 190
   Thr Leu Gln Ser Gly Val Pro Ser Arg Phe Ser Gly Ser Gly Ser Gly
   195 200 205
   Thr Asp Phe Thr Leu Thr Ile Ser Ser Leu Gln Pro Glu Asp Phe Ala
   210 215 220
   Thr Tyr Tyr Cys Gln Gln Leu Asn Ser Tyr Pro Tyr Thr Phe Gly Gln
   225 230 235 240
   Gly Thr Lys Leu Glu Ile Lys
   245
<210> 42
   <211> 239
   <212> PRT
   <213> Artificial Sequence
<220>
   <221> source
   <223> /note="Description of Artificial Sequence: Synthetic polypeptide"
<400> 42
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Trp Met Ser Trp Val Arg Gln Ala Pro Gly Lys Gly Leu Glu Trp Val
           35                  40                  45
   Ala Asn Ile Asn Glu Asp Gly Ser Ala Lys Phe Tyr Val Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Phe Cys
                   85                  90                  95
   Ala Arg Asp Leu Arg Ser Gly Arg Tyr Trp Gly Gln Gly Thr Leu Val
               100                 105                 110
   Thr Val Ser Ser Gly Gly Gly Gly Ser Gly Gly Gly Gly Ser Gly Gly
           115                 120                 125
   Gly Gly Ser Glu Ile Val Leu Thr Gln Ser Pro Gly Thr Leu Ser Leu
       130                 135                 140
   Ser Pro Gly Gly Arg Ala Thr Leu Ser Cys Arg Ala Ser Gln Ser Ile
   145                 150                 155                 160
   Ser Gly Ser Phe Leu Ala Trp Tyr Gln Gln Lys Pro Gly Gln Ala Pro
                   165                 170                 175
   Arg Leu Leu Ile Tyr Gly Ala Ser Ser Arg Ala Thr Gly Ile Pro Asp
               180                 185                 190
   Arg Phe Ser Gly Ser Gly Ser Gly Thr Asp Phe Thr Leu Thr Ile Ser
           195                 200                 205
   Arg Leu Glu Pro Glu Asp Phe Ala Val Tyr Tyr Cys Gln Gln Tyr Gly
       210                 215                 220
   Ser Ser Pro Pro Thr Phe Gly Leu Gly Thr Lys Leu Glu Ile Lys
   225                 230                 235
<210> 43
   <211> 245
   <212> PRT
   <213> Artificial Sequence
<220>
   <221> source
   <223> /note="Description of Artificial Sequence: Synthetic polypeptide"
<400> 43
   Glu Val Gln Leu Gln Gln Ser Gly Pro Gly Leu Val Arg Pro Ser Glu
   1               5                   10                  15
   Thr Leu Ser Leu Thr Cys Thr Val Ser Gly Gly Pro Val Arg Ser Gly
               20                  25                  30
   Ser His Tyr Trp Asn Trp Ile Arg Gln Pro Pro Gly Arg Gly Leu Glu
           35                  40                  45
   Trp Ile Gly Tyr Ile Tyr Tyr Ser Gly Ser Thr Asn Tyr Asn Pro Ser
       50                  55                  60
   Leu Glu Asn Arg Val Thr Ile Ser Ile Asp Thr Ser Asn Asn His Phe
   65                  70                  75                  80
   Ser Leu Lys Leu Ser Ser Val Thr Ala Ala Asp Thr Ala Leu Tyr Phe
                   85                  90                  95
   Cys Ala Arg Gly Thr Ala Thr Phe Asp Trp Asn Phe Pro Phe Asp Ser
               100                 105                 110
   Trp Gly Gln Gly Thr Leu Val Thr Val Ser Ser Gly Gly Gly Gly Ser
                   115                 120                 125
   Gly Gly Gly Gly Ser Gly Ser Gly Gly Ser Asp Ile Gln Met Thr Gln
       130                 135                 140
   Ser Pro Ser Ser Leu Ser Ala Ser Ile Gly Asp Arg Val Thr Ile Thr
   145                 150                 155                 160
   Cys Arg Ala Ser Gln Ser Ile Ser Ser Tyr Leu Asn Trp Tyr Gln Gln
                   165                 170                 175
   Lys Pro Gly Lys Ala Pro Lys Leu Leu Ile Tyr Ala Ala Ser Ser Leu
               180                 185                 190
   Gln Ser Gly Val Pro Ser Arg Phe Ser Gly Ser Gly Ser Gly Thr Asp
           195                 200                 205
   Phe Thr Leu Thr Ile Ser Ser Leu Gln Pro Glu Asp Phe Ala Thr Tyr
       210                 215                 220
   Tyr Cys Gln Gln Ser Tyr Ser Thr Pro Trp Thr Phe Gly Gln Gly Thr
   225                 230                 235                 240
   Lys Leu Glu Ile Lys
                   245
<210> 44
   <211> 249
   <212> PRT
   <213> Artificial Sequence
<220>
   <221> source
   <223> /note="Description of Artificial Sequence: Synthetic polypeptide"
<400> 44
   Gln Val Gln Leu Gln Glu Ser Gly Ala Gly Leu Leu Lys Pro Ser Glu
   1               5                   10                  15
   Thr Leu Ser Leu Thr Cys Ala Val Tyr Gly Gly Ser Phe Ser Gly Tyr
               20                  25                  30
   Tyr Trp Ser Trp Ile Arg Gln Pro Pro Gly Lys Gly Leu Glu Trp Val
           35                  40                  45
   Gly Glu Ile Asn His Ser Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Gly Ser Gly Leu Val Val Tyr Ala Ile Arg Val Gly Ser Gly Trp
               100                 105                 110
   Phe Asp Tyr Trp Gly Gln Gly Thr Leu Val Thr Val Ser Ser Gly Gly
           115                 120                 125
   Gly Gly Ser Gly Gly Gly Asp Ser Gly Gly Gly Gly Ser Asp Ile Gln
       130                 135                 140
   Met Thr Gln Ser Pro Ser Ser Leu Ser Ala Ser Val Gly Asp Arg Val
   145                 150                 155                 160
   Thr Ile Thr Cys Arg Ala Ser Gln Ser Ile Ser Ser Tyr Leu Asn Trp
                   165                 170                 175
   Tyr Gln Gln Lys Pro Gly Lys Ala Pro Lys Leu Leu Met Tyr Ala Ala
               180                 185                 190
   Ser Ser Leu Gln Ser Gly Val Pro Ser Arg Phe Ser Gly Ser Gly Ser
           195                 200                 205
   Gly Thr Asp Phe Thr Leu Thr Ile Ser Ser Leu Gln Pro Glu Asp Phe
       210                 215                 220
   Ala Thr Tyr Tyr Cys Gln Gln Ser Tyr Ser Thr Pro Pro Trp Thr Phe
   225                 230                 235                 240
   Gly Gln Gly Thr Lys Val Asp Ile Lys
                   245
<210> 45
   <211> 255
   <212> PRT
   <213> Artificial Sequence
<220>
   <221> source
   <223> /note="Description of Artificial Sequence: Synthetic polypeptide"
<400> 45
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Ser Tyr
   20 25 30
   Ser Met Asn Trp Val Arg Gln Ala Pro Gly Lys Gly Leu Glu Trp Val
   35 40 45
   Ser Ser Ile Ser Ser Ser Ser Ser Tyr Ile Tyr Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Asp Pro Ser Ser Ser Gly Ser Tyr Tyr Met Glu Asp Ser Tyr
   100 105 110
   Tyr Tyr Gly Met Asp Val Trp Gly Gln Gly Thr Thr Val Thr Val Ser
   115 120 125
   Ser Gly Gly Gly Gly Ser Gly Gly Gly Gly Ser Gly Gly Gly Gly Ser
   130 135 140
   Asn Phe Met Leu Thr Gln Pro His Ser Val Ser Glu Ser Pro Gly Lys
   145 150 155 160
   Thr Val Thr Ile Ser Cys Thr Gly Ser Ser Gly Ser Ile Ala Ser Asn
   165 170 175
   Tyr Val Gln Trp Tyr Gln Gln Arg Pro Gly Ser Ala Pro Thr Thr Val
   180 185 190
   Ile Tyr Glu Asp Asn Gln Arg Pro Ser Gly Val Pro Asp Arg Phe Ser
   195 200 205
   Gly Ser Ile Asp Ser Ser Ser Asn Ser Ala Ser Leu Thr Ile Ser Gly
   210 215 220
   Leu Lys Thr Glu Asp Glu Ala Asp Tyr Tyr Cys Gln Ser Tyr Asp Ser
   225 230 235 240
   Ser Asn Gln Val Val Phe Gly Gly Gly Thr Lys Leu Thr Val Leu
   245 250 255
<210> 46
   <211> 239
   <212> PRT
   <213> Artificial Sequence
<220>
   <221> source
   <223> /note="Description of Artificial Sequence: Synthetic polypeptide"
<400> 46
   Gln Val Asn Leu Arg Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Glu Met Asn Trp Val Arg Gln Ala Pro Gly Lys Gly Leu Glu Trp Val
           35                  40                  45
   Ser Tyr Ile Ser Ser Ser Gly Ser Thr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Ala Leu Gly Ser Ser Trp Glu Trp Gly Gln Gly Thr Thr
               100                 105                 110
   Val Thr Val Ser Ser Gly Gly Gly Gly Ser Gly Gly Gly Gly Ser Gly
           115                 120                 125
   Gly Gly Gly Ser Asp Ile Gln Met Thr Gln Ser Pro Ser Ser Leu Ser
       130                 135                 140
   Ala Ser Val Gly Asp Arg Val Thr Ile Thr Cys Gln Ala Ser Gln Asp
   145                 150                 155                 160
   Ile Ser Asn Tyr Leu Asn Trp Tyr Gln Gln Lys Pro Gly Lys Ala Pro
                   165                 170                 175
   Lys Leu Leu Ile Tyr Asp Ala Ser Asn Leu Glu Thr Gly Val Pro Ser
               180                 185                 190
   Arg Phe Ser Gly Ser Gly Ser Gly Thr Asp Phe Thr Phe Thr Ile Ser
           195                 200                 205
   Ser Leu Gln Pro Glu Asp Ile Ala Thr Tyr Tyr Cys Gln Gln Tyr Asp
       210                 215                 220
   Asn Leu Pro Leu Thr Phe Gly Gly Gly Thr Lys Leu Glu Ile Lys
   225                 230                 235
<210> 47
   <211> 246
   <212> PRT
   <213> Artificial Sequence
<220>
   <221> source
   <223> /note="Description of Artificial Sequence: Synthetic polypeptide"
<400> 47
   Gln Val Gln Leu Val Gln Ser Gly Ala Glu Val Lys Glu Pro Gly Ala
   1               5                   10                  15
   Ser Val Lys Val Ser Cys Lys Ala Pro Ala Asn Thr Phe Ser Asp His
               20                  25                  30
   Val Met His Trp Val Arg Gln Ala Pro Gly Gln Arg Phe Glu Trp Met
           35                  40                  45
   Gly Tyr Ile His Ala Ala Asn Gly Gly Thr His Tyr Ser Gln Lys Phe
       50                  55                  60
   Gln Asp Arg Val Thr Ile Thr Arg Asp Thr Ser Ala Asn Thr Val Tyr
   65                  70                  75                  80
   Met Asp Leu Ser Ser Leu Arg Ser Glu Asp Thr Ala Val Tyr Tyr Cys
                   85                  90                  95
   Ala Arg Gly Gly Tyr Asn Ser Asp Ala Phe Asp Ile Trp Gly Gln Gly
               100                 105                 110
   Thr Met Val Thr Val Ser Ser Gly Gly Gly Gly Ser Gly Gly Gly Gly
           115                 120                 125
   Ser Gly Gly Gly Gly Ser Gly Gly Gly Gly Ser Asp Ile Val Met Thr
       130                 135                 140
   Gln Ser Pro Ser Ser Val Ser Ala Ser Val Gly Asp Arg Val Thr Ile
   145                 150                 155                 160
   Thr Cys Arg Ala Ser Gln Asp Ile Ser Ser Trp Leu Ala Trp Tyr Gln
                   165                 170                 175
   Gln Lys Pro Gly Lys Ala Pro Lys Leu Leu Ile Tyr Ala Ala Ser Ser
               180                 185                 190
   Leu Gln Ser Gly Val Pro Ser Arg Phe Asn Gly Ser Gly Ser Gly Thr
           195                 200                 205
   Asp Phe Thr Leu Thr Ile Ser Ser Leu Gln Pro Glu Asp Phe Ala Thr
       210                 215                 220
   Tyr Tyr Cys Gln Gln Ser Tyr Ser Thr Pro Leu Thr Phe Gly Gly Gly
   225                 230                 235                 240
   Thr Lys Val Glu Ile Lys
                   245
<210> 48
   <211> 248
   <212> PRT
   <213> Artificial Sequence
<220>
   <221> source
   <223> /note="Description of Artificial Sequence: Synthetic polypeptide"
<400> 48
   Gln Val Gln Leu Val Gln Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ser Met Asn Trp Val Arg Gln Ala Pro Gly Lys Gly Leu Glu Trp Val
           35                  40                  45
   Ser Tyr Ile Ser Ser Ser Ser Ser Thr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Leu Ser Val Arg Ala Ile Asp Ala Phe Asp Ile Trp Gly
               100                 105                 110
   Gln Gly Thr Met Val Thr Val Ser Ser Gly Gly Gly Gly Ser Gly Gly
           115                 120                 125
   Gly Gly Ser Gly Gly Gly Gly Ser Gly Gly Gly Gly Ser Asp Ile Val
       130                 135                 140
   Leu Thr Gln Ser Pro Ser Ser Leu Ser Ala Ser Val Gly Asp Arg Val
   145 150 155 160
   Thr Ile Thr Cys Gln Ala Ser Gln Asp Ile Ser Asn Tyr Leu Asn Trp
   165 170 175
   Tyr Gln Gln Lys Pro Gly Lys Ala Pro Lys Leu Leu Ile Tyr Asp Ala
   180 185 190
   Ser Asn Leu Glu Thr Gly Val Pro Ser Arg Phe Ser Gly Ser Gly Ser
   195 200 205
   Gly Thr Asp Phe Thr Phe Thr Ile Ser Ser Leu Gln Pro Glu Asp Phe
   210 215 220
   Ala Thr Tyr Tyr Cys Gln Gln Ala Tyr Ser Thr Pro Phe Thr Phe Gly
   225 230 235 240
   Pro Gly Thr Lys Val Glu Ile Lys
   245
<210> 49
   <211> 246
   <212> PRT
   <213> Artificial Sequence
<220>
   <221> source
   <223> /note="Description of Artificial Sequence: Synthetic polypeptide"
<400> 49
   Glu Val Gln Leu Val Gln Ser Gly Gly Gly Val Val Arg Ser Gly Arg
   1 5 10 15
   Ser Leu Arg Leu Ser Cys Ala Ala Ser Gly Phe Thr Phe Asn Ser Tyr
   20 25 30
   Gly Leu His Trp Val Arg Gln Ala Pro Gly Lys Gly Leu Glu Trp Val
   35 40 45
   Ala Leu Ile Glu Tyr Asp Gly Ser Asn Lys Tyr Tyr Gly Asp Ser Val
   50 55 60
   Lys Gly Arg Phe Thr Ile Ser Arg Asp Lys Ser Lys Ser Thr Leu Tyr
   65 70 75 80
   Leu Gln Met Asp Asn Leu Arg Ala Glu Asp Thr Ala Val Tyr Tyr Cys
   85 90 95
   Ala Arg Glu Gly Asn Glu Asp Leu Ala Phe Asp Ile Trp Gly Gln Gly
   100 105 110
   Thr Leu Val Thr Val Ser Ser Gly Gly Gly Gly Ser Gly Gly Gly Gly
   115 120 125
   Ser Gly Gly Gly Gly Ser Gly Gly Gly Gly Ser Glu Ile Val Leu Thr
   130 135 140
   Gln Ser Pro Ser Ser Leu Ser Ala Ser Val Gly Asp Arg Val Thr Ile
   145 150 155 160
   Thr Cys Gln Ala Ser Gln Phe Ile Lys Lys Asn Leu Asn Trp Tyr Gln
   165 170 175
   His Lys Pro Gly Lys Ala Pro Lys Leu Leu Ile Tyr Asp Ala Ser Ser
   180 185 190
   Leu Gln Thr Gly Val Pro Ser Arg Phe Ser Gly Asn Arg Ser Gly Thr
   195 200 205
   Thr Phe Ser Phe Thr Ile Ser Ser Leu Gln Pro Glu Asp Val Ala Thr
   210 215 220
   Tyr Tyr Cys Gln Gln His Asp Asn Leu Pro Leu Thr Phe Gly Gly Gly
   225 230 235 240
   Thr Lys Val Glu Ile Lys
   245
<210> 50
   <211> 255
   <212> PRT
   <213> Artificial Sequence
<220>
   <221> source
   <223> /note="Description of Artificial Sequence: Synthetic polypeptide"
<400> 50
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Asn Val Ser Ser Asn
   20 25 30
   Tyr Met Thr Trp Val Arg Gln Ala Pro Gly Lys Gly Leu Glu Trp Val
   35 40 45
   Ser Val Ile Tyr Ser Gly Gly Ala Thr Tyr Tyr Gly Asp Ser Val Lys
       50                  55                  60
   Gly Arg Phe Thr Val Ser Arg Asp Asn Ser Lys Asn Thr Val Tyr Leu
   65                  70                  75                  80
   Gln Met Asn Arg Leu Thr Ala Glu Asp Thr Ala Val Tyr Tyr Cys Ala
                   85                  90                  95
   Arg Asp Arg Leu Tyr Cys Gly Asn Asn Cys Tyr Leu Tyr Tyr Tyr Tyr
               100                 105                 110
   Gly Met Asp Val Trp Gly Gln Gly Thr Leu Val Thr Val Ser Ser Gly
           115                 120                 125
   Gly Gly Gly Ser Gly Gly Gly Gly Ser Gly Gly Gly Gly Ser Gly Gly
       130                 135                 140
   Gly Gly Ser Asp Ile Gln Val Thr Gln Ser Pro Ser Ser Leu Ser Ala
   145                 150                 155                 160
   Ser Val Gly Asp Arg Val Thr Ile Thr Cys Arg Ala Ser Gln Ser Ile
                   165                 170                 175
   Ser Ser Tyr Leu Asn Trp Tyr Gln Gln Lys Pro Gly Lys Ala Pro Lys
               180                 185                 190
   Leu Leu Ile Tyr Ala Ala Ser Ser Leu Gln Ser Gly Val Pro Ser Arg
           195                 200                 205
   Phe Ser Gly Ser Gly Ser Gly Thr Asp Phe Thr Leu Thr Ile Ser Ser
       210                 215                 220
   Leu Gln Pro Glu Asp Phe Ala Thr Tyr Tyr Cys Gln Gln Ser Tyr Ser
   225                 230                 235                 240
   Thr Pro Pro Leu Thr Phe Gly Gln Gly Thr Lys Val Glu Ile Lys
                   245                 250                 255
<210> 51
   <211> 247
   <212> PRT
   <213> Artificial Sequence
<220>
   <221> source
   <223> /note="Description of Artificial Sequence: Synthetic polypeptide"
<400> 51
   Gln Val Gln Leu Val Gln Ser Gly Ala Glu Val Lys Lys Ser Gly Ala
   1               5                   10                  15
   Ser Val Lys Val Ser Cys Lys Ala Ser Gly Tyr Pro Phe Thr Gly Tyr
               20                  25                  30
   Tyr Ile Gln Trp Val Arg Gln Ala Pro Gly Gln Gly Leu Glu Trp Met
           35                  40                  45
   Gly Trp Ile Asp Pro Asn Ser Gly Asn Thr Gly Tyr Ala Gln Lys Phe
       50                  55                  60
   Gln Gly Arg Val Thr Met Thr Arg Asn Thr Ser Ile Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Ser Asp Ser Tyr Gly Tyr Tyr Tyr Gly Met Asp Val Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Gly Gly Gly Gly Ser Asp Ile Gln Met
       130                 135                 140
   Thr Gln Ser Pro Ser Ser Leu Ser Ala Ser Val Gly Asp Arg Val Thr
   145                 150                 155                 160
   Phe Thr Cys Arg Ala Ser Gln Gly Ile Ser Ser Ala Leu Ala Trp Tyr
                   165                 170                 175
   Gln Gln Lys Pro Gly Lys Pro Pro Lys Leu Leu Ile Tyr Asp Ala Ser
               180                 185                 190
   Ser Leu Glu Ser Gly Val Pro Ser Arg Phe Ser Gly Ser Gly Ser Gly
           195                 200                 205
   Thr Asp Phe Thr Leu Thr Ile Ser Ser Leu Gln Pro Glu Asp Phe Ala
       210                 215                 220
   Thr Tyr Tyr Cys Gln Gln Phe Asn Asn Tyr Pro Leu Thr Phe Gly Gly
   225                 230                 235                 240
   Gly Thr Lys Val Glu Ile Lys
                   245
<210> 52
   <211> 741
   <212> DNA
   <213> Artificial Sequence
<220>
   <221> source
   <223> /note="Description of Artificial Sequence: Synthetic polynucleotide"
<400> 52
   gaagtgcaac tccaacagtc aggcgcagaa gtcaagaagc ccggatcgtc agtgaaagtg     60
   tcctgcaaag cctccggcgg aaccttcagc tcctacgcaa tcagctgggt gcggcaggcg    120
   cccggacagg gactggagtg gatgggcggt atcattccga tctttggcac cgccaattac    180
   gcccagaagt tccagggacg cgtcacaatc accgccgacg aatcgacttc caccgcctac    240
   atggagctgt cgtccttgag gagcgaagat accgccgtgt actactgcgc tcgggatctg    300
   gagatggcca ctatcatggg gggttactgg ggccagggga ccctggtcac tgtgtcctcg    360
   ggaggagggg gatcaggcgg cggcggttcc gggggaggag gaagccagtc cgcgctgact    420
   cagccagctt ccgtgtctgg ttcgccggga cagtccatca ctattagctg taccggcacc    480
   agcagcgacg tgggcggcta caactatgtg tcatggtacc agcagcaccc ggggaaggcg    540
   cctaagctga tgatctacga cgtgtccaac cgccctagcg gagtgtccaa cagattctcc    600
   ggttcgaagt cagggaacac tgcctccctc acgattagcg ggctgcaagc cgaggatgaa    660
   gccgactact actgctcctc ctatacctcc tcctcgaccc tggacgtggt gttcggagga    720
   ggcaccaagc tcaccgtcct t                                              741
<210> 53
   <211> 735
   <212> DNA
   <213> Artificial Sequence
<220>
   <221> source
   <223> /note="Description of Artificial Sequence: Synthetic polynucleotide"
<400> 53
   gaagtgcaat tggtggaaag cggaggagga gtggtgcaac ctggaggaag cctgagactg        60
   tcatgtgccg cctcgggatt cactttcgat gactacgcaa tgcactgggt ccgccaggcc       120
   cccggaaagg gtctggaatg ggtgtccctc atctccggcg atgggggttc cacttactat       180
   gcggattctg tgaagggccg cttcacaatc tcccgggaca attccaagaa cactctgtac       240
   cttcaaatga actccctgag ggtggaggac accgctgtgt actactgcgc gagagtgttt       300
   gactcgtact atatggacgt ctggggaaag ggcaccaccg tgaccgtgtc cagcggtggc       360
   ggtggatcgg ggggcggcgg ctccgggagc ggaggttccg agattgtgct gactcagtcg       420
   ccgttgtcac tgcctgtcac ccccgggcag ccggcctcca tttcatgccg gtccagccag       480
   tccctggtct acaccgatgg gaacacttac ctcaactggt tccagcagcg cccaggacag       540
   tccccgcgga ggctgatcta caaagtgtca aaccgggact ccggcgtccc cgatcggttc       600
   tcgggaagcg gcagcgacac cgacttcacg ctgaagattt cccgcgtgga agccgaggac       660
   gtgggcatct actactgtat gcagggcacc cactggtcgt ttaccttcgg acaaggaact       720
   aggctcgaga tcaag                                                        735
<210> 54
   <211> 741
   <212> DNA
   <213> Artificial Sequence
<220>
   <221> source
   <223> /note="Description of Artificial Sequence: Synthetic polynucleotide"
<400> 54
   caagtgcagc ttcaagaaag cggtccagga ctcgtcaagc catcagaaac tctttccctc       60
   acttgtaccg tgtcgggagg cagcatctcc tcgagctcct actactgggg ttggattaga      120
   cagcccccgg gaaaggggtt ggagtggatc ggttccatct actactccgg gtcgacctac      180
   tacaaccctt ccctgaaatc tcgggtgtcc atctccgtcg acacctccaa gaaccagttc      240
   agcctgaagc tgaaatatgt gaccgcggcc gatactgccg tgtactattg cgccaccccg      300
   ggaacctact acgacttcct ctcggggtac tacccgtttt actggggaca ggggactctc      360
   gtgaccgtgt cctcgggcgg cggaggttca ggcggtggcg gatcgggggg aggaggctca      420
   gacattgtga tgacccagag cccgtccagc ctgagcgcct ccgtgggcga tagggtcacg      480
   attacttgcc gggcgtccca gggaatctca agctacctgg cctggtacca acagaagccc      540
   ggaaaggcac ccaagttgct gatctatgcc gctagcactc tgcagtccgg ggtgccttcc      600
   cgcttctccg gctccggctc gggcaccgac ttcaccctga ccatttcctc actgcaaccc      660
   gaggacttcg ccacttacta ctgccagcag ctgaactcct acccttacac attcggacag      720
   ggaaccaagc tggaaatcaa g                                                741
<210> 55
   <211> 717
   <212> DNA
   <213> Artificial Sequence
<220>
   <221> source
   <223> /note="Description of Artificial Sequence: Synthetic polynucleotide"
<400> 55
   caagtgcaac tcgtggaatc tggtggagga ctcgtgcaac ccggaggatc attgcgactc       60
   tcgtgtgcgg catccggctt taccttttca tcctactgga tgtcctgggt cagacaggcc      120
   cccgggaagg gactggaatg ggtcgcgaac atcaacgagg acggctcggc caagttctac      180
   gtggactccg tgaagggccg cttcacgatc tcacgggata acgccaagaa ttccctgtat      240
   ctgcaaatga acagcctgag ggccgaggac actgcggtgt acttctgcgc acgcgacctg      300
   aggtccggga gatactgggg acagggcacc ctcgtgaccg tgtcgagcgg aggagggggg      360
   tcgggcggcg gcggttccgg tggcggcggt agcgaaattg tgttgaccca gtcccctgga      420
   accctgagcc tgtcacctgg aggacgcgcc accctgtcct gccgggccag ccagagcatc      480
   tcagggtcct tcctggcttg gtaccagcag aagccgggac aggctccgag acttctgatc      540
   tacggcgcct cctcgcgggc gaccggaatc ccggatcggt tctccggctc gggaagcgga      600
   actgacttca ctcttaccat ttcccgcctg gagccggaag atttcgccgt gtactactgc      660
   cagcagtacg ggtcatcccc tccaaccttc ggcctgggaa ctaagctgga aatcaaa         717
<210> 56
   <211> 735
   <212> DNA
   <213> Artificial Sequence
<220>
   <221> source
   <223> /note="Description of Artificial Sequence: Synthetic polynucleotide"
<400> 56
   gaagtgcaac tccaacaatc cggtccagga ctcgtcagac cctccgaaac tctctcgctt 60
   acatgcactg tgtccggcgg ccctgtgcgg tccggctctc attactggaa ctggattcgc 120
   cagcccccgg gacgcggact ggagtggatc ggctacatct attactcggg gtcgactaac 180
   tacaacccga gcctggaaaa tagagtgacc atctcaatcg acacgtccaa caaccacttc 240
   tcgctgaagt tgtcctccgt gactgccgcc gatactgccc tgtacttctg tgctcgcgga 300
   accgccacct tcgactggaa cttccctttt gactcatggg gccaggggac ccttgtgacc 360
   gtgtccagcg gaggaggagg ctccggtggt ggcgggagcg gtagcggcgg aagcgacatc 420
   cagatgaccc agtcaccgtc ctcgctgtcc gcatccattg gggatcgggt cactattact 480
   tgccgggcgt cccagtccat ctcgtcctac ctgaactggt atcagcagaa gccagggaaa 540
   gcccccaagc tgctgatcta cgcggccagc agcctgcagt caggagtgcc ttcaaggttt 600
   agcggcagcg gatcgggaac cgacttcacc ctgaccattt cctccctcca acccgaggat 660
   ttcgccacct actactgcca gcagtcctac tccaccccgt ggaccttcgg acagggaacc 720
   aagctggaga tcaag 735
<210> 57
   <211> 747
   <212> DNA
   <213> Artificial Sequence
<220>
   <221> source
   <223> /note="Description of Artificial Sequence: Synthetic polynucleotide"
<400> 57
   caagtgcaac ttcaagaatc aggcgcagga cttctcaagc catccgaaac actctccctc 60
   acttgcgcgg tgtacggggg aagcttctcg ggatactact ggtcctggat taggcagcct 120
   cccggcaaag gcctggaatg ggtcggggag atcaaccact ccggttcaac caactacaac 180
   ccgtcgctga agtcccgcgt gaccatttcc gtggacacct ctaagaatca gttcagcctg 240
   aagctctcgt ccgtgaccgc ggcggacacc gccgtctact actgcgctcg gggatcagga 300
   ctggtggtgt acgccatccg cgtgggctcg ggctggttcg attactgggg ccagggaacc 360
   ctggtcactg tgtcgtccgg cggaggaggt tcggggggcg gagacagcgg tggagggggt 420
   agcgacatcc agatgaccca gtccccgtcc tcgctgtccg cctccgtggg agatagagtg 480
   accatcacct gtcgggcatc ccagagcatt tccagctacc tgaactggta tcagcagaag 540
   cccggaaagg cccctaagct gttgatgtac gccgccagca gcttgcagtc gggcgtgccg 600
   agccggtttt ccggttccgg ctccgggact gacttcaccc tgactatctc atccctgcaa 660
   cccgaggact tcgccactta ttactgccag cagtcctact caacccctcc ctggacgttc 720
   ggacagggca ccaaggtcga tatcaag 747
<210> 58
   <211> 765
   <212> DNA
   <213> Artificial Sequence
<220>
   <221> source
   <223> /note="Description of Artificial Sequence: Synthetic polynucleotide"
<400> 58
   gaagtgcaat tggtggaatc tggaggagga cttgtgaaac ctggtggaag cctgagactt 60
   tcctgtgcgg cctcgggatt cactttctcc tcctactcca tgaactgggt cagacaggcc 120
   cctgggaagg gactggaatg ggtgtcatcc atctcctcct catcgtcgta catctactac 180
   gccgatagcg tgaaggggcg gttcaccatt tcccgggaca acgctaagaa cagcctctat 240
   ctgcaaatga attccctccg cgccgaggac actgccgtgt actactgcgc gagggacccc 300
   tcatcaagcg gcagctacta catggaggac tcgtattact acggaatgga cgtctggggc 360
   cagggaacca ctgtgacggt gtcctccggt ggagggggct ccgggggcgg gggatctggc 420
   ggaggaggct ccaacttcat gctgacccag ccgcactccg tgtccgaaag ccccggaaag 480
   accgtgacaa tttcctgcac cgggtcctcc ggctcgatcg catcaaacta cgtgcagtgg 540
   taccagcagc gcccgggcag cgcccccacc actgtcatct acgaggataa ccagcggccg 600
   tcgggtgtcc cagaccggtt ttccggttcg atcgatagca gcagcaacag cgcctccctg 660
   accatttccg gcctcaagac cgaggatgag gctgactact actgccagtc gtatgactcc 720
   tcgaaccaag tggtgttcgg tggcggcacc aagctgactg tgctg 765
<210> 59
   <211> 717
   <212> DNA
   <213> Artificial Sequence
<220>
   <221> source
   <223> /note="Description of Artificial Sequence: Synthetic polynucleotide"
<400> 59
   caagtgaacc tgagagaaag cggcggagga cttgtgcaac ctggaggaag cctgagactg 60
   tcatgtgccg cgtccggctt caccttctcg tcctacgaga tgaactgggt ccgccaggca 120
   ccgggcaaag gactggaatg ggtgtcctac atttcctcgt ccgggtccac catctattac 180
   gccgactccg tgaagggacg gttcaccatc tcccgggaca acgccaagaa ctccctctac 240
   ctccaaatga actcactgag ggcagaggac actgcggtct actactgcgc ccgcgaagct 300
   ttgggtagct cctgggagtg gggccaggga accactgtga ccgtgtcctc gggtggaggg 360
   ggctccggtg gcgggggttc agggggtggc ggaagcgata tccagatgac tcagtcacca 420
   agctccctga gcgcctcagt gggagatcgg gtcacaatca cgtgccaggc gtcccaggac 480
   atttctaact acctcaattg gtaccagcag aagccgggga aggcccccaa gcttctgatc 540
   tacgatgcct ccaacctgga aaccggcgtg ccctcccgct tctcgggatc gggcagcggc 600
   actgacttca cctttaccat ctcgtccctg caacctgagg acatcgccac ctattactgc 660
   cagcagtacg ataacctccc gctgactttc ggaggcggaa ctaagctgga gattaag 717
<210> 60
   <211> 738
   <212> DNA
   <213> Artificial Sequence
<220>
   <221> source
   <223> /note="Description of Artificial Sequence: Synthetic polynucleotide"
<400> 60
   caagtgcaac tcgtccagtc cggtgcagaa gtcaaggaac ccggagcctc cgtgaaagtg 60
   tcctgcaaag ctcctgccaa cactttctcg gaccacgtga tgcactgggt gcgccaggcg 120
   ccgggccagc gcttcgaatg gatgggatac attcatgccg ccaatggcgg tacccactac 180
   tcccaaaagt tccaggatag agtcaccatc acccgggaca ccagcgccaa caccgtgtat 240
   atggatctgt ccagcctgag gtccgaggat accgccgtgt actactgcgc ccggggcgga 300
   tacaactcag acgcgttcga catttgggga cagggtacta tggtcaccgt gtcatccggg 360
   ggcggtggca gcgggggcgg aggctctggc ggaggcggat cagggggagg agggtccgac 420
   atcgtgatga cccagtcccc gtcatcggtg tccgcgtccg tgggagacag agtgaccatc 480
   acgtgtcgcg ccagccagga catctcctcg tggttggcat ggtaccagca gaagcctgga 540
   aaggccccga agctgctcat ctacgccgcc tcctcccttc aatcgggagt gccctcgcgg 600
   ttcaacggaa gcggaagcgg gacagatttt accctgacta ttagctcgct gcagcccgag 660
   gacttcgcta cttactactg ccaacagagc tactccaccc cactgacttt cggcgggggt 720
   accaaggtcg agatcaag 738
<210> 61
   <211> 744
   <212> DNA
   <213> Artificial Sequence
<220>
   <221> source
   <223> /note="Description of Artificial Sequence: Synthetic polynucleotide"
<400> 61
   caagtgcaac ttgttcaatc cggtggaggt cttgtgcagc ccggaggatc actcagactg      60
   tcgtgcgccg cctctgggtt cactttctcc tcatactcga tgaactgggt gcgccaggcg     120
   ccgggaaagg gcctggaatg ggtgtcatac atctcctcct catcctccac catctactac     180
   gccgattccg tgaagggccg cttcactatt tcccgggaca acgcgaaaaa ctcgctctat     240
   ctgcaaatga actccctgcg cgccgaggac accgccgtgt actactgcgc ccgggacctg     300
   agcgtgcggg ctattgatgc gttcgacatc tggggacagg gcaccatggt cacagtgtcc     360
   agcggaggcg gcggcagcgg tggaggagga tcagggggag gaggttcggg gggcggtggc   420
   tccgatatcg tgctgaccca gagcccgtcg agcctctccg cctccgtcgg cgacagagtg     480
   accatcacgt gtcaggcatc ccaggacatt agcaactacc tgaattggta ccagcagaag     540
   cctggaaagg cacccaagtt gctgatctac gacgcctcca acctggaaac cggagtgcca     600
   tccaggttct cgggcagcgg ctcgggaacc gacttcactt ttactatctc ctccctgcaa 660
   cccgaggatt tcgcgaccta ctactgccag caggcctaca gcaccccttt caccttcggg 720
   ccgggaacta aggtcgaaat caag                                            744
<210> 62
   <211> 738
   <212> DNA
   <213> Artificial Sequence
<220>
   <221> source
   <223> /note="Description of Artificial Sequence: Synthetic polynucleotide"
<400> 62
   gaagtgcaat tggtgcaatc aggaggagga gtggtcagat ctggaagaag cctgagactg      60
   tcatgcgcgg cttcgggctt taccttcaac tcctacggcc tccactgggt gcgccaggcc     120
   cccggaaaag gcctcgaatg ggtcgcactg attgagtacg acgggtccaa caagtactac     180
   ggagatagcg tgaagggccg cttcaccatc tcacgggaca agtccaagtc caccctgtat     240
   ctgcaaatgg acaacctgag ggccgaggat actgccgtgt actactgcgc ccgcgaagga     300
   aacgaagatc tggccttcga tatttggggc cagggtactc ttgtgaccgt gtcgagcgga     360
   ggcggaggct ccggtggagg aggatcgggg ggtggtggtt ccggcggcgg ggggagcgaa     420
   atcgtgctga cccagtcgcc ttcctccctc tccgcttccg tgggggaccg ggtcactatt     480
   acgtgtcagg cgtcccaatt catcaagaag aatctgaact ggtaccagca caagccggga     540
   aaggccccca aactgctcat ctacgacgcc agctcgctgc agactggcgt gccttcccgg     600
   ttttccggga accggtcggg aaccaccttc tcattcacca tcagcagcct ccagccggag     660
   gacgtggcga cctactactg ccagcagcat gacaaccttc cactgacttt cggcgggggc     720
   accaaggtcg agattaag                                                   738
<210> 63
   <211> 765
   <212> DNA
   <213> Artificial Sequence
<220>
   <221> source
   <223> /note="Description of Artificial Sequence: Synthetic polynucleotide"
<400> 63
   caagtgcaac tcgtggaatc aggcggagga ctcgtgcaac ccggaggttc ccttagactg      60
   tcatgtgccg cttccgggtt caatgtgtcc agcaactaca tgacctgggt cagacaggcg     120
   ccgggaaagg gacttgaatg ggtgtccgtg atctactccg gtggagcaac atactacgga     180
   gactccgtga aaggccgctt taccgtgtcc cgcgataact cgaagaacac cgtgtacttg     240
   cagatgaaca ggctgactgc cgaggacacc gccgtgtatt attgcgcccg ggacaggctg     300
   tactgtggaa acaactgcta cctgtactac tactacggga tggacgtgtg gggacagggc     360
   actctcgtca ctgtgtcatc cggggggggc ggtagcggtg gcggagggtc cggcggagga     420
   ggctcagggg gaggcggaag cgatatccag gtcacccagt ctccctcctc gctgtccgcc     480
   tccgtgggcg accgcgtcac cattacttgc cgggcgtcgc agtcgatcag ctcctacctg 540
   aactggtacc agcagaagcc tggaaaggcc ccgaagctgc tgatctacgc ggcctcgtcc 600
   ctgcaaagcg gcgtcccgtc gcggttcagc ggttccggtt cgggaaccga cttcaccctg 660
   actatttcct ccctgcaacc cgaggatttc gccacttact actgccagca gtcctactcc 720
   accccacctc tgaccttcgg ccaaggaacc aaggtcgaaa tcaag 765
<210> 64
   <211> 741
   <212> DNA
   <213> Artificial Sequence
<220>
   <221> source
   <223> /note="Description of Artificial Sequence: Synthetic polynucleotide"
<400> 64
   caagtgcaac tcgtccagtc cggtgcagaa gtgaaaaaga gcggagcctc agtgaaagtg 60
   tcctgcaagg cctccggtta ccccttcact ggatactaca ttcagtgggt ccgccaagcc 120
   ccgggacagg gtctggagtg gatggggtgg attgacccta actcgggaaa tacgggatac 180
   gcgcagaagt tccagggccg cgtgaccatg accaggaaca cctcgatcag caccgcctac 240
   atggaactgt cctccctgcg gtcggaggat actgccgtgt actactgcgc ctccgattcc 300
   tatgggtact actacggaat ggacgtctgg ggacagggca ccctcgtgac cgtgtcctcg 360
   ggaggcggag ggagcggcgg gggtggatcg ggaggaggcg gctccggcgg cggcggtagc 420
   gacatccaga tgacccagtc accatcaagc cttagcgcct ccgtgggcga cagagtgaca 480
   ttcacttgtc gggcgtccca gggaatctcc tccgctctgg cttggtatca gcagaagcct 540
   gggaagcctc cgaagctgtt gatctacgac gcgagcagcc tggaatcagg ggtgccctcc 600
   cggttttccg ggtccggttc tggcaccgat ttcaccctga ccatttcgtc cctccaaccc 660
   gaggacttcg ccacttacta ctgccagcag ttcaacaact acccgctgac cttcggagga 720
   ggcactaagg tcgagatcaa g 741
<210> 65
   <211> 120
   <212> PRT
   <213> Artificial Sequence
<220>
   <221> source
   <223> /note="Description of Artificial Sequence: Synthetic polypeptide"
<400> 65
   Glu Val Gln Leu Gln Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Gly Ile Ile Pro Ile Phe Gly Thr Ala Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asp Leu Glu Met Ala Thr Ile Met Gly Gly Tyr Trp Gly Gln
               100                 105                 110
   Gly Thr Leu Val Thr Val Ser Ser
           115                 120
<210> 66
   <211> 118
   <212> PRT
   <213> Artificial Sequence
<220>
   <221> source
   <223> /note="Description of Artificial Sequence: Synthetic polypeptide"
<400> 66
   Glu Val Gln Leu Val Glu Ser Gly Gly Gly Val Val Gln Pro Gly Gly
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Leu Ile Ser Gly Asp Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Val Glu Asp Thr Ala Val Tyr Tyr Cys
                   85                  90                  95
   Ala Arg Val Phe Asp Ser Tyr Tyr Met Asp Val Trp Gly Lys Gly Thr
               100                 105                 110
   Thr Val Thr Val Ser Ser
           115
<210> 67
   <211> 125
   <212> PRT
   <213> Artificial Sequence
<220>
   <221> source
   <223> /note="Description of Artificial Sequence: Synthetic polypeptide"
<400> 67
   Gln Val Gln Leu Gln Glu Ser Gly Pro Gly Leu Val Lys Pro Ser Glu
   1 5 10 15
   Thr Leu Ser Leu Thr Cys Thr Val Ser Gly Gly Ser Ile Ser Ser Ser
   20 25 30
   Ser Tyr Tyr Trp Gly Trp Ile Arg Gln Pro Pro Gly Lys Gly Leu Glu
   35 40 45
   Trp Ile Gly Ser Ile Tyr Tyr Ser Gly Ser Thr Tyr Tyr Asn Pro Ser
   50 55 60
   Leu Lys Ser Arg Val Ser Ile Ser Val Asp Thr Ser Lys Asn Gln Phe
   65 70 75 80
   Ser Leu Lys Leu Lys Tyr Val Thr Ala Ala Asp Thr Ala Val Tyr Tyr
   85 90 95
   Cys Ala Thr Pro Gly Thr Tyr Tyr Asp Phe Leu Ser Gly Tyr Tyr Pro
   100 105 110
   Phe Tyr Trp Gly Gln Gly Thr Leu Val Thr Val Ser Ser
   115 120 125
<210> 68
   <211> 116
   <212> PRT
   <213> Artificial Sequence
<220>
   <221> source
   <223> /note="Description of Artificial Sequence: Synthetic polypeptide"
<400> 68
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Trp Met Ser Trp Val Arg Gln Ala Pro Gly Lys Gly Leu Glu Trp Val
           35                  40                  45
   Ala Asn Ile Asn Glu Asp Gly Ser Ala Lys Phe Tyr Val Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Phe Cys
                   85                  90                  95
   Ala Arg Asp Leu Arg Ser Gly Arg Tyr Trp Gly Gln Gly Thr Leu Val
               100                 105                 110
   Thr Val Ser Ser
           115
<210> 69
   <211> 123
   <212> PRT
   <213> Artificial Sequence
<220>
   <221> source
   <223> /note="Description of Artificial Sequence: Synthetic polypeptide"
<400> 69
   Glu Val Gln Leu Gln Gln Ser Gly Pro Gly Leu Val Arg Pro Ser Glu
   1               5                   10                  15
   Thr Leu Ser Leu Thr Cys Thr Val Ser Gly Gly Pro Val Arg Ser Gly
               20                  25                  30
   Ser His Tyr Trp Asn Trp Ile Arg Gln Pro Pro Gly Arg Gly Leu Glu
           35                  40                  45
   Trp Ile Gly Tyr Ile Tyr Tyr Ser Gly Ser Thr Asn Tyr Asn Pro Ser
       50                  55                  60
   Leu Glu Asn Arg Val Thr Ile Ser Ile Asp Thr Ser Asn Asn His Phe
   65                  70                  75                  80
   Ser Leu Lys Leu Ser Ser Val Thr Ala Ala Asp Thr Ala Leu Tyr Phe
                   85                  90                  95
   Cys Ala Arg Gly Thr Ala Thr Phe Asp Trp Asn Phe Pro Phe Asp Ser
               100                 105                 110
   Trp Gly Gln Gly Thr Leu Val Thr Val Ser Ser
           115                 120
<210> 70
   <211> 126
   <212> PRT
   <213> Artificial Sequence
<220>
   <221> source
   <223> /note="Description of Artificial Sequence: Synthetic polypeptide"
<400> 70
   Gln Val Gln Leu Gln Glu Ser Gly Ala Gly Leu Leu Lys Pro Ser Glu
   1               5                   10                  15
   Thr Leu Ser Leu Thr Cys Ala Val Tyr Gly Gly Ser Phe Ser Gly Tyr
               20                  25                  30
   Tyr Trp Ser Trp Ile Arg Gln Pro Pro Gly Lys Gly Leu Glu Trp Val
           35                  40                  45
   Gly Glu Ile Asn His Ser Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Gly Ser Gly Leu Val Val Tyr Ala Ile Arg Val Gly Ser Gly Trp
               100                 105                 110
   Phe Asp Tyr Trp Gly Gln Gly Thr Leu Val Thr Val Ser Ser
           115                 120                 125
<210> 71
   <211> 129
   <212> PRT
   <213> Artificial Sequence
<220>
   <221> source
   <223> /note="Description of Artificial Sequence: Synthetic polypeptide"
<400> 71
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Ser Tyr
               20                  25                  30
   Ser Met Asn Trp Val Arg Gln Ala Pro Gly Lys Gly Leu Glu Trp Val
           35                  40                  45
   Ser Ser Ile Ser Ser Ser Ser Ser Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Pro Ser Ser Ser Gly Ser Tyr Tyr Met Glu Asp Ser Tyr
               100                 105                 110
   Tyr Tyr Gly Met Asp Val Trp Gly Gln Gly Thr Thr Val Thr Val Ser
           115                 120                 125
   Ser
<210> 72
   <211> 117
   <212> PRT
   <213> Artificial Sequence
<220>
   <221> source
   <223> /note="Description of Artificial Sequence: Synthetic polypeptide"
<400> 72
   Gln Val Asn Leu Arg Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Glu Met Asn Trp Val Arg Gln Ala Pro Gly Lys Gly Leu Glu Trp Val
           35                  40                  45
   Ser Tyr Ile Ser Ser Ser Gly Ser Thr Ile Tyr Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Glu Ala Leu Gly Ser Ser Trp Glu Trp Gly Gln Gly Thr Thr
   100 105 110
   Val Thr Val Ser Ser
   115
<210> 73
   <211> 119
   <212> PRT
   <213> Artificial Sequence
<220>
   <221> source
   <223> /note="Description of Artificial Sequence: Synthetic polypeptide"
<400> 73
   Gln Val Gln Leu Val Gln Ser Gly Ala Glu Val Lys Glu Pro Gly Ala
   1 5 10 15
   Ser Val Lys Val Ser Cys Lys Ala Pro Ala Asn Thr Phe Ser Asp His
   20 25 30
   Val Met His Trp Val Arg Gln Ala Pro Gly Gln Arg Phe Glu Trp Met
   35 40 45
   Gly Tyr Ile His Ala Ala Asn Gly Gly Thr His Tyr Ser Gln Lys Phe
   50 55 60
   Gln Asp Arg Val Thr Ile Thr Arg Asp Thr Ser Ala Asn Thr Val Tyr
   65 70 75 80
   Met Asp Leu Ser Ser Leu Arg Ser Glu Asp Thr Ala Val Tyr Tyr Cys
   85 90 95
   Ala Arg Gly Gly Tyr Asn Ser Asp Ala Phe Asp Ile Trp Gly Gln Gly
   100 105 110
   Thr Met Val Thr Val Ser Ser
   115
<210> 74
   <211> 121
   <212> PRT
   <213> Artificial Sequence
<220>
   <221> source
   <223> /note="Description of Artificial Sequence: Synthetic polypeptide"
<400> 74
   Gln Val Gln Leu Val Gln Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ser Met Asn Trp Val Arg Gln Ala Pro Gly Lys Gly Leu Glu Trp Val
           35                  40                  45
   Ser Tyr Ile Ser Ser Ser Ser Ser Thr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Leu Ser Val Arg Ala Ile Asp Ala Phe Asp Ile Trp Gly
               100                 105                 110
   Gln Gly Thr Met Val Thr Val Ser Ser
           115                 120
<210> 75
   <211> 119
   <212> PRT
   <213> Artificial Sequence
<220>
   <221> source
   <223> /note="Description of Artificial Sequence: Synthetic polypeptide"
<400> 75
   Glu Val Gln Leu Val Gln Ser Gly Gly Gly Val Val Arg Ser Gly Arg
   1               5                   10                  15
   Ser Leu Arg Leu Ser Cys Ala Ala Ser Gly Phe Thr Phe Asn Ser Tyr
               20                  25                  30
   Gly Leu His Trp Val Arg Gln Ala Pro Gly Lys Gly Leu Glu Trp Val
           35                  40                  45
   Ala Leu Ile Glu Tyr Asp Gly Ser Asn Lys Tyr Tyr Gly Asp Ser Val
       50                  55                  60
   Lys Gly Arg Phe Thr Ile Ser Arg Asp Lys Ser Lys Ser Thr Leu Tyr
   65                  70                  75                  80
   Leu Gln Met Asp Asn Leu Arg Ala Glu Asp Thr Ala Val Tyr Tyr Cys
                   85                  90                  95
   Ala Arg Glu Gly Asn Glu Asp Leu Ala Phe Asp Ile Trp Gly Gln Gly
               100                 105                 110
   Thr Leu Val Thr Val Ser Ser
           115
<210> 76
   <211> 127
   <212> PRT
   <213> Artificial Sequence
<220>
   <221> source
   <223> /note="Description of Artificial Sequence: Synthetic polypeptide"
<400> 76
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Asn Val Ser Ser Asn
   20 25 30
   Tyr Met Thr Trp Val Arg Gln Ala Pro Gly Lys Gly Leu Glu Trp Val
   35 40 45
   Ser Val Ile Tyr Ser Gly Gly Ala Thr Tyr Tyr Gly Asp Ser Val Lys
   50 55 60
   Gly Arg Phe Thr Val Ser Arg Asp Asn Ser Lys Asn Thr Val Tyr Leu
   65 70 75 80
   Gln Met Asn Arg Leu Thr Ala Glu Asp Thr Ala Val Tyr Tyr Cys Ala
   85 90 95
   Arg Asp Arg Leu Tyr Cys Gly Asn Asn Cys Tyr Leu Tyr Tyr Tyr Tyr
   100 105 110
   Gly Met Asp Val Trp Gly Gln Gly Thr Leu Val Thr Val Ser Ser
   115 120 125
<210> 77
   <211> 120
   <212> PRT
   <213> Artificial Sequence
<220>
   <221> source
   <223> /note="Description of Artificial Sequence: Synthetic polypeptide"
<400> 77
   Gln Val Gln Leu Val Gln Ser Gly Ala Glu Val Lys Lys Ser Gly Ala
   1               5                   10                  15
   Ser Val Lys Val Ser Cys Lys Ala Ser Gly Tyr Pro Phe Thr Gly Tyr
               20                  25                  30
   Tyr Ile Gln Trp Val Arg Gln Ala Pro Gly Gln Gly Leu Glu Trp Met
           35                  40                  45
   Gly Trp Ile Asp Pro Asn Ser Gly Asn Thr Gly Tyr Ala Gln Lys Phe
       50                  55                  60
   Gln Gly Arg Val Thr Met Thr Arg Asn Thr Ser Ile Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Ser Asp Ser Tyr Gly Tyr Tyr Tyr Gly Met Asp Val Trp Gly Gln
               100                 105                 110
   Gly Thr Leu Val Thr Val Ser Ser
           115                 120
<210> 78
   <211> 112
   <212> PRT
   <213> Artificial Sequence
<220>
   <221> source
   <223> /note="Description of Artificial Sequence: Synthetic polypeptide"
<400> 78
   Gln Ser Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Gly Tyr
               20                  25                  30
   Asn Tyr Val Ser Trp Tyr Gln Gln His Pro Gly Lys Ala Pro Lys Leu
   35 40 45
   Met Ile Tyr Asp Val Ser Asn Arg Pro Ser Gly Val Ser Asn Arg Phe
   50 55 60
   Ser Gly Ser Lys Ser Gly Asn Thr Ala Ser Leu Thr Ile Ser Gly Leu
   65 70 75 80
   Gln Ala Glu Asp Glu Ala Asp Tyr Tyr Cys Ser Ser Tyr Thr Ser Ser
   85 90 95
   Ser Thr Leu Asp Val Val Phe Gly Gly Gly Thr Lys Leu Thr Val Leu
   100 105 110
<210> 79
   <211> 112
   <212> PRT
   <213> Artificial Sequence
<220>
   <221> source
   <223> /note="Description of Artificial Sequence: Synthetic polypeptide"
<400> 79
   Glu Ile Val Leu Thr Gln Ser Pro Leu Ser Leu Pro Val Thr Pro Gly
   1 5 10 15
   Gln Pro Ala Ser Ile Ser Cys Arg Ser Ser Gln Ser Leu Val Tyr Thr
   20 25 30
   Asp Gly Asn Thr Tyr Leu Asn Trp Phe Gln Gln Arg Pro Gly Gln Ser
   35 40 45
   Pro Arg Arg Leu Ile Tyr Lys Val Ser Asn Arg Asp Ser Gly Val Pro
   50 55 60
   Asp Arg Phe Ser Gly Ser Gly Ser Asp Thr Asp Phe Thr Leu Lys Ile
   65 70 75 80
   Ser Arg Val Glu Ala Glu Asp Val Gly Ile Tyr Tyr Cys Met Gln Gly
   85 90 95
   Thr His Trp Ser Phe Thr Phe Gly Gln Gly Thr Arg Leu Glu Ile Lys
   100 105 110
<210> 80
   <211> 107
   <212> PRT
   <213> Artificial Sequence
<220>
   <221> source
   <223> /note="Description of Artificial Sequence: Synthetic polypeptide"
<400> 80
   Asp Ile Val Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Ser Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Leu Asn Ser Tyr Pro Tyr
                   85                  90                  95
   Thr Phe Gly Gln Gly Thr Lys Leu Glu Ile Lys
               100                 105
<210> 81
   <211> 108
   <212> PRT
   <213> Artificial Sequence
<220>
   <221> source
   <223> /note="Description of Artificial Sequence: Synthetic polypeptide"
<400> 81
   Glu Ile Val Leu Thr Gln Ser Pro Gly Thr Leu Ser Leu Ser Pro Gly
   1               5                   10                  15
   Gly Arg Ala Thr Leu Ser Cys Arg Ala Ser Gln Ser Ile Ser Gly Ser
               20                  25                  30
   Phe Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Pro Thr Phe Gly Leu Gly Thr Lys Leu Glu Ile Lys
               100                 105
<210> 82
   <211> 107
   <212> PRT
   <213> Artificial Sequence
<220>
   <221> source
   <223> /note="Description of Artificial Sequence: Synthetic polypeptide"
<400> 82
   Asp Ile Gln Met Thr Gln Ser Pro Ser Ser Leu Ser Ala Ser Ile Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Trp
                   85                  90                  95
   Thr Phe Gly Gln Gly Thr Lys Leu Glu Ile Lys
               100                 105
<210> 83
   <211> 108
   <212> PRT
   <213> Artificial Sequence
<220>
   <221> source
   <223> /note="Description of Artificial Sequence: Synthetic polypeptide"
<400> 83
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Met
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Pro
                   85                  90                  95
   Trp Thr Phe Gly Gln Gly Thr Lys Val Asp Ile Lys
               100                 105
<210> 84
   <211> 111
   <212> PRT
   <213> Artificial Sequence
<220>
   <221> source
   <223> /note="Description of Artificial Sequence: Synthetic polypeptide"
<400> 84
   Asn Phe Met Leu Thr Gln Pro His Ser Val Ser Glu Ser Pro Gly Lys
   1               5                   10                  15
   Thr Val Thr Ile Ser Cys Thr Gly Ser Ser Gly Ser Ile Ala Ser Asn
               20                  25                  30
   Tyr Val Gln Trp Tyr Gln Gln Arg Pro Gly Ser Ala Pro Thr Thr Val
           35                  40                  45
   Ile Tyr Glu Asp Asn Gln Arg Pro Ser Gly Val Pro Asp Arg Phe Ser
       50                  55                  60
   Gly Ser Ile Asp Ser Ser Ser Asn Ser Ala Ser Leu Thr Ile Ser Gly
   65                  70                  75                  80
   Leu Lys Thr Glu Asp Glu Ala Asp Tyr Tyr Cys Gln Ser Tyr Asp Ser
                   85                  90                  95
   Ser Asn Gln Val Val Phe Gly Gly Gly Thr Lys Leu Thr Val Leu
   100 105 110
<210> 85
   <211> 107
   <212> PRT
   <213> Artificial Sequence
<220>
   <221> source
   <223> /note="Description of Artificial Sequence: Synthetic polypeptide"
<400> 85
   Asp Ile Gln Met Thr Gln Ser Pro Ser Ser Leu Ser Ala Ser Val Gly
   1 5 10 15
   Asp Arg Val Thr Ile Thr Cys Gln Ala Ser Gln Asp Ile Ser Asn Tyr
   20 25 30
   Leu Asn Trp Tyr Gln Gln Lys Pro Gly Lys Ala Pro Lys Leu Leu Ile
   35 40 45
   Tyr Asp Ala Ser Asn Leu Glu Thr Gly Val Pro Ser Arg Phe Ser Gly
   50 55 60
   Ser Gly Ser Gly Thr Asp Phe Thr Phe Thr Ile Ser Ser Leu Gln Pro
   65 70 75 80
   Glu Asp Ile Ala Thr Tyr Tyr Cys Gln Gln Tyr Asp Asn Leu Pro Leu
   85 90 95
   Thr Phe Gly Gly Gly Thr Lys Leu Glu Ile Lys
   100 105
<210> 86
   <211> 107
   <212> PRT
   <213> Artificial Sequence
<220>
   <221> source
   <223> /note="Description of Artificial Sequence: Synthetic polypeptide"
<400> 86
   Asp Ile Val Met Thr Gln Ser Pro Ser Ser Val Ser Ala Ser Val Gly
   1               5                   10                  15
   Asp Arg Val Thr Ile Thr Cys Arg Ala Ser Gln Asp Ile Ser Ser Trp
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Asn Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Leu
                   85                  90                  95
   Thr Phe Gly Gly Gly Thr Lys Val Glu Ile Lys
               100                 105
<210> 87
   <211> 107
   <212> PRT
   <213> Artificial Sequence
<220>
   <221> source
   <223> /note="Description of Artificial Sequence: Synthetic polypeptide"
<400> 87
   Asp Ile Val Leu Thr Gln Ser Pro Ser Ser Leu Ser Ala Ser Val Gly
   1               5                   10                  15
   Asp Arg Val Thr Ile Thr Cys Gln Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Asp Ala Ser Asn Leu Glu Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Phe Ala Thr Tyr Tyr Cys Gln Gln Ala Tyr Ser Thr Pro Phe
                   85                  90                  95
   Thr Phe Gly Pro Gly Thr Lys Val Glu Ile Lys
               100                 105
<210> 88
   <211> 107
   <212> **PRT**
   <213> **Artificial** Sequence
<220>
   <221> source
   <223> /note="Description of Artificial Sequence: Synthetic polypeptide"
<400> 88
   Glu Ile Val Leu Thr Gln Ser Pro Ser Ser Leu Ser Ala Ser Val Gly
   1               5                   10                  15
   Asp Arg Val Thr Ile Thr Cys Gln Ala Ser Gln Phe Ile Lys Lys Asn
               20                  25                  30
   Leu Asn Trp Tyr Gln His Lys Pro Gly Lys Ala Pro Lys Leu Leu Ile
           35                  40                  45
   Tyr Asp Ala Ser Ser Leu Gln Thr Gly Val Pro Ser Arg Phe Ser Gly
       50                  55                  60
   Asn Arg Ser Gly Thr Thr Phe Ser Phe Thr Ile Ser Ser Leu Gln Pro
   65                  70                  75                  80
   Glu Asp Val Ala Thr Tyr Tyr Cys Gln Gln His Asp Asn Leu Pro Leu
                   85                  90                  95
   Thr Phe Gly Gly Gly Thr Lys Val Glu Ile Lys
               100                 105
<210> 89
   <211> 108
   <212> PRT
   <213> Artificial Sequence
<220>
   <221> source
   <223> /note="Description of Artificial Sequence: Synthetic polypeptide"
<400> 89
   Asp Ile Gln Val Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Pro
   85 90 95
   Leu Thr Phe Gly Gln Gly Thr Lys Val Glu Ile Lys
   100 105
<210> 90
   <211> 107
   <212> PRT
   <213> Artificial Sequence
<220>
   <221> source
   <223> /note="Description of Artificial Sequence: Synthetic polypeptide"
<400> 90
   Asp Ile Gln Met Thr Gln Ser Pro Ser Ser Leu Ser Ala Ser Val Gly
   1 5 10 15
   Asp Arg Val Thr Phe Thr Cys Arg Ala Ser Gln Gly Ile Ser Ser Ala
   20 25 30
   Leu Ala Trp Tyr Gln Gln Lys Pro Gly Lys Pro Pro Lys Leu Leu Ile
   35 40 45
   Tyr Asp Ala Ser Ser Leu Glu Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Phe Asn Asn Tyr Pro Leu
   85 90 95
   Thr Phe Gly Gly Gly Thr Lys Val Glu Ile Lys
   100 105
<210> 91
<400> 91
   000
<210> 92
<400> 92
   000
<210> 93
<400> 93
   000
<210> 94
<400> 94
   000
<210> 95
<400> 95
   000
<210> 96
<400> 96
   000
<210> 97
<400> 97
   000
<210> 98
<400> 98
   000
<210> 99
<400> 99
   000
<210> 100
<400> 100
   000
<210> 101
<400> 101
   000
<210> 102
<400> 102
   000
<210> 103
<400> 103
   000
<210> 104
<400> 104
   000
<210> 105
<400> 105
   000
<210> 106
<400> 106
   000
<210> 107
<400> 107
   000
<210> 108
<400> 108
   000
<210> 109
<400> 109
   000
<210> 110
<400> 110
   000
<210> 111
<400> 111
   000
<210> 112
<400> 112
   000
<210> 113
<400> 113
   000
<210> 114
<400> 114
   000
<210> 115
<400> 115
   000
<210> 116
<400> 116
   000
<210> 117
   <211> 10
   <212> PRT
   <213> Artificial Sequence
<220>
   <221> source
   <223> /note="Description of Artificial Sequence: Synthetic peptide"
<400> 117
   Gly Gly Thr Phe Ser Ser Tyr Ala Ile Ser
   1               5                   10
<210> 118
   <211> 10
   <212> PRT
   <213> Artificial Sequence
<220>
   <221> source
   <223> /note="Description of Artificial Sequence: Synthetic peptide"
<400> 118
   Gly Phe Thr Phe Asp Asp Tyr Ala Met His
   1               5                   10
<210> 119
   <211> 12
   <212> PRT
   <213> Artificial Sequence
<220>
   <221> source
   <223> /note="Description of Artificial Sequence: Synthetic peptide"
<400> 119
   Gly Gly Ser Ile Ser Ser Ser Ser Tyr Tyr Trp Gly
   1               5                   10
<210> 120
   <211> 10
   <212> PRT
   <213> Artificial Sequence
<220>
   <221> source
   <223> /note="Description of Artificial Sequence: Synthetic peptide"
<400> 120
   Gly Phe Thr Phe Ser Ser Tyr Trp Met Ser
   1               5                   10
<210> 121
   <211> 12
   <212> PRT
   <213> Artificial Sequence
<220>
   <221> source
   <223> /note="Description of Artificial Sequence: Synthetic peptide"
<400> 121
   Gly Gly Pro Val Arg Ser Gly Ser His Tyr Trp Asn
   1               5                   10
<210> 122
   <211> 10
   <212> PRT
   <213> Artificial Sequence
<220>
   <221> source
   <223> /note="Description of Artificial Sequence: Synthetic peptide"
<400> 122
   Gly Gly Ser Phe Ser Gly Tyr Tyr Trp Ser
   1               5                   10
<210> 123
   <211> 10
   <212> PRT
   <213> Artificial Sequence
<220>
   <221> source
   <223> /note="Description of Artificial Sequence: Synthetic peptide"
<400> 123
   Gly Phe Thr Phe Ser Ser Tyr Ser Met Asn
   1               5                   10
<210> 124
   <211> 10
   <212> PRT
   <213> Artificial Sequence
<220>
   <221> source
   <223> /note="Description of Artificial Sequence: Synthetic peptide"
<400> 124
   Gly Phe Thr Phe Ser Ser Tyr Glu Met Asn
   1               5                   10
<210> 125
   <211> 10
   <212> PRT
   <213> Artificial Sequence
<220>
   <221> source
   <223> /note="Description of Artificial Sequence: Synthetic peptide"
<400> 125
   Ala Asn Thr Phe Ser Asp His Val Met His
   1 5 10
<210> 126
   <211> 10
   <212> PRT
   <213> Artificial Sequence
<220>
   <221> source
   <223> /note="Description of Artificial Sequence: Synthetic peptide"
<400> 126
   Gly Phe Thr Phe Ser Ser Tyr Ser Met Asn
   1 5 10
<210> 127
   <211> 10
   <212> PRT
   <213> Artificial Sequence
<220>
   <221> source
   <223> /note="Description of Artificial Sequence: Synthetic peptide"
<400> 127
   Gly Phe Thr Phe Asn Ser Tyr Gly Leu His
   1 5 10
<210> 128
   <211> 10
   <212> PRT
   <213> Artificial Sequence
<220>
   <221> source
   <223> /note="Description of Artificial Sequence: Synthetic peptide"
<400> 128
   Gly Phe Asn Val Ser Ser Asn Tyr Met Thr
   1 5 10
<210> 129
   <211> 10
   <212> PRT
   <213> Artificial Sequence
<220>
   <221> source
   <223> /note="Description of Artificial Sequence: Synthetic peptide"
<400> 129
   Gly Tyr Pro Phe Thr Gly Tyr Tyr Ile Gln
   1 5 10
<210> 130
   <211> 16
   <212> PRT
   <213> Artificial Sequence
<220>
   <221> source
   <223> /note="Description of Artificial Sequence: Synthetic peptide"
<400> 130
   Gly Ile Ile Pro Ile Phe Gly Thr Ala Asn Tyr Ala Gln Lys Phe Gln
   1 5 10 15
<210> 131
   <211> 17
   <212> PRT
   <213> Artificial Sequence
<220>
   <221> source
   <223> /note="Description of Artificial Sequence: Synthetic peptide"
<400> 131
   Leu Ile Ser Gly Asp Gly Gly Ser Thr Tyr Tyr Ala Asp Ser Val Lys
   1 5 10 15
   Gly
<210> 132
   <211> 16
   <212> PRT
   <213> Artificial Sequence
<220>
   <221> source
   <223> /note="Description of Artificial Sequence: Synthetic peptide"
<400> 132
   Ser Ile Tyr Tyr Ser Gly Ser Thr Tyr Tyr Asn Pro Ser Leu Lys Ser
   1 5 10 15
<210> 133
   <211> 17
   <212> PRT
   <213> Artificial Sequence
<220>
   <221> source
   <223> /note="Description of Artificial Sequence: Synthetic peptide"
<400> 133
   Asn Ile Asn Glu Asp Gly Ser Ala Lys Phe Tyr Val Asp Ser Val Lys
   1               5                   10                  15
   Gly
<210> 134
   <211> 16
   <212> PRT
   <213> Artificial Sequence
<220>
   <221> source
   <223> /note="Description of Artificial Sequence: Synthetic peptide"
<400> 134
   Tyr Ile Tyr Tyr Ser Gly Ser Thr Asn Tyr Asn Pro Ser Leu Glu Asn
   1               5                   10                  15
<210> 135
   <211> 16
   <212> PRT
   <213> Artificial Sequence
<220>
   <221> source
   <223> /note="Description of Artificial Sequence: Synthetic peptide"
<400> 135
   Glu Ile Asn His Ser Gly Ser Thr Asn Tyr Asn Pro Ser Leu Lys Ser
   1               5                   10                  15
<210> 136
   <211> 17
   <212> PRT
   <213> Artificial Sequence
<220>
   <221> source
   <223> /note="Description of Artificial Sequence: Synthetic peptide"
<400> 136
   Ser Ile Ser Ser Ser Ser Ser Tyr Ile Tyr Tyr Ala Asp Ser Val Lys
   1               5                   10                  15
   Gly
<210> 137
   <211> 17
   <212> PRT
   <213> Artificial Sequence
<220>
   <221> source
   <223> /note="Description of Artificial Sequence: Synthetic peptide"
<400> 137
   Tyr Ile Ser Ser Ser Gly Ser Thr Ile Tyr Tyr Ala Asp Ser Val Lys
   1               5                   10                  15
   Gly
<210> 138
   <211> 17
   <212> PRT
   <213> Artificial Sequence
<220>
   <221> source
   <223> /note="Description of Artificial Sequence: Synthetic peptide"
<400> 138
   Tyr Ile His Ala Ala Asn Gly Gly Thr His Tyr Ser Gln Lys Phe Gln
   1               5                   10                  15
   Asp
<210> 139
   <211> 17
   <212> PRT
   <213> Artificial Sequence
<220>
   <221> source
   <223> /note="Description of Artificial Sequence: Synthetic peptide"
<400> 139
   Tyr Ile Ser Ser Ser Ser Ser Thr Ile Tyr Tyr Ala Asp Ser Val Lys
     1               5                   10                  15
   Gly
<210> 140
   <211> 17
   <212> PRT
   <213> Artificial Sequence
<220>
   <221> source
   <223> /note="Description of Artificial Sequence: Synthetic peptide"
<400> 140
   Leu Ile Glu Tyr Asp Gly Ser Asn Lys Tyr Tyr Gly Asp Ser Val Lys
   1               5                   10                  15
   Gly
<210> 141
   <211> 16
   <212> PRT
   <213> Artificial Sequence
<220>
   <221> source
   <223> /note="Description of Artificial Sequence: Synthetic peptide"
<400> 141
   Val Ile Tyr Ser Gly Gly Ala Thr Tyr Tyr Gly Asp Ser Val Lys Gly
   1               5                   10                  15
<210> 142
   <211> 17
   <212> PRT
   <213> Artificial Sequence
<220>
   <221> source
   <223> /note="Description of Artificial Sequence: Synthetic peptide"
<400> 142
   Trp Ile Asp Pro Asn Ser Gly Asn Thr Gly Tyr Ala Gln Lys Phe Gln
   1               5                   10                  15
   Gly
<210> 143
   <211> 11
   <212> PRT
   <213> Artificial Sequence
<220>
   <221> source
   <223> /note="Description of Artificial Sequence: Synthetic peptide"
<400> 143
   Asp Leu Glu Met Ala Thr Ile Met Gly Gly Tyr
   1               5                   10
<210> 144
   <211> 9
   <212> PRT
   <213> Artificial Sequence
<220>
   <221> source
   <223> /note="Description of Artificial Sequence: Synthetic peptide"
<400> 144
   Val Phe Asp Ser Tyr Tyr Met Asp Val
   1               5
<210> 145
   <211> 15
   <212> PRT
   <213> Artificial Sequence
<220>
   <221> source
   <223> /note="Description of Artificial Sequence: Synthetic peptide"
<400> 145
   Pro Gly Thr Tyr Tyr Asp Phe Leu Ser Gly Tyr Tyr Pro Phe Tyr
   1               5                   10                  15
<210> 146
   <211> 7
   <212> PRT
   <213> Artificial Sequence
<220>
   <221> source
   <223> /note="Description of Artificial Sequence: Synthetic peptide"
<400> 146
   Asp Leu Arg Ser Gly Arg Tyr
   1               5
<210> 147
   <211> 13
   <212> PRT
   <213> Artificial Sequence
<220>
   <221> source
   <223> /note="Description of Artificial Sequence: Synthetic peptide"
<400> 147
   Gly Thr Ala Thr Phe Asp Trp Asn Phe Pro Phe Asp Ser
   1               5                   10
<210> 148
   <211> 18
   <212> PRT
   <213> Artificial Sequence
<220>
   <221> source
   <223> /note="Description of Artificial Sequence: Synthetic peptide"
<400> 148
   Gly Ser Gly Leu Val Val Tyr Ala Ile Arg Val Gly Ser Gly Trp Phe
   1               5                   10                  15
   Asp Tyr
<210> 149
   <211> 20
   <212> PRT
   <213> Artificial Sequence
<220>
   <221> source
   <223> /note="Description of Artificial Sequence: Synthetic peptide"
<400> 149
   Asp Pro Ser Ser Ser Gly Ser Tyr Tyr Met Glu Asp Ser Tyr Tyr Tyr
   1               5                   10                  15
   Gly Met Asp Val
               20
<210> 150
   <211> 8
   <212> PRT
   <213> Artificial Sequence
<220>
   <221> source
   <223> /note="Description of Artificial Sequence: Synthetic peptide"
<400> 150
   Glu Ala Leu Gly Ser Ser Trp Glu
   1               5
<210> 151
   <211> 10
   <212> PRT
   <213> Artificial Sequence
<220>
   <221> source
   <223> /note="Description of Artificial Sequence: Synthetic peptide"
<400> 151
   Gly Gly Tyr Asn Ser Asp Ala Phe Asp Ile
   1               5                   10
<210> 152
   <211> 12
   <212> PRT
   <213> Artificial Sequence
<220>
   <221> source
   <223> /note="Description of Artificial Sequence: Synthetic peptide"
<400> 152
   Asp Leu Ser Val Arg Ala Ile Asp Ala Phe Asp Ile
   1 5 10
<210> 153
   <211> 10
   <212> PRT
   <213> Artificial Sequence
<220>
   <221> source
   <223> /note="Description of Artificial Sequence: Synthetic peptide"
<400> 153
   Glu Gly Asn Glu Asp Leu Ala Phe Asp Ile
   1 5 10
<210> 154
   <211> 19
   <212> PRT
   <213> Artificial Sequence
<220>
   <221> source
   <223> /note="Description of Artificial Sequence: Synthetic peptide"
<400> 154
   Asp Arg Leu Tyr Cys Gly Asn Asn Cys Tyr Leu Tyr Tyr Tyr Tyr Gly
   1 5 10 15
   Met Asp Val
<210> 155
   <211> 11
   <212> PRT
   <213> Artificial Sequence
<220>
   <221> source
   <223> /note="Description of Artificial Sequence: Synthetic peptide"
<400> 155
   Asp Ser Tyr Gly Tyr Tyr Tyr Gly Met Asp Val
   1 5 10
<210> 156
   <211> 14
   <212> PRT
   <213> Artificial Sequence
<220>
   <221> source
   <223> /note="Description of Artificial Sequence: Synthetic peptide"
<400> 156
   Thr Gly Thr Ser Ser Asp Val Gly Gly Tyr Asn Tyr Val Ser
   1               5                   10
<210> 157
   <211> 16
   <212> PRT
   <213> Artificial Sequence
<220>
   <221> source
   <223> /note="Description of Artificial Sequence: Synthetic peptide"
<400> 157
   Arg Ser Ser Gln Ser Leu Val Tyr Thr Asp Gly Asn Thr Tyr Leu Asn
   1               5                   10                  15
<210> 158
   <211> 11
   <212> PRT
   <213> Artificial Sequence
<220>
   <221> source
   <223> /note="Description of Artificial Sequence: Synthetic peptide"
<400> 158
   Arg Ala Ser Gln Gly Ile Ser Ser Tyr Leu Ala
   1               5                   10
<210> 159
   <211> 12
   <212> PRT
   <213> Artificial Sequence
<220>
   <221> source
   <223> /note="Description of Artificial Sequence: Synthetic peptide"
<400> 159
   Arg Ala Ser Gln Ser Ile Ser Gly Ser Phe Leu Ala
   1               5                   10
<210> 160
   <211> 11
   <212> PRT
   <213> Artificial Sequence
<220>
   <221> source
   <223> /note="Description of Artificial Sequence: Synthetic peptide"
<400> 160
   Arg Ala Ser Gln Ser Ile Ser Ser Tyr Leu Asn
   1               5                   10
<210> 161
   <211> 11
   <212> PRT
   <213> Artificial Sequence
<220>
   <221> source
   <223> /note="Description of Artificial Sequence: Synthetic peptide"
<400> 161
   Arg Ala Ser Gln Ser Ile Ser Ser Tyr Leu Asn
   1               5                   10
<210> 162
   <211> 13
   <212> PRT
   <213> Artificial Sequence
<220>
   <221> source
   <223> /note="Description of Artificial Sequence: Synthetic peptide"
<400> 162
   Thr Gly Ser Ser Gly Ser Ile Ala Ser Asn Tyr Val Gln
   1               5                   10
<210> 163
   <211> 11
   <212> PRT
   <213> Artificial Sequence
<220>
   <221> source
   <223> /note="Description of Artificial Sequence: Synthetic peptide"
<400> 163
   Gln Ala Ser Gln Asp Ile Ser Asn Tyr Leu Asn
   1               5                   10
<210> 164
   <211> 11
   <212> PRT
   <213> Artificial Sequence
<220>
   <221> source
   <223> /note="Description of Artificial Sequence: Synthetic peptide"
<400> 164
   Arg Ala Ser Gln Asp Ile Ser Ser Trp Leu Ala
   1               5                   10
<210> 165
   <211> 11
   <212> PRT
   <213> Artificial Sequence
<220>
   <221> source
   <223> /note="Description of Artificial Sequence: Synthetic peptide"
<400> 165
   Gln Ala Ser Gln Asp Ile Ser Asn Tyr Leu Asn
   1               5                   10
<210> 166
   <211> 11
   <212> PRT
   <213> Artificial Sequence
<220>
   <221> source
   <223> /note="Description of Artificial Sequence: Synthetic peptide"
<400> 166
   Gln Ala Ser Gln Phe Ile Lys Lys Asn Leu Asn
   1               5                   10
<210> 167
   <211> 11
   <212> PRT
   <213> Artificial Sequence
<220>
   <221> source
   <223> /note="Description of Artificial Sequence: Synthetic peptide"
<400> 167
   Arg Ala Ser Gln Ser Ile Ser Ser Tyr Leu Asn
   1               5                   10
<210> 168
   <211> 11
   <212> PRT
   <213> Artificial Sequence
<220>
   <221> source
   <223> /note="Description of Artificial Sequence: Synthetic peptide"
<400> 168
   Arg Ala Ser Gln Gly Ile Ser Ser Ala Leu Ala
   1 5 10
<210> 169
   <211> 7
   <212> PRT
   <213> Artificial Sequence
<220>
   <221> source
   <223> /note="Description of Artificial Sequence: Synthetic peptide"
<400> 169
   Asp Val Ser Asn Arg Pro Ser
   1 5
<210> 170
   <211> 7
   <212> PRT
   <213> Artificial Sequence
<220>
   <221> source
   <223> /note="Description of Artificial Sequence: Synthetic peptide"
<400> 170
   Lys Val Ser Asn Arg Asp Ser
   1 5
<210> 171
   <211> 7
   <212> PRT
   <213> Artificial Sequence
<220>
   <221> source
   <223> /note="Description of Artificial Sequence: Synthetic peptide"
<400> 171
   Ala Ala Ser Thr Leu Gln Ser
   1 5
<210> 172
   <211> 7
   <212> PRT
   <213> Artificial Sequence
<220>
   <221> source
   <223> /note="Description of Artificial Sequence: Synthetic peptide"
<400> 172
   Gly Ala Ser Ser Arg Ala Thr
   1               5
<210> 173
   <211> 7
   <212> PRT
   <213> Artificial Sequence
<220>
   <221> source
   <223> /note="Description of Artificial Sequence: Synthetic peptide"
<400> 173
   Ala Ala Ser Ser Leu Gln Ser
   1               5
<210> 174
   <211> 7
   <212> PRT
   <213> Artificial Sequence
<220>
   <221> source
   <223> /note="Description of Artificial Sequence: Synthetic peptide"
<400> 174
   Ala Ala Ser Ser Leu Gln Ser
   1               5
<210> 175
   <211> 7
   <212> PRT
   <213> Artificial Sequence
<220>
   <221> source
   <223> /note="Description of Artificial Sequence: Synthetic peptide"
<400> 175
   Glu Asp Asn Gln Arg Pro Ser
   1               5
<210> 176
   <211> 7
   <212> PRT
   <213> Artificial Sequence
<220>
   <221> source
   <223> /note="Description of Artificial Sequence: Synthetic peptide"
<400> 176
   Asp Ala Ser Asn Leu Glu Thr
   1               5
<210> 177
   <211> 7
   <212> PRT
   <213> Artificial Sequence
<220>
   <221> source
   <223> /note="Description of Artificial Sequence: Synthetic peptide"
<400> 177
   Ala Ala Ser Ser Leu Gln Ser
   1               5
<210> 178
   <211> 7
   <212> PRT
   <213> Artificial Sequence
<220>
   <221> source
   <223> /note="Description of Artificial Sequence: Synthetic peptide"
<400> 178
   Asp Ala Ser Asn Leu Glu Thr
   1               5
<210> 179
   <211> 7
   <212> PRT
   <213> Artificial Sequence
<220>
   <221> source
   <223> /note="Description of Artificial Sequence: Synthetic peptide"
<400> 179
   Asp Ala Ser Ser Leu Gln Thr
   1               5
<210> 180
   <211> 7
   <212> PRT
   <213> Artificial Sequence
<220>
   <221> source
   <223> /note="Description of Artificial Sequence: Synthetic peptide"
<400> 180
   Ala Ala Ser Ser Leu Gln Ser
   1 5
<210> 181
   <211> 7
   <212> PRT
   <213> Artificial Sequence
<220>
   <221> source
   <223> /note="Description of Artificial Sequence: Synthetic peptide"
<400> 181
   Asp Ala Ser Ser Leu Glu Ser
   1 5
<210> 182
   <211> 12
   <212> PRT
   <213> Artificial Sequence
<220>
   <221> source
   <223> /note="Description of Artificial Sequence: Synthetic peptide"
<400> 182
   Ser Ser Tyr Thr Ser Ser Ser Thr Leu Asp Val Val
   1 5 10
<210> 183
   <211> 9
   <212> PRT
   <213> Artificial Sequence
<220>
   <221> source
   <223> /note="Description of Artificial Sequence: Synthetic peptide"
<400> 183
   Met Gln Gly Thr His Trp Ser Phe Thr
   1 5
<210> 184
   <211> 9
   <212> PRT
   <213> Artificial Sequence
<220>
   <221> source
   <223> /note="Description of Artificial Sequence: Synthetic peptide"
<400> 184
   Gln Gln Leu Asn Ser Tyr Pro Tyr Thr
   1 5
<210> 185
   <211> 9
   <212> PRT
   <213> Artificial Sequence
<220>
   <221> source
   <223> /note="Description of Artificial Sequence: Synthetic peptide"
<400> 185
   Gln Gln Tyr Gly Ser Ser Pro Pro Thr
   1 5
<210> 186
   <211> 9
   <212> PRT
   <213> Artificial Sequence
<220>
   <221> source
   <223> /note="Description of Artificial Sequence: Synthetic peptide"
<400> 186
   Gln Gln Ser Tyr Ser Thr Pro Trp Thr
   1 5
<210> 187
   <211> 10
   <212> PRT
   <213> Artificial Sequence
<220>
   <221> source
   <223> /note="Description of Artificial Sequence: Synthetic peptide"
<400> 187
   Gln Gln Ser Tyr Ser Thr Pro Pro Trp Thr
   1 5 10
<210> 188
   <211> 10
   <212> PRT
   <213> Artificial Sequence
<220>
   <221> source
   <223> /note="Description of Artificial Sequence: Synthetic peptide"
<400> 188
   Gln Ser Tyr Asp Ser Ser Asn Gln Val Val
   1 5 10
<210> 189
   <211> 9
   <212> PRT
   <213> Artificial Sequence
<220>
   <221> source
   <223> /note="Description of Artificial Sequence: Synthetic peptide"
<400> 189
   Gln Gln Tyr Asp Asn Leu Pro Leu Thr
   1 5
<210> 190
   <211> 9
   <212> PRT
   <213> Artificial Sequence
<220>
   <221> source
   <223> /note="Description of Artificial Sequence: Synthetic peptide"
<400> 190
   Gln Gln Ser Tyr Ser Thr Pro Leu Thr
   1 5
<210> 191
   <211> 9
   <212> PRT
   <213> Artificial Sequence
<220>
   <221> source
   <223> /note="Description of Artificial Sequence: Synthetic peptide"
<400> 191
   Gln Gln Ala Tyr Ser Thr Pro Phe Thr
   1 5
<210> 192
   <211> 9
   <212> PRT
   <213> Artificial Sequence
<220>
   <221> source
   <223> /note="Description of Artificial Sequence: Synthetic peptide"
<400> 192
   Gln Gln His Asp Asn Leu Pro Leu Thr
   1 5
<210> 193
   <211> 10
   <212> PRT
   <213> Artificial Sequence
<220>
   <221> source
   <223> /note="Description of Artificial Sequence: Synthetic peptide"
<400> 193
   Gln Gln Ser Tyr Ser Thr Pro Pro Leu Thr
   1 5 10
<210> 194
   <211> 9
   <212> PRT
   <213> Artificial Sequence
<220>
   <221> source
   <223> /note="Description of Artificial Sequence: Synthetic peptide"
<400> 194
   Gln Gln Phe Asn Asn Tyr Pro Leu Thr
   1 5
<210> 195
   <211> 122
   <212> PRT
   <213> Artificial Sequence
<220>
   <221> source
   <223> /note="Description of Artificial Sequence: Synthetic polypeptide"
<400> 195
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Glu Met Asn Trp Val Arg Gln Ala Pro Gly Lys Gly Leu Glu Trp Val
           35                  40                  45
   Ser Tyr Ile Ser Ser Ser Gly Ser Thr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Pro Tyr Ser Ser Ser Trp His Asp Ala Phe Asp Ile Trp
               100                 105                 110
   Gly Gln Gly Thr Met Val Thr Val Ser Ser
           115                 120
<210> 196
   <211> 108
   <212> PRT
   <213> Artificial Sequence
<220>
   <221> source
   <223> /note="Description of Artificial Sequence: Synthetic polypeptide"
<400> 196
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Ser
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Gly Ser Ser Pro
   85 90 95
   Leu Thr Phe Gly Gly Gly Thr Lys Val Asp Ile Lys
   100 105
<210> 197
<400> 197
   000
<210> 198
<400> 198
   000
<210> 199
   <211> 10
   <212> PRT
   <213> Artificial Sequence
<220>
   <221> source
   <223> /note="Description of Artificial Sequence: Synthetic peptide"
<400> 199
   Gly Phe Thr Phe Ser Ser Tyr Glu Met Asn
   1 5 10
<210> 200
   <211> 17
   <212> PRT
   <213> Artificial Sequence
<220>
   <221> source
   <223> /note="Description of Artificial Sequence: Synthetic peptide"
<400> 200
   Tyr Ile Ser Ser Ser Gly Ser Thr Ile Tyr Tyr Ala Asp Ser Val Lys
   1 5 10 15
   Gly
<210> 201
   <211> 13
   <212> PRT
   <213> Artificial Sequence
<220>
   <221> source
   <223> /note="Description of Artificial Sequence: Synthetic peptide"
<400> 201
   Asp Pro Tyr Ser Ser Ser Trp His Asp Ala Phe Asp Ile
   1 5 10
<210> 202
   <211> 12
   <212> PRT
   <213> Artificial Sequence
<220>
   <221> source
   <223> /note="Description of Artificial Sequence: Synthetic peptide"
<400> 202
   Arg Ala Ser Gln Ser Val Ser Ser Ser Tyr Leu Ala
   1 5 10
<210> 203
   <211> 7
   <212> PRT
   <213> Artificial Sequence
<220>
   <221> source
   <223> /note="Description of Artificial Sequence: Synthetic peptide"
<400> 203
   Gly Ala Ser Ser Arg Ala Thr
   1 5
<210> 204
   <211> 9
   <212> PRT
   <213> Artificial Sequence
<220>
   <221> source
   <223> /note="Description of Artificial Sequence: Synthetic peptide"
<400> 204
   Gln Gln Tyr Gly Ser Ser Pro Leu Thr
   1               5
<210> 205
<400> 205
   000
<210> 206
<400> 206
   000
<210> 207
<400> 207
   000
<210> 208
<400> 208
   000
<210> 209
<400> 209
   000
<210> 210
<400> 210
   000
<210> 211
<400> 211
   000
<210> 212
<400> 212
   000
<210> 213
<400> 213
   000
<210> 214
<400> 214
   000
<210> 215
<400> 215
   000
<210> 216
<400> 216
   000
<210> 217
<400> 217
   000
<210> 218
<400> 218
   000
<210> 219
<400> 219
   000
<210> 220
<400> 220
   000
<210> 221
<400> 221
   000
<210> 222
<400> 222
   000
<210> 223
<400> 223
   000
<210> 224
<400> 224
   000
<210> 225
<400> 225
   000
<210> 226
<400> 226
   000
<210> 227
<400> 227
   000
<210> 228
<400> 228
   000
<210> 229
<400> 229
   000
<210> 230
<400> 230
   000
<210> 231
<400> 231
   000
<210> 232
<400> 232
   000
<210> 233
<400> 233
   000
<210> 234
<400> 234
   000
<210> 235
<400> 235
   000
<210> 236
<400> 236
   000
<210> 237
<400> 237
   000
<210> 238
<400> 238
   000
<210> 239
<400> 239
   000
<210> 240
<400> 240
   000
<210> 241
<400> 241
   000
<210> 242
<400> 242
   000
<210> 243
<400> 243
   000
<210> 244
<400> 244
   000
<210> 245
<400> 245
   000
<210> 246
<400> 246
   000
<210> 247
<400> 247
   000
<210> 248
<400> 248
   000
<210> 249
<400> 249
   000
<210> 250
<400> 250
   000
<210> 251
<400> 251
   000
<210> 252
<400> 252
   000
<210> 253
<400> 253
   000
<210> 254
<400> 254
   000
<210> 255
<400> 255
   000
<210> 256
<400> 256
   000
<210> 257
<400> 257
   000
<210> 258
<400> 258
   000
<210> 259
<400> 259
   000
<210> 260
<400> 260
   000
<210> 261
<400> 261
   000
<210> 262
<400> 262
   000
<210> 263
<400> 263
   000
<210> 264
<400> 264
   000
<210> 265
<400> 265
   000
<210> 266
<400> 266
   000
<210> 267
<400> 267
   000
<210> 268
<400> 268
   000
<210> 269
<400> 269
   000
<210> 270
<400> 270
   000
<210> 271
<400> 271
   000
<210> 272
<400> 272
   000
<210> 273
<400> 273
   000
<210> 274
<400> 274
   000
<210> 275
<400> 275
   000
<210> 276
<400> 276
   000
<210> 277
<400> 277
   000
<210> 278
<400> 278
   000
<210> 279
<400> 279
   000
<210> 280
<400> 280
   000
<210> 281
<400> 281
   000
<210> 282
<400> 282
   000
<210> 283
<400> 283
   000
<210> 284
<400> 284
   000
<210> 285
<400> 285
   000
<210> 286
<400> 286
   000
<210> 287
<400> 287
   000
<210> 288
<400> 288
   000
<210> 289
<400> 289
   000
<210> 290
<400> 290
   000
<210> 291
<400> 291
   000
<210> 292
<400> 292
   000
<210> 293
<400> 293
   000
<210> 294
<400> 294
   000
<210> 295
<400> 295
   000
<210> 296
<400> 296
   000
<210> 297
<400> 297
   000
<210> 298
<400> 298
   000
<210> 299
<400> 299
   000
<210> 300
<400> 300
   000
<210> 301
<400> 301
   000
<210> 302
<400> 302
   000
<210> 303
<400> 303
   000
<210> 304
<400> 304
   000
<210> 305
<400> 305
   000
<210> 306
<400> 306
   000
<210> 307
<400> 307
   000
<210> 308
<400> 308
   000
<210> 309
<400> 309
   000
<210> 310
<400> 310
   000
<210> 311
<400> 311
   000
<210> 312
<400> 312
   000
<210> 313
<400> 313
   000
<210> 314
   <211> 5
   <212> PRT
   <213> Artificial Sequence
<220>
   <221> source
   <223> /note="Description of Artificial Sequence: Synthetic peptide"
<400> 314
   Ser Tyr Ala Ile Ser
   1               5
<210> 315
   <211> 5
   <212> PRT
   <213> Artificial Sequence
<220>
   <221> source
   <223> /note="Description of Artificial Sequence: Synthetic peptide"
<400> 315
   Asp Tyr Ala Met His
   1               5
<210> 316
   <211> 7
   <212> PRT
   <213> Artificial Sequence
<220>
   <221> source
   <223> /note="Description of Artificial Sequence: Synthetic peptide"
<400> 316
   Ser Ser Ser Tyr Tyr Trp Gly
   1               5
<210> 317
   <211> 5
   <212> PRT
   <213> Artificial Sequence
<220>
   <221> source
   <223> /note="Description of Artificial Sequence: Synthetic peptide"
<400> 317
   Ser Tyr Trp Met Ser
   1               5
<210> 318
   <211> 7
   <212> PRT
   <213> Artificial Sequence
<220>
   <221> source
   <223> /note="Description of Artificial Sequence: Synthetic peptide"
<400> 318
   Ser Gly Ser His Tyr Trp Asn
   1               5
<210> 319
   <211> 5
   <212> PRT
   <213> Artificial Sequence
<220>
   <221> source
   <223> /note="Description of Artificial Sequence: Synthetic peptide"
<400> 319
   Gly Tyr Tyr Trp Ser
   1               5
<210> 320
   <211> 5
   <212> PRT
   <213> Artificial Sequence
<220>
   <221> source
   <223> /note="Description of Artificial Sequence: Synthetic peptide"
<400> 320
   Ser Tyr Ser Met Asn
   1 5
<210> 321
   <211> 5
   <212> PRT
   <213> Artificial Sequence
<220>
   <221> source
   <223> /note="Description of Artificial Sequence: Synthetic peptide"
<400> 321
   Ser Tyr Glu Met Asn
   1 5
<210> 322
   <211> 5
   <212> PRT
   <213> Artificial Sequence
<220>
   <221> source
   <223> /note="Description of Artificial Sequence: Synthetic peptide"
<400> 322
   Asp His Val Met His
   1 5
<210> 323
   <211> 5
   <212> PRT
   <213> Artificial Sequence
<220>
   <221> source
   <223> /note="Description of Artificial Sequence: Synthetic peptide"
<400> 323
   Ser Tyr Ser Met Asn
   1 5
<210> 324
   <211> 5
   <212> PRT
   <213> Artificial Sequence
<220>
   <221> source
   <223> /note="Description of Artificial Sequence: Synthetic peptide"
<400> 324
   Ser Tyr Gly Leu His
   1 5
<210> 325
   <211> 5
   <212> PRT
   <213> Artificial Sequence
<220>
   <221> source
   <223> /note="Description of Artificial Sequence: Synthetic peptide"
<400> 325
   Ser Asn Tyr Met Thr
   1 5
<210> 326
   <211> 5
   <212> PRT
   <213> Artificial Sequence
<220>
   <221> source
   <223> /note="Description of Artificial Sequence: Synthetic peptide"
<400> 326
   Gly Tyr Tyr Ile Gln
   1 5
<210> 327
   <211> 5
   <212> PRT
   <213> Artificial Sequence
<220>
   <221> source
   <223> /note="Description of Artificial Sequence: Synthetic peptide"
<400> 327
   Ser Tyr Glu Met Asn
   1 5
<210> 328
   <211> 17
   <212> PRT
   <213> Artificial Sequence
<220>
   <221> source
   <223> /note="Description of Artificial Sequence: Synthetic peptide"
<400> 328
   Gly Ile Ile Pro Ile Phe Gly Thr Ala Asn Tyr Ala Gln Lys Phe Gln
   1 5 10 15
   Gly
<210> 329
   <211> 17
   <212> PRT
   <213> Artificial Sequence
<220>
   <221> source
   <223> /note="Description of Artificial Sequence: Synthetic peptide"
<400> 329
   Leu Ile Ser Gly Asp Gly Gly Ser Thr Tyr Tyr Ala Asp Ser Val Lys
   1 5 10 15
   Gly
<210> 330
   <211> 16
   <212> PRT
   <213> Artificial Sequence
<220>
   <221> source
   <223> /note="Description of Artificial Sequence: Synthetic peptide"
<400> 330
   Ser Ile Tyr Tyr Ser Gly Ser Thr Tyr Tyr Asn Pro Ser Leu Lys Ser
   1 5 10 15
<210> 331
   <211> 17
   <212> PRT
   <213> Artificial Sequence
<220>
   <221> source
   <223> /note="Description of Artificial Sequence: Synthetic peptide"
<400> 331
   Asn Ile Asn Glu Asp Gly Ser Ala Lys Phe Tyr Val Asp Ser Val Lys
   1 5 10 15
   Gly
<210> 332
   <211> 16
   <212> PRT
   <213> Artificial Sequence
<220>
   <221> source
   <223> /note="Description of Artificial Sequence: Synthetic peptide"
<400> 332
   Tyr Ile Tyr Tyr Ser Gly Ser Thr Asn Tyr Asn Pro Ser Leu Glu Asn
   1               5                   10                  15
<210> 333
   <211> 16
   <212> PRT
   <213> Artificial Sequence
<220>
   <221> source
   <223> /note="Description of Artificial Sequence: Synthetic peptide"
<400> 333
   Glu Ile Asn His Ser Gly Ser Thr Asn Tyr Asn Pro Ser Leu Lys Ser
   1               5                   10                  15
<210> 334
   <211> 17
   <212> PRT
   <213> Artificial Sequence
<220>
   <221> source
   <223> /note="Description of Artificial Sequence: Synthetic peptide"
<400> 334
   Ser Ile Ser Ser Ser Ser Ser Tyr Ile Tyr Tyr Ala Asp Ser Val Lys
   1               5                   10                  15
   Gly
<210> 335
   <211> 17
   <212> PRT
   <213> Artificial Sequence
<220>
   <221> source
   <223> /note="Description of Artificial Sequence: Synthetic peptide"
<400> 335
   Tyr Ile Ser Ser Ser Gly Ser Thr Ile Tyr Tyr Ala Asp Ser Val Lys
   1               5                   10                  15
   Gly
<210> 336
   <211> 17
   <212> PRT
   <213> Artificial Sequence
<220>
   <221> source
   <223> /note="Description of Artificial Sequence: Synthetic peptide"
<400> 336
   Tyr Ile His Ala Ala Asn Gly Gly Thr His Tyr Ser Gln Lys Phe Gln
   1               5                   10                  15
   Asp
<210> 337
   <211> 17
   <212> PRT
   <213> Artificial Sequence
<220>
   <221> source
   <223> /note="Description of Artificial Sequence: Synthetic peptide"
<400> 337
   Tyr Ile Ser Ser Ser Ser Ser Thr Ile Tyr Tyr Ala Asp Ser Val Lys
   1               5                   10                  15
   Gly
<210> 338
   <211> 17
   <212> PRT
   <213> Artificial Sequence
<220>
   <221> source
   <223> /note="Description of Artificial Sequence: Synthetic peptide"
<400> 338
   Leu Ile Glu Tyr Asp Gly Ser Asn Lys Tyr Tyr Gly Asp Ser Val Lys
   1               5                   10                  15
   Gly
<210> 339
   <211> 16
   <212> PRT
   <213> Artificial Sequence
<220>
   <221> source
   <223> /note="Description of Artificial Sequence: Synthetic peptide"
<400> 339
   Val Ile Tyr Ser Gly Gly Ala Thr Tyr Tyr Gly Asp Ser Val Lys Gly
   1 5 10 15
<210> 340
   <211> 17
   <212> PRT
   <213> Artificial Sequence
<220>
   <221> source
   <223> /note="Description of Artificial Sequence: Synthetic peptide"
<400> 340
   Trp Ile Asp Pro Asn Ser Gly Asn Thr Gly Tyr Ala Gln Lys Phe Gln
   1 5 10 15
   Gly
<210> 341
   <211> 17
   <212> PRT
   <213> Artificial Sequence
<220>
   <221> source
   <223> /note="Description of Artificial Sequence: Synthetic peptide"
<400> 341
   Tyr Ile Ser Ser Ser Gly Ser Thr Ile Tyr Tyr Ala Asp Ser Val Lys
   1 5 10 15
   Gly
<210> 342
   <211> 11
   <212> PRT
   <213> Artificial Sequence
<220>
   <221> source
   <223> /note="Description of Artificial Sequence: Synthetic peptide"
<400> 342
   Asp Leu Glu Met Ala Thr Ile Met Gly Gly Tyr
   1 5 10
<210> 343
   <211> 9
   <212> PRT
   <213> Artificial Sequence
<220>
   <221> source
   <223> /note="Description of Artificial Sequence: Synthetic peptide"
<400> 343
   Val Phe Asp Ser Tyr Tyr Met Asp Val
   1               5
<210> 344
   <211> 15
   <212> PRT
   <213> Artificial Sequence
<220>
   <221> source
   <223> /note="Description of Artificial Sequence: Synthetic peptide"
<400> 344
   Pro Gly Thr Tyr Tyr Asp Phe Leu Ser Gly Tyr Tyr Pro Phe Tyr
   1               5                   10                  15
<210> 345
   <211> 7
   <212> PRT
   <213> Artificial Sequence
<220>
   <221> source
   <223> /note="Description of Artificial Sequence: Synthetic peptide"
<400> 345
   Asp Leu Arg Ser Gly Arg Tyr
   1               5
<210> 346
   <211> 13
   <212> PRT
   <213> Artificial Sequence
<220>
   <221> source
   <223> /note="Description of Artificial Sequence: Synthetic peptide"
<400> 346
   Gly Thr Ala Thr Phe Asp Trp Asn Phe Pro Phe Asp Ser
   1               5                   10
<210> 347
   <211> 18
   <212> PRT
   <213> Artificial Sequence
<220>
   <221> source
   <223> /note="Description of Artificial Sequence: Synthetic peptide"
<400> 347
   Gly Ser Gly Leu Val Val Tyr Ala Ile Arg Val Gly Ser Gly Trp Phe
   1               5                   10                  15
   Asp Tyr
<210> 348
   <211> 20
   <212> PRT
   <213> Artificial Sequence
<220>
   <221> source
   <223> /note="Description of Artificial Sequence: Synthetic peptide"
<400> 348
   Asp Pro Ser Ser Ser Gly Ser Tyr Tyr Met Glu Asp Ser Tyr Tyr Tyr
   1               5                   10                  15
   Gly Met Asp Val
               20
<210> 349
   <211> 8
   <212> PRT
   <213> Artificial Sequence
<220>
   <221> source
   <223> /note="Description of Artificial Sequence: Synthetic peptide"
<400> 349
   Glu Ala Leu Gly Ser Ser Trp Glu
   1               5
<210> 350
   <211> 10
   <212> PRT
   <213> Artificial Sequence
<220>
   <221> source
   <223> /note="Description of Artificial Sequence: Synthetic peptide"
<400> 350
   Gly Gly Tyr Asn Ser Asp Ala Phe Asp Ile
   1               5                   10
<210> 351
   <211> 12
   <212> PRT
   <213> Artificial Sequence
<220>
   <221> source
   <223> /note="Description of Artificial Sequence: Synthetic peptide"
<400> 351
   Asp Leu Ser Val Arg Ala Ile Asp Ala Phe Asp Ile
   1 5 10
<210> 352
   <211> 10
   <212> PRT
   <213> Artificial Sequence
<220>
   <221> source
   <223> /note="Description of Artificial Sequence: Synthetic peptide"
<400> 352
   Glu Gly Asn Glu Asp Leu Ala Phe Asp Ile
   1 5 10
<210> 353
   <211> 19
   <212> PRT
   <213> Artificial Sequence
<220>
   <221> source
   <223> /note="Description of Artificial Sequence: Synthetic peptide"
<400> 353
   Asp Arg Leu Tyr Cys Gly Asn Asn Cys Tyr Leu Tyr Tyr Tyr Tyr Gly
   1 5 10 15
   Met Asp Val
<210> 354
   <211> 11
   <212> PRT
   <213> Artificial Sequence
<220>
   <221> source
   <223> /note="Description of Artificial Sequence: Synthetic peptide"
<400> 354
   Asp Ser Tyr Gly Tyr Tyr Tyr Gly Met Asp Val
   1 5 10
<210> 355
   <211> 13
   <212> PRT
   <213> Artificial Sequence
<220>
   <221> source
   <223> /note="Description of Artificial Sequence: Synthetic peptide"
<400> 355
   Asp Pro Tyr Ser Ser Ser Trp His Asp Ala Phe Asp Ile
   1               5                   10
<210> 356
   <211> 14
   <212> PRT
   <213> Artificial Sequence
<220>
   <221> source
   <223> /note="Description of Artificial Sequence: Synthetic peptide"
<400> 356
   Thr Gly Thr Ser Ser Asp Val Gly Gly Tyr Asn Tyr Val Ser
   1               5                   10
<210> 357
   <211> 16
   <212> PRT
   <213> Artificial Sequence
<220>
   <221> source
   <223> /note="Description of Artificial Sequence: Synthetic peptide"
<400> 357
   Arg Ser Ser Gln Ser Leu Val Tyr Thr Asp Gly Asn Thr Tyr Leu Asn
   1               5                   10                  15
<210> 358
   <211> 11
   <212> PRT
   <213> Artificial Sequence
<220>
   <221> source
   <223> /note="Description of Artificial Sequence: Synthetic peptide"
<400> 358
   Arg Ala Ser Gln Gly Ile Ser Ser Tyr Leu Ala
   1               5                   10
<210> 359
   <211> 12
   <212> PRT
   <213> Artificial Sequence
<220>
   <221> source
   <223> /note="Description of Artificial Sequence: Synthetic peptide"
<400> 359
   Arg Ala Ser Gln Ser Ile Ser Gly Ser Phe Leu Ala
   1               5                   10
<210> 360
   <211> 11
   <212> PRT
   <213> Artificial Sequence
<220>
   <221> source
   <223> /note="Description of Artificial Sequence: Synthetic peptide"
<400> 360
   Arg Ala Ser Gln Ser Ile Ser Ser Tyr Leu Asn
   1               5                   10
<210> 361
   <211> 11
   <212> PRT
   <213> Artificial Sequence
<220>
   <221> source
   <223> /note="Description of Artificial Sequence: Synthetic peptide"
<400> 361
   Arg Ala Ser Gln Ser Ile Ser Ser Tyr Leu Asn
   1               5                   10
<210> 362
   <211> 13
   <212> PRT
   <213> Artificial Sequence
<220>
   <221> source
   <223> /note="Description of Artificial Sequence: Synthetic peptide"
<400> 362
   Thr Gly Ser Ser Gly Ser Ile Ala Ser Asn Tyr Val Gln
   1               5                   10
<210> 363
   <211> 11
   <212> PRT
   <213> Artificial Sequence
<220>
   <221> source
   <223> /note="Description of Artificial Sequence: Synthetic peptide"
<400> 363
   Gln Ala Ser Gln Asp Ile Ser Asn Tyr Leu Asn
   1               5                   10
<210> 364
   <211> 11
   <212> PRT
   <213> Artificial Sequence
<220>
   <221> source
   <223> /note="Description of Artificial Sequence: Synthetic peptide"
<400> 364
   Arg Ala Ser Gln Asp Ile Ser Ser Trp Leu Ala
   1               5                   10
<210> 365
   <211> 11
   <212> PRT
   <213> Artificial Sequence
<220>
   <221> source
   <223> /note="Description of Artificial Sequence: Synthetic peptide"
<400> 365
   Gln Ala Ser Gln Asp Ile Ser Asn Tyr Leu Asn
   1               5                   10
<210> 366
   <211> 11
   <212> PRT
   <213> Artificial Sequence
<220>
   <221> source
   <223> /note="Description of Artificial Sequence: Synthetic peptide"
<400> 366
   Gln Ala Ser Gln Phe Ile Lys Lys Asn Leu Asn
   1               5                   10
<210> 367
   <211> 11
   <212> PRT
   <213> Artificial Sequence
<220>
   <221> source
   <223> /note="Description of Artificial Sequence: Synthetic peptide"
<400> 367
   Arg Ala Ser Gln Ser Ile Ser Ser Tyr Leu Asn
   1               5                   10
<210> 368
   <211> 11
   <212> PRT
   <213> Artificial Sequence
<220>
   <221> source
   <223> /note="Description of Artificial Sequence: Synthetic peptide"
<400> 368
   Arg Ala Ser Gln Gly Ile Ser Ser Ala Leu Ala
   1               5                   10
<210> 369
   <211> 12
   <212> PRT
   <213> Artificial Sequence
<220>
   <221> source
   <223> /note="Description of Artificial Sequence: Synthetic peptide"
<400> 369
   Arg Ala Ser Gln Ser Val Ser Ser Ser Tyr Leu Ala
   1               5                   10
<210> 370
   <211> 7
   <212> PRT
   <213> Artificial Sequence
<220>
   <221> source
   <223> /note="Description of Artificial Sequence: Synthetic peptide"
<400> 370
   Asp Val Ser Asn Arg Pro Ser
   1               5
<210> 371
   <211> 7
   <212> PRT
   <213> Artificial Sequence
<220>
   <221> source
   <223> /note="Description of Artificial Sequence: Synthetic peptide"
<400> 371
   Lys Val Ser Asn Arg Asp Ser
   1               5
<210> 372
   <211> 7
   <212> PRT
   <213> Artificial Sequence
<220>
   <221> source
   <223> /note="Description of Artificial Sequence: Synthetic peptide"
<400> 372
   Ala Ala Ser Thr Leu Gln Ser
   1               5
<210> 373
   <211> 7
   <212> PRT
   <213> Artificial Sequence
<220>
   <221> source
   <223> /note="Description of Artificial Sequence: Synthetic peptide"
<400> 373
   Gly Ala Ser Ser Arg Ala Thr
   1               5
<210> 374
   <211> 7
   <212> PRT
   <213> Artificial Sequence
<220>
   <221> source
   <223> /note="Description of Artificial Sequence: Synthetic peptide"
<400> 374
   Ala Ala Ser Ser Leu Gln Ser
   1               5
<210> 375
   <211> 7
   <212> PRT
   <213> Artificial Sequence
<220>
   <221> source
   <223> /note="Description of Artificial Sequence: Synthetic peptide"
<400> 375
   Ala Ala Ser Ser Leu Gln Ser
   1 5
<210> 376
   <211> 7
   <212> PRT
   <213> Artificial Sequence
<220>
   <221> source
   <223> /note="Description of Artificial Sequence: Synthetic peptide"
<400> 376
   Glu Asp Asn Gln Arg Pro Ser
   1 5
<210> 377
   <211> 7
   <212> PRT
   <213> Artificial Sequence
<220>
   <221> source
   <223> /note="Description of Artificial Sequence: Synthetic peptide"
<400> 377
   Asp Ala Ser Asn Leu Glu Thr
   1 5
<210> 378
   <211> 7
   <212> PRT
   <213> Artificial Sequence
<220>
   <221> source
   <223> /note="Description of Artificial Sequence: Synthetic peptide"
<400> 378
   Ala Ala Ser Ser Leu Gln Ser
   1 5
<210> 379
   <211> 7
   <212> PRT
   <213> Artificial Sequence
<220>
   <221> source
   <223> /note="Description of Artificial Sequence: Synthetic peptide"
<400> 379
   Asp Ala Ser Asn Leu Glu Thr
   1 5
<210> 380
   <211> 7
   <212> PRT
   <213> Artificial Sequence
<220>
   <221> source
   <223> /note="Description of Artificial Sequence: Synthetic peptide"
<400> 380
   Asp Ala Ser Ser Leu Gln Thr
   1 5
<210> 381
   <211> 7
   <212> PRT
   <213> Artificial Sequence
<220>
   <221> source
   <223> /note="Description of Artificial Sequence: Synthetic peptide"
<400> 381
   Ala Ala Ser Ser Leu Gln Ser
   1 5
<210> 382
   <211> 7
   <212> PRT
   <213> Artificial Sequence
<220>
   <221> source
   <223> /note="Description of Artificial Sequence: Synthetic peptide"
<400> 382
   Asp Ala Ser Ser Leu Glu Ser
   1 5
<210> 383
   <211> 7
   <212> PRT
   <213> Artificial Sequence
<220>
   <221> source
   <223> /note="Description of Artificial Sequence: Synthetic peptide"
<400> 383
   Gly Ala Ser Ser Arg Ala Thr
   1 5
<210> 384
   <211> 12
   <212> PRT
   <213> Artificial Sequence
<220>
   <221> source
   <223> /note="Description of Artificial Sequence: Synthetic peptide"
<400> 384
   Ser Ser Tyr Thr Ser Ser Ser Thr Leu Asp Val Val
   1               5                   10
<210> 385
   <211> 9
   <212> PRT
   <213> Artificial Sequence
<220>
   <221> source
   <223> /note="Description of Artificial Sequence: Synthetic peptide"
<400> 385
   Met Gln Gly Thr His Trp Ser Phe Thr
   1               5
<210> 386
   <211> 9
   <212> PRT
   <213> Artificial Sequence
<220>
   <221> source
   <223> /note="Description of Artificial Sequence: Synthetic peptide"
<400> 386
   Gln Gln Leu Asn Ser Tyr Pro Tyr Thr
   1               5
<210> 387
   <211> 9
   <212> PRT
   <213> Artificial Sequence
<220>
   <221> source
   <223> /note="Description of Artificial Sequence: Synthetic peptide"
<400> 387
   Gln Gln Tyr Gly Ser Ser Pro Pro Thr
   1               5
<210> 388
   <211> 9
   <212> PRT
   <213> Artificial Sequence
<220>
   <221> source
   <223> /note="Description of Artificial Sequence: Synthetic peptide"
<400> 388
   Gln Gln Ser Tyr Ser Thr Pro Trp Thr
   1               5
<210> 389
   <211> 10
   <212> PRT
   <213> Artificial Sequence
<220>
   <221> source
   <223> /note="Description of Artificial Sequence: Synthetic peptide"
<400> 389
   Gln Gln Ser Tyr Ser Thr Pro Pro Trp Thr
   1               5                   10
<210> 390
   <211> 10
   <212> PRT
   <213> Artificial Sequence
<220>
   <221> source
   <223> /note="Description of Artificial Sequence: Synthetic peptide"
<400> 390
   Gln Ser Tyr Asp Ser Ser Asn Gln Val Val
   1               5                   10
<210> 391
   <211> 9
   <212> PRT
   <213> Artificial Sequence
<220>
   <221> source
   <223> /note="Description of Artificial Sequence: Synthetic peptide"
<400> 391
   Gln Gln Tyr Asp Asn Leu Pro Leu Thr
   1               5
<210> 392
   <211> 9
   <212> PRT
   <213> Artificial Sequence
<220>
   <221> source
   <223> /note="Description of Artificial Sequence: Synthetic peptide"
<400> 392
   Gln Gln Ser Tyr Ser Thr Pro Leu Thr
   1 5
<210> 393
   <211> 9
   <212> PRT
   <213> Artificial Sequence
<220>
   <221> source
   <223> /note="Description of Artificial Sequence: Synthetic peptide"
<400> 393
   Gln Gln Ala Tyr Ser Thr Pro Phe Thr
   1 5
<210> 394
   <211> 9
   <212> PRT
   <213> Artificial Sequence
<220>
   <221> source
   <223> /note="Description of Artificial Sequence: Synthetic peptide"
<400> 394
   Gln Gln His Asp Asn Leu Pro Leu Thr
   1 5
<210> 395
   <211> 10
   <212> PRT
   <213> Artificial Sequence
<220>
   <221> source
   <223> /note="Description of Artificial Sequence: Synthetic peptide"
<400> 395
   Gln Gln Ser Tyr Ser Thr Pro Pro Leu Thr
   1 5 10
<210> 396
   <211> 9
   <212> PRT
   <213> Artificial Sequence
<220>
   <221> source
   <223> /note="Description of Artificial Sequence: Synthetic peptide"
<400> 396
   Gln Gln Phe Asn Asn Tyr Pro Leu Thr
   1               5
<210> 397
   <211> 9
   <212> PRT
   <213> Artificial Sequence
<220>
   <221> source
   <223> /note="Description of Artificial Sequence: Synthetic peptide"
<400> 397
   Gln Gln Tyr Gly Ser Ser Pro Leu Thr
   1               5
<210> 398
   <211> 7
   <212> PRT
   <213> Artificial Sequence
<220>
   <221> source
   <223> /note="Description of Artificial Sequence: Synthetic peptide"
<400> 398
   Gly Gly Thr Phe Ser Ser Tyr
   1               5
<210> 399
   <211> 7
   <212> PRT
   <213> Artificial Sequence
<220>
   <221> source
   <223> /note="Description of Artificial Sequence: Synthetic peptide"
<400> 399
   Gly Phe Thr Phe Asp Asp Tyr
   1               5
<210> 400
   <211> 9
   <212> PRT
   <213> Artificial Sequence
<220>
   <221> source
   <223> /note="Description of Artificial Sequence: Synthetic peptide"
<400> 400
   Gly Gly Ser Ile Ser Ser Ser Ser Tyr
   1               5
<210> 401
   <211> 7
   <212> PRT
   <213> Artificial Sequence
<220>
   <221> source
   <223> /note="Description of Artificial Sequence: Synthetic peptide"
<400> 401
   Gly Phe Thr Phe Ser Ser Tyr
   1               5
<210> 402
   <211> 9
   <212> PRT
   <213> Artificial Sequence
<220>
   <221> source
   <223> /note="Description of Artificial Sequence: Synthetic peptide"
<400> 402
   Gly Gly Pro Val Arg Ser Gly Ser His
   1               5
<210> 403
   <211> 7
   <212> PRT
   <213> Artificial Sequence
<220>
   <221> source
   <223> /note="Description of Artificial Sequence: Synthetic peptide"
<400> 403
   Gly Gly Ser Phe Ser Gly Tyr
   1               5
<210> 404
   <211> 7
   <212> PRT
   <213> Artificial Sequence
<220>
   <221> source
   <223> /note="Description of Artificial Sequence: Synthetic peptide"
<400> 404
   Gly Phe Thr Phe Ser Ser Tyr
   1               5
<210> 405
   <211> 7
   <212> PRT
   <213> Artificial Sequence
<220>
   <221> source
   <223> /note="Description of Artificial Sequence: Synthetic peptide"
<400> 405
   Gly Phe Thr Phe Ser Ser Tyr
   1               5
<210> 406
   <211> 7
   <212> PRT
   <213> Artificial Sequence
<220>
   <221> source
   <223> /note="Description of Artificial Sequence: Synthetic peptide"
<400> 406
   Ala Asn Thr Phe Ser Asp His
   1               5
<210> 407
   <211> 7
   <212> PRT
   <213> Artificial Sequence
<220>
   <221> source
   <223> /note="Description of Artificial Sequence: Synthetic peptide"
<400> 407
   Gly Phe Thr Phe Ser Ser Tyr
   1               5
<210> 408
   <211> 7
   <212> PRT
   <213> Artificial Sequence
<220>
   <221> source
   <223> /note="Description of Artificial Sequence: Synthetic peptide"
<400> 408
   Gly Phe Thr Phe Asn Ser Tyr
   1 5
<210> 409
   <211> 7
   <212> PRT
   <213> Artificial Sequence
<220>
   <221> source
   <223> /note="Description of Artificial Sequence: Synthetic peptide"
<400> 409
   Gly Phe Asn Val Ser Ser Asn
   1 5
<210> 410
   <211> 7
   <212> PRT
   <213> Artificial Sequence
<220>
   <221> source
   <223> /note="Description of Artificial Sequence: Synthetic peptide"
<400> 410
   Gly Tyr Pro Phe Thr Gly Tyr
   1 5
<210> 411
   <211> 7
   <212> PRT
   <213> Artificial Sequence
<220>
   <221> source
   <223> /note="Description of Artificial Sequence: Synthetic peptide"
<400> 411
   Gly Phe Thr Phe Ser Ser Tyr
   1 5
<210> 412
   <211> 6
   <212> PRT
   <213> Artificial Sequence
<220>
   <221> source
   <223> /note="Description of Artificial Sequence: Synthetic peptide"
<400> 412
   Ile Pro Ile Phe Gly Thr
   1 5
<210> 413
   <211> 6
   <212> PRT
   <213> Artificial Sequence
<220>
   <221> source
   <223> /note="Description of Artificial Sequence: Synthetic peptide"
<400> 413
   Ser Gly Asp Gly Gly Ser
   1 5
<210> 414
   <211> 5
   <212> PRT
   <213> Artificial Sequence
<220>
   <221> source
   <223> /note="Description of Artificial Sequence: Synthetic peptide"
<400> 414
   Tyr Tyr Ser Gly Ser
   1 5
<210> 415
   <211> 6
   <212> PRT
   <213> Artificial Sequence
<220>
   <221> source
   <223> /note="Description of Artificial Sequence: Synthetic peptide"
<400> 415
   Asn Glu Asp Gly Ser Ala
   1 5
<210> 416
   <211> 5
   <212> PRT
   <213> Artificial Sequence
<220>
   <221> source
   <223> /note="Description of Artificial Sequence: Synthetic peptide"
<400> 416
   Tyr Tyr Ser Gly Ser
   1 5
<210> 417
   <211> 5
   <212> PRT
   <213> Artificial Sequence
<220>
   <221> source
   <223> /note="Description of Artificial Sequence: Synthetic peptide"
<400> 417
   Asn His Ser Gly Ser
   1 5
<210> 418
   <211> 6
   <212> PRT
   <213> Artificial Sequence
<220>
   <221> source
   <223> /note="Description of Artificial Sequence: Synthetic peptide"
<400> 418
   Ser Ser Ser Ser Ser Tyr
   1 5
<210> 419
   <211> 6
   <212> PRT
   <213> Artificial Sequence
<220>
   <221> source
   <223> /note="Description of Artificial Sequence: Synthetic peptide"
<400> 419
   Ser Ser Ser Gly Ser Thr
   1 5
<210> 420
   <211> 6
   <212> PRT
   <213> Artificial Sequence
<220>
   <221> source
   <223> /note="Description of Artificial Sequence: Synthetic peptide"
<400> 420
   His Ala Ala Asn Gly Gly
   1 5
<210> 421
   <211> 6
   <212> PRT
   <213> Artificial Sequence
<220>
   <221> source
   <223> /note="Description of Artificial Sequence: Synthetic peptide"
<400> 421
   Ser Ser Ser Ser Ser Thr
   1 5
<210> 422
   <211> 6
   <212> PRT
   <213> Artificial Sequence
<220>
   <221> source
   <223> /note="Description of Artificial Sequence: Synthetic peptide"
<400> 422
   Glu Tyr Asp Gly Ser Asn
   1 5
<210> 423
   <211> 5
   <212> PRT
   <213> Artificial Sequence
<220>
   <221> source
   <223> /note="Description of Artificial Sequence: Synthetic peptide"
<400> 423
   Tyr Ser Gly Gly Ala
   1 5
<210> 424
   <211> 6
   <212> PRT
   <213> Artificial Sequence
<220>
   <221> source
   <223> /note="Description of Artificial Sequence: Synthetic peptide"
<400> 424
   Asp Pro Asn Ser Gly Asn
   1 5
<210> 425
   <211> 6
   <212> PRT
   <213> Artificial Sequence
<220>
   <221> source
   <223> /note="Description of Artificial Sequence: Synthetic peptide"
<400> 425
   Ser Ser Ser Gly Ser Thr
   1               5
<210> 426
   <211> 11
   <212> PRT
   <213> Artificial Sequence
<220>
   <221> source
   <223> /note="Description of Artificial Sequence: Synthetic peptide"
<400> 426
   Asp Leu Glu Met Ala Thr Ile Met Gly Gly Tyr
   1               5                   10
<210> 427
   <211> 9
   <212> PRT
   <213> Artificial Sequence
<220>
   <221> source
   <223> /note="Description of Artificial Sequence: Synthetic peptide"
<400> 427
   Val Phe Asp Ser Tyr Tyr Met Asp Val
   1               5
<210> 428
   <211> 15
   <212> PRT
   <213> Artificial Sequence
<220>
   <221> source
   <223> /note="Description of Artificial Sequence: Synthetic peptide"
<400> 428
   Pro Gly Thr Tyr Tyr Asp Phe Leu Ser Gly Tyr Tyr Pro Phe Tyr
   1               5                   10                  15
<210> 429
   <211> 7
   <212> PRT
   <213> Artificial Sequence
<220>
   <221> source
   <223> /note="Description of Artificial Sequence: Synthetic peptide"
<400> 429
   Asp Leu Arg Ser Gly Arg Tyr
   1               5
<210> 430
   <211> 13
   <212> PRT
   <213> Artificial Sequence
<220>
   <221> source
   <223> /note="Description of Artificial Sequence: Synthetic peptide"
<400> 430
   Gly Thr Ala Thr Phe Asp Trp Asn Phe Pro Phe Asp Ser
   1               5                   10
<210> 431
   <211> 18
   <212> PRT
   <213> Artificial Sequence
<220>
   <221> source
   <223> /note="Description of Artificial Sequence: Synthetic peptide"
<400> 431
   Gly Ser Gly Leu Val Val Tyr Ala Ile Arg Val Gly Ser Gly Trp Phe
   1               5                   10                  15
   Asp Tyr
<210> 432
   <211> 20
   <212> PRT
   <213> Artificial Sequence
<220>
   <221> source
   <223> /note="Description of Artificial Sequence: Synthetic peptide"
<400> 432
   Asp Pro Ser Ser Ser Gly Ser Tyr Tyr Met Glu Asp Ser Tyr Tyr Tyr
   1               5                   10                  15
   Gly Met Asp Val
               20
<210> 433
   <211> 8
   <212> PRT
   <213> Artificial Sequence
<220>
   <221> source
   <223> /note="Description of Artificial Sequence: Synthetic peptide"
<400> 433
   Glu Ala Leu Gly Ser Ser Trp Glu
   1 5
<210> 434
   <211> 10
   <212> PRT
   <213> Artificial Sequence
<220>
   <221> source
   <223> /note="Description of Artificial Sequence: Synthetic peptide"
<400> 434
   Gly Gly Tyr Asn Ser Asp Ala Phe Asp Ile
   1 5 10
<210> 435
   <211> 12
   <212> PRT
   <213> Artificial Sequence
<220>
   <221> source
   <223> /note="Description of Artificial Sequence: Synthetic peptide"
<400> 435
   Asp Leu Ser Val Arg Ala Ile Asp Ala Phe Asp Ile
   1 5 10
<210> 436
   <211> 10
   <212> PRT
   <213> Artificial Sequence
<220>
   <221> source
   <223> /note="Description of Artificial Sequence: Synthetic peptide"
<400> 436
   Glu Gly Asn Glu Asp Leu Ala Phe Asp Ile
   1 5 10
<210> 437
   <211> 19
   <212> PRT
   <213> Artificial Sequence
<220>
   <221> source
   <223> /note="Description of Artificial Sequence: Synthetic peptide"
<400> 437
   Asp Arg Leu Tyr Cys Gly Asn Asn Cys Tyr Leu Tyr Tyr Tyr Tyr Gly
   1               5                   10                  15
   Met Asp Val
<210> 438
   <211> 11
   <212> PRT
   <213> Artificial Sequence
<220>
   <221> source
   <223> /note="Description of Artificial Sequence: Synthetic peptide"
<400> 438
   Asp Ser Tyr Gly Tyr Tyr Tyr Gly Met Asp Val
   1               5                   10
<210> 439
   <211> 13
   <212> PRT
   <213> Artificial Sequence
<220>
   <221> source
   <223> /note="Description of Artificial Sequence: Synthetic peptide"
<400> 439
   Asp Pro Tyr Ser Ser Ser Trp His Asp Ala Phe Asp Ile
   1               5                   10
<210> 440
   <211> 10
   <212> PRT
   <213> Artificial Sequence
<220>
   <221> source
   <223> /note="Description of Artificial Sequence: Synthetic peptide"
<400> 440
   Thr Ser Ser Asp Val Gly Gly Tyr Asn Tyr
   1               5                   10
<210> 441
   <211> 12
   <212> PRT
   <213> Artificial Sequence
<220>
   <221> source
   <223> /note="Description of Artificial Sequence: Synthetic peptide"
<400> 441
   Ser Gln Ser Leu Val Tyr Thr Asp Gly Asn Thr Tyr
   1 5 10
<210> 442
   <211> 7
   <212> PRT
   <213> Artificial Sequence
<220>
   <221> source
   <223> /note="Description of Artificial Sequence: Synthetic peptide"
<400> 442
   Ser Gln Gly Ile Ser Ser Tyr
   1 5
<210> 443
   <211> 8
   <212> PRT
   <213> Artificial Sequence
<220>
   <221> source
   <223> /note="Description of Artificial Sequence: Synthetic peptide"
<400> 443
   Ser Gln Ser Ile Ser Gly Ser Phe
   1 5
<210> 444
   <211> 7
   <212> PRT
   <213> Artificial Sequence
<220>
   <221> source
   <223> /note="Description of Artificial Sequence: Synthetic peptide"
<400> 444
   Ser Gln Ser Ile Ser Ser Tyr
   1 5
<210> 445
   <211> 7
   <212> PRT
   <213> Artificial Sequence
<220>
   <221> source
   <223> /note="Description of Artificial Sequence: Synthetic peptide"
<400> 445
   Ser Gln Ser Ile Ser Ser Tyr
   1               5
<210> 446
   <211> 9
   <212> PRT
   <213> Artificial Sequence
<220>
   <221> source
   <223> /note="Description of Artificial Sequence: Synthetic peptide"
<400> 446
   Ser Ser Gly Ser Ile Ala Ser Asn Tyr
   1               5
<210> 447
   <211> 7
   <212> PRT
   <213> Artificial Sequence
<220>
   <221> source
   <223> /note="Description of Artificial Sequence: Synthetic peptide"
<400> 447
   Ser Gln Asp Ile Ser Asn Tyr
   1               5
<210> 448
   <211> 7
   <212> PRT
   <213> Artificial Sequence
<220>
   <221> source
   <223> /note="Description of Artificial Sequence: Synthetic peptide"
<400> 448
   Ser Gln Asp Ile Ser Ser Trp
   1               5
<210> 449
   <211> 7
   <212> PRT
   <213> Artificial Sequence
<220>
   <221> source
   <223> /note="Description of Artificial Sequence: Synthetic peptide"
<400> 449
   Ser Gln Asp Ile Ser Asn Tyr
   1               5
<210> 450
   <211> 7
   <212> PRT
   <213> Artificial Sequence
<220>
   <221> source
   <223> /note="Description of Artificial Sequence: Synthetic peptide"
<400> 450
   Ser Gln Phe Ile Lys Lys Asn
   1               5
<210> 451
   <211> 7
   <212> PRT
   <213> Artificial Sequence
<220>
   <221> source
   <223> /note="Description of Artificial Sequence: Synthetic peptide"
<400> 451
   Ser Gln Ser Ile Ser Ser Tyr
   1               5
<210> 452
   <211> 7
   <212> PRT
   <213> Artificial Sequence
<220>
   <221> source
   <223> /note="Description of Artificial Sequence: Synthetic peptide"
<400> 452
   Ser Gln Gly Ile Ser Ser Ala
   1               5
<210> 453
   <211> 8
   <212> PRT
   <213> Artificial Sequence
<220>
   <221> source
   <223> /note="Description of Artificial Sequence: Synthetic peptide"
<400> 453
   Ser Gln Ser Val Ser Ser Ser Tyr
   1               5
<210> 454
   <211> 3
   <212> PRT
   <213> Artificial Sequence
<220>
   <221> source
   <223> /note="Description of Artificial Sequence: Synthetic peptide"
<400> 454
   Asp Val Ser
   1
<210> 455
   <211> 3
   <212> PRT
   <213> Artificial Sequence
<220>
   <221> source
   <223> /note="Description of Artificial Sequence: Synthetic peptide"
<400> 455
   Lys Val Ser
   1
<210> 456
   <211> 3
   <212> PRT
   <213> Artificial Sequence
<220>
   <221> source
   <223> /note="Description of Artificial Sequence: Synthetic peptide"
<400> 456
   Ala Ala Ser
   1
<210> 457
   <211> 3
   <212> PRT
   <213> Artificial Sequence
<220>
   <221> source
   <223> /note="Description of Artificial Sequence: Synthetic peptide"
<400> 457
   Gly Ala Ser
   1
<210> 458
   <211> 3
   <212> PRT
   <213> Artificial Sequence
<220>
   <221> source
   <223> /note="Description of Artificial Sequence: Synthetic peptide"
<400> 458
   Ala Ala Ser
   1
<210> 459
   <211> 3
   <212> PRT
   <213> Artificial Sequence
<220>
   <221> source
   <223> /note="Description of Artificial Sequence: Synthetic peptide"
<400> 459
   Ala Ala Ser
   1
<210> 460
   <211> 3
   <212> PRT
   <213> Artificial Sequence
<220>
   <221> source
   <223> /note="Description of Artificial Sequence: Synthetic peptide"
<400> 460
   Glu Asp Asn
   1
<210> 461
   <211> 3
   <212> PRT
   <213> Artificial Sequence
<220>
   <221> source
   <223> /note="Description of Artificial Sequence: Synthetic peptide"
<400> 461
   Asp Ala Ser
   1
<210> 462
   <211> 3
   <212> PRT
   <213> Artificial Sequence
<220>
   <221> source
   <223> /note="Description of Artificial Sequence: Synthetic peptide"
<400> 462
   Ala Ala Ser
   1
<210> 463
   <211> 3
   <212> PRT
   <213> Artificial Sequence
<220>
   <221> source
   <223> /note="Description of Artificial Sequence: Synthetic peptide"
<400> 463
   Asp Ala Ser
   1
<210> 464
   <211> 3
   <212> PRT
   <213> Artificial Sequence
<220>
   <221> source
   <223> /note="Description of Artificial Sequence: Synthetic peptide"
<400> 464
   Asp Ala Ser
   1
<210> 465
   <211> 3
   <212> PRT
   <213> Artificial Sequence
<220>
   <221> source
   <223> /note="Description of Artificial Sequence: Synthetic peptide"
<400> 465
   Ala Ala Ser
   1
<210> 466
   <211> 3
   <212> PRT
   <213> Artificial Sequence
<220>
   <221> source
   <223> /note="Description of Artificial Sequence: Synthetic peptide"
<400> 466
   Asp Ala Ser
   1
<210> 467
   <211> 3
   <212> PRT
   <213> Artificial Sequence
<220>
   <221> source
   <223> /note="Description of Artificial Sequence: Synthetic peptide"
<400> 467
   Gly Ala Ser
   1
<210> 468
   <211> 9
   <212> PRT
   <213> Artificial Sequence
<220>
   <221> source
   <223> /note="Description of Artificial Sequence: Synthetic peptide"
<400> 468
   Tyr Thr Ser Ser Ser Thr Leu Asp Val
   1               5
<210> 469
   <211> 6
   <212> PRT
   <213> Artificial Sequence
<220>
   <221> source
   <223> /note="Description of Artificial Sequence: Synthetic peptide"
<400> 469
   Gly Thr His Trp Ser Phe
   1 5
<210> 470
   <211> 6
   <212> PRT
   <213> Artificial Sequence
<220>
   <221> source
   <223> /note="Description of Artificial Sequence: Synthetic peptide"
<400> 470
   Leu Asn Ser Tyr Pro Tyr
   1 5
<210> 471
   <211> 6
   <212> PRT
   <213> Artificial Sequence
<220>
   <221> source
   <223> /note="Description of Artificial Sequence: Synthetic peptide"
<400> 471
   Tyr Gly Ser Ser Pro Pro
   1 5
<210> 472
   <211> 6
   <212> PRT
   <213> Artificial Sequence
<220>
   <221> source
   <223> /note="Description of Artificial Sequence: Synthetic peptide"
<400> 472
   Ser Tyr Ser Thr Pro Trp
   1 5
<210> 473
   <211> 7
   <212> PRT
   <213> Artificial Sequence
<220>
   <221> source
   <223> /note="Description of Artificial Sequence: Synthetic peptide"
<400> 473
   Ser Tyr Ser Thr Pro Pro Trp
   1 5
<210> 474
   <211> 7
   <212> PRT
   <213> Artificial Sequence
<220>
   <221> source
   <223> /note="Description of Artificial Sequence: Synthetic peptide"
<400> 474
   Tyr Asp Ser Ser Asn Gln Val
   1               5
<210> 475
   <211> 6
   <212> PRT
   <213> Artificial Sequence
<220>
   <221> source
   <223> /note="Description of Artificial Sequence: Synthetic peptide"
<400> 475
   Tyr Asp Asn Leu Pro Leu
   1               5
<210> 476
   <211> 6
   <212> PRT
   <213> Artificial Sequence
<220>
   <221> source
   <223> /note="Description of Artificial Sequence: Synthetic peptide"
<400> 476
   Ser Tyr Ser Thr Pro Leu
   1               5
<210> 477
   <211> 6
   <212> PRT
   <213> Artificial Sequence
<220>
   <221> source
   <223> /note="Description of Artificial Sequence: Synthetic peptide"
<400> 477
   Ala Tyr Ser Thr Pro Phe
   1               5
<210> 478
   <211> 6
   <212> PRT
   <213> Artificial Sequence
<220>
   <221> source
   <223> /note="Description of Artificial Sequence: Synthetic peptide"
<400> 478
   His Asp Asn Leu Pro Leu
   1               5
<210> 479
   <211> 7
   <212> PRT
   <213> Artificial Sequence
<220>
   <221> source
   <223> /note="Description of Artificial Sequence: Synthetic peptide"
<400> 479
   Ser Tyr Ser Thr Pro Pro Leu
   1               5
<210> 480
   <211> 6
   <212> PRT
   <213> Artificial Sequence
<220>
   <221> source
   <223> /note="Description of Artificial Sequence: Synthetic peptide"
<400> 480
   Phe Asn Asn Tyr Pro Leu
   1               5
<210> 481
   <211> 6
   <212> PRT
   <213> Artificial Sequence
<220>
   <221> source
   <223> /note="Description of Artificial Sequence: Synthetic peptide"
<400> 481
   Tyr Gly Ser Ser Pro Leu
   1               5
<210> 482
<400> 482
   000
<210> 483
<400> 483
   000
<210> 484
<400> 484
   000
<210> 485
<400> 485
   000
<210> 486
<400> 486
   000
<210> 487
<400> 487
   000
<210> 488
<400> 488
   000
<210> 489
<400> 489
   000
<210> 490
<400> 490
   000
<210> 491
<400> 491
   000
<210> 492
<400> 492
   000
<210> 493
<400> 493
   000
<210> 494
<400> 494
   000
<210> 495
<400> 495
   000
<210> 496
<400> 496
   000
<210> 497
<400> 497
   000
<210> 498
<400> 498
   000
<210> 499
<400> 499
   000
<210> 500
   <211> 232
   <212> PRT
   <213> Artificial Sequence
<220>
   <221> source
   <223> /note="Description of Artificial Sequence: Synthetic polypeptide"
<400> 500
   Gly Gly Gly Gly Ser Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Ala Val Ser His Glu Asp Pro Glu Val Lys Phe Asn Trp Tyr Val
           50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Ala Ala Pro Ile Glu Lys Thr Ile Ser Lys Ala Lys Gly Gln Pro
           115                 120                 125
   Arg Glu Pro Gln Val Cys Thr Leu Pro Pro Ser Arg Asp Glu Leu Thr
       130                 135                 140
   Lys Asn Gln Val Ser Leu Ser Cys Ala Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Val
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 501
   <211> 232
   <212> PRT
   <213> Artificial Sequence
<220>
   <221> source
   <223> /note="Description of Artificial Sequence: Synthetic polypeptide"
<400> 501
   Gly Gly Gly Gly Ser Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Ala Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Ala Ala Pro Ile Glu Lys Thr Ile Ser Lys Ala Lys Gly Gln Pro
           115                 120                 125
   Arg Glu Pro Gln Val Tyr Thr Leu Pro Pro Cys Arg Asp Glu Leu Thr
       130                 135                 140
   Lys Asn Gln Val Ser Leu Trp Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 502
   <211> 107
   <212> PRT
   <213> Artificial Sequence
<220>
   <221> source
   <223> /note="Description of Artificial Sequence: Synthetic polypeptide"
<400> 502
<210> 503
   <211> 106
   <212> PRT
   <213> Artificial Sequence
<220>
   <221> source
   <223> /note="Description of Artificial Sequence: Synthetic polypeptide"
<400> 503
   Gly Gln Pro Lys Ala Ala Pro Ser Val Thr Leu Phe Pro Pro Ser Ser
   1               5                   10                  15
   Glu Glu Leu Gln Ala Asn Lys Ala Thr Leu Val Cys Leu Ile Ser Asp
               20                  25                  30
   Phe Tyr Pro Gly Ala Val Thr Val Ala Trp Lys Ala Asp Ser Ser Pro
           35                  40                  45
   Val Lys Ala Gly Val Glu Thr Thr Thr Pro Ser Lys Gln Ser Asn Asn
       50                  55                  60
   Lys Tyr Ala Ala Ser Ser Tyr Leu Ser Leu Thr Pro Glu Gln Trp Lys
   65                  70                  75                  80
   Ser His Arg Ser Tyr Ser Cys Gln Val Thr His Glu Gly Ser Thr Val
                   85                  90                  95
   Glu Lys Thr Val Ala Pro Thr Glu Cys Ser
               100                 105
<210> 504
   <211> 330
   <212> PRT
   <213> Artificial Sequence
<220>
   <221> source
   <223> /note="Description of Artificial Sequence: Synthetic polypeptide"
<400> 504
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Arg Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Ala Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
   180 185 190
   His Gln Asp Trp Leu Asn Gly Lys Glu Tyr Lys Cys Lys Val Ser Asn
   195 200 205
   Lys Ala Leu Ala Ala Pro Ile Glu Lys Thr Ile Ser Lys Ala Lys Gly
   210 215 220
   Gln Pro Arg Glu Pro Gln Val Cys Thr Leu Pro Pro Ser Arg Glu Glu
   225 230 235 240
   Met Thr Lys Asn Gln Val Ser Leu Ser Cys Ala Val Lys Gly Phe Tyr
   245 250 255
   Pro Ser Asp Ile Ala Val Glu Trp Glu Ser Asn Gly Gln Pro Glu Asn
   260 265 270
   Asn Tyr Lys Thr Thr Pro Pro Val Leu Asp Ser Asp Gly Ser Phe Phe
   275 280 285
   Leu Val Ser Lys Leu Thr Val Asp Lys Ser Arg Trp Gln Gln Gly Asn
   290 295 300
   Val Phe Ser Cys Ser Val Met His Glu Ala Leu His Asn His Tyr Thr
   305 310 315 320
   Gln Lys Ser Leu Ser Leu Ser Pro Gly Lys
   325 330
<210> 505
   <211> 330
   <212> PRT
   <213> Artificial Sequence
<220>
   <221> source
   <223> /note="Description of Artificial Sequence: Synthetic polypeptide"
<400> 505
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Gln Thr
   65 70 75 80
   Tyr Ile Cys Asn Val Asn His Lys Pro Ser Asn Thr Lys Val Asp Lys
   85 90 95
   Arg Val Glu Pro Lys Ser Cys Asp Lys Thr His Thr Cys Pro Pro Cys
   100 105 110
   Pro Ala Pro Glu Leu Leu Gly Gly Pro Ser Val Phe Leu Phe Pro Pro
   115 120 125
   Lys Pro Lys Asp Thr Leu Met Ile Ser Arg Thr Pro Glu Val Thr Cys
   130 135 140
   Val Val Val Ala Val Ser His Glu Asp Pro Glu Val Lys Phe Asn Trp
   145 150 155 160
   Tyr Val Asp Gly Val Glu Val His Asn Ala Lys Thr Lys Pro Arg Glu
   165 170 175
   Glu Gln Tyr Asn Ser Thr Tyr Arg Val Val Ser Val Leu Thr Val Leu
   180 185 190
   His Gln Asp Trp Leu Asn Gly Lys Glu Tyr Lys Cys Lys Val Ser Asn
   195 200 205
   Lys Ala Leu Ala Ala Pro Ile Glu Lys Thr Ile Ser Lys Ala Lys Gly
   210 215 220
   Gln Pro Arg Glu Pro Gln Val Tyr Thr Leu Pro Pro Cys Arg Glu Glu
   225 230 235 240
   Met Thr Lys Asn Gln Val Ser Leu Trp Cys Leu Val Lys Gly Phe Tyr
   245 250 255
   Pro Ser Asp Ile Ala Val Glu Trp Glu Ser Asn Gly Gln Pro Glu Asn
   260 265 270
   Asn Tyr Lys Thr Thr Pro Pro Val Leu Asp Ser Asp Gly Ser Phe Phe
   275 280 285
   Leu Tyr Ser Lys Leu Thr Val Asp Lys Ser Arg Trp Gln Gln Gly Asn
   290 295 300
   Val Phe Ser Cys Ser Val Met His Glu Ala Leu His Asn His Tyr Thr
   305 310 315 320
   Gln Lys Ser Leu Ser Leu Ser Pro Gly Lys
   325 330
<210> 506
   <211> 245
   <212> PRT
   <213> Artificial Sequence
<220>
   <221> source
   <223> /note="Description of Artificial Sequence: Synthetic polypeptide"
<400> 506
   Gln Val Gln Leu Val Gln Ser Gly Gly Gly Val Val Gln Pro Gly Arg
   1 5 10 15
   Ser Leu Arg Leu Ser Cys Lys Ala Ser Gly Tyr Thr Phe Thr Arg Tyr
   20 25 30
   Thr Met His Trp Val Arg Gln Ala Pro Gly Lys Gly Leu Glu Trp Ile
   35 40 45
   Gly Tyr Ile Asn Pro Ser Arg Gly Tyr Thr Asn Tyr Asn Gln Lys Val
   50 55 60
   Lys Asp Arg Phe Thr Ile Ser Arg Asp Asn Ser Lys Asn Thr Ala Phe
   65 70 75 80
   Leu Gln Met Asp Ser Leu Arg Pro Glu Asp Thr Gly Val Tyr Phe Cys
   85 90 95
   Ala Arg Tyr Tyr Asp Asp His Tyr Ser Leu Asp Tyr Trp Gly Gln Gly
   100 105 110
   Thr Pro Val Thr Val Ser Ser Gly Gly Gly Gly Ser Gly Gly Gly Lys
   115 120 125
   Ser Lys Lys Gly Gly Ser Gly Gly Gly Gly Ser Asp Ile Gln Met Thr
   130 135 140
   Gln Ser Pro Ser Ser Leu Ser Ala Ser Val Gly Asp Arg Val Thr Ile
   145 150 155 160
   Thr Cys Ser Ala Ser Ser Ser Val Ser Tyr Met Asn Trp Tyr Gln Gln
   165 170 175
   Thr Pro Gly Lys Ala Pro Lys Arg Trp Ile Tyr Asp Thr Ser Lys Leu
   180 185 190
   Ala Ser Gly Val Pro Ser Arg Phe Ser Gly Ser Gly Ser Gly Thr Asp
   195 200 205
   Tyr Thr Phe Thr Ile Ser Ser Leu Gln Pro Glu Asp Ile Ala Thr Tyr
   210 215 220
   Tyr Cys Gln Gln Trp Ser Ser Asn Pro Phe Thr Phe Gly Gln Gly Thr
   225 230 235 240
   Lys Leu Gln Ile Thr
   245
<210> 507
   <211> 477
   <212> PRT
   <213> Artificial Sequence
<220>
   <221> source
   <223> /note="Description of Artificial Sequence: Synthetic polypeptide"
<400> 507
   Gln Val Gln Leu Val Gln Ser Gly Gly Gly Val Val Gln Pro Gly Arg
   1 5 10 15
   Ser Leu Arg Leu Ser Cys Lys Ala Ser Gly Tyr Thr Phe Thr Arg Tyr
   20 25 30
   Thr Met His Trp Val Arg Gln Ala Pro Gly Lys Gly Leu Glu Trp Ile
   35 40 45
   Gly Tyr Ile Asn Pro Ser Arg Gly Tyr Thr Asn Tyr Asn Gln Lys Val
   50 55 60
   Lys Asp Arg Phe Thr Ile Ser Arg Asp Asn Ser Lys Asn Thr Ala Phe
   65 70 75 80
   Leu Gln Met Asp Ser Leu Arg Pro Glu Asp Thr Gly Val Tyr Phe Cys
   85 90 95
   Ala Arg Tyr Tyr Asp Asp His Tyr Ser Leu Asp Tyr Trp Gly Gln Gly
   100 105 110
   Thr Pro Val Thr Val Ser Ser Gly Gly Gly Gly Ser Gly Gly Gly Lys
   115 120 125
   Ser Lys Lys Gly Gly Ser Gly Gly Gly Gly Ser Asp Ile Gln Met Thr
       130                 135                 140
   Gln Ser Pro Ser Ser Leu Ser Ala Ser Val Gly Asp Arg Val Thr Ile
   145                 150                 155                 160
   Thr Cys Ser Ala Ser Ser Ser Val Ser Tyr Met Asn Trp Tyr Gln Gln
                   165                 170                 175
   Thr Pro Gly Lys Ala Pro Lys Arg Trp Ile Tyr Asp Thr Ser Lys Leu
               180                 185                 190
   Ala Ser Gly Val Pro Ser Arg Phe Ser Gly Ser Gly Ser Gly Thr Asp
           195                 200                 205
   Tyr Thr Phe Thr Ile Ser Ser Leu Gln Pro Glu Asp Ile Ala Thr Tyr
       210                 215                 220
   Tyr Cys Gln Gln Trp Ser Ser Asn Pro Phe Thr Phe Gly Gln Gly Thr
   225                 230                 235                 240
   Lys Leu Gln Ile Thr Gly Gly Gly Gly Ser Asp Lys Thr His Thr Cys
                   245                 250                 255
   Pro Pro Cys Pro Ala Pro Glu Leu Leu Gly Gly Pro Ser Val Phe Leu
               260                 265                 270
   Phe Pro Pro Lys Pro Lys Asp Thr Leu Met Ile Ser Arg Thr Pro Glu
           275                 280                 285
   Val Thr Cys Val Val Val Ala Val Ser His Glu Asp Pro Glu Val Lys
       290                 295                 300
   Phe Asn Trp Tyr Val Asp Gly Val Glu Val His Asn Ala Lys Thr Lys
   305                 310                 315                 320
   Pro Arg Glu Glu Gln Tyr Asn Ser Thr Tyr Arg Val Val Ser Val Leu
                   325                 330                 335
   Thr Val Leu His Gln Asp Trp Leu Asn Gly Lys Glu Tyr Lys Cys Lys
               340                 345                 350
   Val Ser Asn Lys Ala Leu Ala Ala Pro Ile Glu Lys Thr Ile Ser Lys
           355                 360                 365
   Ala Lys Gly Gln Pro Arg Glu Pro Gln Val Cys Thr Leu Pro Pro Ser
       370                 375                 380
   Arg Asp Glu Leu Thr Lys Asn Gln Val Ser Leu Ser Cys Ala Val Lys
   385 390 395 400
   Gly Phe Tyr Pro Ser Asp Ile Ala Val Glu Trp Glu Ser Asn Gly Gln
   405 410 415
   Pro Glu Asn Asn Tyr Lys Thr Thr Pro Pro Val Leu Asp Ser Asp Gly
   420 425 430
   Ser Phe Phe Leu Val Ser Lys Leu Thr Val Asp Lys Ser Arg Trp Gln
   435 440 445
   Gln Gly Asn Val Phe Ser Cys Ser Val Met His Glu Ala Leu His Asn
   450 455 460
   His Tyr Thr Gln Lys Ser Leu Ser Leu Ser Pro Gly Lys
   465 470 475
<210> 508
   <211> 318
   <212> DNA
   <213> Artificial Sequence
<220>
   <221> source
   <223> /note="Description of Artificial Sequence: Synthetic polynucleotide"
<400> 508
   gacattcaga tgactcagag cccaagctcc ctctccgcct ccgtgggtga tcgcgtgacc 60
   attacttgct ccgcctcgtc atccgtgtca tacatgaact ggtatcagca gacccccgga 120
   aaggccccga agcgctggat ctacgacacc tccaagctgg cttccggcgt gcctagccgg 180
   ttcagcggaa gcggttccgg gaccgactac acttttacca tttcctccct gcaacccgag 240
   gacatcgcga cgtattactg ccagcagtgg tcctccaacc ccttcacctt cggacagggt 300
   acaaagctgc agatcacc 318
<210> 509
   <211> 354
   <212> DNA
   <213> Artificial Sequence
<220>
   <221> source
   <223> /note="Description of Artificial Sequence: Synthetic polynucleotide"
<400> 509
   caagtgcagt tggtgcagtc cggtggtgga gtggtccagc cgggcagatc actgaggctt 60
   agctgcaagg catccgggta caccttcacc cggtacacta tgcactgggt ccgccaagcc 120
   ccgggaaaag gactggaatg gatcggctac atcaacccat cgagagggta caccaactac 180
   aatcagaagg tcaaggaccg gttcactatc tcgagggaca actcaaagaa caccgcgttc 240
   ctgcaaatgg attcgctgcg gccggaggac accggggtgt acttctgtgc ccggtactac 300
   gatgaccact actctctgga ctactggggc cagggcactc ctgtgaccgt gtcc 354
<210> 510
   <211> 735
   <212> DNA
   <213> Artificial Sequence
<220>
   <221> source
   <223> /note="Description of Artificial Sequence: Synthetic polynucleotide"
<400> 510
   caagtgcagt tggtgcagtc cggtggtgga gtggtccagc cgggcagatc actgaggctt 60
   agctgcaagg catccgggta caccttcacc cggtacacta tgcactgggt ccgccaagcc 120
   ccgggaaaag gactggaatg gatcggctac atcaacccat cgagagggta caccaactac 180
   aatcagaagg tcaaggaccg gttcactatc tcgagggaca actcaaagaa caccgcgttc 240
   ctgcaaatgg attcgctgcg gccggaggac accggggtgt acttctgtgc ccggtactac 300
   gatgaccact actctctgga ctactggggc cagggcactc ctgtgaccgt gtcctcgggg 360
   ggaggaggaa gcggcggagg aaaatccaag aagggcggca gcgggggcgg aggctcggac 420
   attcagatga ctcagagccc aagctccctc tccgcctccg tgggtgatcg cgtgaccatt 480
   acttgctccg cctcgtcatc cgtgtcatac atgaactggt atcagcagac ccccggaaag 540
   gccccgaagc gctggatcta cgacacctcc aagctggctt ccggcgtgcc tagccggttc 600
   agcggaagcg gttccgggac cgactacact tttaccattt cctccctgca acccgaggac 660
   atcgcgacgt attactgcca gcagtggtcc tccaacccct tcaccttcgg acagggtaca 720
   aagctgcaga tcacc 735
<210> 511
   <211> 1431
   <212> DNA
   <213> Artificial Sequence
<220>
   <221> source
   <223> /note="Description of Artificial Sequence: Synthetic polynucleotide"
<400> 511
   caagtgcagt tggtgcagtc cggtggtgga gtggtccagc cgggcagatc actgaggctt      60
   agctgcaagg catccgggta caccttcacc cggtacacta tgcactgggt ccgccaagcc     120
   ccgggaaaag gactggaatg gatcggctac atcaacccat cgagagggta caccaactac     180
   aatcagaagg tcaaggaccg gttcactatc tcgagggaca actcaaagaa caccgcgttc     240
   ctgcaaatgg attcgctgcg gccggaggac accggggtgt acttctgtgc ccggtactac     300
   gatgaccact actctctgga ctactggggc cagggcactc ctgtgaccgt gtcctcgggg     360
   ggaggaggaa gcggcggagg aaaatccaag aagggcggca gcgggggcgg aggctcggac     420
   attcagatga ctcagagccc aagctccctc tccgcctccg tgggtgatcg cgtgaccatt     480
   acttgctccg cctcgtcatc cgtgtcatac atgaactggt atcagcagac ccccggaaag     540
   gccccgaagc gctggatcta cgacacctcc aagctggctt ccggcgtgcc tagccggttc     600
   agcggaagcg gttccgggac cgactacact tttaccattt cctccctgca acccgaggac     660
   atcgcgacgt attactgcca gcagtggtcc tccaacccct tcaccttcgg acagggtaca     720
   aagctgcaga tcaccggagg gggcggatcc gacaagaccc acacctgtcc tccttgtcct     780
   gccccggaac tgctgggcgg ccccagcgtg ttcctgttcc cgccgaagcc taaggatact     840
   ctcatgatca gcaggacgcc tgaagtgacc tgtgtcgtgg tggccgtgtc ccatgaagat     900
   ccagaagtca agttcaattg gtacgtggac ggcgtggagg tgcacaacgc caagacaaag     960
   cctagagagg aacagtacaa cagcacctac cgcgtcgtgt ccgtgctgac cgtgctgcac    1020
   caggactggc tgaacgggaa ggagtacaag tgcaaagtgt ccaacaaggc cctggccgcc    1080
   ccaattgaaa agactatctc caaggccaag ggccagcccc gcgagcccca ggtgtgcact    1140
   ctgccgccgt caagagatga actgactaag aaccaggtgt cactgtcctg cgccgtgaaa    1200
   gggttctacc cctccgacat cgccgtggag tgggaaagca acggacagcc tgaaaacaac    1260
   tacaaaacga ctccccctgt gctcgactcc gatggctcgt tcttcttggt gtcgaagctc    1320
   accgtggata agagccggtg gcaacaggga aacgtgtttt cctgctccgt gatgcatgag    1380
   gccctccaca accactacac ccagaaatcc ctctccctgt cgccggggaa g             1431
<210> 512
   <211> 480
   <212> PRT
   <213> Artificial Sequence
<220>
   <221> source
   <223> /note="Description of Artificial Sequence: Synthetic polypeptide"
<400> 512
   Glu Val Gln Leu Val Gln Ser Gly Gly Gly Val Val Arg Ser Gly Arg
   1               5                   10                  15
   Ser Leu Arg Leu Ser Cys Ala Ala Ser Gly Phe Thr Phe Asn Ser Tyr
               20                  25                  30
   Gly Leu His Trp Val Arg Gln Ala Pro Gly Lys Gly Leu Glu Trp Val
           35                  40                  45
   Ala Leu Ile Glu Tyr Asp Gly Ser Asn Lys Tyr Tyr Gly Asp Ser Val
       50                  55                  60
   Lys Gly Arg Phe Thr Ile Ser Arg Asp Lys Ser Lys Ser Thr Leu Tyr
   65                  70                  75                  80
   Leu Gln Met Asp Asn Leu Arg Ala Glu Asp Thr Ala Val Tyr Tyr Cys
                   85                  90                  95
   Ala Arg Glu Gly Asn Glu Asp Leu Ala Phe Asp Ile Trp Gly Gln Gly
               100                 105                 110
   Thr Leu Val Thr Val Ser Ser Gly Gly Gly Gly Ser Gly Gly Gly Gly
           115                 120                 125
   Ser Gly Gly Gly Gly Ser Gly Gly Gly Gly Ser Glu Ile Val Leu Thr
       130                 135                 140
   Gln Ser Pro Ser Ser Leu Ser Ala Ser Val Gly Asp Arg Val Thr Ile
   145                 150                 155                 160
   Thr Cys Gln Ala Ser Gln Phe Ile Lys Lys Asn Leu Asn Trp Tyr Gln
                   165                 170                 175
   His Lys Pro Gly Lys Ala Pro Lys Leu Leu Ile Tyr Asp Ala Ser Ser
               180                 185                 190
   Leu Gln Thr Gly Val Pro Ser Arg Phe Ser Gly Asn Arg Ser Gly Thr
           195                 200                 205
   Thr Phe Ser Phe Thr Ile Ser Ser Leu Gln Pro Glu Asp Val Ala Thr
       210                 215                 220
   Tyr Tyr Cys Gln Gln His Asp Asn Leu Pro Leu Thr Phe Gly Gly Gly
   225                 230                 235                 240
   Thr Lys Val Glu Ile Lys Gly Gly Gly Gly Ser Gly Ser Asp Lys Thr
                   245                 250                 255
   His Thr Cys Pro Pro Cys Pro Ala Pro Glu Leu Leu Gly Gly Pro Ser
               260                 265                 270
   Val Phe Leu Phe Pro Pro Lys Pro Lys Asp Thr Leu Met Ile Ser Arg
           275                 280                 285
   Thr Pro Glu Val Thr Cys Val Val Val Ala Val Ser His Glu Asp Pro
   290 295 300
   Glu Val Lys Phe Asn Trp Tyr Val Asp Gly Val Glu Val His Asn Ala
   305 310 315 320
   Lys Thr Lys Pro Arg Glu Glu Gln Tyr Asn Ser Thr Tyr Arg Val Val
   325 330 335
   Ser Val Leu Thr Val Leu His Gln Asp Trp Leu Asn Gly Lys Glu Tyr
   340 345 350
   Lys Cys Lys Val Ser Asn Lys Ala Leu Ala Ala Pro Ile Glu Lys Thr
   355 360 365
   Ile Ser Lys Ala Lys Gly Gln Pro Arg Glu Pro Gln Val Tyr Thr Leu
   370 375 380
   Pro Pro Cys Arg Asp Glu Leu Thr Lys Asn Gln Val Ser Leu Trp Cys
   385 390 395 400
   Leu Val Lys Gly Phe Tyr Pro Ser Asp Ile Ala Val Glu Trp Glu Ser
   405 410 415
   Asn Gly Gln Pro Glu Asn Asn Tyr Lys Thr Thr Pro Pro Val Leu Asp
   420 425 430
   Ser Asp Gly Ser Phe Phe Leu Tyr Ser Lys Leu Thr Val Asp Lys Ser
   435 440 445
   Arg Trp Gln Gln Gly Asn Val Phe Ser Cys Ser Val Met His Glu Ala
   450 455 460
   Leu His Asn His Tyr Thr Gln Lys Ser Leu Ser Leu Ser Pro Gly Lys
   465 470 475 480
<210> 513
   <211> 321
   <212> DNA
   <213> Artificial Sequence
<220>
   <221> source
   <223> /note="Description of Artificial Sequence: Synthetic polynucleotide"
<400> 513
   gaaattgtgc tgacccagag cccatcgtca ctgtccgcat ccgtcggcga ccgcgtgact 60
   atcacttgcc aagcgtccca gttcatcaag aaaaatctga actggtacca gcataagccg 120
   gggaaggcgc ccaagctgct gatctacgac gcctcatccc tccaaactgg agtgcccagc 180
   agattcagcg gaaaccggtc cggaaccacc ttctcgttta ctatttcgag cctgcaaccc 240
   gaggatgtgg ccacctacta ctgtcagcag cacgacaact tgcctctcac cttcggtggt 300
   ggaaccaaag tggagattaa g 321
<210> 514
   <211> 357
   <212> DNA
   <213> Artificial Sequence
<220>
   <221> source
   <223> /note="Description of Artificial Sequence: Synthetic polynucleotide"
<400> 514
   gaagtgcagc tggtgcagtc gggtggcgga gtggtccggt ccgggcggtc cctgcgcctg 60
   tcgtgcgctg cctccggctt cactttcaac tcatacggac tgcactgggt cagacaggcc 120
   ccggggaagg gactggaatg ggtcgcgctc atcgaatacg atgggtccaa caagtattac 180
   ggcgacagcg tgaagggccg gttcaccatc tcccgcgaca agtccaagtc aaccctgtac 240
   ctccaaatgg ataacctgag ggccgaggac accgccgtgt actactgcgc tcgggaaggg 300
   aacgaggacc tggccttcga tatctggggc cagggaaccc tcgtgacggt gtccagc 357
<210> 515
   <211> 738
   <212> DNA
   <213> Artificial Sequence
<220>
   <221> source
   <223> /note="Description of Artificial Sequence: Synthetic polynucleotide"
<400> 515
   gaagtgcagc tggtgcagtc gggtggcgga gtggtccggt ccgggcggtc cctgcgcctg      60
   tcgtgcgctg cctccggctt cactttcaac tcatacggac tgcactgggt cagacaggcc     120
   ccggggaagg gactggaatg ggtcgcgctc atcgaatacg atgggtccaa caagtattac     180
   ggcgacagcg tgaagggccg gttcaccatc tcccgcgaca agtccaagtc aaccctgtac     240
   ctccaaatgg ataacctgag ggccgaggac accgccgtgt actactgcgc tcgggaaggg     300
   aacgaggacc tggccttcga tatctggggc cagggaaccc tcgtgacggt gtccagcggc     360
   ggcggtggaa gcggcggtgg cgggagcggg ggaggaggat ctggaggcgg aggctccgaa     420
   attgtgctga cccagagccc atcgtcactg tccgcatccg tcggcgaccg cgtgactatc     480
   acttgccaag cgtcccagtt catcaagaaa aatctgaact ggtaccagca taagccgggg     540
   aaggcgccca agctgctgat ctacgacgcc tcatccctcc aaactggagt gcccagcaga     600
   ttcagcggaa accggtccgg aaccaccttc tcgtttacta tttcgagcct gcaacccgag     660
   gatgtggcca cctactactg tcagcagcac gacaacttgc ctctcacctt cggtggtgga     720
   accaaagtgg agattaag                                                   738
<210> 516
   <211> 1440
   <212> DNA
   <213> Artificial Sequence
<220>
   <221> source
   <223> /note="Description of Artificial Sequence: Synthetic polynucleotide"
<400> 516
   gaagtgcagc tggtgcagtc gggtggcgga gtggtccggt ccgggcggtc cctgcgcctg 60
   tcgtgcgctg cctccggctt cactttcaac tcatacggac tgcactgggt cagacaggcc 120
   ccggggaagg gactggaatg ggtcgcgctc atcgaatacg atgggtccaa caagtattac 180
   ggcgacagcg tgaagggccg gttcaccatc tcccgcgaca agtccaagtc aaccctgtac 240
   ctccaaatgg ataacctgag ggccgaggac accgccgtgt actactgcgc tcgggaaggg 300
   aacgaggacc tggccttcga tatctggggc cagggaaccc tcgtgacggt gtccagcggc 360
   ggcggtggaa gcggcggtgg cgggagcggg ggaggaggat ctggaggcgg aggctccgaa 420
   attgtgctga cccagagccc atcgtcactg tccgcatccg tcggcgaccg cgtgactatc 480
   acttgccaag cgtcccagtt catcaagaaa aatctgaact ggtaccagca taagccgggg 540
   aaggcgccca agctgctgat ctacgacgcc tcatccctcc aaactggagt gcccagcaga 600
   ttcagcggaa accggtccgg aaccaccttc tcgtttacta tttcgagcct gcaacccgag 660
   gatgtggcca cctactactg tcagcagcac gacaacttgc ctctcacctt cggtggtgga 720
   accaaagtgg agattaaggg tggcggggga tccggatccg ataagaccca cacctgtcca 780
   ccctgccctg cccccgaact gcttggtggt ccgtccgtgt ttctgttccc gcccaagccc 840
   aaggacaccc tcatgatctc acggactcct gaagtgacct gtgtggtggt cgctgtgtcc 900
   cacgaggacc ccgaagtcaa gttcaattgg tacgtggacg gagtggaagt gcacaacgct 960
   aagaccaagc cccgcgagga acagtacaac tccacttacc gcgtcgtgtc ggtgctgacc 1020
   gtgctgcatc aggattggct gaacggaaag gagtacaagt gcaaggtgtc caacaaggct 1080
   ctggcggcac ccatcgaaaa gaccatcagc aaggccaaag ggcaacctag agaaccacaa 1140
   gtctacaccc tgcctccttg ccgggatgag ctcaccaaga accaggtgtc cctgtggtgc 1200
   ctcgtgaagg gcttctaccc ctctgacatc gcggtggaat gggagtcaaa cggccagcca 1260
   gagaacaact acaagacaac cccccctgtc ctggacagcg acggctcctt cttcctgtac 1320
   tcgaagctga ctgtggataa gagccggtgg caacagggca acgtgttctc atgttcggtc 1380
   atgcacgagg ccctgcataa ccactacact cagaagtccc tgagcctgtc ccctggaaag 1440
<210> 517
   <211> 489
   <212> PRT
   <213> Artificial Sequence
<220>
   <221> source
   <223> /note="Description of Artificial Sequence: Synthetic polypeptide"
<400> 517
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Asn Val Ser Ser Asn
               20                  25                  30
   Tyr Met Thr Trp Val Arg Gln Ala Pro Gly Lys Gly Leu Glu Trp Val
           35                  40                  45
   Ser Val Ile Tyr Ser Gly Gly Ala Thr Tyr Tyr Gly Asp Ser Val Lys
       50                  55                  60
   Gly Arg Phe Thr Val Ser Arg Asp Asn Ser Lys Asn Thr Val Tyr Leu
   65                  70                  75                  80
   Gln Met Asn Arg Leu Thr Ala Glu Asp Thr Ala Val Tyr Tyr Cys Ala
                   85                  90                  95
   Arg Asp Arg Leu Tyr Cys Gly Asn Asn Cys Tyr Leu Tyr Tyr Tyr Tyr
               100                 105                 110
   Gly Met Asp Val Trp Gly Gln Gly Thr Leu Val Thr Val Ser Ser Gly
           115                 120                 125
   Gly Gly Gly Ser Gly Gly Gly Gly Ser Gly Gly Gly Gly Ser Gly Gly
       130                 135                 140
   Gly Gly Ser Asp Ile Gln Val Thr Gln Ser Pro Ser Ser Leu Ser Ala
   145                 150                 155                 160
   Ser Val Gly Asp Arg Val Thr Ile Thr Cys Arg Ala Ser Gln Ser Ile
                   165                 170                 175
   Ser Ser Tyr Leu Asn Trp Tyr Gln Gln Lys Pro Gly Lys Ala Pro Lys
   180 185 190
   Leu Leu Ile Tyr Ala Ala Ser Ser Leu Gln Ser Gly Val Pro Ser Arg
   195 200 205
   Phe Ser Gly Ser Gly Ser Gly Thr Asp Phe Thr Leu Thr Ile Ser Ser
   210 215 220
   Leu Gln Pro Glu Asp Phe Ala Thr Tyr Tyr Cys Gln Gln Ser Tyr Ser
   225 230 235 240
   Thr Pro Pro Leu Thr Phe Gly Gln Gly Thr Lys Val Glu Ile Lys Gly
   245 250 255
   Gly Gly Gly Ser Gly Ser Asp Lys Thr His Thr Cys Pro Pro Cys Pro
   260 265 270
   Ala Pro Glu Leu Leu Gly Gly Pro Ser Val Phe Leu Phe Pro Pro Lys
   275 280 285
   Pro Lys Asp Thr Leu Met Ile Ser Arg Thr Pro Glu Val Thr Cys Val
   290 295 300
   Val Val Ala Val Ser His Glu Asp Pro Glu Val Lys Phe Asn Trp Tyr
   305 310 315 320
   Val Asp Gly Val Glu Val His Asn Ala Lys Thr Lys Pro Arg Glu Glu
   325 330 335
   Gln Tyr Asn Ser Thr Tyr Arg Val Val Ser Val Leu Thr Val Leu His
   340 345 350
   Gln Asp Trp Leu Asn Gly Lys Glu Tyr Lys Cys Lys Val Ser Asn Lys
   355 360 365
   Ala Leu Ala Ala Pro Ile Glu Lys Thr Ile Ser Lys Ala Lys Gly Gln
   370 375 380
   Pro Arg Glu Pro Gln Val Tyr Thr Leu Pro Pro Cys Arg Asp Glu Leu
   385 390 395 400
   Thr Lys Asn Gln Val Ser Leu Trp Cys Leu Val Lys Gly Phe Tyr Pro
   405 410 415
   Ser Asp Ile Ala Val Glu Trp Glu Ser Asn Gly Gln Pro Glu Asn Asn
   420 425 430
   Tyr Lys Thr Thr Pro Pro Val Leu Asp Ser Asp Gly Ser Phe Phe Leu
   435 440 445
   Tyr Ser Lys Leu Thr Val Asp Lys Ser Arg Trp Gln Gln Gly Asn Val
   450 455 460
   Phe Ser Cys Ser Val Met His Glu Ala Leu His Asn His Tyr Thr Gln
   465 470 475 480
   Lys Ser Leu Ser Leu Ser Pro Gly Lys
   485
<210> 518
   <211> 324
   <212> DNA
   <213> Artificial Sequence
<220>
   <221> source
   <223> /note="Description of Artificial Sequence: Synthetic polynucleotide"
<400> 518
   gatattcagg tcacccaatc gccgtcctcc ctgagcgcct ccgtggggga tcgcgtgacg 60
   attacttgcc gggccagcca gagcatctcc tcgtacctga actggtacca gcagaagccg 120
   ggaaaggccc ccaagctgct gatctacgct gcatcaagcc tgcagtccgg cgtgcctagc 180
   cggttttccg gttccggttc gggtaccgac ttcacactga ccatctcctc actgcaacca 240
   gaggatttcg ccacctacta ctgtcagcag tcatactcca ctccgcccct gaccttcgga 300
   caagggacca aagtggaaat caag 324
<210> 519
   <211> 381
   <212> DNA
   <213> Artificial Sequence
<220>
   <221> source
   <223> /note="Description of Artificial Sequence: Synthetic polynucleotide"
<400> 519
   caagtgcagc tggtcgaatc cgggggaggc ctggtgcagc ccggagggtc gctgaggctg 60
   agctgcgcgg cttccggctt caatgtgtcc tccaactaca tgacctgggt cagacaggcc 120
   cctggaaagg gactcgaatg ggtgtcggtg atctactccg gtggcgcaac ctactatgga 180
   gacagcgtga aggggcgctt cactgtgtcc cgcgacaact ccaagaacac tgtgtacctt 240
   cagatgaaca ggctcaccgc cgaggacacc gccgtgtact actgcgcgcg ggaccggctc 300
   tactgtggaa acaactgcta tctgtactac tactacggga tggacgtctg gggccagggc 360
   accctcgtga ctgtgtcgtc t 381
<210> 520
   <211> 765
   <212> DNA
   <213> Artificial Sequence
<220>
   <221> source
   <223> /note="Description of Artificial Sequence: Synthetic polynucleotide"
<400> 520
   caagtgcagc tggtcgaatc cgggggaggc ctggtgcagc ccggagggtc gctgaggctg 60
   agctgcgcgg cttccggctt caatgtgtcc tccaactaca tgacctgggt cagacaggcc 120
   cctggaaagg gactcgaatg ggtgtcggtg atctactccg gtggcgcaac ctactatgga 180
   gacagcgtga aggggcgctt cactgtgtcc cgcgacaact ccaagaacac tgtgtacctt 240
   cagatgaaca ggctcaccgc cgaggacacc gccgtgtact actgcgcgcg ggaccggctc 300
   tactgtggaa acaactgcta tctgtactac tactacggga tggacgtctg gggccagggc 360
   accctcgtga ctgtgtcgtc tggaggaggc ggtagcggtg gaggcggctc cggaggcgga 420
   ggctcgggag ggggaggcag cgatattcag gtcacccaat cgccgtcctc cctgagcgcc 480
   tccgtggggg atcgcgtgac gattacttgc cgggccagcc agagcatctc ctcgtacctg 540
   aactggtacc agcagaagcc gggaaaggcc cccaagctgc tgatctacgc tgcatcaagc 600
   ctgcagtccg gcgtgcctag ccggttttcc ggttccggtt cgggtaccga cttcacactg 660
   accatctcct cactgcaacc agaggatttc gccacctact actgtcagca gtcatactcc 720
   actccgcccc tgaccttcgg acaagggacc aaagtggaaa tcaag 765
<210> 521
   <211> 1467
   <212> DNA
   <213> Artificial Sequence
<220>
   <221> source
   <223> /note="Description of Artificial Sequence: Synthetic polynucleotide"
<400> 521
   caagtgcagc tggtcgaatc cgggggaggc ctggtgcagc ccggagggtc gctgaggctg      60
   agctgcgcgg cttccggctt caatgtgtcc tccaactaca tgacctgggt cagacaggcc     120
   cctggaaagg gactcgaatg ggtgtcggtg atctactccg gtggcgcaac ctactatgga     180
   gacagcgtga aggggcgctt cactgtgtcc cgcgacaact ccaagaacac tgtgtacctt     240
   cagatgaaca ggctcaccgc cgaggacacc gccgtgtact actgcgcgcg ggaccggctc     300
   tactgtggaa acaactgcta tctgtactac tactacggga tggacgtctg gggccagggc     360
   accctcgtga ctgtgtcgtc tggaggaggc ggtagcggtg gaggcggctc cggaggcgga     420
   ggctcgggag ggggaggcag cgatattcag gtcacccaat cgccgtcctc cctgagcgcc      480
   tccgtggggg atcgcgtgac gattacttgc cgggccagcc agagcatctc ctcgtacctg     540
   aactggtacc agcagaagcc gggaaaggcc cccaagctgc tgatctacgc tgcatcaagc     600
   ctgcagtccg gcgtgcctag ccggttttcc ggttccggtt cgggtaccga cttcacactg     660
   accatctcct cactgcaacc agaggatttc gccacctact actgtcagca gtcatactcc     720
   actccgcccc tgaccttcgg acaagggacc aaagtggaaa tcaagggcgg cggaggatcc     780
   ggatccgata agacccacac ctgtccaccc tgccctgccc ccgaactgct tggtggtccg     840
   tccgtgtttc tgttcccgcc caagcccaag gacaccctca tgatctcacg gactcctgaa     900
   gtgacctgtg tggtggtcgc tgtgtcccac gaggaccccg aagtcaagtt caattggtac     960
   gtggacggag tggaagtgca caacgctaag accaagcccc gcgaggaaca gtacaactcc    1020
   acttaccgcg tcgtgtcggt gctgaccgtg ctgcatcagg attggctgaa cggaaaggag    1080
   tacaagtgca aggtgtccaa caaggctctg gcggcaccca tcgaaaagac catcagcaag    1140
   gccaaagggc aacctagaga accacaagtc tacaccctgc ctccttgccg ggatgagctc    1200
   accaagaacc aggtgtccct gtggtgcctc gtgaagggct tctacccctc tgacatcgcg    1260
   gtggaatggg agtcaaacgg ccagccagag aacaactaca agacaacccc ccctgtcctg    1320
   gacagcgacg gctccttctt cctgtactcg aagctgactg tggataagag ccggtggcaa    1380
   cagggcaacg tgttctcatg ttcggtcatg cacgaggccc tgcataacca ctacactcag    1440
   aagtccctga gcctgtcccc tggaaag                                        1467
<210> 522
   <211> 482
   <212> PRT
   <213> Artificial Sequence
<220>
   <221> source
   <223> /note="Description of Artificial Sequence: Synthetic polypeptide"
<400> 522
   Gln Val Gln Leu Val Gln Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ser Met Asn Trp Val Arg Gln Ala Pro Gly Lys Gly Leu Glu Trp Val
           35                  40                  45
   Ser Tyr Ile Ser Ser Ser Ser Ser Thr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Leu Ser Val Arg Ala Ile Asp Ala Phe Asp Ile Trp Gly
               100                 105                 110
   Gln Gly Thr Met Val Thr Val Ser Ser Gly Gly Gly Gly Ser Gly Gly
           115                 120                 125
   Gly Gly Ser Gly Gly Gly Gly Ser Gly Gly Gly Gly Ser Asp Ile Val
       130                 135                 140
   Leu Thr Gln Ser Pro Ser Ser Leu Ser Ala Ser Val Gly Asp Arg Val
   145                 150                 155                 160
   Thr Ile Thr Cys Gln Ala Ser Gln Asp Ile Ser Asn Tyr Leu Asn Trp
                   165                 170                 175
   Tyr Gln Gln Lys Pro Gly Lys Ala Pro Lys Leu Leu Ile Tyr Asp Ala
               180                 185                 190
   Ser Asn Leu Glu Thr Gly Val Pro Ser Arg Phe Ser Gly Ser Gly Ser
           195                 200                 205
   Gly Thr Asp Phe Thr Phe Thr Ile Ser Ser Leu Gln Pro Glu Asp Phe
       210                 215                 220
   Ala Thr Tyr Tyr Cys Gln Gln Ala Tyr Ser Thr Pro Phe Thr Phe Gly
   225                 230                 235                 240
   Pro Gly Thr Lys Val Glu Ile Lys Gly Gly Gly Gly Ser Gly Ser Asp
                   245                 250                 255
   Lys Thr His Thr Cys Pro Pro Cys Pro Ala Pro Glu Leu Leu Gly Gly
               260                 265                 270
   Pro Ser Val Phe Leu Phe Pro Pro Lys Pro Lys Asp Thr Leu Met Ile
           275                 280                 285
   Ser Arg Thr Pro Glu Val Thr Cys Val Val Val Ala Val Ser His Glu
       290                 295                 300
   Asp Pro Glu Val Lys Phe Asn Trp Tyr Val Asp Gly Val Glu Val His
   305                 310                 315                 320
   Asn Ala Lys Thr Lys Pro Arg Glu Glu Gln Tyr Asn Ser Thr Tyr Arg
                   325                 330                 335
   Val Val Ser Val Leu Thr Val Leu His Gln Asp Trp Leu Asn Gly Lys
               340                 345                 350
   Glu Tyr Lys Cys Lys Val Ser Asn Lys Ala Leu Ala Ala Pro Ile Glu
           355                 360                 365
   Lys Thr Ile Ser Lys Ala Lys Gly Gln Pro Arg Glu Pro Gln Val Tyr
       370                 375                 380
   Thr Leu Pro Pro Cys Arg Asp Glu Leu Thr Lys Asn Gln Val Ser Leu
   385                 390                 395                 400
   Trp Cys Leu Val Lys Gly Phe Tyr Pro Ser Asp Ile Ala Val Glu Trp
                   405                 410                 415
   Glu Ser Asn Gly Gln Pro Glu Asn Asn Tyr Lys Thr Thr Pro Pro Val
               420                 425                 430
   Leu Asp Ser Asp Gly Ser Phe Phe Leu Tyr Ser Lys Leu Thr Val Asp
           435                 440                 445
   Lys Ser Arg Trp Gln Gln Gly Asn Val Phe Ser Cys Ser Val Met His
       450                 455                 460
   Glu Ala Leu His Asn His Tyr Thr Gln Lys Ser Leu Ser Leu Ser Pro
   465                 470                 475                 480
   Gly Lys
<210> 523
   <211> 321
   <212> DNA
   <213> Artificial Sequence
<220>
   <221> source
   <223> /note="Description of Artificial Sequence: Synthetic polynucleotide"
<400> 523
   gacatcgtgt tgacccagtc tccttcgtcc ctgagcgctt ccgtgggcga ccgcgtgacc 60
   atcacttgcc aagcctcaca agatatctcc aactacctca attggtacca gcagaagccg 120
   ggaaaggccc ccaagctgct catctacgac gcctccaacc tggaaactgg agtcccgtcg 180
   aggttttccg gaagcggcag cggaaccgac ttcaccttca ccattagcag cctgcagcca 240
   gaggattttg cgacctatta ctgccagcag gcttactcca cccccttcac cttcggacct 300
   ggcaccaagg tcgaaatcaa g 321
<210> 524
   <211> 363
   <212> DNA
   <213> Artificial Sequence
<220>
   <221> source
   <223> /note="Description of Artificial Sequence: Synthetic polynucleotide"
<400> 524
   caagtgcagc tggtgcagtc cggcggaggg ctggtgcagc cgggcggttc gctgagactg 60
   tcctgtgccg cgtcgggctt cacgttctcg tcatactcca tgaactgggt ccgccaggcc 120
   cccggaaaag gcttggaatg ggtgtcgtac atttcgtcct cctcctcaac catctactac 180
   gccgactcag tgaaggggcg gttcactatt tcccgggaca acgccaagaa cagcctgtac 240
   ctccaaatga actcactgcg ggccgaggac actgcggtgt actactgcgc ccgggacctg 300
   tccgtgagag caattgacgc attcgatatc tggggacagg gaaccatggt caccgtgtcc 360
   agc 363
<210> 525
   <211> 744
   <212> DNA
   <213> Artificial Sequence
<220>
   <221> source
   <223> /note="Description of Artificial Sequence: Synthetic polynucleotide"
<400> 525
   caagtgcagc tggtgcagtc cggcggaggg ctggtgcagc cgggcggttc gctgagactg 60
   tcctgtgccg cgtcgggctt cacgttctcg tcatactcca tgaactgggt ccgccaggcc 120
   cccggaaaag gcttggaatg ggtgtcgtac atttcgtcct cctcctcaac catctactac 180
   gccgactcag tgaaggggcg gttcactatt tcccgggaca acgccaagaa cagcctgtac 240
   ctccaaatga actcactgcg ggccgaggac actgcggtgt actactgcgc ccgggacctg 300
   tccgtgagag caattgacgc attcgatatc tggggacagg gaaccatggt caccgtgtcc 360
   agcggtggag gagggtcggg cggcggcggt tcaggcggtg gtggaagcgg cggggggggg 420
   tccgacatcg tgttgaccca gtctccttcg tccctgagcg cttccgtggg cgaccgcgtg 480
   accatcactt gccaagcctc acaagatatc tccaactacc tcaattggta ccagcagaag 540
   ccgggaaagg cccccaagct gctcatctac gacgcctcca acctggaaac tggagtcccg 600
   tcgaggtttt ccggaagcgg cagcggaacc gacttcacct tcaccattag cagcctgcag 660
   ccagaggatt ttgcgaccta ttactgccag caggcttact ccaccccctt caccttcgga 720
   cctggcacca aggtcgaaat caag 744
<210> 526
   <211> 1446
   <212> DNA
   <213> Artificial Sequence
<220>
   <221> source
   <223> /note="Description of Artificial Sequence: Synthetic polynucleotide"
<400> 526
   caagtgcagc tggtgcagtc cggcggaggg ctggtgcagc cgggcggttc gctgagactg        60
   tcctgtgccg cgtcgggctt cacgttctcg tcatactcca tgaactgggt ccgccaggcc       120
   cccggaaaag gcttggaatg ggtgtcgtac atttcgtcct cctcctcaac catctactac       180
   gccgactcag tgaaggggcg gttcactatt tcccgggaca acgccaagaa cagcctgtac       240
   ctccaaatga actcactgcg ggccgaggac actgcggtgt actactgcgc ccgggacctg       300
   tccgtgagag caattgacgc attcgatatc tggggacagg gaaccatggt caccgtgtcc       360
   agcggtggag gagggtcggg cggcggcggt tcaggcggtg gtggaagcgg cggggggggg       420
   tccgacatcg tgttgaccca gtctccttcg tccctgagcg cttccgtggg cgaccgcgtg       480
   accatcactt gccaagcctc acaagatatc tccaactacc tcaattggta ccagcagaag       540
   ccgggaaagg cccccaagct gctcatctac gacgcctcca acctggaaac tggagtcccg       600
   tcgaggtttt ccggaagcgg cagcggaacc gacttcacct tcaccattag cagcctgcag       660
   ccagaggatt ttgcgaccta ttactgccag caggcttact ccaccccctt caccttcgga       720
   cctggcacca aggtcgaaat caagggcgga gggggatccg gatccgataa gacccacacc       780
   tgtccaccct gccctgcccc cgaactgctt ggtggtccgt ccgtgtttct gttcccgccc       840
   aagcccaagg acaccctcat gatctcacgg actcctgaag tgacctgtgt ggtggtcgct       900
   gtgtcccacg aggaccccga agtcaagttc aattggtacg tggacggagt ggaagtgcac       960
   aacgctaaga ccaagccccg cgaggaacag tacaactcca cttaccgcgt cgtgtcggtg      1020
   ctgaccgtgc tgcatcagga ttggctgaac ggaaaggagt acaagtgcaa ggtgtccaac      1080
   aaggctctgg cggcacccat cgaaaagacc atcagcaagg ccaaagggca acctagagaa      1140
   ccacaagtct acaccctgcc tccttgccgg gatgagctca ccaagaacca ggtgtccctg      1200
   tggtgcctcg tgaagggctt ctacccctct gacatcgcgg tggaatggga gtcaaacggc      1260
   cagccagaga acaactacaa gacaaccccc cctgtcctgg acagcgacgg ctccttcttc      1320
   ctgtactcga agctgactgt ggataagagc cggtggcaac agggcaacgt gttctcatgt      1380
   tcggtcatgc acgaggccct gcataaccac tacactcaga agtccctgag cctgtcccct      1440
   ggaaag                                                                  1446
<210> 527
   <211> 473
   <212> PRT
   <213> Artificial Sequence
<220>
   <221> source
   <223> /note="Description of Artificial Sequence: Synthetic polypeptide"
<400> 527
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Trp Met Ser Trp Val Arg Gln Ala Pro Gly Lys Gly Leu Glu Trp Val
           35                  40                  45
   Ala Asn Ile Asn Glu Asp Gly Ser Ala Lys Phe Tyr Val Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Phe Cys
                   85                  90                  95
   Ala Arg Asp Leu Arg Ser Gly Arg Tyr Trp Gly Gln Gly Thr Leu Val
               100                 105                 110
   Thr Val Ser Ser Gly Gly Gly Gly Ser Gly Gly Gly Gly Ser Gly Gly
           115                 120                 125
   Gly Gly Ser Glu Ile Val Leu Thr Gln Ser Pro Gly Thr Leu Ser Leu
       130                 135                 140
   Ser Pro Gly Gly Arg Ala Thr Leu Ser Cys Arg Ala Ser Gln Ser Ile
   145                 150                 155                 160
   Ser Gly Ser Phe Leu Ala Trp Tyr Gln Gln Lys Pro Gly Gln Ala Pro
                   165                 170                 175
   Arg Leu Leu Ile Tyr Gly Ala Ser Ser Arg Ala Thr Gly Ile Pro Asp
   180 185 190
   Arg Phe Ser Gly Ser Gly Ser Gly Thr Asp Phe Thr Leu Thr Ile Ser
   195 200 205
   Arg Leu Glu Pro Glu Asp Phe Ala Val Tyr Tyr Cys Gln Gln Tyr Gly
   210 215 220
   Ser Ser Pro Pro Thr Phe Gly Leu Gly Thr Lys Leu Glu Ile Lys Gly
   225 230 235 240
   Gly Gly Gly Ser Gly Ser Asp Lys Thr His Thr Cys Pro Pro Cys Pro
   245 250 255
   Ala Pro Glu Leu Leu Gly Gly Pro Ser Val Phe Leu Phe Pro Pro Lys
   260 265 270
   Pro Lys Asp Thr Leu Met Ile Ser Arg Thr Pro Glu Val Thr Cys Val
   275 280 285
   Val Val Ala Val Ser His Glu Asp Pro Glu Val Lys Phe Asn Trp Tyr
   290 295 300
   Val Asp Gly Val Glu Val His Asn Ala Lys Thr Lys Pro Arg Glu Glu
   305 310 315 320
   Gln Tyr Asn Ser Thr Tyr Arg Val Val Ser Val Leu Thr Val Leu His
   325 330 335
   Gln Asp Trp Leu Asn Gly Lys Glu Tyr Lys Cys Lys Val Ser Asn Lys
   340 345 350
   Ala Leu Ala Ala Pro Ile Glu Lys Thr Ile Ser Lys Ala Lys Gly Gln
   355 360 365
   Pro Arg Glu Pro Gln Val Tyr Thr Leu Pro Pro Cys Arg Asp Glu Leu
   370 375 380
   Thr Lys Asn Gln Val Ser Leu Trp Cys Leu Val Lys Gly Phe Tyr Pro
   385 390 395 400
   Ser Asp Ile Ala Val Glu Trp Glu Ser Asn Gly Gln Pro Glu Asn Asn
   405 410 415
   Tyr Lys Thr Thr Pro Pro Val Leu Asp Ser Asp Gly Ser Phe Phe Leu
   420 425 430
   Tyr Ser Lys Leu Thr Val Asp Lys Ser Arg Trp Gln Gln Gly Asn Val
   435 440 445
   Phe Ser Cys Ser Val Met His Glu Ala Leu His Asn His Tyr Thr Gln
   450 455 460
   Lys Ser Leu Ser Leu Ser Pro Gly Lys
   465 470
<210> 528
   <211> 324
   <212> DNA
   <213> Artificial Sequence
<220>
   <221> source
   <223> /note="Description of Artificial Sequence: Synthetic polynucleotide"
<400> 528
   gaaattgtgc tgacccaatc gcccggaact ctgtccctgt cccccggtgg acgcgccact 60
   ctctcttgcc gggcctcaca gtcgatctcg ggcagctttc tcgcctggta ccagcagaag 120
   ccgggacagg cgcctcgcct gctgatctac ggagcgtcca gcagagccac cggaatccca 180
   gacagattct ccggctcggg ctccggtacc gactttacgc tgactattag ccggctggag 240
   ccggaggact tcgccgtgta ctactgtcag cagtacggca gctcaccgcc taccttcgga 300
   ctcgggacaa agctggaaat caag 324
<210> 529
   <211> 345
   <212> DNA
   <213> Artificial Sequence
<220>
   <221> source
   <223> /note="Description of Artificial Sequence: Synthetic polynucleotide"
<400> 529
   caagtgcagc tggtcgaatc cggcggaggg ctggtgcagc cgggagggtc actccggctg 60
   tcctgcgccg catcaggatt caccttctcc tcctactgga tgtcctgggt ccgccaggct 120
   cccgggaagg gtctggaatg ggtggccaac atcaacgagg acggctccgc caagttctac 180
   gtggatagcg tgaaaggaag gttcaccatt tcccgggaca acgccaagaa cagcctctat 240
   ctgcaaatga atagcctgag ggcagaagat accgcggtgt acttctgcgc tcgggacctg 300
   agatccggcc gctactgggg ccaggggacc ctggtcaccg tgtcc 345
<210> 530
   <211> 717
   <212> DNA
   <213> Artificial Sequence
<220>
   <221> source
   <223> /note="Description of Artificial Sequence: Synthetic polynucleotide"
<400> 530
   caagtgcagc tggtcgaatc cggcggaggg ctggtgcagc cgggagggtc actccggctg 60
   tcctgcgccg catcaggatt caccttctcc tcctactgga tgtcctgggt ccgccaggct 120
   cccgggaagg gtctggaatg ggtggccaac atcaacgagg acggctccgc caagttctac 180
   gtggatagcg tgaaaggaag gttcaccatt tcccgggaca acgccaagaa cagcctctat 240
   ctgcaaatga atagcctgag ggcagaagat accgcggtgt acttctgcgc tcgggacctg 300
   agatccggcc gctactgggg ccaggggacc ctggtcaccg tgtcctcggg agggggcggc 360
   tccggtggtg gagggagcgg cggaggaggg tccgaaattg tgctgaccca atcgcccgga 420
   actctgtccc tgtcccccgg tggacgcgcc actctctctt gccgggcctc acagtcgatc 480
   tcgggcagct ttctcgcctg gtaccagcag aagccgggac aggcgcctcg cctgctgatc 540
   tacggagcgt ccagcagagc caccggaatc ccagacagat tctccggctc gggctccggt 600
   accgacttta cgctgactat tagccggctg gagccggagg acttcgccgt gtactactgt 660
   cagcagtacg gcagctcacc gcctaccttc ggactcggga caaagctgga aatcaag 717
<210> 531
   <211> 1419
   <212> DNA
   <213> Artificial Sequence
<220>
   <221> source
   <223> /note="Description of Artificial Sequence: Synthetic polynucleotide"
<400> 531
   caagtgcagc tggtcgaatc cggcggaggg ctggtgcagc cgggagggtc actccggctg 60
   tcctgcgccg catcaggatt caccttctcc tcctactgga tgtcctgggt ccgccaggct 120
   cccgggaagg gtctggaatg ggtggccaac atcaacgagg acggctccgc caagttctac 180
   gtggatagcg tgaaaggaag gttcaccatt tcccgggaca acgccaagaa cagcctctat 240
   ctgcaaatga atagcctgag ggcagaagat accgcggtgt acttctgcgc tcgggacctg 300
   agatccggcc gctactgggg ccaggggacc ctggtcaccg tgtcctcggg agggggcggc 360
   tccggtggtg gagggagcgg cggaggaggg tccgaaattg tgctgaccca atcgcccgga 420
   actctgtccc tgtcccccgg tggacgcgcc actctctctt gccgggcctc acagtcgatc 480
   tcgggcagct ttctcgcctg gtaccagcag aagccgggac aggcgcctcg cctgctgatc 540
   tacggagcgt ccagcagagc caccggaatc ccagacagat tctccggctc gggctccggt 600
   accgacttta cgctgactat tagccggctg gagccggagg acttcgccgt gtactactgt 660
   cagcagtacg gcagctcacc gcctaccttc ggactcggga caaagctgga aatcaaggga 720
   ggcggcggat ccggatccga taagacccac acctgtccac cctgccctgc ccccgaactg 780
   cttggtggtc cgtccgtgtt tctgttcccg cccaagccca aggacaccct catgatctca 840
   cggactcctg aagtgacctg tgtggtggtc gctgtgtccc acgaggaccc cgaagtcaag 900
   ttcaattggt acgtggacgg agtggaagtg cacaacgcta agaccaagcc ccgcgaggaa 960
   cagtacaact ccacttaccg cgtcgtgtcg gtgctgaccg tgctgcatca ggattggctg 1020
   aacggaaagg agtacaagtg caaggtgtcc aacaaggctc tggcggcacc catcgaaaag 1080
   accatcagca aggccaaagg gcaacctaga gaaccacaag tctacaccct gcctccttgc 1140
   cgggatgagc tcaccaagaa ccaggtgtcc ctgtggtgcc tcgtgaaggg cttctacccc 1200
   tctgacatcg cggtggaatg ggagtcaaac ggccagccag agaacaacta caagacaacc 1260
   ccccctgtcc tggacagcga cggctccttc ttcctgtact cgaagctgac tgtggataag 1320
   agccggtggc aacagggcaa cgtgttctca tgttcggtca tgcacgaggc cctgcataac 1380
   cactacactc agaagtccct gagcctgtcc cctggaaag 1419
<210> 532
   <211> 480
   <212> PRT
   <213> Artificial Sequence
<220>
   <221> source
   <223> /note="Description of Artificial Sequence: Synthetic polypeptide"
<400> 532
   Gln Val Gln Leu Val Gln Ser Gly Ala Glu Val Lys Glu Pro Gly Ala
   1               5                   10                  15
   Ser Val Lys Val Ser Cys Lys Ala Pro Ala Asn Thr Phe Ser Asp His
               20                  25                  30
   Val Met His Trp Val Arg Gln Ala Pro Gly Gln Arg Phe Glu Trp Met
           35                  40                  45
   Gly Tyr Ile His Ala Ala Asn Gly Gly Thr His Tyr Ser Gln Lys Phe
       50                  55                  60
   Gln Asp Arg Val Thr Ile Thr Arg Asp Thr Ser Ala Asn Thr Val Tyr
   65                  70                  75                  80
   Met Asp Leu Ser Ser Leu Arg Ser Glu Asp Thr Ala Val Tyr Tyr Cys
                   85                  90                  95
   Ala Arg Gly Gly Tyr Asn Ser Asp Ala Phe Asp Ile Trp Gly Gln Gly
               100                 105                 110
   Thr Met Val Thr Val Ser Ser Gly Gly Gly Gly Ser Gly Gly Gly Gly
           115                 120                 125
   Ser Gly Gly Gly Gly Ser Gly Gly Gly Gly Ser Asp Ile Val Met Thr
       130                 135                 140
   Gln Ser Pro Ser Ser Val Ser Ala Ser Val Gly Asp Arg Val Thr Ile
   145                 150                 155                 160
   Thr Cys Arg Ala Ser Gln Asp Ile Ser Ser Trp Leu Ala Trp Tyr Gln
                   165                 170                 175
   Gln Lys Pro Gly Lys Ala Pro Lys Leu Leu Ile Tyr Ala Ala Ser Ser
               180                 185                 190
   Leu Gln Ser Gly Val Pro Ser Arg Phe Asn Gly Ser Gly Ser Gly Thr
           195                 200                 205
   Asp Phe Thr Leu Thr Ile Ser Ser Leu Gln Pro Glu Asp Phe Ala Thr
       210                 215                 220
   Tyr Tyr Cys Gln Gln Ser Tyr Ser Thr Pro Leu Thr Phe Gly Gly Gly
   225                 230                 235                 240
   Thr Lys Val Glu Ile Lys Gly Gly Gly Gly Ser Gly Ser Asp Lys Thr
                   245                 250                 255
   His Thr Cys Pro Pro Cys Pro Ala Pro Glu Leu Leu Gly Gly Pro Ser
               260                 265                 270
   Val Phe Leu Phe Pro Pro Lys Pro Lys Asp Thr Leu Met Ile Ser Arg
           275                 280                 285
   Thr Pro Glu Val Thr Cys Val Val Val Ala Val Ser His Glu Asp Pro
       290                 295                 300
   Glu Val Lys Phe Asn Trp Tyr Val Asp Gly Val Glu Val His Asn Ala
   305                 310                 315                 320
   Lys Thr Lys Pro Arg Glu Glu Gln Tyr Asn Ser Thr Tyr Arg Val Val
                   325                 330                 335
   Ser Val Leu Thr Val Leu His Gln Asp Trp Leu Asn Gly Lys Glu Tyr
   340 345 350
   Lys Cys Lys Val Ser Asn Lys Ala Leu Ala Ala Pro Ile Glu Lys Thr
   355 360 365
   Ile Ser Lys Ala Lys Gly Gln Pro Arg Glu Pro Gln Val Tyr Thr Leu
   370 375 380
   Pro Pro Cys Arg Asp Glu Leu Thr Lys Asn Gln Val Ser Leu Trp Cys
   385 390 395 400
   Leu Val Lys Gly Phe Tyr Pro Ser Asp Ile Ala Val Glu Trp Glu Ser
   405 410 415
   Asn Gly Gln Pro Glu Asn Asn Tyr Lys Thr Thr Pro Pro Val Leu Asp
   420 425 430
   Ser Asp Gly Ser Phe Phe Leu Tyr Ser Lys Leu Thr Val Asp Lys Ser
   435 440 445
   Arg Trp Gln Gln Gly Asn Val Phe Ser Cys Ser Val Met His Glu Ala
   450 455 460
   Leu His Asn His Tyr Thr Gln Lys Ser Leu Ser Leu Ser Pro Gly Lys
   465 470 475 480
<210> 533
   <211> 321
   <212> DNA
   <213> Artificial Sequence
<220>
   <221> source
   <223> /note="Description of Artificial Sequence: Synthetic polynucleotide"
<400> 533
   gatatcgtga tgactcagtc cccctcgtcc gtgagcgcgt ccgtgggcga cagagtgacc 60
   attacgtgcc gcgccagcca agacatttct tcctggctcg cctggtacca gcagaagcct 120
   ggaaaggctc cgaagctgct gatctacgcc gcctcatcgc tccaatccgg agtgccatcg 180
   cggttcaatg gctcggggtc cggaactgac tttaccctga ctattagcag cctgcagcct 240
   gaggacttcg ctacctatta ctgccaacag tcctactcca ccccgctgac cttcgggggt 300
   ggtaccaagg tcgaaatcaa g 321
<210> 534
   <211> 357
   <212> DNA
   <213> Artificial Sequence
<220>
   <221> source
   <223> /note="Description of Artificial Sequence: Synthetic polynucleotide"
<400> 534
   caagtgcagc tggtgcagtc cggagcagaa gtcaaggaac cgggagccag cgtgaaggtg      60
   tcctgtaaag cccccgcaaa cactttcagc gatcacgtca tgcactgggt ccggcaggcc     120
   cccggccaac gcttcgaatg gatggggtac atccatgctg ccaacggcgg aacccactac     180
   agccagaagt ttcaggaccg cgtgacgatc accagggaca catccgcgaa cactgtgtac     240
   atggacctgt catccctgag atcggaggac accgcagtgt actactgcgc ccggggggga     300
   tacaactccg atgcgttcga catctggggc cagggaacca tggtcaccgt gtcatcc        357
<210> 535
   <211> 738
   <212> DNA
   <213> Artificial Sequence
<220>
   <221> source
   <223> /note="Description of Artificial Sequence: Synthetic polynucleotide"
<400> 535
   caagtgcagc tggtgcagtc cggagcagaa gtcaaggaac cgggagccag cgtgaaggtg      60
   tcctgtaaag cccccgcaaa cactttcagc gatcacgtca tgcactgggt ccggcaggcc     120
   cccggccaac gcttcgaatg gatggggtac atccatgctg ccaacggcgg aacccactac     180
   agccagaagt ttcaggaccg cgtgacgatc accagggaca catccgcgaa cactgtgtac     240
   atggacctgt catccctgag atcggaggac accgcagtgt actactgcgc ccggggggga     300
   tacaactccg atgcgttcga catctggggc cagggaacca tggtcaccgt gtcatccggt     360
   ggaggcggct cgggtggcgg aggatcagga ggaggaggca gcgggggcgg aggttccgat     420
   atcgtgatga ctcagtcccc ctcgtccgtg agcgcgtccg tgggcgacag agtgaccatt     480
   acgtgccgcg ccagccaaga catttcttcc tggctcgcct ggtaccagca gaagcctgga     540
   aaggctccga agctgctgat ctacgccgcc tcatcgctcc aatccggagt gccatcgcgg     600
   ttcaatggct cggggtccgg aactgacttt accctgacta ttagcagcct gcagcctgag     660
   gacttcgcta cctattactg ccaacagtcc tactccaccc cgctgacctt cgggggtggt     720
   accaaggtcg aaatcaag                                                   738
<210> 536
   <211> 1440
   <212> DNA
   <213> Artificial Sequence
<220>
   <221> source
   <223> /note="Description of Artificial Sequence: Synthetic polynucleotide"
<400> 536
   caagtgcagc tggtgcagtc cggagcagaa gtcaaggaac cgggagccag cgtgaaggtg 60
   tcctgtaaag cccccgcaaa cactttcagc gatcacgtca tgcactgggt ccggcaggcc 120
   cccggccaac gcttcgaatg gatggggtac atccatgctg ccaacggcgg aacccactac 180
   agccagaagt ttcaggaccg cgtgacgatc accagggaca catccgcgaa cactgtgtac 240
   atggacctgt catccctgag atcggaggac accgcagtgt actactgcgc ccggggggga 300
   tacaactccg atgcgttcga catctggggc cagggaacca tggtcaccgt gtcatccggt 360
   ggaggcggct cgggtggcgg aggatcagga ggaggaggca gcgggggcgg aggttccgat 420
   atcgtgatga ctcagtcccc ctcgtccgtg agcgcgtccg tgggcgacag agtgaccatt 480
   acgtgccgcg ccagccaaga catttcttcc tggctcgcct ggtaccagca gaagcctgga 540
   aaggctccga agctgctgat ctacgccgcc tcatcgctcc aatccggagt gccatcgcgg 600
   ttcaatggct cggggtccgg aactgacttt accctgacta ttagcagcct gcagcctgag 660
   gacttcgcta cctattactg ccaacagtcc tactccaccc cgctgacctt cgggggtggt 720
   accaaggtcg aaatcaaggg agggggcgga tccggatccg ataagaccca cacctgtcca 780
   ccctgccctg cccccgaact gcttggtggt ccgtccgtgt ttctgttccc gcccaagccc 840
   aaggacaccc tcatgatctc acggactcct gaagtgacct gtgtggtggt cgctgtgtcc 900
   cacgaggacc ccgaagtcaa gttcaattgg tacgtggacg gagtggaagt gcacaacgct 960
   aagaccaagc cccgcgagga acagtacaac tccacttacc gcgtcgtgtc ggtgctgacc 1020
   gtgctgcatc aggattggct gaacggaaag gagtacaagt gcaaggtgtc caacaaggct 1080
   ctggcggcac ccatcgaaaa gaccatcagc aaggccaaag ggcaacctag agaaccacaa 1140
   gtctacaccc tgcctccttg ccgggatgag ctcaccaaga accaggtgtc cctgtggtgc 1200
   ctcgtgaagg gcttctaccc ctctgacatc gcggtggaat gggagtcaaa cggccagcca 1260
   gagaacaact acaagacaac cccccctgtc ctggacagcg acggctcctt cttcctgtac 1320
   tcgaagctga ctgtggataa gagccggtgg caacagggca acgtgttctc atgttcggtc 1380
   atgcacgagg ccctgcataa ccactacact cagaagtccc tgagcctgtc ccctggaaag 1440
<210> 537
   <211> 489
   <212> PRT
   <213> Artificial Sequence
<220>
   <221> source
   <223> /note="Description of Artificial Sequence: Synthetic polypeptide"
<400> 537
   Glu Val Gln Leu Val Glu Ser Gly Gly Gly Leu Val Lys Pro Gly Gly
   1 5 10 15
   Ser Leu Arg Leu Ser Cys Ala Ala Ser Gly Phe Thr Phe Ser Ser Tyr
   20 25 30
   Ser Met Asn Trp Val Arg Gln Ala Pro Gly Lys Gly Leu Glu Trp Val
   35 40 45
   Ser Ser Ile Ser Ser Ser Ser Ser Tyr Ile Tyr Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Asp Pro Ser Ser Ser Gly Ser Tyr Tyr Met Glu Asp Ser Tyr
   100 105 110
   Tyr Tyr Gly Met Asp Val Trp Gly Gln Gly Thr Thr Val Thr Val Ser
   115 120 125
   Ser Gly Gly Gly Gly Ser Gly Gly Gly Gly Ser Gly Gly Gly Gly Ser
   130 135 140
   Asn Phe Met Leu Thr Gln Pro His Ser Val Ser Glu Ser Pro Gly Lys
   145 150 155 160
   Thr Val Thr Ile Ser Cys Thr Gly Ser Ser Gly Ser Ile Ala Ser Asn
   165 170 175
   Tyr Val Gln Trp Tyr Gln Gln Arg Pro Gly Ser Ala Pro Thr Thr Val
   180 185 190
   Ile Tyr Glu Asp Asn Gln Arg Pro Ser Gly Val Pro Asp Arg Phe Ser
   195 200 205
   Gly Ser Ile Asp Ser Ser Ser Asn Ser Ala Ser Leu Thr Ile Ser Gly
   210 215 220
   Leu Lys Thr Glu Asp Glu Ala Asp Tyr Tyr Cys Gln Ser Tyr Asp Ser
   225                 230                 235                 240
   Ser Asn Gln Val Val Phe Gly Gly Gly Thr Lys Leu Thr Val Leu Gly
                   245                 250                 255
   Gly Gly Gly Ser Gly Ser Asp Lys Thr His Thr Cys Pro Pro Cys Pro
               260                 265                 270
   Ala Pro Glu Leu Leu Gly Gly Pro Ser Val Phe Leu Phe Pro Pro Lys
           275                 280                 285
   Pro Lys Asp Thr Leu Met Ile Ser Arg Thr Pro Glu Val Thr Cys Val
       290                 295                 300
   Val Val Ala Val Ser His Glu Asp Pro Glu Val Lys Phe Asn Trp Tyr
   305                 310                 315                 320
   Val Asp Gly Val Glu Val His Asn Ala Lys Thr Lys Pro Arg Glu Glu
                   325                 330                 335
   Gln Tyr Asn Ser Thr Tyr Arg Val Val Ser Val Leu Thr Val Leu His
               340                 345                 350
   Gln Asp Trp Leu Asn Gly Lys Glu Tyr Lys Cys Lys Val Ser Asn Lys
           355                 360                 365
   Ala Leu Ala Ala Pro Ile Glu Lys Thr Ile Ser Lys Ala Lys Gly Gln
       370                 375                 380
   Pro Arg Glu Pro Gln Val Tyr Thr Leu Pro Pro Cys Arg Asp Glu Leu
   385                 390                 395                 400
   Thr Lys Asn Gln Val Ser Leu Trp Cys Leu Val Lys Gly Phe Tyr Pro
                   405                 410                 415
   Ser Asp Ile Ala Val Glu Trp Glu Ser Asn Gly Gln Pro Glu Asn Asn
               420                 425                 430
   Tyr Lys Thr Thr Pro Pro Val Leu Asp Ser Asp Gly Ser Phe Phe Leu
           435                 440                 445
   Tyr Ser Lys Leu Thr Val Asp Lys Ser Arg Trp Gln Gln Gly Asn Val
       450                 455                 460
   Phe Ser Cys Ser Val Met His Glu Ala Leu His Asn His Tyr Thr Gln
   465                 470                 475                 480
   Lys Ser Leu Ser Leu Ser Pro Gly Lys
   485
<210> 538
   <211> 333
   <212> DNA
   <213> Artificial Sequence
<220>
   <221> source
   <223> /note="Description of Artificial Sequence: Synthetic polynucleotide"
<400> 538
   aacttcatgc tgacccagcc tcactccgtg tccgaatccc cggggaaaac cgtgactatc 60
   agctgcaccg gctccagcgg ctcgatcgcg tcgaactacg tgcagtggta tcaacagcgc 120
   cccggttccg cccccaccac cgtgatctac gaagataacc agcggccttc cggagtcccg 180
   gatagattct ccggttccat tgactcctca tccaactccg cctcgctcac tattagcggc 240
   ctcaagacgg aggacgaagc cgattactac tgtcagtcct acgactcgag caatcaagtg 300
   gtgttcggag ggggcaccaa gctgaccgtg ctg 333
<210> 539
   <211> 387
   <212> DNA
   <213> Artificial Sequence
<220>
   <221> source
   <223> /note="Description of Artificial Sequence: Synthetic polynucleotide"
<400> 539
   gaagtgcagc tcgtggagtc gggcggtgga ttggtcaagc cgggcggaag cctgcggctg 60
   tcatgcgccg cttctgggtt caccttctcc tcctactcca tgaactgggt cagacaggcg 120
   cccggaaagg gactggaatg ggtgtcctca atctcgtcgt cctcgtccta catctattac 180
   gccgactcag tgaaggggcg ctttactatt tcgcgggaca acgctaagaa ctccctgtac 240
   ctccaaatga acagcctgcg ggcggaggac accgccgtgt actactgcgc aagggaccca 300
   agcagctccg gctcatacta catggaggac tcctactact acggaatgga cgtctgggga 360
   cagggaacca ctgtgaccgt gtcatcc 387
<210> 540
   <211> 765
   <212> DNA
   <213> Artificial Sequence
<220>
   <221> source
   <223> /note="Description of Artificial Sequence: Synthetic polynucleotide"
<400> 540
   gaagtgcagc tcgtggagtc gggcggtgga ttggtcaagc cgggcggaag cctgcggctg 60
   tcatgcgccg cttctgggtt caccttctcc tcctactcca tgaactgggt cagacaggcg 120
   cccggaaagg gactggaatg ggtgtcctca atctcgtcgt cctcgtccta catctattac 180
   gccgactcag tgaaggggcg ctttactatt tcgcgggaca acgctaagaa ctccctgtac 240
   ctccaaatga acagcctgcg ggcggaggac accgccgtgt actactgcgc aagggaccca 300
   agcagctccg gctcatacta catggaggac tcctactact acggaatgga cgtctgggga 360
   cagggaacca ctgtgaccgt gtcatccggc ggcggtggta gcgggggcgg aggaagcggg 420
   gggggaggct ccaacttcat gctgacccag cctcactccg tgtccgaatc cccggggaaa 480
   accgtgacta tcagctgcac cggctccagc ggctcgatcg cgtcgaacta cgtgcagtgg 540
   tatcaacagc gccccggttc cgcccccacc accgtgatct acgaagataa ccagcggcct 600
   tccggagtcc cggatagatt ctccggttcc attgactcct catccaactc cgcctcgctc 660
   actattagcg gcctcaagac ggaggacgaa gccgattact actgtcagtc ctacgactcg 720
   agcaatcaag tggtgttcgg agggggcacc aagctgaccg tgctg 765
<210> 541
   <211> 1467
   <212> DNA
   <213> Artificial Sequence
<220>
   <221> source
   <223> /note="Description of Artificial Sequence: Synthetic polynucleotide"
<400> 541
   gaagtgcagc tcgtggagtc gggcggtgga ttggtcaagc cgggcggaag cctgcggctg 60
   tcatgcgccg cttctgggtt caccttctcc tcctactcca tgaactgggt cagacaggcg 120
   cccggaaagg gactggaatg ggtgtcctca atctcgtcgt cctcgtccta catctattac 180
   gccgactcag tgaaggggcg ctttactatt tcgcgggaca acgctaagaa ctccctgtac 240
   ctccaaatga acagcctgcg ggcggaggac accgccgtgt actactgcgc aagggaccca 300
   agcagctccg gctcatacta catggaggac tcctactact acggaatgga cgtctgggga 360
   cagggaacca ctgtgaccgt gtcatccggc ggcggtggta gcgggggcgg aggaagcggg 420
   gggggaggct ccaacttcat gctgacccag cctcactccg tgtccgaatc cccggggaaa 480
   accgtgacta tcagctgcac cggctccagc ggctcgatcg cgtcgaacta cgtgcagtgg 540
   tatcaacagc gccccggttc cgcccccacc accgtgatct acgaagataa ccagcggcct 600
   tccggagtcc cggatagatt ctccggttcc attgactcct catccaactc cgcctcgctc 660
   actattagcg gcctcaagac ggaggacgaa gccgattact actgtcagtc ctacgactcg 720
   agcaatcaag tggtgttcgg agggggcacc aagctgaccg tgctgggcgg tggaggatcc 780
   ggatccgata agacccacac ctgtccaccc tgccctgccc ccgaactgct tggtggtccg 840
   tccgtgtttc tgttcccgcc caagcccaag gacaccctca tgatctcacg gactcctgaa 900
   gtgacctgtg tggtggtcgc tgtgtcccac gaggaccccg aagtcaagtt caattggtac 960
   gtggacggag tggaagtgca caacgctaag accaagcccc gcgaggaaca gtacaactcc 1020
   acttaccgcg tcgtgtcggt gctgaccgtg ctgcatcagg attggctgaa cggaaaggag 1080
   tacaagtgca aggtgtccaa caaggctctg gcggcaccca tcgaaaagac catcagcaag 1140
   gccaaagggc aacctagaga accacaagtc tacaccctgc ctccttgccg ggatgagctc 1200
   accaagaacc aggtgtccct gtggtgcctc gtgaagggct tctacccctc tgacatcgcg 1260
   gtggaatggg agtcaaacgg ccagccagag aacaactaca agacaacccc ccctgtcctg 1320
   gacagcgacg gctccttctt cctgtactcg aagctgactg tggataagag ccggtggcaa 1380
   cagggcaacg tgttctcatg ttcggtcatg cacgaggccc tgcataacca ctacactcag 1440
   aagtccctga gcctgtcccc tggaaag 1467
<210> 542
   <211> 481
   <212> PRT
   <213> Artificial Sequence
<220>
   <221> source
   <223> /note="Description of Artificial Sequence: Synthetic polypeptide"
<400> 542
   Gln Val Gln Leu Val Gln Ser Gly Ala Glu Val Lys Lys Ser Gly Ala
   1               5                   10                  15
   Ser Val Lys Val Ser Cys Lys Ala Ser Gly Tyr Pro Phe Thr Gly Tyr
               20                  25                  30
   Tyr Ile Gln Trp Val Arg Gln Ala Pro Gly Gln Gly Leu Glu Trp Met
           35                  40                  45
   Gly Trp Ile Asp Pro Asn Ser Gly Asn Thr Gly Tyr Ala Gln Lys Phe
       50                  55                  60
   Gln Gly Arg Val Thr Met Thr Arg Asn Thr Ser Ile Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Ser Asp Ser Tyr Gly Tyr Tyr Tyr Gly Met Asp Val Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Gly Gly Gly Gly Ser Asp Ile Gln Met
       130                 135                 140
   Thr Gln Ser Pro Ser Ser Leu Ser Ala Ser Val Gly Asp Arg Val Thr
   145                 150                 155                 160
   Phe Thr Cys Arg Ala Ser Gln Gly Ile Ser Ser Ala Leu Ala Trp Tyr
                   165                 170                 175
   Gln Gln Lys Pro Gly Lys Pro Pro Lys Leu Leu Ile Tyr Asp Ala Ser
               180                 185                 190
   Ser Leu Glu Ser Gly Val Pro Ser Arg Phe Ser Gly Ser Gly Ser Gly
           195                 200                 205
   Thr Asp Phe Thr Leu Thr Ile Ser Ser Leu Gln Pro Glu Asp Phe Ala
       210                 215                 220
   Thr Tyr Tyr Cys Gln Gln Phe Asn Asn Tyr Pro Leu Thr Phe Gly Gly
   225                 230                 235                 240
   Gly Thr Lys Val Glu Ile Lys Gly Gly Gly Gly Ser Gly Ser Asp Lys
                   245                 250                 255
   Thr His Thr Cys Pro Pro Cys Pro Ala Pro Glu Leu Leu Gly Gly Pro
               260                 265                 270
   Ser Val Phe Leu Phe Pro Pro Lys Pro Lys Asp Thr Leu Met Ile Ser
           275                 280                 285
   Arg Thr Pro Glu Val Thr Cys Val Val Val Ala Val Ser His Glu Asp
       290                 295                 300
   Pro Glu Val Lys Phe Asn Trp Tyr Val Asp Gly Val Glu Val His Asn
   305                 310                 315                 320
   Ala Lys Thr Lys Pro Arg Glu Glu Gln Tyr Asn Ser Thr Tyr Arg Val
                   325                 330                 335
   Val Ser Val Leu Thr Val Leu His Gln Asp Trp Leu Asn Gly Lys Glu
               340                 345                 350
   Tyr Lys Cys Lys Val Ser Asn Lys Ala Leu Ala Ala Pro Ile Glu Lys
   355 360 365
   Thr Ile Ser Lys Ala Lys Gly Gln Pro Arg Glu Pro Gln Val Tyr Thr
   370 375 380
   Leu Pro Pro Cys Arg Asp Glu Leu Thr Lys Asn Gln Val Ser Leu Trp
   385 390 395 400
   Cys Leu Val Lys Gly Phe Tyr Pro Ser Asp Ile Ala Val Glu Trp Glu
   405 410 415
   Ser Asn Gly Gln Pro Glu Asn Asn Tyr Lys Thr Thr Pro Pro Val Leu
   420 425 430
   Asp Ser Asp Gly Ser Phe Phe Leu Tyr Ser Lys Leu Thr Val Asp Lys
   435 440 445
   Ser Arg Trp Gln Gln Gly Asn Val Phe Ser Cys Ser Val Met His Glu
   450 455 460
   Ala Leu His Asn His Tyr Thr Gln Lys Ser Leu Ser Leu Ser Pro Gly
   465 470 475 480
   Lys
<210> 543
   <211> 321
   <212> DNA
   <213> Artificial Sequence
<220>
   <221> source
   <223> /note="Description of Artificial Sequence: Synthetic polynucleotide"
<400> 543
   gacattcaga tgacccagtc accatcctcc ctgtccgcct ccgtcgggga tagagtgacc 60
   ttcacctgtc gggcctccca aggaatctca agcgctctgg cctggtacca gcagaagcct 120
   ggaaagccgc ccaagctgtt gatctacgat gcctcgagcc tggaatccgg cgtgccgagc 180
   cggttcagcg gtagcggctc gggaaccgac ttcacgctca ccatctcgtc cctgcaaccg 240
   gaggacttcg cgacttacta ctgccagcaa ttcaacaact accctctgac ctttggtggt 300
   ggtactaagg tcgagatcaa g 321
<210> 544
   <211> 357
   <212> DNA
   <213> Artificial Sequence
<220>
   <221> source
   <223> /note="Description of Artificial Sequence: Synthetic polynucleotide"
<400> 544
   caagtgcagc tggtgcagtc cggggccgaa gtgaaaaagt ccggtgcatc cgtgaaagtg       60
   tcgtgcaagg cctcaggcta tcccttcacc ggatactaca ttcagtgggt ccgccaggct      120
   ccgggacagg gcctcgaatg gatgggctgg atcgatccca actccggcaa tactggctac      180
   gcgcagaagt tccagggacg cgtgaccatg actcggaaca cctccatttc caccgcctac      240
   atggaactgt cgtcactgag gtccgaggac accgccgtgt attactgcgc gtcggacagc      300
   tacggatact actacgggat ggacgtgtgg ggacagggaa ctctggtcac cgtgtcg         357
<210> 545
   <211> 741
   <212> DNA
   <213> Artificial Sequence
<220>
   <221> source
   <223> /note="Description of Artificial Sequence: Synthetic polynucleotide"
<400> 545
   caagtgcagc tggtgcagtc cggggccgaa gtgaaaaagt ccggtgcatc cgtgaaagtg       60
   tcgtgcaagg cctcaggcta tcccttcacc ggatactaca ttcagtgggt ccgccaggct      120
   ccgggacagg gcctcgaatg gatgggctgg atcgatccca actccggcaa tactggctac      180
   gcgcagaagt tccagggacg cgtgaccatg actcggaaca cctccatttc caccgcctac      240
   atggaactgt cgtcactgag gtccgaggac accgccgtgt attactgcgc gtcggacagc      300
   tacggatact actacgggat ggacgtgtgg ggacagggaa ctctggtcac cgtgtcgtcc      360
   ggaggcggag gcagcggcgg gggcggctcc gggggagggg ggtcgggcgg aggcggaagc      420
   gacattcaga tgacccagtc accatcctcc ctgtccgcct ccgtcgggga tagagtgacc      480
   ttcacctgtc gggcctccca aggaatctca agcgctctgg cctggtacca gcagaagcct      540
   ggaaagccgc ccaagctgtt gatctacgat gcctcgagcc tggaatccgg cgtgccgagc      600
   cggttcagcg gtagcggctc gggaaccgac ttcacgctca ccatctcgtc cctgcaaccg      660
   gaggacttcg cgacttacta ctgccagcaa ttcaacaact accctctgac ctttggtggt      720
   ggtactaagg tcgagatcaa g                                                741
<210> 546
   <211> 1443
   <212> **DNA**
   <213> Artificial Sequence
<220>
   <221> source
   <223> /note="Description of Artificial Sequence: Synthetic polynucleotide"
<400> 546
   caagtgcagc tggtgcagtc cggggccgaa gtgaaaaagt ccggtgcatc cgtgaaagtg 60
   tcgtgcaagg cctcaggcta tcccttcacc ggatactaca ttcagtgggt ccgccaggct 120
   ccgggacagg gcctcgaatg gatgggctgg atcgatccca actccggcaa tactggctac 180
   gcgcagaagt tccagggacg cgtgaccatg actcggaaca cctccatttc caccgcctac 240
   atggaactgt cgtcactgag gtccgaggac accgccgtgt attactgcgc gtcggacagc 300
   tacggatact actacgggat ggacgtgtgg ggacagggaa ctctggtcac cgtgtcgtcc 360
   ggaggcggag gcagcggcgg gggcggctcc gggggagggg ggtcgggcgg aggcggaagc 420
   gacattcaga tgacccagtc accatcctcc ctgtccgcct ccgtcgggga tagagtgacc 480
   ttcacctgtc gggcctccca aggaatctca agcgctctgg cctggtacca gcagaagcct 540
   ggaaagccgc ccaagctgtt gatctacgat gcctcgagcc tggaatccgg cgtgccgagc 600
   cggttcagcg gtagcggctc gggaaccgac ttcacgctca ccatctcgtc cctgcaaccg 660
   gaggacttcg cgacttacta ctgccagcaa ttcaacaact accctctgac ctttggtggt 720
   ggtactaagg tcgagatcaa gggtggagga ggatccggat ccgataagac ccacacctgt 780
   ccaccctgcc ctgcccccga actgcttggt ggtccgtccg tgtttctgtt cccgcccaag 840
   cccaaggaca ccctcatgat ctcacggact cctgaagtga cctgtgtggt ggtcgctgtg 900
   tcccacgagg accccgaagt caagttcaat tggtacgtgg acggagtgga agtgcacaac 960
   gctaagacca agccccgcga ggaacagtac aactccactt accgcgtcgt gtcggtgctg 1020
   accgtgctgc atcaggattg gctgaacgga aaggagtaca agtgcaaggt gtccaacaag 1080
   gctctggcgg cacccatcga aaagaccatc agcaaggcca aagggcaacc tagagaacca 1140
   caagtctaca ccctgcctcc ttgccgggat gagctcacca agaaccaggt gtccctgtgg 1200
   tgcctcgtga agggcttcta cccctctgac atcgcggtgg aatgggagtc aaacggccag 1260
   ccagagaaca actacaagac aaccccccct gtcctggaca gcgacggctc cttcttcctg 1320
   tactcgaagc tgactgtgga taagagccgg tggcaacagg gcaacgtgtt ctcatgttcg 1380
   gtcatgcacg aggccctgca taaccactac actcagaagt ccctgagcct gtcccctgga 1440
   aag 1443
<210> 547
   <211> 471
   <212> PRT
   <213> Artificial Sequence
<220>
   <221> source
   <223> /note="Description of Artificial Sequence: Synthetic polypeptide"
<400> 547
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Trp Met Ser Trp Val Arg Gln Ala Pro Gly Lys Gly Leu Glu Trp Val
           35                  40                  45
   Ala Asn Ile Asn Glu Asp Gly Ser Ala Lys Phe Tyr Val Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Phe Cys
                   85                  90                  95
   Ala Arg Asp Leu Arg Ser Gly Arg Tyr Trp Gly Gln Gly Thr Leu Val
               100                 105                 110
   Thr Val Ser Ser Gly Gly Gly Gly Ser Gly Gly Gly Gly Ser Gly Gly
           115                 120                 125
   Gly Gly Ser Glu Ile Val Leu Thr Gln Ser Pro Gly Thr Leu Ser Leu
       130                 135                 140
   Ser Pro Gly Gly Arg Ala Thr Leu Ser Cys Arg Ala Ser Gln Ser Ile
   145                 150                 155                 160
   Ser Gly Ser Phe Leu Ala Trp Tyr Gln Gln Lys Pro Gly Gln Ala Pro
                   165                 170                 175
   Arg Leu Leu Ile Tyr Gly Ala Ser Ser Arg Ala Thr Gly Ile Pro Asp
               180                 185                 190
   Arg Phe Ser Gly Ser Gly Ser Gly Thr Asp Phe Thr Leu Thr Ile Ser
           195                 200                 205
   Arg Leu Glu Pro Glu Asp Phe Ala Val Tyr Tyr Cys Gln Gln Tyr Gly
       210                 215                 220
   Ser Ser Pro Pro Thr Phe Gly Leu Gly Thr Lys Leu Glu Ile Lys Gly
   225                 230                 235                 240
   Gly Gly Gly Ser Asp Lys Thr His Thr Cys Pro Pro Cys Pro Ala Pro
                   245                 250                 255
   Glu Leu Leu Gly Gly Pro Ser Val Phe Leu Phe Pro Pro Lys Pro Lys
               260                 265                 270
   Asp Thr Leu Met Ile Ser Arg Thr Pro Glu Val Thr Cys Val Val Val
           275                 280                 285
   Ala Val Ser His Glu Asp Pro Glu Val Lys Phe Asn Trp Tyr Val Asp
       290                 295                 300
   Gly Val Glu Val His Asn Ala Lys Thr Lys Pro Arg Glu Glu Gln Tyr
   305                 310                 315                 320
   Asn Ser Thr Tyr Arg Val Val Ser Val Leu Thr Val Leu His Gln Asp
                   325                 330                 335
   Trp Leu Asn Gly Lys Glu Tyr Lys Cys Lys Val Ser Asn Lys Ala Leu
               340                 345                 350
   Ala Ala Pro Ile Glu Lys Thr Ile Ser Lys Ala Lys Gly Gln Pro Arg
           355                 360                 365
   Glu Pro Gln Val Tyr Thr Leu Pro Pro Cys Arg Asp Glu Leu Thr Lys
       370                 375                 380
   Asn Gln Val Ser Leu Trp Cys Leu Val Lys Gly Phe Tyr Pro Ser Asp
   385                 390                 395                 400
   Ile Ala Val Glu Trp Glu Ser Asn Gly Gln Pro Glu Asn Asn Tyr Lys
                   405                 410                 415
   Thr Thr Pro Pro Val Leu Asp Ser Asp Gly Ser Phe Phe Leu Tyr Ser
               420                 425                 430
   Lys Leu Thr Val Asp Lys Ser Arg Trp Gln Gln Gly Asn Val Phe Ser
           435                 440                 445
   Cys Ser Val Met His Glu Ala Leu His Asn His Tyr Thr Gln Lys Ser
       450                 455                 460
   Leu Ser Leu Ser Pro Gly Lys
   465                 470
<210> 548
   <211> 324
   <212> DNA
   <213> Artificial Sequence
<220>
   <221> source
   <223> /note="Description of Artificial Sequence: Synthetic polynucleotide"
<400> 548
   gaaattgtgt tgacccagtc gcctggaacc ctttccctgt cgcccggcgg acgggctacc        60
   ctgtcgtgcc gcgctagcca gtcgatctcc ggatcttttc tcgcctggta ccagcagaag       120
   cccggacagg cccctaggct gctgatctac ggggccagct cacgcgcaac cggtattccg       180
   gatcggttct ccggttccgg gtcgggaact gacttcaccc tgactatctc ccggctggaa       240
   ccagaggatt tcgcggtcta ctactgccag cagtatggaa gctcaccgcc gaccttcggc       300
   ttgggaacca agctggaaat caag                                              324
<210> 549
   <211> 348
   <212> DNA
   <213> Artificial Sequence
<220>
   <221> source
   <223> /note="Description of Artificial Sequence: Synthetic polynucleotide"
<400> 549
   caagtgcagc tcgtcgaaag cggtggcggg ctggtgcagc cgggcggctc gctgagactg        60
   tcctgcgccg cgagcggctt caccttctcc tcctactgga tgtcctgggt ccgccaagcc       120
   cccggaaagg ggctggaatg ggtggccaac attaacgagg acggttccgc caagttctac       180
   gtggattccg tgaaaggccg gtttaccatc tcgagggaca acgccaagaa ttccctctac       240
   ctccaaatga actccctgag agcggaggac actgccgtgt acttctgtgc acgcgacctg       300
   agatcaggcc ggtactgggg ccaggggaca ctcgtgaccg tgtcaagc                    348
<210> 550
   <211> 717
   <212> DNA
   <213> Artificial Sequence
<220>
   <221> source
   <223> /note="Description of Artificial Sequence: Synthetic polynucleotide"
<400> 550
   caagtgcagc tcgtcgaaag cggtggcggg ctggtgcagc cgggcggctc gctgagactg 60
   tcctgcgccg cgagcggctt caccttctcc tcctactgga tgtcctgggt ccgccaagcc 120
   cccggaaagg ggctggaatg ggtggccaac attaacgagg acggttccgc caagttctac 180
   gtggattccg tgaaaggccg gtttaccatc tcgagggaca acgccaagaa ttccctctac 240
   ctccaaatga actccctgag agcggaggac actgccgtgt acttctgtgc acgcgacctg 300
   agatcaggcc ggtactgggg ccaggggaca ctcgtgaccg tgtcaagcgg aggcggtggc 360
   tccggaggag gaggttccgg gggaggaggc agcgaaattg tgttgaccca gtcgcctgga 420
   accctttccc tgtcgcccgg cggacgggct accctgtcgt gccgcgctag ccagtcgatc 480
   tccggatctt ttctcgcctg gtaccagcag aagcccggac aggcccctag gctgctgatc 540
   tacggggcca gctcacgcgc aaccggtatt ccggatcggt tctccggttc cgggtcggga 600
   actgacttca ccctgactat ctcccggctg gaaccagagg atttcgcggt ctactactgc 660
   cagcagtatg gaagctcacc gccgaccttc ggcttgggaa ccaagctgga aatcaag 717
<210> 551
   <211> 1413
   <212> DNA
   <213> Artificial Sequence
<220>
   <221> source
   <223> /note="Description of Artificial Sequence: Synthetic polynucleotide"
<400> 551
   caagtgcagc tcgtcgaaag cggtggcggg ctggtgcagc cgggcggctc gctgagactg        60
   tcctgcgccg cgagcggctt caccttctcc tcctactgga tgtcctgggt ccgccaagcc       120
   cccggaaagg ggctggaatg ggtggccaac attaacgagg acggttccgc caagttctac       180
   gtggattccg tgaaaggccg gtttaccatc tcgagggaca acgccaagaa ttccctctac       240
   ctccaaatga actccctgag agcggaggac actgccgtgt acttctgtgc acgcgacctg       300
   agatcaggcc ggtactgggg ccaggggaca ctcgtgaccg tgtcaagcgg aggcggtggc       360
   tccggaggag gaggttccgg gggaggaggc agcgaaattg tgttgaccca gtcgcctgga       420
   accctttccc tgtcgcccgg cggacgggct accctgtcgt gccgcgctag ccagtcgatc       480
   tccggatctt ttctcgcctg gtaccagcag aagcccggac aggcccctag gctgctgatc       540
   tacggggcca gctcacgcgc aaccggtatt ccggatcggt tctccggttc cgggtcggga       600
   actgacttca ccctgactat ctcccggctg gaaccagagg atttcgcggt ctactactgc       660
   cagcagtatg gaagctcacc gccgaccttc ggcttgggaa ccaagctgga aatcaagggg       720
   gggggcggat ccgataagac ccacacctgt ccaccctgcc ctgcccccga actgcttggt       780
   ggtccgtccg tgtttctgtt cccgcccaag cccaaggaca ccctcatgat ctcacggact       840
   cctgaagtga cctgtgtggt ggtcgctgtg tcccacgagg accccgaagt caagttcaat       900
   tggtacgtgg acggagtgga agtgcacaac gctaagacca agccccgcga ggaacagtac       960
   aactccactt accgcgtcgt gtcggtgctg accgtgctgc atcaggattg gctgaacgga      1020
   aaggagtaca agtgcaaggt gtccaacaag gctctggcgg cacccatcga aaagaccatc      1080
   agcaaggcca aagggcaacc tagagaacca caagtctaca ccctgcctcc ttgccgggat      1140
   gagctcacca agaaccaggt gtccctgtgg tgcctcgtga agggcttcta cccctctgac      1200
   atcgcggtgg aatgggagtc aaacggccag ccagagaaca actacaagac aaccccccct      1260
   gtcctggaca gcgacggctc cttcttcctg tactcgaagc tgactgtgga taagagccgg      1320
   tggcaacagg gcaacgtgtt ctcatgttcg gtcatgcacg aggccctgca taaccactac      1380
   actcagaagt ccctgagcct gtcccctgga aag                                   1413
<210> 552
   <211> 477
   <212> PRT
   <213> Artificial Sequence
<220>
   <221> source
   <223> /note="Description of Artificial Sequence: Synthetic polypeptide"
<400> 552
   Glu Val Gln Leu Val Glu Ser Gly Gly Gly Val Val Gln Pro Gly Gly
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Leu Ile Ser Gly Asp Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Val Glu Asp Thr Ala Val Tyr Tyr Cys
                   85                  90                  95
   Ala Arg Val Phe Asp Ser Tyr Tyr Met Asp Val Trp Gly Lys Gly Thr
               100                 105                 110
   Thr Val Thr Val Ser Ser Gly Gly Gly Gly Ser Gly Gly Gly Gly Ser
           115                 120                 125
   Gly Ser Gly Gly Ser Glu Ile Val Leu Thr Gln Ser Pro Leu Ser Leu
   130 135 140
   Pro Val Thr Pro Gly Gln Pro Ala Ser Ile Ser Cys Arg Ser Ser Gln
   145 150 155 160
   Ser Leu Val Tyr Thr Asp Gly Asn Thr Tyr Leu Asn Trp Phe Gln Gln
   165 170 175
   Arg Pro Gly Gln Ser Pro Arg Arg Leu Ile Tyr Lys Val Ser Asn Arg
   180 185 190
   Asp Ser Gly Val Pro Asp Arg Phe Ser Gly Ser Gly Ser Asp Thr Asp
   195 200 205
   Phe Thr Leu Lys Ile Ser Arg Val Glu Ala Glu Asp Val Gly Ile Tyr
   210 215 220
   Tyr Cys Met Gln Gly Thr His Trp Ser Phe Thr Phe Gly Gln Gly Thr
   225 230 235 240
   Arg Leu Glu Ile Lys Gly Gly Gly Gly Ser Asp Lys Thr His Thr Cys
   245 250 255
   Pro Pro Cys Pro Ala Pro Glu Leu Leu Gly Gly Pro Ser Val Phe Leu
   260 265 270
   Phe Pro Pro Lys Pro Lys Asp Thr Leu Met Ile Ser Arg Thr Pro Glu
   275 280 285
   Val Thr Cys Val Val Val Ala Val Ser His Glu Asp Pro Glu Val Lys
   290 295 300
   Phe Asn Trp Tyr Val Asp Gly Val Glu Val His Asn Ala Lys Thr Lys
   305 310 315 320
   Pro Arg Glu Glu Gln Tyr Asn Ser Thr Tyr Arg Val Val Ser Val Leu
   325 330 335
   Thr Val Leu His Gln Asp Trp Leu Asn Gly Lys Glu Tyr Lys Cys Lys
   340 345 350
   Val Ser Asn Lys Ala Leu Ala Ala Pro Ile Glu Lys Thr Ile Ser Lys
   355 360 365
   Ala Lys Gly Gln Pro Arg Glu Pro Gln Val Tyr Thr Leu Pro Pro Cys
   370 375 380
   Arg Asp Glu Leu Thr Lys Asn Gln Val Ser Leu Trp Cys Leu Val Lys
   385                 390                 395                 400
   Gly Phe Tyr Pro Ser Asp Ile Ala Val Glu Trp Glu Ser Asn Gly Gln
                   405                 410                 415
   Pro Glu Asn Asn Tyr Lys Thr Thr Pro Pro Val Leu Asp Ser Asp Gly
               420                 425                 430
   Ser Phe Phe Leu Tyr Ser Lys Leu Thr Val Asp Lys Ser Arg Trp Gln
           435                 440                 445
   Gln Gly Asn Val Phe Ser Cys Ser Val Met His Glu Ala Leu His Asn
       450                 455                 460
   His Tyr Thr Gln Lys Ser Leu Ser Leu Ser Pro Gly Lys
   465                 470                 475
<210> 553
   <211> 336
   <212> DNA
   <213> Artificial Sequence
<220>
   <221> source
   <223> /note="Description of Artificial Sequence: Synthetic polynucleotide"
<400> 553
   gaaattgtcc tcacccaatc cccgctgtca ctgcccgtga cccctggcca gccggcatcc      60
   atcagctgcc ggagcagcca gtccctggtg tacactgacg gaaatactta cctgaactgg     120
   ttccagcaac gcccggggca gagcccacgc agactgatct acaaggtgtc aaacagggac     180
   tctggagtgc ccgataggtt ctcgggttcc gggtcggaca ccgattttac actgaagatc     240
   tcccgggtgg aagcggagga cgtgggcatc tattactgta tgcaggggac ccattggtcc     300
   ttcacgttcg gacagggcac tcggctggaa atcaag                               336
<210> 554
   <211> 354
   <212> DNA
   <213> Artificial Sequence
<220>
   <221> source
   <223> /note="Description of Artificial Sequence: Synthetic polynucleotide"
<400> 554
   gaagtgcagt tggtggagag cggaggcggc gtggtgcagc ccggaggttc cctgcggctg 60
   tcgtgcgcgg cctcgggttt cacttttgat gactacgcca tgcactgggt cagacaggcc 120
   cctggaaagg gcctcgaatg ggtgtcgctg atttccggag atggaggcag cacctactat 180
   gccgattccg tcaaggggag attcaccatt tcccgcgaca acagcaaaaa caccttgtac 240
   ctccaaatga actccctgcg ggtggaggac accgctgtgt actactgcgc ccgcgtgttc 300
   gactcatact acatggacgt ctggggaaag gggaccactg tgaccgtgtc cagc 354
<210> 555
   <211> 735
   <212> DNA
   <213> Artificial Sequence
<220>
   <221> source
   <223> /note="Description of Artificial Sequence: Synthetic polynucleotide"
<400> 555
   gaagtgcagt tggtggagag cggaggcggc gtggtgcagc ccggaggttc cctgcggctg 60
   tcgtgcgcgg cctcgggttt cacttttgat gactacgcca tgcactgggt cagacaggcc 120
   cctggaaagg gcctcgaatg ggtgtcgctg atttccggag atggaggcag cacctactat 180
   gccgattccg tcaaggggag attcaccatt tcccgcgaca acagcaaaaa caccttgtac 240
   ctccaaatga actccctgcg ggtggaggac accgctgtgt actactgcgc ccgcgtgttc 300
   gactcatact acatggacgt ctggggaaag gggaccactg tgaccgtgtc cagcggggga 360
   ggcggctccg gcggcggcgg atcgggttca ggagggtccg aaattgtcct cacccaatcc 420
   ccgctgtcac tgcccgtgac ccctggccag ccggcatcca tcagctgccg gagcagccag 480
   tccctggtgt acactgacgg aaatacttac ctgaactggt tccagcaacg cccggggcag 540
   agcccacgca gactgatcta caaggtgtca aacagggact ctggagtgcc cgataggttc 600
   tcgggttccg ggtcggacac cgattttaca ctgaagatct cccgggtgga agcggaggac 660
   gtgggcatct attactgtat gcaggggacc cattggtcct tcacgttcgg acagggcact 720
   cggctggaaa tcaag 735
<210> 556
   <211> 1431
   <212> DNA
   <213> Artificial Sequence
<220>
   <221> source
   <223> /note="Description of Artificial Sequence: Synthetic polynucleotide"
<400> 556
   gaagtgcagt tggtggagag cggaggcggc gtggtgcagc ccggaggttc cctgcggctg      60
   tcgtgcgcgg cctcgggttt cacttttgat gactacgcca tgcactgggt cagacaggcc     120
   cctggaaagg gcctcgaatg ggtgtcgctg atttccggag atggaggcag cacctactat     180
   gccgattccg tcaaggggag attcaccatt tcccgcgaca acagcaaaaa caccttgtac     240
   ctccaaatga actccctgcg ggtggaggac accgctgtgt actactgcgc ccgcgtgttc     300
   gactcatact acatggacgt ctggggaaag gggaccactg tgaccgtgtc cagcggggga     360
   ggcggctccg gcggcggcgg atcgggttca ggagggtccg aaattgtcct cacccaatcc     420
   ccgctgtcac tgcccgtgac ccctggccag ccggcatcca tcagctgccg gagcagccag     480
   tccctggtgt acactgacgg aaatacttac ctgaactggt tccagcaacg cccggggcag     540
   agcccacgca gactgatcta caaggtgtca aacagggact ctggagtgcc cgataggttc     600
   tcgggttccg ggtcggacac cgattttaca ctgaagatct cccgggtgga agcggaggac     660
   gtgggcatct attactgtat gcaggggacc cattggtcct tcacgttcgg acagggcact     720
   cggctggaaa tcaagggagg aggcggatcc gataagaccc acacctgtcc accctgccct     780
   gcccccgaac tgcttggtgg tccgtccgtg tttctgttcc cgcccaagcc caaggacacc     840
   ctcatgatct cacggactcc tgaagtgacc tgtgtggtgg tcgctgtgtc ccacgaggac     900
   cccgaagtca agttcaattg gtacgtggac ggagtggaag tgcacaacgc taagaccaag     960
   ccccgcgagg aacagtacaa ctccacttac cgcgtcgtgt cggtgctgac cgtgctgcat    1020
   caggattggc tgaacggaaa ggagtacaag tgcaaggtgt ccaacaaggc tctggcggca    1080
   cccatcgaaa agaccatcag caaggccaaa gggcaaccta gagaaccaca agtctacacc    1140
   ctgcctcctt gccgggatga gctcaccaag aaccaggtgt ccctgtggtg cctcgtgaag    1200
   ggcttctacc cctctgacat cgcggtggaa tgggagtcaa acggccagcc agagaacaac    1260
   tacaagacaa ccccccctgt cctggacagc gacggctcct tcttcctgta ctcgaagctg    1320
   actgtggata agagccggtg gcaacagggc aacgtgttct catgttcggt catgcacgag    1380
   gccctgcata accactacac tcagaagtcc ctgagcctgt cccctggaaa g              1431
<210> 557
<400> 557
   000
<210> 558
<400> 558
   000
<210> 559
<400> 559
   000
<210> 560
<400> 560
   000
<210> 561
<400> 561
   000
<210> 562
<400> 562
   000
<210> 563
<400> 563
   000
<210> 564
<400> 564
   000
<210> 565
<400> 565
   000
<210> 566
<400> 566
   000
<210> 567
<400> 567
   000
<210> 568
<400> 568
   000
<210> 569
<400> 569
   000
<210> 570
<400> 570
   000
<210> 571
<400> 571
   000
<210> 572
<400> 572
   000
<210> 573
<400> 573
   000
<210> 574
<400> 574
   000
<210> 575
<400> 575
   000
<210> 576
<400> 576
   000
<210> 577
<400> 577
   000
<210> 578
<400> 578
   000
<210> 579
<400> 579
   000
<210> 580
<400> 580
   000
<210> 581
<400> 581
   000
<210> 582
<400> 582
   000
<210> 583
<400> 583
   000
<210> 584
<400> 584
   000
<210> 585
<400> 585
   000
<210> 586
<400> 586
   000
<210> 587
<400> 587
   000
<210> 588
<400> 588
   000
<210> 589
<400> 589
   000
<210> 590
<400> 590
   000
<210> 591
<400> 591
   000
<210> 592
<400> 592
   000
<210> 593
<400> 593
   000
<210> 594
<400> 594
   000
<210> 595
<400> 595
   000
<210> 596
<400> 596
   000
<210> 597
<400> 597
   000
<210> 598
<400> 598
   000
<210> 599
<400> 599
   000
<210> 600
<400> 600
   000
<210> 601
<400> 601
   000
<210> 602
<400> 602
   000
<210> 603
<400> 603
   000
<210> 604
<400> 604
   000
<210> 605
<400> 605
   000
<210> 606
<400> 606
   000
<210> 607
<400> 607
   000
<210> 608
<400> 608
   000
<210> 609
<400> 609
   000
<210> 610
<400> 610
   000
<210> 611
<400> 611
   000
<210> 612
<400> 612
   000
<210> 613
<400> 613
   000
<210> 614
<400> 614
   000
<210> 615
<400> 615
   000
<210> 616
<400> 616
   000
<210> 617
<400> 617
   000
<210> 618
<400> 618
   000
<210> 619
<400> 619
   000
<210> 620
<400> 620
   000
<210> 621
<400> 621
   000
<210> 622
<400> 622
   000
<210> 623
<400> 623
   000
<210> 624
<400> 624
   000
<210> 625
<400> 625
   000
<210> 626
<400> 626
   000
<210> 627
<400> 627
   000
<210> 628
<400> 628
   000
<210> 629
<400> 629
   000
<210> 630
<400> 630
   000
<210> 631
<400> 631
   000
<210> 632
<400> 632
   000
<210> 633
<400> 633
   000
<210> 634
<400> 634
   000
<210> 635
<400> 635
   000
<210> 636
<400> 636
   000
<210> 637
<400> 637
   000
<210> 638
<400> 638
   000
<210> 639
<400> 639
   000
<210> 640
<400> 640
   000
<210> 641
<400> 641
   000
<210> 642
<400> 642
   000
<210> 643
<400> 643
   000
<210> 644
<400> 644
   000
<210> 645
<400> 645
   000
<210> 646
<400> 646
   000
<210> 647
<400> 647
   000
<210> 648
<400> 648
   000
<210> 649
<400> 649
   000
<210> 650
<400> 650
   000
<210> 651
<400> 651
   000
<210> 652
<400> 652
   000
<210> 653
<400> 653
   000
<210> 654
<400> 654
   000
<210> 655
<400> 655
   000
<210> 656
<400> 656
   000
<210> 657
<400> 657
   000
<210> 658
<400> 658
   000
<210> 659
<400> 659
   000
<210> 660
<400> 660
   000
<210> 661
<400> 661
   000
<210> 662
<400> 662
   000
<210> 663
<400> 663
   000
<210> 664
<400> 664
   000
<210> 665
<400> 665
   000
<210> 666
<400> 666
   000
<210> 667
<400> 667
   000
<210> 668
<400> 668
   000
<210> 669
<400> 669
   000
<210> 670
<400> 670
   000
<210> 671
<400> 671
   000
<210> 672
<400> 672
   000
<210> 673
<400> 673
   000
<210> 674
<400> 674
   000
<210> 675
<400> 675
   000
<210> 676
<400> 676
   000
<210> 677
<400> 677
   000
<210> 678
<400> 678
   000
<210> 679
<400> 679
   000
<210> 680
<400> 680
   000
<210> 681
<400> 681
   000
<210> 682
<400> 682
   000
<210> 683
<400> 683
   000
<210> 684
<400> 684
   000
<210> 685
<400> 685
   000
<210> 686
<400> 686
   000
<210> 687
<400> 687
   000
<210> 688
<400> 688
   000
<210> 689
<400> 689
   000
<210> 690
<400> 690
   000
<210> 691
<400> 691
   000
<210> 692
<400> 692
   000
<210> 693
<400> 693
   000
<210> 694
<400> 694
   000
<210> 695
<400> 695
   000
<210> 696
<400> 696
   000
<210> 697
<400> 697
   000
<210> 698
<400> 698
   000
<210> 699
<400> 699
   000
<210> 700
<400> 700
   000
<210> 701
<400> 701
   000
<210> 702
<400> 702
   000
<210> 703
<400> 703
   000
<210> 704
<400> 704
   000
<210> 705
<400> 705
   000
<210> 706
<400> 706
   000
<210> 707
<400> 707
   000
<210> 708
<400> 708
   000
<210> 709
<400> 709
   000
<210> 710
<400> 710
   000
<210> 711
<400> 711
   000
<210> 712
<400> 712
   000
<210> 713
<400> 713
   000
<210> 714
<400> 714
   000
<210> 715
<400> 715
   000
<210> 716
<400> 716
   000
<210> 717
<400> 717
   000
<210> 718
<400> 718
   000
<210> 719
<400> 719
   000
<210> 720
<400> 720
   000
<210> 721
<400> 721
   000
<210> 722
<400> 722
   000
<210> 723
<400> 723
   000
<210> 724
<400> 724
   000
<210> 725
<400> 725
   000
<210> 726
<400> 726
   000
<210> 727
<400> 727
   000
<210> 728
<400> 728
   000
<210> 729
<400> 729
   000
<210> 730
<400> 730
   000
<210> 731
<400> 731
   000
<210> 732
<400> 732
   000
<210> 733
<400> 733
   000
<210> 734
<400> 734
   000
<210> 735
<400> 735
   000
<210> 736
<400> 736
   000
<210> 737
<400> 737
   000
<210> 738
<400> 738
   000
<210> 739
<400> 739
   000
<210> 740
<400> 740
   000
<210> 741
<400> 741
   000
<210> 742
<400> 742
   000
<210> 743
<400> 743
   000
<210> 744
<400> 744
   000
<210> 745
<400> 745
   000
<210> 746
<400> 746
   000
<210> 747
<400> 747
   000
<210> 748
<400> 748
   000
<210> 749
<400> 749
   000
<210> 750
<400> 750
   000
<210> 751
<400> 751
   000
<210> 752
<400> 752
   000
<210> 753
<400> 753
   000
<210> 754
<400> 754
   000
<210> 755
<400> 755
   000
<210> 756
<400> 756
   000
<210> 757
<400> 757
   000
<210> 758
<400> 758
   000
<210> 759
<400> 759
   000
<210> 760
<400> 760
   000
<210> 761
<400> 761
   000
<210> 762
<400> 762
   000
<210> 763
<400> 763
   000
<210> 764
<400> 764
   000
<210> 765
<400> 765
   000
<210> 766
<400> 766
   000
<210> 767
<400> 767
   000
<210> 768
<400> 768
   000
<210> 769
<400> 769
   000
<210> 770
<400> 770
   000
<210> 771
<400> 771
   000
<210> 772
<400> 772
   000
<210> 773
<400> 773
   000
<210> 774
<400> 774
   000
<210> 775
<400> 775
   000
<210> 776
<400> 776
   000
<210> 777
<400> 777
   000
<210> 778
<400> 778
   000
<210> 779
<400> 779
   000
<210> 780
<400> 780
   000
<210> 781
<400> 781
   000
<210> 782
<400> 782
   000
<210> 783
<400> 783
   000
<210> 784
<400> 784
   000
<210> 785
<400> 785
   000
<210> 786
<400> 786
   000
<210> 787
<400> 787
   000
<210> 788
<400> 788
   000
<210> 789
<400> 789
   000
<210> 790
<400> 790
   000
<210> 791
<400> 791
   000
<210> 792
<400> 792
   000
<210> 793
<400> 793
   000
<210> 794
<400> 794
   000
<210> 795
<400> 795
   000
<210> 796
<400> 796
   000
<210> 797
<400> 797
   000
<210> 798
<400> 798
   000
<210> 799
<400> 799
   000
<210> 800
<400> 800
   000
<210> 801
<400> 801
   000
<210> 802
<400> 802
   000
<210> 803
<400> 803
   000
<210> 804
<400> 804
   000
<210> 805
<400> 805
   000
<210> 806
<400> 806
   000
<210> 807
<400> 807
   000
<210> 808
<400> 808
   000
<210> 809
<400> 809
   000
<210> 810
<400> 810
   000
<210> 811
<400> 811
   000
<210> 812
<400> 812
   000
<210> 813
<400> 813
   000
<210> 814
<400> 814
   000
<210> 815
<400> 815
   000
<210> 816
<400> 816
   000
<210> 817
<400> 817
   000
<210> 818
<400> 818
   000
<210> 819
<400> 819
   000
<210> 820
<400> 820
   000
<210> 821
<400> 821
   000
<210> 822
<400> 822
   000
<210> 823
<400> 823
   000
<210> 824
<400> 824
   000
<210> 825
<400> 825
   000
<210> 826
<400> 826
   000
<210> 827
<400> 827
   000
<210> 828
<400> 828
   000
<210> 829
<400> 829
   000
<210> 830
<400> 830
   000
<210> 831
<400> 831
   000
<210> 832
<400> 832
   000
<210> 833
<400> 833
   000
<210> 834
<400> 834
   000
<210> 835
<400> 835
   000
<210> 836
<400> 836
   000
<210> 837
<400> 837
   000
<210> 838
<400> 838
   000
<210> 839
<400> 839
   000
<210> 840
<400> 840
   000
<210> 841
<400> 841
   000
<210> 842
<400> 842
   000
<210> 843
<400> 843
   000
<210> 844
<400> 844
   000
<210> 845
<400> 845
   000
<210> 846
<400> 846
   000
<210> 847
<400> 847
   000
<210> 848
<400> 848
   000
<210> 849
<400> 849
   000
<210> 850
<400> 850
   000
<210> 851
<400> 851
   000
<210> 852
<400> 852
   000
<210> 853
<400> 853
   000
<210> 854
<400> 854
   000
<210> 855
<400> 855
   000
<210> 856
<400> 856
   000
<210> 857
<400> 857
   000
<210> 858
<400> 858
   000
<210> 859
<400> 859
   000
<210> 860
<400> 860
   000
<210> 861
<400> 861
   000
<210> 862
<400> 862
   000
<210> 863
<400> 863
   000
<210> 864
<400> 864
   000
<210> 865
<400> 865
   000
<210> 866
<400> 866
   000
<210> 867
<400> 867
   000
<210> 868
<400> 868
   000
<210> 869
<400> 869
   000
<210> 870
<400> 870
   000
<210> 871
<400> 871
   000
<210> 872
<400> 872
   000
<210> 873
<400> 873
   000
<210> 874
<400> 874
   000
<210> 875
<400> 875
   000
<210> 876
<400> 876
   000
<210> 877
<400> 877
   000
<210> 878
<400> 878
   000
<210> 879
<400> 879
   000
<210> 880
<400> 880
   000
<210> 881
<400> 881
   000
<210> 882
<400> 882
   000
<210> 883
<400> 883
   000
<210> 884
<400> 884
   000
<210> 885
<400> 885
   000
<210> 886
<400> 886
   000
<210> 887
<400> 887
   000
<210> 888
<400> 888
   000
<210> 889
<400> 889
   000
<210> 890
<400> 890
   000
<210> 891
<400> 891
   000
<210> 892
<400> 892
   000
<210> 893
<400> 893
   000
<210> 894
<400> 894
   000
<210> 895
<400> 895
   000
<210> 896
<400> 896
   000
<210> 897
<400> 897
   000
<210> 898
<400> 898
   000
<210> 899
<400> 899
   000
<210> 900
<400> 900
   000
<210> 901
<400> 901
   000
<210> 902
<400> 902
   000
<210> 903
<400> 903
   000
<210> 904
<400> 904
   000
<210> 905
<400> 905
   000
<210> 906
<400> 906
   000
<210> 907
<400> 907
   000
<210> 908
<400> 908
   000
<210> 909
<400> 909
   000
<210> 910
<400> 910
   000
<210> 911
<400> 911
   000
<210> 912
<400> 912
   000
<210> 913
<400> 913
   000
<210> 914
<400> 914
   000
<210> 915
<400> 915
   000
<210> 916
<400> 916
   000
<210> 917
<400> 917
   000
<210> 918
<400> 918
   000
<210> 919
<400> 919
   000
<210> 920
<400> 920
   000
<210> 921
<400> 921
   000
<210> 922
<400> 922
   000
<210> 923
<400> 923
   000
<210> 924
<400> 924
   000
<210> 925
<400> 925
   000
<210> 926
<400> 926
   000
<210> 927
<400> 927
   000
<210> 928
<400> 928
   000
<210> 929
<400> 929
   000
<210> 930
<400> 930
   000
<210> 931
<400> 931
   000
<210> 932
<400> 932
   000
<210> 933
<400> 933
   000
<210> 934
<400> 934
   000
<210> 935
<400> 935
   000
<210> 936
<400> 936
   000
<210> 937
<400> 937
   000
<210> 938
<400> 938
   000
<210> 939
<400> 939
   000
<210> 940
<400> 940
   000
<210> 941
<400> 941
   000
<210> 942
<400> 942
   000
<210> 943
<400> 943
   000
<210> 944
<400> 944
   000
<210> 945
<400> 945
   000
<210> 946
<400> 946
   000
<210> 947
<400> 947
   000
<210> 948
<400> 948
   000
<210> 949
<400> 949
   000
<210> 950
<400> 950
   000
<210> 951
<400> 951
   000
<210> 952
<400> 952
   000
<210> 953
<400> 953
   000
<210> 954
<400> 954
   000
<210> 955
<400> 955
   000
<210> 956
<400> 956
   000
<210> 957
<400> 957
   000
<210> 958
<400> 958
   000
<210> 959
<400> 959
   000
<210> 960
<400> 960
   000
<210> 961
<400> 961
   000
<210> 962
<400> 962
   000
<210> 963
<400> 963
   000
<210> 964
<400> 964
   000
<210> 965
<400> 965
   000
<210> 966
<400> 966
   000
<210> 967
<400> 967
   000
<210> 968
<400> 968
   000
<210> 969
<400> 969
   000
<210> 970
<400> 970
   000
<210> 971
<400> 971
   000
<210> 972
<400> 972
   000
<210> 973
<400> 973
   000
<210> 974
<400> 974
   000
<210> 975
<400> 975
   000
<210> 976
<400> 976
   000
<210> 977
<400> 977
   000
<210> 978
<400> 978
   000
<210> 979
<400> 979
   000
<210> 980
<400> 980
   000
<210> 981
<400> 981
   000
<210> 982
<400> 982
   000
<210> 983
<400> 983
   000
<210> 984
<400> 984
   000
<210> 985
<400> 985
   000
<210> 986
<400> 986
   000
<210> 987
<400> 987
   000
<210> 988
<400> 988
   000
<210> 989
<400> 989
   000
<210> 990
<400> 990
   000
<210> 991
<400> 991
   000
<210> 992
<400> 992
   000
<210> 993
<400> 993
   000
<210> 994
<400> 994
   000
<210> 995
<400> 995
   000
<210> 996
<400> 996
   000
<210> 997
<400> 997
   000
<210> 998
<400> 998
   000
<210> 999
<400> 999
   000
<210> 1000
<400> 1000
   000
<210> 1001
<400> 1001
   000
<210> 1002
<400> 1002
   000
<210> 1003
<400> 1003
   000
<210> 1004
<400> 1004
   000
<210> 1005
<400> 1005
   000
<210> 1006
<400> 1006
   000
<210> 1007
<400> 1007
   000
<210> 1008
<400> 1008
   000
<210> 1009
<400> 1009
   000
<210> 1010
<400> 1010
   000
<210> 1011
<400> 1011
   000
<210> 1012
<400> 1012
   000
<210> 1013
<400> 1013
   000
<210> 1014
<400> 1014
   000
<210> 1015
<400> 1015
   000
<210> 1016
<400> 1016
   000
<210> 1017
<400> 1017
   000
<210> 1018
<400> 1018
   000
<210> 1019
<400> 1019
   000
<210> 1020
<400> 1020
   000
<210> 1021
<400> 1021
   000
<210> 1022
<400> 1022
   000
<210> 1023
<400> 1023
   000
<210> 1024
<400> 1024
   000
<210> 1025
<400> 1025
   000
<210> 1026
<400> 1026
   000
<210> 1027
<400> 1027
   000
<210> 1028
<400> 1028
   000
<210> 1029
<400> 1029
   000
<210> 1030
<400> 1030
   000
<210> 1031
<400> 1031
   000
<210> 1032
<400> 1032
   000
<210> 1033
<400> 1033
   000
<210> 1034
<400> 1034
   000
<210> 1035
<400> 1035
   000
<210> 1036
<400> 1036
   000
<210> 1037
<400> 1037
   000
<210> 1038
<400> 1038
   000
<210> 1039
<400> 1039
   000
<210> 1040
<400> 1040
   000
<210> 1041
<400> 1041
   000
<210> 1042
<400> 1042
   000
<210> 1043
<400> 1043
   000
<210> 1044
<400> 1044
   000
<210> 1045
<400> 1045
   000
<210> 1046
<400> 1046
   000
<210> 1047
<400> 1047
   000
<210> 1048
<400> 1048
   000
<210> 1049
<400> 1049
   000
<210> 1050
<400> 1050
   000
<210> 1051
<400> 1051
   000
<210> 1052
<400> 1052
   000
<210> 1053
<400> 1053
   000
<210> 1054
<400> 1054
   000
<210> 1055
<400> 1055
   000
<210> 1056
<400> 1056
   000
<210> 1057
<400> 1057
   000
<210> 1058
<400> 1058
   000
<210> 1059
<400> 1059
   000
<210> 1060
<400> 1060
   000
<210> 1061
<400> 1061
   000
<210> 1062
<400> 1062
   000
<210> 1063
<400> 1063
   000
<210> 1064
<400> 1064
   000
<210> 1065
<400> 1065
   000
<210> 1066
<400> 1066
   000
<210> 1067
<400> 1067
   000
<210> 1068
<400> 1068
   000
<210> 1069
<400> 1069
   000
<210> 1070
<400> 1070
   000
<210> 1071
<400> 1071
   000
<210> 1072
<400> 1072
   000
<210> 1073
<400> 1073
   000
<210> 1074
<400> 1074
   000
<210> 1075
<400> 1075
   000
<210> 1076
<400> 1076
   000
<210> 1077
<400> 1077
   000
<210> 1078
<400> 1078
   000
<210> 1079
<400> 1079
   000
<210> 1080
<400> 1080
   000
<210> 1081
<400> 1081
   000
<210> 1082
<400> 1082
   000
<210> 1083
<400> 1083
   000
<210> 1084
<400> 1084
   000
<210> 1085
<400> 1085
   000
<210> 1086
<400> 1086
   000
<210> 1087
<400> 1087
   000
<210> 1088
<400> 1088
   000
<210> 1089
<400> 1089
   000
<210> 1090
<400> 1090
   000
<210> 1091
<400> 1091
   000
<210> 1092
<400> 1092
   000
<210> 1093
<400> 1093
   000
<210> 1094
<400> 1094
   000
<210> 1095
<400> 1095
   000
<210> 1096
<400> 1096
   000
<210> 1097
<400> 1097
   000
<210> 1098
<400> 1098
   000
<210> 1099
<400> 1099
   000
<210> 1100
<400> 1100
   000
<210> 1101
<400> 1101
   000
<210> 1102
<400> 1102
   000
<210> 1103
<400> 1103
   000
<210> 1104
<400> 1104
   000
<210> 1105
<400> 1105
   000
<210> 1106
<400> 1106
   000
<210> 1107
<400> 1107
   000
<210> 1108
   <211> 18
   <212> PRT
   <213> Artificial Sequence
<220>
   <221> source
   <223> /note="Description of Artificial Sequence: Synthetic peptide"
<400> 1108
   Gly Ser Thr Ser Gly Ser Gly Lys Pro Gly Ser Gly Glu Gly Ser Thr
   1               5                   10                  15
   Lys Gly
<210> 1109
<400> 1109
   000
<210> 1110
<400> 1110
   000
<210> 1111
<400> 1111
   000
<210> 1112
<400> 1112
   000
<210> 1113
<400> 1113
   000
<210> 1114
<400> 1114
   000
<210> 1115
<400> 1115
   000
<210> 1116
<400> 1116
   000
<210> 1117
<400> 1117
   000
<210> 1118
<400> 1118
   000
<210> 1119
<400> 1119
   000
<210> 1120
<400> 1120
   000
<210> 1121
<400> 1121
   000
<210> 1122
<400> 1122
   000
<210> 1123
<400> 1123
   000
<210> 1124
<400> 1124
   000
<210> 1125
<400> 1125
   000
<210> 1126
<400> 1126
   000
<210> 1127
<400> 1127
   000
<210> 1128
<400> 1128
   000
<210> 1129
<400> 1129
   000
<210> 1130
<400> 1130
   000
<210> 1131
<400> 1131
   000
<210> 1132
<400> 1132
   000
<210> 1133
<400> 1133
   000
<210> 1134
<400> 1134
   000
<210> 1135
<400> 1135
   000
<210> 1136
<400> 1136
   000
<210> 1137
<400> 1137
   000
<210> 1138
<400> 1138
   000
<210> 1139
<400> 1139
   000
<210> 1140
<400> 1140
   000
<210> 1141
<400> 1141
   000
<210> 1142
<400> 1142
   000
<210> 1143
<400> 1143
   000
<210> 1144
<400> 1144
   000
<210> 1145
<400> 1145
   000
<210> 1146
<400> 1146
   000
<210> 1147
<400> 1147
   000
<210> 1148
<400> 1148
   000
<210> 1149
<400> 1149
   000
<210> 1150
<400> 1150
   000
<210> 1151
<400> 1151
   000
<210> 1152
<400> 1152
   000
<210> 1153
<400> 1153
   000
<210> 1154
<400> 1154
   000
<210> 1155
<400> 1155
   000
<210> 1156
<400> 1156
   000
<210> 1157
<400> 1157
   000
<210> 1158
<400> 1158
   000
<210> 1159
<400> 1159
   000
<210> 1160
<400> 1160
   000
<210> 1161
<400> 1161
   000
<210> 1162
<400> 1162
   000
<210> 1163
<400> 1163
   000
<210> 1164
<400> 1164
   000
<210> 1165
<400> 1165
   000
<210> 1166
<400> 1166
   000
<210> 1167
<400> 1167
   000
<210> 1168
<400> 1168
   000
<210> 1169
<400> 1169
   000
<210> 1170
<400> 1170
   000
<210> 1171
<400> 1171
   000
<210> 1172
<400> 1172
   000
<210> 1173
<400> 1173
   000
<210> 1174
<400> 1174
   000
<210> 1175
<400> 1175
   000
<210> 1176
<400> 1176
   000
<210> 1177
<400> 1177
   000
<210> 1178
<400> 1178
   000
<210> 1179
<400> 1179
   000
<210> 1180
<400> 1180
   000
<210> 1181
<400> 1181
   000
<210> 1182
<400> 1182
   000
<210> 1183
<400> 1183
   000
<210> 1184
<400> 1184
   000
<210> 1185
<400> 1185
   000
<210> 1186
<400> 1186
   000
<210> 1187
<400> 1187
   000
<210> 1188
<400> 1188
   000
<210> 1189
<400> 1189
   000
<210> 1190
<400> 1190
   000
<210> 1191
<400> 1191
   000
<210> 1192
<400> 1192
   000
<210> 1193
<400> 1193
   000
<210> 1194
<400> 1194
   000
<210> 1195
<400> 1195
   000
<210> 1196
<400> 1196
   000
<210> 1197
<400> 1197
   000
<210> 1198
<400> 1198
   000
<210> 1199
<400> 1199
   000
<210> 1200
   <211> 119
   <212> PRT
   <213> Artificial Sequence
<220>
   <221> source
   <223> /note="Description of Artificial Sequence: Synthetic polypeptide"
<400> 1200
<210> 1201
   <211> 106
   <212> PRT
   <213> Artificial Sequence
<220>
   <221> source
   <223> /note="Description of Artificial Sequence: Synthetic polypeptide"
<400> 1201
<210> 1202
   <211> 125
   <212> PRT
   <213> Artificial Sequence
<220>
   <221> source
   <223> /note="Description of Artificial Sequence: Synthetic polypeptide"
<400> 1202
   Glu Val Gln Leu Val Glu Ser Gly Gly Gly Leu Val Gln Pro Lys Gly
   1 5 10 15
   Ser Leu Lys Leu Ser Cys Ala Ala Ser Gly Phe Thr Phe Asn Thr Tyr
   20 25 30
   Ala Met Asn Trp Val Arg Gln Ala Pro Gly Lys Gly Leu Glu Trp Val
   35 40 45
   Ala Arg Ile Arg Ser Lys Tyr Asn Asn Tyr Ala Thr Tyr Tyr Ala Asp
   50 55 60
   Ser Val Lys Asp Arg Phe Thr Ile Ser Arg Asp Asp Ser Gln Ser Ile
   65 70 75 80
   Leu Tyr Leu Gln Met Asn Asn Leu Lys Thr Glu Asp Thr Ala Met Tyr
   85 90 95
   Tyr Cys Val Arg His Gly Asn Phe Gly Asn Ser Tyr Val Ser Trp Phe
   100 105 110
   Ala Tyr Trp Gly Gln Gly Thr Leu Val Thr Val Ser Ala
   115 120 125
<210> 1203
   <211> 109
   <212> PRT
   <213> Artificial Sequence
<220>
   <221> source
   <223> /note="Description of Artificial Sequence: Synthetic polypeptide"
<400> 1203
   Gln Ala Val Val Thr Gln Glu Ser Ala Leu Thr Thr Ser Pro Gly Glu
   1               5                   10                  15
   Thr Val Thr Leu Thr Cys Arg Ser Ser Thr Gly Ala Val Thr Thr Ser
               20                  25                  30
   Asn Tyr Ala Asn Trp Val Gln Glu Lys Pro Asp His Leu Phe Thr Gly
           35                  40                  45
   Leu Ile Gly Gly Thr Asn Lys Arg Ala Pro Gly Val Pro Ala Arg Phe
       50                  55                  60
   Ser Gly Ser Leu Ile Gly Asp Lys Ala Ala Leu Thr Ile Thr Gly Ala
   65                  70                  75                  80
   Gln Thr Glu Asp Glu Ala Ile Tyr Phe Cys Ala Leu Trp Tyr Ser Asn
                   85                  90                  95
   Leu Trp Val Phe Gly Gly Gly Thr Lys Leu Thr Val Leu
               100                 105
<210> 1204
   <211> 119
   <212> PRT
   <213> Artificial Sequence
<220>
   <221> source
   <223> /note="Description of Artificial Sequence: Synthetic polypeptide"
<400> 1204
   Gln Val Gln Leu Gln Gln Ser Gly Ala Glu Leu Ala Arg Pro Gly Ala
   1               5                   10                  15
   Ser Val Lys Met Ser Cys Lys Ala Ser Gly Tyr Thr Phe Thr Ser Tyr
   20 25 30
   Thr Met His Trp Val Lys Gln Arg Pro Gly Gln Gly Leu Glu Trp Ile
   35 40 45
   Gly Tyr Ile Asn Pro Ser Ser Gly Tyr Thr Lys Tyr Asn Gln Lys Phe
   50 55 60
   Lys Asp Lys Ala Thr Leu Thr Ala Asp Lys Ser Ser Ser Thr Ala Tyr
   65 70 75 80
   Met Gln Leu Ser Ser Leu Thr Ser Glu Asp Ser Ala Val Tyr Tyr Cys
   85 90 95
   Ala Arg Trp Gln Asp Tyr Asp Val Tyr Phe Asp Tyr Trp Gly Gln Gly
   100 105 110
   Thr Thr Leu Thr Val Ser Ser
   115
<210> 1205
   <211> 106
   <212> PRT
   <213> Artificial Sequence
<220>
   <221> source
   <223> /note="Description of Artificial Sequence: Synthetic polypeptide"
<400> 1205
   Gln Ile Val Leu Ser Gln Ser Pro Ala Ile Leu Ser Ala Ser Pro Gly
   1               5                   10                  15
   Glu Lys Val Thr Met Thr Cys Arg Ala Ser Ser Ser Val Ser Tyr Met
               20                  25                  30
   His Trp Tyr Gln Gln Lys Pro Gly Ser Ser Pro Lys Pro Trp Ile Tyr
           35                  40                  45
   Ala Thr Ser Asn Leu Ala Ser Gly Val Pro Ala Arg Phe Ser Gly Ser
       50                  55                  60
   Gly Ser Gly Thr Ser Tyr Ser Leu Thr Ile Ser Arg Val Glu Ala Glu
   65                  70                  75                  80
   Asp Ala Ala Thr Tyr Tyr Cys Gln Gln Trp Ser Ser Asn Pro Pro Thr
                   85                  90                  95
   Phe Gly Gly Gly Thr Lys Leu Glu Thr Lys
                       100                 105
<210> 1206
   <211> 119
   <212> PRT
   <213> Artificial Sequence
<220>
   <221> source
   <223> /note="Description of Artificial Sequence: Synthetic polypeptide"
<400> 1206
<210> 1207
   <211> 106
   <212> PRT
   <213> Artificial Sequence
<220>
   <221> source
   <223> /note="Description of Artificial Sequence: Synthetic polypeptide"
<400> 1207
   Gln Ile Val Leu Thr Gln Ser Pro Ala Ile Met Ser Ala Ser Pro Gly
   1 5 10 15
   Glu Lys Val Thr Met Thr Cys Arg Ala Ser Ser Ser Val Ser Tyr Met
   20 25 30
   Asn Trp Tyr Gln Gln Lys Ser Gly Thr Ser Pro Lys Arg Trp Ile Tyr
   35 40 45
   Asp Thr Ser Lys Val Ala Ser Gly Val Pro Tyr Arg Phe Ser Gly Ser
   50 55 60
   Gly Ser Gly Thr Ser Tyr Ser Leu Thr Ile Ser Ser Met Glu Ala Glu
   65 70 75 80
   Asp Ala Ala Thr Tyr Tyr Cys Gln Gln Trp Ser Ser Asn Pro Leu Thr
   85 90 95
   Phe Gly Ser Gly Thr Lys Leu Glu Ile Asn
   100 105
<210> 1208
   <211> 119
   <212> PRT
   <213> Artificial Sequence
<220>
   <221> source
   <223> /note="Description of Artificial Sequence: Synthetic polypeptide"
<400> 1208
<210> 1209
   <211> 106
   <212> PRT
   <213> Artificial Sequence
<220>
   <221> source
   <223> /note="Description of Artificial Sequence: Synthetic polypeptide"
<400> 1209
<210> 1210
   <211> 118
   <212> PRT
   <213> Artificial Sequence
<220>
   <221> source
   <223> /note="Description of Artificial Sequence: Synthetic polypeptide"
<400> 1210
<210> 1211
   <211> 108
   <212> PRT
   <213> Artificial Sequence
<220>
   <221> source
   <223> /note="Description of Artificial Sequence: Synthetic polypeptide"
<400> 1211
<210> 1212
   <211> 124
   <212> PRT
   <213> Artificial Sequence
<220>
   <221> source
   <223> /note="Description of Artificial Sequence: Synthetic polypeptide"
<400> 1212
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Ala Met Asn Trp Val Arg Gln Ala Pro Gly Lys Gly Leu Glu Trp Val
           35                  40                  45
   Gly Arg Ile Arg Ser Lys Tyr Asn Asn Tyr Ala Thr Tyr Tyr Ala Asp
       50                  55                  60
   Ser Val Lys Asp Arg Phe Ile Ser Arg Asp Asp Ser Lys Asn Ser Leu
   65                  70                  75                  80
   Tyr Leu Gln Met Asn Ser Leu Lys Thr Glu Asp Thr Ala Val Tyr Tyr
                   85                  90                  95
   Cys Val Arg His Gly Asn Phe Gly Asn Ser Tyr Val Ser Trp Phe Ala
               100                 105                 110
   Tyr Trp Gly Gln Gly Thr Leu Val Thr Val Ser Ser
           115                 120
<210> 1213
   <211> 107
   <212> PRT
   <213> Artificial Sequence
<220>
   <221> source
   <223> /note="Description of Artificial Sequence: Synthetic polypeptide"
<400> 1213
   Gln Ala Val Val Thr Gln Glu Pro Ser Leu Thr Val Ser Pro Gly Gly
   1               5                   10                  15
   Thr Val Thr Leu Thr Cys Arg Ser Ser Thr Gly Ala Val Thr Thr Ser
               20                  25                  30
   Asn Tyr Ala Asn Trp Val Gln Gln Lys Pro Gly Gln Ala Pro Arg Gly
           35                  40                  45
   Leu Ile Gly Gly Thr Asn Lys Arg Ala Pro Trp Thr Pro Ala Arg Phe
       50                  55                  60
   Ser Gly Ser Leu Leu Gly Gly Lys Ala Ala Leu Ile Gly Ala Gln Ala
   65                  70                  75                  80
   Glu Asp Glu Ala Asp Tyr Tyr Cys Ala Leu Trp Tyr Ser Asn Leu Trp
                   85                  90                  95
   Val Phe Gly Gly Gly Thr Lys Leu Thr Val Leu
               100                 105
<210> 1214
   <211> 119
   <212> PRT
   <213> Artificial Sequence
<220>
   <221> source
   <223> /note="Description of Artificial Sequence: Synthetic polypeptide"
<400> 1214
   Asp Ile Lys Leu Gln Gln Ser Gly Ala Glu Leu Ala Arg Pro Gly Ala
   1               5                   10                  15
   Ser Val Lys Met Ser Cys Lys Thr Ser Gly Tyr Thr Phe Thr Arg Tyr
               20                  25                  30
   Thr Met His Trp Val Lys Gln Arg Pro Gly Gln Gly Leu Glu Trp Ile
           35                  40                  45
   Gly Tyr Ile Asn Pro Ser Arg Gly Tyr Thr Asn Tyr Asn Gln Lys Phe
       50                  55                  60
   Lys Asp Lys Ala Thr Leu Thr Thr Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Tyr Tyr Asp Asp His Tyr Cys Leu Asp Tyr Trp Gly Gln Gly
               100                 105                 110
   Thr Thr Leu Thr Val Ser Ser
           115
<210> 1215
   <211> 105
   <212> PRT
   <213> Artificial Sequence
<220>
   <221> source
   <223> /note="Description of Artificial Sequence: Synthetic polypeptide"
<400> 1215
   Asp Ile Gln Leu Thr Gln Ser Pro Ala Ile Met Ser Ala Ser Pro Gly
   1               5                   10                  15
   Glu Lys Val Thr Met Thr Cys Arg Ala Ser Ser Ser Val Ser Tyr Met
               20                  25                  30
   Asn Trp Tyr Gln Gln Lys Ser Gly Thr Ser Pro Lys Arg Trp Ile Tyr
           35                  40                  45
   Asp Thr Ser Lys Val Ala Ser Gly Val Pro Tyr Arg Phe Ser Gly Ser
       50                  55                  60
   Gly Ser Gly Thr Ser Tyr Ser Leu Ile Ser Ser Met Glu Ala Glu Asp
   65                  70                  75                  80
   Ala Ala Thr Tyr Tyr Cys Gln Gln Trp Ser Ser Asn Pro Leu Thr Phe
                   85                  90                  95
   Gly Ala Gly Thr Lys Leu Glu Leu Lys
               100                 105
<210> 1216
   <211> 124
   <212> PRT
   <213> Artificial Sequence
<220>
   <221> source
   <223> /note="Description of Artificial Sequence: Synthetic polypeptide"
<400> 1216
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asn Thr Tyr
               20                  25                  30
   Ala Met Asn Trp Val Arg Gln Ala Pro Gly Lys Gly Leu Glu Trp Val
           35                  40                  45
   Ala Arg Ile Arg Ser Lys Tyr Asn Asn Tyr Ala Thr Tyr Tyr Ala Asp
       50                  55                  60
   Ser Val Lys Asp Arg Phe Ile Ser Arg Asp Asp Ser Lys Asn Ser Leu
   65 70 75 80
   Tyr Leu Gln Met Asn Ser Leu Lys Thr Glu Asp Thr Ala Val Tyr Tyr
   85 90 95
   Cys Val Arg His Gly Asn Phe Gly Asn Ser Tyr Val Ser Trp Phe Ala
   100 105 110
   Tyr Trp Gly Gln Gly Thr Leu Val Thr Val Ser Ser
   115 120
<210> 1217
   <211> 107
   <212> PRT
   <213> Artificial Sequence
<220>
   <221> source
   <223> /note="Description of Artificial Sequence: Synthetic polypeptide"
<400> 1217
   Gln Ala Val Val Thr Gln Glu Pro Ser Leu Thr Val Ser Pro Gly Gly
   1 5 10 15
   Thr Val Thr Leu Thr Cys Arg Ser Ser Thr Gly Ala Val Thr Thr Ser
   20 25 30
   Asn Tyr Ala Asn Trp Val Gln Gln Lys Pro Gly Gln Ala Pro Arg Gly
   35 40 45
   Leu Ile Gly Gly Thr Asn Lys Arg Ala Pro Trp Thr Pro Ala Arg Phe
   50 55 60
   Ser Gly Ser Leu Leu Gly Gly Lys Ala Ala Leu Ile Gly Ala Gln Ala
   65 70 75 80
   Glu Asp Glu Ala Asp Tyr Tyr Cys Ala Leu Trp Tyr Ser Asn Leu Trp
   85 90 95
   Val Phe Gly Gly Gly Thr Lys Leu Thr Val Leu
   100 105
<210> 1218
   <211> 125
   <212> PRT
   <213> Artificial Sequence
<220>
   <221> source
   <223> /note="Description of Artificial Sequence: Synthetic polypeptide"
<400> 1218
   Glu Val Lys Leu Leu Glu Ser Gly Gly Gly Leu Val Gln Pro Lys Gly
   1               5                   10                  15
   Ser Leu Lys Leu Ser Cys Ala Ala Ser Gly Phe Thr Phe Asn Thr Tyr
               20                  25                  30
   Ala Met Asn Trp Val Arg Gln Ala Pro Gly Lys Gly Leu Glu Trp Val
           35                  40                  45
   Ala Arg Ile Arg Ser Lys Tyr Asn Asn Tyr Ala Thr Tyr Tyr Ala Asp
       50                  55                  60
   Ser Val Lys Asp Arg Phe Thr Ile Ser Arg Asp Asp Ser Gln Ser Ile
   65                  70                  75                  80
   Leu Tyr Leu Gln Met Asn Asn Leu Lys Thr Glu Asp Thr Ala Met Tyr
                   85                  90                  95
   Tyr Cys Val Arg His Gly Asn Phe Gly Asn Ser Tyr Val Ser Trp Phe
               100                 105                 110
   Ala Tyr Trp Gly Gln Gly Thr Leu Val Thr Val Ser Ala
           115                 120                 125
<210> 1219
   <211> 109
   <212> PRT
   <213> Artificial Sequence
<220>
   <221> source
   <223> /note="Description of Artificial Sequence: Synthetic polypeptide"
<400> 1219
   Gln Ala Val Val Thr Gln Glu Ser Ala Leu Thr Thr Ser Pro Gly Glu
   1               5                   10                  15
   Thr Val Thr Leu Thr Cys Arg Ser Ser Thr Gly Ala Val Thr Thr Ser
               20                  25                  30
   Asn Tyr Ala Asn Trp Val Gln Glu Lys Pro Asp His Leu Phe Thr Gly
           35                  40                  45
   Leu Ile Gly Gly Thr Asn Lys Arg Ala Pro Gly Val Pro Ala Arg Phe
       50                  55                  60
   Ser Gly Ser Leu Ile Gly Asp Lys Ala Ala Leu Thr Ile Thr Gly Ala
   65                  70                  75                  80
   Gln Thr Glu Asp Glu Ala Ile Tyr Phe Cys Ala Leu Trp Tyr Ser Asn
                   85                  90                  95
   Leu Trp Val Phe Gly Gly Gly Thr Lys Leu Thr Val Leu
               100                 105
<210> 1220
   <211> 125
   <212> PRT
   <213> Artificial Sequence
<220>
   <221> source
   <223> /note="Description of Artificial Sequence: Synthetic polypeptide"
<400> 1220
   Glu Val Gln Leu Val Glu Ser Gly Gly Gly Leu Val Gln Pro Gly Gly
   1               5                   10                  15
   Ser Leu Lys Leu Ser Cys Ala Ala Ser Gly Phe Thr Phe Asn Ser Tyr
               20                  25                  30
   Ala Met Asn Trp Val Arg Gln Ala Pro Gly Lys Gly Leu Glu Trp Val
           35                  40                  45
   Ala Arg Ile Arg Ser Lys Tyr Asn Asn Tyr Ala Thr Tyr Tyr Ala Asp
       50                  55                  60
   Ser Val Lys Gly Arg Phe Thr Ile Ser Arg Asp Asp Ser Lys Asn Thr
   65                  70                  75                  80
   Ala Tyr Leu Gln Met Asn Asn Leu Lys Thr Glu Asp Thr Ala Val Tyr
                   85                  90                  95
   Tyr Cys Val Arg His Gly Asn Phe Gly Asn Ser Tyr Val Ser Trp Trp
               100                 105                 110
   Ala Tyr Trp Gly Gln Gly Thr Leu Val Thr Val Ser Ser
           115                 120                 125
<210> 1221
   <211> 109
   <212> PRT
   <213> Artificial Sequence
<220>
   <221> source
   <223> /note="Description of Artificial Sequence: Synthetic polypeptide"
<400> 1221
   Gln Thr Val Val Thr Gln Glu Pro Ser Leu Thr Val Ser Pro Gly Gly
   1               5                   10                  15
   Thr Val Thr Leu Thr Cys Gly Ser Ser Thr Gly Ala Val Thr Ser Gly
               20                  25                  30
   Asn Tyr Pro Asn Trp Val Gln Gln Lys Pro Gly Gln Ala Pro Arg Gly
           35                  40                  45
   Leu Ile Gly Gly Thr Lys Phe Leu Ala Pro Gly Thr Pro Gln Arg Phe
       50                  55                  60
   Ser Gly Ser Leu Leu Gly Gly Lys Ala Ala Leu Thr Leu Ser Gly Val
   65                  70                  75                  80
   Gln Pro Glu Asp Glu Ala Glu Tyr Tyr Cys Val Leu Trp Tyr Ser Asn
                   85                  90                  95
   Arg Trp Val Phe Gly Gly Gly Thr Lys Leu Thr Val Leu
               100                 105
<210> 1222
   <211> 125
   <212> PRT
   <213> Artificial Sequence
<220>
   <221> source
   <223> /note="Description of Artificial Sequence: Synthetic polypeptide"
<400> 1222
   Glu Val Gln Leu Val Glu Ser Gly Gly Gly Leu Val Gln Pro Gly Gly
   1               5                   10                  15
   Ser Leu Lys Leu Ser Cys Ala Ala Ser Gly Phe Thr Phe Asn Lys Tyr
               20                  25                  30
   Ala Met Asn Trp Val Arg Gln Ala Pro Gly Lys Gly Leu Glu Trp Val
           35                  40                  45
   Ala Arg Ile Arg Ser Lys Tyr Asn Asn Tyr Ala Thr Tyr Tyr Ala Asp
       50                  55                  60
   Ser Val Lys Asp Arg Phe Thr Ile Ser Arg Asp Asp Ser Lys Asn Thr
   65                  70                  75                  80
   Ala Tyr Leu Gln Met Asn Asn Leu Lys Thr Glu Asp Thr Ala Val Tyr
                   85                  90                  95
   Tyr Cys Val Arg His Gly Asn Phe Gly Asn Ser Tyr Ile Ser Tyr Trp
   100 105 110
   Ala Tyr Trp Gly Gln Gly Thr Leu Val Thr Val Ser Ser
   115 120 125
<210> 1223
   <211> 109
   <212> PRT
   <213> Artificial Sequence
<220>
   <221> source
   <223> /note="Description of Artificial Sequence: Synthetic polypeptide"
<400> 1223
   Gln Thr Val Val Thr Gln Glu Pro Ser Leu Thr Val Ser Pro Gly Gly
   1 5 10 15
   Thr Val Thr Leu Thr Cys Gly Ser Ser Thr Gly Ala Val Thr Ser Gly
   20 25 30
   Asn Tyr Pro Asn Trp Val Gln Gln Lys Pro Gly Gln Ala Pro Arg Gly
   35 40 45
   Leu Ile Gly Gly Thr Lys Phe Leu Ala Pro Gly Thr Pro Ala Arg Phe
   50 55 60
   Ser Gly Ser Leu Leu Gly Gly Lys Ala Ala Leu Thr Leu Ser Gly Val
   65 70 75 80
   Gln Pro Glu Asp Glu Ala Glu Tyr Tyr Cys Val Leu Trp Tyr Ser Asn
   85 90 95
   Arg Trp Val Phe Gly Gly Gly Thr Lys Leu Thr Val Leu
   100 105
<210> 1224
   <211> 119
   <212> PRT
   <213> Artificial Sequence
<220>
   <221> source
   <223> /note="Description of Artificial Sequence: Synthetic polypeptide"
<400> 1224
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Arg Tyr
   20 25 30
   Thr Met His Trp Val Arg Gln Ala Pro Gly Gln Gly Leu Glu Trp Met
   35 40 45
   Gly Tyr Ile Asn Pro Ser Arg Gly Tyr Thr Asn Tyr Asn Gln Lys Phe
   50 55 60
   Lys Asp Arg Val Thr Met Thr Thr Asp Thr Ser Ile Ser Thr Ala Tyr
   65 70 75 80
   Met Glu Leu Ser Arg Leu Arg Ser Asp Asp Thr Ala Val Tyr Tyr Cys
   85 90 95
   Ala Arg Tyr Tyr Asp Asp His Tyr Cys Leu Asp Tyr Trp Gly Gln Gly
   100 105 110
   Thr Leu Val Thr Val Ser Ser
   115
<210> 1225
   <211> 106
   <212> PRT
   <213> Artificial Sequence
<220>
   <221> source
   <223> /note="Description of Artificial Sequence: Synthetic polypeptide"
<400> 1225
   Glu Ile Val Leu Thr Gln Ser Pro Ala Thr Leu Ser Leu Ser Pro Gly
   1 5 10 15
   Glu Arg Ala Thr Leu Ser Cys Ser Ala Ser Ser Ser Val Ser Tyr Met
   20 25 30
   Asn Trp Tyr Gln Gln Lys Pro Gly Gln Ala Pro Arg Leu Leu Ile Tyr
   35 40 45
   Asp Thr Ser Lys Leu Ala Ser Gly Val Pro Ala His Phe Arg Gly Ser
   50 55 60
   Gly Ser Gly Thr Asp Phe Thr Leu Thr Ile Ser Ser Leu Glu Pro Glu
   65 70 75 80
   Asp Phe Ala Val Tyr Tyr Cys Gln Gln Trp Ser Ser Asn Pro Phe Thr
   85 90 95
   Phe Gly Gln Gly Thr Lys Val Glu Ile Lys
   100 105
<210> 1226
   <211> 125
   <212> PRT
   <213> Artificial Sequence
<220>
   <221> source
   <223> /note="Description of Artificial Sequence: Synthetic polypeptide"
<400> 1226
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Thr Tyr
   20 25 30
   Ala Met Asn Trp Val Arg Gln Ala Pro Gly Lys Gly Leu Glu Trp Val
   35 40 45
   Ser Arg Ile Arg Ser Lys Tyr Asn Asn Tyr Ala Thr Tyr Tyr Ala Asp
   50 55 60
   Ser Val Lys Asp Arg Phe Thr Ile Ser Arg Asp Asp Ser Lys Asn Thr
   65 70 75 80
   Leu Tyr Leu Gln Met Asn Ser Leu Arg Ala Glu Asp Thr Ala Val Tyr
   85 90 95
   Tyr Cys Ala Arg His Gly Asn Phe Gly Asn Ser Tyr Val Ser Trp Phe
   100 105 110
   Ala Tyr Trp Gly Gln Gly Thr Met Val Thr Val Ser Ser
   115 120 125
<210> 1227
   <211> 109
   <212> PRT
   <213> Artificial Sequence
<220>
   <221> source
   <223> /note="Description of Artificial Sequence: Synthetic polypeptide"
<400> 1227
   Gln Ala Val Val Thr Gln Glu Pro Ser Leu Thr Val Ser Pro Gly Gly
   1               5                   10                  15
   Thr Val Thr Leu Thr Cys Arg Ser Ser Thr Gly Ala Val Thr Thr Ser
               20                  25                  30
   Asn Tyr Ala Asn Trp Val Gln Gln Lys Pro Gly Gln Ala Pro Arg Gly
           35                  40                  45
   Leu Ile Gly Gly Thr Asn Lys Arg Ala Pro Gly Thr Pro Ala Arg Phe
       50                  55                  60
   Ser Gly Ser Leu Leu Gly Gly Lys Ala Ala Leu Thr Leu Ser Gly Val
   65                  70                  75                  80
   Gln Pro Glu Asp Glu Ala Glu Tyr Tyr Cys Ala Leu Trp Tyr Ser Asn
                   85                  90                  95
   Leu Trp Val Phe Gly Gly Gly Thr Lys Leu Thr Val Leu
               100                 105
<210> 1228
   <211> 125
   <212> PRT
   <213> Artificial Sequence
<220>
   <221> source
   <223> /note="Description of Artificial Sequence: Synthetic polypeptide"
<400> 1228
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asn Thr Tyr
               20                  25                  30
   Ala Met Asn Trp Val Arg Gln Ala Pro Gly Lys Gly Leu Glu Trp Val
           35                  40                  45
   Gly Arg Ile Arg Ser Lys Tyr Asn Asn Tyr Ala Thr Tyr Tyr Ala Asp
       50                  55                  60
   Ser Val Lys Asp Arg Phe Thr Ile Ser Arg Asp Asp Ser Lys Asn Ser
   65                  70                  75                  80
   Leu Tyr Leu Gln Met Asn Ser Leu Lys Thr Glu Asp Thr Ala Val Tyr
                   85                  90                  95
   Tyr Cys Val Arg His Gly Asn Phe Gly Asn Ser Tyr Val Ser Trp Phe
               100                 105                 110
   Ala Tyr Trp Gly Gln Gly Thr Leu Val Thr Val Ser Ser
             115                 120                 125
<210> 1229
   <211> 109
   <212> PRT
   <213> Artificial Sequence
<220>
   <221> source
   <223> /note="Description of Artificial Sequence: Synthetic polypeptide"
<400> 1229
   Gln Ala Val Val Thr Gln Glu Pro Ser Leu Thr Val Ser Pro Gly Gly
   1               5                   10                  15
   Thr Val Thr Leu Thr Cys Arg Ser Ser Thr Gly Ala Val Thr Thr Ser
               20                  25                  30
   Asn Tyr Ala Asn Trp Val Gln Glu Lys Pro Gly Gln Ala Pro Arg Gly
           35                  40                  45
   Leu Ile Gly Gly Thr Asn Lys Arg Ala Pro Trp Thr Pro Ala Arg Phe
       50                  55                  60
   Ser Gly Ser Leu Leu Gly Gly Lys Ala Ala Leu Thr Ile Thr Gly Ala
   65                  70                  75                  80
   Gln Ala Glu Asp Glu Ala Asp Tyr Tyr Cys Ala Leu Trp Tyr Ser Asn
                   85                  90                  95
   Leu Trp Val Phe Gly Gly Gly Thr Lys Leu Thr Val Leu
               100                 105
<210> 1230
   <211> 125
   <212> PRT
   <213> Artificial Sequence
<220>
   <221> source
   <223> /note="Description of Artificial Sequence: Synthetic polypeptide"
<400> 1230
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asn Thr Tyr
               20                  25                  30
   Ala Met Asn Trp Val Arg Gln Ala Pro Gly Lys Gly Leu Glu Trp Val
           35                  40                  45
   Gly Arg Ile Arg Ser Lys Tyr Asn Asn Tyr Ala Thr Tyr Tyr Ala Asp
       50                  55                  60
   Ser Val Lys Gly Arg Phe Thr Ile Ser Arg Asp Asp Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Val Arg His Gly Asn Phe Gly Asn Ser Tyr Val Ser Trp Phe
               100                 105                 110
   Ala Tyr Trp Gly Gln Gly Thr Leu Val Thr Val Ser Ser
           115                 120                 125
<210> 1231
   <211> 109
   <212> PRT
   <213> Artificial Sequence
<220>
   <221> source
   <223> /note="Description of Artificial Sequence: Synthetic polypeptide"
<400> 1231
   Gln Ala Val Val Thr Gln Glu Pro Ser Leu Thr Val Ser Pro Gly Gly
   1               5                   10                  15
   Thr Val Thr Leu Thr Cys Gly Ser Ser Thr Gly Ala Val Thr Thr Ser
               20                  25                  30
   Asn Tyr Ala Asn Trp Val Gln Gln Lys Pro Gly Gln Ala Pro Arg Gly
           35                  40                  45
   Leu Ile Gly Gly Thr Asn Lys Arg Ala Pro Gly Val Pro Ala Arg Phe
       50                  55                  60
   Ser Gly Ser Leu Leu Gly Gly Lys Ala Ala Leu Thr Leu Ser Gly Ala
   65                  70                  75                  80
   Gln Pro Glu Asp Glu Ala Glu Tyr Tyr Cys Ala Leu Trp Tyr Ser Asn
                   85                  90                  95
   Leu Trp Val Phe Gly Gly Gly Thr Lys Leu Thr Val Leu
               100                 105
<210> 1232
   <211> 125
   <212> PRT
   <213> Artificial Sequence
<220>
   <221> source
   <223> /note="Description of Artificial Sequence: Synthetic polypeptide"
<400> 1232
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Ala Met Asn Trp Val Arg Gln Ala Pro Gly Lys Gly Leu Glu Trp Val
           35                  40                  45
   Gly Arg Ile Arg Ser Lys Tyr Asn Asn Tyr Ala Thr Tyr Tyr Ala Asp
       50                  55                  60
   Ser Val Lys Gly Arg Phe Thr Ile Ser Arg Asp Asp Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Val Arg His Gly Asn Phe Gly Asp Ser Tyr Val Ser Trp Phe
               100                 105                 110
   Ala Tyr Trp Gly Gln Gly Thr Leu Val Thr Val Ser Ser
           115                 120                 125
<210> 1233
   <211> 109
   <212> PRT
   <213> Artificial Sequence
<220>
   <221> source
   <223> /note="Description of Artificial Sequence: Synthetic polypeptide"
<400> 1233
   Gln Ala Val Val Thr Gln Glu Pro Ser Leu Thr Val Ser Pro Gly Gly
   1 5 10 15
   Thr Val Thr Leu Thr Cys Gly Ser Ser Thr Gly Ala Val Thr Thr Ser
   20 25 30
   Asn Tyr Ala Asn Trp Val Gln Gln Lys Pro Gly Lys Ser Pro Arg Gly
   35 40 45
   Leu Ile Gly Gly Thr Asn Lys Arg Ala Pro Gly Val Pro Ala Arg Phe
   50 55 60
   Ser Gly Ser Leu Leu Gly Gly Lys Ala Ala Leu Thr Ile Ser Gly Ala
   65 70 75 80
   Gln Pro Glu Asp Glu Ala Asp Tyr Tyr Cys Ala Leu Trp Tyr Ser Asn
   85 90 95
   His Trp Val Phe Gly Gly Gly Thr Lys Leu Thr Val Leu
   100 105
<210> 1234
   <211> 119
   <212> PRT
   <213> Artificial Sequence
<220>
   <221> source
   <223> /note="Description of Artificial Sequence: Synthetic polypeptide"
<400> 1234
<210> 1235
   <211> 101
   <212> PRT
   <213> Artificial Sequence
<220>
   <221> source
   <223> /note="Description of Artificial Sequence: Synthetic polypeptide"
<400> 1235
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Ser Ser Val Ser Tyr Met
               20                  25                  30
   Asn Trp Tyr Gln Gln Thr Pro Gly Lys Ala Pro Lys Arg Trp Ile Tyr
           35                  40                  45
   Asp Thr Ser Lys Leu Ala Ser Gly Val Pro Ser Arg Phe Ser Gly Ser
       50                  55                  60
   Gly Ser Gly Thr Asp Tyr Thr Phe Thr Ile Ser Ser Leu Gln Pro Glu
   65                  70                  75                  80
   Asp Ile Ala Thr Tyr Tyr Cys Gln Gln Trp Ser Ser Asn Pro Phe Thr
                   85                  90                  95
   Phe Gly Gln Gly Thr
               100
<210> 1236
   <211> 119
   <212> PRT
   <213> Artificial Sequence
<220>
   <221> source
   <223> /note="Description of Artificial Sequence: Synthetic polypeptide"
<400> 1236
   Gln Val Gln Leu Val Gln Ser Gly Gly Gly Val Val Gln Pro Gly Arg
   1               5                   10                  15
   Ser Leu Arg Leu Ser Cys Lys Ala Ser Gly Tyr Thr Phe Thr Arg Tyr
               20                  25                  30
   Thr Met His Trp Val Arg Gln Ala Pro Gly Lys Gly Leu Glu Trp Ile
           35                  40                  45
   Gly Tyr Ile Asn Pro Ser Arg Gly Tyr Thr Asn Tyr Asn Gln Lys Val
       50                  55                  60
   Lys Asp Arg Phe Thr Ile Ser Arg Asp Asn Ser Lys Asn Thr Ala Phe
   65 70 75 80
   Leu Gln Met Asp Ser Leu Arg Pro Glu Asp Thr Gly Val Tyr Phe Cys
   85 90 95
   Ala Arg Tyr Tyr Asp Asp His Tyr Ser Leu Asp Tyr Trp Gly Gln Gly
   100 105 110
   Thr Pro Val Thr Val Ser Ser
   115
<210> 1237
   <211> 101
   <212> PRT
   <213> Artificial Sequence
<220>
   <221> source
   <223> /note="Description of Artificial Sequence: Synthetic polypeptide"
<400> 1237
   Asp Ile Gln Met Thr Gln Ser Pro Ser Ser Leu Ser Ala Ser Val Gly
   1 5 10 15
   Asp Arg Val Thr Ile Thr Cys Ser Ala Ser Ser Ser Val Ser Tyr Met
   20 25 30
   Asn Trp Tyr Gln Gln Thr Pro Gly Lys Ala Pro Lys Arg Trp Ile Tyr
   35 40 45
   Asp Thr Ser Lys Leu Ala Ser Gly Val Pro Ser Arg Phe Ser Gly Ser
   50 55 60
   Gly Ser Gly Thr Asp Tyr Thr Phe Thr Ile Ser Ser Leu Gln Pro Glu
   65 70 75 80
   Asp Ile Ala Thr Tyr Tyr Cys Gln Gln Trp Ser Ser Asn Pro Phe Thr
   85 90 95
   Phe Gly Gln Gly Thr
   100
<210> 1238
<400> 1238
   000
<210> 1239
<400> 1239
   000
<210> 1240
<400> 1240
   000
<210> 1241
<400> 1241
   000
<210> 1242
<400> 1242
   000
<210> 1243
<400> 1243
   000
<210> 1244
<400> 1244
   000
<210> 1245
<400> 1245
   000
<210> 1246
<400> 1246
   000
<210> 1247
<400> 1247
   000
<210> 1248
<400> 1248
   000
<210> 1249
<400> 1249
   000
<210> 1250
<400> 1250
   000
<210> 1251
<400> 1251
   000
<210> 1252
<400> 1252
   000
<210> 1253
<400> 1253
   000
<210> 1254
<400> 1254
   000
<210> 1255
<400> 1255
   000
<210> 1256
<400> 1256
   000
<210> 1257
<400> 1257
   000
<210> 1258
<400> 1258
   000
<210> 1259
<400> 1259
   000
<210> 1260
<400> 1260
   000
<210> 1261
<400> 1261
   000
<210> 1262
<400> 1262
   000
<210> 1263
<400> 1263
   000
<210> 1264
<400> 1264
   000
<210> 1265
<400> 1265
   000
<210> 1266
<400> 1266
   000
<210> 1267
<400> 1267
   000
<210> 1268
<400> 1268
   000
<210> 1269
<400> 1269
   000
<210> 1270
<400> 1270
   000
<210> 1271
<400> 1271
   000
<210> 1272
<400> 1272
   000
<210> 1273
<400> 1273
   000
<210> 1274
<400> 1274
   000
<210> 1275
<400> 1275
   000
<210> 1276
<400> 1276
   000
<210> 1277
<400> 1277
   000
<210> 1278
<400> 1278
   000
<210> 1279
<400> 1279
   000
<210> 1280
<400> 1280
   000
<210> 1281
<400> 1281
   000
<210> 1282
<400> 1282
   000
<210> 1283
<400> 1283
   000
<210> 1284
<400> 1284
   000
<210> 1285
<400> 1285
   000
<210> 1286
<400> 1286
   000
<210> 1287
<400> 1287
   000
<210> 1288
<400> 1288
   000
<210> 1289
<400> 1289
   000
<210> 1290
<400> 1290
   000
<210> 1291
<400> 1291
   000
<210> 1292
<400> 1292
   000
<210> 1293
<400> 1293
   000
<210> 1294
<400> 1294
   000
<210> 1295
<400> 1295
   000
<210> 1296
<400> 1296
   000
<210> 1297
<400> 1297
   000
<210> 1298
<400> 1298
   000
<210> 1299
<400> 1299
   000
<210> 1300
   <211> 10
   <212> PRT
   <213> Artificial Sequence
<220>
   <221> source
   <223> /note="Description of Artificial Sequence: Synthetic peptide"
<400> 1300
   Gly Tyr Thr Phe Thr Arg Tyr Thr Met His
   1 5 10
<210> 1301
   <211> 10
   <212> PRT
   <213> Artificial Sequence
<220>
   <221> source
   <223> /note="Description of Artificial Sequence: Synthetic peptide"
<400> 1301
   Ser Ala Ser Ser Ser Val Ser Tyr Met Asn
   1 5 10
<210> 1302
   <211> 10
   <212> PRT
   <213> Artificial Sequence
<220>
   <221> source
   <223> /note="Description of Artificial Sequence: Synthetic peptide"
<400> 1302
   Gly Phe Thr Phe Asn Thr Tyr Ala Met Asn
   1 5 10
<210> 1303
   <211> 14
   <212> PRT
   <213> Artificial Sequence
<220>
   <221> source
   <223> /note="Description of Artificial Sequence: Synthetic peptide"
<400> 1303
   Arg Ser Ser Thr Gly Ala Val Thr Thr Ser Asn Tyr Ala Asn
   1 5 10
<210> 1304
   <211> 10
   <212> PRT
   <213> Artificial Sequence
<220>
   <221> source
   <223> /note="Description of Artificial Sequence: Synthetic peptide"
<400> 1304
   Gly Tyr Thr Phe Thr Ser Tyr Thr Met His
   1 5 10
<210> 1305
   <211> 10
   <212> PRT
   <213> Artificial Sequence
<220>
   <221> source
   <223> /note="Description of Artificial Sequence: Synthetic peptide"
<400> 1305
   Arg Ala Ser Ser Ser Val Ser Tyr Met His
   1 5 10
<210> 1306
   <211> 10
   <212> PRT
   <213> Artificial Sequence
<220>
   <221> source
   <223> /note="Description of Artificial Sequence: Synthetic peptide"
<400> 1306
   Gly Tyr Thr Phe Thr Arg Tyr Thr Met His
   1 5 10
<210> 1307
   <211> 10
   <212> PRT
   <213> Artificial Sequence
<220>
   <221> source
   <223> /note="Description of Artificial Sequence: Synthetic peptide"
<400> 1307
   Arg Ala Ser Ser Ser Val Ser Tyr Met Asn
   1 5 10
<210> 1308
   <211> 10
   <212> PRT
   <213> Artificial Sequence
<220>
   <221> source
   <223> /note="Description of Artificial Sequence: Synthetic peptide"
<400> 1308
   Gly Tyr Thr Phe Thr Arg Tyr Thr Met His
   1 5 10
<210> 1309
   <211> 10
   <212> PRT
   <213> Artificial Sequence
<220>
   <221> source
   <223> /note="Description of Artificial Sequence: Synthetic peptide"
<400> 1309
   Ser Ala Ser Ser Ser Val Ser Tyr Met Asn
   1 5 10
<210> 1310
   <211> 10
   <212> PRT
   <213> Artificial Sequence
<220>
   <221> source
   <223> /note="Description of Artificial Sequence: Synthetic peptide"
<400> 1310
   Gly Phe Lys Phe Ser Gly Tyr Gly Met His
   1 5 10
<210> 1311
   <211> 11
   <212> PRT
   <213> Artificial Sequence
<220>
   <221> source
   <223> /note="Description of Artificial Sequence: Synthetic peptide"
<400> 1311
   Arg Ala Ser Gln Ser Val Ser Ser Tyr Leu Ala
   1 5 10
<210> 1312
   <211> 10
   <212> PRT
   <213> Artificial Sequence
<220>
   <221> source
   <223> /note="Description of Artificial Sequence: Synthetic peptide"
<400> 1312
   Gly Phe Thr Phe Ser Thr Tyr Ala Met Asn
   1 5 10
<210> 1313
   <211> 14
   <212> PRT
   <213> Artificial Sequence
<220>
   <221> source
   <223> /note="Description of Artificial Sequence: Synthetic peptide"
<400> 1313
   Arg Ser Ser Thr Gly Ala Val Thr Thr Ser Asn Tyr Ala Asn
   1 5 10
<210> 1314
   <211> 10
   <212> PRT
   <213> Artificial Sequence
<220>
   <221> source
   <223> /note="Description of Artificial Sequence: Synthetic peptide"
<400> 1314
   Gly Tyr Thr Phe Thr Arg Tyr Thr Met His
   1 5 10
<210> 1315
   <211> 10
   <212> PRT
   <213> Artificial Sequence
<220>
   <221> source
   <223> /note="Description of Artificial Sequence: Synthetic peptide"
<400> 1315
   Arg Ala Ser Ser Ser Val Ser Tyr Met Asn
   1 5 10
<210> 1316
   <211> 10
   <212> PRT
   <213> Artificial Sequence
<220>
   <221> source
   <223> /note="Description of Artificial Sequence: Synthetic peptide"
<400> 1316
   Gly Phe Thr Phe Asn Thr Tyr Ala Met Asn
   1 5 10
<210> 1317
   <211> 14
   <212> PRT
   <213> Artificial Sequence
<220>
   <221> source
   <223> /note="Description of Artificial Sequence: Synthetic peptide"
<400> 1317
   Arg Ser Ser Thr Gly Ala Val Thr Thr Ser Asn Tyr Ala Asn
   1 5 10
<210> 1318
   <211> 10
   <212> PRT
   <213> Artificial Sequence
<220>
   <221> source
   <223> /note="Description of Artificial Sequence: Synthetic peptide"
<400> 1318
   Gly Phe Thr Phe Asn Thr Tyr Ala Met Asn
   1 5 10
<210> 1319
   <211> 14
   <212> PRT
   <213> Artificial Sequence
<220>
   <221> source
   <223> /note="Description of Artificial Sequence: Synthetic peptide"
<400> 1319
   Arg Ser Ser Thr Gly Ala Val Thr Thr Ser Asn Tyr Ala Asn
   1 5 10
<210> 1320
   <211> 10
   <212> PRT
   <213> Artificial Sequence
<220>
   <221> source
   <223> /note="Description of Artificial Sequence: Synthetic peptide"
<400> 1320
   Gly Phe Thr Phe Asn Ser Tyr Ala Met Asn
   1 5 10
<210> 1321
   <211> 14
   <212> PRT
   <213> Artificial Sequence
<220>
   <221> source
   <223> /note="Description of Artificial Sequence: Synthetic peptide"
<400> 1321
   Gly Ser Ser Thr Gly Ala Val Thr Ser Gly Asn Tyr Pro Asn
   1 5 10
<210> 1322
   <211> 10
   <212> PRT
   <213> Artificial Sequence
<220>
   <221> source
   <223> /note="Description of Artificial Sequence: Synthetic peptide"
<400> 1322
   Gly Phe Thr Phe Asn Lys Tyr Ala Met Asn
   1 5 10
<210> 1323
   <211> 14
   <212> PRT
   <213> Artificial Sequence
<220>
   <221> source
   <223> /note="Description of Artificial Sequence: Synthetic peptide"
<400> 1323
   Gly Ser Ser Thr Gly Ala Val Thr Ser Gly Asn Tyr Pro Asn
   1 5 10
<210> 1324
   <211> 10
   <212> PRT
   <213> Artificial Sequence
<220>
   <221> source
   <223> /note="Description of Artificial Sequence: Synthetic peptide"
<400> 1324
   Gly Tyr Thr Phe Thr Arg Tyr Thr Met His
   1 5 10
<210> 1325
   <211> 10
   <212> PRT
   <213> Artificial Sequence
<220>
   <221> source
   <223> /note="Description of Artificial Sequence: Synthetic peptide"
<400> 1325
   Ser Ala Ser Ser Ser Val Ser Tyr Met Asn
   1 5 10
<210> 1326
   <211> 10
   <212> PRT
   <213> Artificial Sequence
<220>
   <221> source
   <223> /note="Description of Artificial Sequence: Synthetic peptide"
<400> 1326
   Gly Phe Thr Phe Ser Thr Tyr Ala Met Asn
   1 5 10
<210> 1327
   <211> 14
   <212> PRT
   <213> Artificial Sequence
<220>
   <221> source
   <223> /note="Description of Artificial Sequence: Synthetic peptide"
<400> 1327
   Arg Ser Ser Thr Gly Ala Val Thr Thr Ser Asn Tyr Ala Asn
   1 5 10
<210> 1328
   <211> 10
   <212> PRT
   <213> Artificial Sequence
<220>
   <221> source
   <223> /note="Description of Artificial Sequence: Synthetic peptide"
<400> 1328
   Gly Phe Thr Phe Asn Thr Tyr Ala Met Asn
   1               5                   10
<210> 1329
   <211> 14
   <212> PRT
   <213> Artificial Sequence
<220>
   <221> source
   <223> /note="Description of Artificial Sequence: Synthetic peptide"
<400> 1329
   Arg Ser Ser Thr Gly Ala Val Thr Thr Ser Asn Tyr Ala Asn
   1               5                   10
<210> 1330
   <211> 10
   <212> PRT
   <213> Artificial Sequence
<220>
   <221> source
   <223> /note="Description of Artificial Sequence: Synthetic peptide"
<400> 1330
   Gly Phe Thr Phe Asn Thr Tyr Ala Met Asn
   1               5                   10
<210> 1331
   <211> 14
   <212> PRT
   <213> Artificial Sequence
<220>
   <221> source
   <223> /note="Description of Artificial Sequence: Synthetic peptide"
<400> 1331
   Gly Ser Ser Thr Gly Ala Val Thr Thr Ser Asn Tyr Ala Asn
   1               5                   10
<210> 1332
   <211> 10
   <212> PRT
   <213> Artificial Sequence
<220>
   <221> source
   <223> /note="Description of Artificial Sequence: Synthetic peptide"
<400> 1332
   Gly Phe Thr Phe Ser Thr Tyr Ala Met Asn
   1               5                   10
<210> 1333
   <211> 14
   <212> PRT
   <213> Artificial Sequence
<220>
   <221> source
   <223> /note="Description of Artificial Sequence: Synthetic peptide"
<400> 1333
   Gly Ser Ser Thr Gly Ala Val Thr Thr Ser Asn Tyr Ala Asn
   1               5                   10
<210> 1334
   <211> 10
   <212> PRT
   <213> Artificial Sequence
<220>
   <221> source
   <223> /note="Description of Artificial Sequence: Synthetic peptide"
<400> 1334
   Gly Tyr Thr Phe Thr Arg Tyr Thr Met His
   1               5                   10
<210> 1335
   <211> 10
   <212> PRT
   <213> Artificial Sequence
<220>
   <221> source
   <223> /note="Description of Artificial Sequence: Synthetic peptide"
<400> 1335
   Ser Ala Ser Ser Ser Val Ser Tyr Met Asn
   1               5                   10
<210> 1336
   <211> 10
   <212> PRT
   <213> Artificial Sequence
<220>
   <221> source
   <223> /note="Description of Artificial Sequence: Synthetic peptide"
<400> 1336
   Gly Tyr Thr Phe Thr Arg Tyr Thr Met His
   1 5 10
<210> 1337
   <211> 10
   <212> PRT
   <213> Artificial Sequence
<220>
   <221> source
   <223> /note="Description of Artificial Sequence: Synthetic peptide"
<400> 1337
   Ser Ala Ser Ser Ser Val Ser Tyr Met Asn
   1 5 10
<210> 1338
   <211> 17
   <212> PRT
   <213> Artificial Sequence
<220>
   <221> source
   <223> /note="Description of Artificial Sequence: Synthetic peptide"
<400> 1338
   Tyr Ile Asn Pro Ser Arg Gly Tyr Thr Asn Tyr Asn Gln Lys Phe Lys
   1 5 10 15
   Asp
<210> 1339
   <211> 7
   <212> PRT
   <213> Artificial Sequence
<220>
   <221> source
   <223> /note="Description of Artificial Sequence: Synthetic peptide"
<400> 1339
   Asp Thr Ser Lys Leu Ala Ser
   1 5
<210> 1340
   <211> 19
   <212> PRT
   <213> Artificial Sequence
<220>
   <221> source
   <223> /note="Description of Artificial Sequence: Synthetic peptide"
<400> 1340
   Arg Ile Arg Ser Lys Tyr Asn Asn Tyr Ala Thr Tyr Tyr Ala Asp Ser
   1               5                   10                  15
   Val Lys Asp
<210> 1341
   <211> 7
   <212> PRT
   <213> Artificial Sequence
<220>
   <221> source
   <223> /note="Description of Artificial Sequence: Synthetic peptide"
<400> 1341
   Gly Thr Asn Lys Arg Ala Pro
   1               5
<210> 1342
   <211> 17
   <212> PRT
   <213> Artificial Sequence
<220>
   <221> source
   <223> /note="Description of Artificial Sequence: Synthetic peptide"
<400> 1342
   Tyr Ile Asn Pro Ser Ser Gly Tyr Thr Lys Tyr Asn Gln Lys Phe Lys
   1               5                   10                  15
   Asp
<210> 1343
   <211> 7
   <212> PRT
   <213> Artificial Sequence
<220>
   <221> source
   <223> /note="Description of Artificial Sequence: Synthetic peptide"
<400> 1343
   Ala Thr Ser Asn Leu Ala Ser
   1               5
<210> 1344
   <211> 17
   <212> PRT
   <213> Artificial Sequence
<220>
   <221> source
   <223> /note="Description of Artificial Sequence: Synthetic peptide"
<400> 1344
   Tyr Ile Asn Pro Ser Arg Gly Tyr Thr Asn Tyr Asn Gln Lys Phe Lys
   1               5                   10                  15
   Asp
<210> 1345
   <211> 7
   <212> PRT
   <213> Artificial Sequence
<220>
   <221> source
   <223> /note="Description of Artificial Sequence: Synthetic peptide"
<400> 1345
   Asp Thr Ser Lys Val Ala Ser
   1               5
<210> 1346
   <211> 17
   <212> PRT
   <213> Artificial Sequence
<220>
   <221> source
   <223> /note="Description of Artificial Sequence: Synthetic peptide"
<400> 1346
   Tyr Ile Asn Pro Ser Arg Gly Tyr Thr Asn Tyr Asn Gln Lys Val Lys
   1               5                   10                  15
   Asp
<210> 1347
   <211> 7
   <212> PRT
   <213> Artificial Sequence
<220>
   <221> source
   <223> /note="Description of Artificial Sequence: Synthetic peptide"
<400> 1347
   Asp Thr Ser Lys Leu Ala Ser
   1               5
<210> 1348
   <211> 17
   <212> PRT
   <213> Artificial Sequence
<220>
   <221> source
   <223> /note="Description of Artificial Sequence: Synthetic peptide"
<400> 1348
   Val Ile Trp Tyr Asp Gly Ser Lys Lys Tyr Tyr Val Asp Ser Val Lys
   1 5 10 15
   Gly
<210> 1349
   <211> 7
   <212> PRT
   <213> Artificial Sequence
<220>
   <221> source
   <223> /note="Description of Artificial Sequence: Synthetic peptide"
<400> 1349
   Asp Ala Ser Asn Arg Ala Thr
   1 5
<210> 1350
   <211> 18
   <212> PRT
   <213> Artificial Sequence
<220>
   <221> source
   <223> /note="Description of Artificial Sequence: Synthetic peptide"
<400> 1350
   Arg Ile Arg Ser Lys Tyr Asn Asn Tyr Ala Thr Tyr Tyr Ala Asp Ser
   1 5 10 15
   Val Lys
<210> 1351
   <211> 7
   <212> PRT
   <213> Artificial Sequence
<220>
   <221> source
   <223> /note="Description of Artificial Sequence: Synthetic peptide"
<400> 1351
   Gly Thr Asn Lys Arg Ala Pro
   1 5
<210> 1352
   <211> 17
   <212> PRT
   <213> Artificial Sequence
<220>
   <221> source
   <223> /note="Description of Artificial Sequence: Synthetic peptide"
<400> 1352
   Tyr Ile Asn Pro Ser Arg Gly Tyr Thr Asn Tyr Asn Gln Lys Phe Lys
   1 5 10 15
   Asp
<210> 1353
   <211> 7
   <212> PRT
   <213> Artificial Sequence
<220>
   <221> source
   <223> /note="Description of Artificial Sequence: Synthetic peptide"
<400> 1353
   Asp Thr Ser Lys Val Ala Ser
   1 5
<210> 1354
   <211> 18
   <212> PRT
   <213> Artificial Sequence
<220>
   <221> source
   <223> /note="Description of Artificial Sequence: Synthetic peptide"
<400> 1354
   Arg Ile Arg Ser Lys Tyr Asn Asn Tyr Ala Thr Tyr Tyr Ala Asp Ser
   1 5 10 15
   Val Lys
<210> 1355
   <211> 7
   <212> PRT
   <213> Artificial Sequence
<220>
   <221> source
   <223> /note="Description of Artificial Sequence: Synthetic peptide"
<400> 1355
   Gly Thr Asn Lys Arg Ala Pro
   1 5
<210> 1356
   <211> 19
   <212> PRT
   <213> Artificial Sequence
<220>
   <221> source
   <223> /note="Description of Artificial Sequence: Synthetic peptide"
<400> 1356
   Arg Ile Arg Ser Lys Tyr Asn Asn Tyr Ala Thr Tyr Tyr Ala Asp Ser
   1 5 10 15
   Val Lys Asp
<210> 1357
   <211> 7
   <212> PRT
   <213> Artificial Sequence
<220>
   <221> source
   <223> /note="Description of Artificial Sequence: Synthetic peptide"
<400> 1357
   Gly Thr Asn Lys Arg Ala Pro
   1 5
<210> 1358
   <211> 19
   <212> PRT
   <213> Artificial Sequence
<220>
   <221> source
   <223> /note="Description of Artificial Sequence: Synthetic peptide"
<400> 1358
   Arg Ile Arg Ser Lys Tyr Asn Asn Tyr Ala Thr Tyr Tyr Ala Asp Ser
   1 5 10 15
   Val Lys Gly
<210> 1359
   <211> 7
   <212> PRT
   <213> Artificial Sequence
<220>
   <221> source
   <223> /note="Description of Artificial Sequence: Synthetic peptide"
<400> 1359
   Gly Thr Lys Phe Leu Ala Pro
   1 5
<210> 1360
   <211> 19
   <212> PRT
   <213> Artificial Sequence
<220>
   <221> source
   <223> /note="Description of Artificial Sequence: Synthetic peptide"
<400> 1360
   Arg Ile Arg Ser Lys Tyr Asn Asn Tyr Ala Thr Tyr Tyr Ala Asp Ser
   1 5 10 15
   Val Lys Asp
<210> 1361
   <211> 7
   <212> PRT
   <213> Artificial Sequence
<220>
   <221> source
   <223> /note="Description of Artificial Sequence: Synthetic peptide"
<400> 1361
   Gly Thr Lys Phe Leu Ala Pro
   1 5
<210> 1362
   <211> 17
   <212> PRT
   <213> Artificial Sequence
<220>
   <221> source
   <223> /note="Description of Artificial Sequence: Synthetic peptide"
<400> 1362
   Tyr Ile Asn Pro Ser Arg Gly Tyr Thr Asn Tyr Asn Gln Lys Phe Lys
   1 5 10 15
   Asp
<210> 1363
   <211> 7
   <212> PRT
   <213> Artificial Sequence
<220>
   <221> source
   <223> /note="Description of Artificial Sequence: Synthetic peptide"
<400> 1363
   Asp Thr Ser Lys Leu Ala Ser
   1               5
<210> 1364
   <211> 19
   <212> PRT
   <213> Artificial Sequence
<220>
   <221> source
   <223> /note="Description of Artificial Sequence: Synthetic peptide"
<400> 1364
   Arg Ile Arg Ser Lys Tyr Asn Asn Tyr Ala Thr Tyr Tyr Ala Asp Ser
   1               5                   10                  15
   Val Lys Asp
<210> 1365
   <211> 7
   <212> PRT
   <213> Artificial Sequence
<220>
   <221> source
   <223> /note="Description of Artificial Sequence: Synthetic peptide"
<400> 1365
   Gly Thr Asn Lys Arg Ala Pro
   1               5
<210> 1366
   <211> 19
   <212> PRT
   <213> Artificial Sequence
<220>
   <221> source
   <223> /note="Description of Artificial Sequence: Synthetic peptide"
<400> 1366
   Arg Ile Arg Ser Lys Tyr Asn Asn Tyr Ala Thr Tyr Tyr Ala Asp Ser
   1 5 10 15
   Val Lys Asp
<210> 1367
   <211> 7
   <212> PRT
   <213> Artificial Sequence
<220>
   <221> source
   <223> /note="Description of Artificial Sequence: Synthetic peptide"
<400> 1367
   Gly Thr Asn Lys Arg Ala Pro
   1 5
<210> 1368
   <211> 19
   <212> PRT
   <213> Artificial Sequence
<220>
   <221> source
   <223> /note="Description of Artificial Sequence: Synthetic peptide"
<400> 1368
   Arg Ile Arg Ser Lys Tyr Asn Asn Tyr Ala Thr Tyr Tyr Ala Asp Ser
   1 5 10 15
   Val Lys Gly
<210> 1369
   <211> 7
   <212> PRT
   <213> Artificial Sequence
<220>
   <221> source
   <223> /note="Description of Artificial Sequence: Synthetic peptide"
<400> 1369
   Gly Thr Asn Lys Arg Ala Pro
   1 5
<210> 1370
   <211> 19
   <212> PRT
   <213> Artificial Sequence
<220>
   <221> source
   <223> /note="Description of Artificial Sequence: Synthetic peptide"
<400> 1370
   Arg Ile Arg Ser Lys Tyr Asn Asn Tyr Ala Thr Tyr Tyr Ala Asp Ser
   1               5                   10                  15
   Val Lys Gly
<210> 1371
   <211> 7
   <212> PRT
   <213> Artificial Sequence
<220>
   <221> source
   <223> /note="Description of Artificial Sequence: Synthetic peptide"
<400> 1371
   Gly Thr Asn Lys Arg Ala Pro
   1               5
<210> 1372
   <211> 17
   <212> PRT
   <213> Artificial Sequence
<220>
   <221> source
   <223> /note="Description of Artificial Sequence: Synthetic peptide"
<400> 1372
   Tyr Ile Asn Pro Ser Arg Gly Tyr Thr Asn Tyr Asn Gln Lys Val Lys
   1               5                   10                  15
   Asp
<210> 1373
   <211> 7
   <212> PRT
   <213> Artificial Sequence
<220>
   <221> source
   <223> /note="Description of Artificial Sequence: Synthetic peptide"
<400> 1373
   Asp Thr Ser Lys Leu Ala Ser
   1               5
<210> 1374
   <211> 17
   <212> PRT
   <213> Artificial Sequence
<220>
   <221> source
   <223> /note="Description of Artificial Sequence: Synthetic peptide"
<400> 1374
   Tyr Ile Asn Pro Ser Arg Gly Tyr Thr Asn Tyr Asn Gln Lys Val Lys
   1               5                   10                  15
   Asp
<210> 1375
   <211> 7
   <212> PRT
   <213> Artificial Sequence
<220>
   <221> source
   <223> /note="Description of Artificial Sequence: Synthetic peptide"
<400> 1375
   Asp Thr Ser Lys Leu Ala Ser
   1               5
<210> 1376
   <211> 10
   <212> PRT
   <213> Artificial Sequence
<220>
   <221> source
   <223> /note="Description of Artificial Sequence: Synthetic peptide"
<400> 1376
   Tyr Tyr Asp Asp His Tyr Cys Leu Asp Tyr
   1               5                   10
<210> 1377
   <211> 9
   <212> PRT
   <213> Artificial Sequence
<220>
   <221> source
   <223> /note="Description of Artificial Sequence: Synthetic peptide"
<400> 1377
   Gln Gln Trp Ser Ser Asn Pro Phe Thr
   1               5
<210> 1378
   <211> 14
   <212> PRT
   <213> Artificial Sequence
<220>
   <221> source
   <223> /note="Description of Artificial Sequence: Synthetic peptide"
<400> 1378
   His Gly Asn Phe Gly Asn Ser Tyr Val Ser Trp Phe Ala Tyr
   1 5 10
<210> 1379
   <211> 9
   <212> PRT
   <213> Artificial Sequence
<220>
   <221> source
   <223> /note="Description of Artificial Sequence: Synthetic peptide"
<400> 1379
   Ala Leu Trp Tyr Ser Asn Leu Trp Val
   1 5
<210> 1380
   <211> 10
   <212> PRT
   <213> Artificial Sequence
<220>
   <221> source
   <223> /note="Description of Artificial Sequence: Synthetic peptide"
<400> 1380
   Trp Gln Asp Tyr Asp Val Tyr Phe Asp Tyr
   1 5 10
<210> 1381
   <211> 9
   <212> PRT
   <213> Artificial Sequence
<220>
   <221> source
   <223> /note="Description of Artificial Sequence: Synthetic peptide"
<400> 1381
   Gln Gln Trp Ser Ser Asn Pro Pro Thr
   1 5
<210> 1382
   <211> 10
   <212> PRT
   <213> Artificial Sequence
<220>
   <221> source
   <223> /note="Description of Artificial Sequence: Synthetic peptide"
<400> 1382
   Tyr Tyr Asp Asp His Tyr Cys Leu Asp Tyr
   1               5                   10
<210> 1383
   <211> 9
   <212> PRT
   <213> Artificial Sequence
<220>
   <221> source
   <223> /note="Description of Artificial Sequence: Synthetic peptide"
<400> 1383
   Gln Gln Trp Ser Ser Asn Pro Leu Thr
   1               5
<210> 1384
   <211> 10
   <212> PRT
   <213> Artificial Sequence
<220>
   <221> source
   <223> /note="Description of Artificial Sequence: Synthetic peptide"
<400> 1384
   Tyr Tyr Asp Asp His Tyr Cys Leu Asp Tyr
   1               5                   10
<210> 1385
   <211> 9
   <212> PRT
   <213> Artificial Sequence
<220>
   <221> source
   <223> /note="Description of Artificial Sequence: Synthetic peptide"
<400> 1385
   Gln Gln Trp Ser Ser Asn Pro Phe Thr
   1               5
<210> 1386
   <211> 9
   <212> PRT
   <213> Artificial Sequence
<220>
   <221> source
   <223> /note="Description of Artificial Sequence: Synthetic peptide"
<400> 1386
   Gln Met Gly Tyr Trp His Phe Asp Leu
   1               5
<210> 1387
   <211> 10
   <212> PRT
   <213> Artificial Sequence
<220>
   <221> source
   <223> /note="Description of Artificial Sequence: Synthetic peptide"
<400> 1387
   Gln Gln Arg Ser Asn Trp Pro Pro Leu Thr
   1               5                   10
<210> 1388
   <211> 14
   <212> PRT
   <213> Artificial Sequence
<220>
   <221> source
   <223> /note="Description of Artificial Sequence: Synthetic peptide"
<400> 1388
   His Gly Asn Phe Gly Asn Ser Tyr Val Ser Trp Phe Ala Tyr
   1               5                   10
<210> 1389
   <211> 9
   <212> PRT
   <213> Artificial Sequence
<220>
   <221> source
   <223> /note="Description of Artificial Sequence: Synthetic peptide"
<400> 1389
   Ala Leu Trp Tyr Ser Asn Leu Trp Val
   1               5
<210> 1390
   <211> 10
   <212> PRT
   <213> Artificial Sequence
<220>
   <221> source
   <223> /note="Description of Artificial Sequence: Synthetic peptide"
<400> 1390
   Tyr Tyr Asp Asp His Tyr Cys Leu Asp Tyr
   1 5 10
<210> 1391
   <211> 9
   <212> PRT
   <213> Artificial Sequence
<220>
   <221> source
   <223> /note="Description of Artificial Sequence: Synthetic peptide"
<400> 1391
   Gln Gln Trp Ser Ser Asn Pro Leu Thr
   1 5
<210> 1392
   <211> 14
   <212> PRT
   <213> Artificial Sequence
<220>
   <221> source
   <223> /note="Description of Artificial Sequence: Synthetic peptide"
<400> 1392
   His Gly Asn Phe Gly Asn Ser Tyr Val Ser Trp Phe Ala Tyr
   1 5 10
<210> 1393
   <211> 9
   <212> PRT
   <213> Artificial Sequence
<220>
   <221> source
   <223> /note="Description of Artificial Sequence: Synthetic peptide"
<400> 1393
   Ala Leu Trp Tyr Ser Asn Leu Trp Val
   1 5
<210> 1394
   <211> 14
   <212> PRT
   <213> Artificial Sequence
<220>
   <221> source
   <223> /note="Description of Artificial Sequence: Synthetic peptide"
<400> 1394
   His Gly Asn Phe Gly Asn Ser Tyr Val Ser Trp Phe Ala Tyr
   1 5 10
<210> 1395
   <211> 9
   <212> PRT
   <213> Artificial Sequence
<220>
   <221> source
   <223> /note="Description of Artificial Sequence: Synthetic peptide"
<400> 1395
   Ala Leu Trp Tyr Ser Asn Leu Trp Val
   1 5
<210> 1396
   <211> 14
   <212> PRT
   <213> Artificial Sequence
<220>
   <221> source
   <223> /note="Description of Artificial Sequence: Synthetic peptide"
<400> 1396
   His Gly Asn Phe Gly Asn Ser Tyr Val Ser Trp Trp Ala Tyr
   1 5 10
<210> 1397
   <211> 9
   <212> PRT
   <213> Artificial Sequence
<220>
   <221> source
   <223> /note="Description of Artificial Sequence: Synthetic peptide"
<400> 1397
   Val Leu Trp Tyr Ser Asn Arg Trp Val
   1 5
<210> 1398
   <211> 14
   <212> PRT
   <213> Artificial Sequence
<220>
   <221> source
   <223> /note="Description of Artificial Sequence: Synthetic peptide"
<400> 1398
   His Gly Asn Phe Gly Asn Ser Tyr Ile Ser Tyr Trp Ala Tyr
   1 5 10
<210> 1399
   <211> 9
   <212> PRT
   <213> Artificial Sequence
<220>
   <221> source
   <223> /note="Description of Artificial Sequence: Synthetic peptide"
<400> 1399
   Val Leu Trp Tyr Ser Asn Arg Trp Val
   1               5
<210> 1400
   <211> 10
   <212> PRT
   <213> Artificial Sequence
<220>
   <221> source
   <223> /note="Description of Artificial Sequence: Synthetic peptide"
<400> 1400
   Tyr Tyr Asp Asp His Tyr Cys Leu Asp Tyr
   1               5                   10
<210> 1401
   <211> 9
   <212> PRT
   <213> Artificial Sequence
<220>
   <221> source
   <223> /note="Description of Artificial Sequence: Synthetic peptide"
<400> 1401
   Gln Gln Trp Ser Ser Asn Pro Phe Thr
   1               5
<210> 1402
   <211> 14
   <212> PRT
   <213> Artificial Sequence
<220>
   <221> source
   <223> /note="Description of Artificial Sequence: Synthetic peptide"
<400> 1402
   His Gly Asn Phe Gly Asn Ser Tyr Val Ser Trp Phe Ala Tyr
   1               5                   10
<210> 1403
   <211> 9
   <212> PRT
   <213> Artificial Sequence
<220>
   <221> source
   <223> /note="Description of Artificial Sequence: Synthetic peptide"
<400> 1403
   Ala Leu Trp Tyr Ser Asn Leu Trp Val
   1               5
<210> 1404
   <211> 14
   <212> PRT
   <213> Artificial Sequence
<220>
   <221> source
   <223> /note="Description of Artificial Sequence: Synthetic peptide"
<400> 1404
   His Gly Asn Phe Gly Asn Ser Tyr Val Ser Trp Phe Ala Tyr
   1               5                   10
<210> 1405
   <211> 9
   <212> PRT
   <213> Artificial Sequence
<220>
   <221> source
   <223> /note="Description of Artificial Sequence: Synthetic peptide"
<400> 1405
   Ala Leu Trp Tyr Ser Asn Leu Trp Val
   1               5
<210> 1406
   <211> 14
   <212> PRT
   <213> Artificial Sequence
<220>
   <221> source
   <223> /note="Description of Artificial Sequence: Synthetic peptide"
<400> 1406
   His Gly Asn Phe Gly Asn Ser Tyr Val Ser Trp Phe Ala Tyr
   1               5                   10
<210> 1407
   <211> 9
   <212> PRT
   <213> Artificial Sequence
<220>
   <221> source
   <223> /note="Description of Artificial Sequence: Synthetic peptide"
<400> 1407
   Ala Leu Trp Tyr Ser Asn Leu Trp Val
   1               5
<210> 1408
   <211> 14
   <212> PRT
   <213> Artificial Sequence
<220>
   <221> source
   <223> /note="Description of Artificial Sequence: Synthetic peptide"
<400> 1408
   His Gly Asn Phe Gly Asp Ser Tyr Val Ser Trp Phe Ala Tyr
   1               5                   10
<210> 1409
   <211> 9
   <212> PRT
   <213> Artificial Sequence
<220>
   <221> source
   <223> /note="Description of Artificial Sequence: Synthetic peptide"
<400> 1409
   Ala Leu Trp Tyr Ser Asn His Trp Val
   1               5
<210> 1410
   <211> 10
   <212> PRT
   <213> Artificial Sequence
<220>
   <221> source
   <223> /note="Description of Artificial Sequence: Synthetic peptide"
<400> 1410
   Tyr Tyr Asp Asp His Tyr Cys Leu Asp Tyr
   1               5                   10
<210> 1411
   <211> 9
   <212> PRT
   <213> Artificial Sequence
<220>
   <221> source
   <223> /note="Description of Artificial Sequence: Synthetic peptide"
<400> 1411
   Gln Gln Trp Ser Ser Asn Pro Phe Thr
   1 5
<210> 1412
   <211> 10
   <212> PRT
   <213> Artificial Sequence
<220>
   <221> source
   <223> /note="Description of Artificial Sequence: Synthetic peptide"
<400> 1412
   Tyr Tyr Asp Asp His Tyr Ser Leu Asp Tyr
   1 5 10
<210> 1413
   <211> 9
   <212> PRT
   <213> Artificial Sequence
<220>
   <221> source
   <223> /note="Description of Artificial Sequence: Synthetic peptide"
<400> 1413
   Gln Gln Trp Ser Ser Asn Pro Phe Thr
   1 5
<210> 1414
<400> 1414
   000
<210> 1415
<400> 1415
   000
<210> 1416
<400> 1416
   000
<210> 1417
<400> 1417
   000
<210> 1418
<400> 1418
   000
<210> 1419
<400> 1419
   000
<210> 1420
<400> 1420
   000
<210> 1421
<400> 1421
   000
<210> 1422
<400> 1422
   000
<210> 1423
<400> 1423
   000
<210> 1424
<400> 1424
   000
<210> 1425
<400> 1425
   000
<210> 1426
<400> 1426
   000
<210> 1427
<400> 1427
   000
<210> 1428
<400> 1428
   000
<210> 1429
<400> 1429
   000
<210> 1430
<400> 1430
   000
<210> 1431
<400> 1431
   000
<210> 1432
<400> 1432
   000
<210> 1433
<400> 1433
   000
<210> 1434
<400> 1434
   000
<210> 1435
<400> 1435
   000
<210> 1436
<400> 1436
   000
<210> 1437
<400> 1437
   000
<210> 1438
<400> 1438
   000
<210> 1439
<400> 1439
   000
<210> 1440
<400> 1440
   000
<210> 1441
<400> 1441
   000
<210> 1442
<400> 1442
   000
<210> 1443
<400> 1443
   000
<210> 1444
<400> 1444
   000
<210> 1445
<400> 1445
   000
<210> 1446
<400> 1446
   000
<210> 1447
<400> 1447
   000
<210> 1448
<400> 1448
   000
<210> 1449
<400> 1449
   000
<210> 1450
<400> 1450
   000
<210> 1451
<400> 1451
   000
<210> 1452
<400> 1452
   000
<210> 1453
<400> 1453
   000
<210> 1454
<400> 1454
   000
<210> 1455
<400> 1455
   000
<210> 1456
<400> 1456
   000
<210> 1457
<400> 1457
   000
<210> 1458
<400> 1458
   000
<210> 1459
<400> 1459
   000
<210> 1460
<400> 1460
   000
<210> 1461
<400> 1461
   000
<210> 1462
<400> 1462
   000
<210> 1463
<400> 1463
   000
<210> 1464
<400> 1464
   000
<210> 1465
<400> 1465
   000
<210> 1466
<400> 1466
   000
<210> 1467
<400> 1467
   000
<210> 1468
<400> 1468
   000
<210> 1469
<400> 1469
   000
<210> 1470
<400> 1470
   000
<210> 1471
<400> 1471
   000
<210> 1472
<400> 1472
   000
<210> 1473
<400> 1473
   000
<210> 1474
<400> 1474
   000
<210> 1475
<400> 1475
   000
<210> 1476
<400> 1476
   000
<210> 1477
<400> 1477
   000
<210> 1478
<400> 1478
   000
<210> 1479
<400> 1479
   000
<210> 1480
<400> 1480
   000
<210> 1481
<400> 1481
   000
<210> 1482
<400> 1482
   000
<210> 1483
<400> 1483
   000
<210> 1484
<400> 1484
   000
<210> 1485
<400> 1485
   000
<210> 1486
<400> 1486
   000
<210> 1487
<400> 1487
   000
<210> 1488
<400> 1488
   000
<210> 1489
<400> 1489
   000
<210> 1490
<400> 1490
   000
<210> 1491
<400> 1491
   000
<210> 1492
<400> 1492
   000
<210> 1493
<400> 1493
   000
<210> 1494
<400> 1494
   000
<210> 1495
<400> 1495
   000
<210> 1496
<400> 1496
   000
<210> 1497
<400> 1497
   000
<210> 1498
<400> 1498
   000
<210> 1499
<400> 1499
   000
<210> 1500
   <211> 7
   <212> PRT
   <213> Artificial Sequence
<220>
   <221> source
   <223> /note="Description of Artificial Sequence: Synthetic peptide"
<400> 1500
   Gly Tyr Thr Phe Thr Arg Tyr
   1               5
<210> 1501
   <211> 6
   <212> PRT
   <213> Artificial Sequence
<220>
   <221> source
   <223> /note="Description of Artificial Sequence: Synthetic peptide"
<400> 1501
   Ser Ser Ser Val Ser Tyr
   1               5
<210> 1502
   <211> 7
   <212> PRT
   <213> Artificial Sequence
<220>
   <221> source
   <223> /note="Description of Artificial Sequence: Synthetic peptide"
<400> 1502
   Gly Phe Thr Phe Asn Thr Tyr
   1               5
<210> 1503
   <211> 10
   <212> PRT
   <213> Artificial Sequence
<220>
   <221> source
   <223> /note="Description of Artificial Sequence: Synthetic peptide"
<400> 1503
   Ser Thr Gly Ala Val Thr Thr Ser Asn Tyr
   1               5                   10
<210> 1504
   <211> 7
   <212> PRT
   <213> Artificial Sequence
<220>
   <221> source
   <223> /note="Description of Artificial Sequence: Synthetic peptide"
<400> 1504
   Gly Tyr Thr Phe Thr Ser Tyr
   1               5
<210> 1505
   <211> 6
   <212> PRT
   <213> Artificial Sequence
<220>
   <221> source
   <223> /note="Description of Artificial Sequence: Synthetic peptide"
<400> 1505
   Ser Ser Ser Val Ser Tyr
   1               5
<210> 1506
   <211> 7
   <212> PRT
   <213> Artificial Sequence
<220>
   <221> source
   <223> /note="Description of Artificial Sequence: Synthetic peptide"
<400> 1506
   Gly Tyr Thr Phe Thr Arg Tyr
   1               5
<210> 1507
   <211> 6
   <212> PRT
   <213> Artificial Sequence
<220>
   <221> source
   <223> /note="Description of Artificial Sequence: Synthetic peptide"
<400> 1507
   Ser Ser Ser Val Ser Tyr
   1               5
<210> 1508
   <211> 7
   <212> PRT
   <213> Artificial Sequence
<220>
   <221> source
   <223> /note="Description of Artificial Sequence: Synthetic peptide"
<400> 1508
   Gly Tyr Thr Phe Thr Arg Tyr
   1               5
<210> 1509
   <211> 6
   <212> PRT
   <213> Artificial Sequence
<220>
   <221> source
   <223> /note="Description of Artificial Sequence: Synthetic peptide"
<400> 1509
   Ser Ser Ser Val Ser Tyr
   1               5
<210> 1510
   <211> 7
   <212> PRT
   <213> Artificial Sequence
<220>
   <221> source
   <223> /note="Description of Artificial Sequence: Synthetic peptide"
<400> 1510
   Gly Phe Lys Phe Ser Gly Tyr
   1               5
<210> 1511
   <211> 7
   <212> PRT
   <213> Artificial Sequence
<220>
   <221> source
   <223> /note="Description of Artificial Sequence: Synthetic peptide"
<400> 1511
   Ser Gln Ser Val Ser Ser Tyr
   1               5
<210> 1512
   <211> 7
   <212> PRT
   <213> Artificial Sequence
<220>
   <221> source
   <223> /note="Description of Artificial Sequence: Synthetic peptide"
<400> 1512
   Gly Phe Thr Phe Ser Thr Tyr
   1 5
<210> 1513
   <211> 10
   <212> PRT
   <213> Artificial Sequence
<220>
   <221> source
   <223> /note="Description of Artificial Sequence: Synthetic peptide"
<400> 1513
   Ser Thr Gly Ala Val Thr Thr Ser Asn Tyr
   1 5 10
<210> 1514
   <211> 7
   <212> PRT
   <213> Artificial Sequence
<220>
   <221> source
   <223> /note="Description of Artificial Sequence: Synthetic peptide"
<400> 1514
   Gly Tyr Thr Phe Thr Arg Tyr
   1 5
<210> 1515
   <211> 6
   <212> PRT
   <213> Artificial Sequence
<220>
   <221> source
   <223> /note="Description of Artificial Sequence: Synthetic peptide"
<400> 1515
   Ser Ser Ser Val Ser Tyr
   1 5
<210> 1516
   <211> 7
   <212> PRT
   <213> Artificial Sequence
<220>
   <221> source
   <223> /note="Description of Artificial Sequence: Synthetic peptide"
<400> 1516
   Gly Phe Thr Phe Asn Thr Tyr
   1 5
<210> 1517
   <211> 10
   <212> PRT
   <213> Artificial Sequence
<220>
   <221> source
   <223> /note="Description of Artificial Sequence: Synthetic peptide"
<400> 1517
   Ser Thr Gly Ala Val Thr Thr Ser Asn Tyr
   1 5 10
<210> 1518
   <211> 7
   <212> PRT
   <213> Artificial Sequence
<220>
   <221> source
   <223> /note="Description of Artificial Sequence: Synthetic peptide"
<400> 1518
   Gly Phe Thr Phe Asn Thr Tyr
   1 5
<210> 1519
   <211> 10
   <212> PRT
   <213> Artificial Sequence
<220>
   <221> source
   <223> /note="Description of Artificial Sequence: Synthetic peptide"
<400> 1519
   Ser Thr Gly Ala Val Thr Thr Ser Asn Tyr
   1 5 10
<210> 1520
   <211> 7
   <212> PRT
   <213> Artificial Sequence
<220>
   <221> source
   <223> /note="Description of Artificial Sequence: Synthetic peptide"
<400> 1520
   Gly Phe Thr Phe Asn Ser Tyr
   1 5
<210> 1521
   <211> 10
   <212> PRT
   <213> Artificial Sequence
<220>
   <221> source
   <223> /note="Description of Artificial Sequence: Synthetic peptide"
<400> 1521
   Ser Thr Gly Ala Val Thr Ser Gly Asn Tyr
   1 5 10
<210> 1522
   <211> 7
   <212> PRT
   <213> Artificial Sequence
<220>
   <221> source
   <223> /note="Description of Artificial Sequence: Synthetic peptide"
<400> 1522
   Gly Phe Thr Phe Asn Lys Tyr
   1 5
<210> 1523
   <211> 10
   <212> PRT
   <213> Artificial Sequence
<220>
   <221> source
   <223> /note="Description of Artificial Sequence: Synthetic peptide"
<400> 1523
   Ser Thr Gly Ala Val Thr Ser Gly Asn Tyr
   1 5 10
<210> 1524
   <211> 7
   <212> PRT
   <213> Artificial Sequence
<220>
   <221> source
   <223> /note="Description of Artificial Sequence: Synthetic peptide"
<400> 1524
   Gly Tyr Thr Phe Thr Arg Tyr
   1 5
<210> 1525
   <211> 6
   <212> PRT
   <213> Artificial Sequence
<220>
   <221> source
   <223> /note="Description of Artificial Sequence: Synthetic peptide"
<400> 1525
   Ser Ser Ser Val Ser Tyr
   1 5
<210> 1526
   <211> 7
   <212> PRT
   <213> Artificial Sequence
<220>
   <221> source
   <223> /note="Description of Artificial Sequence: Synthetic peptide"
<400> 1526
   Gly Phe Thr Phe Ser Thr Tyr
   1 5
<210> 1527
   <211> 10
   <212> PRT
   <213> Artificial Sequence
<220>
   <221> source
   <223> /note="Description of Artificial Sequence: Synthetic peptide"
<400> 1527
   Ser Thr Gly Ala Val Thr Thr Ser Asn Tyr
   1 5 10
<210> 1528
   <211> 7
   <212> PRT
   <213> Artificial Sequence
<220>
   <221> source
   <223> /note="Description of Artificial Sequence: Synthetic peptide"
<400> 1528
   Gly Phe Thr Phe Asn Thr Tyr
   1 5
<210> 1529
   <211> 10
   <212> PRT
   <213> Artificial Sequence
<220>
   <221> source
   <223> /note="Description of Artificial Sequence: Synthetic peptide"
<400> 1529
   Ser Thr Gly Ala Val Thr Thr Ser Asn Tyr
   1 5 10
<210> 1530
   <211> 7
   <212> PRT
   <213> Artificial Sequence
<220>
   <221> source
   <223> /note="Description of Artificial Sequence: Synthetic peptide"
<400> 1530
   Gly Phe Thr Phe Asn Thr Tyr
   1 5
<210> 1531
   <211> 10
   <212> PRT
   <213> Artificial Sequence
<220>
   <221> source
   <223> /note="Description of Artificial Sequence: Synthetic peptide"
<400> 1531
   Ser Thr Gly Ala Val Thr Thr Ser Asn Tyr
   1 5 10
<210> 1532
   <211> 7
   <212> PRT
   <213> Artificial Sequence
<220>
   <221> source
   <223> /note="Description of Artificial Sequence: Synthetic peptide"
<400> 1532
   Gly Phe Thr Phe Ser Thr Tyr
   1 5
<210> 1533
   <211> 10
   <212> PRT
   <213> Artificial Sequence
<220>
   <221> source
   <223> /note="Description of Artificial Sequence: Synthetic peptide"
<400> 1533
   Ser Thr Gly Ala Val Thr Thr Ser Asn Tyr
   1 5 10
<210> 1534
   <211> 7
   <212> PRT
   <213> Artificial Sequence
<220>
   <221> source
   <223> /note="Description of Artificial Sequence: Synthetic peptide"
<400> 1534
   Gly Tyr Thr Phe Thr Arg Tyr
   1 5
<210> 1535
   <211> 6
   <212> PRT
   <213> Artificial Sequence
<220>
   <221> source
   <223> /note="Description of Artificial Sequence: Synthetic peptide"
<400> 1535
   Ser Ser Ser Val Ser Tyr
   1 5
<210> 1536
   <211> 7
   <212> PRT
   <213> Artificial Sequence
<220>
   <221> source
   <223> /note="Description of Artificial Sequence: Synthetic peptide"
<400> 1536
   Gly Tyr Thr Phe Thr Arg Tyr
   1 5
<210> 1537
   <211> 6
   <212> PRT
   <213> Artificial Sequence
<220>
   <221> source
   <223> /note="Description of Artificial Sequence: Synthetic peptide"
<400> 1537
   Ser Ser Ser Val Ser Tyr
   1 5
<210> 1538
   <211> 6
   <212> PRT
   <213> Artificial Sequence
<220>
   <221> source
   <223> /note="Description of Artificial Sequence: Synthetic peptide"
<400> 1538
   Asn Pro Ser Arg Gly Tyr
   1 5
<210> 1539
   <211> 3
   <212> PRT
   <213> Artificial Sequence
<220>
   <221> source
   <223> /note="Description of Artificial Sequence: Synthetic peptide"
<400> 1539
   Asp Thr Ser
   1
<210> 1540
   <211> 8
   <212> PRT
   <213> Artificial Sequence
<220>
   <221> source
   <223> /note="Description of Artificial Sequence: Synthetic peptide"
<400> 1540
   Arg Ser Lys Tyr Asn Asn Tyr Ala
   1 5
<210> 1541
   <211> 3
   <212> PRT
   <213> Artificial Sequence
<220>
   <221> source
   <223> /note="Description of Artificial Sequence: Synthetic peptide"
<400> 1541
   Gly Thr Asn
   1
<210> 1542
   <211> 6
   <212> PRT
   <213> Artificial Sequence
<220>
   <221> source
   <223> /note="Description of Artificial Sequence: Synthetic peptide"
<400> 1542
   Asn Pro Ser Ser Gly Tyr
   1 5
<210> 1543
   <211> 3
   <212> PRT
   <213> Artificial Sequence
<220>
   <221> source
   <223> /note="Description of Artificial Sequence: Synthetic peptide"
<400> 1543
   Ala Thr Ser
   1
<210> 1544
   <211> 6
   <212> PRT
   <213> Artificial Sequence
<220>
   <221> source
   <223> /note="Description of Artificial Sequence: Synthetic peptide"
<400> 1544
   Asn Pro Ser Arg Gly Tyr
   1 5
<210> 1545
   <211> 3
   <212> PRT
   <213> Artificial Sequence
<220>
   <221> source
   <223> /note="Description of Artificial Sequence: Synthetic peptide"
<400> 1545
   Asp Thr Ser
   1
<210> 1546
   <211> 6
   <212> PRT
   <213> Artificial Sequence
<220>
   <221> source
   <223> /note="Description of Artificial Sequence: Synthetic peptide"
<400> 1546
   Asn Pro Ser Arg Gly Tyr
   1 5
<210> 1547
   <211> 3
   <212> PRT
   <213> Artificial Sequence
<220>
   <221> source
   <223> /note="Description of Artificial Sequence: Synthetic peptide"
<400> 1547
   Asp Thr Ser
   1
<210> 1548
   <211> 6
   <212> PRT
   <213> Artificial Sequence
<220>
   <221> source
   <223> /note="Description of Artificial Sequence: Synthetic peptide"
<400> 1548
   Trp Tyr Asp Gly Ser Lys
   1 5
<210> 1549
   <211> 3
   <212> PRT
   <213> Artificial Sequence
<220>
   <221> source
   <223> /note="Description of Artificial Sequence: Synthetic peptide"
<400> 1549
   Asp Ala Ser
   1
<210> 1550
   <211> 9
   <212> PRT
   <213> Artificial Sequence
<220>
   <221> source
   <223> /note="Description of Artificial Sequence: Synthetic peptide"
<400> 1550
   Arg Ser Lys Tyr Asn Asn Tyr Ala Thr
   1 5
<210> 1551
   <211> 3
   <212> PRT
   <213> Artificial Sequence
<220>
   <221> source
   <223> /note="Description of Artificial Sequence: Synthetic peptide"
<400> 1551
   Gly Thr Asn
   1
<210> 1552
   <211> 6
   <212> PRT
   <213> Artificial Sequence
<220>
   <221> source
   <223> /note="Description of Artificial Sequence: Synthetic peptide"
<400> 1552
   Asn Pro Ser Arg Gly Tyr
   1 5
<210> 1553
   <211> 3
   <212> PRT
   <213> Artificial Sequence
<220>
   <221> source
   <223> /note="Description of Artificial Sequence: Synthetic peptide"
<400> 1553
   Asp Thr Ser
   1
<210> 1554
   <211> 9
   <212> PRT
   <213> Artificial Sequence
<220>
   <221> source
   <223> /note="Description of Artificial Sequence: Synthetic peptide"
<400> 1554
   Arg Ser Lys Tyr Asn Asn Tyr Ala Thr
   1               5
<210> 1555
   <211> 3
   <212> PRT
   <213> Artificial Sequence
<220>
   <221> source
   <223> /note="Description of Artificial Sequence: Synthetic peptide"
<400> 1555
   Gly Thr Asn
   1
<210> 1556
   <211> 8
   <212> PRT
   <213> Artificial Sequence
<220>
   <221> source
   <223> /note="Description of Artificial Sequence: Synthetic peptide"
<400> 1556
   Arg Ser Lys Tyr Asn Asn Tyr Ala
   1               5
<210> 1557
   <211> 3
   <212> PRT
   <213> Artificial Sequence
<220>
   <221> source
   <223> /note="Description of Artificial Sequence: Synthetic peptide"
<400> 1557
   Gly Thr Asn
   1
<210> 1558
   <211> 8
   <212> PRT
   <213> Artificial Sequence
<220>
   <221> source
   <223> /note="Description of Artificial Sequence: Synthetic peptide"
<400> 1558
   Arg Ser Lys Tyr Asn Asn Tyr Ala
   1               5
<210> 1559
   <211> 3
   <212> PRT
   <213> Artificial Sequence
<220>
   <221> source
   <223> /note="Description of Artificial Sequence: Synthetic peptide"
<400> 1559
   Gly Thr Lys
   1
<210> 1560
   <211> 8
   <212> PRT
   <213> Artificial Sequence
<220>
   <221> source
   <223> /note="Description of Artificial Sequence: Synthetic peptide"
<400> 1560
   Arg Ser Lys Tyr Asn Asn Tyr Ala
   1               5
<210> 1561
   <211> 3
   <212> PRT
   <213> Artificial Sequence
<220>
   <221> source
   <223> /note="Description of Artificial Sequence: Synthetic peptide"
<400> 1561
   Gly Thr Lys
   1
<210> 1562
   <211> 6
   <212> PRT
   <213> Artificial Sequence
<220>
   <221> source
   <223> /note="Description of Artificial Sequence: Synthetic peptide"
<400> 1562
   Asn Pro Ser Arg Gly Tyr
   1 5
<210> 1563
   <211> 3
   <212> PRT
   <213> Artificial Sequence
<220>
   <221> source
   <223> /note="Description of Artificial Sequence: Synthetic peptide"
<400> 1563
   Asp Thr Ser
   1
<210> 1564
   <211> 8
   <212> PRT
   <213> Artificial Sequence
<220>
   <221> source
   <223> /note="Description of Artificial Sequence: Synthetic peptide"
<400> 1564
   Arg Ser Lys Tyr Asn Asn Tyr Ala
   1 5
<210> 1565
   <211> 3
   <212> PRT
   <213> Artificial Sequence
<220>
   <221> source
   <223> /note="Description of Artificial Sequence: Synthetic peptide"
<400> 1565
   Gly Thr Asn
   1
<210> 1566
   <211> 8
   <212> PRT
   <213> Artificial Sequence
<220>
   <221> source
   <223> /note="Description of Artificial Sequence: Synthetic peptide"
<400> 1566
   Arg Ser Lys Tyr Asn Asn Tyr Ala
   1               5
<210> 1567
   <211> 3
   <212> PRT
   <213> Artificial Sequence
<220>
   <221> source
   <223> /note="Description of Artificial Sequence: Synthetic peptide"
<400> 1567
   Gly Thr Asn
   1
<210> 1568
   <211> 8
   <212> PRT
   <213> Artificial Sequence
<220>
   <221> source
   <223> /note="Description of Artificial Sequence: Synthetic peptide"
<400> 1568
   Arg Ser Lys Tyr Asn Asn Tyr Ala
   1               5
<210> 1569
   <211> 3
   <212> PRT
   <213> Artificial Sequence
<220>
   <221> source
   <223> /note="Description of Artificial Sequence: Synthetic peptide"
<400> 1569
   Gly Thr Asn
   1
<210> 1570
   <211> 8
   <212> PRT
   <213> Artificial Sequence
<220>
   <221> source
   <223> /note="Description of Artificial Sequence: Synthetic peptide"
<400> 1570
   Arg Ser Lys Tyr Asn Asn Tyr Ala
   1 5
<210> 1571
   <211> 3
   <212> PRT
   <213> Artificial Sequence
<220>
   <221> source
   <223> /note="Description of Artificial Sequence: Synthetic peptide"
<400> 1571
   Gly Thr Asn
   1
<210> 1572
   <211> 6
   <212> PRT
   <213> Artificial Sequence
<220>
   <221> source
   <223> /note="Description of Artificial Sequence: Synthetic peptide"
<400> 1572
   Asn Pro Ser Arg Gly Tyr
   1 5
<210> 1573
   <211> 3
   <212> PRT
   <213> Artificial Sequence
<220>
   <221> source
   <223> /note="Description of Artificial Sequence: Synthetic peptide"
<400> 1573
   Asp Thr Ser
   1
<210> 1574
   <211> 6
   <212> PRT
   <213> Artificial Sequence
<220>
   <221> source
   <223> /note="Description of Artificial Sequence: Synthetic peptide"
<400> 1574
   Asn Pro Ser Arg Gly Tyr
   1 5
<210> 1575
   <211> 3
   <212> PRT
   <213> Artificial Sequence
<220>
   <221> source
   <223> /note="Description of Artificial Sequence: Synthetic peptide"
<400> 1575
   Asp Thr Ser
   1
<210> 1576
   <211> 10
   <212> PRT
   <213> Artificial Sequence
<220>
   <221> source
   <223> /note="Description of Artificial Sequence: Synthetic peptide"
<400> 1576
   Tyr Tyr Asp Asp His Tyr Cys Leu Asp Tyr
   1 5 10
<210> 1577
   <211> 6
   <212> PRT
   <213> Artificial Sequence
<220>
   <221> source
   <223> /note="Description of Artificial Sequence: Synthetic peptide"
<400> 1577
   Trp Ser Ser Asn Pro Phe
   1 5
<210> 1578
   <211> 14
   <212> PRT
   <213> Artificial Sequence
<220>
   <221> source
   <223> /note="Description of Artificial Sequence: Synthetic peptide"
<400> 1578
   His Gly Asn Phe Gly Asn Ser Tyr Val Ser Trp Phe Ala Tyr
   1 5 10
<210> 1579
   <211> 6
   <212> PRT
   <213> Artificial Sequence
<220>
   <221> source
   <223> /note="Description of Artificial Sequence: Synthetic peptide"
<400> 1579
   Trp Tyr Ser Asn Leu Trp
   1 5
<210> 1580
   <211> 10
   <212> PRT
   <213> Artificial Sequence
<220>
   <221> source
   <223> /note="Description of Artificial Sequence: Synthetic peptide"
<400> 1580
   Trp Gln Asp Tyr Asp Val Tyr Phe Asp Tyr
   1 5 10
<210> 1581
   <211> 6
   <212> PRT
   <213> Artificial Sequence
<220>
   <221> source
   <223> /note="Description of Artificial Sequence: Synthetic peptide"
<400> 1581
   Trp Ser Ser Asn Pro Pro
   1 5
<210> 1582
   <211> 10
   <212> PRT
   <213> Artificial Sequence
<220>
   <221> source
   <223> /note="Description of Artificial Sequence: Synthetic peptide"
<400> 1582
   Tyr Tyr Asp Asp His Tyr Cys Leu Asp Tyr
   1 5 10
<210> 1583
   <211> 6
   <212> PRT
   <213> Artificial Sequence
<220>
   <221> source
   <223> /note="Description of Artificial Sequence: Synthetic peptide"
<400> 1583
   Trp Ser Ser Asn Pro Leu
   1               5
<210> 1584
   <211> 10
   <212> PRT
   <213> Artificial Sequence
<220>
   <221> source
   <223> /note="Description of Artificial Sequence: Synthetic peptide"
<400> 1584
   Tyr Tyr Asp Asp His Tyr Cys Leu Asp Tyr
   1               5                   10
<210> 1585
   <211> 6
   <212> PRT
   <213> Artificial Sequence
<220>
   <221> source
   <223> /note="Description of Artificial Sequence: Synthetic peptide"
<400> 1585
   Trp Ser Ser Asn Pro Phe
   1               5
<210> 1586
   <211> 9
   <212> PRT
   <213> Artificial Sequence
<220>
   <221> source
   <223> /note="Description of Artificial Sequence: Synthetic peptide"
<400> 1586
   Gln Met Gly Tyr Trp His Phe Asp Leu
   1               5
<210> 1587
   <211> 7
   <212> PRT
   <213> Artificial Sequence
<220>
   <221> source
   <223> /note="Description of Artificial Sequence: Synthetic peptide"
<400> 1587
   Arg Ser Asn Trp Pro Pro Leu
   1               5
<210> 1588
   <211> 13
   <212> PRT
   <213> Artificial Sequence
<220>
   <221> source
   <223> /note="Description of Artificial Sequence: Synthetic peptide"
<400> 1588
   His Gly Asn Phe Gly Asn Ser Tyr Val Ser Trp Phe Ala
   1               5                   10
<210> 1589
   <211> 6
   <212> PRT
   <213> Artificial Sequence
<220>
   <221> source
   <223> /note="Description of Artificial Sequence: Synthetic peptide"
<400> 1589
   Trp Tyr Ser Asn Leu Trp
   1               5
<210> 1590
   <211> 10
   <212> PRT
   <213> Artificial Sequence
<220>
   <221> source
   <223> /note="Description of Artificial Sequence: Synthetic peptide"
<400> 1590
   Tyr Tyr Asp Asp His Tyr Cys Leu Asp Tyr
   1               5                   10
<210> 1591
   <211> 6
   <212> PRT
   <213> Artificial Sequence
<220>
   <221> source
   <223> /note="Description of Artificial Sequence: Synthetic peptide"
<400> 1591
   Trp Ser Ser Asn Pro Leu
   1 5
<210> 1592
   <211> 13
   <212> PRT
   <213> Artificial Sequence
<220>
   <221> source
   <223> /note="Description of Artificial Sequence: Synthetic peptide"
<400> 1592
   His Gly Asn Phe Gly Asn Ser Tyr Val Ser Trp Phe Ala
   1 5 10
<210> 1593
   <211> 6
   <212> PRT
   <213> Artificial Sequence
<220>
   <221> source
   <223> /note="Description of Artificial Sequence: Synthetic peptide"
<400> 1593
   Trp Tyr Ser Asn Leu Trp
   1 5
<210> 1594
   <211> 14
   <212> PRT
   <213> Artificial Sequence
<220>
   <221> source
   <223> /note="Description of Artificial Sequence: Synthetic peptide"
<400> 1594
   His Gly Asn Phe Gly Asn Ser Tyr Val Ser Trp Phe Ala Tyr
   1 5 10
<210> 1595
   <211> 6
   <212> PRT
   <213> Artificial Sequence
<220>
   <221> source
   <223> /note="Description of Artificial Sequence: Synthetic peptide"
<400> 1595
   Trp Tyr Ser Asn Leu Trp
   1 5
<210> 1596
   <211> 14
   <212> PRT
   <213> Artificial Sequence
<220>
   <221> source
   <223> /note="Description of Artificial Sequence: Synthetic peptide"
<400> 1596
   His Gly Asn Phe Gly Asn Ser Tyr Val Ser Trp Trp Ala Tyr
   1 5 10
<210> 1597
   <211> 6
   <212> PRT
   <213> Artificial Sequence
<220>
   <221> source
   <223> /note="Description of Artificial Sequence: Synthetic peptide"
<400> 1597
   Trp Tyr Ser Asn Arg Trp
   1 5
<210> 1598
   <211> 14
   <212> PRT
   <213> Artificial Sequence
<220>
   <221> source
   <223> /note="Description of Artificial Sequence: Synthetic peptide"
<400> 1598
   His Gly Asn Phe Gly Asn Ser Tyr Ile Ser Tyr Trp Ala Tyr
   1 5 10
<210> 1599
   <211> 6
   <212> PRT
   <213> Artificial Sequence
<220>
   <221> source
   <223> /note="Description of Artificial Sequence: Synthetic peptide"
<400> 1599
   Trp Tyr Ser Asn Arg Trp
   1 5
<210> 1600
   <211> 10
   <212> PRT
   <213> Artificial Sequence
<220>
   <221> source
   <223> /note="Description of Artificial Sequence: Synthetic peptide"
<400> 1600
   Tyr Tyr Asp Asp His Tyr Cys Leu Asp Tyr
   1               5                   10
<210> 1601
   <211> 6
   <212> PRT
   <213> Artificial Sequence
<220>
   <221> source
   <223> /note="Description of Artificial Sequence: Synthetic peptide"
<400> 1601
   Trp Ser Ser Asn Pro Phe
   1               5
<210> 1602
   <211> 14
   <212> PRT
   <213> Artificial Sequence
<220>
   <221> source
   <223> /note="Description of Artificial Sequence: Synthetic peptide"
<400> 1602
   His Gly Asn Phe Gly Asn Ser Tyr Val Ser Trp Phe Ala Tyr
   1               5                   10
<210> 1603
   <211> 6
   <212> PRT
   <213> Artificial Sequence
<220>
   <221> source
   <223> /note="Description of Artificial Sequence: Synthetic peptide"
<400> 1603
   Trp Tyr Ser Asn Leu Trp
   1               5
<210> 1604
   <211> 14
   <212> PRT
   <213> Artificial Sequence
<220>
   <221> source
   <223> /note="Description of Artificial Sequence: Synthetic peptide"
<400> 1604
   His Gly Asn Phe Gly Asn Ser Tyr Val Ser Trp Phe Ala Tyr
   1 5 10
<210> 1605
   <211> 6
   <212> PRT
   <213> Artificial Sequence
<220>
   <221> source
   <223> /note="Description of Artificial Sequence: Synthetic peptide"
<400> 1605
   Trp Tyr Ser Asn Leu Trp
   1 5
<210> 1606
   <211> 14
   <212> PRT
   <213> Artificial Sequence
<220>
   <221> source
   <223> /note="Description of Artificial Sequence: Synthetic peptide"
<400> 1606
   His Gly Asn Phe Gly Asn Ser Tyr Val Ser Trp Phe Ala Tyr
   1 5 10
<210> 1607
   <211> 6
   <212> PRT
   <213> Artificial Sequence
<220>
   <221> source
   <223> /note="Description of Artificial Sequence: Synthetic peptide"
<400> 1607
   Trp Tyr Ser Asn Leu Trp
   1 5
<210> 1608
   <211> 14
   <212> PRT
   <213> Artificial Sequence
<220>
   <221> source
   <223> /note="Description of Artificial Sequence: Synthetic peptide"
<400> 1608
   His Gly Asn Phe Gly Asp Ser Tyr Val Ser Trp Phe Ala Tyr
   1 5 10
<210> 1609
   <211> 6
   <212> PRT
   <213> Artificial Sequence
<220>
   <221> source
   <223> /note="Description of Artificial Sequence: Synthetic peptide"
<400> 1609
   Trp Tyr Ser Asn His Trp
   1 5
<210> 1610
   <211> 10
   <212> PRT
   <213> Artificial Sequence
<220>
   <221> source
   <223> /note="Description of Artificial Sequence: Synthetic peptide"
<400> 1610
   Tyr Tyr Asp Asp His Tyr Cys Leu Asp Tyr
   1 5 10
<210> 1611
   <211> 6
   <212> PRT
   <213> Artificial Sequence
<220>
   <221> source
   <223> /note="Description of Artificial Sequence: Synthetic peptide"
<400> 1611
   Trp Ser Ser Asn Pro Phe
   1 5
<210> 1612
   <211> 10
   <212> PRT
   <213> Artificial Sequence
<220>
   <221> source
   <223> /note="Description of Artificial Sequence: Synthetic peptide"
<400> 1612
   Tyr Tyr Asp Asp His Tyr Ser Leu Asp Tyr
   1               5                   10
<210> 1613
   <211> 6
   <212> PRT
   <213> Artificial Sequence
<220>
   <221> source
   <223> /note="Description of Artificial Sequence: Synthetic peptide"
<400> 1613
   Trp Ser Ser Asn Pro Phe
   1               5
<210> 1614
<400> 1614
   000
<210> 1615
<400> 1615
   000
<210> 1616
<400> 1616
   000
<210> 1617
<400> 1617
   000
<210> 1618
<400> 1618
   000
<210> 1619
<400> 1619
   000
<210> 1620
<400> 1620
   000
<210> 1621
<400> 1621
   000
<210> 1622
<400> 1622
   000
<210> 1623
<400> 1623
   000
<210> 1624
<400> 1624
   000
<210> 1625
<400> 1625
   000
<210> 1626
<400> 1626
   000
<210> 1627
<400> 1627
   000
<210> 1628
<400> 1628
   000
<210> 1629
<400> 1629
   000
<210> 1630
<400> 1630
   000
<210> 1631
<400> 1631
   000
<210> 1632
<400> 1632
   000
<210> 1633
<400> 1633
   000
<210> 1634
<400> 1634
   000
<210> 1635
<400> 1635
   000
<210> 1636
<400> 1636
   000
<210> 1637
<400> 1637
   000
<210> 1638
<400> 1638
   000
<210> 1639
<400> 1639
   000
<210> 1640
<400> 1640
   000
<210> 1641
<400> 1641
   000
<210> 1642
<400> 1642
   000
<210> 1643
<400> 1643
   000
<210> 1644
<400> 1644
   000
<210> 1645
<400> 1645
   000
<210> 1646
<400> 1646
   000
<210> 1647
<400> 1647
   000
<210> 1648
<400> 1648
   000
<210> 1649
<400> 1649
   000
<210> 1650
<400> 1650
   000
<210> 1651
<400> 1651
   000
<210> 1652
<400> 1652
   000
<210> 1653
<400> 1653
   000
<210> 1654
<400> 1654
   000
<210> 1655
<400> 1655
   000
<210> 1656
<400> 1656
   000
<210> 1657
<400> 1657
   000
<210> 1658
<400> 1658
   000
<210> 1659
<400> 1659
   000
<210> 1660
<400> 1660
   000
<210> 1661
<400> 1661
   000
<210> 1662
<400> 1662
   000
<210> 1663
<400> 1663
   000
<210> 1664
<400> 1664
   000
<210> 1665
<400> 1665
   000
<210> 1666
<400> 1666
   000
<210> 1667
<400> 1667
   000
<210> 1668
<400> 1668
   000
<210> 1669
<400> 1669
   000
<210> 1670
<400> 1670
   000
<210> 1671
<400> 1671
   000
<210> 1672
<400> 1672
   000
<210> 1673
<400> 1673
   000
<210> 1674
<400> 1674
   000
<210> 1675
<400> 1675
   000
<210> 1676
<400> 1676
   000
<210> 1677
<400> 1677
   000
<210> 1678
<400> 1678
   000
<210> 1679
<400> 1679
   000
<210> 1680
<400> 1680
   000
<210> 1681
<400> 1681
   000
<210> 1682
<400> 1682
   000
<210> 1683
<400> 1683
   000
<210> 1684
<400> 1684
   000
<210> 1685
<400> 1685
   000
<210> 1686
<400> 1686
   000
<210> 1687
<400> 1687
   000
<210> 1688
<400> 1688
   000
<210> 1689
<400> 1689
   000
<210> 1690
<400> 1690
   000
<210> 1691
<400> 1691
   000
<210> 1692
<400> 1692
   000
<210> 1693
<400> 1693
   000
<210> 1694
<400> 1694
   000
<210> 1695
<400> 1695
   000
<210> 1696
<400> 1696
   000
<210> 1697
<400> 1697
   000
<210> 1698
<400> 1698
   000
<210> 1699
<400> 1699
   000
<210> 1700
   <211> 5
   <212> PRT
   <213> Artificial Sequence
<220>
   <221> source
   <223> /note="Description of Artificial Sequence: Synthetic peptide"
<400> 1700
   Arg Tyr Thr Met His
   1               5
<210> 1701
   <211> 10
   <212> PRT
   <213> Artificial Sequence
<220>
   <221> source
   <223> /note="Description of Artificial Sequence: Synthetic peptide"
<400> 1701
   Ser Ala Ser Ser Ser Val Ser Tyr Met Asn
   1               5                   10
<210> 1702
   <211> 5
   <212> PRT
   <213> Artificial Sequence
<220>
   <221> source
   <223> /note="Description of Artificial Sequence: Synthetic peptide"
<400> 1702
   Thr Tyr Ala Met Asn
   1               5
<210> 1703
   <211> 14
   <212> PRT
   <213> Artificial Sequence
<220>
   <221> source
   <223> /note="Description of Artificial Sequence: Synthetic peptide"
<400> 1703
   Arg Ser Ser Thr Gly Ala Val Thr Thr Ser Asn Tyr Ala Asn
   1               5                   10
<210> 1704
   <211> 5
   <212> PRT
   <213> Artificial Sequence
<220>
   <221> source
   <223> /note="Description of Artificial Sequence: Synthetic peptide"
<400> 1704
   Ser Tyr Thr Met His
   1               5
<210> 1705
   <211> 10
   <212> PRT
   <213> Artificial Sequence
<220>
   <221> source
   <223> /note="Description of Artificial Sequence: Synthetic peptide"
<400> 1705
   Arg Ala Ser Ser Ser Val Ser Tyr Met His
   1               5                   10
<210> 1706
   <211> 5
   <212> PRT
   <213> Artificial Sequence
<220>
   <221> source
   <223> /note="Description of Artificial Sequence: Synthetic peptide"
<400> 1706
   Arg Tyr Thr Met His
   1               5
<210> 1707
   <211> 10
   <212> PRT
   <213> Artificial Sequence
<220>
   <221> source
   <223> /note="Description of Artificial Sequence: Synthetic peptide"
<400> 1707
   Arg Ala Ser Ser Ser Val Ser Tyr Met Asn
   1               5                   10
<210> 1708
   <211> 5
   <212> PRT
   <213> Artificial Sequence
<220>
   <221> source
   <223> /note="Description of Artificial Sequence: Synthetic peptide"
<400> 1708
   Arg Tyr Thr Met His
   1               5
<210> 1709
   <211> 10
   <212> PRT
   <213> Artificial Sequence
<220>
   <221> source
   <223> /note="Description of Artificial Sequence: Synthetic peptide"
<400> 1709
   Ser Ala Ser Ser Ser Val Ser Tyr Met Asn
   1               5                   10
<210> 1710
   <211> 5
   <212> PRT
   <213> Artificial Sequence
<220>
   <221> source
   <223> /note="Description of Artificial Sequence: Synthetic peptide"
<400> 1710
   Gly Tyr Gly Met His
   1               5
<210> 1711
   <211> 11
   <212> PRT
   <213> Artificial Sequence
<220>
   <221> source
   <223> /note="Description of Artificial Sequence: Synthetic peptide"
<400> 1711
   Arg Ala Ser Gln Ser Val Ser Ser Tyr Leu Ala
   1               5                   10
<210> 1712
   <211> 5
   <212> PRT
   <213> Artificial Sequence
<220>
   <221> source
   <223> /note="Description of Artificial Sequence: Synthetic peptide"
<400> 1712
   Thr Tyr Ala Met Asn
   1               5
<210> 1713
   <211> 14
   <212> PRT
   <213> Artificial Sequence
<220>
   <221> source
   <223> /note="Description of Artificial Sequence: Synthetic peptide"
<400> 1713
   Arg Ser Ser Thr Gly Ala Val Thr Thr Ser Asn Tyr Ala Asn
   1 5 10
<210> 1714
   <211> 5
   <212> PRT
   <213> Artificial Sequence
<220>
   <221> source
   <223> /note="Description of Artificial Sequence: Synthetic peptide"
<400> 1714
   Arg Tyr Thr Met His
   1 5
<210> 1715
   <211> 10
   <212> PRT
   <213> Artificial Sequence
<220>
   <221> source
   <223> /note="Description of Artificial Sequence: Synthetic peptide"
<400> 1715
   Arg Ala Ser Ser Ser Val Ser Tyr Met Asn
   1 5 10
<210> 1716
   <211> 5
   <212> PRT
   <213> Artificial Sequence
<220>
   <221> source
   <223> /note="Description of Artificial Sequence: Synthetic peptide"
<400> 1716
   Thr Tyr Ala Met Asn
   1 5
<210> 1717
   <211> 14
   <212> PRT
   <213> Artificial Sequence
<220>
   <221> source
   <223> /note="Description of Artificial Sequence: Synthetic peptide"
<400> 1717
   Arg Ser Ser Thr Gly Ala Val Thr Thr Ser Asn Tyr Ala Asn
   1 5 10
<210> 1718
   <211> 5
   <212> PRT
   <213> Artificial Sequence
<220>
   <221> source
   <223> /note="Description of Artificial Sequence: Synthetic peptide"
<400> 1718
   Thr Tyr Ala Met Asn
   1 5
<210> 1719
   <211> 14
   <212> PRT
   <213> Artificial Sequence
<220>
   <221> source
   <223> /note="Description of Artificial Sequence: Synthetic peptide"
<400> 1719
   Arg Ser Ser Thr Gly Ala Val Thr Thr Ser Asn Tyr Ala Asn
   1 5 10
<210> 1720
   <211> 5
   <212> PRT
   <213> Artificial Sequence
<220>
   <221> source
   <223> /note="Description of Artificial Sequence: Synthetic peptide"
<400> 1720
   Ser Tyr Ala Met Asn
   1 5
<210> 1721
   <211> 14
   <212> PRT
   <213> Artificial Sequence
<220>
   <221> source
   <223> /note="Description of Artificial Sequence: Synthetic peptide"
<400> 1721
   Gly Ser Ser Thr Gly Ala Val Thr Ser Gly Asn Tyr Pro Asn
   1 5 10
<210> 1722
   <211> 5
   <212> PRT
   <213> Artificial Sequence
<220>
   <221> source
   <223> /note="Description of Artificial Sequence: Synthetic peptide"
<400> 1722
   Lys Tyr Ala Met Asn
   1 5
<210> 1723
   <211> 14
   <212> PRT
   <213> Artificial Sequence
<220>
   <221> source
   <223> /note="Description of Artificial Sequence: Synthetic peptide"
<400> 1723
   Gly Ser Ser Thr Gly Ala Val Thr Ser Gly Asn Tyr Pro Asn
   1 5 10
<210> 1724
   <211> 5
   <212> PRT
   <213> Artificial Sequence
<220>
   <221> source
   <223> /note="Description of Artificial Sequence: Synthetic peptide"
<400> 1724
   Arg Tyr Thr Met His
   1 5
<210> 1725
   <211> 10
   <212> PRT
   <213> Artificial Sequence
<220>
   <221> source
   <223> /note="Description of Artificial Sequence: Synthetic peptide"
<400> 1725
   Ser Ala Ser Ser Ser Val Ser Tyr Met Asn
   1 5 10
<210> 1726
   <211> 5
   <212> PRT
   <213> Artificial Sequence
<220>
   <221> source
   <223> /note="Description of Artificial Sequence: Synthetic peptide"
<400> 1726
   Thr Tyr Ala Met Asn
   1               5
<210> 1727
   <211> 14
   <212> PRT
   <213> Artificial Sequence
<220>
   <221> source
   <223> /note="Description of Artificial Sequence: Synthetic peptide"
<400> 1727
   Arg Ser Ser Thr Gly Ala Val Thr Thr Ser Asn Tyr Ala Asn
   1               5                   10
<210> 1728
   <211> 5
   <212> PRT
   <213> Artificial Sequence
<220>
   <221> source
   <223> /note="Description of Artificial Sequence: Synthetic peptide"
<400> 1728
   Thr Tyr Ala Met Asn
   1               5
<210> 1729
   <211> 14
   <212> PRT
   <213> Artificial Sequence
<220>
   <221> source
   <223> /note="Description of Artificial Sequence: Synthetic peptide"
<400> 1729
   Arg Ser Ser Thr Gly Ala Val Thr Thr Ser Asn Tyr Ala Asn
   1               5                   10
<210> 1730
   <211> 5
   <212> PRT
   <213> Artificial Sequence
<220>
   <221> source
   <223> /note="Description of Artificial Sequence: Synthetic peptide"
<400> 1730
   Thr Tyr Ala Met Asn
   1 5
<210> 1731
   <211> 14
   <212> PRT
   <213> Artificial Sequence
<220>
   <221> source
   <223> /note="Description of Artificial Sequence: Synthetic peptide"
<400> 1731
   Gly Ser Ser Thr Gly Ala Val Thr Thr Ser Asn Tyr Ala Asn
   1 5 10
<210> 1732
   <211> 5
   <212> PRT
   <213> Artificial Sequence
<220>
   <221> source
   <223> /note="Description of Artificial Sequence: Synthetic peptide"
<400> 1732
   Thr Tyr Ala Met Asn
   1 5
<210> 1733
   <211> 14
   <212> PRT
   <213> Artificial Sequence
<220>
   <221> source
   <223> /note="Description of Artificial Sequence: Synthetic peptide"
<400> 1733
   Gly Ser Ser Thr Gly Ala Val Thr Thr Ser Asn Tyr Ala Asn
   1 5 10
<210> 1734
   <211> 5
   <212> PRT
   <213> Artificial Sequence
<220>
   <221> source
   <223> /note="Description of Artificial Sequence: Synthetic peptide"
<400> 1734
   Arg Tyr Thr Met His
   1 5
<210> 1735
   <211> 10
   <212> PRT
   <213> Artificial Sequence
<220>
   <221> source
   <223> /note="Description of Artificial Sequence: Synthetic peptide"
<400> 1735
   Ser Ala Ser Ser Ser Val Ser Tyr Met Asn
   1 5 10
<210> 1736
   <211> 5
   <212> PRT
   <213> Artificial Sequence
<220>
   <221> source
   <223> /note="Description of Artificial Sequence: Synthetic peptide"
<400> 1736
   Arg Tyr Thr Met His
   1 5
<210> 1737
   <211> 10
   <212> PRT
   <213> Artificial Sequence
<220>
   <221> source
   <223> /note="Description of Artificial Sequence: Synthetic peptide"
<400> 1737
   Ser Ala Ser Ser Ser Val Ser Tyr Met Asn
   1 5 10
<210> 1738
   <211> 17
   <212> PRT
   <213> Artificial Sequence
<220>
   <221> source
   <223> /note="Description of Artificial Sequence: Synthetic peptide"
<400> 1738
   Tyr Ile Asn Pro Ser Arg Gly Tyr Thr Asn Tyr Asn Gln Lys Phe Lys
   1 5 10 15
   Asp
<210> 1739
   <211> 7
   <212> PRT
   <213> Artificial Sequence
<220>
   <221> source
   <223> /note="Description of Artificial Sequence: Synthetic peptide"
<400> 1739
   Asp Thr Ser Lys Leu Ala Ser
   1 5
<210> 1740
   <211> 19
   <212> PRT
   <213> Artificial Sequence
<220>
   <221> source
   <223> /note="Description of Artificial Sequence: Synthetic peptide"
<400> 1740
   Arg Ile Arg Ser Lys Tyr Asn Asn Tyr Ala Thr Tyr Tyr Ala Asp Ser
   1 5 10 15
   Val Lys Asp
<210> 1741
   <211> 7
   <212> PRT
   <213> Artificial Sequence
<220>
   <221> source
   <223> /note="Description of Artificial Sequence: Synthetic peptide"
<400> 1741
   Gly Thr Asn Lys Arg Ala Pro
   1 5
<210> 1742
   <211> 17
   <212> PRT
   <213> Artificial Sequence
<220>
   <221> source
   <223> /note="Description of Artificial Sequence: Synthetic peptide"
<400> 1742
   Tyr Ile Asn Pro Ser Ser Gly Tyr Thr Lys Tyr Asn Gln Lys Phe Lys
   1               5                   10                  15
   Asp
<210> 1743
   <211> 7
   <212> PRT
   <213> Artificial Sequence
<220>
   <221> source
   <223> /note="Description of Artificial Sequence: Synthetic peptide"
<400> 1743
   Ala Thr Ser Asn Leu Ala Ser
   1               5
<210> 1744
   <211> 17
   <212> PRT
   <213> Artificial Sequence
<220>
   <221> source
   <223> /note="Description of Artificial Sequence: Synthetic peptide"
<400> 1744
   Tyr Ile Asn Pro Ser Arg Gly Tyr Thr Asn Tyr Asn Gln Lys Phe Lys
   1               5                   10                  15
   Asp
<210> 1745
   <211> 7
   <212> PRT
   <213> Artificial Sequence
<220>
   <221> source
   <223> /note="Description of Artificial Sequence: Synthetic peptide"
<400> 1745
   Asp Thr Ser Lys Val Ala Ser
   1 5
<210> 1746
   <211> 17
   <212> PRT
   <213> Artificial Sequence
<220>
   <221> source
   <223> /note="Description of Artificial Sequence: Synthetic peptide"
<400> 1746
   Tyr Ile Asn Pro Ser Arg Gly Tyr Thr Asn Tyr Asn Gln Lys Val Lys
   1 5 10 15
   Asp
<210> 1747
   <211> 7
   <212> PRT
   <213> Artificial Sequence
<220>
   <221> source
   <223> /note="Description of Artificial Sequence: Synthetic peptide"
<400> 1747
   Asp Thr Ser Lys Leu Ala Ser
   1 5
<210> 1748
   <211> 17
   <212> PRT
   <213> Artificial Sequence
<220>
   <221> source
   <223> /note="Description of Artificial Sequence: Synthetic peptide"
<400> 1748
   Val Ile Trp Tyr Asp Gly Ser Lys Lys Tyr Tyr Val Asp Ser Val Lys
   1 5 10 15
   Gly
<210> 1749
   <211> 7
   <212> PRT
   <213> Artificial Sequence
<220>
   <221> source
   <223> /note="Description of Artificial Sequence: Synthetic peptide"
<400> 1749
   Asp Ala Ser Asn Arg Ala Thr
   1 5
<210> 1750
   <211> 15
   <212> PRT
   <213> Artificial Sequence
<220>
   <221> source
   <223> /note="Description of Artificial Sequence: Synthetic peptide"
<400> 1750
   Arg Ile Arg Ser Lys Tyr Asn Asn Tyr Ala Thr Tyr Tyr Ala Asp
   1 5 10 15
<210> 1751
   <211> 7
   <212> PRT
   <213> Artificial Sequence
<220>
   <221> source
   <223> /note="Description of Artificial Sequence: Synthetic peptide"
<400> 1751
   Gly Thr Asn Lys Arg Ala Pro
   1 5
<210> 1752
   <211> 17
   <212> PRT
   <213> Artificial Sequence
<220>
   <221> source
   <223> /note="Description of Artificial Sequence: Synthetic peptide"
<400> 1752
   Tyr Ile Asn Pro Ser Arg Gly Tyr Thr Asn Tyr Asn Gln Lys Phe Lys
   1 5 10 15
   Asp
<210> 1753
   <211> 7
   <212> PRT
   <213> Artificial Sequence
<220>
   <221> source
   <223> /note="Description of Artificial Sequence: Synthetic peptide"
<400> 1753
   Asp Thr Ser Lys Val Ala Ser
   1               5
<210> 1754
   <211> 15
   <212> PRT
   <213> Artificial Sequence
<220>
   <221> source
   <223> /note="Description of Artificial Sequence: Synthetic peptide"
<400> 1754
   Arg Ile Arg Ser Lys Tyr Asn Asn Tyr Ala Thr Tyr Tyr Ala Asp
   1               5                   10                  15
<210> 1755
   <211> 7
   <212> PRT
   <213> Artificial Sequence
<220>
   <221> source
   <223> /note="Description of Artificial Sequence: Synthetic peptide"
<400> 1755
   Gly Thr Asn Lys Arg Ala Pro
   1               5
<210> 1756
   <211> 19
   <212> PRT
   <213> Artificial Sequence
<220>
   <221> source
   <223> /note="Description of Artificial Sequence: Synthetic peptide"
<400> 1756
   Arg Ile Arg Ser Lys Tyr Asn Asn Tyr Ala Thr Tyr Tyr Ala Asp Ser
   1               5                   10                  15
   Val Lys Asp
<210> 1757
   <211> 7
   <212> PRT
   <213> Artificial Sequence
<220>
   <221> source
   <223> /note="Description of Artificial Sequence: Synthetic peptide"
<400> 1757
   Gly Thr Asn Lys Arg Ala Pro
   1 5
<210> 1758
   <211> 19
   <212> PRT
   <213> Artificial Sequence
<220>
   <221> source
   <223> /note="Description of Artificial Sequence: Synthetic peptide"
<400> 1758
   Arg Ile Arg Ser Lys Tyr Asn Asn Tyr Ala Thr Tyr Tyr Ala Asp Ser
   1 5 10 15
   Val Lys Gly
<210> 1759
   <211> 7
   <212> PRT
   <213> Artificial Sequence
<220>
   <221> source
   <223> /note="Description of Artificial Sequence: Synthetic peptide"
<400> 1759
   Gly Thr Lys Phe Leu Ala Pro
   1 5
<210> 1760
   <211> 19
   <212> PRT
   <213> Artificial Sequence
<220>
   <221> source
   <223> /note="Description of Artificial Sequence: Synthetic peptide"
<400> 1760
   Arg Ile Arg Ser Lys Tyr Asn Asn Tyr Ala Thr Tyr Tyr Ala Asp Ser
   1 5 10 15
   Val Lys Asp
<210> 1761
   <211> 7
   <212> PRT
   <213> Artificial Sequence
<220>
   <221> source
   <223> /note="Description of Artificial Sequence: Synthetic peptide"
<400> 1761
   Gly Thr Lys Phe Leu Ala Pro
   1 5
<210> 1762
   <211> 17
   <212> PRT
   <213> Artificial Sequence
<220>
   <221> source
   <223> /note="Description of Artificial Sequence: Synthetic peptide"
<400> 1762
   Tyr Ile Asn Pro Ser Arg Gly Tyr Thr Asn Tyr Asn Gln Lys Phe Lys
   1 5 10 15
   Asp
<210> 1763
   <211> 7
   <212> PRT
   <213> Artificial Sequence
<220>
   <221> source
   <223> /note="Description of Artificial Sequence: Synthetic peptide"
<400> 1763
   Asp Thr Ser Lys Leu Ala Ser
   1 5
<210> 1764
   <211> 19
   <212> PRT
   <213> Artificial Sequence
<220>
   <221> source
   <223> /note="Description of Artificial Sequence: Synthetic peptide"
<400> 1764
   Arg Ile Arg Ser Lys Tyr Asn Asn Tyr Ala Thr Tyr Tyr Ala Asp Ser
   1               5                   10                  15
   Val Lys Asp
<210> 1765
   <211> 7
   <212> PRT
   <213> Artificial Sequence
<220>
   <221> source
   <223> /note="Description of Artificial Sequence: Synthetic peptide"
<400> 1765
   Gly Thr Asn Lys Arg Ala Pro
   1               5
<210> 1766
   <211> 19
   <212> PRT
   <213> Artificial Sequence
<220>
   <221> source
   <223> /note="Description of Artificial Sequence: Synthetic peptide"
<400> 1766
   Arg Ile Arg Ser Lys Tyr Asn Asn Tyr Ala Thr Tyr Tyr Ala Asp Ser
   1               5                   10                  15
   Val Lys Asp
<210> 1767
   <211> 7
   <212> PRT
   <213> Artificial Sequence
<220>
   <221> source
   <223> /note="Description of Artificial Sequence: Synthetic peptide"
<400> 1767
   Gly Thr Asn Lys Arg Ala Pro
   1               5
<210> 1768
   <211> 19
   <212> PRT
   <213> Artificial Sequence
<220>
   <221> source
   <223> /note="Description of Artificial Sequence: Synthetic peptide"
<400> 1768
   Arg Ile Arg Ser Lys Tyr Asn Asn Tyr Ala Thr Tyr Tyr Ala Asp Ser
   1 5 10 15
   Val Lys Gly
<210> 1769
   <211> 7
   <212> PRT
   <213> Artificial Sequence
<220>
   <221> source
   <223> /note="Description of Artificial Sequence: Synthetic peptide"
<400> 1769
   Gly Thr Asn Lys Arg Ala Pro
   1 5
<210> 1770
   <211> 19
   <212> PRT
   <213> Artificial Sequence
<220>
   <221> source
   <223> /note="Description of Artificial Sequence: Synthetic peptide"
<400> 1770
   Arg Ile Arg Ser Lys Tyr Asn Asn Tyr Ala Thr Tyr Tyr Ala Asp Ser
   1 5 10 15
   Val Lys Gly
<210> 1771
   <211> 7
   <212> PRT
   <213> Artificial Sequence
<220>
   <221> source
   <223> /note="Description of Artificial Sequence: Synthetic peptide"
<400> 1771
   Gly Thr Asn Lys Arg Ala Pro
   1 5
<210> 1772
   <211> 17
   <212> PRT
   <213> Artificial Sequence
<220>
   <221> source
   <223> /note="Description of Artificial Sequence: Synthetic peptide"
<400> 1772
   Tyr Ile Asn Pro Ser Arg Gly Tyr Thr Asn Tyr Asn Gln Lys Val Lys
   1               5                   10                  15
   Asp
<210> 1773
   <211> 7
   <212> PRT
   <213> Artificial Sequence
<220>
   <221> source
   <223> /note="Description of Artificial Sequence: Synthetic peptide"
<400> 1773
   Asp Thr Ser Lys Leu Ala Ser
   1               5
<210> 1774
   <211> 17
   <212> PRT
   <213> Artificial Sequence
<220>
   <221> source
   <223> /note="Description of Artificial Sequence: Synthetic peptide"
<400> 1774
   Tyr Ile Asn Pro Ser Arg Gly Tyr Thr Asn Tyr Asn Gln Lys Val Lys
   1               5                   10                  15
   Asp
<210> 1775
   <211> 7
   <212> PRT
   <213> Artificial Sequence
<220>
   <221> source
   <223> /note="Description of Artificial Sequence: Synthetic peptide"
<400> 1775
   Asp Thr Ser Lys Leu Ala Ser
   1               5
<210> 1776
   <211> 10
   <212> PRT
   <213> Artificial Sequence
<220>
   <221> source
   <223> /note="Description of Artificial Sequence: Synthetic peptide"
<400> 1776
   Tyr Tyr Asp Asp His Tyr Cys Leu Asp Tyr
   1               5                   10
<210> 1777
   <211> 9
   <212> PRT
   <213> Artificial Sequence
<220>
   <221> source
   <223> /note="Description of Artificial Sequence: Synthetic peptide"
<400> 1777
   Gln Gln Trp Ser Ser Asn Pro Phe Thr
   1               5
<210> 1778
   <211> 14
   <212> PRT
   <213> Artificial Sequence
<220>
   <221> source
   <223> /note="Description of Artificial Sequence: Synthetic peptide"
<400> 1778
   His Gly Asn Phe Gly Asn Ser Tyr Val Ser Trp Phe Ala Tyr
   1               5                   10
<210> 1779
   <211> 9
   <212> PRT
   <213> Artificial Sequence
<220>
   <221> source
   <223> /note="Description of Artificial Sequence: Synthetic peptide"
<400> 1779
   Ala Leu Trp Tyr Ser Asn Leu Trp Val
   1               5
<210> 1780
   <211> 10
   <212> PRT
   <213> Artificial Sequence
<220>
   <221> source
   <223> /note="Description of Artificial Sequence: Synthetic peptide"
<400> 1780
   Trp Gln Asp Tyr Asp Val Tyr Phe Asp Tyr
   1               5                   10
<210> 1781
   <211> 9
   <212> PRT
   <213> Artificial Sequence
<220>
   <221> source
   <223> /note="Description of Artificial Sequence: Synthetic peptide"
<400> 1781
   Gln Gln Trp Ser Ser Asn Pro Pro Thr
   1               5
<210> 1782
   <211> 10
   <212> PRT
   <213> Artificial Sequence
<220>
   <221> source
   <223> /note="Description of Artificial Sequence: Synthetic peptide"
<400> 1782
   Tyr Tyr Asp Asp His Tyr Cys Leu Asp Tyr
   1               5                   10
<210> 1783
   <211> 9
   <212> PRT
   <213> Artificial Sequence
<220>
   <221> source
   <223> /note="Description of Artificial Sequence: Synthetic peptide"
<400> 1783
   Gln Gln Trp Ser Ser Asn Pro Leu Thr
   1               5
<210> 1784
   <211> 10
   <212> PRT
   <213> Artificial Sequence
<220>
   <221> source
   <223> /note="Description of Artificial Sequence: Synthetic peptide"
<400> 1784
   Tyr Tyr Asp Asp His Tyr Cys Leu Asp Tyr
   1 5 10
<210> 1785
   <211> 9
   <212> PRT
   <213> Artificial Sequence
<220>
   <221> source
   <223> /note="Description of Artificial Sequence: Synthetic peptide"
<400> 1785
   Gln Gln Trp Ser Ser Asn Pro Phe Thr
   1 5
<210> 1786
   <211> 9
   <212> PRT
   <213> Artificial Sequence
<220>
   <221> source
   <223> /note="Description of Artificial Sequence: Synthetic peptide"
<400> 1786
   Gln Met Gly Tyr Trp His Phe Asp Leu
   1 5
<210> 1787
   <211> 10
   <212> PRT
   <213> Artificial Sequence
<220>
   <221> source
   <223> /note="Description of Artificial Sequence: Synthetic peptide"
<400> 1787
   Gln Gln Arg Ser Asn Trp Pro Pro Leu Thr
   1 5 10
<210> 1788
   <211> 16
   <212> PRT
   <213> Artificial Sequence
<220>
   <221> source
   <223> /note="Description of Artificial Sequence: Synthetic peptide"
<400> 1788
   Val Arg His Gly Asn Phe Gly Asn Ser Tyr Val Ser Trp Phe Ala Tyr
   1 5 10 15
<210> 1789
   <211> 9
   <212> PRT
   <213> Artificial Sequence
<220>
   <221> source
   <223> /note="Description of Artificial Sequence: Synthetic peptide"
<400> 1789
   Ala Leu Trp Tyr Ser Asn Leu Trp Val
   1 5
<210> 1790
   <211> 10
   <212> PRT
   <213> Artificial Sequence
<220>
   <221> source
   <223> /note="Description of Artificial Sequence: Synthetic peptide"
<400> 1790
   Tyr Tyr Asp Asp His Tyr Cys Leu Asp Tyr
   1 5 10
<210> 1791
   <211> 9
   <212> PRT
   <213> Artificial Sequence
<220>
   <221> source
   <223> /note="Description of Artificial Sequence: Synthetic peptide"
<400> 1791
   Gln Gln Trp Ser Ser Asn Pro Leu Thr
   1 5
<210> 1792
   <211> 16
   <212> PRT
   <213> Artificial Sequence
<220>
   <221> source
   <223> /note="Description of Artificial Sequence: Synthetic peptide"
<400> 1792
   Val Arg His Gly Asn Phe Gly Asn Ser Tyr Val Ser Trp Phe Ala Tyr
   1 5 10 15
<210> 1793
   <211> 9
   <212> PRT
   <213> Artificial Sequence
<220>
   <221> source
   <223> /note="Description of Artificial Sequence: Synthetic peptide"
<400> 1793
   Ala Leu Trp Tyr Ser Asn Leu Trp Val
   1 5
<210> 1794
   <211> 14
   <212> PRT
   <213> Artificial Sequence
<220>
   <221> source
   <223> /note="Description of Artificial Sequence: Synthetic peptide"
<400> 1794
   His Gly Asn Phe Gly Asn Ser Tyr Val Ser Trp Phe Ala Tyr
   1 5 10
<210> 1795
   <211> 9
   <212> PRT
   <213> Artificial Sequence
<220>
   <221> source
   <223> /note="Description of Artificial Sequence: Synthetic peptide"
<400> 1795
   Ala Leu Trp Tyr Ser Asn Leu Trp Val
   1 5
<210> 1796
   <211> 14
   <212> PRT
   <213> Artificial Sequence
<220>
   <221> source
   <223> /note="Description of Artificial Sequence: Synthetic peptide"
<400> 1796
   His Gly Asn Phe Gly Asn Ser Tyr Val Ser Trp Trp Ala Tyr
   1 5 10
<210> 1797
   <211> 9
   <212> PRT
   <213> Artificial Sequence
<220>
   <221> source
   <223> /note="Description of Artificial Sequence: Synthetic peptide"
<400> 1797
   Val Leu Trp Tyr Ser Asn Arg Trp Val
   1 5
<210> 1798
   <211> 14
   <212> PRT
   <213> Artificial Sequence
<220>
   <221> source
   <223> /note="Description of Artificial Sequence: Synthetic peptide"
<400> 1798
   His Gly Asn Phe Gly Asn Ser Tyr Ile Ser Tyr Trp Ala Tyr
   1 5 10
<210> 1799
   <211> 9
   <212> PRT
   <213> Artificial Sequence
<220>
   <221> source
   <223> /note="Description of Artificial Sequence: Synthetic peptide"
<400> 1799
   Val Leu Trp Tyr Ser Asn Arg Trp Val
   1 5
<210> 1800
   <211> 10
   <212> PRT
   <213> Artificial Sequence
<220>
   <221> source
   <223> /note="Description of Artificial Sequence: Synthetic peptide"
<400> 1800
   Tyr Tyr Asp Asp His Tyr Cys Leu Asp Tyr
   1 5 10
<210> 1801
   <211> 9
   <212> PRT
   <213> Artificial Sequence
<220>
   <221> source
   <223> /note="Description of Artificial Sequence: Synthetic peptide"
<400> 1801
   Gln Gln Trp Ser Ser Asn Pro Phe Thr
   1 5
<210> 1802
   <211> 14
   <212> PRT
   <213> Artificial Sequence
<220>
   <221> source
   <223> /note="Description of Artificial Sequence: Synthetic peptide"
<400> 1802
   His Gly Asn Phe Gly Asn Ser Tyr Val Ser Trp Phe Ala Tyr
   1 5 10
<210> 1803
   <211> 9
   <212> PRT
   <213> Artificial Sequence
<220>
   <221> source
   <223> /note="Description of Artificial Sequence: Synthetic peptide"
<400> 1803
   Ala Leu Trp Tyr Ser Asn Leu Trp Val
   1 5
<210> 1804
   <211> 14
   <212> PRT
   <213> Artificial Sequence
<220>
   <221> source
   <223> /note="Description of Artificial Sequence: Synthetic peptide"
<400> 1804
   His Gly Asn Phe Gly Asn Ser Tyr Val Ser Trp Phe Ala Tyr
   1 5 10
<210> 1805
   <211> 9
   <212> PRT
   <213> Artificial Sequence
<220>
   <221> source
   <223> /note="Description of Artificial Sequence: Synthetic peptide"
<400> 1805
   Ala Leu Trp Tyr Ser Asn Leu Trp Val
   1 5
<210> 1806
   <211> 14
   <212> PRT
   <213> Artificial Sequence
<220>
   <221> source
   <223> /note="Description of Artificial Sequence: Synthetic peptide"
<400> 1806
   His Gly Asn Phe Gly Asn Ser Tyr Val Ser Trp Phe Ala Tyr
   1 5 10
<210> 1807
   <211> 9
   <212> PRT
   <213> Artificial Sequence
<220>
   <221> source
   <223> /note="Description of Artificial Sequence: Synthetic peptide"
<400> 1807
   Ala Leu Trp Tyr Ser Asn Leu Trp Val
   1 5
<210> 1808
   <211> 14
   <212> PRT
   <213> Artificial Sequence
<220>
   <221> source
   <223> /note="Description of Artificial Sequence: Synthetic peptide"
<400> 1808
   His Gly Asn Phe Gly Asp Ser Tyr Val Ser Trp Phe Ala Tyr
   1 5 10
<210> 1809
   <211> 9
   <212> PRT
   <213> Artificial Sequence
<220>
   <221> source
   <223> /note="Description of Artificial Sequence: Synthetic peptide"
<400> 1809
   Ala Leu Trp Tyr Ser Asn His Trp Val
   1               5
<210> 1810
   <211> 10
   <212> PRT
   <213> Artificial Sequence
<220>
   <221> source
   <223> /note="Description of Artificial Sequence: Synthetic peptide"
<400> 1810
   Tyr Tyr Asp Asp His Tyr Cys Leu Asp Tyr
   1               5                   10
<210> 1811
   <211> 9
   <212> PRT
   <213> Artificial Sequence
<220>
   <221> source
   <223> /note="Description of Artificial Sequence: Synthetic peptide"
<400> 1811
   Gln Gln Trp Ser Ser Asn Pro Phe Thr
   1               5
<210> 1812
   <211> 10
   <212> PRT
   <213> Artificial Sequence
<220>
   <221> source
   <223> /note="Description of Artificial Sequence: Synthetic peptide"
<400> 1812
   Tyr Tyr Asp Asp His Tyr Ser Leu Asp Tyr
   1               5                   10
<210> 1813
   <211> 9
   <212> PRT
   <213> Artificial Sequence
<220>
   <221> source
   <223> /note="Description of Artificial Sequence: Synthetic peptide"
<400> 1813
   Gln Gln Trp Ser Ser Asn Pro Phe Thr
   1               5
<210> 1814
<400> 1814
   000
<210> 1815
<400> 1815
   000
<210> 1816
<400> 1816
   000
<210> 1817
<400> 1817
   000
<210> 1818
<400> 1818
   000
<210> 1819
<400> 1819
   000
<210> 1820
<400> 1820
   000
<210> 1821
<400> 1821
   000
<210> 1822
<400> 1822
   000
<210> 1823
<400> 1823
   000
<210> 1824
<400> 1824
   000
<210> 1825
<400> 1825
   000
<210> 1826
<400> 1826
   000
<210> 1827
<400> 1827
   000
<210> 1828
<400> 1828
   000
<210> 1829
<400> 1829
   000
<210> 1830
<400> 1830
   000
<210> 1831
<400> 1831
   000
<210> 1832
<400> 1832
   000
<210> 1833
<400> 1833
   000
<210> 1834
<400> 1834
   000
<210> 1835
<400> 1835
   000
<210> 1836
<400> 1836
   000
<210> 1837
<400> 1837
   000
<210> 1838
<400> 1838
   000
<210> 1839
<400> 1839
   000
<210> 1840
<400> 1840
   000
<210> 1841
<400> 1841
   000
<210> 1842
<400> 1842
   000
<210> 1843
<400> 1843
   000
<210> 1844
<400> 1844
   000
<210> 1845
<400> 1845
   000
<210> 1846
<400> 1846
   000
<210> 1847
<400> 1847
   000
<210> 1848
<400> 1848
   000
<210> 1849
<400> 1849
   000
<210> 1850
<400> 1850
   000
<210> 1851
<400> 1851
   000
<210> 1852
<400> 1852
   000
<210> 1853
<400> 1853
   000
<210> 1854
<400> 1854
   000
<210> 1855
<400> 1855
   000
<210> 1856
<400> 1856
   000
<210> 1857
<400> 1857
   000
<210> 1858
<400> 1858
   000
<210> 1859
<400> 1859
   000
<210> 1860
<400> 1860
   000
<210> 1861
<400> 1861
   000
<210> 1862
<400> 1862
   000
<210> 1863
<400> 1863
   000
<210> 1864
<400> 1864
   000
<210> 1865
<400> 1865
   000
<210> 1866
<400> 1866
   000
<210> 1867
<400> 1867
   000
<210> 1868
<400> 1868
   000
<210> 1869
<400> 1869
   000
<210> 1870
<400> 1870
   000
<210> 1871
<400> 1871
   000
<210> 1872
<400> 1872
   000
<210> 1873
<400> 1873
   000
<210> 1874
<400> 1874
   000
<210> 1875
<400> 1875
   000
<210> 1876
<400> 1876
   000
<210> 1877
<400> 1877
   000
<210> 1878
<400> 1878
   000
<210> 1879
<400> 1879
   000
<210> 1880
<400> 1880
   000
<210> 1881
<400> 1881
   000
<210> 1882
<400> 1882
   000
<210> 1883
<400> 1883
   000
<210> 1884
<400> 1884
   000
<210> 1885
<400> 1885
   000
<210> 1886
<400> 1886
   000
<210> 1887
<400> 1887
   000
<210> 1888
<400> 1888
   000
<210> 1889
<400> 1889
   000
<210> 1890
<400> 1890
   000
<210> 1891
<400> 1891
   000
<210> 1892
<400> 1892
   000
<210> 1893
<400> 1893
   000
<210> 1894
<400> 1894
   000
<210> 1895
<400> 1895
   000
<210> 1896
<400> 1896
   000
<210> 1897
<400> 1897
   000
<210> 1898
<400> 1898
   000
<210> 1899
<400> 1899
   000
<210> 1900
   <211> 119
   <212> PRT
   <213> Artificial Sequence
<220>
   <221> source
   <223> /note="Description of Artificial Sequence: Synthetic polypeptide"
<400> 1900
<210> 1901
   <211> 106
   <212> PRT
   <213> Artificial Sequence
<220>
   <221> source
   <223> /note="Description of Artificial Sequence: Synthetic polypeptide"
<400> 1901
<210> 1902
   <211> 105
   <212> PRT
   <213> Artificial Sequence
<220>
   <221> source
   <223> /note="Description of Artificial Sequence: Synthetic polypeptide"
<400> 1902
   Asp Ile Gln Leu Thr Gln Ser Pro Ala Ile Met Ser Ala Ser Pro Gly
   1 5 10 15
   Glu Lys Val Thr Met Thr Cys Arg Ala Ser Ser Ser Val Ser Tyr Met
   20 25 30
   Asn Trp Tyr Gln Gln Lys Ser Gly Thr Ser Pro Lys Arg Trp Ile Tyr
   35 40 45
   Asp Thr Ser Lys Val Ala Ser Gly Val Pro Tyr Arg Phe Ser Gly Ser
   50 55 60
   Gly Ser Gly Thr Ser Tyr Ser Leu Ile Ser Ser Met Glu Ala Glu Asp
   65 70 75 80
   Ala Ala Thr Tyr Tyr Cys Gln Gln Trp Ser Ser Asn Pro Leu Thr Phe
   85 90 95
   Gly Ala Gly Thr Lys Leu Glu Leu Lys
   100 105
<210> 1903
   <211> 119
   <212> PRT
   <213> Artificial Sequence
<220>
   <221> source
   <223> /note="Description of Artificial Sequence: Synthetic polypeptide"
<400> 1903
   Asp Ile Lys Leu Gln Gln Ser Gly Ala Glu Leu Ala Arg Pro Gly Ala
   1               5                   10                  15
   Ser Val Lys Met Ser Cys Lys Thr Ser Gly Tyr Thr Phe Thr Arg Tyr
               20                  25                  30
   Thr Met His Trp Val Lys Gln Arg Pro Gly Gln Gly Leu Glu Trp Ile
           35                  40                  45
   Gly Tyr Ile Asn Pro Ser Arg Gly Tyr Thr Asn Tyr Asn Gln Lys Phe
       50                  55                  60
   Lys Asp Lys Ala Thr Leu Thr Thr Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Tyr Tyr Asp Asp His Tyr Cys Leu Asp Tyr Trp Gly Gln Gly
               100                 105                 110
   Thr Thr Leu Thr Val Ser Ser
           115
<210> 1904
   <211> 109
   <212> PRT
   <213> Artificial Sequence
<220>
   <221> source
   <223> /note="Description of Artificial Sequence: Synthetic polypeptide"
<400> 1904
   Gln Ala Val Val Thr Gln Glu Ser Ala Leu Thr Thr Ser Pro Gly Glu
   1               5                   10                  15
   Thr Val Thr Leu Thr Cys Arg Ser Ser Thr Gly Ala Val Thr Thr Ser
               20                  25                  30
   Asn Tyr Ala Asn Trp Val Gln Glu Lys Pro Asp His Leu Phe Thr Gly
           35                  40                  45
   Leu Ile Gly Gly Thr Asn Lys Arg Ala Pro Gly Val Pro Ala Arg Phe
       50                  55                  60
   Ser Gly Ser Leu Ile Gly Asp Lys Ala Ala Leu Thr Ile Thr Gly Ala
   65                  70                  75                  80
   Gln Thr Glu Asp Glu Ala Ile Tyr Phe Cys Ala Leu Trp Tyr Ser Asn
                   85                  90                  95
   Leu Trp Val Phe Gly Gly Gly Thr Lys Leu Thr Val Leu
               100                 105
<210> 1905
   <211> 125
   <212> PRT
   <213> Artificial Sequence
<220>
   <221> source
   <223> /note="Description of Artificial Sequence: Synthetic polypeptide"
<400> 1905
   Glu Val Gln Leu Val Glu Ser Gly Gly Gly Leu Val Gln Pro Lys Gly
   1               5                   10                  15
   Ser Leu Lys Leu Ser Cys Ala Ala Ser Gly Phe Thr Phe Asn Thr Tyr
               20                  25                  30
   Ala Met Asn Trp Val Arg Gln Ala Pro Gly Lys Gly Leu Glu Trp Val
           35                  40                  45
   Ala Arg Ile Arg Ser Lys Tyr Asn Asn Tyr Ala Thr Tyr Tyr Ala Asp
       50                  55                  60
   Ser Val Lys Asp Arg Phe Thr Ile Ser Arg Asp Asp Ser Gln Ser Ile
   65                  70                  75                  80
   Leu Tyr Leu Gln Met Asn Asn Leu Lys Thr Glu Asp Thr Ala Met Tyr
                   85                  90                  95
   Tyr Cys Val Arg His Gly Asn Phe Gly Asn Ser Tyr Val Ser Trp Phe
               100                 105                 110
   Ala Tyr Trp Gly Gln Gly Thr Leu Val Thr Val Ser Ala
           115                 120                 125
<210> 1906
   <211> 107
   <212> PRT
   <213> Artificial Sequence
<220>
   <221> source
   <223> /note="Description of Artificial Sequence: Synthetic polypeptide"
<400> 1906
   Gln Ala Val Val Thr Gln Glu Pro Ser Leu Thr Val Ser Pro Gly Gly
   1               5                   10                  15
   Thr Val Thr Leu Thr Cys Arg Ser Ser Thr Gly Ala Val Thr Thr Ser
               20                  25                  30
   Asn Tyr Ala Asn Trp Val Gln Gln Lys Pro Gly Gln Ala Pro Arg Gly
           35                  40                  45
   Leu Ile Gly Gly Thr Asn Lys Arg Ala Pro Trp Thr Pro Ala Arg Phe
       50                  55                  60
   Ser Gly Ser Leu Leu Gly Gly Lys Ala Ala Leu Ile Gly Ala Gln Ala
   65                  70                  75                  80
   Glu Asp Glu Ala Asp Tyr Tyr Cys Ala Leu Trp Tyr Ser Asn Leu Trp
                   85                  90                  95
   Val Phe Gly Gly Gly Thr Lys Leu Thr Val Leu
               100                 105
<210> 1907
   <211> 124
   <212> PRT
   <213> Artificial Sequence
<220>
   <221> source
   <223> /note="Description of Artificial Sequence: Synthetic polypeptide"
<400> 1907
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Ala Met Asn Trp Val Arg Gln Ala Pro Gly Lys Gly Leu Glu Trp Val
           35                  40                  45
   Gly Arg Ile Arg Ser Lys Tyr Asn Asn Tyr Ala Thr Tyr Tyr Ala Asp
       50                  55                  60
   Ser Val Lys Asp Arg Phe Ile Ser Arg Asp Asp Ser Lys Asn Ser Leu
   65 70 75 80
   Tyr Leu Gln Met Asn Ser Leu Lys Thr Glu Asp Thr Ala Val Tyr Tyr
   85 90 95
   Cys Val Arg His Gly Asn Phe Gly Asn Ser Tyr Val Ser Trp Phe Ala
   100 105 110
   Tyr Trp Gly Gln Gly Thr Leu Val Thr Val Ser Ser
   115 120
<210> 1908
   <211> 107
   <212> PRT
   <213> Artificial Sequence
<220>
   <221> source
   <223> /note="Description of Artificial Sequence: Synthetic polypeptide"
<400> 1908
   Gln Ala Val Val Thr Gln Glu Pro Ser Leu Thr Val Ser Pro Gly Gly
   1 5 10 15
   Thr Val Thr Leu Thr Cys Arg Ser Ser Thr Gly Ala Val Thr Thr Ser
   20 25 30
   Asn Tyr Ala Asn Trp Val Gln Gln Lys Pro Gly Gln Ala Pro Arg Gly
   35 40 45
   Leu Ile Gly Gly Thr Asn Lys Arg Ala Pro Trp Thr Pro Ala Arg Phe
   50 55 60
   Ser Gly Ser Leu Leu Gly Gly Lys Ala Ala Leu Ile Gly Ala Gln Ala
   65 70 75 80
   Glu Asp Glu Ala Asp Tyr Tyr Cys Ala Leu Trp Tyr Ser Asn Leu Trp
   85 90 95
   Val Phe Gly Gly Gly Thr Lys Leu Thr Val Leu
   100 105
<210> 1909
   <211> 124
   <212> PRT
   <213> Artificial Sequence
<220>
   <221> source
   <223> /note="Description of Artificial Sequence: Synthetic polypeptide"
<400> 1909
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asn Thr Tyr
               20                  25                  30
   Ala Met Asn Trp Val Arg Gln Ala Pro Gly Lys Gly Leu Glu Trp Val
           35                  40                  45
   Ala Arg Ile Arg Ser Lys Tyr Asn Asn Tyr Ala Thr Tyr Tyr Ala Asp
       50                  55                  60
   Ser Val Lys Asp Arg Phe Ile Ser Arg Asp Asp Ser Lys Asn Ser Leu
   65                  70                  75                  80
   Tyr Leu Gln Met Asn Ser Leu Lys Thr Glu Asp Thr Ala Val Tyr Tyr
                   85                  90                  95
   Cys Val Arg His Gly Asn Phe Gly Asn Ser Tyr Val Ser Trp Phe Ala
               100                 105                 110
   Tyr Trp Gly Gln Gly Thr Leu Val Thr Val Ser Ser
           115                 120
<210> 1910
   <211> 107
   <212> PRT
   <213> Artificial Sequence
<220>
   <221> source
   <223> /note="Description of Artificial Sequence: Synthetic polypeptide"
<400> 1910
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Ser Ser Tyr
   20 25 30
   Leu Asn Trp Tyr Gln Gln Lys Pro Gly Lys Ala Pro Lys Leu Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Ser Tyr Ser Thr Pro Pro
   85 90 95
   Thr Phe Gly Gln Gly Thr Lys Val Glu Ile Lys
   100 105
<210> 1911
   <211> 118
   <212> PRT
   <213> Artificial Sequence
<220>
   <221> source
   <223> /note="Description of Artificial Sequence: Synthetic polypeptide"
<400> 1911
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Arg Ser Tyr
               20                  25                  30
   Gly Met His Trp Val Arg Gln Ala Pro Gly Lys Gly Leu Glu Trp Val
           35                  40                  45
   Ala Ile Ile Trp Tyr Ser Gly Ser Lys Lys Asn Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Thr Gly Tyr Asn Trp Phe Asp Pro Trp Gly Gln Gly Thr
               100                 105                 110
   Leu Val Thr Val Ser Ser
           115

## Description

### Field of the Invention

The present invention relates to the field of immunology. Specifically, the invention relates to multispecific and/or multivalent molecules targeting C-type lectin-like-1 (CLL-1) and methods of making and using thereof.

### Related Applications

This application claims priority to PCT Application No. PCT/CN2016/071599, filed January 21, 2016.

### Background of the Invention

Multispecific molecules that are capable of binding two or more antigens are known in the art and offer significant clinical benefits, for example, for diagnostic enzyme assays, as vaccine adjuvants, for delivering thrombolytic agents, for treating diseases, for targeting immune complexes to cell surface receptors, or for delivering immunotoxins to tumor cells, etc. (Burrows, F. J. and Thorpe, P. E. (1993) Proc Natl Acad Sci USA 90:8996 9000; Zhu, Z. et al. (1996) Bio/Technology 14:192 196; Holliger, P. et al. (1996) Protein Engin. 9:299 305; Morrison et al., (1997) Nature Biotech. 15:159-163; Huang, X. et al. (1997) Science 275:547 550Alt et al. (1999) FEBS Letters 454: 90-94; Zuo et al., (2000) Protein Engineering 13:361-367; Lu et al., (2004) JBC 279:2856-2865; Lu et al., (2005) JBC 280:19665-19672; Marvin et al., (2005) Acta Pharmacologica Sinica 26:649-658; Marvin et al., (2006) Curr Opin Drug Disc Develop 9:184-193; Shen et al., (2007) J Immun Methods 218:65-74; Wu et al., (2007) Nat Biotechnol. 11:1290-1297; Dimasi et al., (2009) J Mol Biol. 393:672-692; and Michaelson et al., (2009) mAbs 1:128-141).

Most patients with acute myeloid leukemia (AML) are incurable using standard therapy (Mrozek et al, 2012, J Clin Oncol, 30:4515-23) and those with relapsed or refractory AML (RR-AML) have a particularly poor prognosis (Kern et al, 2003, Blood 2003, 101:64-70; Wheatley et al, 1999, Br J Haematol, 107:69-79). C-type lectin-like-1 (CLL-1) is also known as MICL, CLEC12A, CLEC-1, Dendritic Cell-Associated Lectin 1, and DCAL-2. CLL-1 is a glycoprotein receptor and member of the large family of C-type lectin-like receptors involved in immune regulation. CLL-1 is expressed in hematopoietic cells, primarily on innate immune cells including monocytes, DCs, pDCs, and granulocytes (Cancer Res. 2004; J Immunol 2009) and myeloid progenitor cells (Blood, 2007). CLL-1 is also found on acute myeloid leukemia (AML) blasts and leukemic stem cells (e.g., CD34+/CD38-) (Zhao et al., Haematologica. 2010, 95(1):71-78.). CLL-1 expression may also be relevant for other myeloid leukemias, such as acute myelomonocytic leukemia, acute monocytic leukemia, acute promyelocytic leukemia, chronic myeloid leukemia (CML), and myelodysplastic syndrome (MDS).

WO2014153002 discloses bispecific antibodies with an anti-CD3 antibody or antibody fragment and a second antibody or antibody fragment, directed to a variety of targets, including CLL.

There is, however, an unmet medical need for multispecific molecules which target CLL-1.

### Summary of the Invention

In one aspect the invention relates to a multispecific molecule including a first antigen binding domain and a second antigen binding domain, wherein the first antigen binding domain is an anti-CLL-1 binding domain and the second antigen binding domain binds an immune effector cell antigen that is an antigen expressed on a T cell, wherein the antigen expressed on a T cell is CD3, and wherein the multispecific molecule is as further defined in the claims.

Also disclosed herein is a multispecific molecule including a first antigen binding domain and a second antigen binding domain, wherein the first antigen binding domain is an anti-CLL-1 binding domain including a heavy chain complementary determining region 1 (HC CDR1), a heavy chain complementary determining region 2 (HC CDR2), and a heavy chain complementary determining region 3 (HC CDR3) of any anti-CLL-1 binding domain amino acid sequence listed in Table 2.

In embodiments of the disclosure, the anti-CLL-1 binding domain further comprises a light chain complementary determining region 1 (LC CDR1), a light chain complementary determining region 2 (LC CDR2), and a light chain complementary determining region 3 (LC CDR3) of any anti-CLL-1 binding domain amino acid sequence listed in Table 2.

In embodiments of the disclosure, including the multispecific molecule of the preceding aspects or embodiments, the anti-CLL-1 binding domain includes LC CDR1, LC CDR2, and LC CDR3 that are the LC CDR sequences listed in Table 4, 6 or 8.

In embodiments of the disclosure, including in each of the preceding aspects or embodiments, the anti-CLL-1 binding domain includes HC CDR1, HC CDR2 and HC CDR3 that are the HC CDR sequences listed in Table 3, 5 or 7.

In embodiments of the disclosure, including in each of the preceding aspects or embodiments, the anti-CLL-1 binding domain of the multispecific molecule includes:
(i) the amino acid sequence of any light chain variable region listed in Table 2;
(ii) an amino acid sequence having at least one, two or three modifications but not more than 30, 20 or 10 modifications of the amino acid sequence of any of the light chain variable regions provided in Table 2; or
(iii) an amino acid sequence with 95-99% identity to the amino acid sequence of any of the light chain variable regions provided in Table 2.

In embodiments of the disclosure, including in each of the preceding aspects or embodiments, the anti-CLL-1 binding domain of the multispecific molecule includes:
(i) the amino acid sequence of any heavy chain variable region listed in Table 2;
(ii) an amino acid sequence having at least one, two or three modifications but not more than 30, 20 or 10 modifications of the amino acid sequence of any of the heavy chain variable regions provided in Table 2; or
(ii) an amino acid sequence with 95-99% identity to the amino acid sequence of any of the heavy chain variable regions provided in Table 2.

In embodiments of the disclosure, including in each of the preceding aspects or embodiments, the multispecific molecule includes an anti-CLL-1 binding domain that includes the amino acid sequence of any light chain variable region listed in Table 2, and the amino acid sequence of any heavy chain variable region listed in Table 2. In embodiments, the anti-CLL-1 binding domain that includes the amino acid sequence of the light chain variable region and the amino acid sequence of the heavy chain variable region of any anti-CLL-1 binding domain listed in Table 2, e.g., of any of CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, or CLL-1-13.

In embodiments of the disclosure, including in each of the preceding aspects or embodiments, the anti-CLL-1 binding domain of the multispecific molecule includes:
(i) any amino acid sequence of any of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, or SEQ ID NO: 77;
(ii) an amino acid sequence having at least one, two or three modifications but not more than 30, 20 or 10 modifications to any of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, or SEQ ID NO: 77; or
(iii) an amino acid sequence with 95-99% identify to any of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, or SEQ ID NO: 77.

In aspects of the invention, where said anti-CD3 binding domain includes a VL sequence of SEQ ID NO: 1209 and a VH sequence of SEQ ID NO: 1236, the multispecific molecule includes an anti-CLL-1 binding domain that includes:
a) a VL sequence of SEQ ID NO: 88 and a VH sequence of SEQ ID NO: 75;
b) a VL sequence of SEQ ID NO: 89 and a VH sequence of SEQ ID NO: 76;
c) a VL sequence of SEQ ID NO: 87 and a VH sequence of SEQ ID NO: 74;
d) a VL sequence of SEQ ID NO: 85 and a VH sequence of SEQ ID NO: 72;
e) a VL sequence of SEQ ID NO: 86 and a VH sequence of SEQ ID NO: 73;
f) a VL sequence of SEQ ID NO: 83 and a VH sequence of SEQ ID NO: 70;
g) a VL sequence of SEQ ID NO: 90 and a VH sequence of SEQ ID NO: 77;
h) a VL sequence of SEQ ID NO: 79 and a VH sequence of SEQ ID NO: 66; or
i) a VL sequence of SEQ ID NO: 84 and a VH sequence of SEQ ID NO: 71.

In aspects of the invention, where said anti-CD3 binding domain includes SEQ ID NO: 506; the multispecific molecule includes an anti-CLL-1 binding domain that includes:
a) SEQ ID NO: 49;
b) SEQ ID NO: 50;
c) SEQ ID NO: 48;
d) SEQ ID NO: 46;
e) SEQ ID NO: 47;
f) SEQ ID NO: 44;
g) SEQ ID NO: 51;
h) SEQ ID NO: 40; or
i) SEQ ID NO: 45.

In aspects of the invention, where said anti-CD3 binding domain includes, e.g., consists of, SEQ ID NO: 507; the multispecific molecule includes a polypeptide comprising an anti-CLL-1 binding domain that includes, e.g., consists of:
a) SEQ ID NO: 512;
b) SEQ ID NO: 517;
c) SEQ ID NO: 522;
d) SEQ ID NO: 527;
e) SEQ ID NO: 532;
f) SEQ ID NO: 537;
g) SEQ ID NO: 542;
h) SEQ ID NO: 547; or
i) SEQ ID NO: 552.

In another aspect the disclosure relates to multispecific molecules, e.g., anti-CLL-1 multispecific molecules, including as described in each of the preceding aspects or embodiments, including a second antigen-binding domain that binds a cancer antigen other than CLL-1. In embodiments of the disclosure, the cancer antigen other than CLL-1 is expressed on a cell that also expresses CLL-1. In embodiments of the disclosure, the cancer antigen other than CLL-1 is expressed on an acute myeloid leukemia (AML), e.g., is an AML cell antigen. In embodiments of the disclosure, the cancer antigen is selected from the group consisting of CD123, CD33, CD34, FLT3, and folate receptor beta.

In another aspect the disclosure relates to multispecific molecules, e.g., anti-CLL-1 multispecific molecules, including as described in each of the preceding aspects or embodiments, including a second antigen-binding domain that binds an immune effector cell antigen. In embodiments of the disclosure, the immune effector cell antigen is an antigen expressed on a NK cell, e.g., is CD16 (Fc Receptor gamma III) or CD64 (Fc Receptor gamma I). In other embodiments of the disclosure, the immune effector cell antigen is an antigen expressed on a T cell, e.g., is an immune costimulatory molecule, e.g., is selected from the group consisting of an MHC class I molecule, a TNF receptor protein, an immunoglobulin-like protein, a cytokine receptor, an integrin, a signaling lymphocytic activation molecule (SLAM protein), an activating NK cell receptor, BTLA, Toll ligand receptor, OX40, CD2, CD7, CD27, CD28, CD30, CD40, CD47, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), TLR7, LTBR, LAT, GADS, SLP-76, PAG/Cbp, CD19a, and a ligand that specifically binds with CD83. In other embodiments of the disclosure, the immune effector cell antigen is an antigen expressed on a T cell, e.g., is an immune inhibitory molecule, e.g., is selected from the group consisting of PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta.

In embodiments according to the invention, the immune effector cell antigen is an antigen expressed on a T cell is CD3. In embodiments of the disclosure, the anti-CD3 binding domain includes a heavy chain complementary determining region 1 (HC CDR1), a heavy chain complementary determining region 2 (HC CDR2), and a heavy chain complementary determining region 3 (HC CDR3) of any anti-CD3 binding domain VH amino acid sequence listed in Table 26. In embodiments of the disclosure, said anti-CD3 binding domain further includes a light chain complementary determining region 1 (LC CDR1), a light chain complementary determining region 2 (LC CDR2), and a light chain complementary determining region 3 (LC CDR3) of any anti-CD3 binding domain VL amino acid sequence listed in Table 26.

In embodiments of the disclosure, said LC CDR1, LC CDR2, and LC CDR3 are the LC CDR sequences of any anti-CD3 binding domain listed in Table 27, 28 or 29. In embodiments of the disclosure, said HC CDR1, HC CDR2 and HC CDR3 are the HC CDR sequences of any anti-CD3 binding domain listed in Table 27, 28 or 29.

In embodiments of the disclosure, the anti-CD3 binding domain includes:
(i) the amino acid sequence of any light chain variable region listed in Table 26;
(ii) an amino acid sequence having at least one, two or three modifications but not more than 30, 20 or 10 modifications of the amino acid sequence of any of the light chain variable regions provided in Table 26; or
(iii) an amino acid sequence with 95-99% identity to the amino acid sequence of any of the light chain variable regions provided in Table 26.

In embodiments of the disclosure, the anti-CD3 binding domain includes:
(i) the amino acid sequence of any heavy chain variable region listed in table 26;
(ii) an amino acid sequence having at least one, two or three modifications but not more than 30, 20 or 10 modifications of the amino acid sequence of any of the heavy chain variable regions provided in Table 26; or
(iii) an amino acid sequence with 95-99% identity to the amino acid sequence of any of the heavy chain variable regions provided in Table 26.

In embodiments of the disclosure, the anti-CD3 binding domain includes the amino acid sequence of any light chain variable region listed in Table 26, and the amino acid sequence of any heavy chain variable region listed in Table 26. In embodiments of the disclosure, the anti-CD3 binding domain includes the amino acid sequence of any light chain variable region and the amino acid sequence of any heavy chain variable region of any anti-CD3 binding domain listed in Table 26. In embodiments of the disclosure, the anti-CD3 binding domain includes a heavy chain variable region amino acid sequence selected from the group consisting of SEQ ID NO: 1200, 1202, 1204, 1206, 1208, 1210, 1212, 1214, 1216, 1218, 1220, 1222, 1224, 1226, 1228, 1230, 1232, 1234, and 1236; and a light chain variable amino acid sequence selected from the group consisting of SEQ ID NO: 1201, 1203, 1205, 1207, 1209, 1211, 1213, 1215, 1217, 1219, 1221, 1223, 1225, 1227, 1229, 1231, 1233, 1235, and 1237.

In one aspect, including in any of the preceding aspects or embodiments, the multispecific molecule is multivalent, e.g., bivalent, with respect to the first antigen binding domain or second antigen binding domain. In embodiments, the multispecific molecule is bivalent with respect to the anti-CLL-1 binding domain. In embodiments, the two or more anti-CLL-1 binding domains bind to the same epitope of CLL-1, e.g., include the same anti-CLL-1 binding domain (e.g., the same CDRs, the same VH and VL or the same scFv sequences). In embodiments, the two or more anti-CLL-1 binding domains bind to different epitopes of CLL-1.

The disclosure provides for the multispecific molecules in a variety of formats. In one aspect, the disclosure provides for the multispecific molecule of any of the preceding embodiments as a bispecific antibody. In one aspect, the disclosure provides for the multispecific molecule of any of the preceding embodiments as a dualbody. In one aspect, the disclosure provides for the multispecific molecule of any of the preceding embodiments as a scFv-Fc. In one aspect, the disclosure provides for the multispecific molecule of any of the preceding embodiments as a mixed chain multispecific molecule. In one aspect, the disclosure provides for the multispecific molecule of any of the preceding embodiments as a tandem scFv. Each of these formats is described in more detail below.

The disclosure further provides for bispecific molecules of any of the preceding embodiments.

In embodiments, including in any of the preceding aspects and embodiments that include one or more heavy chain constant domains, the heavy chain constant domain may be selected from SEQ ID NO: 500, SEQ ID NO: 501, SEQ ID NO: 504 and SEQ ID NO: 505. In a preferred embodiment, the antigen binding domains of the multispecific molecule are scFvs, and the first heavy chain constant domain includes, e.g., is, SEQ ID NO: 500, and the second heavy chain constant domain includes, e.g., is, SEQ ID NO: 501. In another preferred embodiment, the antigen binding domains are Fabs, and the first heavy chain constant domain comprises, e.g., is, SEQ ID NO: 504, and the first light chain constant domain comprises, e.g., is, SEQ ID NO: 502; and the second heavy chain constant domain comprises, e.g., is, SEQ ID NO: 505, and the first light chain constant domain comprises, e.g., is, SEQ ID NO: 503.

In an embodiment the invention relates to multispecific molecules, including of any of the preceding embodiments of the invention, further including CH3, and optionally, CH2 domains. In one aspect, including in any of the preceding embodiments and aspects, the polypeptide(s) of the multispecific molecules that include the HC CDR sequences of the first and/or second antigen binding domains further include a CH3 domain, and optionally a CH2 domain. The disclosure further provides multispecific molecules, including of any of the preceding embodiments, wherein at least one, e.g., both, of said CH3 domains include one or more modifications to enhance heterodimerization, e.g., as described herein.

In one aspect of the invention, the multispecific molecule that includes one or more modifications to enhance heterodimerization includes introduction of a knob in a first CH3 domain and a hole in a second CH3 domain, or vice versa, such that heterodimerization of the polypeptide that includes the first CH3 domain and the polypeptide that includes the second CH3 domain is favored relative to polypeptides that include only unmodified CH3 domains.

In embodiments, the knob or hole is introduced to the first CH3 domain at residue 366, 405 or 407 according to the EU numbering scheme of Kabat et al. (pp. 688-696 in Sequences of proteins of immunological interest, 5th ed., Vol. 1 (1991; NIH, Bethesda, Md.)) to create either a knob or hole, and a complimentary hole or knob is introduced to the second CH3 domain at residue 407 if residue 366 is mutated in the first CH3 domain, residue 394 if residue 405 is mutated in the first CH3 domain, or residue 366 if residue 407 is mutated in the first CH3 domain, according to the EU numbering scheme of Kabat et al. (pp. 688-696 in Sequences of proteins of immunological interest, 5th ed., Vol. 1 (1991; NIH, Bethesda, Md.)).

In embodiments, the first CH3 domain includes introduction of a knob at position 366, and the second CH3 domain includes introduction of a hole at position 366, position 368 and position 407, according to the EU numbering scheme of Kabat et al. (pp. 688-696 in Sequences of proteins of immunological interest, 5th ed., Vol. 1 (1991; NIH, Bethesda, Md.)), or vice versa.

In embodiments, the first CH3 domain includes a tyrosine or tryptophan at position 366, and the second CH3 domain includes a serine at position 366, alanine at position 368 and valine at position 407, according to the EU numbering scheme of Kabat et al. (pp. 688-696 in Sequences of proteins of immunological interest, 5th ed., Vol. 1 (1991; NIH, Bethesda, Md.)), or vice versa.

In another aspect, the one or more modifications to enhance heterodimerization include IgG heterodimerization modifications. In embodiments, the first CH3 domain includes the mutation K409R and the second CH3 domain includes the mutation F405L, or vice versa.

In another aspect, the one or more modifications to enhance heterodimerization include polar bridge modifications. In embodiments, said one or more modifications include modifications to the first CH3 domain that are selected from a group consisting of: S364L, T366V, L368Q, D399K, F405S, K409F, T411K, and combinations thereof. In embodiments, including those that include first CH3 domain mutations, the second CH3 domain may include one or more modifications that are selected from the group consisting of Y407F, K409Q and T411D, and combinations thereof.

In another aspect, the multispecific molecule of any of the preceding aspects or embodiments may further include a first CH3 domain including a cysteine capable for forming a disulfide bond with a cysteine of the second CH3 domain. In embodiments, the first CH3 domain includes a cysteine at position 354 and the second CH3 domain includes a cysteine at position 349, or vice versa.

In another aspect, the multispecific molecule of any of the preceding aspects or embodiments may further (or alternatively) include a constant domain that comprises one or more mutations to reduce, e.g., silence, antibody-dependent cell-mediated cytotoxicity (ADCC) and/or compliment-dependent cytotoxicity (CDC). In an embodiment, the one or more mutations to reduce, e.g., silence ADCC and/or CDC include, e.g., are, the DAPA mutations (e.g., D265A and P329A, according to EU numbering). In an embodiment, the one or more mutations to reduce, e.g., silence ADCC and/or CDC include, e.g., are, the LALA mutations (e.g., L234A and L235A, according to EU numbering). In an embodiment, the one or more mutations to reduce, e.g., silence ADCC and/or CDC include, e.g., is, N279A, according to EU numbering. Constant domains comprising combinations of any of the above may also be included.

In another aspect, the disclosure provides isolated nucleic acid, e.g., one or more polynucleotides, encoding the multispecific molecule of any of the preceding aspects or embodiments. In embodiments, the isolated nucleic acid is disposed on a single continuous polynucleotide. In other embodiments, the isolated polynucleotide is disposed on two or more continuous polynucleotides.

In embodiments, the nucleic acid includes sequence encoding an anti-CD3 binding domain. In embodiments of the invention, including in any of the aforementioned nucleic acid aspects and embodiments of the invention, the nucleic acid includes SEQ ID NO: 508 and SEQ ID NO: 509.

In embodiments of the invention, including in any of the aforementioned nucleic acid aspects and embodiments of the invention, the isolated nucleic acid includes SEQ ID NO: 510.

In embodiments of the invention, including in any of the aforementioned nucleic acid aspects and embodiments of the invention, the isolated nucleic acid includes SEQ ID NO: 511.

In embodiments of the invention, the nucleic acid includes sequence encoding an anti-CLL-1 binding domain as defined in the claims. In embodiments, including in any of the aforementioned nucleic acid aspects and embodiments, the isolated nucleic acid includes:
a) SEQ ID NO: 513 and SEQ ID NO: 514;
b) SEQ ID NO: 518 and SEQ ID NO: 519;
c) SEQ ID NO: 523 and SEQ ID NO: 524;
d) SEQ ID NO: 528 and SEQ ID NO: 529;
e) SEQ ID NO: 533 and SEQ ID NO: 534;
f) SEQ ID NO: 538 and SEQ ID NO: 539;
g) SEQ ID NO: 543 and SEQ ID NO: 544;
h) SEQ ID NO: 548 and SEQ ID NO: 549; or
i) SEQ ID NO: 553 and SEQ ID NO: 554.

In embodiments of the invention, including in any of the aforementioned nucleic acid aspects and embodiments of the invention, the isolated nucleic acid includes:
a) SEQ ID NO: 515;
b) SEQ ID NO: 520;
c) SEQ ID NO: 525;
d) SEQ ID NO: 530;
e) SEQ ID NO: 535;
f) SEQ ID NO: 540;
g) SEQ ID NO: 545;
h) SEQ ID NO: 550; or
i) SEQ ID NO: 555.

In embodiments of the invention, including in any of the aforementioned nucleic acid aspects and embodiments of the invention, the isolated nucleic acid includes:
a) SEQ ID NO: 516;
b) SEQ ID NO: 521;
c) SEQ ID NO: 526;
d) SEQ ID NO: 531;
e) SEQ ID NO: 536;
f) SEQ ID NO: 541;
g) SEQ ID NO: 546;
h) SEQ ID NO: 551; or
i) SEQ ID NO: 556.

In embodiments, the isolated nucleic acid includes sequence encoding an anti-CD3 binding domain, for example, as described herein, and sequence encoding an anti-CLL-1 binding domain, for example, as described herein. In embodiments, the sequence encoding the anti-CD3 binding domain and the sequence encoding the anti-CLL-1 binding domain are disposed on separate polynucleotides. In embodiments, the sequence encoding the anti-CD3 binding domain and the sequence encoding the anti-CLL-1 binding domain are disposed on a single polynucleotide.

In another aspect, the disclosure provides a vector e.g., one or more vectors, that includes the isolated nucleic acid described above.

In another aspect, the disclosure provides a cell including the isolated nucleic acid or vector described above.

In another aspect, the disclosure provides for methods of treatment. In one aspect, the disclosure provides a method of treating a mammal having a disease associated with expression of CLL-1 including administering to the mammal an effective amount of a multispecific molecule of any of the preceding aspects or embodiments, the isolated nucleic acid described herein, the vector described herein or the cell described herein.

In embodiments, the disease associated with CLL-1 expression is:
(i) a cancer or malignancy, or a precancerous condition chosen from one or more of a myelodysplasia, a myelodysplastic syndrome or a preleukemia, or
(ii) a non-cancer related indication associated with expression of CLL-1.

In embodiments, the disease is a hematologic cancer. In embodiments the disease is acute myeloid leukemia (AML), acute lymphoblastic B-cell leukemia (B-cell acute lymphoid leukemia, BALL), acute lymphoblastic T-cell leukemia (T-cell acute lymphoid leukemia (TALL), B-cell prolymphocytic leukemia, chronic lymphocytic leukemia, chronic myeloid leukemia (CML), myelodysplastic syndrome, plasma cell myeloma, or a combination thereof. In embodiments, the disease is acute myeloid leukemia (AML).

In another aspect, the disclosure provides for the use of the multispecific molecule of any of the preceding aspects or embodiments, the isolated nucleic acid described above, the vector described above or the cell described above, in the manufacture of a medicament.

In another aspect, the invention relates to the multispecific molecule as defined in the claims, for use as a medicament.

In another aspect, the invention relates to the multispecific molecule as defined in the claims, for use in a therapy.

In another aspect, the invention relates to the multispecific molecule as defined in the claims, for use in treating a subject having a hematologic cancer.

In another aspect, the invention relates to the multispecific molecule as defined in the claims, for use in treating a subject having acute myeloid leukemia (AML).

In another aspect, the invention relates to compositions including the multispecific molecule as defined in the claims. In embodiments, the composition is a pharmaceutical composition. In embodiments, the compositions may further include a pharmaceutically acceptable diluent or carrier. In embodiments, the compositions include a therapeutically effective amount of the multispecific molecule as defined in the claims.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. Headings, sub-headings or numbered or lettered elements, e.g., (a), (b), (i) etc, are presented merely for ease of reading. The use of headings or numbered or lettered elements in this document does not require the steps or elements be performed in alphabetical order or that the steps or elements are necessarily discrete from one another.

### Brief Description of the Drawings

FIG. 1 shows various exemplary formats for multispecific, e.g., bispecific, molecules that incorporate an anti-CLL-1 binding domain, e.g., a human or humanized anti-CLL-1 binding domain.
FIG 2. Shows *in vitro* T cell killing of CLL1-expressing cancer cell line HL60 with CD3 x CLL1 bispecific antibodies.
FIG 3. Shows CD3 x CLL1 bispecific antibody-mediated T cell activation in vitro via engagement with CLL1-expressing cancer cell line U937, as demonstrated through an NFAT luciferase reporter gene in the Jurkat human T cell line.

### Detailed Description of the Invention

### Definitions

In order that the present invention may be more readily understood, certain terms are first defined. Additional definitions are set forth throughout the detailed description. Unless defined otherwise, all technical and scientific terms used herein have the same meaning as commonly understood by those of ordinary skill in the art to which this invention pertains.

The term "antibody" as used herein refers to a polypeptide (or set of polyptptides) of the immunoglobulin family that is capable of binding an antigen non-covalently, reversibly and specifically. For example, a naturally occurring "antibody" of the IgG type is a tetramer comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CHI, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen, which is sometimes referred to herein as the antigen binding domain. The constant regions of the antibodies may mediate the binding of the immunoglobulin to host tissues or factors, including various cells of the immune system (e.g., effector cells) and the first component (Clq) of the classical complement system. The term "antibody" includes, but is not limited to, monoclonal antibodies, human antibodies, humanized antibodies, camelised antibodies, chimeric antibodies, bispecific or multispecific antibodies and anti-idiotypic (anti-Id) antibodies (including, e.g., anti-Id antibodies to antibodies of the invention). The antibodies can be of any isotype/class (e.g., IgG, IgE, IgM, IgD, IgA and IgY) or subclass (e.g., IgG1, IgG2, IgG3, IgG4, IgA1 and IgA2).

Both the light and heavy chains are divided into regions of structural and functional homology. The terms "constant" and "variable" are used functionally. In this regard, it will be appreciated that the variable domains of both the light (VL) and heavy (VH) chain portions determine antigen recognition and specificity. Conversely, the constant domains of the light chain (CL) and the heavy chain (CHI, CH2 or CH3) confer important biological properties such as secretion, transplacental mobility, Fc receptor binding, complement binding, and the like. By convention the numbering of the constant region domains increases as they become more distal from the antigen binding site or amino-terminus of the antibody. The N-terminus is a variable region and at the C-terminus is a constant region; the CH3 and CL domains actually comprise the carboxy-terminus of the heavy and light chain, respectively.

The term "antibody fragment" as used herein refers to one or more portions of an antibody. In some embodiments, these portions are part of the contact domain(s) of an antibody. In some other embodiments, these portion(s) are antigen-binding fragments that retain the ability of binding an antigen non-covalently, reversibly and specifically, sometimes referred to herein as the antigen binding domain. Examples of binding fragments include, but are not limited to, single-chain Fvs (scFv), a Fab fragment, a monovalent fragment consisting of the VL, VH, CL and CH1 domains; a F(ab)₂ fragment, a bivalent fragment comprising two Fab fragments linked by a disulfide bridge at the hinge region; a Fd fragment consisting of the VH and CH1 domains; a Fv fragment consisting of the VL and VH domains of a single arm of an antibody; a dAb fragment (Ward et al., (1989) Nature 341:544-546), which consists of a VH domain; and an isolated complementarity determining region (CDR).

Antibody fragments can also be incorporated into single domain antibodies, maxibodies, minibodies, intrabodies, diabodies, triabodies, tetrabodies, v-NAR and bis-scFv (see, e.g., Hollinger and Hudson, (2005) Nature Biotechnology 23: 1126-1136). Antibody fragments can be grafted into scaffolds based on polypeptides such as Fibronectin type III (Fn3) (see U.S. Pat. No. 6,703,199, which describes fibronectin polypeptide monobodies).

Antibody fragments can be incorporated into single chain molecules comprising a pair of tandem Fv segments (for example, VH-CH1-VH-CH1) which, together with complementary light chain polypeptides (for example, VL-VC-VL-VC), form a pair of antigen binding regions (Zapata et al., (1995) Protein Eng. 8:1057-1062; and U.S. Pat. No. 5,641,870).

The term "half antibody" refers to a portion of an antibody molecule, antibody fragment, antibody-like molecule or multispecific molecule that comprises a single antigen binding domain. In an embodiment, a half antibody refers to a heavy and light chain pair of, for example, an IgG antibody. In one embodiment, a half antibody refers to a polypeptide comprising a VL domain and a CL domain, and a second polypeptide comprising a VH domain, a CH1 domain, a hinge domain, a CH2 domain, and a CH3 domain, wherein said VL and VH domains comprise an antigen binding domain. In another embodiment, a half antibody refers to a polypeptide comprising a scFv domain, a CH2 domain and a CH3 domain. In some multispecific molecule embodiments, a first half antibody will associate, e.g., heterodimerize, with a second half antibody. In some multispecific molecules a first half antibody will be covalently linked to a second half antibody. In some multispecific molecules, either a first half antibody, a second half antibody, or both a first and second half antibody may comprise an additional antigen binding domain.

The term "antibody-like molecule" refers to a molecule comprising an antibody or a fragment thereof.

The terms "complementarity determining region" or "CDR," as used herein, refer to the sequences of amino acids within antibody variable regions which confer antigen specificity and binding affinity. For example, in general, there are three CDRs in each heavy chain variable region (e.g., HCDR1, HCDR2, and HCDR3) and three CDRs in each light chain variable region (LCDR1, LCDR2, and LCDR3). The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), or a combination thereof. Under the Kabat numbering scheme, in some embodiments, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under the Chothia numbering scheme, in some embodiments, the CDR amino acids in the VH are numbered 26-32 (HCDR1), 52-56 (HCDR2), and 95-102 (HCDR3); and the CDR amino acid residues in the VL are numbered 26-32 (LCDR1), 50-52 (LCDR2), and 91-96 (LCDR3). In a combined Kabat and Chothia numbering scheme, in some embodiments, the CDRs correspond to the amino acid residues that are part of a Kabat CDR, a Chothia CDR, or both. For instance, in some embodiments, the CDRs correspond to amino acid residues 26-35 (HCDR1), 50-65 (HCDR2), and 95-102 (HCDR3) in a human VH, e.g., a mammalian VH, e.g., a human VH; and amino acid residues 24-34 (LCDR1), 50-56 (LCDR2), and 89-97 (LCDR3) in humana VL, e.g., a mammalian VL, e.g., a human VL.

The term "single-chain Fv" or "scFv" as used herein refers to antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the scFv to form the desired structure for antigen binding. For a review of scFv see Plückthun in The Pharmacology of Monoclonal Antibodies, vol. 113, Rosenburg and Moore eds., (1994) Springer-Verlag, New York, pp. 269-315.

The term "diabody" as used herein refers to small antibody fragments with two antigen-binding sites, which fragments comprise a heavy chain variable domain (VH) connected to a light chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., (1993) Proc. Natl. Acad. Sci. USA 90:6444-6448.

The term "monospecific molecule," as used herein, refers to a molecule that binds to one epitope on a target antigen. In some embodiments, a mono-specific molecule of the present invention is a monospecific antibody-like molecule. In some embodiments, a mono-specific molecule of the present invention is a monospecific antibody.

As used herein, the terms "CLL-1" and "CLL1" are used interchangeably, and refer to C-type lectin-like molecule-1, which is an antigenic determinant detectable on leukemia precursor cells and on normal immune cells. C-type lectin-like-1 (CLL-1) is also known as MICL, CLEC12A, CLEC-1, Dendritic Cell-Associated Lectin 1, and DCAL-2. The human and murine amino acid and nucleic acid sequences can be found in a public database, such as GenBank, UniProt and Swiss-Prot. For example, the amino acid sequence of human CLL-1 can be found as UniProt/Swiss-Prot Accession No. Q5QGZ9 and the nucleotide sequence encoding of the human CLL-1 can be found at Accession Nos. NM 001207010.1, NM 138337.5, NM 201623.3, and NM 201625.1. In one embodiment, the multispecific molecule, e.g., bispecific molecule, e.g., bispecific antibody or antibody-like molecule comprises at least one, e.g., one, anti-CLL-1 binding domain and binds an epitope within the extracellular domain of the CLL-1 protein or a fragment thereof. In one embodiment, the CLL-1 protein is expressed on a cancer cell.

The terms "CD3" or "CD-3," as used interchangeably herein, refer to the cluster of differentiation 3 co-receptor (or co-receptor complex, or polypeptide chain of the co-receptor complex) of the T cell receptor. The amino acid sequence of the polypeptide chains of human CD3 are provided in NCBI Accession P04234, P07766 and P09693. CD3 proteins may also include variants. CD3 proteins may also include fragments. CD3 proteins also include post-translational modifications of the CD3 amino acid sequences. Post-translational modifications include, but are not limited to, N-and O-linked glycosylation.

The term "bispecific molecule" as used herein refers to a molecule that binds to two different epitopes on one antigen (also referred to herein as "biparatopic") or two different antigens. In some embodiments, a bispecific molecule of the present invention is a bispecific antibody-like molecule. In some embodiments, a bispecific molecule of the present invention is a bispecific antibody. In some embodiments, the bispecific molecule of the present invention is not biparatropic.

The term "multispecific molecule" as used herein refers to a molecule that binds to two or more different epitopes on one antigen (also referred to herein as "multiparatopic") or on two or more different antigens. Recognition of each antigen is generally accomplished with an "antigen binding domain". In some embodiments, a multispecific molecule of the present invention is a multispecific antibody-like molecule. In some embodiments, a multispecific molecule of the present invention is a multispecific antibody. In some embodiments, the bispecific molecule of the present inveniton is not multiparatropic. The term "multispecific" includes "bispecific." In some aspects, the multispecific molecules consist of one polypeptide chain that comprises a plurality, e.g., two or more, e.g., two, antigen binding domains. In some aspects the multispecific molecules comprise two, three, four or more polypeptide chains that together comprise a plurality, e.g., two or more, e.g., two, antigen binding domains.

The term "a tandem of VH domains (or VHs)" as used herein refers to a string of VH domains, consisting of multiple numbers of identical VH domains of an antibody. Each of the VH domains, except the last one at the end of the tandem, has its C-terminus connected to the N-terminus of another VH domain with or without a linker. A tandem has at least 2, 3, 4, 5, 6, 7, 8, 9, 10, 11, 12, 13, 14, 15, 16, 17, 18, 19, 20, 25, 30, 50, or 100 VH domains. The tandem of VH can be produced by joining the encoding genes of each VH domain in a desired order using recombinant methods with or without a linker (e.g., a synthetic linker) that enables them to be made as a single protein. In one aspect, the VH domains in the tandem, alone or in combination with VL domains of the same antibody , retain the binding specificity of the original antibody. The N-terminus of the first VH domain in the tandem is defined as the N-terminus of the tandem, while the C-terminus of the last VH domain in the tandem is defined as the C-terminus of the tandem.

The term "a tandem of VL domains (or VLs)" as used herein refers to a string of VL domains, consisting of multiple numbers of identical VL domains of an antibody. Each of the VL domains, except the last one at the end of the tandem, has its C-terminus connected to the N-terminus of another VH with or without a linker. A tandem has at least 2, 3, 4, 5, 6, 7, 8, 9, 10, 11, 12, 13, 14, 15, 16, 17, 18, 19, 20, 25, 30, 50, or 100 VL domains. The tandem of VL can be produced by joining the encoding gene of each VL domain in a desired order using recombinant methods with or without a linker (e.g., a synthetic linker) that enables them to be made as a single protein. Preferably, the VL domains in the tandem, alone or in combination with VH domains of the same antibody, retain the binding specificity of the original antibodies. The N-terminus of the first VL domain in the tandem is defined as the N-terminus of the tandem, while the C-terminus of the last VL domain in the tandem is defined as the C-terminus of the tandem.

The term "monovalent molecule" as used herein refers to a molecule that has a single antigen binding domain. In some embodiments, a monovalent molecule of the present invention is a monovalent antibody-like molecule. In some embodiments, a monovalent molecule of the present invention is a monovalent antibody.

The term "bivalent molecule" as used herein refers to a molecule that has two antigen binding domains. In some embodiments, a bivalent molecule of the present invention is a bivalent antibody-like molecule. In some embodiments, a bivalent molecule of the present invention is a bivalent antibody.

The term "trivalent molecule" as used herein refers to a molecule that has three antigen binding domains. In some embodiments, a trivalent molecule of the present invention is a trivalent antibody-like molecule. In some embodiments, a trivalent molecule of the present invention is a trivalent antibody. In some embodiments, a trivalent molecule may consist of two antigen binding domains capable of binding to the same epitope of the same antigen, and a third antigen binding domain that binds to a distinct epitope or antigen, e.g., a distinct antigen. Such embodiments are considered trivalent bispecific molecules.

The term "tetravalent molecule" as used herein refers to a molecule that has four antigen binding domains. In some embodiments, a tetravalent molecule of the present invention is a tetravalent antibody-like molecule. In some embodiments, a tetravalent molecule of the present invention is a tetravalent antibody. In some embodiments, a tetravalent molecule may consist of two antigen binding domains capable of binding to the same epitope of the same antigen, and two additional antigen binding domains that bind to a distinct epitope or antigen, e.g., a distinct antigen. When such two additional antigen binding domains bind to the same epitope or antigen, e.g., the same distinct antigen, such embodiments are considered tetravalent bispecific molecules. When such two additional antigen binding domains bind to different epitopes or antigens, e.g., different distinct antigens, such embodiments are considered tetravalent trispecific molecules.

The term "multivalent molecule" as used herein refers to a molecule that has at least two antigen binding sites. In some embodiments, a multivalent molecule of the present invention is a multivalent antibody-like molecule. In some embodiments, a multivalent molecule of the present invention is a multivalent antibody. In some embodiments, a multivalent molecule is a bivalent molecule, trivalent molecule or a tetravalent molecule.

The term "substantially similar," or "substantially the same," as used herein refers to a sufficiently high degree of similarity between two numeric values (generally one associated with an antibody-like molecule of the invention and the other associated with a reference/comparator antibody or antibody-like molecule) such that one of skill in the art would consider the difference between the two values to be of little or no biological and/or statistical significance within the context of the biological characteristic measured by said values (e.g., Tm values or the amount of the assembled antibodies). The difference between said two values is preferably less than about 50%, preferably less than about 40%, preferably less than about 30%, preferably less than about 20%, preferably less than about 10% as a function of the value for the reference/comparator antibody.

The term "substantially same yield" as used herein refers to the amount of an assembled molecule (e.g. antibody or antibody-like molecule) of the present invention is substantially the same as that of the antibodies it derived from when being prepared under similar conditions in similar cell types. Relative yields of antibody or antibody like products can be determined using standard methods including scanning densitometry of SDS-PAGE gels and/or immunoblots and the AME5-RP assay.

The term "thermostability" as used herein refers to the ability of a protein (e.g., an antibody or an antibody-like molecule) to retain the characteristic property when heated moderately. When exposed to heat, proteins will experience denaturing/unfolding process and will expose hydrophobic residues. A protein is completely unfolded in response to heat at a characteristic temperature. The temperature at the mid-point of the protein unfolding process is defined as Tm, which is an important physical characteristic of a protein, and can be measured with the techniques known in the art, such as by monitoring the denaturing process using Sypro orange dye labeling hydrophobic residues of denatured proteins or by using differential scanning calorimetry (DSC) techniques.

The term "epitope" as used herein refers to any determinant capable of binding with high affinity to an antibody or an antibody-like molecule. An epitope is a region of an antigen that is bound by an antibody (or an antibody-like molecule) that specifically targets that antigen, and when the antigen is a protein, includes specific amino acids that directly contact the antibody or the antibody-like molecule. Most often, epitopes reside on proteins, but in some instances, may reside on other kinds of molecules, such as nucleic acids. Epitope determinants may include chemically active surface groupings of molecules such as amino acids, sugar side chains, phosphoryl or sulfonyl groups, and may have specific three dimensional structural characteristics, and/or specific charge characteristics.

Generally, antibodies or antibody-like molecules specific for a particular target antigen will preferentially recognize an epitope on the target antigen in a complex mixture of proteins and/or macromolecules.

Regions of a given polypeptide that include an epitope can be identified using any number of epitope mapping techniques, well known in the art. See, e.g., Epitope Mapping Protocols in Methods in Molecular Biology, Vol. 66 (Glenn E.Morris, Ed., 1996) Humana Press, Totowa, New Jersey. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871; Geysen et al., (1984) Proc. Natl. Acad. Sci. USA 8:3998-4002; Geysen et al., (1985) Proc. Natl. Acad. Sci. USA 82:78-182; Geysen et al., (1986) Mol. Immunol. 23:709-715. Similarly, conformational epitopes are readily identified by determining spatial conformation of amino acids such as by, e.g., x-ray crystallography and two-dimensional nuclear magnetic resonance. See, e.g., Epitope Mapping Protocols, supra. Antigenic regions of proteins can also be identified using standard antigenicity and hydropathy plots, such as those calculated using, e.g., the Omiga version 1.0 software program available from the Oxford Molecular Group. This computer program employs the Hopp/Woods method, Hopp et al., (1981) Proc. Natl. Acad. Sci USA 78:3824-3828; for determining antigenicity profiles, and the Kyte-Doolittle technique, Kyte et al., (1982) J.MoI. Biol. 157:105-132; for hydropathy plots.

Specific binding between two entities means a binding with an equilibrium constant (K_{A}) (kₒₙ/k_{off}) of at least 10²M⁻¹, at least 5x10²M⁻¹, at least 10³M⁻¹, at least 5x10³M⁻¹, at least 10⁴M⁻¹ at least 5x10⁴M⁻¹, at least 10⁵M⁻¹, at least 5x10⁵M⁻¹, at least 10⁶M⁻¹, at least 5x10⁶M⁻¹, at least 10⁷M⁻¹, at least 5x10⁷M⁻¹, at least 10⁸M⁻¹, at least 5x10⁸M⁻¹, at least 10⁹M^{- 1}, at least 5x10⁹M⁻¹, at least 10¹⁰M⁻¹, at least 5x10¹⁰M⁻¹, at least 10¹¹M⁻¹, at least 5x10¹¹M⁻¹, at least 10¹²M⁻¹, at least 5x10¹²M⁻¹, at least 10¹³M⁻¹, at least 5x10¹³ M⁻¹, at least 10¹⁴M⁻¹, at least 5x10¹⁴M⁻¹, at least 10¹⁵M⁻¹, or at least 5x10¹⁵M⁻¹.

The term "specifically (or selectively) binds" to an antigen or an epitope refers to a binding reaction that is determinative of the presence of a cognate antigen or an epitope in a heterogeneous population of proteins and other biologics. In addition to the equilibrium constant (K_{A}) noted above, an antibody or antibody-like molecule of the invention typically also has a dissociation rate constant (K_{D}) (k_{off}/kₒₙ) of less than 5x10⁻²M, less than 10⁻²M, less than 5x10⁻³M, less than 10⁻³M, less than 5x10⁻⁴M, less than 10⁻⁴M, less than 5x10⁻⁵M, less than 10⁻⁵M, less than 5x10⁻⁶M, less than 10⁻⁶M, less than 5x10⁻⁷M, less than 10⁻⁷M, less than 5x10⁻⁸M, less than 10⁻⁸M, less than 5x10⁻⁹M, less than 10⁻⁹M, less than 5x10⁻¹⁰M, less than 10⁻¹⁰M, less than 5x10⁻¹¹M, less than 10⁻¹¹M, less than 5x10⁻¹²M, less than 10⁻¹²M, less than 5x10⁻¹³M, less than 10⁻¹³M, less than 5x10⁻¹⁴M, less than 10⁻¹⁴M, less than 5x10⁻¹⁵M, or less than 10⁻15M or lower, and binds to the target antigen with an affinity that is at least two-fold greater than its affinity for binding to a non-specific antigen (*e.g*., HSA).

The terms "a molecule (e.g. an antibody or an antibody-like molecule) recognizing an antigen (or an epitope)" and "a molecule specific for an antigen (or an epitope)" are used interchangeably herein with the term "a molecule which binds specifically to an antigen (or an epitope)". In one embodiment, the molecule (e.g. antibody or antibody-like molecule) or fragment thereof has dissociation constant (K_{d}) of less than 3000 pM, less than 2500 pM, less than 2000 pM, less than 1500 pM, less than 1000 pM, less than 750 pM, less than 500 pM, less than 250 pM, less than 200 pM, less than 150 pM, less than 100 pM, less than 75 pM, less than 10 pM, less than 1 pM as assessed using a method described herein or known to one of skill in the art (e.g., a BIAcore assay, ELISA, FACS, SET) (Biacore International AB, Uppsala, Sweden). The term "K_{assoc}" or "Kₐ", as used herein, refers to the association rate of a particular antibody-antigen interaction, whereas the term "K_{dis}" or "K_{d}," as used herein, refers to the dissociation rate of a particular antibody-antigen interaction. The term "K_{D}", as used herein, refers to the dissociation constant, which is obtained from the ratio of K_{d} to Kₐ (i.e. K_{d}/Kₐ) and is expressed as a molar concentration (M). K_{D} values for antibodies can be determined using methods well established in the art. A method for determining the K_{D} of an antibody is by using surface plasmon resonance, or using a biosensor system such as a Biacore® system.

The term "multiple binding specificities" as used herein refers to that a molecule of the present invention (e.g., an antibody or an antibody like molecule) is capable of specifically binding at least two, three, four, five, six, seven, eight, nine, or ten different epitopes either on the same antigen or on at least two, three, four, five, seven, eight, nine or ten different antigens.

The term "dual binding specificity" as used herein refers to that a molecule of the present invention (e.g., an antibody or an antibody like molecule) is capable of binding two different epitopes either on the same antigen or on two different antigens.

The term "isolated antibody" or "isolated antibody-like molecule" as used herein refers to an antibody or an antibody-like molecule that is substantially free of other antibodies or antibody-like molecules having different antigenic specificities. Moreover, an isolated antibody or antibody-like molecule may be substantially free of other cellular material and/or chemicals.

The term "monoclonal antibody" or "monoclonal antibody composition" as used herein refers to polypeptides, including antibodies, antibody fragments, molecules, etc. that have substantially identical to amino acid sequence or are derived from the same genetic source. This term also includes preparations of antibody molecules of single molecular composition. A monoclonal antibody composition displays a single binding specificity and affinity for a particular epitope.

The term "humanized" forms of non-human (e.g., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin lo sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1: 105-115 (1998); Harris, Biochem. Soc. Transactions 23:1035-1038 (1995); Hurle and Gross, Curr. Op. Biotech. 5:428-433 (1994).

The term "human antibody" as used herein includes antibodies having variable regions in which both the framework and CDR regions are derived from sequences of human origin. Furthermore, if the antibody contains a constant region, the constant region also is derived from such human sequences, e.g., human germline sequences, or mutated versions of human germline sequences or antibody containing consensus framework sequences derived from human framework sequences analysis, for example, as described in Knappik, et al. (2000. J Mol Biol 296, 57-86). The structures and locations of immunoglobulin variable domains, e.g., CDRs, may be defined using well known numbering schemes, e.g., the Kabat numbering scheme, the Chothia numbering scheme, or a combination of Kabat and Chothia (see, e.g., Sequences of Proteins of Immunological Interest, U.S. Department of Health and Human Services (1991), eds. Kabat *et al.;* Al Lazikani et al., (1997) J. Mol. Bio. 273:927 948); Kabat et al., (1991) Sequences of Proteins of Immunological Interest, 5th edit., NIH Publication no. 91-3242 U.S. Department of Health and Human Services; Chothia et al., (1987) J. Mol. Biol. 196:901-917; Chothia et al., (1989) Nature 342:877-883; and Al-Lazikani et al., (1997) J. Mol. Biol. 273:927-948.

The human antibodies of the invention may include amino acid residues not encoded by human sequences (e.g., mutations introduced by random or site-specific mutagenesis in vitro or by somatic mutation in vivo, or a conservative substitution to promote stability or manufacturing). However, the term "human antibody", as used herein, is not intended to include antibodies in which CDR sequences derived from the germline of another mammalian species, such as a mouse, have been grafted onto human framework sequences.

A "modification" or "mutation" of an amino acid residue/position, as used herein, refers to a change of a primary amino acid sequence as compared to a starting amino acid sequence, wherein the change results from a sequence alteration involving said amino acid residue/positions. For example, typical modifications include substitution of the residue (or at said position) with another amino acid (e.g., a conservative or non-conservative substitution), insertion of one or more amino acids adjacent to said residue/position, and deletion of said residue/position. An "amino acid substitution," or variation thereof, refers to the replacement of an existing amino acid residue in a predetermined (starting) amino acid sequence with a different amino acid residue. Generally and preferably, the modification results in alteration in at least one physicobiochemical activity of the variant polypeptide compared to a polypeptide comprising the starting (or "wild type") amino acid sequence. For example, in the case of an antibody, a physicobiochemical activity that is altered can be binding affinity, binding capability and/or binding effect upon a target molecule.

The term "conservatively modified variant" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

For polypeptide sequences, "conservatively modified variants" include individual substitutions, deletions or additions to a polypeptide sequence which result in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention. The following eight groups contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, *e.g*., Creighton, Proteins (1984)). In some embodiments, the phrase "conservative sequence modifications" are used to refer to amino acid modifications that do not significantly affect or alter the binding characteristics of the antibody or the antibody-like molecule containing the amino acid sequence.

The terms "percent identical" or "percent identity," in the context of two or more nucleic acids or polypeptide sequences, refers to two or more sequences or subsequences that are the same. Two sequences are "substantially identical" if two sequences have a specified percentage of amino acid residues or nucleotides that are the same (*i.e.,* 60% identity, optionally 65%, 70%, 75%, 80%, 85%, 90%, 95%, or 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides (or 10 amino acids) in length, or more preferably over a region that is 100 to 500 or 1000 or more nucleotides (or 20, 50, 200 or more amino acids) in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

The term "comparison window" as used herein includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted, *e.g*., by the local homology algorithm of Smith and Waterman (1970) Adv. Appl. Math. 2:482c, by the homology alignment algorithm of Needleman and Wunsch (1970) J. Mol. Biol. 48:443, by the search for similarity method of Pearson and Lipman, (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, *e.g.,* Brent et al., (2003) Current Protocols in Molecular Biology).

Two examples of algorithms that are suitable for determining percent sequence identity and sequence similarity are the BLAST and BLAST 2.0 algorithms, which are described in Altschul et al., (1977) Nuc. Acids Res. 25:3389-3402; and Altschul et al., (1990) J. Mol. Biol. 215:403-410, respectively. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*)*.* These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) or 10, M=5, N=-4 and a comparison of both strands. For amino acid sequences, the BLASTP program uses as defaults a wordlength of 3, and expectation (E) of 10, and the BLOSUM62 scoring matrix (see Henikoff and Henikoff, (1989) Proc. Natl. Acad. Sci. USA 89:10915) alignments (B) of 50, expectation (E) of 10, M=5, N=-4, and a comparison of both strands.

The BLAST algorithm also performs a statistical analysis of the similarity between two sequences (see, *e.g.,* Karlin and Altschul (1993) Proc. Natl. Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The percent identity between two amino acid sequences can also be determined using the algorithm of E. Meyers and W. Miller (Comput. Appl. Biosci. 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol, Biol. 48:444-453 (1970)) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

Other than percentage of sequence identity noted above, another indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. Another indication that two nucleic acid sequences are substantially identical is that the two molecules or their complements hybridize to each other under stringent conditions, as described below. Yet another indication that two nucleic acid sequences are substantially identical is that the same primers can be used to amplify the sequence.

The term "nucleic acid" is used herein interchangeably with the term "polynucleotide" and refers to deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

Unless otherwise indicated, a particular nucleic acid sequence also implicitly encompasses conservatively modified variants thereof (*e.g*., degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., (1991) Nucleic Acid Res. 19:5081; Ohtsuka et al., (1985) J. Biol. Chem. 260:2605-2608; and Rossolini et al., (1994) Mol. Cell. Probes 8:91-98).

The term "operably linked" or functionally linked, as used herein, refers to a functional relationship between two or more polynucleotide (*e.g.*, DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, *i.e.,* they are cis-acting. However, some transcriptional regulatory sequences, such as enhancers, need not be physically contiguous or located in close proximity to the coding sequences whose transcription they enhance.

The terms "polypeptide" and "protein" are used interchangeably herein to refer to a polymer of amino acid residues. The phrases also apply to amino acid polymers in which one or more amino acid residue is an artificial chemical mimetic of a corresponding naturally occurring amino acid, as well as to naturally occurring amino acid polymers and non-naturally occurring amino acid polymer. Unless otherwise indicated, a particular polypeptide sequence also implicitly encompasses conservatively modified variants thereof.

The term *"in vivo* half life", as used herein, refers to the half-life of the molecule of interest or variants thereof circulating in the blood of a given mammal.

The term "subject" includes human and non-human animals. Non-human animals include all vertebrates, *e.g.*, mammals and non-mammals, such as non-human primates, sheep, dog, cow, chickens, amphibians, and reptiles. Except when noted, the terms "patient" or "subject" are used herein interchangeably.

The term "cancer" refers to a disease characterized by the rapid and uncontrolled growth of aberrant cells. Cancer cells can spread locally or through the bloodstream and lymphatic system to other parts of the body. Examples of various cancers are described herein and include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like. Preferred cancers treated by the methods described herein include multiple myeloma, Hodgkin's lymphoma or non-Hodgkin's lymphoma.

The terms "tumor" and "cancer" are used interchangeably herein, e.g., both terms encompass solid and liquid, e.g., diffuse or circulating, tumors. As used herein, the term "cancer" or "tumor" includes premalignant, as well as malignant cancers and tumors.

The terms "cancer antigen," "cancer associated antigen" or "tumor antigen" interchangeably refer to a molecule (typically a protein, carbohydrate or lipid) that is expressed on the surface of a cancer cell, either entirely or as a fragment (e.g., MHC/peptide), and which is useful for the preferential targeting of a pharmacological agent to the cancer cell. In some embodiments, a tumor antigen is a marker expressed by both normal cells and cancer cells, e.g., a lineage marker, e.g., CD19 on B cells. In some embodiments, a tumor antigen is a cell surface molecule that is overexpressed in a cancer cell in comparison to a normal cell, for instance, 1-fold over expression, 2-fold overexpression, 3-fold overexpression or more in comparison to a normal cell. In some embodiments, a tumor antigen is a cell surface molecule that is inappropriately synthesized in the cancer cell, for instance, a molecule that contains deletions, additions or mutations in comparison to the molecule expressed on a normal cell. In some embodiments, a tumor antigen will be expressed exclusively on the cell surface of a cancer cell, entirely or as a fragment (e.g., MHC/peptide), and not synthesized or expressed on the surface of a normal cell. In some embodiments, the multispecific molecules of the present invention includes multispecific molecules comprising an antigen binding domain (e.g., antibody or antibody fragment) that binds to a MHC presented peptide. Normally, peptides derived from endogenous proteins fill the pockets of Major histocompatibility complex (MHC) class I molecules, and are recognized by T cell receptors (TCRs) on CD8 + T lymphocytes. The MHC class I complexes are constitutively expressed by all nucleated cells. In cancer, virus-specific and/or tumor-specific peptide/MHC complexes represent a unique class of cell surface targets for immunotherapy. TCR-like antibodies targeting peptides derived from viral or tumor antigens in the context of human leukocyte antigen (HLA)-A1 or HLA-A2 have been described (see, e.g., Sastry et al., J Virol. 2011 85(5):1935-1942; Sergeeva et al., Blood, 2011 117(16):4262-4272; Verma et al., J Immunol 2010 184(4):2156-2165; Willemsen et al., Gene Ther 2001 8(21) :1601-1608 ; Dao et al., Sci Transl Med 2013 5(176) :176ra33 ; Tassev et al., Cancer Gene Ther 2012 19(2):84-100). For example, TCR-like antibody can be identified from screening a library, such as a human scFv phage displayed library.

As used herein, the terms "treat", "treatment" and "treating" refer to the reduction or amelioration of the progression, severity and/or duration of a proliferative disorder, or the amelioration of one or more symptoms (preferably, one or more discernible symptoms) of a proliferative disorder resulting from the administration of one or more therapies (e.g., one or more therapeutic agents such as a multispecific molecule, e.g., bispecific molecule, e.g., bispecific antibody or bispecific antibody-like molecule, of the invention). In specific embodiments, the terms "treat", "treatment" and "treating" refer to the amelioration of at least one measurable physical parameter of a proliferative disorder, such as growth of a tumor, not necessarily discernible by the patient. In other embodiments the terms "treat", "treatment" and "treating" -refer to the inhibition of the progression of a proliferative disorder, either physically by, e.g., stabilization of a discernible symptom, physiologically by, e.g., stabilization of a physical parameter, or both. In other embodiments the terms "treat", "treatment" and "treating" refer to the reduction or stabilization of tumor size or cancerous cell count.

Various aspects of the invention are described in further detail in the following sections and subsections.

### I. Anti-CLL-1 Binding Domains

In one aspect, the invention provides a number of multispecific molecules, e.g., bispecific molecules, e.g., bispecific antibodies, comprising an antibody or antibody fragment engineered for enhanced binding to a CLL-1 protein, as defined in the claims.

In one aspect the anti-CLL-1 binding domain of the multispecific molecule, e.g., a bispecific molecule, e.g., a bispecific antibody, comprises more than one polypeptide. By way of example, the anti-CLL-1 binding domain of a multispecific molecule, e.g., a bispecific molecule, e.g., a bispecific antibody, may comprise a light chain variable region, e.g., as described herein, disposed on a first polypeptide and a heavy chain variable region, e.g., as described herein, disposed on a second polypeptide. In one aspect the polypeptide comprising the light chain variable region and the polypeptide comprising the heavy chain polypeptide form a half antibody. In another aspect, the anti-CLL-1 binding domain consists of one polypeptide, e.g., a polypeptide comprising both a light chain variable region and a heavy chain variable region of an anti-CLL-1 binding domain, e.g., as described herein. In one aspect, the anti-CLL-1 antigen binding portion of the multispecific molecule, e.g., of the bispecific molecule, e.g., of the bispecific antibody or bispecific antibody-like molecule, is a scFv antibody fragment. In one aspect such antibody fragments are functional in that they retain the equivalent binding affinity, e.g., they bind the same antigen with comparable efficacy, as the IgG antibody from which it is derived. In other embodiments, the antibody fragment has a lower binding affinity, e.g., it binds the same antigen with a lower binding affinity than the antibody from which it is derived, but is functional in that it provides a biological response described herein. In one embodiment, the multispecific molecule, e.g., of the bispecific molecule, e.g., of the bispecific antibody or bispecific antibody-like molecule, molecule comprises an antibody fragment that has a binding affinity KD of 10-4 M to 10-8 M, e.g., 10-5 M to 10-7 M, e.g., 10-6 M or 10-7 M, for the target antigen. In one embodiment, the antibody fragment has a binding affinity that is at least five-fold, 10-fold, 20-fold, 30-fold, 50-fold, 100-fold or 1,000-fold less than a reference antibody, e.g., an antibody described herein.

In one aspect such antibody fragments are functional in that they provide a biological response that can include, but is not limited to, activation of an immune response, inhibition of signal-transduction origination from its target antigen, inhibition of kinase activity, and the like, as will be understood by a skilled artisan. In one aspect, the anti-CLL-1 antigen binding domain of the multispecific molecule, e.g., of the bispecific molecule, e.g., of the bispecific antibody or bispecific antibody-like molecule, is a scFv antibody fragment that is humanized compared to the murine sequence of the scFv from which it is derived. In one embodiment, the anti-CLL-1 antigen binding domain is a human anti-CLL-1 antigen binding domain. In one embodiment, the anti-CLL-1 antigen binding domain is a humanized anti-CLL-1 antigen binding domain.

In some aspects, the anti-CLL-1 binding domains of the invention are incorporated into a multispecific molecule, e.g., of the bispecific molecule, e.g., of the bispecific antibody or bispecific antibody-like molecule as defined in the claims. In one aspect, the multispecific molecule, e.g., of the bispecific molecule, e.g., of the bispecific antibody or bispecific antibody-like molecule, comprises a CLL-1 binding domain comprising a sequence of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, or SEQ ID NO: 51. In one aspect, the scFv domains are human.

In one aspect, the anti-CLL-1 binding domain, e.g., human or humanized scFv, portion of a multispecific molecule, e.g., of a bispecific molecule, e.g., of a bispecific antibody, of the invention is encoded by a transgene whose sequence has been codon optimized for expression in a mammalian cell. In one aspect, the chains, e.g., the entire construct, of the multispecific molecule of the invention is encoded by a transgene whose entire sequence has been codon optimized for expression in a mammalian cell. Codon optimization refers to the discovery that the frequency of occurrence of synonymous codons (i.e., codons that code for the same amino acid) in coding DNA is biased in different species. Such codon degeneracy allows an identical polypeptide to be encoded by a variety of nucleotide sequences. A variety of codon optimization methods is known in the art, and include, e.g., methods disclosed in at least US Patent Numbers 5,786,464 and 6,114,148.

In one aspect of the invention, the human anti-CLL-1 binding domain comprises the scFv portion provided in SEQ ID NO: 39. In one embodiment of the invention, the human anti- CLL-1 binding domain comprises the scFv portion provided in SEQ ID NO:40. In one embodiment of the invention, the human anti-CLL-1 binding domain comprises the scFv portion provided in SEQ ID NO:41. In one embodiment of the invention, the human anti-CLL-1 binding domain comprises the scFv portion provided in SEQ ID NO:42. In one embodiment of the invention, the human anti-CLL-1 binding domain comprises the scFv portion provided in SEQ ID NO:43. In one embodiment of the invention, the human anti-CLL-1 binding domain comprises the scFv portion provided in SEQ ID NO:44. In one embodiment of the invention, the human anti-CLL-1 binding domain comprises the scFv portion provided in SEQ ID NO:45. In one embodiment of the invention, the human anti-CLL-1 binding domain comprises the scFv portion provided in SEQ ID NO:46. In one embodiment of the invention, the human anti-CLL-1 binding domain comprises the scFv portion provided in SEQ ID NO:47. In one embodiment of the invention, the human anti-CLL-1 binding domain comprises the scFv portion provided in SEQ ID NO:48. In one embodiment of the invention, the human anti-CLL-1 binding domain comprises the scFv portion provided in SEQ ID NO:49. In one embodiment of the invention, the human anti-CLL-1 binding domain comprises the scFv portion provided in SEQ ID NO:50. In one embodiment of the invention, the human anti-CLL-1 binding domain comprises the scFv portion provided in SEQ ID NO:51.

Furthermore, the present invention provides CLL-1 multispecific molecule, e.g., bispecific molecule, e.g., bispecific antibody or bispecific antibody-like molecule as defined in the claims, compositions and their use in medicaments or methods for treating, among other diseases, cancer or any malignancy or autoimmune diseases involving cells or tissues which express CLL-1.

In one aspect, the multispecific molecule, e.g., bispecific molecule, e.g., bispecific antibody or bispecific antibody-like molecule, of the invention can be used to eradicate CLL-1-expressing normal cells, and is thereby applicable for use as a conditioning therapy prior to cell transplantation. In one aspect, the CLL-1-expressing normal cell is a CLL-1-expressing normal stem cell and the cell transplantation is a stem cell transplantation.

The present disclosure includes a multispecific molecule, e.g., bispecific molecule, e.g., bispecific antibody or bispecific antibody-like molecule, that comprises an anti-CLL-1 binding domain (e.g., human or humanized CLL-1 binding domain as described herein), and at least a second antigen binding domain, and wherein said anti-CLL-1 binding domain comprises a heavy chain complementary determining region 1 (HC CDR1), a heavy chain complementary determining region 2 (HC CDR2), and a heavy chain complementary determining region 3 (HC CDR3) of any anti-CLL-1 heavy chain binding domain amino acid sequence listed in Table 2. The anti-CLL-1 binding domain of the multispecific molecule, e.g., bispecific molecule, e.g., bispecific antibody or bispecific antibody-like molecule, can further comprise a light chain complementary determining region 1 (LC CDR1), a light chain complementary determining region 2 (LC CDR2), and a light chain complementary determining region 3 (LC CDR3) of any anti-CLL-1 light chain binding domain amino acid sequence listed in Table 2.

The present disclosure also provides nucleic acid molecules encoding the multispecific molecule, e.g., bispecific molecule, e.g., bispecific antibody or bispecific antibody-like molecule, e.g., as described herein, e.g., encoding a multispecific molecule, e.g., bispecific molecule, e.g., bispecific antibody or bispecific antibody-like molecule that comprises an anti-CLL-1 binding domain (e.g., human or humanized CLL-1 binding domain as described herein), wherein said anti-CLL-1 binding domain comprises a heavy chain complementary determining region 1 (HC CDR1), a heavy chain complementary determining region 2 (HC CDR2), and a heavy chain complementary determining region 3 (HC CDR3) of any anti-CLL-1 heavy chain binding domain amino acid sequence listed in Table 2. In embodiments, the encoded anti-CLL-1 binding domain of the multispecific molecule, e.g., bispecific molecule, e.g., bispecific antibody or bispecific antibody-like molecule can further comprise a light chain complementary determining region 1 (LC CDR1), a light chain complementary determining region 2 (LC CDR2), and a light chain complementary determining region 3 (LC CDR3) of any anti-CLL-1 light chain binding domain amino acid sequence listed in Table 2.

In specific aspects of the invention, a multispecific molecule, e.g., bispecific molecule, e.g., bispecific antibody or bispecific antibody-like molecule, construct of the invention comprises a scFv domain selected from the group consisting of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, and SEQ ID NO: 51, wherein the scFv may be preceded by an optional leader sequence such as provided in SEQ ID NO: 1. Also included in the invention is a nucleotide sequence that encodes the polypeptide of any of the scFv fragments selected from the group consisting of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, and SEQ ID NO: 51.

Also included in the invention is nucleic acid molecule comprising a nucleotide sequence that encodes the polypeptide of any of the scFv fragments selected from the group consisting of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, and SEQ ID NO: 51, and each of the domains of SEQ ID NO: 1, plus any of the additional domains of the CLL-1 multispecific molecule, e.g., bispecific molecule, e.g., bispecific antibody or bispecific antibody-like molecule, of the invention.

In one aspect, an exemplary CLL-1 multispecific molecule, e.g., bispecific molecule, e.g., bispecific antibody or bispecific antibody-like molecule, construct comprises an optional leader sequence. Specific CLL-1 multispecific molecule, e.g., bispecific molecule, e.g., bispecific antibody or bispecific antibody-like molecule, constructs containing human scFv domains of the invention are provided as SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, and SEQ ID NO: 51.

An exemplary leader sequence is provided as SEQ ID NO: 1.

In one aspect, the present invention encompasses a recombinant nucleic acid construct comprising a nucleic acid molecule encoding a multispecific molecule, e.g., bispecific molecule, e.g., bispecific antibody or bispecific antibody-like molecule, wherein the nucleic acid molecule comprises the nucleic acid sequence encoding an anti-CLL-1 binding domain, or fragment thereof (e.g., a VL or VH domain) as defined in the claims. In one aspect, the nucleic acid encoding the anti-CLL-1 binding domain is selected from one or more of SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63 and SEQ ID NO: 64. In one aspect, the nucleic acid encoding the anti-CLL-1 binding domain comprises SEQ ID NO: 52. In one aspect, the nucleic acid encoding the anti-CLL-1 binding domain comprises SEQ ID NO: 53. In one aspect, the nucleic acid encoding the anti-CLL-1 binding domain comprises SEQ ID NO: 54. In one aspect, the nucleic acid encoding the anti-CLL-1 binding domain comprises SEQ ID NO: 55. In one aspect, the nucleic acid encoding the anti-CLL-1 binding domain comprises SEQ ID NO: 56. In one aspect, the nucleic acid encoding the anti-CLL-1 binding domain comprises SEQ ID NO: 57. In one aspect, the nucleic acid encoding the anti-CLL-1 binding domain comprises SEQ ID NO: 58. In one aspect, the nucleic acid encoding the anti-CLL-1 binding domain comprises SEQ ID NO: 59. In one aspect, the nucleic acid encoding the anti-CLL-1 binding domain comprises SEQ ID NO: 60. In one aspect, the nucleic acid encoding the anti-CLL-1 binding domain comprises SEQ ID NO: 61. In one aspect, the nucleic acid encoding the anti-CLL-1 binding domain comprises SEQ ID NO: 62. In one aspect, the nucleic acid encoding the anti-CLL-1 binding domain comprises SEQ ID NO: 63. In one aspect, the nucleic acid encoding the anti-CLL-1 binding domain comprises SEQ ID NO: 64.

In one aspect, the present disclosure encompasses a recombinant nucleic acid construct comprising a nucleic acid molecule encoding a domain, e.g., a VH or a VL domain, of a multispecific molecule, e.g., bispecific molecule, e.g., bispecific antibody or bispecific antibody-like molecule, wherein the nucleic acid molecule comprises a nucleic acid sequence encoding a domain (e.g., a VH or VL) of an anti-CLL-1 binding domain selected from one or more of a VH or VL of any of SEQ ID NO: 39-51.

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the nucleic acid of interest can be produced synthetically, rather than cloned.

The present disclosure includes vector constructs, e.g., plasmid, retroviral and lentiviral vector constructs expressing a multispecific molecule, e.g., bispecific molecule, e.g., bispecific antibody or bispecific antibody-like molecule, that can be directly transduced into a cell.

The present disclosure also includes an RNA construct that can be directly transfected into a cell. A method for generating mRNA for use in transfection involves in vitro transcription (IVT) of a template with specially designed primers, followed by polyA addition, to produce a construct containing 3' and 5' untranslated sequence ("UTR"), a 5' cap and/or Internal Ribosome Entry Site (IRES), the nucleic acid to be expressed, and a polyA tail, typically 50-2000 bases in length (SEQ ID NO:35). RNA so produced can efficiently transfect different kinds of cells. In one embodiment, the template includes sequences for the polypeptides of the multispecific molecule, e.g., bispecific molecule, e.g., bispecific antibody or bispecific antibody-like molecule. In an embodiment, an RNA vector is transduced into a cell by electroporation.

The multispecific molecules, e.g., bispecific molecules, e.g., bispecific antibodies, of the present disclosure comprise a target-specific binding domain. The choice of moiety depends upon the type and number of ligands that define the surface of a target cell. For example, the antigen binding domain may be chosen to recognize an antigen that acts as a cell surface marker on target cells associated with a particular disease state.

In one aspect, the multispecific molecule, e.g., bispecific molecule, e.g., bispecific antibody or bispecific antibody-like molecule, of the present invention comprises a binding domain that specifically binds CLL-1. In one aspect, the multispecific molecule, e.g., bispecific molecule, e.g., bispecific antibody or bispecific antibody-like molecule, of the present invention comprises an antigen binding domain that specifically binds human CLL-1.

Each of the antigen binding domains, e.g., the anti-CLL-1 binding domain, can be any protein that binds to the antigen including but not limited to a monoclonal antibody, a polyclonal antibody, a recombinant antibody, a human antibody, a humanized antibody, and a functional fragment thereof, including but not limited to a single-domain antibody such as a heavy chain variable domain (VH), a light chain variable domain (VL) and a variable domain (VHH) of camelid derived nanobody, a Fab, a scFv, and to an alternative scaffold known in the art to function as antigen binding domain, such as a recombinant fibronectin domain, and the like. In some instances, it is beneficial for the antigen binding domain to be derived from the same species in which the multispecific molecule, e.g., bispecific molecule, e.g., bispecific antibody or bispecific antibody-like molecule, will ultimately be used in. For example, for use in humans, it may be beneficial for the antigen binding domain of the multispecific molecule, e.g., bispecific molecule, e.g., bispecific antibody or bispecific antibody-like molecule, to comprise human or humanized residues for the antigen binding domain of an antibody or antibody fragment.

Thus, in one aspect, the antigen binding domain comprises a human or a humanized antibody or an antibody fragment. In one embodiment of the invention, the human anti-CLL-1 binding domain comprises one or more (e.g., all three) light chain complementary determining region 1 (LC CDR1), light chain complementary determining region 2 (LC CDR2), and light chain complementary determining region 3 (LC CDR3) of a human anti-CLL-1 binding domain described herein (e.g., in Table 2) as defined in the claims, and/or one or more (e.g., all three) heavy chain complementary determining region 1 (HC CDR1), heavy chain complementary determining region 2 (HC CDR2), and heavy chain complementary determining region 3 (HC CDR3) of a human anti-CLL-1 binding domain as defined in the claims (e.g., in Table 2), e.g., a human anti-CLL-1 binding domain comprising one or more, e.g., all three, LC CDRs and one or more, e.g., all three, HC CDRs, as defined in the claims. In one embodiment, the human anti-CLL-1 binding domain comprises one or more (e.g., all three) heavy chain complementary determining region 1 (HC CDR1), heavy chain complementary determining region 2 (HC CDR2), and heavy chain complementary determining region 3 (HC CDR3) of a human anti-CLL-1 binding domain as defined in the claims (e.g., in Table 2), e.g., the human anti-CLL-1 binding domain has two variable heavy chain regions, each comprising a HC CDR1, a HC CDR2 and a HC CDR3 as defined in the claims. In one embodiment, the human anti-CLL-1 binding domain comprises a human light chain variable region described herein (e.g., in Table 2) and/or a human heavy chain variable region described herein (e.g., in Table 2) and as defined in the claims. In one embodiment, the human anti-CLL-1 binding domain comprises a human heavy chain variable region described herein (e.g., in Table 2), e.g., at least two human heavy chain variable regions described herein (e.g., in Table 2) as defined in the claims. In one embodiment, the anti-CLL-1 binding domain is a scFv comprising a light chain and a heavy chain of an amino acid sequence of Table 2 as defined in the claims. In an embodiment of the disclosure, the anti-CLL-1 binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in Table 2, or a sequence with 95-99% identity with an amino acid sequence of Table 2; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in Table 2, or a sequence with 95-99% identity to an amino acid sequence of Table 2.

In one embodiment, the human anti-CLL-1 binding domain comprises a sequence selected from a group consisting of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, and SEQ ID NO: 51.. In one embodiment, the nucleic acid sequence encoding the human anti-CLL-1 binding domain comprises a sequence selected from a group consisting of SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, and SEQ ID NO: 64. In one embodiment, the human anti-CLL-1 binding domain is a scFv, and a light chain variable region comprising an amino acid sequence described herein, e.g., in Table 2, is attached to a heavy chain variable region comprising an amino acid sequence described herein, e.g., in Table 2, via a linker, e.g., a linker described herein. In one embodiment, the human anti-CLL-1 binding domain includes a (Gly₄-Ser)n linker, wherein n is 1, 2, 3, 4, 5, or 6, preferably 3 or 4 (SEQ ID NO:26). The light chain variable region and heavy chain variable region of a scFv can be, e.g., in any of the following orientations: light chain variable region-linker-heavy chain variable region or heavy chain variable region-linker-light chain variable region. In one aspect, the anti-CLL-1 antigen binding domain portion comprises one or more sequence selected from SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, and SEQ ID NO: 51.

In one embodiment, the anti- CLL-1 binding domain comprises a light chain variable region described herein (e.g., in Table 2) and/or a heavy chain variable region described herein (e.g., in Table 2) as defined in the claims. In one embodiment, the anti-CLL-1 binding domain comprises a polypeptide comprising a light chain variable region described herein (e.g., in Table 2) and a polypeptide comprising a heavy chain variable region described herein (e.g., in Table 2) as defined in the claims. In some embodiments, the polypeptide comprising the light chain variable region described herein (e.g., in Table 2) and the polypeptide comprising a heavy chain variable region described herein (e.g., in Table 2) as defined in the claims form a half antibody, or antibody fragment thereof (e.g., noncovalently associate or covalently associate to form a half antibody, or antibody fragment thereof), comprising an anti-CLL-1 binding domain.

In one embodiment, the anti-CLL-1 binding domain comprises a light chain variable region provided in SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, or SEQ ID NO: 196, and/or a heavy chain variable region provided in SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, or SEQ ID NO: 195 as defined in the claims. In one embodiment, the encoded anti- CLL-1 binding domain is a scFv comprising a light chain and a heavy chain of an amino acid sequence of Table 2 as defined in the claims. In an embodiment of the disclosure, the human or humanized anti-CLL-1 binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, or SEQ ID NO: 196, or a sequence with 95-99% identity thereof; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, or SEQ ID NO: 195, or a sequence with 95-99% identity thereof. In one embodiment, the encoded anti-CLL-1 binding domain includes a (Gly4-Ser)n linker, wherein n is 1, 2, 3, 4, 5, or 6, preferably 3 or 4 (SEQ ID NO:26). The light chain variable region and heavy chain variable region of a scFv can be, e.g., in any of the following orientations: light chain variable region-linker-heavy chain variable region or heavy chain variable region-linker-light chain variable region.

In embodiments of the invention, the anti-CLL-1 binding domain comprises the light chain variable region provided in SEQ ID NO: 78 and the heavy chain variable region provided in SEQ ID NO: 65. In embodiments of the disclosure, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 78, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 65, or a sequence with 95-99% identity thereof.

In embodiments of the invention, the anti-CLL-1 binding domain comprises the light chain variable region provided in SEQ ID NO: 79 and the heavy chain variable region provided in SEQ ID NO: 66. In embodiments of the disclosure, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 79, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 66, or a sequence with 95-99% identity thereof.

In embodiments of the invention, the anti-CLL-1 binding domain comprises the light chain variable region provided in SEQ ID NO: 80 and the heavy chain variable region provided in SEQ ID NO: 67. In embodiments of the disclosure, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 80, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 67, or a sequence with 95-99% identity thereof.

In embodiments of the invention, the anti-CLL-1 binding domain comprises the light chain variable region provided in SEQ ID NO: 81 and the heavy chain variable region provided in SEQ ID NO: 68. In embodiments of the disclosure, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 81, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 68, or a sequence with 95-99% identity thereof.

In embodiments of the invention, the anti-CLL-1 binding domain comprises the light chain variable region provided in SEQ ID NO: 82 and the heavy chain variable region provided in SEQ ID NO: 69. In embodiments of the disclosure, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 82, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 69, or a sequence with 95-99% identity thereof.

In embodiments of the invention, the anti-CLL-1 binding domain comprises the light chain variable region provided in SEQ ID NO: 83 and the heavy chain variable region provided in SEQ ID NO: 70. In embodiments of the disclosure, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 83, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 70, or a sequence with 95-99% identity thereof.

In embodiments of the invention, the anti-CLL-1 binding domain comprises the light chain variable region provided in SEQ ID NO: 84 and the heavy chain variable region provided in SEQ ID NO: 71. In embodiments of the disclosure, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 84, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 71, or a sequence with 95-99% identity thereof.

In embodiments of the invention, the anti-CLL-1 binding domain comprises the light chain variable region provided in SEQ ID NO: 85 and the heavy chain variable region provided in SEQ ID NO: 72. In embodiments of the disclosure, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 85, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 72, or a sequence with 95-99% identity thereof.

In embodiments of the invention, the anti-CLL-1 binding domain comprises the light chain variable region provided in SEQ ID NO: 86 and the heavy chain variable region provided in SEQ ID NO: 73. In embodiments of the disclosure, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 86, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 73, or a sequence with 95-99% identity thereof.

In embodiments of the invention, the anti-CLL-1 binding domain comprises the light chain variable region provided in SEQ ID NO: 87 and the heavy chain variable region provided in SEQ ID NO: 74. In embodiments of the disclosure, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 87, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 74, or a sequence with 95-99% identity thereof.

In embodiments of the invention, the anti-CLL-1 binding domain comprises the light chain variable region provided in SEQ ID NO: 88 and the heavy chain variable region provided in SEQ ID NO: 75. In embodiments of the disclosure, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 88, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 75, or a sequence with 95-99% identity thereof.

In embodiments of the invention, the anti-CLL-1 binding domain comprises the light chain variable region provided in SEQ ID NO: 89 and the heavy chain variable region provided in SEQ ID NO: 76. In embodiments of the disclosure, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 89, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 76, or a sequence with 95-99% identity thereof.

In embodiments of the invention, the anti-CLL-1 binding domain comprises the light chain variable region provided in SEQ ID NO: 90 and the heavy chain variable region provided in SEQ ID NO: 77. In embodiments of the disclosure, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 90, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 77, or a sequence with 95-99% identity thereof.

In embodiments of the invention, the anti-CLL-1 binding domain comprises the light chain variable region provided in SEQ ID NO: 196 and the heavy chain variable region provided in SEQ ID NO: 195. In embodiments of the disclosure, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 196, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 195, or a sequence with 95-99% identity thereof.

In embodiments, the anti-CLL-1 binding domain comprises a light chain variable region and a heavy chain variable region of any anti-CLL-1 binding domain of Table 2, as defined in the claims. In embodiments, the anti-CLL-1 binding domain comprises a light chain variable region amino acid sequence selected from the group consisting of SEQ ID NO: 78-90, and a heavy chain variable region amino acid sequence selected from the group consisting of SEQ ID NO: 65-77, as defined in the claims.

In some aspects, a non-human anti-CLL-1 binding domain is humanized, where specific sequences or regions of the antibody are modified to increase similarity to an antibody naturally produced in a human or fragment thereof. In one aspect, the antigen binding domain is humanized.

A humanized antibody can be produced using a variety of techniques known in the art, including but not limited to, CDR-grafting (see, e.g., European Patent No. EP 239,400; International Publication No. WO 91/09967; and U.S. Pat. Nos. 5,225,539, 5,530,101, and 5,585,089), veneering or resurfacing (see, e.g., European Patent Nos. EP 592,106 and EP 519,596; Padlan, 1991, Molecular Immunology, 28(4/5):489-498; Studnicka et al., 1994, Protein Engineering, 7(6):805-814; and Roguska et al., 1994, PNAS, 91:969-973), chain shuffling (see, e.g., U.S. Pat. No. 5,565,332), and techniques disclosed in, e.g., U.S. Patent Application Publication No. US2005/0042664, U.S. Patent Application Publication No. US2005/0048617, U.S. Pat. No. 6,407,213, U.S. Pat. No. 5,766,886, International Publication No. WO 9317105, Tan et al., J. Immunol., 169:1119-25 (2002), Caldas et al., Protein Eng., 13(5):353-60 (2000), Morea et al., Methods, 20(3):267-79 (2000), Baca et al., J. Biol. Chem., 272(16):10678-84 (1997), Roguska et al., Protein Eng., 9(10):895-904 (1996), Couto et al., Cancer Res., 55 (23 Supp):5973s-5977s (1995), Couto et al., Cancer Res., 55(8):1717-22 (1995), Sandhu J S, Gene, 150(2):409-10 (1994), and Pedersen et al., J. Mol. Biol., 235(3):959-73 (1994). Often, framework residues in the framework regions will be substituted with the corresponding residue from the CDR donor antibody to alter, for example improve, antigen binding. These framework substitutions, e.g., conservative substitutions are identified by methods well-known in the art, e.g., by modeling of the interactions of the CDR and framework residues to identify framework residues important for antigen binding and sequence comparison to identify unusual framework residues at particular positions. (See, e.g., Queen et al., U.S. Pat. No. 5,585,089; and Riechmann et al., 1988, Nature, 332:323.)

A humanized antibody or antibody fragment has one or more amino acid residues remaining in it from a source which is nonhuman. These nonhuman amino acid residues are often referred to as "import" residues, which are typically taken from an "import" variable domain. As provided herein, humanized antibodies or antibody fragments comprise one or more CDRs from nonhuman immunoglobulin molecules and framework regions wherein the amino acid residues comprising the framework are derived completely or mostly from human germline. Multiple techniques for humanization of antibodies or antibody fragments are well-known in the art and can essentially be performed following the method of Winter and coworkers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody, i.e., CDR-grafting (EP 239,400; PCT Publication No. WO 91/09967; and U.S. Pat. Nos. 4,816,567; 6,331,415; 5,225,539; 5,530,101; 5,585,089; 6,548,640). In such humanized antibodies and antibody fragments, substantially less than an intact human variable domain has been substituted by the corresponding sequence from a nonhuman species. Humanized antibodies are often human antibodies in which some CDR residues and possibly some framework (FR) residues are substituted by residues from analogous sites in rodent antibodies. Humanization of antibodies and antibody fragments can also be achieved by veneering or resurfacing (EP 592,106; EP 519,596; Padlan, 1991, Molecular Immunology, 28(4/5):489-498; Studnicka et al., Protein Engineering, 7(6):805-814 (1994); and Roguska et al., PNAS, 91:969-973 (1994)) or chain shuffling (U.S. Pat. No. 5,565,332).

The choice of human variable domains, both light and heavy, to be used in making the humanized antibodies is to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987)). Another method uses a particular framework derived from the consensus sequence of all human antibodies of a particular subgroup of light or heavy chains. The same framework may be used for several different humanized antibodies (see, e.g., Nicholson et al. Mol. Immun. 34 (16-17): 1157-1165 (1997); Carter et al., Proc. Natl. Acad. Sci. USA, 89:4285 (1992); Presta et al., J. Immunol., 151:2623 (1993)). In some embodiments, the framework region, e.g., all four framework regions, of the heavy chain variable region are derived from a VH4_4-59 germline sequence. In one embodiment, the framework region can comprise, one, two, three, four or five modifications, e.g., substitutions, e.g., conservative substitutions, e.g., from the amino acid at the corresponding murine sequence. In one embodiment, the framework region, e.g., all four framework regions of the light chain variable region are derived from a VK3_1.25 germline sequence. In one embodiment, the framework region can comprise, one, two, three, four or five modifications, e.g., substitutions, e.g., conservative substitutions, e.g., from the amino acid at the corresponding murine sequence.

In some aspects, the portion of a multispecific molecule, e.g., bispecific molecule, e.g., bispecific antibody or bispecific antibody-like molecule, composition of the invention that comprises an antibody fragment is humanized with retention of high affinity for the target antigen and other favorable biological properties. According to one aspect of the invention, humanized antibodies and antibody fragments are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, e.g., the analysis of residues that influence the ability of the candidate immunoglobulin to bind the target antigen. In this way, FR residues can be selected and combined from the recipient and import sequences so that the desired antibody or antibody fragment characteristic, such as increased affinity for the target antigen, is achieved. In general, the CDR residues are directly and most substantially involved in influencing antigen binding.

A humanized antibody or antibody fragment may retain a similar antigenic specificity as the original antibody, e.g., in the present invention, the ability to bind human CLL-1. In some embodiments, a humanized antibody or antibody fragment may have improved affinity and/or specificity of binding to human CLL-1.

In one aspect, the anti-CLL-1 binding domain is characterized by particular functional features or properties of an antibody or antibody fragment. For example, in one aspect, the portion of a multispecific molecule, e.g., bispecific molecule, e.g., bispecific antibody or bispecific antibody-like molecule, composition of the invention that comprises an antigen binding domain to CLL-1 specifically binds human CLL-1.

In one aspect, the anti-CLL-1 antigen binding domain has the same or a similar binding specificity to human CLL-1 as mouse CLL-1. In one aspect, the invention relates to an antigen binding domain comprising an antibody or antibody fragment, wherein the antibody binding domain specifically binds to a CLL-1 protein or fragment thereof, wherein the antibody or antibody fragment comprises a variable light chain and/or a variable heavy chain that includes an amino acid sequence of SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 195, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, or SEQ ID NO: 196 as defined in the claims. In one aspect, the antigen binding domain comprises an amino acid sequence of an scFv selected from SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, and SEQ ID NO: 51. In certain aspects, the scFv is contiguous with and in the same reading frame as a leader sequence. In one aspect the leader sequence is the polypeptide sequence provided as SEQ ID NO:1.

In one aspect, the anti-CLL-1 binding domain is a fragment, e.g., a single chain variable fragment (scFv). In one aspect, the anti-CLL-1 binding domain is a Fv, a Fab, a (Fab')2, or a bi-functional (e.g. bi-specific) hybrid antibody (e.g., Lanzavecchia et al., Eur. J. Immunol. 17, 105 (1987)). In one aspect, the antibodies and fragments thereof of the invention binds a CLL-1 protein with wild-type or enhanced affinity.

In some instances, scFvs can be prepared according to method known in the art (see, for example, Bird et al., (1988) Science 242:423-426 and Huston et al., (1988) Proc. Natl. Acad. Sci. USA 85:5879-5883). ScFv molecules can be produced by linking VH and VL regions together using flexible polypeptide linkers. The scFv molecules comprise a linker (e.g., a Ser-Gly linker) with an optimized length and/or amino acid composition. The linker length can greatly affect how the variable regions of a scFv fold and interact. In fact, if a short polypeptide linker is employed (e.g., between 5-10 amino acids) intrachain folding is prevented. Interchain folding is also required to bring the two variable regions together to form a functional epitope binding site. For examples of linker orientation and size see, e.g., Hollinger et al. 1993 Proc Natl Acad. Sci. U.S.A. 90:6444-6448, U.S. Patent Application Publication Nos. 2005/0100543, 2005/0175606, 2007/0014794, and PCT publication Nos. WO2006/020258 and WO2007/024715.

An scFv can comprise a linker of at least 1, 2, 3, 4, 5, 6, 7, 8, 9, 10, 11, 12, 13, 14, 15, 16, 17, 18, 19, 20, 25, 30, 35, 40, 45, 50, or more amino acid residues between its VL and VH regions. The linker sequence may comprise any naturally occurring amino acid. In some embodiments, the linker sequence comprises amino acids glycine and serine. In another embodiment, the linker sequence comprises sets of glycine and serine repeats such as (Gly₄Ser)n, where n is a positive integer equal to or greater than 1 (SEQ ID NO:25). In one embodiment, the linker can be (Gly₄Ser)₄ (SEQ ID NO:27) or (Gly₄Ser)₃(SEQ ID NO:28). Variation in the linker length may retain or enhance activity, giving rise to superior efficacy in activity studies.

### Exemplary Anti-CLL-1 Binding Domains

Exemplary CLL-1 multispecific molecule, e.g., bispecific molecule, e.g., bispecific antibody or bispecific antibody-like molecule, constructs disclosed herein comprise an scFv (e.g., a scFv as disclosed in Table 2, optionally preceded with an optional leader sequence (e.g., SEQ ID NO:1 and SEQ ID NO:12 for exemplary leader amino acid and nucleotide sequences, respectively)). The sequences of the scFv fragments (SEQ ID NOs: 39-51, not including the optional leader sequence) are provided herein in Table 2 and the description below. The CLL-1 multispecific molecule, e.g., bispecific molecule, e.g., bispecific antibody or bispecific antibody-like molecule, construct can further comprise one or more additional antibody domains, e.g., one or more LCs, one or more CH1s, one or more CH2s, one or more CH3s, one or more hinge domains, and/or one or more additional VL and/or VH domains, e.g., as described herein. In certain embodiments, the domains are contiguous with and in the same reading frame to form single polypeptide. In other embodiments, the domain are in separate polypeptides, e.g., as in a multispecific antibody or antibody-like molecule described herein.

In certain embodiments of the disclosure, the CLL-1 multispecific molecule includes an amino acid sequence (e.g., a VL, a VH and/or a scFv sequence) of, or includes an amino acid sequence ((e.g., a VL, a VH and/or a scFv sequence) encoded by the nucleotide sequence of, CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264, provided in Table 2, or a sequence substantially (e.g., 95-99%) identical thereto.

In certain embodiments of the disclosure, the CLL-1 multispecific molecule, or the anti-CLL-1 antigen binding domain, includes the scFv amino acid sequence of, CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264 provided in Table 2 (with or without the leader sequence), or a sequence substantially identical (e.g., 95-99% identical, or up to 20, 15, 10, 8, 6, 5, 4, 3, 2, or 1 amino acid changes, e.g., substitutions (e.g., conservative substitutions)) to any of the aforesaid sequences.

In certain embodiments of the disclosure, the CLL-1 multispecific molecule, or the anti-CLL-1 antigen binding domain, includes the heavy chain variable region and/or the light chain variable region of, CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264 provided in Table 2, or a sequence substantially identical (e.g., 95-99% identical, or up to 20, 15, 10, 8, 6, 5, 4, 3, 2, or 1 amino acid changes, e.g., substitutions (e.g., conservative substitutions)) to any of the aforesaid sequences.

In certain embodiments of the disclosure, the CLL-1 multispecific molecule, or the anti-CLL-1 antigen binding domain, includes one, two or three CDRs from the heavy chain variable region (e.g., HCDR1, HCDR2 and/or HCDR3) of CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264 provided in Table 3; and/or one, two or three CDRs from the light chain variable region (e.g., LCDR1, LCDR2 and/or LCDR3) of CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264, provided in Table 4; or a sequence substantially identical (e.g., 95-99% identical, or up to 20, 15, 10, 8, 6, 5, 4, 3, 2, or 1 amino acid changes, e.g., substitutions (e.g., conservative substitutions)) to any of the aforesaid sequences.

In certain embodiments of the disclosure, the CLL-1 multispecific molecule, or the anti-CLL-1 antigen binding domain, includes one, two or three CDRs from the heavy chain variable region (e.g., HCDR1, HCDR2 and/or HCDR3) of CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264, provided in Table 5; and/or one, two or three CDRs from the light chain variable region (e.g., LCDR1, LCDR2 and/or LCDR3) of CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264, provided in Table 6; or a sequence substantially identical (e.g., 95-99% identical, or up to 20, 15, 10, 8, 6, 5, 4, 3, 2, or 1 amino acid changes, e.g., substitutions (e.g., conservative substitutions)) to any of the aforesaid sequences.

In certain embodiments of the disclosure, the CLL-1 multispecific molecule, or the anti-CLL-1 antigen binding domain, includes one, two or three CDRs from the heavy chain variable region (e.g., HCDR1, HCDR2 and/or HCDR3) of CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264, provided in Table 7; and/or one, two or three CDRs from the light chain variable region (e.g., LCDR1, LCDR2 and/or LCDR3) of CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264, provided in Table 8; or a sequence substantially identical (e.g., 95-99% identical, or up to 20, 15, 10, 8, 6, 5, 4, 3, 2, or 1 amino acid changes, e.g., substitutions (e.g., conservative substitutions)) to any of the aforesaid sequences.

The amino acid and nucleic acid sequences of exemplary CLL-1 scFv domains and exemplary CLL-1 VL and VH domains are provided in Table 2.

Table 1 below designates the nicknames for the CLL-1 constructs with respect to the DNA ID number, also listed in Table 1.

**Table 1: Names of exemplary anti-CLL-1 constructs.**

| Construct ID | Nickname |
|---|---|
| 139115 | CLL-1-1 |
| 139116 | CLL-1-2 |
| 139117 | CLL-1-3 |
| 139118 | CLL-1-4 |
| 139119 | CLL-1-5 |
| 139120 | CLL-1-6 |
| 139121 | CLL-1-7 |
| 139122 | CLL-1-8 |
| 146259 | CLL-1-9 |
| 146261 | CLL-1-10 |
| 146262 | CLL-1-11 |
| 146263 | CLL-1-12 |
| 146264 | CLL-1-13 |

Table 2: Human CLL-1 binding domain sequences

**Table 2: Amino Acid and Nucleic Acid Sequences of anti-CLL-1 binding domains**

| **Name/Description** | **SEQ ID NO:** | **Sequence** |
|---|---|---|
| **139115** | | |
| **139115- aa ScFv domain CLL-1-1** | 39 | |
| **139115- nt ScFv domain CLL-1-1** | 52 | |
| **139115- aa VH of ScFv CLL-1-1** | 65 | |
| **139115- aa VL of ScFv CLL-1-1** | 78 | |

| **139116** | | |
|---|---|---|
| **139116- aa ScFv domain CLL-1-2** | 40 | |
| **139116- nt ScFv domain CLL-1-2** | 53 | |
| **139116- aa VH of ScFv CLL-1-2** | 66 | |
| **139116- aa VL of ScFv CLL-1-2** | 79 | |

| **139118** | | |
|---|---|---|
| **139118- aa ScFv domain CLL-1-3** | 41 | |
| **139118- nt ScFv domain CLL-1-3** | 54 | |
| **139118- aa VH of ScFv CLL-1-3** | 67 | |
| **139118- aa VL of ScFv CLL-1-3** | 80 | |

| **139122** | | |
|---|---|---|
| **139122- aa ScFv domain CLL-1-4** | 42 | |
| **139122- nt ScFv domain CLL-1-4** | 55 | |
| **139122- aa VH of ScFv CLL-1-4** | 68 | |
| **139122- aa VL of ScFv CLL-1-4** | 81 | |

| **139117** | | |
|---|---|---|
| **139117- aa ScFv domain CLL-1-5** | 43 | |
| **139117-nt ScFv domain CLL-1-5** | 56 | |
| **139117- aa VH of ScFv CLL-1-5** | 69 | |
| **139117- aa VL of ScFv CLL-1-5** | 82 | |

| **139119** | | |
|---|---|---|
| **139119- aa ScFv domain CLL-1-6** | 44 | |
| **139119- nt ScFv domain CLL-1-6** | 57 | |
| **139119- aa VH of ScFv CLL-1-6** | 70 | |
| **139119- aa VL of ScFv CLL-1-6** | 83 | |

| **139120** | | |
|---|---|---|
| **139120- aa ScFv domain CLL-1-7** | 45 | |
| | | |
| **139120- nt ScFv domain CLL-1-7** | 58 | |
| **139120- aa VH of ScFv CLL-1-7** | 71 | |
| **139120- aa VL of ScFv CLL-1-7** | 84 | |

| **139121** | | |
|---|---|---|
| **139121- aa ScFv domain CLL-1-8** | 46 | |
| **139121- nt ScFv domain CLL-1-8** | 59 | |
| **139121- aa VH of ScFv CLL-1-8** | 72 | |
| **139121- aa VL of ScFv CLL-1-8** | 85 | |

| **146259** | | |
|---|---|---|
| **146259- aa ScFv domain CLL-1-9** | 47 | |
| | | |
| **146259- nt ScFv domain CLL-1-9** | 60 | |
| **146259- aa VH of ScFv CLL-1-9** | 73 | |
| **146259- aa VL of ScFv CLL-1-9** | 86 | |

| **146261** | | |
|---|---|---|
| **146261- aa ScFv domain CLL-1-10** | 48 | |
| **146261- nt ScFv domain CLL-1-10** | 61 | |
| **146261- aa VH of ScFv CLL-1-10** | 74 | |
| **146261- aa VL of ScFv CLL-1-10** | 87 | |

| **146262** | | |
|---|---|---|
| **146262- aa ScFv domain CLL-1-11** | 49 | |
| | | |
| **146262- nt ScFv domain CLL-1-11** | 62 | |
| **146262- aa VH of ScFv CLL-1-11** | 75 | |
| **146262- aa VL of ScFv CLL-1-11** | 88 | |

| **146263** | | |
|---|---|---|
| **146263- aa ScFv domain CLL-1-12** | 50 | |
| **146263- nt ScFv domain CLL-1-12** | 63 | |
| **146263- aa VH of ScFv CLL-1-12** | 76 | |
| **146263- aa VL of ScFv CLL-1-12** | 89 | |

| **146264** | | |
|---|---|---|
| **146264- aa ScFv domain CLL-1-13** | 51 | |
| **146264- nt ScFv domain CLL-1-13** | 64 | |
| **146264- aa VH of ScFv CLL-1-13** | 77 | |
| **146264- aa VL of ScFv CLL-1-13** | 90 | |

| **181268** | | |
|---|---|---|
| **181268- aa VH of ScFv** | 195 | |
| **181268- aa VL of ScFv** | 196 | |

In embodiments of the disclosure, additional exemplary CLL-1 multispecific molecule, e.g., bispecific molecule, e.g., bispecific antibody or bispecific antibody-like molecule, constructs comprise an antigen binding domain comprising one or more, e.g., one, two, or three, CDRs of the heavy chain variable domain and/or one or more, e.g., one, two, or three, CDRs of the light chain variable domain, or the VH and/or VL, or the scFv sequence, of the scFv sequence the anti-CLL-1 (CLEC12A) antibody disclosed in PCT Publication WO2014/051433.

In embodiments, the VL of the CLL-1 binding domain precedes the VH ("L2H"). In other embodiments, the VH of the CLL-1 binding domain precedes the VL ("H2L").

The antigen binding domain of the multispecific molecule, e.g., bispecific molecule, e.g., bispecific antibody or bispecific antibody-like molecule, construct can include a Gly/Ser linker having one or more of the following sequences: GGGGS (SEQ ID NO:25); encompassing 1-6 "Gly Gly Gly Gly Ser" repeating units, e.g., GGGGSGGGGS GGGGSGGGGS GGGGSGGGGS (SEQ ID NO:26); GGGGSGGGGS GGGGSGGGGS (SEQ ID NO:27); GGGGSGGGGS GGGGS (SEQ ID NO:28); GGGS (SEQ ID NO:29); or encompassing 1-10 "Gly Gly Gly Ser" repeating units, e.g., GGGSGGGSGG GSGGGSGGGS GGGSGGGSGG GSGGGSGGGS (SEQ ID NO:38). Alternatively, the antigen binding domain of the multispecific molecule, e.g., bispecific molecule, e.g., bispecific antibody or bispecific antibody-like molecule, construct can include, for example, a linker including the sequence GSTSGSGKPGSGEGSTKG (SEQ ID NO: 1108)

In embodiments, the nucleic acid construct encoding a multispecific molecule, e.g., bispecific molecule, e.g., bispecific antibody or bispecific antibody-like molecule, includes a poly A sequence, e.g., a sequence encompassing 50-5000 or 100-5000 adenines (e.g., SEQ ID NO:30, SEQ ID NO:33, SEQ ID NO:34 or SEQ ID NO:35), or a sequence encompassing 50-5000 thymines (e.g., SEQ ID NO:31, SEQ ID NO:32).

The human CDR sequences of the scFv domains are shown in Tables 3, 5, and 7 for the heavy chain variable domains and in Tables 4, 6, and 8 for the light chain variable domains. "ID" stands for the respective SEQ ID NO for each CDR.

**Table 3. Heavy Chain Variable Domain CDRs according to a combination of the Kabat and Chothia numbering scheme.**

| **Candidate** | **HCDR1** | **ID** | **HCDR2** | **ID** | **HCDR3** | **ID** |
|---|---|---|---|---|---|---|
| CLL-1-9 | ANTFSDHVMH | 125 | | 138 | GGYNSDAFDI | 151 |
| CLL-1-6 | GGSFSGYYWS | 122 | | 135 | GSGLVVYAIRVGSGWFDY | 148 |
| CLL-1-10 | GFTFSSYSMN | 126 | | 139 | DLSVRAIDAFDI | 152 |
| CLL-1-11 | GFTFNSYGLH | 127 | | 140 | EGNEDLAFDI | 153 |
| CLL-1-12 | GFNVSSNYMT | 128 | | 141 | | 154 |
| CLL-1-1 | GGTFSSYAIS | 117 | GIIPIFGTANYAQKFQ | 130 | DLEMATIMGGY | 143 |
| CLL-1-2 | GFTFDDYAMH | 118 | | 131 | VFDSYYMDV | 144 |
| CLL-1-3 | GGSISSSSYYWG | 119 | | 132 | PGTYYDFLSGYYPFY | 145 |
| CLL-1-4 | GFTFSSYWMS | 120 | | 133 | DLRSGRY | 146 |
| CLL-1-5 | | 121 | | 134 | GTATFDWNFPFDS | 147 |
| CLL-1-7 | GFTFSSYSMN | 123 | | 136 | | 149 |
| CLL-1-8 | GFTFSSYEMN | 124 | | 137 | EALGSSWE | 150 |
| CLL-1-13 | GYPFTGYYIQ | 129 | | 142 | DSYGYYYGMDV | 155 |
| 181268 | GFTFSSYEMN | 199 | | 200 | DPYSSSWHDAFDI | 201 |

**Table 4. Light Chain Variable Domain CDRs according to a combination of the Kabat and Chothia numbering scheme.**

| **Candidate** | **LCDR1** | **ID** | **LCDR2** | **ID** | **LCDR3** | **ID** |
|---|---|---|---|---|---|---|
| CLL-1-9 | RASQDISSWLA | 164 | AASSLQS | 177 | QQSYSTPLT | 190 |
| CLL-1-6 | RASQSISSYLN | 161 | AASSLQS | 174 | QQSYSTPPWT | 187 |
| CLL-1-10 | QASQDISNYLN | 165 | DASNLET | 178 | QQAYSTPFT | 191 |
| CLL-1-11 | QASQFIKKNLN | 166 | DASSLQT | 179 | QQHDNLPLT | 192 |
| CLL-1-12 | RASQSISSYLN | 167 | AASSLQS | 180 | QQSYSTPPLT | 193 |
| CLL-1-1 | TGTSSDVGGYNYVS | 156 | DVSNRPS | 169 | SSYTSSSTLDVV | 182 |
| CLL-1-2 | RSSQSLVYTDGNTYLN | 157 | KVSNRDS | 170 | MQGTHWSFT | 183 |
| CLL-1-3 | RASQGISSYLA | 158 | AASTLQS | 171 | QQLNSYPYT | 184 |
| CLL-1-4 | RASQSISGSFLA | 159 | GASSRAT | 172 | QQYGSSPPT | 185 |
| CLL-1-5 | RASQSISSYLN | 160 | AASSLQS | 173 | QQSYSTPWT | 186 |
| CLL-1-7 | TGSSGSIASNYVQ | 162 | EDNQRPS | 175 | QSYDSSNQVV | 188 |
| CLL-1-8 | QASQDISNYLN | 163 | DASNLET | 176 | QQYDNLPLT | 189 |
| CLL-1-13 | RASQGISSALA | 168 | DASSLES | 181 | QQFNNYPLT | 194 |
| 181268 | RASQSVSSSYLA | 202 | GASSRAT | 203 | QQYGSSPLT | 204 |

**Table 5. Heavy Chain Variable Domain CDRs according to the Kabat numbering scheme (Kabat et al. (1991), "Sequences of Proteins of Immunological Interest," 5th Ed. Public Health Service, National Institutes of Health, Bethesda, MD)**

| **Candidate** | **HCDR1** | **ID** | **HCDR2** | **ID** | **HCDR3** | **ID** |
|---|---|---|---|---|---|---|
| 146259-CLL-1-9 | DHVMH | 322 | | 336 | GGYNSDAFDI | 350 |
| 139119-CLL-1-6 | GYYWS | 319 | EINHSGSTNYNPSLKS | 333 | | 347 |
| 146261-CLL-1-10 | SYSMN | 323 | | 337 | DLSVRAIDAFDI | 351 |
| 146262-CLL-1-11 | SYGLH | 324 | | 338 | EGNEDLAFDI | 352 |
| 146263-CLL-1-12 | SNYMT | 325 | | 339 | | 353 |
| 139115-CLL-1-1 | SYAIS | 314 | | 328 | DLEMATIMGGY | 342 |
| 139116-CLL-1-2 | DYAMH | 315 | | 329 | VFDSYYMDV | 343 |
| 139118-CLL-1-3 | SSSYYWG | 316 | SIYYSGSTYYNPSLKS | 330 | | 344 |
| 139122-CLL-1-4 | SYWMS | 317 | | 331 | DLRSGRY | 345 |
| 139117-CLL-1-5 | SGSHYWN | 318 | YIYYSGSTNYNPSLEN | 332 | GTATFDWNFPFDS | 346 |
| 139120-CLL-1-7 | SYSMN | 320 | | 334 | | 348 |
| 139121-CLL-1-8 | SYEMN | 321 | | 335 | EALGSSWE | 349 |
| 146264-CLL-1-13 | GYYIQ | 326 | | 340 | DSYGYYYGMDV | 354 |
| 181268 | SYEMN | 327 | | 341 | DPYSSSWHDAFDI | 355 |

**Table 6. Light Chain Variable Domain CDRs according to the Kabat numbering scheme (Kabat et al. (1991), "Sequences of Proteins of Immunological Interest," 5th Ed. Public Health Service, National Institutes of Health, Bethesda, MD)**

| **Candidate** | **LCDR1** | **ID** | **LCDR2** | **ID** | **LCDR3** | **ID** |
|---|---|---|---|---|---|---|
| 146259-CLL-1-9 | RASQDISSWLA | 364 | AASSLQS | 378 | QQSYSTPLT | 392 |
| 139119-CLL-1-6 | RASQSISSYLN | 361 | AASSLQS | 375 | QQSYSTPPWT | 389 |
| 146261-CLL-1-10 | QASQDISNYLN | 365 | DASNLET | 379 | QQAYSTPFT | 393 |
| 146262-CLL-1-11 | QASQFIKKNLN | 366 | DASSLQT | 380 | QQHDNLPLT | 394 |
| 146263-CLL-1-12 | RASQSISSYLN | 367 | AASSLQS | 381 | QQSYSTPPLT | 395 |
| 139115-CLL-1-1 | TGTSSDVGGYNYVS | 356 | DVSNRPS | 370 | SSYTSSSTLDVV | 384 |
| 139116-CLL-1-2 | RSSQSLVYTDGNTYLN | 357 | KVSNRDS | 371 | MQGTHWSFT | 385 |
| 139118-CLL-1-3 | RASQGISSYLA | 358 | AASTLQS | 372 | QQLNSYPYT | 386 |
| 139122-CLL-1-4 | RASQSISGSFLA | 359 | GASSRAT | 373 | QQYGSSPPT | 387 |
| 139117-CLL-1-5 | RASQSISSYLN | 360 | AASSLQS | 374 | QQSYSTPWT | 388 |
| 139120-CLL-1-7 | TGSSGSIASNYVQ | 362 | EDNQRPS | 376 | QSYDSSNQVV | 390 |
| 139121-CLL-1-8 | QASQDISNYLN | 363 | DASNLET | 377 | QQYDNLPLT | 391 |
| 146264-CLL-1-13 | RASQGISSALA | 368 | DASSLES | 382 | QQFNNYPLT | 396 |
| 181268 | RASQSVSSSYLA | 369 | GASSRAT | 383 | QQYGSSPLT | 397 |

**Table 7. Heavy Chain Variable Domain CDRs according to the Chothia numbering scheme (Al-Lazikani et al., (1997) JMB 273,927-948)**

| **Candidate** | **HCDR1** | **ID** | **HCDR2** | **ID** | **HCDR3** | **ID** |
|---|---|---|---|---|---|---|
| 146259-CLL-1-9 | ANTFSDH | 406 | HAANGG | 420 | GGYNSDAFDI | 434 |
| 139119-CLL-1-6 | GGSFSGY | 403 | NHSGS | 417 | GSGLVVYAIRVGSGWFDY | 431 |
| 146261-CLL-1-10 | GFTFSSY | 407 | SSSSST | 421 | DLSVRAIDAFDI | 435 |
| 146262-CLL-1-11 | GFTFNSY | 408 | EYDGSN | 422 | EGNEDLAFDI | 436 |
| 146263-CLL-1-12 | GFNVSSN | 409 | YSGGA | 423 | DRLYCGNNCYLYYYYGMDV | 437 |
| 139115-CLL-1-1 | GGTFSSY | 398 | IPIFGT | 412 | DLEMATIMGGY | 426 |
| 139116-CLL-1-2 | GFTFDDY | 399 | SGDGGS | 413 | VFDSYYMDV | 427 |
| 139118-CLL-1-3 | GGSISSSSY | 400 | YYSGS | 414 | PGTYYDFLSGYYPFY | 428 |
| 139122-CLL-1-4 | GFTFSSY | 401 | NEDGSA | 415 | DLRSGRY | 429 |
| 139117-CLL-1-5 | GGPVRSGSH | 402 | YYSGS | 416 | GTATFDWNFPFDS | 430 |
| 139120-CLL-1-7 | GFTFSSY | 404 | SSSSSY | 418 | DPSSSGSYYMEDSYYYGMDV | 432 |
| 139121-CLL-1-8 | GFTFSSY | 405 | SSSGST | 419 | EALGSSWE | 433 |
| 146264-CLL-1-13 | GYPFTGY | 410 | DPNSGN | 424 | DSYGYYYGMDV | 438 |
| 181268 | GFTFSSY | 411 | SSSGST | 425 | DPYSSSWHDAFDI | 439 |

**Table 8. Light Chain Variable Domain CDRs according to the Chothia numbering scheme (Al-Lazikani et al., (1997) JMB 273,927-948)**

| **Candidate** | **LCDR1** | **ID** | **LCDR2** | **ID** | **LCDR3** | **ID** |
|---|---|---|---|---|---|---|
| 146259-CLL-1-9 | SQDISSW | 448 | AAS | 462 | SYSTPL | 476 |
| 139119-CLL-1-6 | SQSISSY | 445 | AAS | 459 | SYSTPPW | 473 |
| 146261-CLL-1-10 | SQDISNY | 449 | DAS | 463 | AYSTPF | 477 |
| 146262-CLL-1-11 | SQFIKKN | 450 | DAS | 464 | HDNLPL | 478 |
| 146263-CLL-1-12 | SQSISSY | 451 | AAS | 465 | SYSTPPL | 479 |
| 139115-CLL-1-1 | TSSDVGGYNY | 440 | DVS | 454 | YTSSSTLDV | 468 |
| 139116-CLL-1-2 | SQSLVYTDGNTY | 441 | KVS | 455 | GTHWSF | 469 |
| 139118-CLL-1-3 | SQGISSY | 442 | AAS | 456 | LNSYPY | 470 |
| 139122-CLL-1-4 | SQSISGSF | 443 | GAS | 457 | YGSSPP | 471 |
| 139117-CLL-1-5 | SQSISSY | 444 | AAS | 458 | SYSTPW | 472 |
| 139120-CLL-1-7 | SSGSIASNY | 446 | EDN | 460 | YDSSNQV | 474 |
| 139121-CLL-1-8 | SQDISNY | 447 | DAS | 461 | YDNLPL | 475 |
| 146264-CLL-1-13 | SQGISSA | 452 | DAS | 466 | FNNYPL | 480 |
| 181268 | SQSVSSSY | 453 | GAS | 467 | YGSSPL | 481 |

In certain embodiments, the multispecific molecule, e.g., bispecific molecule, e.g., bispecific antibody or bispecific antibody-like molecule, described herein (*e.g.,* the multispecific molecule, e.g., bispecific molecule, e.g., bispecific antibody or bispecific antibody-like molecule, nucleic acid or a multispecific molecule, e.g., bispecific molecule, e.g., bispecific antibody or bispecific antibody-like molecule, polypeptide) or a CLL-1 binding domain includes a CLL-1 binding domain that includes:
(1) one, two, or three light chain (LC) CDRs chosen from one of the following:
   (i) a LC CDR1 of SEQ ID NO: 156, LC CDR2 of SEQ ID NO: 169 and LC CDR3 of SEQ ID NO: 182 of CLL-1-1;
   (ii) a LC CDR1 of SEQ ID NO: 157, LC CDR2 of SEQ ID NO: 170 and LC CDR3 of SEQ ID NO: 183 of CLL-1-2;
   (iii) a LC CDR1 of SEQ ID NO: 158, LC CDR2 of SEQ ID NO: 171 and LC CDR3 of SEQ ID NO: 184 of CLL-1-3;
   (iv) a LC CDR1 of SEQ ID NO: 159, LC CDR2 of SEQ ID NO: 172 and LC CDR3 of SEQ ID NO: 185 of CLL-1-4;
   (v) a LC CDR1 of SEQ ID NO: 160, LC CDR2 of SEQ ID NO: 173 and LC CDR3 of SEQ ID NO: 186 of CLL-1-5;
   (vi) a LC CDR1 of SEQ ID NO: 161, LC CDR2 of SEQ ID NO: 174 and LC CDR3 of SEQ ID NO: 187 of CLL-1-6;
   (vii) a LC CDR1 of SEQ ID NO: 162, LC CDR2 of SEQ ID NO: 175 and LC CDR3 of SEQ ID NO: 188 of CLL-1-7;
   (viii) a LC CDR1 of SEQ ID NO: 163, LC CDR2 of SEQ ID NO: 176 and LC CDR3 of SEQ ID NO: 189 of CLL-1-8; or
   (ix) a LC CDR1 of SEQ ID NO: 164, LC CDR2 of SEQ ID NO: 177 and LC CDR3 of SEQ ID NO: 190 of CLL-1-9;
   (x) a LC CDR1 of SEQ ID NO: 165, LC CDR2 of SEQ ID NO: 178 and LC CDR3 of SEQ ID NO: 191 of CLL-1-10;
   (xi) a LC CDR1 of SEQ ID NO: 166, LC CDR2 of SEQ ID NO: 179 and LC CDR3 of SEQ ID NO: 192 of CLL-1-11;
   (xii) a LC CDR1 of SEQ ID NO: 167, LC CDR2 of SEQ ID NO: 180 and LC CDR3 of SEQ ID NO: 193 of CLL-1-12;
   (xiii) a LC CDR1 of SEQ ID NO: 168, LC CDR2 of SEQ ID NO: 181 and LC CDR3 of SEQ ID NO: 194 of CLL-1-13;
   (xiv) a LC CDR1 of SEQ ID NO: 202, LC CDR2 of SEQ ID NO: 203 and LC CDR3 of SEQ ID NO: 204 of 181286; and/or
(2) one, two, or three heavy chain (HC) CDRs from one of the following:
   (i) a HC CDR1 of SEQ ID NO: 117, HC CDR2 of SEQ ID NO: 130 and HC CDR3 of SEQ ID NO: 143 of CLL-1-1;
   (ii) a HC CDR1 of SEQ ID NO: 118, HC CDR2 of SEQ ID NO: 131 and HC CDR3 of SEQ ID NO: 144 of CLL-1-2;
   (iii) a HC CDR1 of SEQ ID NO: 119, HC CDR2 of SEQ ID NO: 132 and HC CDR3 of SEQ ID NO: 145 of CLL-1-3;
   (iv) a HC CDR1 of SEQ ID NO: 120, HC CDR2 of SEQ ID NO: 133 and HC CDR3 of SEQ ID NO: 146 of CLL-1-4;
   (v) a HC CDR1 of SEQ ID NO: 121, HC CDR2 of SEQ ID NO: 134 and HC CDR3 of SEQ ID NO: 147 of CLL-1-5;
   (vi) a HC CDR1 of SEQ ID NO: 122, HC CDR2 of SEQ ID NO: 135 and HC CDR3 of SEQ ID NO: 148 of CLL-1-6;
   (vii) a HC CDR1 of SEQ ID NO: 123, HC CDR2 of SEQ ID NO: 136 and HC CDR3 of SEQ ID NO: 149 of CLL-1-7;
   (viii) a HC CDR1 of SEQ ID NO: 124, HC CDR2 of SEQ ID NO: 137 and HC CDR3 of SEQ ID NO: 150 of CLL-1-8; or
   (ix) a HC CDR1 of SEQ ID NO: 125, HC CDR2 of SEQ ID NO: 138 and HC CDR3 of SEQ ID NO: 151 of CLL-1-9;
   (x) a HC CDR1 of SEQ ID NO: 126, HC CDR2 of SEQ ID NO: 139 and HC CDR3 of SEQ ID NO: 152 of CLL-1-10;
   (xi) a HC CDR1 of SEQ ID NO: 127, HC CDR2 of SEQ ID NO: 140 and HC CDR3 of SEQ ID NO: 153 of CLL-1-11;
   (xii) a HC CDR1 of SEQ ID NO: 128, HC CDR2 of SEQ ID NO: 141 and HC CDR3 of SEQ ID NO: 154 of CLL-1-12;
   (xiii) a HC CDR1 of SEQ ID NO: 129, HC CDR2 of SEQ ID NO: 142 and HC CDR3 of SEQ ID NO: 155 of CLL-1-13;
   (xiv) a HC CDR1 of SEQ ID NO: 199, HC CDR2 of SEQ ID NO: 200 and HC CDR3 of SEQ ID NO: 201 of 181286
   and is as defined in the claims.

In certain embodiments, the multispecific molecule, e.g., bispecific molecule, e.g., bispecific antibody or bispecific antibody-like molecule, described herein (*e.g.,* the multispecific molecule, e.g., bispecific molecule, e.g., bispecific antibody or bispecific antibody-like molecule, nucleic acid or a multispecific molecule, e.g., bispecific molecule, e.g., bispecific antibody or bispecific antibody-like molecule, polypeptide) or a CLL-1 binding domain includes a CLL-1 binding domain that includes:
(1) one, two, or three light chain (LC) CDRs chosen from one of the following:
   (i) a LC CDR1 of SEQ ID NO: 356, LC CDR2 of SEQ ID NO: 370 and LC CDR3 of SEQ ID NO: 384 of CLL-1-1;
   (ii) a LC CDR1 of SEQ ID NO: 357, LC CDR2 of SEQ ID NO: 371 and LC CDR3 of SEQ ID NO: 385 of CLL-1-2;
   (iii) a LC CDR1 of SEQ ID NO: 358, LC CDR2 of SEQ ID NO: 372 and LC CDR3 of SEQ ID NO: 386 of CLL-1-3;
   (iv) a LC CDR1 of SEQ ID NO: 359, LC CDR2 of SEQ ID NO: 373 and LC CDR3 of SEQ ID NO: 387 of CLL-1-4;
   (v) a LC CDR1 of SEQ ID NO: 360, LC CDR2 of SEQ ID NO: 374 and LC CDR3 of SEQ ID NO: 388 of CLL-1-5;
   (vi) a LC CDR1 of SEQ ID NO: 361, LC CDR2 of SEQ ID NO: 375 and LC CDR3 of SEQ ID NO: 389 of CLL-1-6;
   (vii) a LC CDR1 of SEQ ID NO: 362, LC CDR2 of SEQ ID NO: 376 and LC CDR3 of SEQ ID NO: 390 of CLL-1-7;
   (viii) a LC CDR1 of SEQ ID NO: 363, LC CDR2 of SEQ ID NO: 377 and LC CDR3 of SEQ ID NO: 391 of CLL-1-8; or
   (ix) a LC CDR1 of SEQ ID NO: 364, LC CDR2 of SEQ ID NO: 378 and LC CDR3 of SEQ ID NO: 392 of CLL-1-9;
   (x) a LC CDR1 of SEQ ID NO: 365, LC CDR2 of SEQ ID NO: 379 and LC CDR3 of SEQ ID NO: 393 of CLL-1-10;
   (xi) a LC CDR1 of SEQ ID NO: 366, LC CDR2 of SEQ ID NO: 380 and LC CDR3 of SEQ ID NO: 394 of CLL-1-11;
   (xii) a LC CDR1 of SEQ ID NO: 367, LC CDR2 of SEQ ID NO: 381 and LC CDR3 of SEQ ID NO: 395 of CLL-1-12;
   (xiii) a LC CDR1 of SEQ ID NO: 368, LC CDR2 of SEQ ID NO: 382 and LC CDR3 of SEQ ID NO: 396 of CLL-1-13;
   (xiv) a LC CDR1 of SEQ ID NO: 369, LC CDR2 of SEQ ID NO: 383 and LC CDR3 of SEQ ID NO: 397 of 181286; and/or
(2) one, two, or three heavy chain (HC) CDRs from one of the following:
   (i) a HC CDR1 of SEQ ID NO: 314, HC CDR2 of SEQ ID NO: 328 and HC CDR3 of SEQ ID NO: 342 of CLL-1-1;
   (ii) a HC CDR1 of SEQ ID NO: 315, HC CDR2 of SEQ ID NO: 329 and HC CDR3 of SEQ ID NO: 343 of CLL-1-2;
   (iii) a HC CDR1 of SEQ ID NO: 316, HC CDR2 of SEQ ID NO: 330 and HC CDR3 of SEQ ID NO: 344 of CLL-1-3;
   (iv) a HC CDR1 of SEQ ID NO: 317, HC CDR2 of SEQ ID NO: 331 and HC CDR3 of SEQ ID NO: 345 of CLL-1-4;
   (v) a HC CDR1 of SEQ ID NO: 318, HC CDR2 of SEQ ID NO: 332 and HC CDR3 of SEQ ID NO: 346 of CLL-1-5;
   (vi) a HC CDR1 of SEQ ID NO: 319, HC CDR2 of SEQ ID NO: 333 and HC CDR3 of SEQ ID NO: 347 of CLL-1-6;
   (vii) a HC CDR1 of SEQ ID NO: 320, HC CDR2 of SEQ ID NO: 334 and HC CDR3 of SEQ ID NO: 348 of CLL-1-7;
   (viii) a HC CDR1 of SEQ ID NO: 321, HC CDR2 of SEQ ID NO: 335 and HC CDR3 of SEQ ID NO: 349 of CLL-1-8; or
   (ix) a HC CDR1 of SEQ ID NO: 322, HC CDR2 of SEQ ID NO: 336 and HC CDR3 of SEQ ID NO: 350 of CLL-1-9;
   (x) a HC CDR1 of SEQ ID NO: 323, HC CDR2 of SEQ ID NO: 337 and HC CDR3 of SEQ ID NO: 351 of CLL-1-10;
   (xi) a HC CDR1 of SEQ ID NO: 324, HC CDR2 of SEQ ID NO: 338 and HC CDR3 of SEQ ID NO: 352 of CLL-1-11;
   (xii) a HC CDR1 of SEQ ID NO: 325, HC CDR2 of SEQ ID NO: 339 and HC CDR3 of SEQ ID NO: 353 of CLL-1-12;
   (xiii) a HC CDR1 of SEQ ID NO: 326, HC CDR2 of SEQ ID NO: 340 and HC CDR3 of SEQ ID NO: 354 of CLL-1-13;
   (xiv) a HC CDR1 of SEQ ID NO: 327, HC CDR2 of SEQ ID NO: 341 and HC CDR3 of SEQ ID NO: 355 of 181286
   and is as defined in the claims.

In certain embodiments, the multispecific molecule, e.g., bispecific molecule, e.g., bispecific antibody or bispecific antibody-like molecule, described herein (*e.g.,* the multispecific molecule, e.g., bispecific molecule, e.g., bispecific antibody or bispecific antibody-like molecule, nucleic acid or a multispecific molecule, e.g., bispecific molecule, e.g., bispecific antibody or bispecific antibody-like molecule, polypeptide) or a CLL-1 binding domain includes a CLL-1 binding domain that includes:
(1) one, two, or three light chain (LC) CDRs chosen from one of the following:
   (i) a LC CDR1 of SEQ ID NO: 440, LC CDR2 of SEQ ID NO: 454 and LC CDR3 of SEQ ID NO: 468 of CLL-1-1;
   (ii) a LC CDR1 of SEQ ID NO: 441, LC CDR2 of SEQ ID NO: 455 and LC CDR3 of SEQ ID NO: 469 of CLL-1-2;
   (iii) a LC CDR1 of SEQ ID NO: 442, LC CDR2 of SEQ ID NO: 456 and LC CDR3 of SEQ ID NO: 470 of CLL-1-3;
   (iv) a LC CDR1 of SEQ ID NO: 443, LC CDR2 of SEQ ID NO: 457 and LC CDR3 of SEQ ID NO: 471 of CLL-1-4;
   (v) a LC CDR1 of SEQ ID NO: 444, LC CDR2 of SEQ ID NO: 458 and LC CDR3 of SEQ ID NO: 472 of CLL-1-5;
   (vi) a LC CDR1 of SEQ ID NO: 445, LC CDR2 of SEQ ID NO: 459 and LC CDR3 of SEQ ID NO: 473 of CLL-1-6;
   (vii) a LC CDR1 of SEQ ID NO: 446, LC CDR2 of SEQ ID NO: 460 and LC CDR3 of SEQ ID NO: 474 of CLL-1-7;
   (viii) a LC CDR1 of SEQ ID NO: 447, LC CDR2 of SEQ ID NO: 461 and LC CDR3 of SEQ ID NO: 475 of CLL-1-8; or
   (ix) a LC CDR1 of SEQ ID NO: 448, LC CDR2 of SEQ ID NO: 462 and LC CDR3 of SEQ ID NO: 476 of CLL-1-9;
   (x) a LC CDR1 of SEQ ID NO: 449, LC CDR2 of SEQ ID NO: 463 and LC CDR3 of SEQ ID NO: 477 of CLL-1-10;
   (xi) a LC CDR1 of SEQ ID NO: 450, LC CDR2 of SEQ ID NO: 464 and LC CDR3 of SEQ ID NO: 478 of CLL-1-11;
   (xii) a LC CDR1 of SEQ ID NO: 451, LC CDR2 of SEQ ID NO: 465 and LC CDR3 of SEQ ID NO: 479 of CLL-1-12;
   (xiii) a LC CDR1 of SEQ ID NO: 452, LC CDR2 of SEQ ID NO: 466 and LC CDR3 of SEQ ID NO: 480 of CLL-1-13;
   (xiv) a LC CDR1 of SEQ ID NO: 453, LC CDR2 of SEQ ID NO: 467 and LC CDR3 of SEQ ID NO: 481 of 181286; and/or
(2) one, two, or three heavy chain (HC) CDRs from one of the following:
   (i) a HC CDR1 of SEQ ID NO: 398, HC CDR2 of SEQ ID NO: 412 and HC CDR3 of SEQ ID NO: 426 of CLL-1-1;
   (ii) a HC CDR1 of SEQ ID NO: 399, HC CDR2 of SEQ ID NO: 413 and HC CDR3 of SEQ ID NO: 427 of CLL-1-2;
   (iii) a HC CDR1 of SEQ ID NO: 400, HC CDR2 of SEQ ID NO: 414 and HC CDR3 of SEQ ID NO: 428 of CLL-1-3;
   (iv) a HC CDR1 of SEQ ID NO: 401, HC CDR2 of SEQ ID NO: 415 and HC CDR3 of SEQ ID NO: 429 of CLL-1-4;
   (v) a HC CDR1 of SEQ ID NO: 402, HC CDR2 of SEQ ID NO: 416 and HC CDR3 of SEQ ID NO: 430 of CLL-1-5;
   (vi) a HC CDR1 of SEQ ID NO: 403, HC CDR2 of SEQ ID NO: 417 and HC CDR3 of SEQ ID NO: 431 of CLL-1-6;
   (vii) a HC CDR1 of SEQ ID NO: 404, HC CDR2 of SEQ ID NO: 418 and HC CDR3 of SEQ ID NO: 432 of CLL-1-7;
   (viii) a HC CDR1 of SEQ ID NO: 405, HC CDR2 of SEQ ID NO: 419 and HC CDR3 of SEQ ID NO: 433 of CLL-1-8; or
   (ix) a HC CDR1 of SEQ ID NO: 406, HC CDR2 of SEQ ID NO: 420 and HC CDR3 of SEQ ID NO: 434 of CLL-1-9;
   (x) a HC CDR1 of SEQ ID NO: 407, HC CDR2 of SEQ ID NO: 421 and HC CDR3 of SEQ ID NO: 435 of CLL-1-10;
   (xi) a HC CDR1 of SEQ ID NO: 408, HC CDR2 of SEQ ID NO: 422 and HC CDR3 of SEQ ID NO: 436 of CLL-1-11;
   (xii) a HC CDR1 of SEQ ID NO: 409, HC CDR2 of SEQ ID NO: 423 and HC CDR3 of SEQ ID NO: 437 of CLL-1-12;
   (xiii) a HC CDR1 of SEQ ID NO: 410, HC CDR2 of SEQ ID NO: 424 and HC CDR3 of SEQ ID NO: 438 of CLL-1-13;
   (xiv) a HC CDR1 of SEQ ID NO: 411, HC CDR2 of SEQ ID NO: 425 and HC CDR3 of SEQ ID NO: 439 of 181286
   and is as defined in the claims.

Exemplary anti-CLL-1 scFvs include but are not limited to CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12 and CLL-1-13. The sequences of human anti-CLL-1 scFv fragments (SEQ ID NOS: 39-51), are provided in Table 2 (and the name designations are provided in Table 1).

In embodiments of the disclosed multispecific molecule, e.g., bispecific molecule, e.g., bispecific antibody or bispecific antibody-like molecule, constructs are generated using scFv fragments, e.g., the human scFv fragments (e.g., SEQ ID NOs: 39-51), in combination with additional sequences, such as those shown below.

It is noted that the scFv, VH and VL fragments described herein, e.g., in Table 2 or in SEQ ID NOS: 39-51, 65-77 or 78-90, without a leader sequence (e.g., without the amino acid sequence of SEQ ID NO: 1 or the nucleotide sequence of SEQ ID NO:12), are encompassed by the present invention. In other embodiments, scFv, VH and VL fragments described herein, e.g., in Table 2 or in SEQ ID NOS: 39-51, 65-77 or 78-90, with an optional leader sequence (e.g., without the amino acid sequence of SEQ ID NO: 1 or the nucleotide sequence of SEQ ID NO:12), are also encompassed by the present invention.
**Exemplary leader (amino acid sequence) (SEQ ID NO: 1)**
   MALPVTALLLPLALLLHAARP
**Exemplary leader (nucleic acid sequence) (SEQ ID NO: 12)**
**Gly/Ser (SEQ ID NO:25)**
   GGGGS
**Gly/Ser (SEQ ID NO:26): This sequence may encompass 1-6 "Gly Gly Gly Gly Ser" repeating units**
   GGGGSGGGGS GGGGSGGGGS GGGGSGGGGS
**Gly/Ser (SEQ ID NO:27)**
   GGGGSGGGGS GGGGSGGGGS
**Gly/Ser (SEQ ID NO:28)**
   GGGGSGGGGS GGGGS
**Gly/Ser (SEQ ID NO:29)**
   GGGS
**Gly/Ser (SEQ ID NO:38): This sequence may encompass 1-10 "Gly Gly Gly Ser" repeating units**
   GGGSGGGSGG GSGGGSGGGS GGGSGGGSGG GSGGGSGGGS

### II. Additional Antigen Binding Domains

In one aspect, the disclosure provides multispecific molecules comprising an anti-CLL-1 binding domain, e.g., as described herein, and a domain that binds one or more, e.g., a second, additional antigen(s) or epitope(s). Various additional antigens or epitopes are contemplated by the present disclosure, and are described more fully below. In one aspect, the additional antigen or epitope is a unique (e.g., not recognized by the first anti-CLL-1 binding domain) epitope on CLL-1. In one aspect of the disclosure, the additional antigen or epitope is an antigen of a target (e.g., a protein) other than CLL-1. In one aspect, the additional antigen or epitope is a cancer antigen or tumor antigen. In one aspect, the additional antigen or epitope is an antigen or epitope of an immune effector cell, e.g., a T cell or NK cell.

### a. Immune Effector Antigen Binding Domains

In one aspect, the present disclosure provides multispecific molecules comprising an anti-CLL-1 binding domain, e.g., as described herein, and an antigen binding domain that binds an antigen or epitope of an immune effector cell, e.g., a T cell or NK cell. As the term is used herein, an "immune effector cell" refers to a cell that is involved in an immune response, e.g., in the promotion of an immune effector response.

In one aspect the antigen or epitope of an immune effector cell is an epitope of a T cell. In one aspect the antigen is CD3.

Various anti-CD3 binding domains known in the art are suitable for use in a multispecific molecule, e.g., a bispecific antibody or antibody-like molecule, comprising an anti-CLL-1 binding domain described herein. Anti-CD3 binding domains that may be incorporated into the multispecific constructs of the present invention include those described in, for example, US2015/011825, WO2010/037835, WO2015/026894, WO2015/026892, US2014/0302064, US9029508, US2015/0118252, WO2014/051433 and WO2010/037835. Exemplary anti-CD3 binding domains are provided in Table 26.

**Table 26. Sequences of VL and VH domains of anti-CD3 binding domains.**

| **Bindin g Domai n** | **Chain** | **Sequence** | **SEQ ID NO:** |
|---|---|---|---|
| CD3-1 | VH | | 1200 |
| | VL | | 1201 |
| CD3-2 | VH | | 1202 |
| | VL | | 1203 |
| CD3-3 | VH | | 1204 |
| | VL | | 1205 |
| CD3-5 | VH | | 1206 |
| | VL | | 1207 |
| CD3-6 | VH | | 1208 |
| | VL | | 1209 |
| CD3-7 | VH | | 1210 |
| | VL | | 1211 |
| CD3-8 | VH | | 1212 |
| | VL | | 1213 |
| CD3-9 | VH | | 1214 |
| | VL | | 1215 |
| CD3-10 | VH | | 1216 |
| | VL | | 1217 |
| CD3-11 | VH | | 1218 |
| | VL | | 1219 |
| CD3-12 | VH | | 1220 |
| | VL | | 1221 |
| CD3-13 | VH | | 1222 |
| | VL | | 1223 |
| CD3-14 | VH | | 1224 |
| | VL | | 1225 |
| CD3-15 | VH | | 1226 |
| | VL | | 1227 |
| CD3-19 | VH | | 1228 |
| | VL | | 1229 |
| CD3-21 | VH | | 1230 |
| | VL | | 1231 |
| CD3-22 | VH | | 1232 |
| | VL | | 1233 |
| CD3-23 | VH | | 1234 |
| | VL | | 1235 |
| CD3-24 | VH | | 1236 |
| | VL | | 1237 |
| CD3-25 | VH | | 1236 |
| | VL | | 1209 |

**Table 27. HCDR and LCDR sequences of anti-CD3 binding domains (CDR1, CDR2 and CDR3 associated with a VH chain refer to HCDR1, HCDR2 and HCDR3, respectively (also referred to herein as HC CDR1, HC CDR2 and HC CDR3, respectively); CDR1, CDR2 and CDR3 associated with a VL chain refer to LCDR1, LCDR2 and LCDR3, respectively (also referred to herein as LC CDR1, LC CDR2 and LC CDR3, respectively)), according to the Kabat numbering scheme (Kabat et al. (1991), "Sequences of Proteins of Immunological Interest," 5th Ed. Public Health Service, National Institutes of Health, Bethesda, MD).**

| **Binding Domain** | **Chain** | **CDR1** | **SEQ ID NO:** | **CDR2** | **SEQ ID NO:** | **CDR3** | **SEQ ID NO:** |
|---|---|---|---|---|---|---|---|
| CD3-1 | VH | RYTMH | 1700 | YINPSRGYTNYNQKFKD | 1738 | YYDDHYCLDY | 1776 |
| | VL | SASSSVSYM N | 1701 | DTSKLAS | 1739 | QQWSSNPFT | 1777 |
| CD3-2 | VH | TYAMN | 1702 | | 1740 | | 1778 |
| | VL | RSSTGAVTTSN YAN | 1703 | GTNKRAP | 1741 | ALWYSNLWV | 1779 |
| CD3-3 | VH | SYTM H | 1704 | YINPSSGYTKYNQKFKD | 1742 | WQDYDVYFDY | 1780 |
| | VL | RASSSVSYM H | 1705 | ATSNLAS | 1743 | QQWSSNPPT | 1781 |
| CD3-5 | VH | RYTMH | 1706 | YINPSRGYTNYNQKFKD | 1744 | YYDDHYCLDY | 1782 |
| | VL | RASSSVSYM N | 1707 | DTSKVAS | 1745 | QQWSSNPLT | 1783 |
| CD3-6 | VH | RYTMH | 1708 | YINPSRGYTNYNQKVKD | 1746 | YYDDHYCLDY | 1784 |
| | VL | SASSSVSYM N | 1709 | DTSKLAS | 1747 | QQWSSNPFT | 1785 |
| CD3-7 | VH | GYGMH | 1710 | VIWYDGSKKYYVDSVKG | 1748 | QMGYWHFDL | 1786 |
| | VL | RASQSVSSYLA | 1711 | DASNRAT | 1749 | QQRSNWPPLT | 1787 |
| CD3-8 | VH | TYAMN | 1712 | RIRSKYNNYATYYAD | 1750 | | 1788 |
| | VL | | 1713 | GTNKRAP | 1751 | ALWYSNLWV | 1789 |
| CD3-9 | VH | RYTMH | 1714 | YINPSRGYTNYNQKFKD | 1752 | YYDDHYCLDY | 1790 |
| | VL | RASSSVSYM N | 1715 | DTSKVAS | 1753 | QQWSSNPLT | 1791 |
| CD3-10 | VH | TYAMN | 1716 | RIRSKYNNYATYYAD | 1754 | | 1792 |
| | VL | | 1717 | GTNKRAP | 1755 | ALWYSNLWV | 1793 |
| CD3-11 | VH | TYAMN | 1718 | | 1756 | | 1794 |
| | VL | | 1719 | GTNKRAP | 1757 | ALWYSNLWV | 1795 |
| CD3-12 | VH | SYAM N | 1720 | | 1758 | | 1796 |
| | VL | | 1721 | GTKFLAP | 1759 | VLWYSNRWV | 1797 |
| CD3-13 | VH | KYAM N | 1722 | | 1760 | HGNFGNSYISYWAY | 1798 |
| | VL | | 1723 | GTKFLAP | 1761 | VLWYSNRWV | 1799 |
| CD3-14 | VH | RYTM H | 1724 | YINPSRGYTNYNQKFKD | 1762 | YYDDHYCLDY | 1800 |
| | VL | SASSSVSYM N | 1725 | DTSKLAS | 1763 | QQWSSNPFT | 1801 |
| CD3-15 | VH | TYAMN | 1726 | | 1764 | | 1802 |
| | VL | | 1727 | GTNKRAP | 1765 | ALWYSNLWV | 1803 |
| CD3-19 | VH | TYAMN | 1728 | | 1766 | | 1804 |
| | VL | | 1729 | GTNKRAP | 1767 | ALWYSNLWV | 1805 |
| CD3-21 | VH | TYAMN | 1730 | | 1768 | | 1806 |
| | VL | | 1731 | GTNKRAP | 1769 | ALWYSNLWV | 1807 |
| CD3-22 | VH | TYAMN | 1732 | | 1770 | HGNFGDSYVSWFAY | 1808 |
| | VL | | 1733 | GTNKRAP | 1771 | ALWYSNHWV | 1809 |
| CD3-23 | VH | RYTM H | 1734 | YINPSRGYTNYNQKVKD | 1772 | YYDDHYCLDY | 1810 |
| | VL | SASSSVSYM N | 1735 | DTSKLAS | 1773 | QQWSSNPFT | 1811 |
| CD3-24 | VH | RYTM H | 1736 | YINPSRGYTNYNQKVKD | 1774 | YYDDHYSLDY | 1812 |
| | VL | SASSSVSYM N | 1737 | DTSKLAS | 1775 | QQWSSNPFT | 1813 |

**Table 28. HCDR and LCDR sequences of anti-CD3 binding domains (CDR1, CDR2 and CDR3 associated with a VH chain refer to HCDR1, HCDR2 and HCDR3, respectively (also referred to herein as HC CDR1, HC CDR2 and HC CDR3, respectively); CDR1, CDR2 and CDR3 associated with a VL chain refer to LCDR1, LCDR2 and LCDR3, respectively (also referred to herein as LC CDR1, LC CDR2 and LC CDR3, respectively)), according to the Chothia numbering scheme (Al-Lazikani et al., (1997) JMB 273,927-948).**

| Binding Domain | Chain | CDR1 | SEQ ID NO: | CDR2 | SEQ ID NO: | CDR3 | SEQ ID NO: |
|---|---|---|---|---|---|---|---|
| CD3-1 | VH | GYTFTRY | 1500 | NPSRGY | 1538 | YYDDHYCLDY | 1576 |
| | VL | SSSVSY | 1501 | DTS | 1539 | WSSNPF | 1577 |
| CD3-2 | VH | GFTFNTY | 1502 | RSKYNNYA | 1540 | HGNFGNSYVSWFAY | 1578 |
| | VL | STGAVTTSNY | 1503 | GTN | 1541 | WYSNLW | 1579 |
| CD3-3 | VH | GYTFTSY | 1504 | NPSSGY | 1542 | WQDYDVYFDY | 1580 |
| | VL | SSSVSY | 1505 | ATS | 1543 | WSSNPP | 1581 |
| CD3-5 | VH | GYTFTRY | 1506 | NPSRGY | 1544 | YYDDHYCLDY | 1582 |
| | VL | SSSVSY | 1507 | DTS | 1545 | WSSNPL | 1583 |
| CD3-6 | VH | GYTFTRY | 1508 | NPSRGY | 1546 | YYDDHYCLDY | 1584 |
| | VL | SSSVSY | 1509 | DTS | 1547 | WSSNPF | 1585 |
| CD3-7 | VH | GFKFSGY | 1510 | WYDGSK | 1548 | QMGYWHFDL | 1586 |
| | VL | SQSVSSY | 1511 | DAS | 1549 | RSNWPPL | 1587 |
| CD3-8 | VH | GFTFSTY | 1512 | RSKYNNYAT | 1550 | HGNFGNSYVSWFA | 1588 |
| | VL | STGAVTTSNY | 1513 | GTN | 1551 | WYSNLW | 1589 |
| CD3-9 | VH | GYTFTRY | 1514 | NPSRGY | 1552 | YYDDHYCLDY | 1590 |
| | VL | SSSVSY | 1515 | DTS | 1553 | WSSNPL | 1591 |
| CD3-10 | VH | GFTFNTY | 1516 | RSKYNNYAT | 1554 | HGNFGNSYVSWFA | 1592 |
| | VL | STGAVTTSNY | 1517 | GTN | 1555 | WYSNLW | 1593 |
| CD3-11 | VH | GFTFNTY | 1518 | RSKYNNYA | 1556 | HGNFGNSYVSWFAY | 1594 |
| | VL | STGAVTTSNY | 1519 | GTN | 1557 | WYSNLW | 1595 |
| CD3-12 | VH | GFTFNSY | 1520 | RSKYNNYA | 1558 | HGNFGNSYVSWWAY | 1596 |
| | VL | STGAVTSGNY | 1521 | GTK | 1559 | WYSNRW | 1597 |
| CD3-13 | VH | GFTFNKY | 1522 | RSKYNNYA | 1560 | HGNFGNSYISYWAY | 1598 |
| | VL | STGAVTSGNY | 1523 | GTK | 1561 | WYSNRW | 1599 |
| CD3-14 | VH | GYTFTRY | 1524 | NPSRGY | 1562 | YYDDHYCLDY | 1600 |
| | VL | SSSVSY | 1525 | DTS | 1563 | WSSNPF | 1601 |
| CD3-15 | VH | G FTFSTY | 1526 | RSKYNNYA | 1564 | HGNFGNSYVSWFAY | 1602 |
| | VL | STGAVTTSNY | 1527 | GTN | 1565 | WYSNLW | 1603 |
| CD3-19 | VH | GFTFNTY | 1528 | RSKYNNYA | 1566 | HGNFGNSYVSWFAY | 1604 |
| | VL | STGAVTTSNY | 1529 | GTN | 1567 | WYSNLW | 1605 |
| CD3-21 | VH | GFTFNTY | 1530 | RSKYNNYA | 1568 | HGNFGNSYVSWFAY | 1606 |
| | VL | STGAVTTSNY | 1531 | GTN | 1569 | WYSNLW | 1607 |
| CD3-22 | VH | G FTFSTY | 1532 | RSKYNNYA | 1570 | HGNFGDSYVSWFAY | 1608 |
| | VL | STGAVTTSNY | 1533 | GTN | 1571 | WYSNHW | 1609 |
| CD3-23 | VH | GYTFTRY | 1534 | NPSRGY | 1572 | YYDDHYCLDY | 1610 |
| | VL | SSSVSY | 1535 | DTS | 1573 | WSSNPF | 1611 |
| CD3-24 | VH | GYTFTRY | 1536 | NPSRGY | 1574 | YYDDHYSLDY | 1612 |
| | VL | SSSVSY | 1537 | DTS | 1575 | WSSNPF | 1613 |

**Table 29. HCDR and LCDR sequences of anti-CD3 binding domains (CDR1, CDR2 and CDR3 associated with a VH chain refer to HCDR1, HCDR2 and HCDR3, respectively (also referred to herein as HC CDR1, HC CDR2 and HC CDR3, respectively); CDR1, CDR2 and CDR3 associated with a VL chain refer to LCDR1, LCDR2 and LCDR3, respectively (also referred to herein as LC CDR1, LC CDR2 and LC CDR3, respectively)), according to a combination of the Kabat numbering scheme (Kabat et al. (1991), "Sequences of Proteins of Immunological Interest," 5th Ed. Public Health Service, National Institutes of Health, Bethesda, MD) and the Chothia numbering scheme (Al-Lazikani et al., (1997) JMB 273,927-948).**

| Binding Domain | Chain | CDR1 | SEQ ID NO: | CDR2 | SEQ ID NO: | CDR3 | SEQ ID NO: |
|---|---|---|---|---|---|---|---|
| CD3-1 | VH | GYTFTRYTM H | 1300 | YINPSRGYTNYNQKFKD | 1338 | YYDDHYCLDY | 1376 |
| | VL | SASSSVSYM N | 1301 | DTSKLAS | 1339 | QQWSSNPFT | 1377 |
| CD3-2 | VH | GFTFNTYAMN | 1302 | | 1340 | | 1378 |
| | VL | | 1303 | GTNKRAP | 1341 | ALWYSNLWV | 1379 |
| CD3-3 | VH | GYTFTSYTM H | 1304 | YINPSSGYTKYNQKFKD | 1342 | WQDYDVYFDY | 1380 |
| | VL | RASSSVSYM H | 1305 | ATSNLAS | 1343 | QQWSSNPPT | 1381 |
| CD3-5 | VH | GYTFTRYTM H | 1306 | YINPSRGYTNYNQKFKD | 1344 | YYDDHYCLDY | 1382 |
| | VL | RASSSVSYM N | 1307 | DTSKVAS | 1345 | QQWSSNPLT | 1383 |
| CD3-6 | VH | GYTFTRYTM H | 1308 | YINPSRGYTNYNQKVKD | 1346 | YYDDHYCLDY | 1384 |
| | VL | SASSSVSYM N | 1309 | DTSKLAS | 1347 | QQWSSNPFT | 1385 |
| CD3-7 | VH | GFKFSGYGMH | 1310 | VIWYDGSKKYYVDSVK G | 1348 | QMGYWHFDL | 1386 |
| | VL | RASQSVSSYLA | 1311 | DASNRAT | 1349 | QQRSNWPPLT | 1387 |
| CD3-8 | VH | GFTFSTYAMN | 1312 | | 1350 | | 1388 |
| | VL | | 1313 | GTNKRAP | 1351 | ALWYSNLWV | 1389 |
| CD3-9 | VH | GYTFTRYTM H | 1314 | YINPSRGYTNYNQKFKD | 1352 | YYDDHYCLDY | 1390 |
| | VL | RASSSVSYM N | 1315 | DTSKVAS | 1353 | QQWSSNPLT | 1391 |
| CD3-10 | VH | GFTFNTYAMN | 1316 | | 1354 | | 1392 |
| | VL | | 1317 | GTNKRAP | 1355 | ALWYSNLWV | 1393 |
| CD3-11 | VH | GFTFNTYAMN | 1318 | | 1356 | | 1394 |
| | VL | | 1319 | GTNKRAP | 1357 | ALWYSNLWV | 1395 |
| CD3-12 | VH | GFTFNSYAMN | 1320 | | 1358 | | 1396 |
| | VL | | 1321 | GTKFLAP | 1359 | VLWYSNRWV | 1397 |
| CD3-13 | VH | GFTFNKYAMN | 1322 | | 1360 | | 1398 |
| | VL | | 1323 | GTKFLAP | 1361 | VLWYSNRWV | 1399 |
| CD3-14 | VH | GYTFTRYTM H | 1324 | YINPSRGYTNYNQKFKD | 1362 | YYDDHYCLDY | 1400 |
| | VL | SASSSVSYM N | 1325 | DTSKLAS | 1363 | QQWSSNPFT | 1401 |
| CD3-15 | VH | G FTFSTYAM N | 1326 | | 1364 | | 1402 |
| | VL | | 1327 | GTNKRAP | 1365 | ALWYSNLWV | 1403 |
| CD3-19 | VH | GFTFNTYAMN | 1328 | | 1366 | | 1404 |
| | VL | | 1329 | GTNKRAP | 1367 | ALWYSNLWV | 1405 |
| CD3-21 | VH | GFTFNTYAMN | 1330 | | 1368 | | 1406 |
| | VL | | 1331 | GTNKRAP | 1369 | ALWYSNLWV | 1407 |
| CD3-22 | VH | G FTFSTYAM N | 1332 | | 1370 | | 1408 |
| | VL | | 1333 | GTNKRAP | 1371 | ALWYSNHWV | 1409 |
| CD3-23 | VH | GYTFTRYTM H | 1334 | YINPSRGYTNYNQKVKD | 1372 | YYDDHYCLDY | 1410 |
| | VL | SASSSVSYM N | 1335 | DTSKLAS | 1373 | QQWSSNPFT | 1411 |
| CD3-24 | VH | GYTFTRYTM H | 1336 | YINPSRGYTNYNQKVKD | 1374 | YYDDHYSLDY | 1412 |
| | VL | SASSSVSYM N | 1337 | DTSKLAS | 1375 | QQWSSNPFT | 1413 |

In one aspect of the disclosure, the anti-CD3 binding domain comprises the VL of SEQ ID NO: [1900] (QVQLQQSGAELARPGASVKMSCKASGYTFTRYTMHWVKQRPGQGLEWIGYINPSR GYTNYNQKFKDKATLTTDKSSSTAYMQLSSLTSEDSAVYYCARYYDDHYCLDYWG QGTTLTVSS) and the VH of SEQ ID NO: [1901] (QIVLTQSPAIMSASPGEKVTMTCSASSSVSYMNWYQQKSGTSPKRWIYDTSKLASG VPAHFRGSGSGTSYSLTISGMEAEDAATYYCQQWSSNPFTFGSGTKLEIN). In one aspect of the disclosure, the anti-CD3 binding domain comprises a VL having at least 75%, at least 80%, at least 85%, at least 90%, at least 95%, at least 96%, at least 97%, at least 98%, or at least 99% sequence identity to SEQ ID NO: [1900] and a VH having at least 75%, at least 80%, at least 85%, at least 90%, at least 95%, at least 96%, at least 97%, at least 98%, or at least 99% sequence identity to SEQ ID NO: [1901]. In one aspect of the disclosure, the anti-CD3 binding domain comprises a VL having at least 1, 2, 3, 4, 5, 6, 7, 8, 9, or 10, but no more than 50, 60, 70, 80, 90 or 100 amino acid substitutions relative to the amino acids of SEQ ID NO: [1900], and a VH having at least 1, 2, 3, 4, 5, 6, 7, 8, 9, or 10, but no more than 50, 60, 70, 80, 90 or 100 amino acid substitutions relative to the amino acids of SEQ ID NO: [1901].

In one aspect of the disclosure, the anti-CD3 binding domain comprises the VL of SEQ ID NO: [1902] (DIQLTQSPAIMSASPGEKVTMTCRASSSVSYMNWYQQKSGTSPKRWIYDTSKVASG VPYRFSGSGSGTSYSLISSMEAEDAATYYCQQWSSNPLTFGAGTKLELK) and the VH of SEQ ID NO: [1903] (DIKLQQSGAELARPGASVKMSCKTSGYTFTRYTMHWVKQRPGQGLEWIGYINPSRG YTNYNQKFKDKATLTTDKSSSTAYMQLSSLTSEDSAVYYCARYYDDHYCLDYWGQ GTTLTVSS). In one aspect of the disclosure, the anti-CD3 binding domain comprises a VL having at least 75%, at least 80%, at least 85%, at least 90%, at least 95%, at least 96%, at least 97%, at least 98%, or at least 99% sequence identity to SEQ ID NO: [1902] and a VH having at least 75%, at least 80%, at least 85%, at least 90%, at least 95%, at least 96%, at least 97%, at least 98%, or at least 99% sequence identity to SEQ ID NO: [1903]. In one aspect, the anti-CD3 binding domain comprises a VL having at least 1, 2, 3, 4, 5, 6, 7, 8, 9, or 10, but no more than 50, 60, 70, 80, 90 or 100 amino acid substitutions relative to the amino acids of SEQ ID NO: [1902], and a VH having at least 1, 2, 3, 4, 5, 6, 7, 8, 9, or 10, but no more than 50, 60, 70, 80, 90 or 100 amino acid substitutions relative to the amino acids of SEQ ID NO: [1903].

In one aspect of the disclosure, the anti-CD3 binding domain comprises the VL of SEQ ID NO: [1904] (QAVVTQESALTTSPGETVTLTCRSSTGAVTTSNYANWVQEKPDHLFTGLIGGTNKR APGVPARFSGSLIGDKAALTITGAQTEDEAIYFCALWYSNLWVFGGGTKLTVL) and the VH of SEQ ID NO: [1905] (EVQLVESGGGLVQPKGSLKLSCAASGFTFNTYAMNWVRQAPGKGLEWVARIRSKY NNYATYYADSVKDRFTISRDDSQSILYLQMNNLKTEDTAMYYCVRHGNFGNSYVS WFAYWGQGTLVTVSA). In one aspect of the disclosure, the anti-CD3 binding domain comprises a VL having at least 75%, at least 80%, at least 85%, at least 90%, at least 95%, at least 96%, at least 97%, at least 98%, or at least 99% sequence identity to SEQ ID NO: [1904] and a VH having at least 75%, at least 80%, at least 85%, at least 90%, at least 95%, at least 96%, at least 97%, at least 98%, or at least 99% sequence identity to SEQ ID NO: [1905]. In one aspect of the disclosure, the anti-CD3 binding domain comprises a VL having at least 1, 2, 3, 4, 5, 6, 7, 8, 9, or 10, but no more than 50, 60, 70, 80, 90 or 100 amino acid substitutions relative to the amino acids of SEQ ID NO: [1904], and a VH having at least 1, 2, 3, 4, 5, 6, 7, 8, 9, or 10, but no more than 50, 60, 70, 80, 90 or 100 amino acid substitutions relative to the amino acids of SEQ ID NO: [1905].

In one aspect of the disclosure, the anti-CD3 binding domain comprises the VL of SEQ ID NO: [1906] (QAVVTQEPSLTVSPGGTVTLTCRSSTGAVTTSNYANWVQQKPGQAPRGLIGGTNKR APWTPARFSGSLLGGKAALIGAQAEDEADYYCALWYSNLWVFGGGTKLTVL) and the VH of SEQ ID NO: [1907] (EVQLVESGGGLVQPGGSLRLSCAASGFTFSTYAMNWVRQAPGKGLEWVGRIRSKY NNYATYYADSVKDRFISRDDSKNSLYLQMNSLKTEDTAVYYCVRHGNFGNSYVSW FAYWGQGTLVTVSS). In one aspect of the disclosure, the anti-CD3 binding domain comprises a VL having at least 75%, at least 80%, at least 85%, at least 90%, at least 95%, at least 96%, at least 97%, at least 98%, or at least 99% sequence identity to SEQ ID NO: [1906] and a VH having at least 75%, at least 80%, at least 85%, at least 90%, at least 95%, at least 96%, at least 97%, at least 98%, or at least 99% sequence identity to SEQ ID NO: [1907]. In one aspect of the disclosure, the anti-CD3 binding domain comprises a VL having at least 1, 2, 3, 4, 5, 6, 7, 8, 9, or 10, but no more than 50, 60, 70, 80, 90 or 100 amino acid substitutions relative to the amino acids of SEQ ID NO: [1906], and a VH having at least 1, 2, 3, 4, 5, 6, 7, 8, 9, or 10, but no more than 50, 60, 70, 80, 90 or 100 amino acid substitutions relative to the amino acids of SEQ ID NO: [1907].

In one aspect of the disclosure, the anti-CD3 binding domain comprises the VL of SEQ ID NO: [1908] (QAVVTQEPSLTVSPGGTVTLTCRSSTGAVTTSNYANWVQQKPGQAPRGLIGGTNKR APWTPARFSGSLLGGKAALIGAQAEDEADYYCALWYSNLWVFGGGTKLTVL) and the VH of SEQ ID NO: [1909] (EVQLVESGGGLVQPGGSLRLSCAASGFTFNTYAMNWVRQAPGKGLEWVARIRSKY NNYATYYADSVKDRFISRDDSKNSLYLQMNSLKTEDTAVYYCVRHGNFGNSYVSW FAYWGQGTLVTVSS). In one aspect of the disclosure, the anti-CD3 binding domain comprises a VL having at least 75%, at least 80%, at least 85%, at least 90%, at least 95%, at least 96%, at least 97%, at least 98%, or at least 99% sequence identity to SEQ ID NO: [1908] and a VH having at least 75%, at least 80%, at least 85%, at least 90%, at least 95%, at least 96%, at least 97%, at least 98%, or at least 99% sequence identity to SEQ ID NO: [1909]. In one aspect of the disclsoure, the anti-CD3 binding domain comprises a VL having at least 1, 2, 3, 4, 5, 6, 7, 8, 9, or 10, but no more than 50, 60, 70, 80, 90 or 100 amino acid substitutions relative to the amino acids of SEQ ID NO: [1908], and a VH having at least 1, 2, 3, 4, 5, 6, 7, 8, 9, or 10, but no more than 50, 60, 70, 80, 90 or 100 amino acid substitutions relative to the amino acids of SEQ ID NO: [1909].

In one aspect of the disclosure, the anti-CD3 binding domain comprises the VL of SEQ ID NO: [1910] (DIQMTQSPSSLSASVGDRVTITCRASQSISSYLNWYQQKPGKAPKLLIYAASSLQSGV PSR FSGSGSGTDFTLTISSLQPEDFATYYCQQSYSTPPTFGQGTKVEIK) and the VH of SEQ ID NO: [1911] (QVQLVESGGGVVQPGRSLRLSCAASGFTFRSYGMHWVRQAPGKGLEWVAIIWYSG SK KNYADSVKGRFTISRDNSKNTLYLQMNSLRAEDTAVYYCARGTGYNWFDPWGQGT LV TVSS). In one aspect of the disclosure, the anti-CD3 binding domain comprises a VL having at least 75%, at least 80%, at least 85%, at least 90%, at least 95%, at least 96%, at least 97%, at least 98%, or at least 99% sequence identity to SEQ ID NO: [1910] and a VH having at least 75%, at least 80%, at least 85%, at least 90%, at least 95%, at least 96%, at least 97%, at least 98%, or at least 99% sequence identity to SEQ ID NO: [1911]. In one aspect of the disclosure, the anti-CD3 binding domain comprises a VL having at least 1, 2, 3, 4, 5, 6, 7, 8, 9, or 10, but no more than 50, 60, 70, 80, 90 or 100 amino acid substitutions relative to the amino acids of SEQ ID NO: [1910], and a VH having at least 1, 2, 3, 4, 5, 6, 7, 8, 9, or 10, but no more than 50, 60, 70, 80, 90 or 100 amino acid substitutions relative to the amino acids of SEQ ID NO: [1911].

Virtually any other T cell antigen or epitope is useful in the present disclosure. By way of example, the T cell antigen or epitope may be an immune costimulatory molecule (or epitope thereof). As the terms is used herein, an "immune costimulatory molecule" or "costimulatory molecule" (used interchangeably herein) refer to the cognate binding partner on a T cell that specifically binds with a costimulatory ligand, thereby mediating a costimulatory response by the T cell, such as, but not limited to, proliferation. Costimulatory molecules are cell surface molecules other than antigen receptors or their ligands that are required for an efficient immune response. Costimulatory molecules include, but are not limited to an MHC class I molecule, TNF receptor proteins, Immunoglobulin-like proteins, cytokine receptors, integrins, signalling lymphocytic activation molecules (SLAM proteins), activating NK cell receptors, BTLA, Toll ligand receptor, OX40, CD2, CD7, CD27, CD28, CD30, CD40, CD47, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), TLR7, LTBR, LAT, GADS, SLP-76, PAG/Cbp, CD19a, and a ligand that specifically binds with CD83.

By way of example, the T cell antigen or epitope may be an immune inhibitory molecule, e.g., a checkpoint protein. As the term is used herein, an "immune inhibitory molecule" refers to a molecule expressed on the surface of a cell that, when bound by its cognate binding partner, serves causes a reduction in an immune response. Examples of immune inhibitory (e.g., checkpoint) molecules include PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta.

In another aspect of the disclosure, the antigen or epitope of an immune effector cell is an epitope of a NK cell. In one aspect the antigen is CD16 (Fc Receptor gamma III). Any anti-CD 16 binding domain, including those known in the art, may be useful in the multispecific molecules of the present disclosure. In embodiments of the disclosure, the anti-CD16 binding domain comprises an anti-CD 16 binding domain described in, for example, WO2005/0089519 or US2015/0218275. In one aspect of the disclosure the antigen is CD64 (Fc Receptor gamma I). Any anti-CD64 binding domain, including those known in the art, may be useful in the multispecific molecules of the present disclosure. In embodiments of the disclosure, the anti-CD64 binding domain comprises an anti-CD64 binding domain described in, for example, WO2006/002438.

### b. Cancer Cell Antigen Binding Domains

In one aspect of the disclosure, the multispecific molecule, e.g., bispecific molecule, e.g., bispecific antibody or bispecific antibody-like molecule, is characterized by a first anti-CLL-1 binding domain, e.g., comprises a scFv as described herein, e.g., as described in Table 2, or comprises the light chain CDRs and/or heavy chain CDRs from a CLL-1 scFv described herein, and a second antigen binding domain that has binding specificity for a cancer antigen, e.g., a cancer antigen expressed on a cell that also expresses CLL-1. In some aspects of the disclosure the second antigen binding domain has specificity for an antigen expressed on AML cells, e.g., an antigen other than CLL-1. In some aspects of the disclosure the second antigen binding domain has binding specificity for an antigen expressed on multiple myeloma (MM) cells. The following examples in this paragraph are aspects of the disclosed multispecific molecules. For example, the second antigen binding domain has binding specificity for CD123. As another example, the second antigen binding domain has binding specificity for CD33. As another example, the second antigen binding domain has binding specificity for CD34. As another example, the second antigen binding domain has binding specificity for FLT3. For example, the second antigen binding domain has binding specificity for folate receptor beta. As another example, the second antigen binding domain has binding specificity for BCMA. In some aspects, the second antigen binding domain has binding specificity for an antigen expressed on B-cells, for example, CD10, CD19, CD20, CD22, CD34, CD123, FLT-3, ROR1, CD79b, CD179b, or CD79a.

### III. Formats for Multispecific Molecules

In some aspects a multispecific molecule is a bispecific antibody or bispecific antibody-like molecule. In some aspects, the bispecific antibody or antibody-like molecule may be multivalent, e.g., bivalent, with respect to one antigen and monovalent with respect to the other antigen. An exemplary bispecific antibody molecule or bispecific antibody-like molecule as disclosed herein is characterized by a first antigen binding domain (e.g., comprising a first VL disposed on a first polypeptide and a first VH disposed on a second polypeptide) which has binding specificity for a first antigen or epitope (e.g., CLL-1) and a second antigen binding domain (e.g., comprising a second VL disposed on a third polypeptide and a second VH disposed on a fourth polypeptide) that has binding specificity for a second epitope (e.g., an epitope of an immune effector cell or of a tumor or malignant cell, e.g., as described herein). In embodiments the first and second epitopes are on the same antigen, e.g., the same protein (or subunit of a multimeric protein). In embodiments the first and second epitopes overlap. In embodiments the first and second epitopes do not overlap. In embodiments the first and second epitopes are on different antigens, e.g., different proteins (or different subunits of a multimeric protein). In embodiments a bispecific antibody molecule or bispecific antibody-like molecule comprises a heavy chain variable domain sequence and a light chain variable domain sequence which have binding specificity for a first epitope or antigen, and a heavy chain variable domain sequence and a light chain variable domain sequence which have binding specificity for a second epitope or antigen. In embodiments a bispecific antibody molecule or antibody-like molecule comprises a half antibody having binding specificity for a first epitope or antigen, and a half antibody having binding specificity for a second epitope or antigen. In an embodiment a bispecific antibody molecule or antibody-like molecule comprises a half antibody, or fragment thereof, having binding specificity for a first epitope or antigen, and a half antibody, or fragment thereof, having binding specificity for a second epitope or antigen. In embodiments a bispecific antibody molecule or bispecific antibody-like molecule comprises a scFv, or fragment thereof, having binding specificity for a first epitope or antigen and a scFv, or fragment thereof, have binding specificity for a second epitope or antigen.

In certain embodiments, the antibody or antibody-like molecule is a multispecific (*e.g.,* a bispecific or a trispecific) antibody or antibody-like molecule. Protocols for generating bispecific or heterodimeric antibody or antibody-like molecules are known in the art; including but not limited to, for example, the "knob in a hole" approach described in, *e.g.,* US 5731168; the electrostatic steering Fc pairing as described in, *e.g.,* WO 09/089004, WO 06/106905 and WO 2010/129304; Strand Exchange Engineered Domains (SEED) heterodimer formation as described in, *e.g.,* WO 07/110205; Fab arm exchange as described in, *e.g.*, WO 08/119353, WO 2011/131746, and WO 2013/060867; double antibody conjugate, *e.g.*, by antibody cross-linking to generate a bi-specific structure using a heterobifunctional reagent having an amine-reactive group and a sulfhydryl reactive group as described in, *e.g.,* US 4433059; bispecific antibody or antibody-like molecule determinants generated by recombining half antibodies (heavy-light chain pairs or Fabs) from different antibodies or antibody-like molecules through cycle of reduction and oxidation of disulfide bonds between the two heavy chains, as described in, *e.g.,* US 4444878; trifunctional antibodies, *e.g*., three Fab' fragments cross-linked through sulfhdryl reactive groups, as described in, *e.g.,* US5273743; biosynthetic binding proteins, *e.g.,* pair of scFvs cross-linked through C-terminal tails preferably through disulfide or amine-reactive chemical cross-linking, as described in, *e.g.,* US5534254; bifunctional antibodies, *e.g.,* Fab fragments with different binding specificities dimerized through leucine zippers (*e.g.,* c-fos and c-jun) that have replaced the constant domain, as described in, *e.g*., US5582996; bispecific and oligospecific mono-and oligovalent receptors, *e.g.*, VH-CH1 regions of two antibodies (two Fab fragments) linked through a polypeptide spacer between the CH1 region of one antibody and the VH region of the other antibody typically with associated light chains, as described in, *e.g.,* US5591828; bispecific DNA-antibody conjugates, *e.g.,* crosslinking of antibodies or Fab fragments through a double stranded piece of DNA, as described in, *e.g.,* US5635602; bispecific fusion proteins, *e.g*., an expression construct containing two scFvs with a hydrophilic helical peptide linker between them and a full constant region, as described in, *e.g.,* US5637481; multivalent and multispecific binding proteins, *e.g.,* dimer of polypeptides having first domain with binding region of Ig heavy chain variable region, and second domain with binding region of Ig light chain variable region, generally termed diabodies (higher order structures are also encompassed creating for bispecific, trispecific, or tetraspecific molecules, as described in, *e.g.*, US5837242; minibody constructs with linked VL and VH chains further connected with peptide spacers to an antibody hinge region and CH3 region, which can be dimerized to form bispecific/multivalent molecules, as described in, *e.g.,* US5837821; VH and VL domains linked with a short peptide linker (*e.g.,* 5 or 10 amino acids) or no linker at all in either orientation, which can form dimers to form bispecific diabodies; trimers and tetramers, as described in, *e.g.,* US5844094; String of VH domains (or VL domains in family members) connected by peptide linkages with crosslinkable groups at the C-terminus further associated with VL domains to form a series of FVs (or scFvs), as described in, *e.g.,* US5864019; VL and VH domains, scFvs, or Fabs wherein one of the antigens is bound monovalently and one of the antigens is bound bivalently, optionally comprising heterodimeric Fc regions, as described in, e.g., WO2011/028952; and single chain binding polypeptides with both a VH and a VL domain linked through a peptide linker are combined into multivalent structures through non-covalent or chemical crosslinking to form, *e.g.,* homobivalent, heterobivalent, trivalent, and tetravalent structures using both scFv or diabody type format, as described in, *e.g.,* US5869620. Additional exemplary multispecific and bispecific molecules and methods of making the same are found, for example, in US5910573, US5932448, US5959083, US5989830, US6005079, US6239259, US6294353, US6333396, US6476198, US6511663, US6670453, US6743896, US6809185, US6833441, US7129330, US7183076, US7521056, US7527787, US7534866, US7612181, US2002004587A1, US2002076406A1, US2002103345A1, US2003207346A1, US2003211078A1, US2004219643A1, US2004220388A1, US2004242847A1, US2005003403A1, US2005004352A1, US2005069552A1, US2005079170A1, US2005100543A1, US2005136049A1, US2005136051A1, US2005163782A1, US2005266425A1, US2006083747A1, US2006120960A1, US2006204493A1, US2006263367A1, US2007004909A1, US2007087381A1, US2007128150A1, US2007141049A1, US2007154901A1, US2007274985A1, US2008050370A1, US2008069820A1, US2008152645A1, US2008171855A1, US2008241884A1, US2008254512A1, US2008260738A1, US2009130106A1, US2009148905A1, US2009155275A1, US2009162359A1, US2009162360A1, US2009175851A1, US2009175867A1, US2009232811A1, US2009234105A1, US2009263392A1, US2009274649A1, EP346087A2, WO0006605A2, WO02072635A2, WO04081051A1, WO06020258A2, WO2007044887A2, WO2007095338A2, WO2007137760A2, WO2008119353A1, WO2009021754A2, WO2009068630A1, WO9103493A1, WO9323537A1, WO9409131A1, WO9412625A2, WO9509917A1, WO9637621A2, WO9964460A1. Accordingly, in some embodiments, the anti-CLL-1 multispecific molecules of the present invention comprises an anti-CLL-1 binding domain in any one of the multispecific or bispecific formats known in the art and described above. Additional formats contemplated herein are described in more detail below.

Within each antibody or antibody fragment (e.g., scFv) of a bispecific antibody or antibody-like molecule, the VH can be upstream or downstream of the VL. In some embodiments, the upstream antibody or antibody fragment (e.g., scFv) is arranged with its VH (VHi) upstream of its VL (VL₁) and the downstream antibody or antibody fragment (e.g., scFv) is arranged with its VL (VL₂) upstream of its VH (VH₂), such that the overall bispecific antibody or antibody-like molecule has the arrangement VH₁-VL₁-VL₂-VH₂. In other embodiments, the upstream antibody or antibody fragment (e.g., scFv) is arranged with its VL (VL₁) upstream of its VH (VHi) and the downstream antibody or antibody fragment (e.g., scFv) is arranged with its VH (VH₂) upstream of its VL (VL₂), such that the overall bispecific antibody or antibody-like molecule has the arrangement VL₁-VH₁-VH₂-VL₂. Optionally, a linker is disposed between the two antibodies or antibody fragments (e.g., scFvs), e.g., between VL₁ and VL₂ if the construct is arranged as VH₁-VL₁-VL₂-VH₂, or between VH₁ and VH₂ if the construct is arranged as VL₁-VH₁-VH₂-VL₂. The linker may be a linker as described herein, e.g., a (Gly₄-Ser)n linker, wherein n is 1, 2, 3, 4, 5, or 6, preferably 4 (SEQ ID NO: 26). In general, the linker between the two scFvs should be long enough to avoid mispairing between the domains of the two scFvs. Optionally, a linker is disposed between the VL and VH of the first scFv. Optionally, a linker is disposed between the VL and VH of the second scFv. In constructs that have multiple linkers, any two or more of the linkers can be the same or different. Accordingly, in some embodiments of the disclosure, the anti-CLL-1 bispecific molecule of the present invention comprises VLs, VHs, (e.g., VLs and/or VHs from any of the anti-CLL-1 binding domains described herein) and optionally one or more linkers in an arrangement as described herein, together with, for example, VLs, VHs targeting a second epitope or antigen (e.g., a second epitope or antigen described herein, e.g., CD3 or CD16).

The present invention includes multispecific molecules, comprising an anti-CLL-1 binding domain, of various formats. Preferred formats for the multispecific molecules of the present invention are described in more detail below.

### Format 1: Dualbody

In one aspect, the present invention includes a multispecific molecule comprising a first heavy chain and a first light chain that together comprise a first antigen binding domain, and a second heavy chain and a second light chain that together comprise a second antigen binding domain. In an aspect, the dualbody is formed from a half antibody having specificity for a first antigen or epitope and a half antibody have specificity for a second antigen or epitope.

This molecule comprises a first polypeptide comprising a VL, and optionally a CL, and a second polypeptide comprising a VH, CH1, hinge, CH2 and CH3 domain, wherein the first and second polypeptide comprise a first antigen binding domain; and a third polypeptide comprising a VL, and optionally a CL, a fourth polypeptide comprising a VH, CHI, hinge, CH2 and CH3 domain, wherein the third and fourth polypeptides comprise a second antigen binding domain. In one embodiment the dualbody comprises (preferably from N-terminus to C-terminus; numbers following the domain name refer to, e.g., a first ("1" or "-1") or second ("2" or "-2") copy or version of that domain in the dualbody)):
Polypeptide 1: VL1-CL1
Polypeptide 2: VH1-CH1-1-hinge1-CH2-1-CH3-1
Polypeptide 3: VL2-CL2
Polypeptide 4: VH2-CH1-2-hinge2-CH2-2-CH3-2

In embodiments, the CL1 and CL2 domains are derived from the same antibody type or class e.g., comprise the same sequence. In other embodiments, the CL1 and CL2 domains are derived from different antibody or chain types or classes and/or comprise different sequences, for example CL1 may be derived from a lamba antibody and CL2 may be derived from a kappa antibody chain. Without being bound by theory, it is believed that deriving CL domains from different antibody types favors correct pairing of VL and VH domains (e.g., VH1 with VL1 and VH2 with VL2) to form functional antigen binding domains. In embodiments, the CH1-1 and CH1-2 domains are derived from the same antibody type or class and/or comprise the same sequence. In embodiments, the CH1-1 and CH1-2 domains are derived from different antibody types or classes and/or comprise different sequences. In embodiments, the hinge-1 and hinge-2 domains are derived from the same antibody type or class and/or comprise the same sequence. In embodiments, the hinge-1 and hinge-2 domains are derived from different antibody types or classes and/or comprise different sequences. In embodiments, the CH2-1 and CH2-2 domains are derived from the same antibody type or class and/or comprise the same sequence. In embodiments, the CH2-1 and CH2-2 domains are derived from different antibody types or classes and/or comprise different sequences. In some aspects, the CH2 domains that comprise different sequences comprise one or more mutations to favor heterodimerization of the two polypeptide chains comprising the CH2 domains, relative to unmodified polypeptide chains. Mutations for favoring heterodimerization are described in detail below. In embodiments, the CH2-1 and CH2-2 domains are derived from the same antibody type or class and/or comprise the same sequence. In embodiments, the CH3-1 and CH3-2 domains are derived from different antibody types or classes and/or comprise different sequences. In some aspects, the CH3 domains that comprise different sequences comprise one or more mutations to favor heterodimerization of the two polypeptide chains comprising the CH3 domains, relative to unmodified polypeptide chains. Mutations for favoring heterodimerization are described in detail below.

In embodiments, the polypeptides of the dualbody may additionally comprise one or more additional antibody fragments, e.g., one or more additional variable or constant domains, to form an "extended" dualbody. In one aspect, each polypeptide of the dualbody may additionally comprise a second variable domain (preferably of the same type as the first variable domain of the chain), preferably disposed between the first variable domain and the first constant domain of said polypeptide (if present), or C-terminal to the first variable domain. For example, the first polypeptide of the dualbody may comprise, from N-terminus to C-terminus: VL1-VL1-CL1. The one or more additional variable domains may be the same or different than the first variable domain. In other embodiments, the "extended" dualbody comprises one or more additional antibody constant domains, e.g., one or more copies or versions of the constant domain already present in the polypeptide chain being extended. For example, the first polypeptide of the dualbody may comprise, from N-terminus to C-terminus: VL1-CL1-CL1.

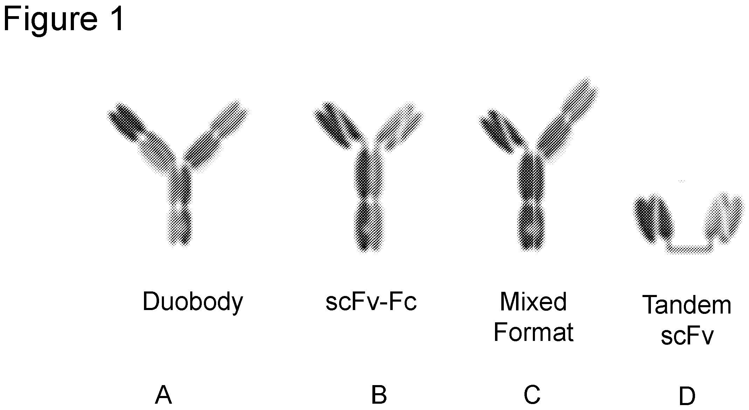

In embodiments, the dualbody has the structure depicted in Figure 1A. In embodiments, the dualbody comprises a first polypeptide chain having a VL and CL domains, a second polypeptide chain having a VH, CHI, hinge, CH2 and CH3 domains (said first and second chains making up the first half antibody that comprises the first antigen-binding domain, e.g., an anti-CLL-1 binding domain, e.g., as described herein), a third chain having a VL and CL domains, and a fourth chain having a VH, CHI, hinge, CH2 and CH3 domains (said third and fourth chains making up the second half antibody that comprises the second antigen-binding domain, e.g., an anti-CD3 binding domain, e.g., as described herein). Heterodimerization of the first and second half antibodies (shown here, favored by optional heterodimerization mutations to the CH3 domains, e.g., as described herein, as well as a stabilizing cysteine bond, e.g., as described herein) yields the multispecific molecule.

One or more chains of the multispecific molecules of the dualbody format described above may further comprise another antigen recognition domain, e.g., an scFv. In one embodiment, the scFv is disposed at the C-terminus of the chain. In one embodiment, the scFv is disposed C-terminal to the most C-terminal domain of a polypeptide comprising a heavy chain constant domain, e.g., a CH2 domain or CH3 domain. In one embodiment, the scFv is disposed C-terminal to the CH3 domain of a one or more polypeptides of the dualbody or extended dualbody. The one or more scFvs may recognize the same or different antigen or epitope as that recognized by either the first or second antigen binding domain of the dualbody or extended dualbody. Where the one or more antigen binding domains, e.g., scFvs, recognizes an epitope or antigen identical to one of the first or second antigen binding domains, the molecule is multivalent, e.g., bivalent, with respect to that antigen or epitope. In one embodiment of the disclosure, the antigen recognition domains of the dualbody recognize one or more cancer antigens, e.g., CLL-1, and the additional antigen binding domain, e.g., scFv, recognizes an antigen or epitope of an immune effector cell, e.g., a T cell or NK cell, e.g., CD3, CD64 or CD16.

It will be appreciated by the skilled artisan that the dualbody or extended dualbody molecules of the present invention may be stabilized by covalently linking one or more of the polypeptide chains of the molecule. Linkers are known in the art at may be chemical or peptidic. The linking may be along the polypeptide backbone (e.g., terminus to terminus), amino acid side chain to side chain, or amino acid side chain to terminus.

In embodiments, CL1 and CL2 may be independently selected from :
(a)
(b)
In a preferred embodiment, CL1 and CL2 are different (e.g., CL1 comprises SEQ ID NO: 502 and CL2 comprises SEQ ID NO: 503, or vice versa).

In embodiments, the heavy chain constant region of peptide 2 and peptide 4 comprise an amino acid sequence independently selected from:
(a)
(b)
In embodiments, peptide 2 (e.g., the heavy chain constant region of peptide 2) comprises SEQ ID NO: 504, and peptide 4 (e.g., the heavy chain constant region of peptide 4) comprises SEQ ID NO: 505, or vice versa.

Dualbody formats are known in the art, and are additionally described in, for example, WO2008/119353 and WO2011/131746. Examples of the dualbody format, including the "extended" dualbody format, are shown in Figure 1.

### Format 2: scFv-Fc

In one aspect, the present invention includes a multispecific molecule comprising a first polypeptide comprising a first scFv, a hinge domain, a CH2 domain and preferably, a CH3 domain; and a second polypeptide comprising a second scFv (preferably recognizing a different antigen or epitope than the first scFv), a hinge domain, a CH2 domain, and preferably, a CH3 domain. In one embodiment, the scFv is disposed N-terminal to the hinge and constant domains. In another embodiment, the scFv domains are disposed C-terminal to the hinge and constant domains. In another embodiment, the first scFv domain is disposed N-terminal to the hinge and constant domains of the first polypeptide, and the second scFv domain is disposed C-terminal to the hinge and constant domains of the second polypeptide. In embodiments, regardless of the orientation between the scFv domain and the constant domains, the hinge domain is disposed between the scFv domain and the constant domains.

In some aspects, the CH2 domains and/or CH3 domains of the scFv-Fc multispecific molecule comprise one or more mutations to favor heterodimerization of the two polypeptide chains comprising the CH2 and or CH3 domains of the scFv-Fc, relative to unmodified polypeptide chains. Mutations for favoring heterodimerization are described in detail below.

In embodiments, the dualbody has the structure depicted in Figure 1B. In embodiments, the scFv-Fc multispecific molecule comprises a first polypeptide chain comprising a scFv (e.g., the first antigen binding domain,., an anti-CLL-1 binding domain, as defined in the claims), a hinge, CH2 and CH3 domain (i.e., the first half antibody), and a second polypeptide chain consisting of a scFv (the second antigen binding domain, an anti-CD3 binding domain, as defined in the claims), a hinge, a CH2 and a CH3 domain (e.g., the second half antibody). Heterodimerization of the first and second half antibodies (shown here, favored by optional heterodimerization mutations to the CH3 domains, e.g., as described herein, as well as a stabilizing cysteine bond, e.g., as described herein) yields the multispecific molecule.

One or more chains of the multispecific molecules of the scFv-Fc format described above may further comprise another antigen recognition domain, e.g., an scFv. In one embodiment, the scFv is disposed at the C-terminus of the chain. In one embodiment, the scFv is disposed C-terminal to the most C-terminal domain of a polypeptide comprising a heavy chain constant domain, e.g., a CH2 domain or CH3 domain. In one embodiment, the scFv is disposed C-terminal to the CH3 domain of a one or more polypeptides of the scFv-Fc. The one or more scFvs may recognize the same or different antigen or epitope as that recognized by either the first or second antigen binding domain of the scFv-Fc. Where the one or more antigen binding domains, e.g., scFvs, recognizes an epitope or antigen identical to one of the first or second antigen binding domains, the molecule is multivalent, e.g., bivalent, with respect to that antigen or epitope. In one embodiment of the disclosure, the antigen recognition domains of the scFv-Fc recognize one or more cancer antigens, e.g., CLL-1, and the additional antigen binding domain, e.g., scFv, recognizes an antigen or epitope of an immune effector cell, e.g., a T cell or NK cell, e.g., CD3, CD64 or CD16.

It will be appreciated by the skilled artisan that the scFv-Fc molecules of the present invention may be stabilized by covalently linking one or more of the polypeptide chains of the molecule. Linkers are known in the art at may be chemical or peptidic. The linking may be along the polypeptide backbone (e.g., terminus to terminus), amino acid side chain to side chain, or amino acid side chain to terminus.

In embodiments, the first polypeptide chain may include a first antigen-binding domain, e.g., an scFv, and a first heavy chain constant region having the following sequence: and the second polypeptide may include a second antigen-binding domain, e.g., an scFv, and a second heavy chain constant region having the following sequence:

Each of the first and/or second polypeptides may comprise additional amino acid residues disposed between the antigen-binding domain and the constant region, e.g., a hinge. Alternatively, the amino acids of the antigen-binding domain may be directly connected to the amino acids of the constant domain (e.g., SEQ ID NO: 500 and/or SEQ ID NO: 501) without intervening amino acids.

Alternatively, the attachment of the constant domains may be reversed: first polypeptide may comprise SEQ ID NO: 501 and the second polypeptide may comprise SEQ ID NO: 500.

Examples of the scFv-Fc format are shown in Figure 1.

### Format 3: Mixed Chain Multispecific Molecules

In one aspect, the present invention includes a multispecific molecule comprising a first half antibody comprising a first polypeptide comprising a first scFv, a hinge domain, a CH2 domain and preferably, a CH3 domain; and a second half antibody comprising two polypeptides, the first comprising a VL domain and optionally, a CL domain, and the second comprising a VH domain, a CH1 domain, a hinge domain, and a CH2 domain and/or a CH3 domain. Thus, the mixed chain multispecific molecular format includes a half antibody derived from a typical antibody structure heterodimerized with a half antibody of the scFv-Fc format, described above.

In embodiments, the mixed format multispecific molecule has the structure depicted in Figure 1C. In embodiments, the mixed format multispecific molecule comprises a first half antibody comprising a first polypeptide chain having a VL and CL domains and a second polypeptide chain having a VH, CH1, hinge, CH2 and CH3 domains (said VL and VH domains of the first and second polypeptide chains, respectively, making up the first antigen binding domain, e.g., an anti-CLL-1 binding domain, e.g., as described herein), and a second half antibody comprising a third polypeptide chain having a scFv (comprising the second antigen binding domain, e.g., an anti-CD3 binding domain, e.g., as described herein), hinge, CH2 and CH3 domain. Heterodimerization of the first and second half antibodies (shown here, favored by optional heterodimerization mutations to the CH3 domains, e.g., as described herein, as well as a stabilizing cysteine bond, e.g., as described herein) yields the multispecific molecule.

In some aspects, the CH2 domains and/or CH3 domains of the mixed chain multispecific molecule comprise one or more mutations to favor heterodimerization of the two polypeptide chains comprising the CH2 and or CH3 domains of the mixed chain multispecific molecule, relative to unmodified polypeptide chains. Mutations for favoring heterodimerization are described in detail below.

One or more chains of the multispecific molecules of the mixed chain multispecific format described above may further comprise another antigen recognition domain, e.g., an scFv. In one embodiment, the scFv is disposed at the C-terminus of the chain. In one embodiment, the scFv is disposed C-terminal to the most C-terminal domain of a polypeptide comprising a heavy chain constant domain, e.g., a CH2 domain or CH3 domain. In one embodiment, the scFv is disposed C-terminal to the CH3 domain of a one or more polypeptides of the mixed chain multispecific molecule. The one or more scFvs may recognize the same or different antigen or epitope as that recognized by either the first or second antigen binding domain of the mixed chain multispecific molecule. Where the one or more antigen binding domains, e.g., scFvs, recognizes an epitope or antigen identical to one of the first or second antigen binding domains, the molecule is multivalent, e.g., bivalent, with respect to that antigen or epitope. In one embodiment of the disclosure, the antigen recognition domains of the mixed chain multispecific molecule recognize one or more cancer antigens, e.g., CLL-1, and the additional antigen binding domain, e.g., scFv, recognizes an antigen or epitope of an immune effector cell, e.g., a T cell or NK cell, e.g., CD3, CD64 or CD16.

It will be appreciated by the skilled artisan that the mixed chain multispecific molecules of the present invention may be stabilized by covalently linking one or more of the polypeptide chains of the molecule. Linkers are known in the art at may be chemical or peptidic. The linking may be along the polypeptide backbone (e.g., terminus to terminus), amino acid side chain to side chain, or amino acid side chain to terminus.

In embodiments, either half of the mixed format molecule may comprise one or more of the constant region sequences described above in the sections on scFv-Fc or dualbodies. In a preferred embodiment, the half of the mixed format molecule comprising the scFv comprises a constant region sequence described above in the section on scFv-Fc, and the half of the mixed format molecule comprising separate light chain and heavy chain polypeptides comprises constant regions sequences described above in the section on dualbodies.

Examples of the mixed chain multispecific molecule format are shown in Figure 1.

### Format 4: Tandem scFv

In one aspect, the present invention includes a multispecific molecule comprising single polypeptide comprising two or more scFv domains, linked by a suitable linker. By way of example, the polypeptide of the tandem scFv comprises, from N-terminus to C-terminus: VL1-linker-VH1-linker2-VH2-linker3-VL2. By way of example, the polypeptide of the tandem scFv comprises, from N-terminus to C-terminus: VL1-linker-VH1-linker2-VL2-linker3-VH2. The linkers may be the same or different.

The scFv domains of the tandem scFv may additionally be separated by a molecule which adds improved stability to the construct, for example, a human serum albumin protein or fragment thereof.

In embodiments, the multispecific Tandem scFv has the structure depicted in Figure 1D. In embodiments, the tandem scFv multispecific molecule comprises a first scFv (comprising the first antigen-binding domain, an anti-CLL-1 binding domain, as defined in the claims) and a second scFv (comprising the second antigen-binding domain, an anti-CD3 binding domain, as defined in the claims), connected by a linker.

### Examples of CD3 x CLL-1 Multispecific (bispecific) Antibodies

The antibodies in the table below are examples of CD3 x CLL-1 bispecific antibodies. These bispecific antibodies were generated in a scFv-Fc format, with a CD3 binding arm in the scFv-Fc format pairing with a CLL-1 binding arm in scFv-Fc format via knob and hole mutations to favor heterodimerization of the Fc domains.

**Table 11. Examplary CD3 x CLL-1 bispecific antibodies. For each of the CLL-1xCD3 bispecific molecules, the recited CLL-1 scFv-Fc was paired with the anti-CD3-Fc (SEQ ID NO: 507) to form the bispecific molecule.**

| **Bispecific Antibody Chain** | **SEQ ID NO:** | |
|---|---|---|
| **CD3 Binding Domain, and Exemplary scFv-Fc Chain** | | |
| CD3 VL | 1209 | |
| CD3 VH | 1236 | |
| CD3 scFv | 506 | |
| CD3 scFv-Fc | 507 | |
| CD3 VL DNA | 508 | |
| CD3 VH DNA | 509 | |
| | | |
| CD3 scFv DNA | 510 | |
| CD3 scFv-Fc DNA | 511 | |

| **CD3 x CLL1_11** | | |
|---|---|---|
| CLL1 _11 VL | 88 | |
| CLL1 _11 VH | 75 | |
| CLL1_11 scFv | 49 | |
| CLL1_11 scFv-Fc | 512 | |
| CLL1_11 VL DNA | 513 | |
| CLL1_11 VH DNA | 514 | |
| CLL1_11 scFv DNA | 515 | |
| CLL1_11 scFv-Fc DNA | 516 | |
| | | |

| **CD3 x CLL1_12** | | |
|---|---|---|
| CLL1_ 12 VL | 89 | |
| CLL1_12 VH | 76 | |
| CLL1_12 scFv | 50 | |
| CLL1scFv-Fc | 517 | |
| CLL1_12 VL DNA | 518 | |
| CLL1_12 VH DNA | 519 | |
| CLL1_12 scFv DNA | 520 | |
| | | |
| CLL1_12 scFv-Fc DNA | 521 | |

| **CD3 x CLL1_10** | | |
|---|---|---|
| CLL1 _10 VL | 87 | |
| CLL1 _10 VH | 74 | |
| CLL1_10 scFv | 48 | |
| CLL1_10 scFv-Fc | 522 | |
| | | |
| CLL1_10 VL DNA | 523 | |
| CLL1_10 VH DNA | 524 | |
| CLL1_10 scFv DNA | 525 | |
| CLL1_10 scFv-Fc DNA | 526 | |
| | | |

| **CD3 x CLL1_08** | | |
|---|---|---|
| CLL1_ 8 VL | 85 | |
| CLL1_ 8 VH | 72 | |
| CLL1_8 scFv | 46 | |
| CLL1_8 scFv-Fc | 527 | |
| CLL1_8 VL DNA | 528 | |
| CLL1_8 VH DNA | 529 | |
| CLL1_8 scFv DNA | 531 | |
| CLL1_8 scFv-Fc DNA | 531 | |

| **CD3 x CLL1_09** | | |
|---|---|---|
| CLL1 9 VL | 86 | |
| CLL1 9 VH | 73 | |
| CLL1_9 scFv | 47 | |
| CLL1_9 scFv-Fc | 532 | |
| CLL1_9 VL DNA | 533 | |
| | | |
| CLL1 9 VH DNA | 534 | |
| CLL1_9 scFv DNA | 535 | |
| CLL1_9 scFv-Fc DNA | 536 | |

| **CD3 x CLL1_06** | | |
|---|---|---|
| CLL1_6 VL | 83 | |
| CLL1_6 VH | 70 | |
| | | |
| CLL1_6 scFv | 44 | |
| CLL1_6 scFv-Fc | 537 | |
| CLL1_6 VL DNA | 538 | |
| CLL1_6 VH DNA | 539 | |
| CLL1_6 scFv DNA | 540 | |
| CLL1_6 scFv-Fc DNA | 541 | |
| | | |

| **CD3 x CLL1_13** | | |
|---|---|---|
| CLL1_13 VL | 90 | |
| CLL1_13 VH | 77 | |
| CLL1_13 scFv | 51 | |
| CLL1_13 scFv-Fc | 542 | |
| CLL1_13 VL DNA | 543 | |
| CLL1_13 VH DNA | 544 | |
| CLL1_13 scFv DNA | 545 | |
| CLL1_13 scFv-Fc DNA | 546 | |

| **CD3 x CLL1_07** | | |
|---|---|---|
| CLL1_7 VL | 84 | |
| CLL1_7 VH | 71 | |
| CLL1_7 scFv | 45 | |
| | | |
| CLL1_7 scFv-Fc | 547 | |
| CLL1_7 VL DNA | 548 | |
| CLL1_7 VH DNA | 549 | |
| CLL1_7 scFv DNA | 550 | |
| CLL1_7 scFv-Fc DNA | 551 | |
| | | |

| **CD3 x CLL1_02** | | |
|---|---|---|
| CLL1_2 VL | 79 | |
| CLL1_2 VH | 66 | |
| CLL1_2 scFv | 40 | |
| CLL1_2 scFv-Fc | 552 | |
| CLL1_2 VL DNA | 553 | |
| CLL1_2 VH DNA | 554 | |
| CLL1_2 scFv DNA | 555 | |
| | | |
| CLL1_2 scFv-Fc DNA | 556 | |

In aspects, the invention provides a multispecific molecule, e.g., a bispecific molecule, comprising an anti-CD3 binding domain comprising a VL sequence of SEQ ID NO: 1209 and a VH sequence of SEQ ID NO: 1236, and an anti-CLL-1 binding domain comprising:
a) a VL sequence of SEQ ID NO: 88 and a VH sequence of SEQ ID NO: 75;
b) a VL sequence of SEQ ID NO: 89 and a VH sequence of SEQ ID NO: 76;
c) a VL sequence of SEQ ID NO: 87 and a VH sequence of SEQ ID NO: 74;
d) a VL sequence of SEQ ID NO: 85 and a VH sequence of SEQ ID NO: 72;
e) a VL sequence of SEQ ID NO: 86 and a VH sequence of SEQ ID NO: 73;
f) a VL sequence of SEQ ID NO: 83 and a VH sequence of SEQ ID NO: 70;
g) a VL sequence of SEQ ID NO: 90 and a VH sequence of SEQ ID NO: 77;
h) a VL sequence of SEQ ID NO: 79 and a VH sequence of SEQ ID NO: 66; or
i) a VL sequence of SEQ ID NO: 84 and a VH sequence of SEQ ID NO: 71.

In aspects, the invention provides a multispecific molecule, e.g., a bispecific molecule, comprising an anti-CD3 binding domain comprising SEQ ID NO: 506, and an anti-CLL-1 binding domain comprising:
a) SEQ ID NO: 49;
b) SEQ ID NO: 50;
c) SEQ ID NO: 48;
d) SEQ ID NO: 46;
e) SEQ ID NO: 47;
f) SEQ ID NO: 44;
g) SEQ ID NO: 51;
h) SEQ ID NO: 40; or
i) SEQ ID NO: 45.

In aspects, the invention provides a multispecific molecule, e.g., a bispecific molecule (e.g., a bispecific molecule having a scFv-Fc format), comprising a first polypeptide comprising an anti-CD3 binding domain, wherein said first polypeptide chain comprises, e.g., consists of, SEQ ID NO: 507, and a second polypeptide comprising an anti-CLL-1 binding domain, wherein said second polypeptide chain comprises, e.g., consists of:
a) SEQ ID NO: 512;
b) SEQ ID NO: 517;
c) SEQ ID NO: 522;
d) SEQ ID NO: 527;
e) SEQ ID NO: 532;
f) SEQ ID NO: 537;
g) SEQ ID NO: 542;
h) SEQ ID NO: 547; or
i) SEQ ID NO: 552.

### IV. Binding Specificity

The molecules of the present invention can be multivalent multispecific, multivalent monospecific, monovalent multispecific, or monovalent monospecific.

In one embodiment, the molecule is a bivalent molecule (e.g., a bivalent antibody or antibody-like molecule). In one particular aspect, the present molecule has dual binding specificities if the first antigen binding domain and second antigen binding domain recognize two different antigens or two different epitopes on the same antigen. In another particular aspect, if the first and second antigen binding domains bind to the same antigen/epitope, the molecule is a mono-specific molecule. Incorporation of additional antigen binding domains, e.g., by incorporation of one or more additional scFv antigen binding domains may form a multispecific molecule when the one or more additional scFv antigen binding domains recognize a different antigen/epitope than the first and second antigen binding domains.

Standard assays to evaluate the binding specificity of the antibodies or antibody-like molecules toward various epitopes and/or antigens are known in the art, including for example, Biacore analysis, or FACS relative affinity (Scatchard), ELISAs, western blots and RIAs. Suitable assays are described in detail in the Definition and Examples.

### V. Physical Property/Yield

In certain embodiments, the present molecule offers desirable physical properties, such as a thermo-stability substantially same as or increased relative to that of natural antibodies.

Thermostability refers to protein stability during heat stress, which is an ability of a protein to retain the characteristic property when heated moderately. When exposed to heat, proteins will experience denaturing/unfolding process and will expose hydrophobic residues. Each protein is completely unfolded in response to heat at a characteristic temperature. The temperature at the mid-point of the protein unfolding process is defined as Tm, which is an important physical characteristic for a protein, and can be measured with the techniques known in the art. A multispecific molecule having a relatively high value of Tm is usually desirable because a high value of Tm often indicates less aggregation when it is used for preparing a pharmaceutical composition. In addition, higher Tm may also result in higher expression and yield.

In one particular embodiment, the multispecific molecule of the present invention has substantially same Tm as compared to that of an IgG antibody.

In yet another embodiment, the present disclosure includes a method of generating a multispecific molecule having substantially the same thermostability as a reference antibody comprising 1) designing a molecule of one of the formats described herein; 2) producing the molecule in a host cell; and 3) measuring and comparing Tm of the molecule and the reference antibody.

Cell culture systems have been widely used for expressing antibody fragments, but there have been few attempts to express and recover functional completely assembled full-length antibodies in high yield. Because of the complex structure and large size of completely assembled full-length antibodies or antibody-like molecules, it is often difficult to achieve proper folding and assembly of the expressed heavy and light chains, especially in bacterial cells. This problem is especially challenging when producing multispecific molecules. Because of the random pairing of two different antibody heavy and light chains within the host cells, only small percentage of the assembled antibody or antibody-like species is the desired, functional multispecific molecules. Due to the presence of mispaired byproducts, and significantly reduced production yields, sophisticated purification procedures are required (see e.g. Morrison, S. L., Nature Biotech 25 (2007) 1233-1234).

In some embodiments, the present molecules (e.g. antibodies or antibody-like molecules), when recombinantly produced in comparable cell cultures, have substantially same yield as when producing a reference antibody. In particular, under the same culture condition, being expressed by the same type of host cells, the molecules have substantially the same expression levels as a reference antibody. The expression levels of the produced molecule can be measured with the standard techniques in the art, such as, scanning densitometry of SDS-PAGE gels and/or immunoblots and the AME5-RP assay. Antibody or antibody-like molecule yield can also be quantified by protein A sensor chip using Qctec Red (Fotrte Bio).

The present disclosure includes a method of generating a multispecific molecule and having substantially same yield as production of a reference antibody comprising 1) designing a molecule of the present invention described herein; 2) producing the molecule in a host cell; and 3) measuring and comparing the expression level of said molecule with said reference antibody.

### VI. Modification of the Molecules of the Present Invention

### I.Molecules with Enhanced Heterodimerization

Inadequate heterodimerization of two antibody heavy chain domains has always been an obstacle for increasing the yield of desired multispecific molecules and represents challenges for purification. A variety of approaches available in the art can be used in for enhancing dimerization of the two heavy chain domains of bispecific or multispecific antibody or antibody-like molecules, as disclosed in EP 1870459A1; U.S. Pat. No. 5,582,996; U.S. Pat. No. 5,731,168; U.S. Pat. No. 5,910,573; U.S. Pat. No. 5,932,448; U.S. Pat. No. 6,833,441; U.S. Pat. No. 7,183,076; U.S. Patent Application Publication No. 2006204493A1; and PCT Publication No. WO2009/089004A1.

The present disclosure provides methods of enhancing dimerization (hetero-dimerization) of two interacting heterologous polypeptides and/or reducing dimerization (homo-dimerization) of two identical polypeptides. Typically, each of the two interacting polypeptides comprises a CH3 domain of an antibody. The CH3 domains are derived from the constant region of an antibody of any isotype, class or subclass, and preferably of IgG (IgG1, IgG2, IgG3 and IgG4) class.

Typically, the polypeptides comprise other antibody fragments in addition to CH3 domains, such as, CH1 domains, CH2 domains, hinge domain, VH domain(s), VL domain(s), CDR(s), and/or antigen-binding fragments described herein. These antibody fragments are derived from various types of antibodies described herein, for example, polyclonal antibody, monoclonal antibodies, chimeric antibodies, humanized antibodies, human antibodies, bispecific or multispecific antibodies, camelised antibodies, anti-idiotypic (anti-Id) antibodies and antibody conjugates. In some embodiments, the two hetero-polypeptides are two heavy chains forming a bispecific or multispecific molecules. Heterodimerzation of the two different heavy chains at CH3 domains give rise to the desired antibody or antibody-like molecule, while homodimerization of identical heavy chains will reduce yield of the desired antibody or molecule. In an exemplary embodiment, the two or more hetero-polypeptide chains comprise two chains comprising CH3 domains and forming the molecules of any of the multispecific molecule Formats described above of the present invention. In an embodiment, the two hetero-polypeptide chains comprising CH3 domains comprise modifications that favor heterodimeric association of the polypeptides, relative to unmodified chains. Various examples of modification strategies are provided below.

### Knob-in-Hole (KIH)

Multispecific molecules, e.g., multispecific antibody or antibody-like molecules, of the present invention may comprise one or more, e.g., a plurality, of mutations to one or more of the constant domains, e.g., to the CH3 domains. In one example, the multispecific molecule of the present invention comprises two polypeptides that each comprise a heavy chain constant domain of an antibody, e.g., a CH2 or CH3 domain. In an example, the two heavy chain constant domains, e.g., the CH2 or CH3 domains of the multispecific molecule comprise one or more mutations that allow for a heterodimeric association between the two chains. In one aspect, the one or more mutations are disposed on the CH2 domain of the two heavy chains of the multispecific, e.g., bispecific, antibody or antibody-like molecule. In one aspect, the one or more mutations are disposed on the CH3 domains of at least two polypeptides of the multispecific molecule. In one aspect, the one or more mutations to a first polypeptide of the multispecific molecule comprising a heavy chain constant domain creates a "knob" and the one or more mutations to a second polypeptide of the multispecific molecule comprising a heavy chain constant domain creates a "hole," such that heterodimerization of the polypeptide of the multispecific molecule comprising a heavy chain constant domain causes the "knob" to interface (e.g., interact, e.g., a CH2 domain of a first polypeptide interacting with a CH2 domain of a second polypeptide, or a CH3 domain of a first polypeptide interacting with a CH3 domain of a second polypeptide) with the "hole." As the term is used herein, a "knob" refers to at least one amino acid side chain which projects from the interface of a first polypeptide of the multispecific molecule comprising a heavy chain constant domain and is therefore positionable in a compensatory "hole" in the interface with a second polypeptide of the multispecific molecule comprising a heavy chain constant domain so as to stabilize the heteromultimer, and thereby favor heteromultimer formation over homomultimer formation, for example. The knob may exist in the original interface or may be introduced synthetically (e.g. by altering nucleic acid encoding the interface). The preferred import residues for the formation of a knob are generally naturally occurring amino acid residues and are preferably selected from arginine (R), phenylalanine (F), tyrosine (Y) and tryptophan (W). Most preferred are tryptophan and tyrosine. In the preferred embodiment, the original residue for the formation of the protuberance has a small side chain volume, such as alanine, asparagine, aspartic acid, glycine, serine, threonine or valine.

A "hole" refers to at least one amino acid side chain which is recessed from the interface of a second polypeptide of the multispecific molecule comprising a heavy chain constant domain and therefore accommodates a corresponding knob on the adjacent interfacing surface of a first polypeptide of the multispecific molecule comprising a heavy chain constant domain. The hole may exist in the original interface or may be introduced synthetically (e.g. by altering nucleic acid encoding the interface). The preferred import residues for the formation of a hole are usually naturally occurring amino acid residues and are preferably selected from alanine (A), serine (S), threonine (T) and valine (V). Most preferred are serine, alanine or threonine. In the preferred embodiment, the original residue for the formation of the hole has a large side chain volume, such as tyrosine, arginine, phenylalanine or tryptophan.

In a preferred embodiment, a first CH3 domain is mutated at residue 366, 405 or 407 according to the EU numbering scheme of Kabat et al. (pp. 688-696 in Sequences of proteins of immunological interest, 5th ed., Vol. 1 (1991; NIH, Bethesda, Md.)) to create either a "knob" or a hole" (as described above), and the second CH3 domain that heterodimerizes with the first CH3 domain is mutated at: residue 407 if residue 366 is mutated in the first CH3 domain, residue 394 if residue 405 is mutated in the first CH3 domain, or residue 366 if residue 407 is mutated in the first CH3 domain, according to the EU numbering scheme of Kabat et al. (pp. 688-696 in Sequences of proteins of immunological interest, 5th ed., Vol. 1 (1991; NIH, Bethesda, Md.)), to create a "hole" or "knob" complementary to the "knob" or "hole" of the first CH3 domain.

In another preferred embodiment, a first CH3 domain is mutated at residue 366 according to the EU numbering scheme of Kabat et al. (pp. 688-696 in Sequences of proteins of immunological interest, 5th ed., Vol. 1 (1991; NIH, Bethesda, Md.)) to create either a "knob" or a hole" (as described above), and the second CH3 domain that heterodimerizes with the first CH3 domain is mutated at residues 366, 368 and/or 407, according to the EU numbering scheme of Kabat et al. (pp. 688-696 in Sequences of proteins of immunological interest, 5th ed., Vol. 1 (1991; NIH, Bethesda, Md.)), to create a "hole" or "knob" complementary to the "knob" or "hole" of the first CH3 domain. In one embodiment, the mutation to the first CH3 domain introduces a tyrosine (Y) residue at position 366. In an embodiment, the mutation to the first CH3 is T366Y. In one embodiment, the mutation to the first CH3 domain introduces a tryptophan (W) residue at position 366. In an embodiment, the mutation to the first CH3 is T366W. In embodiments, the mutation to the second CH3 domain that heterodimerizes with the first CH3 domain mutated at position 366 (e.g., has a tyrosine (Y) or tryptophan (W) introduced at position 366, e.g., comprises the mutation T366Y or T366W), comprises a mutation at position 366, a mutation at position 368 and a mutation at position 407, according to the EU numbering scheme of Kabat et al. (pp. 688-696 in Sequences of proteins of immunological interest, 5th ed., Vol. 1 (1991; NIH, Bethesda, Md.)) In embodiments, the mutation at position 366 introduces a serine (S) residue, the mutation at position 368 introduces an alanine (A), and the mutation at position 407 introduces a valine (V). In embodiments, the mutations comprise T366S, L368A and Y407V. In one embodiment the first CH3 domain of the multispecific molecule comprises the mutation T366Y, and the second CH3 domain that heterodimerizes with the first CH3 domain comprises the mutations T366S, L368A and Y407V, or vice versa. In one embodiment the first CH3 domain of the multispecific molecule comprises the mutation T366W, and the second CH3 domain that heterodimerizes with the first CH3 domain comprises the mutations T366S, L368A and Y407V, or vice versa.

Additional knob in hole mutation pairs suitable for use in any of the multispecific molecules of the present invention are further described in, for example, WO1996/027011, and Merchant et al., Nat. Biotechnol., 16:677-681 (1998).

In any of the embodiments described herein, the CH3 domains may be additionally mutated to introduce a pair of cysteine residues. Without being bound by theory, it is believed that the introduction of a pair of cysteine residues capable of forming a disulfide bond provide stability to the heterodimerized multispecific molecule. In embodiments, the first CH3 domain comprises a cysteine at position 354, according to the EU numbering scheme of Kabat et al. (pp. 688-696 in Sequences of proteins of immunological interest, 5th ed., Vol. 1 (1991; NIH, Bethesda, Md.)), and the second CH3 domain that heterodimerizes with the first CH3 domain comprises a cysteine at position 349, according to the EU numbering scheme of Kabat et al. (pp. 688-696 in Sequences of proteins of immunological interest, 5th ed., Vol. 1 (1991; NIH, Bethesda, Md.)) In embodiments, the first CH3 domain of the multispecific molecule comprises a cysteine at position 354 (e.g., comprises the mutation S354C) and a tyrosine (Y) at position 366 (e.g., comprises the mutation T366Y), and the second CH3 domain that heterodimerizes with the first CH3 domain comprises a cysteine at position 349 (e.g., comprises the mutation Y349C), a serine at position 366 (e.g., comprises the mutation T366S), an alanine at position 368 (e.g., comprises the mutation L368A), and a valine at position 407 (e.g., comprises the mutation Y407V). In embodiments, the first CH3 domain of the multispecific molecule comprises a cysteine at position 354 (e.g., comprises the mutation S354C) and a tryptophan (W) at position 366 (e.g., comprises the mutation T366W), and the second CH3 domain that heterodimerizes with the first CH3 domain comprises a cysteine at position 349 (e.g., comprises the mutation Y349C), a serine at position 366 (e.g., comprises the mutation T366S), an alanine at position 368 (e.g., comprises the mutation L368A), and a valine at position 407 (e.g., comprises the mutation Y407V).

### IgG Heterodimerization

In one aspect, heterodimerization of the polypeptide chains (e.g., of the half antibodies) of the multispecific molecule is increased by introducing one or more mutations in a CH3 domain which is derived from the IgG1 antibody class. In an embodiment, the mutations comprise a K409R mutation to one CH3 domain paired with F405L mutation in the second CH3 domain, according to the EU numbering scheme of Kabat et al. (pp. 688-696 in Sequences of proteins of immunological interest, 5th ed., Vol. 1 (1991; NIH, Bethesda, Md.)). Additional mutations may also, or alternatively, be at positions 366, 368, 370, 399, 405, 407, and 409 according to the EU numbering scheme of Kabat et al. (pp. 688-696 in Sequences of proteins of immunological interest, 5th ed., Vol. 1 (1991; NIH, Bethesda, Md.)). Preferably, heterodimerization of polypeptides comprising such mutations is achieved under reducing conditions, e.g., 10-100 mM 2-MEA (e.g., 25, 50, or 100 mM 2-MEA) for 1-10, e.g., 1.5-5, e.g., 5, hours at 25-37C, e.g., 25C or 37C.

The amino acid replacements described herein are introduced into the CH3 domains using techniques which are well known in the art. Normally the DNA encoding the heavy chain(s) is genetically engineered using the techniques described in Mutagenesis: a Practical Approach. Oligonucleotide-mediated mutagenesis is a preferred method for preparing substitution variants of the DNA encoding the two hybrid heavy chains. This technique is well known in the art as described by Adelman et al., (1983) DNA, 2:183.

The IgG heterodimerization strategy is described in, for example, WO2008/119353, WO2011/131746, and WO2013/060867.

In any of the embodiments described herein, the CH3 domains may be additionally mutated to introduce a pair of cysteine residues. Without being bound by theory, it is believed that the introduction of a pair of cysteine residues capable of forming a disulfide bond provide stability to the heterodimerized multispecific molecule. In embodiments, the first CH3 domain comprises a cysteine at position 354, according to the EU numbering scheme of Kabat et al. (pp. 688-696 in Sequences of proteins of immunological interest, 5th ed., Vol. 1 (1991; NIH, Bethesda, Md.)), and the second CH3 domain that heterodimerizes with the first CH3 domain comprises a cysteine at position 349, according to the EU numbering scheme of Kabat et al. (pp. 688-696 in Sequences of proteins of immunological interest, 5th ed., Vol. 1 (1991; NIH, Bethesda, Md.))

### Polar Bridge

In one aspect, heterodimerization of the polypeptide chains (e.g., of the half antibodies) of the multispecific molecule is increased by introducing mutations based on the "polar-bridging" rational, which is to make residues at the binding interface of the two polypeptide chains to interact with residues of similar (or complimentary) physical property in the heterodimer configuration, while with residues of different physical property in the homodimer configuration. In particular, these mutations are designed so that, in the heterodimer formation, polar residues interact with polar residues, while hydrophobic residues interact with hydrophobic residues. In contrast, in the homodimer formation, residues are mutated so that polar residues interact with hydrophobic residues. The favorable interactions in the heterodimer configuration and the unfavorable interactions in the homodimer configuration work together to make it more likely for CH3 domains to form heterodimers than to form homodimers.

In an exemplary embodiment, the above mutations are generated at one or more positions of residues 364, 368, 399, 405, 409, and 411 of CH3 domain, amino acid numbering according to the EU numbering scheme of Kabat et al. (pp. 688-696 in Sequences of proteins of immunological interest, 5th ed., Vol. 1 (1991; NIH, Bethesda, Md.)).

In one aspect, one or more mutations selected from a group consisting of: Ser364Leu, Thr366Val, Leu368Gln, Asp399Lys, Phe405Ser, Lys409Phe and Thr411Lys are introduced into one of the two CH3 domains. (Ser364Leu: original residue of serine at position 364 is replaced by leucine; Thr366Val: original residue of threonine at position 366 is replaced by valine; Leu368Gln: original residue of leucine at position 368 is replaced by glutamine; Asp399Lys: original residue aspartic acid at position 399 is replaced by lysine; Phe405Ser: original residue phenylalanine at position 405 is replaced by serine; Lys409Phe: original residue lysine at position 409 is replaced by phenylalanine; Thr411Lys: original residue of threonine at position 411 is replaced by lysine.).

In another aspect, the other CH3 can be introduced with one or more mutations selected from a group consisting of: Tyr407Phe, Lys409Gln and Thr411Asp (Tyr407Phe: original residue tyrosine at position 407 is replaced by phenyalanine; Lys409Glu: original residue lysine at position 409 is replaced by glutamic acid; Thr411Asp: original residue of threonine at position 411 is replaced by aspartic acid).

In a further aspect, one CH3 domain has one or more mutations selected from a group consisting of: Ser364Leu, Thr366Val, Leu368Gln, Asp399Lys, Phe405Ser, Lys409Phe and Thr411Lys, while the other CH3 domain has one or more mutations selected from a group consisting of: Tyr407Phe, Lys409Gln and Thr411Asp.

In one exemplary embodiment, the original residue of threonine at position 366 of one CH3 domain is replaced by valine, while the original residue of tyrosine at position 407 of the other CH3 domain is replaced by phenylalanine.

In another exemplary embodiment, the original residue of serine at position 364 of one CH3 domain is replaced by leucine, while the original residue of leucine at position 368 of the same CH3 domain is replaced by glutamine.

In yet another exemplary embodiment, the original residue of phenylalanine at position 405 of one CH3 domain is replaced by serine and the original residue of lysine at position 409 of this CH3 domain is replaced by phenylalanine, while the original residue of lysine at position 409 of the other CH3 domain is replaced by glutamine.

In yet another exemplary embodiment, the original residue of aspartic acid at position 399 of one CH3 domain is replaced by lysine, and the original residue of threonine at position 411 of the same CH3 domain is replaced by lysine, while the original residue of threonine at position 411 of the other CH3 domain is replaced by aspartic acid.

The amino acid replacements described herein are introduced into the CH3 domains using techniques which are well known in the art. Normally the DNA encoding the heavy chain(s) is genetically engineered using the techniques described in Mutagenesis: a Practical Approach. Oligonucleotide-mediated mutagenesis is a preferred method for preparing substitution variants of the DNA encoding the two hybrid heavy chains. This technique is well known in the art as described by Adelman et al., (1983) DNA, 2:183.

The polar bridge strategy is described in, for example, WO2006/106905, WO2009/089004 and K.Gunasekaran, et al. (2010) The Journal of Biological Chemistry, 285:19637-19646.

In any of the embodiments described herein, the CH3 domains may be additionally mutated to introduce a pair of cysteine residues. Without being bound by theory, it is believed that the introduction of a pair of cysteine residues capable of forming a disulfide bond provide stability to the heterodimerized multispecific molecule. In embodiments, the first CH3 domain comprises a cysteine at position 354, according to the EU numbering scheme of Kabat et al. (pp. 688-696 in Sequences of proteins of immunological interest, 5th ed., Vol. 1 (1991; NIH, Bethesda, Md.)), and the second CH3 domain that heterodimerizes with the first CH3 domain comprises a cysteine at position 349, according to the EU numbering scheme of Kabat et al. (pp. 688-696 in Sequences of proteins of immunological interest, 5th ed., Vol. 1 (1991; NIH, Bethesda, Md.))

### II. Molecules With Variable Region Modifications

Each of the N-terminal VH and VL domains and C-terminal VH and VL domains of the molecule (e.g. antibody or antibody-like molecule) of the present invention comprises hypervariable regions CDR1, CDR2, and CDR3 sequences. In certain embodiments, one or more of these CDR sequences have conservative modifications of the amino acid sequences, and wherein the modified molecules retain or have enhanced binding properties as compared to the parent antibodies.

In addition, it has been found that in certain instances it is beneficial to mutate residues within the framework regions to maintain or enhance the antigen binding ability of the antibody (see *e.g.,* U.S. Patent Nos. 5,530,101; 5,585,089; 5,693,762 and 6,180,370 to Queen et al). The molecules (e.g. antibodies or antibody-like molecules) of the present invention can be modified by introducing such mutations to its variable region frameworks in order to improve the binding properties.

Another type of variable region modification is to mutate amino acid residues within the VH and/or VL CDR1, CDR2 and/or CDR3 domains to thereby improve one or more binding properties (*e.g*., affinity) of the molecule (e.g. antibody or antibody-like molecule) of interest, known as "affinity maturation." Site-directed mutagenesis or PCR-mediated mutagenesis can be performed to introduce the mutation(s) and the effect on antibody binding, or other functional property of interest, can be evaluated in *in vitro* or *in vivo* assays as described herein and provided in the Examples. Conservative modifications (as discussed above) can be introduced. The mutations may be amino acid substitutions, additions or deletions. Moreover, typically no more than one, two, three, four or five residues within a CDR region are altered.

Amino acid sequence variants of the present molecules can be prepared by introducing appropriate nucleotide changes into the encoding DNAs, or by synthesis of the desired variants. Such variants include, for example, deletions from, or insertions or substitutions of, residues within the amino acid sequences of present molecules. Any combination of deletion, insertion, and substitution is made to arrive at the final construct, provided that the final construct possesses the desired antigen-binding characteristics. The amino acid changes also may alter post-translational processes of the molecules, such as changing the number or position of glycosylation sites.

The present application includes variants of the molecules described herein and/or fragments thereof having amino acid conservative modifications in variable regions and/or constant regions.

### III. Molecules with an Extended in vivo Half-life.

The present molecule can be further modified to have an extended half-life *in vivo.*

A variety of strategies can be used to extend the half life of the molecules of the present invention. For example, by chemical linkage to polyethyleneglycol (PEG), reCODE PEG, antibody scaffold, polysialic acid (PSA), hydroxyethyl starch (HES), albumin-binding ligands, and carbohydrate shields; by genetic fusion to proteins binding to serum proteins, such as albumin, IgG, FcRn, and transferring; by coupling (genetically or chemically) to other binding moieties that bind to serum proteins, such as nanobodies, Fabs, DARPins, avimers, affibodies, and anticalins; by genetic fusion to rPEG, albumin, domain of albumin, albumin-binding proteins, and Fc; or by incorporation into nanocarriers, slow release formulations, or medical devices.

The molecules of the present invention having an increased half-life *in vivo* can also be generated introducing one or more amino acid modifications (*i.e.,* substitutions, insertions or deletions) into an IgG constant domain, or FcRn binding fragment thereof (preferably a Fc or hinge Fc domain fragment). See, e.g., International Publication No. WO 98/23289; International Publication No. WO 97/34631; and U.S. Patent No. 6,277,375.

Further, the molecules can be conjugated to albumin in order to make the molecules more stable *in vivo* or have a longer half life in *vivo.* The techniques are well-known in the art, see, *e.g.,* International Publication Nos. WO 93/15199, WO 93/15200, and WO 01/77137; and European Patent No. EP 413,622.

The molecules of the present invention may also be fused to one or more human serum albumin (HSA) polypeptides, or a portion thereof. The use of albumin as a component of an albumin fusion protein as a carrier for various proteins has been suggested in WO 93/15199, WO 93/15200, and EP 413 622. The use of N-terminal fragments of HSA for fusions to polypeptides has also been proposed (EP 399 666). Accordingly, by genetically or chemically fusing or conjugating the molecules to albumin, can stabilize or extend the shelf-life, and/or to retain the molecule's activity for extended periods of time in solution, in vitro and/or in vivo. Additional methods pertaining to HSA fusions can be found, for example, in WO 2001077137 and WO 200306007. In a specific embodiment, the expression of the fusion protein is performed in mammalian cell lines, for example, CHO cell lines.

### IV. Fc Silencing

Without being bound by theory, in embodiments that incorporate one or more constant domains, e.g., heavy chain constant regions, it may be beneficial to include one or mutations to silence, e.g., ADCC and/or CDC effector function within hFc. Activation of the immune cell occurs preferentially in the presence of crosslinking to the target cell. However, human Fc may bind to high and low affinity FcR gamma receptors. Therefore, crosslinking of receptors (e.g. CD3) on the immune cell and subsequent agonism may occur upon binding in the absence of tumor targeting. Additionally, crosslinking of Fc via gamma receptors may induce antibody dependent cellular cytotoxicity (ADCC). Human Fc when complexed at the cell surface can also bind complement proteins and induce complement dependent cytotoxicity (CDC). Mutations to residues in Fc which reduce or abrogate these interactions may thus limit these effects and focus the impact of the molecules described herein upon the tumor target cell.. In embodiments, one or more, e.g., all, of the heavy chain constant region domains of the multispecific molecule, e.g., bispecific molecule, e.g., bispecific antibody or antibody-like molecule comprise the DAPA mutation (e.g. D265A and P329A in EU numbering). See e.g., Shields RL, Namenuk AK, Hong K, Meng YG, Rae J, Briggs J, Xie D, Lai J, Stadlen A, Li B, Fox JA, Presta LG. High resolution mapping of the binding site on human IgG1 for Fc gamma RI, Fc gamma RII, Fc gamma RIII, and FcRn and design of IgG1 variants with improved binding to the Fc gamma R. J Biol Chem. 2001;276(9):6591-604; U.S. Patent Publication US2015/0320880 A1. In embodiments, one or more, e.g., all, of the heavy chain constant region domains of the multispecific molecule, e.g., bispecific molecule, e.g., bispecific antibody or antibody-like molecule comprise the LALA mutation (e.g., L234A and L235A in EU numbering). E.g., Hezareh M, Hessell AJ, Jensen RC, van de Winkel JGJ, Parren PWHI. Effector Function Activities of a Panel of Mutants of a Broadly Neutralizing Antibody against Human Immunodeficiency Virus Type 1. Journal of Virology. 2001;75(24):12161-12168; Shields RL, Namenuk AK, Hong K, Meng YG, Rae J, Briggs J, Xie D, Lai J, Stadlen A, Li B, Fox JA, Presta LG. High resolution mapping of the binding site on human IgG1 for Fc gamma RI, Fc gamma RII, Fc gamma RIII, and FcRn and design of IgG1 variants with improved binding to the Fc gamma R. J Biol Chem. 2001;276(9):6591-604. In embodiments, one or more, e.g., all, of the heavy chain constant region domains of the multispecific molecule, e.g., bispecific molecule, e.g., bispecific antibody or antibody-like molecule comprise an N279A mutation (according to EU numbering). E.g., Tao MH(1), Morrison SL. Studies of aglycosylated chimeric mouse-human IgG. Role of carbohydrate in the structure and effector functions mediated by the human IgG constant region. J Immunol. 1989; 143(8):2595-601; Shields RL, Namenuk AK, Hong K, Meng YG, Rae J, Briggs J, Xie D, Lai J, Stadlen A, Li B, Fox JA, Presta LG. High resolution mapping of the binding site on human IgG1 for Fc gamma RI, Fc gamma RII, Fc gamma RIII, and FcRn and design of IgG1 variants with improved binding to the Fc gamma R. J Biol Chem. 2001;276(9):6591-604.

### V. Conjugates

The present invention includes multispecific molecules (e.g. antibodies or antibody-like molecules) or the fragments thereof recombinantly fused or chemically conjugated (including both covalent and non-covalent conjugations) to a heterologous protein or polypeptide (or fragment thereof, preferably to a polypeptide of at least 10, at least 20, at least 30, at least 40, at least 50, at least 60, at least 70, at least 80, at least 90 or at least 100 amino acids) to generate fusion proteins. Methods for fusing or conjugating proteins, polypeptides, or peptides to an antibody or an antibody fragment are known in the art. See, *e.g.,* U.S. Patent Nos. 5,336,603, 5,622,929, 5,359,046, 5,349,053, 5,447,851, and 5,112,946; European Patent Nos. EP 307,434 and EP 367,166; International Publication Nos. WO 96/04388 and WO 91/06570; Ashkenazi et al., (1991) Proc. Natl. Acad. Sci. USA 88:10535-10539; Zheng et al., (1995) J. Immunol. 154:5590-5600; and Vil et al., (1992) Proc. Natl. Acad. Sci. USA 89:11337- 11341.

Additional fusion proteins may be generated through the techniques of gene-shuffling, motif-shuffling, exon-shuffling, and/or codon-shuffling (collectively referred to as "DNA shuffling"). DNA shuffling may be employed to alter the activities of molecules of the invention or fragments thereof (*e.g*., molecules or fragments thereof with higher affinities and lower dissociation rates). See, generally, U.S. Patent Nos. 5,605,793, 5,811,238, 5,830,721, 5,834,252, and 5,837,458; Patten et al., (1997) Curr. Opinion Biotechnol. 8:724-33; Harayama, (1998) Trends Biotechnol. 16(2):76-82; Hansson et al., (1999) J. Mol. Biol. 287:265-76; and Lorenzo and Blasco, (1998) Biotechniques 24(2):308- 313. The molecules described herein or fragments thereof may be altered by being subjected to random mutagenesis by error-prone PCR, random nucleotide insertion or other methods prior to recombination. A polynucleotide encoding a fragment of the present molecule may be recombined with one or more components, motifs, sections, parts, domains, fragments, etc. of one or more heterologous molecules.

Moreover, the present molecules or fragments thereof can be fused to marker sequences, such as a peptide to facilitate purification. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., (1989) Proc. Natl. Acad. Sci. USA 86:821-824, for instance, hexa-histidine provides for convenient purification of the fusion protein. Other peptide tags useful for purification include, but are not limited to, the hemagglutinin ("HA") tag, which corresponds to an epitope derived from the influenza hemagglutinin protein (Wilson et al., (1984) Cell 37:767), and the "flag" tag.

In other embodiments, the molecules of the present invention or fragments thereof are conjugated to a diagnostic or detectable agent. Such molecules can be useful for monitoring or prognosing the onset, development, progression and/or severity of a disease or disorder as part of a clinical testing procedure, such as determining the efficacy of a particular therapy. Such diagnosis and detection can accomplished by coupling the molecules to detectable substances including, but not limited to, various enzymes, such as, but not limited to, horseradish peroxidase, alkaline phosphatase, beta-galactosidase, or acetylcholinesterase; prosthetic groups, such as, but not limited to, streptavidinlbiotin and avidin/biotin; fluorescent materials, such as, but not limited to, umbelliferone, fluorescein, fluorescein isothiocynate, rhodamine, dichlorotriazinylamine fluorescein, dansyl chloride or phycoerythrin; luminescent materials, such as, but not limited to, luminol; bioluminescent materials, such as but not limited to, luciferase, luciferin, and aequorin; radioactive materials, such as, but not limited to, iodine (¹³¹I, ¹²¹I, ¹²¹I, and ¹²¹I,), carbon (¹⁴C), sulfur (³⁵S), tritium (³H), indium (¹¹⁵In, ¹¹³In, ¹¹²In, and ¹¹¹In,), technetium (⁹⁹Tc), thallium (²⁰¹Ti), gallium (⁶⁸Ga, ⁶⁷Ga), palladium (¹⁰³Pd), molybdenum (⁹⁹Mo), xenon (¹³³Xe), fluorine (¹⁸F), ¹⁵³Sm, ¹⁷⁷Lu, ¹⁵⁹Gd, ¹⁴⁹Pm, ¹⁴⁰La, ¹⁷⁵Yb, ¹⁶⁶Ho, ⁹⁰Y, 47Sc, ¹⁸⁶Re, ¹⁸⁸Re, ¹⁴²Pr, ¹⁰⁵Rh, ⁹⁷Ru, ⁶⁸Ge, ⁵⁷Co, ⁶⁵Zn, ⁸⁵Sr, ³²P, ¹⁵³Gd, ¹⁶⁹Yb, ⁵¹Cr, ⁵⁴Mn, ⁷⁵Se, ¹¹³Sn, and ¹¹⁷Tin; and positron emitting metals using various positron emission tomographies, and nonradioactive paramagnetic metal ions.

The present application further encompasses uses of the present molecules or fragments thereof conjugated to a therapeutic moiety. The molecules of the present invention or fragments thereof may be conjugated to a therapeutic moiety such as a cytotoxin, *e.g.,* a cytostatic or cytocidal agent, a therapeutic agent or a radioactive metal ion, *e.g.,* alpha-emitters. A cytotoxin or cytotoxic agent includes any agent that is detrimental to cells.

Further, the present molecule or fragment thereof may be conjugated to a therapeutic moiety or drug moiety that modifies a given biological response. Therapeutic moieties or drug moieties are not to be construed as limited to classical chemical therapeutic agents. For example, the drug moiety may be a protein, peptide, or polypeptide possessing a desired biological activity. Such proteins may include, for example, a toxin such as abrin, ricin A, pseudomonas exotoxin, cholera toxin, or diphtheria toxin; a protein such as tumor necrosis factor, α-interferon, β-interferon, nerve growth factor, platelet derived growth factor, tissue plasminogen activator, an apoptotic agent, an anti-angiogenic agent; or, a biological response modifier such as, for example, a lymphokine.

In one embodiment, the present molecule, or a fragment thereof, is conjugated to a therapeutic moiety, such as a cytotoxin, a drug (e.g., an immunosuppressant) or a radiotoxin. Such conjugates are referred to herein as "immunoconjugates". Immunoconjugates that include one or more cytotoxins are referred to as "immunotoxins." A cytotoxin or cytotoxic agent includes any agent that is detrimental to (e.g., kills) cells. Examples include taxon, cytochalasin B, gramicidin D, ethidium bromide, emetine, mitomycin, etoposide, tenoposide, vincristine, vinblastine, t. colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents also include, for example, antimetabolites (e.g., methotrexate, 6-mercaptopurine, 6-thioguanine, cytarabine, 5-fluorouracil decarbazine), ablating agents (e.g., mechlorethamine, thioepa chloraxnbucil, meiphalan, carmustine (BSNU) and lomustine (CCNU), cyclothosphamide, busulfan, dibromomannitol, streptozotocin, mitomycin C, and cis-dichlorodiamine platinum (II) (DDP) cisplatin, anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine). (See e.g., Seattle Genetics US20090304721).

Other examples of therapeutic cytotoxins that can be conjugated to the molecules of the present invention include duocarmycins, calicheamicins, maytansines and auristatins, and derivatives thereof. An example of a calicheamicin antibody conjugate is commercially available (Mylotarg™; Wyeth-Ayerst).

Cytoxins can be conjugated to the molecules of the invention using linker technology available in the art. Examples of linker types that have been used to conjugate a cytotoxin to an antibody include, but are not limited to, hydrazones, thioethers, esters, disulfides and peptide-containing linkers. A linker can be chosen that is, for example, susceptible to cleavage by low pH within the lysosomal compartment or susceptible to cleavage by proteases, such as proteases preferentially expressed in tumor tissue such as cathepsins (e.g., cathepsins B, C, D).

For further discussion of types of cytotoxins, linkers and methods for conjugating therapeutic agents to the molecules, see also Saito et al., (2003) Adv. Drug Deliv. Rev. 55:199-215; Trail et al., (2003) Cancer Immunol. Immunother. 52:328-337; Payne, (2003) Cancer Cell 3:207-212; Allen, (2002) Nat. Rev. Cancer 2:750-763; Pastan and Kreitman, (2002) Curr. Opin. Investig. Drugs 3:1089-1091; Senter and Springer, (2001) Adv. Drug Deliv. Rev. 53:247-264.

The molecules of the present invention also can be conjugated to a radioactive isotope to generate cytotoxic radiopharmaceuticals, also referred to as radioimmunoconjugates. Examples of radioactive isotopes that can be conjugated to molecules for use diagnostically or therapeutically include, but are not limited to, iodine¹³¹, indium¹¹¹, yttrium⁹⁰, and lutetium¹⁷⁷. Method for preparing radioimmunconjugates are established in the art. Examples of radioimmunoconjugates are commercially available, including Zevalin™ (DEC Pharmaceuticals) and Bexxar™ (Corixa Pharmaceuticals), and similar methods can be used to prepare radioimmunoconjugates using the molecules of the invention. In certain embodiments, the macrocyclic chelator is 1,4,7,10-tetraazacyclododecane-N,N',N",N'''-tetraacetic acid (DOTA) which can be attached to the antibody via a linker molecule. Such linker molecules are commonly known in the art and described in Denardo et al., (1998) Clin Cancer Res. 4(10):2483-90; Peterson et al., (1999) Bioconjug. Chem. 10(4):553-7; and Zimmerman et al., (1999) Nucl. Med. Biol. 26(8):943-50.

Techniques for conjugating therapeutic moieties to antibodies or antibody-like molecules are well known, see, *e.g.,* Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies 84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., (1982) Immunol. Rev. 62:119-58.

The molecules may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

### VII. Methods of Making the Molecules of the Present Invention

Where polypeptides of the multispecific molecules of the present invention are crosslinked, these functional linkages can be accomplished using methods known in the art. A variety of coupling or cross-linking agents can be used for covalent conjugation. Examples of cross-linking agents include protein A, carbodiimide, N-succinimidyl-S-acetyl-thioacetate (SATA), 5,5'-dithiobis(2-nitrobenzoic acid) (DTNB), o-phenylenedimaleimide (oPDM), N-succinimidyl-3-(2-pyridyldithio)propionate (SPDP), and sulfosuccinimidyl 4-(N-maleimidomethyl) cyclohexane-1-carboxylate (sulfo-SMCC) (see *e.g.,* Karpovsky et al., (1984) J. Exp. Med. 160:1686; Liu et al. (1985) Proc. Natl. Acad. Sci. USA 82:8648). Other methods include those described in Paulus (1985) Behring Ins. Mitt. No. 78:118-132; Brennan et al., (1985) Science 229:81-83), and Glennie et al., (1987) J. Immunol. 139: 2367-2375). Conjugating agents are SATA and sulfo-SMCC, both available from Pierce Chemical Co. (Rockford, IL).

Alternatively, the present molecules can be generated recombinantly by introducing DNA constructs encoding the desired molecules into expression vectors and expressing and assembling the desired molecules in the same host cells.

### A. Preparing Polypeptide Chains

The first step of producing the present molecules is preparing the half-antibodies or component polypeptides (i.e., the one or more polypeptide chains of the molecules comprising the first and second antigen-binding domains). If the molecules are produced recombinantly, the nucleic acid molecules encoding the first and second half antibodies may be prepared first.

Polypeptides and antibodies and fragments thereof (e.g., half antibodies) can be produced by a variety of techniques, including conventional monoclonal antibody methodology *e.g.,* the standard somatic cell hybridization technique of Kohler and Milstein, (1975) Nature 256: 495. Many techniques for producing monoclonal antibody can be employed *e.g.,* viral or oncogenic transformation of B lymphocytes.

An animal system for preparing hybridomas is the murine system. Hybridoma production in the mouse is a well-established procedure. Immunization protocols and techniques for isolation of immunized splenocytes for fusion are known in the art. Fusion partners (*e.g*., murine myeloma cells) and fusion procedures are also known.

Chimeric or humanized antibodies used in the present invention can be prepared based on the sequence of a murine monoclonal antibody prepared as described above. DNA encoding the heavy and light chain immunoglobulins can be obtained from the murine hybridoma of interest and engineered to contain non-murine (*e.g.*, human) immunoglobulin sequences using standard molecular biology techniques. For example, to create a chimeric antibody, the murine variable regions can be linked to human constant regions using methods known in the art (see *e.g.,* U.S. Pat. No. 4,816,567 to Cabilly et al.). To create a humanized antibody, the murine CDR regions can be inserted into a human framework using methods known in the art. See *e.g.,* U.S. Pat. No. 5225539 to Winter, and U.S. Pat. Nos. 5,530,101; 5,585,089; 5,693,762 and 6,180,370 to Queen et al.

In a certain embodiment, the antibody or antibody-like molecules of the invention are human monoclonal antibodies. Such human monoclonal antibodies can be generated using transgenic or transchromosomic mice carrying parts of the human immune system rather than the mouse system. These transgenic and transchromosomic mice include mice referred to herein as HuMAb mice and KM mice, respectively, and are collectively referred to herein as "human Ig mice."

The HuMAb mouse® (Medarex, Inc.) contains human immunoglobulin gene miniloci that encode un-rearranged human heavy (µ and γ) and κ light chain immunoglobulin sequences, together with targeted mutations that inactivate the endogenous µ and κ chain loci (see *e.g.,* Lonberg, et al., (1994) Nature 368(6474): 856-859). Accordingly, the mice exhibit reduced expression of mouse IgM or κ, and in response to immunization, the introduced human heavy and light chain transgenes undergo class switching and somatic mutation to generate high affinity human IgGκ monoclonal (Lonberg *et al*., (1994) supra; reviewed in Lonberg, (1994) Handbook of Experimental Pharmacology 113:49-101; Lonberg and Huszar, (1995) Intern. Rev. Immunol.13: 65-93, and Harding and Lonberg, (1995) Ann. N. Y. Acad. Sci. 764:536-546). The preparation and use of HuMAb mice, and the genomic modifications carried by such mice, is further described in Taylor et al., (1992) Nucleic Acids Research 20:6287-6295; Chen et al., (1993) International Immunology 5: 647-656; Tuaillon et al., (1993) Proc. Natl. Acad. Sci. USA 94:3720-3724; Choi et al., (1993) Nature Genetics 4:117-123; Chen et al., (1993) EMBO J. 12:821-830; Tuaillon et al., (1994) J. Immunol. 152:2912-2920; Taylor et al., (1994) International Immunology 579-591; and Fishwild et al., (1996) Nature Biotechnology 14: 845-851. See further, U.S. Pat. Nos. 5,545,806; 5,569,825; 5,625,126; 5,633,425; 5,789,650; 5,877,397; 5,661,016; 5,814,318; 5,874,299; and 5,770,429; all to Lonberg and Kay; U.S. Patent No. 5,545,807 to Surani et al.; PCT Publication Nos. WO 92103918, WO 93/12227, WO 94/25585, WO 97113852, WO 98/24884 and WO 99/45962, all to Lonberg and Kay; and PCT Publication No. WO 01/14424 to Korman et al.

In another embodiment, human antibodies used in the present invention can be raised using a mouse that carries human immunoglobulin sequences on transgenes and transchomosomes such as a mouse that carries a human heavy chain transgene and a human light chain transchromosome. Such mice, referred to herein as "KM mice", are described in detail in PCT Publication WO 02/43478 to Ishida et al.

Still further, alternative transgenic animal systems expressing human immunoglobulin genes are available in the art and can be used to raise human antibodies used in the present invention. For example, an alternative transgenic system referred to as the Xenomouse (Abgenix, Inc.) can be used. Such mice are described in, *e.g.,* U.S. Pat. Nos. 5,939,598; 6,075,181; 6,114,598; 6, 150,584 and 6,162,963 to Kucherlapati et al.

Moreover, alternative transchromosomic animal systems expressing human immunoglobulin genes are available in the art and can be used to raise the human antibodies used in the invention. For example, mice carrying both a human heavy chain transchromosome and a human light chain tranchromosome, referred to as "TC mice" can be used; such mice are described in Tomizuka et al., (2000) Proc. Natl. Acad. Sci. USA 97:722-727. Furthermore, cows carrying human heavy and light chain transchromosomes have been described in the art (Kuroiwa et al., (2002) Nature Biotechnology 20:889-894) and can be used to raise human antibodies used in the present application.

Human monoclonal antibodies can also be prepared using phage display methods for screening libraries of human immunoglobulin genes. Such phage display methods for isolating human antibodies are established in the art or described in the examples below. See for example: U.S. Pat. Nos. 5,223,409; 5,403,484; and 5,571,698 to Ladner et al.; U.S. Patent Nos. 5,427,908 and 5,580,717 to Dower et al.; U.S. Patent Nos. 5,969,108 and 6,172,197 to McCafferty et al.; and U.S. Patent Nos. 5,885,793; 6,521,404; 6,544,731; 6,555,313; 6,582,915 and 6,593,081 to Griffiths et al.

Human monoclonal antibodies used in the invention can also be prepared using SCID mice into which human immune cells have been reconstituted such that a human antibody response can be generated upon immunization. Such mice are described in, for example, U.S. Pat. Nos. 5,476,996 and 5,698,767 to Wilson et al.

Methods of making bispecific antibodies are known in the art and discussed in the present application.

### B. Methods of Producing Molecules of the Present Invention Recombinantly

In one embodiment, the present application provides a method of producing the one or more polypeptide chains of the multispecific molecule recombinantly, comprising: 1) producing one or more DNA constructs comprising a nucleic acid molecule encoding each of the polypeptide chains of the multispecific molecule; 2) introducing said DNA construct(s) into one or more expression vectors; 3) co-transfecting said expression vector(s) in one or more host cells; and 4) expressing and assembling the molecule in a host cell or in solution.

In this respect, the disclosure provides isolated nucleic acid, e.g., one or more polynucleotides, encoding the multispecific molecule described herein, for example a multispecific molecule that includes an anti-CD3 binding domain, e.g., as described herein, and an anti-CLL-1 binding domain, e.g., as describe herein. In embodiments, the isolated nucleic acid is disposed on a single continuous polynucleotide. In other embodiments, the isolated polynucleotide is disposed on two or more continuous polynucleotides.

In aspects, the nucleic acid includes sequence encoding an anti-CD3 binding domain. In aspects, the nucleic acid includes SEQ ID NO: 508 and SEQ ID NO: 509.

In aspects, the isolated nucleic acid includes SEQ ID NO: 510.

In aspects, the isolated nucleic acid includes SEQ ID NO: 511.

In aspects, the nucleic acid includes sequence encoding an anti-CLL-1 binding domain. In aspects, the isolated nucleic acid includes:
a) SEQ ID NO: 513 and SEQ ID NO: 514;
b) SEQ ID NO: 518 and SEQ ID NO: 519;
c) SEQ ID NO: 523 and SEQ ID NO: 524;
d) SEQ ID NO: 528 and SEQ ID NO: 529;
e) SEQ ID NO: 533 and SEQ ID NO: 534;
f) SEQ ID NO: 538 and SEQ ID NO: 539;
g) SEQ ID NO: 543 and SEQ ID NO: 544;
h) SEQ ID NO: 548 and SEQ ID NO: 549; or
i) SEQ ID NO: 553 and SEQ ID NO: 554.

In aspects, the isolated nucleic acid includes:
a) SEQ ID NO: 515;
b) SEQ ID NO: 520;
c) SEQ ID NO: 525;
d) SEQ ID NO: 530;
e) SEQ ID NO: 535;
f) SEQ ID NO: 540;
g) SEQ ID NO: 545;
h) SEQ ID NO: 550; or
i) SEQ ID NO: 555.

In aspects, the isolated nucleic acid includes:
a) SEQ ID NO: 516;
b) SEQ ID NO: 521;
c) SEQ ID NO: 526;
d) SEQ ID NO: 531;
e) SEQ ID NO: 536;
f) SEQ ID NO: 541;
g) SEQ ID NO: 546;
h) SEQ ID NO: 551; or
i) SEQ ID NO: 556.

In aspects, the isolated nucleic acid includes sequence encoding an anti-CD3 binding domain, for example, as described herein, and sequence encoding an anti-CLL-1 binding domain, for example, as described herein. In aspects, the sequence encoding the anti-CD3 binding domain and the sequence encoding the anti-CLL-1 binding domain are disposed on separate polynucleotides. In aspects, the sequence encoding the anti-CD3 binding domain and the sequence encoding the anti-CLL-1 binding domain are disposed on a single polynucleotide.

In an exemplary embodiment, the DNA sequences encoding the light chain of the first half antibody, the DNA sequence encoding the heavy chain of the first half antibody, the DNA sequences encoding the light chain of the second half antibody, and the DNA sequence encoding the heavy chain of the second half antibody are placed in separate expression vectors. The expression vectors are then co-transfected into a host cell at a ratio giving rise to optimal assembly. The encoded heavy chains and light chains are expressed in the host cell and assemble into functional molecules.

In another exemplary embodiment, the DNA sequences encoding the heavy and light chains of the first half antibody are placed in one expression vector, and the DNA sequences encoding the heavy and light chains of the second half antibody are placed in a second expression vector. The expression vectors may then be co-transfected into a host cell at a ratio giving rise to optimal assembly. The encoded heavy chains and light chains are expressed in the host cell and assemble into functional molecules. Alternatively, the expression vectors may be transfected into different host cell populations, and the multispecific molecule assembled in solution.

Desired mutations on the variable region or the constant region of the molecule described herein, such as, for enhancing hetero-dimerization, can be introduced at this stage as described herein.

The DNA sequences can be produced by de novo solid-phase DNA synthesis or by PCR mutagenesis of an existing sequence (*e.g.*, sequences as described in the Examples below) encoding heavy or light chains of the present molecules. Direct chemical synthesis of nucleic acids can be accomplished by methods known in the art, such as the phosphotriester method of Narang et al., (1979) Meth. Enzymol. 68:90; the phosphodiester method of Brown et al., (1979) Meth. Enzymol. 68:109; the diethylphosphoramidite method of Beaucage et al., (1981) Tetra. Lett., 22:1859; and the solid support method of U.S. Patent No. 4,458,066. Introducing mutations to a polynucleotide sequence by PCR can be performed as described in, *e.g.,* PCR Technology: Principles and Applications for DNA Amplification, H.A. Erlich (Ed.), Freeman Press, NY, NY, 1992; PCR Protocols: A Guide to Methods and Applications, Innis et al. (Ed.), Academic Press, San Diego, CA, 1990; Mattila et al., (1991) Nucleic Acids Res. 19:967; and Eckert et al., (1991) PCR Methods and Applications 1:17.

Also provided in the invention are expression vectors and host cells for producing the molecules in accordance with the invention as defined in the claims. Various expression vectors can be employed to express the polynucleotides encoding chains or binding domains of the molecule. Both viral-based and nonviral expression vectors can be used to produce the antibodies in a mammalian host cell. Nonviral vectors and systems include plasmids, episomal vectors, typically with an expression cassette for expressing a protein or RNA, and human artificial chromosomes (see, *e.g.,* Harrington et al., (1997) Nat Genet 15:345). For example, nonviral vectors useful for expression of the polynucleotides and polypeptides in mammalian (*e.g.*, human) cells include pThioHis A, B & C, pcDNA3.1/His, pEBVHis A, B & C, (Invitrogen, San Diego, CA), MPSV vectors, and numerous other vectors known in the art for expressing other proteins. Useful viral vectors include vectors based on retroviruses, adenoviruses, adeno associated viruses, herpes viruses, vectors based on SV40, papilloma virus, HBP Epstein Barr virus, vaccinia virus vectors and Semliki Forest virus (SFV). See, Brent *et al*., (1995) supra; Smith, Annu. Rev. Microbiol. 49:807; and Rosenfeld et al., (1992) Cell 68:143.

The choice of expression vector depends on the intended host cells in which the vector is to be expressed. Typically, the expression vectors contain a promoter and other regulatory sequences (*e.g.*, enhancers) that are operably linked to the polynucleotides encoding an antibody chain or fragment. In some embodiments, an inducible promoter is employed to prevent expression of inserted sequences except under inducing conditions. Inducible promoters include, *e.g.,* arabinose, lacZ, metallothionein promoter or a heat shock promoter. Cultures of transformed organisms can be expanded under noninducing conditions without biasing the population for coding sequences whose expression products are better tolerated by the host cells. In addition to promoters, other regulatory elements may also be required or desired for efficient expression of the heavy chains and light chains of the multispecific molecules. These elements typically include an ATG initiation codon and adjacent ribosome binding site or other sequences. In addition, the efficiency of expression may be enhanced by the inclusion of enhancers appropriate to the cell system in use (see, *e.g.,* Scharf et al., (1994) Results Probl. Cell Differ. 20:125; and Bittner et al., (1987) Meth. Enzymol., 153:516). For example, the SV40 enhancer or CMV enhancer may be used to increase expression in mammalian host cells.

The expression vectors may also provide a secretion signal sequence position to form a fusion protein with polypeptides encoded by inserting the above-described sequences of heavy chain and/or light chain or fragments thereof. More often, the inserted antibody or antibody-like molecule sequences are linked to a signal sequences before inclusion in the vector. Vectors to be used to receive sequences encoding light and heavy chain variable domains sometimes also encode constant regions or parts thereof. Such vectors allow expression of the variable regions as fusion proteins with the constant regions thereby leading to production of intact antibodies or antibody-like molecules or fragments thereof. Typically, such constant regions are human.

The host cells for harboring and expressing the present molecules can be either prokaryotic or eukaryotic. E. coli is one prokaryotic host useful for cloning and expressing the polynucleotides of the present invention. Other microbial hosts suitable for use include bacilli, such as Bacillus subtilis, and other enterobacteriaceae, such as Salmonella, Serratia, and various Pseudomonas species. In these prokaryotic hosts, one can also make expression vectors, which typically contain expression control sequences compatible with the host cell (*e.g.,* an origin of replication). In addition, any number of a variety of well-known promoters will be present, such as the lactose promoter system, a tryptophan (trp) promoter system, a beta-lactamase promoter system, or a promoter system from phage lambda. The promoters typically control expression, optionally with an operator sequence, and have ribosome binding site sequences and the like, for initiating and completing transcription and translation. Other microbes, such as yeast, can also be employed to express the antibody of the invention. Insect cells in combination with baculovirus vectors can also be used.

In some preferred embodiments, mammalian host cells are used to express and produce the molecules of the present invention. For example, they can be either a hybridoma cell line expressing endogenous immunoglobulin genes (*e.g.,* the 1D6.C9 myeloma hybridoma clone as described in the Examples) or a mammalian cell line harboring an exogenous expression vector (*e.g.,* the SP2/0 myeloma cells exemplified below). These include any normal mortal or normal or abnormal immortal animal or human cell. For example, a number of suitable host cell lines capable of secreting intact immunoglobulins have been developed including the CHO cell lines, various Cos cell lines, HeLa cells, myeloma cell lines, transformed B-cells and hybridomas. The use of mammalian tissue cell culture to express polypeptides is discussed generally in, *e.g.,* Winnacker, FROM GENES TO CLONES, VCH Publishers, N.Y., N.Y., 1987. Expression vectors for mammalian host cells can include expression control sequences, such as an origin of replication, a promoter, and an enhancer (see, *e.g.,* Queen et al., (1986) Immunol. Rev. 89:49-68), and necessary processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage-specific, and/or modulatable or regulatable. Useful promoters include, but are not limited to, the metallothionein promoter, the constitutive adenovirus major late promoter, the dexamethasone-inducible MMTV promoter, the SV40 promoter, the MRP polIII promoter, the constitutive MPSV promoter, the tetracycline-inducible CMV promoter (such as the human immediate-early CMV promoter), the constitutive CMV promoter, and promoter-enhancer combinations known in the art.

Methods for introducing expression vectors containing the polynucleotide sequences of interest vary depending on the type of cellular host. For example, calcium chloride transfection is commonly utilized for prokaryotic cells, whereas calcium phosphate treatment or electroporation may be used for other cellular hosts. (See generally Sambrook, *et al*., supra). Other methods include, *e.g.,* electroporation, calcium phosphate treatment, liposome-mediated transformation, injection and microinjection, ballistic methods, virosomes, immunoliposomes, polycation:nucleic acid conjugates, naked DNA, artificial virions, fusion to the herpes virus structural protein VP22 (Elliot and O'Hare, (1997) Cell 88:223), agent-enhanced uptake of DNA, and ex vivo transduction. For long-term, high-yield production of recombinant proteins, stable expression will often be desired. For example, cell lines which stably express antibody chains or binding fragments can be prepared using expression vectors of the invention which contain viral origins of replication or endogenous expression elements and a selectable marker gene. Following the introduction of the vector, cells may be allowed to grow for 1-2 days in an enriched media before they are switched to selective media. The purpose of the selectable marker is to confer resistance to selection, and its presence allows growth of cells which successfully express the introduced sequences in selective media. Resistant, stably transfected cells can be proliferated using tissue culture techniques appropriate to the cell type.

The present molecule preferably is generally recovered from the culture medium as a secreted polypeptide, although it also may be recovered from host cell lysate when directly produced without a secretory signal. If the molecule is membrane-bound, it can be released from the membrane using a suitable detergent solution (e.g. Triton-X 100).

When the molecule is produced in a recombinant cell other than one of human origin, it is completely free of proteins or polypeptides of human origin. However, it is necessary to purify the molecule from recombinant cell proteins or polypeptides to obtain preparations that are substantially homogeneous as to heteromultimer. As a first step, the culture medium or lysate is normally centrifuged to remove particulate cell debris. The produced molecules can be conveniently purified by hydroxylapatite chromatography, gel electrophoresis, dialysis, or affinity chromatography, with affinity chromatography being the preferred purification technique. Other techniques for protein purification such as fractionation on an ion-exchange column, ethanol precipitation, reverse phase HPLC, chromatography on silica, chromatography on heparin Sepharose, chromatography on an anion or cation exchange resin (such as a polyaspartic acid column), chromatofocusing, SDS-PAGE, and ammonium sulfate precipitation are also available.

### XI. Use of the Molecules of the Present Invention

### A. Diagnostic and Therapeutic Use

Depending on the antigens that are recognized by the molecules of the present invention, the present molecules have many diagnostic and therapeutic applications. For instance, they can be used for enzyme immunoassay, with N-terminal arms binding a specific epitope on the enzyme and C-terminal arms binding the immobilizing matrix. The enzyme immunoassay using antibody-like molecules is discussed by Nolan *et al.* (Nolan et al., (1990) Biochem. Biophys. Acta. 1040:1-11). The multispecific molecules can also be used for diagnosis of various diseases such as cancer (Songsivilai et al., (1990) Clin. Exp. Immunol. 79:315). In particular, one antigen binding domain of the molecule can bind a cancer antigen and the other binding site can bind a detectable marker described herein, for example, a chelator which tightly binds a radionuclide. (Le Doussal et al., (1992) Int. J. Cancer Suppl. 7:58-62 and Le Doussal et al., (1993) J. Nucl. Med. 34:1662-1671; Stickney et al., (1995) Cancer Res. 51:6650-6655).

The present molecules find therapeutic uses for treating various human diseases, for example, cancer, autoimmune diseases, and infectious diseases, etc.

For instance, with at least one antigen binding domain binding a tumor target or a pathogen target and at least a second antigen binding domain binding an antigen of an immune effector cell, e.g., a T cell or NK cell, the present molecules of the disclosure are capable of killing tumor cells or pathogens by using the patient's immune defense system using the approach discussed in Segal et al., Chem. Immunol. 47:179 (1989) and Segal et al., Biologic Therapy of Cancer 2(4) DeVita et al. eds. J. B. Lippincott, Philadelphia (1992) p. 1.

Similarly, the present molecules can also mediate killing by T cells, for example by linking the CD3 complex on T cells to a tumor-associated antigen.

The present molecules may also be used as fibrinolytic agents or vaccine adjuvants. Furthermore, the antibodies or antibody-like molecules may be used in the treatment of infectious diseases (e.g. for targeting of effector cells to virally infected cells such as HIV or influenza virus or protozoa such as Toxoplasma gondii), used to deliver immunotoxins to tumor cells, or target immune complexes to cell surface receptors (Romet-Lemonne, Fanger and Segal Eds., Lienhart (1991) p. 249.). The present molecules may also be used to deliver immunotoxin to tumor cells.

### B. Pharmaceutical Compositions

To prepare pharmaceutical or sterile compositions including the molecule of the present invention, the molecule is mixed with a pharmaceutically acceptable carrier or excipient.

Formulations of therapeutic and diagnostic agents can be prepared by mixing with physiologically acceptable carriers, excipients, or stabilizers in the form of, e.g., lyophilized powders, slurries, aqueous solutions, lotions, or suspensions (see, e.g., Hardman, et al. (2001) Goodman and Gilman's The Pharmacological Basis of Therapeutics, McGraw-Hill, New York, N.Y.; Gennaro (2000) Remington: The Science and Practice of Pharmacy, Lippincott, Williams, and Wilkins, New York, N.Y.; Avis, et al. (eds.) (1993) Pharmaceutical Dosage Forms: eral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, N.Y.).

Selecting an administration regimen for a therapeutic depends on several factors, including the serum or tissue turnover rate of the entity, the level of symptoms, the immunogenicity of the entity, and the accessibility of the target cells in the biological matrix. In certain embodiments, an administration regimen maximizes the amount of therapeutic delivered to the patient consistent with an acceptable level of side effects. Accordingly, the amount of biologic delivered depends in part on the particular entity and the severity of the condition being treated. Guidance in selecting appropriate doses of antibodies, cytokines, and small molecules are available (see, e.g., Wawrzynczak (1996) Antibody Therapy, Bios Scientific Pub. Ltd, Oxfordshire, UK; Kresina (ed.) (1991) Monoclonal Antibodies, Cytokines and Arthritis, Marcel Dekker, New York, N.Y.; Bach (ed.) (1993) Monoclonal Antibodies and Peptide Therapy in Autoimmune Diseases, Marcel Dekker, New York, N.Y.; Baert, et al. (2003) New Engl. J. Med. 348:601-608; Milgrom, et al. (1999) New Engl. J. Med. 341:1966-1973; Slamon, et al. (2001) New Engl. J. Med. 344:783-792; Beniaminovitz, et al. (2000) New Engl. J. Med. 342:613-619; Ghosh, et al. (2003) New Engl. J. Med. 348:24-32; Lipsky, et al. (2000) New Engl. J. Med. 343:1594-1602).

Determination of the appropriate dose is made by the clinician, e.g., using parameters or factors known or suspected in the art to affect treatment or predicted to affect treatment. Generally, the dose begins with an amount somewhat less than the optimum dose and it is increased by small increments thereafter until the desired or optimum effect is achieved relative to any negative side effects. Important diagnostic measures include those of symptoms of, e.g., the inflammation or level of inflammatory cytokines produced.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors known in the medical arts.

Compositions comprising the molecules or fragments thereof of the present application can be provided by continuous infusion, or by doses at intervals of, e.g., one day, one week, or 1-7 times per week. Doses may be provided intravenously, subcutaneously, topically, orally, nasally, rectally, intramuscular, intracerebrally, or by inhalation. A specific dose protocol is one involving the maximal dose or dose frequency that avoids significant undesirable side effects. A total weekly dose may be at least 0.05 µg/kg body weight, at least 0.2 µg/kg, at least 0.5 µg/kg, at least 1 µg/kg, at least 10 µg/kg, at least 100 µg/kg, at least 0.2 mg/kg, at least 1.0 mg/kg, at least 2.0 mg/kg, at least 10 mg/kg, at least 25 mg/kg, or at least 50 mg/kg (see, e.g., Yang, et al. (2003) New Engl. J. Med. 349:427-434; Herold, et al. (2002) New Engl. J. Med. 346:1692-1698; Liu, et al. (1999) J. Neurol. Neurosurg. Psych. 67:451-456; Portielji, et al. (2003) Cancer Immunol. Immunother. 52:133-144). The desired dose of the molecules or fragments thereof is about the same as for an antibody or polypeptide, on a moles/kg body weight basis. The desired plasma concentration of the molecules or fragments thereof is about, on a moles/kg body weight basis. The dose may be at least 15 µg at least 20 µg, at least 25 µg, at least 30 µg, at least 35 µg, at least 40 µg, at least 45 µg, at least 50 µg, at least 55 µg, at least 60 µg, at least 65 µg, at least 70 µg, at least 75 µg, at least 80 µg, at least 85 µg, at least 90 µg, at least 95 µg, or at least 100 µg. The doses administered to a subject may number at least 1, 2, 3, 4, 5, 6, 7, 8, 9, 10, 11, or 12, or more.

For the molecules or fragments thereof of the invention, the dosage administered to a patient may be 0.0001 mg/kg to 100 mg/kg of the patient's body weight. The dosage may be between 0.0001 mg/kg and 20 mg/kg, 0.0001 mg/kg and 10 mg/kg, 0.0001 mg/kg and 5 mg/kg, 0.0001 and 2 mg/kg, 0.0001 and 1 mg/kg, 0.0001 mg/kg and 0.75 mg/kg, 0.0001 mg/kg and 0.5 mg/kg, 0.0001 mg/kg to 0.25 mg/kg, 0.0001 to 0.15 mg/kg, 0.0001 to 0.10 mg/kg, 0.001 to 0.5 mg/kg, 0.01 to 0.25 mg/kg or 0.01 to 0.10 mg/kg of the patient's body weight.

The dosage of the molecules or fragments thereof of the present application may be calculated using the patient's weight in kilograms (kg) multiplied by the dose to be administered in mg/kg. The dosage of the molecules or fragments thereof, of the present application may be 150 µg/kg or less, 125 µg/kg or less, 100 µg/kg or less, 95 µg/kg or less, 90 µg/kg or less, 85 µg/kg or less, 80 µg/kg or less, 75 µg/kg or less, 70 µg/kg or less, 65 µg/kg or less, 60 µg/kg or less, 55 µg/kg or less, 50 µg/kg or less, 45 µg/kg or less, 40 µg/kg or less, 35 µg/kg or less, 30 µg/kg or less, 25 µg/kg or less, 20 µg/kg or less, 15 µg/kg or less, 10 µg/kg or less, 5 µg/kg or less, 2.5 µg/kg or less, 2 µg/kg or less, 1.5 µg/kg or less, 1 µg/kg or less, 0.5 µg/kg or less, or 0.5 µg/kg or less of a patient's body weight.

Unit dose of the molecules or fragments thereof of the present application may be 0.1 mg to 20 mg, 0.1 mg to 15 mg, 0.1 mg to 12 mg, 0.1 mg to 10 mg, 0.1 mg to 8 mg, 0.1 mg to 7 mg, 0.1 mg to 5 mg, 0.1 to 2.5 mg, 0.25 mg to 20 mg, 0.25 to 15 mg, 0.25 to 12 mg, 0.25 to 10 mg, 0.25 to 8 mg, 0.25 mg to 7 mg, 0.25 mg to 5 mg, 0.5 mg to 2.5 mg, 1 mg to 20 mg, 1 mg to 15 mg, 1 mg to 12 mg, 1 mg to 10 mg, 1 mg to 8 mg, 1 mg to 7 mg, 1 mg to 5 mg, or 1 mg to 2.5 mg.

The dosage of the molecules or fragments thereof of the present application may achieve a serum titer of at least 0.1 µg/ml, at least 0.5 µg/ml, at least 1 µg/ml, at least 2 µg/ml, at least 5 µg/ml, at least 6 µg/ml, at least 10 µg/ml, at least 15 µg/ml, at least 20 µg/ml, at least 25 µg/ml, at least 50 µg/ml, at least 100 µg/ml, at least 125 µg/ml, at least 150 µg/ml, at least 175 µg/ml, at least 200 µg/ml, at least 225 µg/ml, at least 250 µg/ml, at least 275 µg/ml, at least 300 µg/ml, at least 325 µg/ml, at least 350 µg/ml, at least 375 µg/ml, or at least 400 µg/ml in a subject. Alternatively, the dosage of the molecule or fragments thereof, of the present application may achieve a serum titer of at least 0.1 µg/ml, at least 0.5 µg/ml, at least 1 µg/ml, at least, 2 µg/ml, at least 5 µg/ml, at least 6 µg/ml, at least 10 µg/ml, at least 15 µg/ml, at least 20 µg /ml, at least 25 µg/ml, at least 50 µg/ml, at least 100 µg/ml, at least 125 µg/ml, at least 150 µg/ml, at least 175 µg/ml, at least 200 µg/ml, at least 225 µg/ml, at least 250 µg/ml, at least 275 µg/ml, at least 300 µg/ml, at least 325 µg/ml, at least 350 µg/ml, at least 375 µg/ml, or at least 400 µg/ml in the subject.

Doses of the molecules or fragments thereof of the application may be repeated and the administrations may be separated by at least 1 day, 2 days, 3 days, 5 days, 10 days, 15 days, 30 days, 45 days, 2 months, 75 days, 3 months, or at least 6 months.

An effective amount for a particular patient may vary depending on factors such as the condition being treated, the overall health of the patient, the method route and dose of administration and the severity of side effects (see, e.g., Maynard, et al. (1996) A Handbook of SOPs for Good Clinical Practice, Interpharm Press, Boca Raton, Fla.; Dent (2001) Good Laboratory and Good Clinical Practice, Urch Publ., London, UK).

The route of administration may be by, e.g., topical or cutaneous application, injection or infusion by intravenous, intraperitoneal, intracerebral, intramuscular, intraocular, intraarterial, intracerebrospinal, intralesional, or by sustained release systems or an implant (see, e.g., Sidman et al. (1983) Biopolymers 22:547-556; Langer, et al. (1981) J. Biomed. Mater. Res. 15:167-277; Langer (1982) Chem. Tech. 12:98-105; Epstein, et al. (1985) Proc. Natl. Acad. Sci. USA 82:3688-3692; Hwang, et al. (1980) Proc. Natl. Acad. Sci. USA 77:4030-4034; U.S. Pat. Nos. 6,350,466 and 6,316,024). Where necessary, the composition may also include a solubilizing agent and a local anesthetic such as lidocaine to ease pain at the site of the injection. In addition, pulmonary administration can also be employed, e.g., by use of an inhaler or nebulizer, and formulation with an aerosolizing agent. See, e.g., U.S. Pat. Nos. 6,019,968, 5,985,320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903.

A composition of the present invention may also be administered via one or more routes of administration using one or more of a variety of methods known in the art. As will be appreciated by the skilled artisan, the route and/or mode of administration will vary depending upon the desired results. Selected routes of administration for molecules or fragments thereof of the invention include intravenous, intramuscular, intradermal, intraperitoneal, subcutaneous, spinal or other eral routes of administration, for example by injection or infusion eral administration may represent modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. Alternatively, a composition of the present application can be administered via a non-eral route, such as a topical, epidermal or mucosal route of administration, for example, intranasally, orally, vaginally, rectally, sublingually or topically. In one embodiment, the molecules or fragments thereof of the invention is administered by infusion. In another embodiment, the multispecific epitope binding protein of the invention is administered subcutaneously.

If the molecules or fragments thereof of the invention are administered in a controlled release or sustained release system, a pump may be used to achieve controlled or sustained release (see Langer, supra; Sefton, 1987, CRC Crit. Ref Biomed. Eng. 14:20; Buchwald et al., 1980, Surgery 88:507; Saudek et al., 1989, N. Engl. J. Med. 321:574). Polymeric materials can be used to achieve controlled or sustained release of the therapies of the invention (see e.g., Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger and Peppas, 1983, J., Macromol. Sci. Rev. Macromol. Chem. 23:61; see also Levy et al., 1985, Science 228:190; During et al., 1989, Ann. Neurol. 25:351; Howard et al., 1989, J. Neurosurg. 7 1:105); U.S. Pat. No. 5,679,377; U.S. Pat. No. 5,916,597; U.S. Pat. No. 5,912,015; U.S. Pat. No. 5,989,463; U.S. Pat. No. 5,128,326; PCT Publication No. WO 99/15154; and PCT Publication No. WO 99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. In one embodiment, the polymer used in a sustained release formulation is inert, free of leachable impurities, stable on storage, sterile, and biodegradable. A controlled or sustained release system can be placed in proximity of the prophylactic or therapeutic target, thus requiring only a fraction of the systemic dose (see, e.g., Goodson, in Medical Applications of Controlled Release, supra, vol. 2, pp. 115-138 (1984)).

Controlled release systems are discussed in the review by Langer (1990, Science 249:1527-1533). Any technique known to one of skill in the art can be used to produce sustained release formulations comprising one or more molecules or fragments thereof of the present application. See, e.g., U.S. Pat. No. 4,526,938, PCT publication WO 91/05548, PCT publication WO 96/20698, Ning et al., 1996, "Intratumoral Radioimmunotheraphy of a Human Colon Cancer Xenograft Using a Sustained-Release Gel," Radiotherapy & Oncology 39:179-189, Song et al., 1995, "Antibody Mediated Lung Targeting of Long-Circulating Emulsions," PDA Journal of Pharmaceutical Science & Technology 50:372-397, Cleek et al., 1997, "Biodegradable Polymeric Carriers for a bFGF Antibody for Cardiovascular Application," Pro. Int'l. Symp. Control. Rel. Bioact. Mater. 24:853-854, and Lam et al., 1997, "Microencapsulation of Recombinant Humanized Monoclonal Antibody for Local Delivery," Proc. Int'l. Symp. Control Rel. Bioact. Mater. 24:759-760.

If the molecules or fragments thereof of the invention are administered topically, they can be formulated in the form of an ointment, cream, transdermal patch, lotion, gel, shampoo, spray, aerosol, solution, emulsion, or other form well-known to one of skill in the art. See, e.g., Remington's Pharmaceutical Sciences and Introduction to Pharmaceutical Dosage Forms, 19th ed., Mack Pub. Co., Easton, Pa. (1995). For non-sprayable topical dosage forms, viscous to semi-solid or solid forms comprising a carrier or one or more excipients compatible with topical application and having a dynamic viscosity, in some instances, greater than water are typically employed. Suitable formulations include, without limitation, solutions, suspensions, emulsions, creams, ointments, powders, liniments, salves, and the like, which are, if desired, sterilized or mixed with auxiliary agents (e.g., preservatives, stabilizers, wetting agents, buffers, or salts) for influencing various properties, such as, for example, osmotic pressure. Other suitable topical dosage forms include sprayable aerosol preparations wherein the active ingredient, in some instances, in combination with a solid or liquid inert carrier, is packaged in a mixture with a pressurized volatile (e.g., a gaseous propellant, such as freon) or in a squeeze bottle. Moisturizers or humectants can also be added to pharmaceutical compositions and dosage forms if desired. Examples of such additional ingredients are well-known in the art.

If the compositions comprising the molecules or fragments thereof are administered intranasally, it can be formulated in an aerosol form, spray, mist or in the form of drops. In particular, prophylactic or therapeutic agents for use according to the present invention can be conveniently delivered in the form of an aerosol spray presentation from pressurized packs or a nebuliser, with the use of a suitable propellant (e.g., dichlorodifluoromethane, trichlorofluoromethane, dichlorotetrafluoroethane, carbon dioxide or other suitable gas). In the case of a pressurized aerosol the dosage unit may be determined by providing a valve to deliver a metered amount. Capsules and cartridges (composed of, e.g., gelatin) for use in an inhaler or insufflator may be formulated containing a powder mix of the compound and a suitable powder base such as lactose or starch.

Methods for co-administration or treatment with a second therapeutic agent, e.g., a cytokine, steroid, chemotherapeutic agent, antibiotic, or radiation, are known in the art (see, e.g., Hardman, et al. (eds.) (2001) Goodman and Gilman's The Pharmacological Basis of Therapeutics, 10.sup.th ed., McGraw-Hill, New York, N.Y.; Poole and Peterson (eds.) (2001) Pharmacotherapeutics for Advanced Practice:A Practical Approach, Lippincott, Williams & Wilkins, Phila., Pa.; Chabner and Longo (eds.) (2001) Cancer Chemotherapy and Biotherapy, Lippincott, Williams & Wilkins, Phila., Pa.). An effective amount of therapeutic may decrease the symptoms by at least 10%; by at least 20%; at least about 30%; at least 40%, or at least 50%.

Additional therapies (e.g., prophylactic or therapeutic agents), which can be administered in combination with the molecules or fragments thereof of the present application may be administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours apart from the molecules or fragments thereof of the invention. The two or more therapies may be administered within one same patient visit.

The molecules or fragments thereof of the invention and the other therapies may be cyclically administered. Cycling therapy involves the administration of a first therapy (e.g., a first prophylactic or therapeutic agent) for a period of time, followed by the administration of a second therapy (e.g., a second prophylactic or therapeutic agent) for a period of time, optionally, followed by the administration of a third therapy (e.g., prophylactic or therapeutic agent) for a period of time and so forth, and repeating this sequential administration, i.e., the cycle in order to reduce the development of resistance to one of the therapies, to avoid or reduce the side effects of one of the therapies, and/or to improve the efficacy of the therapies.

In certain embodiments, the molecules or fragments thereof of the invention can be formulated to ensure proper distribution in vivo. For example, the blood-brain barrier (BBB) excludes many highly hydrophilic compounds. To ensure that the therapeutic compounds of the invention cross the BBB (if desired), they can be formulated, for example, in liposomes. For methods of manufacturing liposomes, see, e.g., U.S. Pat. Nos. 4,522,811; 5,374,548; and 5,399,331. The liposomes may comprise one or more moieties which are selectively transported into specific cells or organs, thus enhance targeted drug delivery (see, e.g., V. V. Ranade (1989) J. Clin. Pharmacol. 29:685). Exemplary targeting moieties include folate or biotin (see, e.g., U.S. Pat. No. 5,416,016 to Low et al); mannosides (Umezawa et al., (1988) Biochem. Biophys. Res. Commun. 153:1038); antibodies (P. G. Bloeman et al. (1995) FEBS Lett. 357:140; M. Owais et al. (1995) Antimicrob. Agents Chemother. 39:180); surfactant protein A receptor (Briscoe et al. (1995) Am. J. Physiol. 1233:134); p 120 (Schreier et al (1994) J. Biol. Chem. 269:9090); see also K. Keinanen; M. L. Laukkanen (1994) FEBS Lett. 346:123; J. J. Killion; I. J. Fidler (1994) Immunomethods 4:273.

The present application provides protocols for the administration of pharmaceutical composition comprising molecules or fragments thereof of the present application alone or in combination with other therapies to a subject in need thereof. The therapies (e.g., prophylactic or therapeutic agents) of the combination therapies of the present invention can be administered concomitantly or sequentially to a subject. The therapy (e.g., prophylactic or therapeutic agents) of the combination therapies of the present invention can also be cyclically administered. Cycling therapy involves the administration of a first therapy (e.g., a first prophylactic or therapeutic agent) for a period of time, followed by the administration of a second therapy (e.g., a second prophylactic or therapeutic agent) for a period of time and repeating this sequential administration, i.e., the cycle, in order to reduce the development of resistance to one of the therapies (e.g., agents) to avoid or reduce the side effects of one of the therapies (e.g., agents), and/or to improve, the efficacy of the therapies.

The therapies (e.g., prophylactic or therapeutic agents) of the combination therapies of the invention can be administered to a subject concurrently. The term "concurrently" is not limited to the administration of therapies (e.g., prophylactic or therapeutic agents) at exactly the same time, but rather it is meant that a pharmaceutical composition comprising molecules or fragments thereof of the present application are administered to a subject in a sequence and within a time interval such that the molecules of the invention can act together with the other therapy(ies) to provide an increased benefit than if they were administered otherwise. For example, each therapy may be administered to a subject at the same time or sequentially in any order at different points in time; however, if not administered at the same time, they should be administered sufficiently close in time so as to provide the desired therapeutic or prophylactic effect. Each therapy can be administered to a subject separately, in any appropriate form and by any suitable route. In various embodiments, the therapies (e.g., prophylactic or therapeutic agents) are administered to a subject less than 15 minutes, less than 30 minutes, less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, 24 hours apart, 48 hours apart, 72 hours apart, or 1 week apart. In other embodiments, two or more therapies (e.g., prophylactic or therapeutic agents) are administered to a within the same patient visit.

The prophylactic or therapeutic agents of the combination therapies can be administered to a subject in the same pharmaceutical composition. Alternatively, the prophylactic or therapeutic agents of the combination therapies can be administered concurrently to a subject in separate pharmaceutical compositions. The prophylactic or therapeutic agents may be administered to a subject by the same or different routes of administration.

Where a series of doses are administered, these may, for example, be administered approximately every week, approximately every 2 weeks, approximately every 3 weeks, or approximately every 4 weeks, but preferably approximately every 3 weeks. The doses may, for example, continue to be administered until disease progression, adverse event, or other time as determined by the physician. For example, from about two, three, or four, up to about 17 or more fixed doses may be administered.

Aside from the multispecific molecule and antimetabolite chemotherapeutic agent, other therapeutic regimens may be combined therewith. For example, a second (third, fourth, etc) chemotherapeutic agent(s) may be administered, wherein the second chemotherapeutic agent is either another, different antimetabolite chemotherapeutic agent, or a chemotherapeutic agent that is not an antimetabolite. For example, the second chemotherapeutic agent may be a taxane (such as paclitaxel or docetaxel), capecitabine, or platinum-based chemotherapeutic agent (such as carboplatin, cisplatin, or oxaliplatin), anthracycline (such as doxorubicin, including, liposomal doxorubicin), topotecan, pemetrexed, vinca alkaloid (such as vinorelbine), and TLK 286. "Cocktails" of different chemotherapeutic agents may be administered.

In addition to the above therapeutic regimes, the patient may be subjected to surgical removal of cancer cells and/or radiation therapy.

### C. Therapeutic Application to CLL-1 Associated Diseases and/or Disorders

The present invention provides, among other things, compositions and methods for treating cancer. In one aspect, the cancer is a hematologic cancer including but is not limited to leukemia (such as acute myelogenous leukemia (AML), chronic myelogenous leukemia (CML), acute lymphoid leukemia, chronic lymphoid leukemia, acute lymphoblastic B-cell leukemia (B-cell acute lymphoid leukemia, BALL), acute lymphoblastic T-cell leukemia (T-cell acute lymphoid leukemia (TALL), B-cell prolymphocytic leukemia, plasma cell myeloma, and myelodysplastic syndrome) and malignant lymphoproliferative conditions, including lymphoma (such as multiple myeloma, non-Hodgkin's lymphoma, Burkitt's lymphoma, and small cell- and large cell-follicular lymphoma).

In one aspect, the invention provides methods for treating a disease associated with CLL-1 expression. In one aspect, the invention provides methods for treating a disease wherein part of the tumor is negative for CLL-1 and part of the tumor is positive for CLL-1. For example, the multispecific molecule, e.g., bispecific molecule, e.g., bispecific antibody or bispecific antibody-like molecule, of the invention is useful for treating subjects that have undergone treatment for a disease associated with elevated expression of CLL-1, wherein the subject that has undergone treatment for elevated levels of CLL-1 exhibits a disease associated with elevated levels of CLL-1. In embodiments, the multispecific molecule, e.g., bispecific molecule, e.g., bispecific antibody or bispecific antibody-like molecule, of the invention is useful for treating subjects that have undergone treatment for a disease associated with expression of CLL-1, wherein the subject that has undergone treatment related to expression of CLL-1 exhibits a disease associated with expression of CLL-1.

In one embodiment, the invention provides methods for treating a disease wherein CLL-1 is expressed on both normal cells and cancers cells, but is expressed at lower levels on normal cells. In one embodiment, the method further comprises selecting a multispecific molecule, e.g., bispecific molecule, e.g., bispecific antibody or bispecific antibody-like molecule, of the invention that binds with an affinity that allows the CLL-1 multispecific molecule, e.g., bispecific molecule, e.g., bispecific antibody or bispecific antibody-like molecule, to bind and mediate the killing of the cancer cells expressing CLL-1 but less than 30%, 25%, 20%, 15%, 10%, 5% or less of the normal cells expressing CLL-1 are killed, e.g., as determined by an assay described herein. For example, a killing assay such as flow cytometry based on Cr51 CTL can be used. In one embodiment, the CLL-1 multispecific molecule, e.g., bispecific molecule, e.g., bispecific antibody or bispecific antibody-like molecule, has an antigen binding domain that has a binding affinity KD of 10⁻⁴ M to 10⁻⁸ M, e.g., 10⁻⁵ M to 10⁻⁷ M, e.g., 10⁻⁶ M or 10⁻⁷ M, for the target antigen. In one embodiment, the CLL-1 antigen binding domain has a binding affinity that is at least five-fold, 10-fold, 20-fold, 30-fold, 50-fold, 100-fold or 1,000-fold less than a reference antibody, e.g., an antibody described herein.

In one aspect, the invention pertains to a vector comprising nucleic acid encoding the one or more polypeptide chains of a CLL-1 multispecific molecule, e.g., bispecific molecule, e.g., bispecific antibody or bispecific antibody-like molecule, operably linked to promoter for expression in mammalian cells, e.g., T cells or NK cells. In one aspect, the invention provides a recombinant immune effector cell, e.g., T cell or NK cell, expressing the CLL-1 multispecific molecule, e.g., bispecific molecule, e.g., bispecific antibody or bispecific antibody-like molecule, for use in treating CLL-1-expressing tumors, wherein the recombinant immune effector cell (e.g., T cell or NK cell) expressing the CLL-1 multispecific molecule, e.g., bispecific molecule, e.g., bispecific antibody or bispecific antibody-like molecule, is termed a CLL-1 multispecific molecule-expressing cell (e.g., CLL-1 multispecific molecule-expressing T cell, or CLL-1 multispecific molecule-expressing NK cell). In one aspect, the CLL-1 multispecific molecule, e.g., bispecific molecule, e.g., bispecific antibody or bispecific antibody-like molecule,-expressing cell of the invention is capable of contacting a tumor cell with at least one CLL-1 multispecific molecule, e.g., bispecific molecule, e.g., bispecific antibody or bispecific antibody-like molecule, of the invention expressed on its surface or secreted (and, in an embodiment, bound by an antigen-binding domain targeting an antigen expressed on such cell) such that the CLL-1 multispecific molecule-expressing cell (e.g., CLL-1 multispecific molecule-expressing T cell, or CLL-1 multispecific molecule-expressing NK cell) targets the tumor cell and growth of the tumor is inhibited.

Generally, the molecules comprising a CLL-1 binding domain described herein (e.g., the multispecific molecules described herein), and compositions comprising said molecules may be utilized in the treatment and prevention of diseases that arise in individuals who are immunocompromised. In particular, the molecules comprising a CLL-1 binding domain described herein (e.g., the multispecific molecules described herein), and compositions comprising said molecules of the invention are used in the treatment of diseases, disorders and conditions associated with expression of CLL-1. In certain aspects, the molecules and compositions of the invention are used in the treatment of patients at risk for developing diseases, disorders and conditions associated with expression of CLL-1. Thus, the present invention provides methods for the treatment or prevention of diseases, disorders and conditions associated with expression of CLL-1 comprising administering to a subject in need thereof, a therapeutically effective amount of the molecules comprising a CLL-1 binding domain described herein (e.g., the multispecific molecules described herein), or compositions comprising said molecules of the invention.

In one aspect the molecules comprising a CLL-1 binding domain described herein (e.g., the multispecific molecules described herein), and compositions comprising said molecules of the invention may be used to treat a proliferative disease such as a cancer or malignancy or is a precancerous condition such as a myelodysplasia, a myelodysplastic syndrome or a preleukemia. In one aspect, a cancer associated with expression of CLL-1 is a hematological cancer, preleukemia, hyperproliferative disorder, hyperplasia or a dysplasia, which is characterized by abnormal growth of cells.

In one aspect, the molecules comprising a CLL-1 binding domain described herein (e.g., the multispecific molecules described herein), and compositions comprising said molecules of the invention are used to treat a cancer, wherein the cancer is a hematological cancer. Hematological cancer conditions are the types of cancer such as leukemia and malignant lymphoproliferative conditions that affect blood, bone marrow and the lymphatic system. In one aspect, the hematological cancer by be, for example, leukemia (such as acute myelogenous leukemia, chronic myelogenous leukemia, acute lymphoid leukemia, chronic lymphoid leukemia and myelodysplastic syndrome) and malignant lymphoproliferative conditions, including lymphoma (such as multiple myeloma, non-Hodgkin's lymphoma, Burkitt's lymphoma, and small cell- and large cell-follicular lymphoma). In other embodiments, a hematologic cancer can include minimal residual disease, MRD, e.g., of a leukemia, e.g., of AML or MDS.

In one aspect, the molecules comprising a CLL-1 binding domain described herein (e.g., the multispecific molecules described herein), and compositions comprising said molecules of the present invention are particularly useful for treating myeloid leukemias, AML and its subtypes, chronic myeloid leukemia (CML), and myelodysplastic syndrome (MDS).

Leukemia can be classified as acute leukemia and chronic leukemia. Acute leukemia can be further classified as acute myelogenous leukemia (AML) and acute lymphoid leukemia (ALL). Chronic leukemia includes chronic myelogenous leukemia (CML) and chronic lymphoid leukemia (CLL). Other related conditions include myelodysplastic syndromes (MDS, formerly known as "preleukemia") which are a diverse collection of hematological conditions united by ineffective production (or dysplasia) of myeloid blood cells and risk of transformation to AML.

Lymphoma is a group of blood cell tumors that develop from lymphocytes. Exemplary lymphomas include non-Hodgkin lymphoma and Hodgkin lymphoma.

In AML, malignant transformation and uncontrolled proliferation of an abnormally differentiated, long-lived myeloid progenitor cell results in high circulating numbers of immature blood forms and replacement of normal marrow by malignant cells. Symptoms include fatigue, pallor, easy bruising and bleeding, fever, and infection; symptoms of leukemic infiltration are present in only about 5% of patients (often as skin manifestations). Examination of peripheral blood smear and bone marrow is diagnostic. Existing treatment includes induction chemotherapy to achieve remission and post-remission chemotherapy (with or without stem cell transplantation) to avoid relapse.

AML has a number of subtypes that are distinguished from each other by morphology, immunophenotype, and cytochemistry. Five classes are described, based on predominant cell type, including myeloid, myeloid-monocytic, monocytic, erythroid, and megakaryocytic.

Remission induction rates range from 50 to 85%. Long-term disease-free survival reportedly occurs in 20 to 40% of patients and increases to 40 to 50% in younger patients treated with stem cell transplantation.

Prognostic factors help determine treatment protocol and intensity; patients with strongly negative prognostic features are usually given more intense forms of therapy, because the potential benefits are thought to justify the increased treatment toxicity. The most important prognostic factor is the leukemia cell karyotype; favorable karyotypes include t(15;17), t(8;21), and inv16 (p13;q22). Negative factors include increasing age, a preceding myelodysplastic phase, secondary leukemia, high WBC count, and absence of Auer rods.

Initial therapy attempts to induce remission and differs most from ALL in that AML responds to fewer drugs. The basic induction regimen includes cytarabine by continuous IV infusion or high doses for 5 to 7 days; daunorubicin or idarubicin is given IV for 3 days during this time. Some regimens include 6-thioguanine, etoposide, vincristine, and prednisone, but their contribution is unclear. Treatment usually results in significant myelosuppression, with infection or bleeding; there is significant latency before marrow recovery. During this time, meticulous preventive and supportive care is vital.

Chronic myelogenous (or myeloid) leukemia (CML) is also known as chronic granulocytic leukemia, and is characterized as a cancer of the white blood cells. Common treatment regimens for CML include Bcr-Abl tyrosine kinase inhibitors, imatinib (Gleevec®), dasatinib and nilotinib. Bcr-Abl tyrosine kinase inhibitors are specifically useful for CML patients with the Philadelphia chromosome translocation.

Myelodysplastic syndromes (MDS) are hematological medical conditions characterized by disorderly and ineffective hematopoiesis, or blood production. Thus, the number and quality of blood-forming cells decline irreversibly. Some patients with MDS can develop severeanemia, while others are asymptomatic. The classification scheme for MDS is known in the art, with criteria designating the ratio or frequency of particular blood cell types, e.g., myeloblasts, monocytes, and red cell precursors. MDS includes refractory anemia, refractory anemia with ring sideroblasts, refractory anemia with excess blasts, refractory anemia with excess blasts in transformation, chronic myelomonocytic leukemia (CML).

Treatments for MDS vary with the severity of the symptoms. Aggressive forms of treatment for patients experiencing severe symptoms include bone marrow transplants and supportive care with blood product support (e.g., blood transfusions) and hematopoietic growth factors (e.g., erythropoietin). Other agents are frequently used to treat MDS: 5-azacytidine, decitabine, and lenalidomide. In some cases, iron chelators deferoxamine (Desferal®) and deferasirox (Exjade®) may also be administered.

In another embodiment, the molecules comprising a CLL-1 binding domain described herein (e.g., the multispecific molecules described herein), and compositions comprising said molecules of the present invention are used to treat cancers or leukemias with leukemia stem cells. For example, the leukemia stem cells are CD34⁺/CD38⁻ leukemia cells.

The present invention provides, among other things, compositions and methods for treating cancer. In one aspect, the cancer is a hematologic cancer including but is not limited to leukemia (such as acute myelogenous leukemia, chronic myelogenous leukemia, acute lymphoid leukemia, chronic lymphoid leukemia and myelodysplastic syndrome) and malignant lymphoproliferative conditions, including lymphoma (such as multiple myeloma, non-Hodgkin's lymphoma, Burkitt's lymphoma, and small cell- and large cell-follicular lymphoma).

In one aspect, the molecules comprising a CLL-1 binding domain described herein (e.g., the multispecific molecules described herein), and compositions comprising said molecules of the invention may be used to treat other cancers and malignancies such as, but not limited to, e.g., acute leukemias including but not limited to, e.g., B-cell acute lymphoid leukemia ("BALL"), T-cell acute lymphoid leukemia ("TALL"), acute lymphoid leukemia (ALL); one or more chronic leukemias including but not limited to, e.g., chronic myelogenous leukemia (CML), chronic lymphocytic leukemia (CLL); additional hematologic cancers or hematologic conditions including, but not limited to, e.g., B cell prolymphocytic leukemia, blastic plasmacytoid dendritic cell neoplasm, Burkitt's lymphoma, diffuse large B cell lymphoma, Follicular lymphoma, Hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin's lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and the like. The molecules comprising a CLL-1 binding domain described herein (e.g., the multispecific molecules described herein), and compositions comprising said molecules of the present invention may be administered either alone, or as a pharmaceutical composition in combination with diluents and/or with other components such as IL-2 or other cytokines, other molecules, or cell populations.

The present invention also provides methods for inhibiting the proliferation or reducing a CLL-1-expressing cell population, the methods comprising contacting a population of cells comprising a CLL-1-expressing cell with a molecule comprising a CLL-1 binding domain described herein (e.g., the multispecific molecules described herein), or composition comprising said molecule, of the invention that binds to the CLL-1-expressing cell. In a specific aspect, the present invention provides methods for inhibiting the proliferation or reducing the population of cancer cells expressing CLL-1, the methods comprising contacting the CLL-1-expressing cancer cell population with a molecule comprising a CLL-1 binding domain as defined in the claims, or composition comprising said molecule, of the invention that binds to the CLL-1-expressing cell. In one aspect, the present invention provides methods for inhibiting the proliferation or reducing the population of cancer cells expressing CLL-1, the methods comprising contacting the CLL-1-expressing cancer cell population with a molecule comprising a CLL-1 binding domainas defined in the claims), or composition comprising said molecule, of the invention that binds to the CLL-1-expressing cell. In certain aspects, the molecule comprising a CLL-1 binding domainas defined in the claims, or composition comprising said molecule, of the invention reduces the quantity, number, amount or percentage of cells and/or cancer cells by at least 25%, at least 30%, at least 40%, at least 50%, at least 65%, at least 75%, at least 85%, at least 95%, or at least 99% in a subject with or animal model for myeloid leukemia or another cancer associated with CLL-1-expressing cells relative to a negative control. In one aspect, the subject is a human.

The present invention also provides methods for preventing, treating and/or managing a disease associated with CLL-1-expressing cells (e.g., a hematologic cancer or atypical cancer expessing CLL-1), the methods comprising administering to a subject in need a molecule comprising a CLL-1 binding domainas defined in the claims, or composition comprising said molecule, of the invention that binds to the CLL-1-expressing cell. In one aspect, the subject is a human. Non-limiting examples of disorders associated with CLL-1-expressing cells include autoimmune disorders (such as lupus), inflammatory disorders (such as allergies and asthma) and cancers (such as hematological cancers or atypical cancers expressing CLL-1).

The present invention also provides methods for preventing, treating and/or managing a disease associated with CLL-1-expressing cells, the methods comprising administering to a subject in need a molecule comprising a CLL-1 binding domainas defined in the claims, or composition comprising said molecule, of the invention that binds to the CLL-1-expressing cell. In one aspect, the subject is a human.

The present invention provides methods for preventing relapse of cancer associated with CLL-1-expressing cells, the methods comprising administering to a subject in need thereof a molecule comprising a CLL-1 binding domain as defined in the claims), or composition comprising said molecule, of the invention that binds to the CLL-1-expressing cell. In one aspect, the methods comprise administering to the subject in need thereof an effective amount of a molecule comprising a CLL-1 binding domain as defined in the claims), or composition comprising said molecule described herein, that binds to the CLL-1-expressing cell in combination with an effective amount of another therapy.

A multispecific molecule, e.g., a bispecific molecule, e.g., a bispecific antibody or antibody-like molecule, comprising a CLL-1 binding domain, e.g., as described herein, may be used in combination with other known agents and therapies. Administered "in combination", as used herein, means that two (or more) different treatments are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

A multispecific molecule, e.g., a bispecific molecule, e.g., a bispecific antibody or antibody-like molecule, comprising a CLL-1 binding domain, e.g., as described herein, and the at least one additional therapeutic agent can be administered simultaneously, in the same or in separate compositions, or sequentially. For sequential administration, the multispecific molecule, e.g., a bispecific molecule, e.g., a bispecific antibody or antibody-like molecule, comprising a CLL-1 binding domain, e.g., as described herein, can be administered first, and the additional agent can be administered second, or the order of administration can be reversed.

The multispecific molecule, e.g., a bispecific molecule, e.g., a bispecific antibody or antibody-like molecule, comprising a CLL-1 binding domain, e.g., as described herein, and/or other therapeutic agents, procedures or modalities can be administered during periods of active disorder, or during a period of remission or less active disease. The multispecific molecule, e.g., a bispecific molecule, e.g., a bispecific antibody or antibody-like molecule, comprising a CLL-1 binding domain, e.g., as described herein, can be administered before the other treatment, concurrently with the treatment, post-treatment, or during remission of the disorder.

When administered in combination, the multispecific molecule, e.g., a bispecific molecule, e.g., a bispecific antibody or antibody-like molecule, comprising a CLL-1 binding domain, e.g., as described herein, and the additional agent (e.g., second or third agent), or all, can be administered in an amount or dose that is higher, lower or the same than the amount or dosage of each agent used individually, e.g., as a monotherapy. In certain embodiments, the administered amount or dosage of the multispecific molecule, e.g., a bispecific molecule, e.g., a bispecific antibody or antibody-like molecule, comprising a CLL-1 binding domain, e.g., as described herein, the additional agent (e.g., second or third agent), or all, is lower (e.g., at least 20%, at least 30%, at least 40%, or at least 50%) than the amount or dosage of each agent used individually, e.g., as a monotherapy. In other embodiments, the amount or dosage of the multispecific molecule, e.g., a bispecific molecule, e.g., a bispecific antibody or antibody-like molecule, comprising a CLL-1 binding domain, e.g., as described herein, the additional agent (e.g., second or third agent), or all, that results in a desired effect (e.g., treatment of cancer) is lower (e.g., at least 20%, at least 30%, at least 40%, or at least 50% lower) than the amount or dosage of each agent used individually, e.g., as a monotherapy, required to achieve the same therapeutic effect.

In further aspects, a multispecific molecule, e.g., a bispecific molecule, e.g., a bispecific antibody or antibody-like molecule, comprising a CLL-1 binding domain, e.g., as described herein, may be used in a treatment regimen in combination with surgery, chemotherapy, radiation, immunosuppressive agents, such as cyclosporin, azathioprine, methotrexate, mycophenolate, and FK506, cellular therapies (e.g., cellular immunotherapies, e.g., chimeric antigen receptor T cell therapy), antibodies, or other immunoablative agents such as CAMPATH, anti-CD3 antibodies or other antibody therapies, cytoxin, fludarabine, cyclosporin, FK506, rapamycin, mycophenolic acid, steroids, FR901228, cytokines, and irradiation. peptide vaccine, such as that described in Izumoto et al. 2008 J Neurosurg 108:963-971.

In certain instances, compounds of the present invention are combined with other therapeutic agents, such as other anti-cancer agents, anti-allergic agents, anti-nausea agents (or anti-emetics), pain relievers, cytoprotective agents, and combinations thereof.

### EXAMPLES

The following examples are provided to further illustrate the invention but not to limit its scope.

### Example 1 - Generation and binding of human anti-CLL-1 binding domains

Phage display libraries were panned against immobilized CLL-1. Bound phage were isolated and sequenced, and binding to target confirmed by ELISA and/or FACs. 13 scFvs were confirmed to bind to human CLL-1, and were designated CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, and CLL-1-13.

After selection and confirmation, to assess the binding and biophysical characteristics antihuman CLL-1 scFvs identified by phage panning, scFv constructs were transiently produced and purified from HEK293F cells. Transient expression and purification in HEK293F cells was performed with standard methodology. Briefly, 100ml of HEK293F cells at 3x106cells/ml were transfected with 100 µg plasmid and 300 µg polyethylenimine. The cells were incubated at 37°C with 8% CO2 and rotated at 80 rpm. After six days, the cells were harvested by centrifugation at 3500g for 20 minutes. The supernatant was purified by binding the scFv to 200 µl Ni-NTA agarose beads (Qiagen) overnight at 4°C. The protein was eluted with 200 µl 300mM imidazole, and dialyzed against phosphate buffered saline.

Next, the affinity for human CLL-1 of a representative number of anti-CLL-1 scFvs was tested by Biacore. Briefly, anti-Fc antibody (Jackson ImmunoReasearch, catalog # 109-005-098) was immobilized to a CM5 sensor chip via amine coupling, and human CLL-1 Fc was then captured at a density of 120 RU. ScFv samples were serial diluted 3-fold and injected over the chip at a constant flow rate. Association and dissociation rates of the protein complex were monitored for 270 s and 400 s, respectively. Double referencing was performed against an anti-huFc coated flow cell and a buffer blank and the data was fit using a 1:1 Langmuir or steady state affinity model with the Biacore T200 evaluation software. The results are reported in Table 9. Binding of clones CLL-1-6, CLL-1-8, CLL-1-9, CLL-1-10, and CLL1-13 to human CLL-1 was confirmed. As well, CLL-1-1 was found to bind weakly under these experimental conditions, and CLL-1-11 and CLL-1-12 did not exhibit binding under these assay conditions.

**Table 9. Binding kinetics and affinity of anti-CLL-1 scFvs to human CLL-1.**

| **Sample** | **Fit** | **ka (1/Ms)** | **kd (1/s)** | **KD (nM)** |
|---|---|---|---|---|
| CLL-1-1 | Limited Binding | | | |
| CLL-1-6 | 1:1 Binding | 3.78E+04 | 1.02E-03 | 26.9 |
| CLL-1-8 | 1:1 Binding | 2.16E+05 | 9.27E-04 | 4.3 |
| CLL-1-9 | 1:1 Binding | 1.41E+05 | 1.28E-03 | 9.1 |
| CLL-1-10 | 1:1 Binding | 1.42E+05 | 2.14E-03 | 15 |
| CLL-1-11 | No Binding | | | |
| CLL-1-12 | No Binding | | | |
| CLL-1-13 | 1:1 Binding | 2.57E+04 | 1.19E-03 | 46.1 |

These data confirm the binding of anti-CLL-1 scFvs to human scFv and show the clones generated have affinity for human CLL-1 ranging from 4.3 nM to 46.1 nM.

### Example 2 - In vitro assessment of CD3xCLL1 bispecific antibodies against CLL-1-expressing cancer cells

### Materials and Methods

A panel of CD3 x CLL1 antibodies were used to demonstrate the utility of these CLL1 bispecifics as a cancer therapeutic. These bispecific antibodies were constructed in the scFv-Fc format, and are composed of an anti-CD3 scFv fused to hIgG1 CH2 and CH3 domains (Fc) (SEQ ID NO: 507), paired with an anti-CLLl scFv-Fc (sequences are shown in Table 11). A non-CLL1-binding negative control bispecific antibody used in these assays is of a scFv-Fc format, containing an anti-CD3 scFv and an anti-GH scFv, which is specific for human cytomegalovirus envelope glycoprotein H. Antibody dose titrations for in vitro cell based assays represented here range from 1pM to 100nM.

PBMCs Peripheral blood mononuclear cells (PBMC) were isolated from the blood of a healthy human donor using a Ficoll-Paque PLUS (GE Healthcare #17-1440-02) density gradient. T-cells (pan) were isolated from the PBMC fraction by negative selection (Miltenyi #130-096-535, 130-041-407, 130-042-401). These isolated T-cells were activated for expansion with a 3X ratio of Human T-Activator CD3/CD28 Dynabeads (Gibco #11132D) for nine days, magnetically debeaded and stored as frozen aliquots in liquid nitrogen. Frozen aliquots were thawed, counted and used immediately in T-cell killing assays at an E:T ratio of 3:1 with target cancer cell line.

Target human cancer cell line HL60, expressing moderate levels of human CLL1, was transduced to constitutively express luciferase. Luciferase is used to measure cell viability/survival with the BrightGlo reagent (Promega E2650). Target cells were plated 30,000 cells per well in a 96 well plate (Costar 3904) together with 90,000 thawed T-cells, and a serial dilution of bispecific antibody, all in media containing RPMI/1640, 10% FBS, 2mM L-glutamine, 0.1mM Non-essential amino acids, 1mM Sodium pyruvate, 10mM HEPES, 0.055mM 2-mercaptoethanol (Gibco 22400089, 16140, 25030-081, 11140-050, 11360-070, 15630-080, 21985-023 respectively).

The assay was incubated at 37°C/5% CO2 for 20-24 hours, followed by measurements of target cell viability (BrightGlo, Promega # E2650), and interferon gamma cytokine levels in the culture supernatants (MSD # N05049A-1) as a measure of T-cell activation, following vendor supplied protocols.

A Jurkat NFAT luciferase (JNL) gene reporter assay (RGA) was also used to evaluate ability of CD3 bispecific antibodies to activate T-cells through engagement with CLL1 expressing target cell line U937. U937 cells were seeded in 96 well plates at a density of 20,000 cells per well. 100,000 JNL cells were added per well (E:T = 5:1), as well as serial dilutions of CD3 x CLL1 bispecific antibodies. The assay was incubated for 4 hours at 37°C, followed by luciferase activity measurements using the Promega OneGlo assay (Promega #E-6120) according to vendor protocol. Resulting luminescence values were plotted in GraphPad PRISM, and EC50 values determined by logistic regression analysis.

### Results

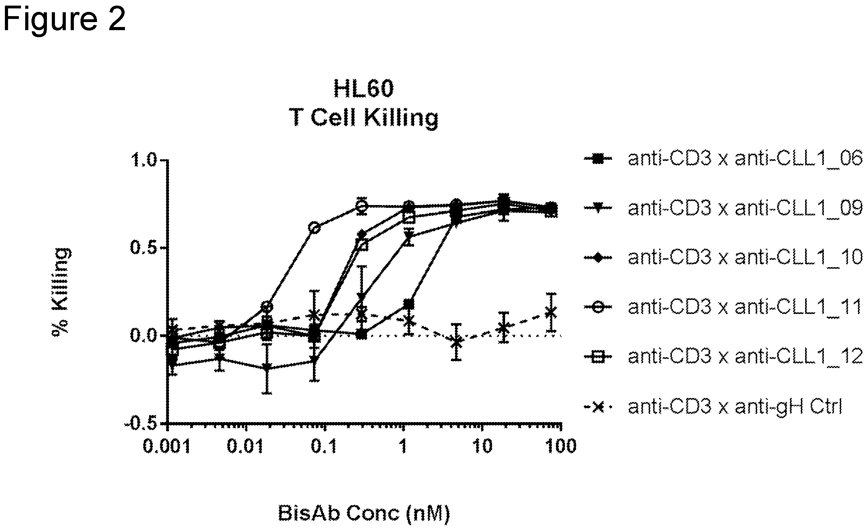

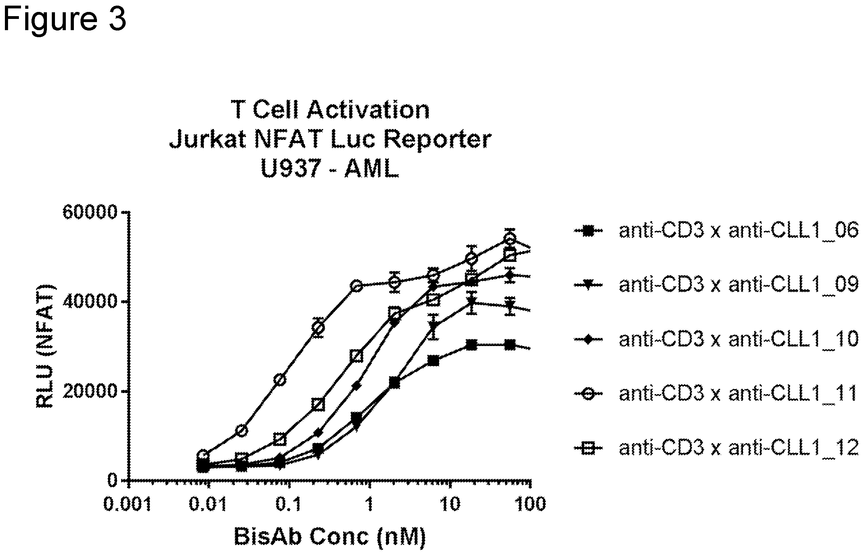

Figure 2 shows the *in vitro* ability of the CD3 x CLL-1 bispecific antibodies to meditate T cell killing of CLL1-expressing cancer cell line HL60. Figure 3 shows the ability of CD3 x CLL1 bispecific antibodies to mediate T cell activation *in vitro* via engagement with CLL1-expressing cancer cell line U937. Table 10 summarizes the results of these two experiments (NFAT = results of T-cell activation assay; Killing = results of T-cell mediated CLL1-positive cancer cell line killing assay).

**Table 10. Summary of in vitro results. CD3 x CLL-1 bispecific antibodies in the Table are listed in rank order according to potency.**

| | EC50 (nM) | |
|---|---|---|
| | **NFAT** | **Killing** |
| **Bispecific Antibody** | **U937** | **HL60** |
| CD3 x CLL1_11 | 0.08 | 0.03 |
| CD3 x CLL1_12 | 0.63 | 0.20 |
| CD3 x CLL1_10 | 0.85 | 0.27 |
| CD3 x CLL1_08 | 1.26 | 0.31 |
| CD3 x CLL1_09 | 1.74 | 0.36 |
| CD3 x CLL1_06 | 0.96 | 1.27 |
| CD3 x CLL1_13 | 2.16 | 4.24 |
| CD3 x CLL1_07 | - | 44.32 |
| CD3 x CLL1_02 | - | >300 |
| CD3 x GH (Ctrl) | - | >300 |

We have developed of a panel of CD3 x CLL1 bispecific antibodies and have shown many of them to potently redirect T cell killing of CLL1-expressing cells *in vitro.* Figure 2 demonstrates that CD3 x CLL1 bispecific antibodies can specifically and effectively engage T-cell killing of CLL1-expressing tumor cell line HL60 at an E:T ratio of 3:1 in 22 hours. Anti-CD3 x Anti-CLLl bispecific antibody clone #11 shows the greatest *in vitro* potency, with an EC50 value of 31pM. The other clones have potency ranging from 44 nM to 200 pM, as summarized in Table 10. Without being bound by theory, it is believed that these CD3 x CLL-1 bispecific antibodies are able to mediate T cell killing of CLL-1-expressing cells at good potency in part because of the combination of the highly specific CLL-1 binding domains coupled with a CD3 binding domain that binds CD3 at high affinity.

## Claims

1. A multispecific molecule comprising a first antigen binding domain and a second antigen binding domain, wherein the second antigen binding domain binds an immune effector cell antigen that is an antigen expressed on a T cell, wherein the antigen expressed on a T cell is CD3, and wherein the first antigen binding domain is an anti-CLL-1 binding domain comprising:
a heavy chain complementary determining region 1 (HC CDR1) of SEQ ID NO: 322, a heavy chain complementary determining region 2 (HC CDR2) of SEQ ID NO: 336, a heavy chain complementary determining region 3 (HC CDR3) of SEQ ID NO:350 and a light chain complementary determining region 1 (LC CDR1) of SEQ ID NO: 364, a light chain complementary determining region 2 (LC CDR2) of SEQ ID NO:378, and a light chain complementary determining region 3 (LC CDR3) of SEQ ID NO:392;
a heavy chain complementary determining region 1 (HC CDR1) of SEQ ID NO: 319, a heavy chain complementary determining region 2 (HC CDR2) of SEQ ID NO: 333, a heavy chain complementary determining region 3 (HC CDR3) of SEQ ID NO:347 and a light chain complementary determining region 1 (LC CDR1) of SEQ ID NO: 361, a light chain complementary determining region 2 (LC CDR2) of SEQ ID NO:375, and a light chain complementary determining region 3 (LC CDR3) of SEQ ID NO:389;
a heavy chain complementary determining region 1 (HC CDR1) of SEQ ID NO: 323, a heavy chain complementary determining region 2 (HC CDR2) of SEQ ID NO: 337, a heavy chain complementary determining region 3 (HC CDR3) of SEQ ID NO:351 and a light chain complementary determining region 1 (LC CDR1) of SEQ ID NO: 365, a light chain complementary determining region 2 (LC CDR2) of SEQ ID NO:379, and a light chain complementary determining region 3 (LC CDR3) of SEQ ID NO:393;
a heavy chain complementary determining region 1 (HC CDR1) of SEQ ID NO: 324, a heavy chain complementary determining region 2 (HC CDR2) of SEQ ID NO: 338, a heavy chain complementary determining region 3 (HC CDR3) of SEQ ID NO:352 and a light chain complementary determining region 1 (LC CDR1) of SEQ ID NO: 366, a light chain complementary determining region 2 (LC CDR2) of SEQ ID NO:380, and a light chain complementary determining region 3 (LC CDR3) of SEQ ID NO:394;
a heavy chain complementary determining region 1 (HC CDR1) of SEQ ID NO: 325, a heavy chain complementary determining region 2 (HC CDR2) of SEQ ID NO: 339, a heavy chain complementary determining region 3 (HC CDR3) of SEQ ID NO:353 and a light chain complementary determining region 1 (LC CDR1) of SEQ ID NO: 367, a light chain complementary determining region 2 (LC CDR2) of SEQ ID NO:381, and a light chain complementary determining region 3 (LC CDR3) of SEQ ID NO:395;
a heavy chain complementary determining region 1 (HC CDR1) of SEQ ID NO: 314, a heavy chain complementary determining region 2 (HC CDR2) of SEQ ID NO: 328, a heavy chain complementary determining region 3 (HC CDR3) of SEQ ID NO:342 and a light chain complementary determining region 1 (LC CDR1) of SEQ ID NO: 356, a light chain complementary determining region 2 (LC CDR2) of SEQ ID NO:370, and a light chain complementary determining region 3 (LC CDR3) of SEQ ID NO:384;
a heavy chain complementary determining region 1 (HC CDR1) of SEQ ID NO: 315, a heavy chain complementary determining region 2 (HC CDR2) of SEQ ID NO: 329, a heavy chain complementary determining region 3 (HC CDR3) of SEQ ID NO:343 and a light chain complementary determining region 1 (LC CDR1) of SEQ ID NO: 357, a light chain complementary determining region 2 (LC CDR2) of SEQ ID NO:371, and a light chain complementary determining region 3 (LC CDR3) of SEQ ID NO:385;
a heavy chain complementary determining region 1 (HC CDR1) of SEQ ID NO: 316, a heavy chain complementary determining region 2 (HC CDR2) of SEQ ID NO: 330, a heavy chain complementary determining region 3 (HC CDR3) of SEQ ID NO:344 and a light chain complementary determining region 1 (LC CDR1) of SEQ ID NO: 358, a light chain complementary determining region 2 (LC CDR2) of SEQ ID NO:372, and a light chain complementary determining region 3 (LC CDR3) of SEQ ID NO:386;
a heavy chain complementary determining region 1 (HC CDR1) of SEQ ID NO: 317, a heavy chain complementary determining region 2 (HC CDR2) of SEQ ID NO: 331, a heavy chain complementary determining region 3 (HC CDR3) of SEQ ID NO:345 and a light chain complementary determining region 1 (LC CDR1) of SEQ ID NO: 359, a light chain complementary determining region 2 (LC CDR2) of SEQ ID NO:373, and a light chain complementary determining region 3 (LC CDR3) of SEQ ID NO:387;
a heavy chain complementary determining region 1 (HC CDR1) of SEQ ID NO: 318, a heavy chain complementary determining region 2 (HC CDR2) of SEQ ID NO: 332, a heavy chain complementary determining region 3 (HC CDR3) of SEQ ID NO:346 and a light chain complementary determining region 1 (LC CDR1) of SEQ ID NO: 360, a light chain complementary determining region 2 (LC CDR2) of SEQ ID NO:374, and a light chain complementary determining region 3 (LC CDR3) of SEQ ID NO:388;
a heavy chain complementary determining region 1 (HC CDR1) of SEQ ID NO: 320, a heavy chain complementary determining region 2 (HC CDR2) of SEQ ID NO: 334, a heavy chain complementary determining region 3 (HC CDR3) of SEQ ID NO:348 and a light chain complementary determining region 1 (LC CDR1) of SEQ ID NO: 362, a light chain complementary determining region 2 (LC CDR2) of SEQ ID NO:376, and a light chain complementary determining region 3 (LC CDR3) of SEQ ID NO:390;
a heavy chain complementary determining region 1 (HC CDR1) of SEQ ID NO: 321, a heavy chain complementary determining region 2 (HC CDR2) of SEQ ID NO: 335, a heavy chain complementary determining region 3 (HC CDR3) of SEQ ID NO:349 and a light chain complementary determining region 1 (LC CDR1) of SEQ ID NO: 363, a light chain complementary determining region 2 (LC CDR2) of SEQ ID NO:377, and a light chain complementary determining region 3 (LC CDR3) of SEQ ID NO:391;
a heavy chain complementary determining region 1 (HC CDR1) of SEQ ID NO: 326, a heavy chain complementary determining region 2 (HC CDR2) of SEQ ID NO: 340, a heavy chain complementary determining region 3 (HC CDR3) of SEQ ID NO:354 and a light chain complementary determining region 1 (LC CDR1) of SEQ ID NO: 368, a light chain complementary determining region 2 (LC CDR2) of SEQ ID NO:382, and a light chain complementary determining region 3 (LC CDR3) of SEQ ID NO:396;
or
a heavy chain complementary determining region 1 (HC CDR1) of SEQ ID NO: 327, a heavy chain complementary determining region 2 (HC CDR2) of SEQ ID NO: 341, a heavy chain complementary determining region 3 (HC CDR3) of SEQ ID NO:355 and a light chain complementary determining region 1 (LC CDR1) of SEQ ID NO: 369, a light chain complementary determining region 2 (LC CDR2) of SEQ ID NO:383, and a light chain complementary determining region 3 (LC CDR3) of SEQ ID NO:397.

2. The multispecific molecule of claim 1, wherein the anti-CLL-1 binding domain comprises an amino acid sequence of a heavy chain variable region (VH) and an amino acid sequence of a light chain variable region (VL) selected from the group consisting of:
VH: SEQ ID NO: 73 and VL: SEQ ID NO: 86,
VH: SEQ ID NO: 70 and VL: SEQ ID NO: 83,
VH: SEQ ID NO: 74 and VL: SEQ ID NO: 87,
VH: SEQ ID NO: 75 and VL: SEQ ID NO: 88,
VH: SEQ ID NO: 76 and VL: SEQ ID NO: 89,
VH: SEQ ID NO: 65 and VL: SEQ ID NO: 78,
VH: SEQ ID NO: 66 and VL: SEQ ID NO: 79,
VH: SEQ ID NO: 67 and VL: SEQ ID NO: 80,
VH: SEQ ID NO: 68 and VL: SEQ ID NO: 81,
VH: SEQ ID NO: 69 and VL: SEQ ID NO: 82,
VH: SEQ ID NO: 71 and VL: SEQ ID NO: 84,
VH: SEQ ID NO: 72 and VL: SEQ ID NO: 85,
VH: SEQ ID NO: 77 and VL: SEQ ID NO: 90, and
VH: SEQ ID NO: 195 and VL: SEQ ID NO: 196.

3. The multispecific molecule of claim 1 or 2, wherein the anti-CLL-1 binding domain comprises an amino acid sequence of an scFv selected from the group consisting of:
SEQ ID NO: 47,
SEQ ID NO: 44,
SEQ ID NO: 48,
SEQ ID NO: 49,
SEQ ID NO: 50,
SEQ ID NO: 39,
SEQ ID NO: 40,
SEQ ID NO: 41,
SEQ ID NO: 42,
SEQ ID NO: 43,
SEQ ID NO: 45,
SEQ ID NO: 46, and
SEQ ID NO: 51.

4. The multispecific molecule according to any one of claims 1 to 3, wherein the second antigen binding domain binds an immune effector cell antigen which is an antigen expressed on a T cell, wherein the antigen expressed on a T cell is CD3, and wherein the anti-CD3 antigen binding domain comprises:
a heavy chain complementary determining region 1 (HC CDR1) of SEQ ID NO: 1700, a heavy chain complementary determining region 2 (HC CDR2) of SEQ ID NO: 1738, a heavy chain complementary determining region 3 (HC CDR3) of SEQ ID NO:1776; and a light chain complementary determining region 1 (LC CDR1) of SEQ ID NO: 1701, a light chain complementary determining region 2 (LC CDR2) of SEQ ID NO:1739, and a light chain complementary determining region 3 (LC CDR3) of SEQ ID NO:1777;
a heavy chain complementary determining region 1 (HC CDR1) of SEQ ID NO: 1702, a heavy chain complementary determining region 2 (HC CDR2) of SEQ ID NO: 1740, a heavy chain complementary determining region 3 (HC CDR3) of SEQ ID NO: 1778; and a light chain complementary determining region 1 (LC CDR1) of SEQ ID NO: 1703, a light chain complementary determining region 2 (LC CDR2) of SEQ ID NO: 1741, and a light chain complementary determining region 3 (LC CDR3) of SEQ ID NO: 1779;
a heavy chain complementary determining region 1 (HC CDR1) of SEQ ID NO: 1704, a heavy chain complementary determining region 2 (HC CDR2) of SEQ ID NO: 1742 a heavy chain complementary determining region 3 (HC CDR3) of SEQ ID NO: 1780; and a light chain complementary determining region 1 (LC CDR1) of SEQ ID NO: 1705, a light chain complementary determining region 2 (LC CDR2) of SEQ ID NO: 1743, and a light chain complementary determining region 3 (LC CDR3) of SEQ ID NO: 1781;
a heavy chain complementary determining region 1 (HC CDR1) of SEQ ID NO: 1706, a heavy chain complementary determining region 2 (HC CDR2) of SEQ ID NO: 1744, a heavy chain complementary determining region 3 (HC CDR3) of SEQ ID NO: 1782; and a light chain complementary determining region 1 (LC CDR1) of SEQ ID NO: 1707, a light chain complementary determining region 2 (LC CDR2) of SEQ ID NO: 1745, and a light chain complementary determining region 3 (LC CDR3) of SEQ ID NO: 1783;
a heavy chain complementary determining region 1 (HC CDR1) of SEQ ID NO: 1708, a heavy chain complementary determining region 2 (HC CDR2) of SEQ ID NO: 1746, a heavy chain complementary determining region 3 (HC CDR3) of SEQ ID NO: 1784; and a light chain complementary determining region 1 (LC CDR1) of SEQ ID NO: 1709, a light chain complementary determining region 2 (LC CDR2) of SEQ ID NO: 1747, and a light chain complementary determining region 3 (LC CDR3) of SEQ ID NO: 1785;
a heavy chain complementary determining region 1 (HC CDR1) of SEQ ID NO: 1710, a heavy chain complementary determining region 2 (HC CDR2) of SEQ ID NO: 1748, a heavy chain complementary determining region 3 (HC CDR3) of SEQ ID NO: 1786; and a light chain complementary determining region 1 (LC CDR1) of SEQ ID NO: 1711, a light chain complementary determining region 2 (LC CDR2) of SEQ ID NO: 1749, and a light chain complementary determining region 3 (LC CDR3) of SEQ ID NO: 1787;
a heavy chain complementary determining region 1 (HC CDR1) of SEQ ID NO: 1712, a heavy chain complementary determining region 2 (HC CDR2) of SEQ ID NO: 1750, a heavy chain complementary determining region 3 (HC CDR3) of SEQ ID NO: 1788; and a light chain complementary determining region 1 (LC CDR1) of SEQ ID NO: 1713, a light chain complementary determining region 2 (LC CDR2) of SEQ ID NO: 1751, and a light chain complementary determining region 3 (LC CDR3) of SEQ ID NO: 1789;
a heavy chain complementary determining region 1 (HC CDR1) of SEQ ID NO: 1714, a heavy chain complementary determining region 2 (HC CDR2) of SEQ ID NO: 1752, a heavy chain complementary determining region 3 (HC CDR3) of SEQ ID NO: 1790; and a light chain complementary determining region 1 (LC CDR1) of SEQ ID NO: 1715, a light chain complementary determining region 2 (LC CDR2) of SEQ ID NO: 1753, and a light chain complementary determining region 3 (LC CDR3) of SEQ ID NO: 1791;
a heavy chain complementary determining region 1 (HC CDR1) of SEQ ID NO: 1716, a heavy chain complementary determining region 2 (HC CDR2) of SEQ ID NO: 1754, a heavy chain complementary determining region 3 (HC CDR3) of SEQ ID NO: 1792; and a light chain complementary determining region 1 (LC CDR1) of SEQ ID NO: 1717, a light chain complementary determining region 2 (LC CDR2) of SEQ ID NO: 1755, and a light chain complementary determining region 3 (LC CDR3) of SEQ ID NO: 1793;
a heavy chain complementary determining region 1 (HC CDR1) of SEQ ID NO: 1718, a heavy chain complementary determining region 2 (HC CDR2) of SEQ ID NO: 1756, a heavy chain complementary determining region 3 (HC CDR3) of SEQ ID NO: 1794; and a light chain complementary determining region 1 (LC CDR1) of SEQ ID NO: 1719, a light chain complementary determining region 2 (LC CDR2) of SEQ ID NO: 1757, and a light chain complementary determining region 3 (LC CDR3) of SEQ ID NO: 1795;
a heavy chain complementary determining region 1 (HC CDR1) of SEQ ID NO: 1720, a heavy chain complementary determining region 2 (HC CDR2) of SEQ ID NO: 1758, a heavy chain complementary determining region 3 (HC CDR3) of SEQ ID NO: 1796; and a light chain complementary determining region 1 (LC CDR1) of SEQ ID NO: 1721, a light chain complementary determining region 2 (LC CDR2) of SEQ ID NO: 1759, and a light chain complementary determining region 3 (LC CDR3) of SEQ ID NO: 1797;
a heavy chain complementary determining region 1 (HC CDR1) of SEQ ID NO: 1722, a heavy chain complementary determining region 2 (HC CDR2) of SEQ ID NO: 1760, a heavy chain complementary determining region 3 (HC CDR3) of SEQ ID NO: 1798; and a light chain complementary determining region 1 (LC CDR1) of SEQ ID NO: 1723, a light chain complementary determining region 2 (LC CDR2) of SEQ ID NO: 1761, and a light chain complementary determining region 3 (LC CDR3) of SEQ ID NO: 1799;
a heavy chain complementary determining region 1 (HC CDR1) of SEQ ID NO: 1724, a heavy chain complementary determining region 2 (HC CDR2) of SEQ ID NO: 1762, a heavy chain complementary determining region 3 (HC CDR3) of SEQ ID NO: 1800; and a light chain complementary determining region 1 (LC CDR1) of SEQ ID NO: 1725, a light chain complementary determining region 2 (LC CDR2) of SEQ ID NO: 1763, and a light chain complementary determining region 3 (LC CDR3) of SEQ ID NO: 1801;
a heavy chain complementary determining region 1 (HC CDR1) of SEQ ID NO: 1726, a heavy chain complementary determining region 2 (HC CDR2) of SEQ ID NO: 1764, a heavy chain complementary determining region 3 (HC CDR3) of SEQ ID NO: 1802; and a light chain complementary determining region 1 (LC CDR1) of SEQ ID NO: 1727, a light chain complementary determining region 2 (LC CDR2) of SEQ ID NO: 1765, and a light chain complementary determining region 3 (LC CDR3) of SEQ ID NO: 1803;
a heavy chain complementary determining region 1 (HC CDR1) of SEQ ID NO: 1728, a heavy chain complementary determining region 2 (HC CDR2) of SEQ ID NO: 1766, a heavy chain complementary determining region 3 (HC CDR3) of SEQ ID NO: 1804; and a light chain complementary determining region 1 (LC CDR1) of SEQ ID NO: 1729, a light chain complementary determining region 2 (LC CDR2) of SEQ ID NO: 1767, and a light chain complementary determining region 3 (LC CDR3) of SEQ ID NO: 1805;
a heavy chain complementary determining region 1 (HC CDR1) of SEQ ID NO: 1730, a heavy chain complementary determining region 2 (HC CDR2) of SEQ ID NO: 1768, a heavy chain complementary determining region 3 (HC CDR3) of SEQ ID NO: 1806; and a light chain complementary determining region 1 (LC CDR1) of SEQ ID NO: 1731, a light chain complementary determining region 2 (LC CDR2) of SEQ ID NO: 1769, and a light chain complementary determining region 3 (LC CDR3) of SEQ ID NO: 1807;
a heavy chain complementary determining region 1 (HC CDR1) of SEQ ID NO: 1732, a heavy chain complementary determining region 2 (HC CDR2) of SEQ ID NO: 1770, a heavy chain complementary determining region 3 (HC CDR3) of SEQ ID NO: 1808; and a light chain complementary determining region 1 (LC CDR1) of SEQ ID NO: 1733, a light chain complementary determining region 2 (LC CDR2) of SEQ ID NO: 1771, and a light chain complementary determining region 3 (LC CDR3) of SEQ ID NO: 1809;
a heavy chain complementary determining region 1 (HC CDR1) of SEQ ID NO: 1734, a heavy chain complementary determining region 2 (HC CDR2) of SEQ ID NO: 1772, a heavy chain complementary determining region 3 (HC CDR3) of SEQ ID NO: 1810; and a light chain complementary determining region 1 (LC CDR1) of SEQ ID NO: 1735, a light chain complementary determining region 2 (LC CDR2) of SEQ ID NO: 1773, and a light chain complementary determining region 3 (LC CDR3) of SEQ ID NO: 1811;
or
a heavy chain complementary determining region 1 (HC CDR1) of SEQ ID NO: 1736, a heavy chain complementary determining region 2 (HC CDR2) of SEQ ID NO: 1774, a heavy chain complementary determining region 3 (HC CDR3) of SEQ ID NO: 1812 and a light chain complementary determining region 1 (LC CDR1) of SEQ ID NO: 1737, a light chain complementary determining region 2 (LC CDR2) of SEQ ID NO: 1775, and a light chain complementary determining region 3 (LC CDR3) of SEQ ID NO: 1813.

5. The multispecific molecule according to claim 4, wherein the anti-CD3 antigen binding domain comprises an amino acid sequence of a heavy chain variable region (VH) and a amino acid sequence of the light chain variable region (VL) selected from the group consisting of:
VH: SEQ ID NO: 1200 and VL: SEQ ID NO: 1201,
VH: SEQ ID NO: 1202 and VL: SEQ ID NO: 1203,
VH: SEQ ID NO: 1204 and VL: SEQ ID NO: 1205,
VH: SEQ ID NO: 1206 and VL: SEQ ID NO: 1207,
VH: SEQ ID NO: 1208 and VL: SEQ ID NO: 1209,
VH: SEQ ID NO: 1210 and VL: SEQ ID NO: 1211,
VH: SEQ ID NO: 1212 and VL: SEQ ID NO: 1213,
VH: SEQ ID NO: 1214 and VL: SEQ ID NO:1215,
VH: SEQ ID NO: 1216 and VL: SEQ ID NO: 1217,
VH: SEQ ID NO: 1218 and VL: SEQ ID NO: 1219,
VH: SEQ ID NO: 1220 and VL: SEQ ID NO: 1221,
VH: SEQ ID NO: 1222 and VL: SEQ ID NO: 1223,
VH: SEQ ID NO: 1224 and VL: SEQ ID NO: 1225,
VH: SEQ ID NO: 1226 and VL: SEQ ID NO: 1227,
VH: SEQ ID NO: 1230 and VL: SEQ ID NO: 1231,
VH: SEQ ID NO: 1232 and VL: SEQ ID NO: 1233,
VH: SEQ ID NO: 1234 and VL: SEQ ID NO: 1235, and
VH: SEQ ID NO: 1236 and VL: SEQ ID NO: 1237.

6. The multispecific molecule according to any one of claims 1 to 5, wherein the second antigen binding domain binds CD3 and comprises a VL sequence of SEQ ID NO:1209 and a VH sequence of SEQ ID NO: 1236; and wherein the anti-CLL-1 binding domain comprises:
a VL sequence of SEQ ID NO: 88 and a VH sequence of SEQ ID NO: 75,
a VL sequence of SEQ ID NO: 89 and a VH sequence of SEQ ID NO: 76,
a VL sequence of SEQ ID NO: 87 and a VH sequence of SEQ ID NO: 74,
a VL sequence of SEQ ID NO: 85 and a VH sequence of SEQ ID NO: 72,
a VL sequence of SEQ ID NO: 86 and a VH sequence of SEQ ID NO: 73,
a VL sequence of SEQ ID NO: 83 and a VH sequence of SEQ ID NO: 70,
a VL sequence of SEQ ID NO: 90 and a VH sequence of SEQ ID NO: 77,
a VL sequence of SEQ ID NO: 79 and a VH sequence of SEQ ID NO: 66, or
a VL sequence of SEQ ID NO: 84 and a VH sequence of SEQ ID NO: 71.

7. The multispecific molecule according to any one of claims 1 to 6, wherein the second antigen binding domain binds CD3 and comprises SEQ ID NO:506; and wherein the anti-CLL-1 binding domain comprises:
SEQ ID NO: 49,
SEQ ID NO: 50,
SEQ ID NO: 48,
SEQ ID NO: 46,
SEQ ID NO: 47,
SEQ ID NO: 44,
SEQ ID NO: 51,
SEQ ID NO: 40, or
SEQ ID NO: 45.

8. The multispecific molecule according to any one of claims 1 to 7, wherein the polypeptide comprising the second antigen binding domain binds CD3 and comprises SEQ ID NO:507; and wherein the polypeptide comprising the anti-CLL-1 binding domain comprises:
SEQ ID NO: 512,
SEQ ID NO: 517,
SEQ ID NO: 522,
SEQ ID NO: 527,
SEQ ID NO: 532,
SEQ ID NO: 537,
SEQ ID NO: 542,
SEQ ID NO: 547, or
SEQ ID NO: 552.

9. The multispecific molecule according to any one of claims 1 to 8, wherein the polypeptides comprising the heavy chain CDR sequences of the first and/or second antigen binding domain further comprises a CH3 domain, and optionally a CH2 domain.

10. The multispecific molecule of claim 9, wherein the polypeptides comprising the heavy chain CDR sequences of the first and second antigen binding domain further comprises a CH3 domain, wherein both of said CH3 domains comprise one or more modifications to enhance heterodimerization, wherein such modifications comprise introduction of a knob in a first CH3 domain and a hole in a second CH3 domain, or vice versa, such that heterodimerization of the polypeptide comprising the first CH3 domain and the polypeptide comprising the second CH3 domain is favored relative to polypeptides comprising unmodified CH3 domains.

11. An isolated nucleic acid encoding the multispecific molecule of any one of claims 1 to 10.

12. The isolated nucleic acid according to claim 11 comprising:
SEQ ID NO: 508 and SEQ ID NO: 509,
SEQ ID NO: 513 and SEQ ID NO: 514,
SEQ ID NO: 518 and SEQ ID NO: 519,
SEQ ID NO: 523 and SEQ ID NO: 524,
SEQ ID NO: 528 and SEQ ID NO: 529,
SEQ ID NO: 533 and SEQ ID NO: 534,
SEQ ID NO: 538 and SEQ ID NO: 539,
SEQ ID NO: 543 and SEQ ID NO: 544,
SEQ ID NO: 548 and SEQ ID NO: 549,
SEQ ID NO: 553 and SEQ ID NO: 554,
SEQ ID NO: 515,
SEQ ID NO: 520,
SEQ ID NO: 525,
SEQ ID NO: 530,
SEQ ID NO: 535,
SEQ ID NO: 540,
SEQ ID NO: 545,
SEQ ID NO: 550,
SEQ ID NO: 555,
SEQ ID NO: 516,
SEQ ID NO: 521,
SEQ ID NO: 526,
SEQ ID NO: 531,
SEQ ID NO: 536,
SEQ ID NO: 541,
SEQ ID NO: 546,
SEQ ID NO: 551, or
SEQ ID NO: 556.

13. A vector comprising the isolated nucleic acid of claim 11 or 12.

14. The multispecific molecule of any one of claims 1 to 10 for use as a medicament, or for use in a therapy, or for use in treating a subject having a hematologic cancer or for use in treating a subject having acute myeloid leukemia (AML).

15. A pharmaceutical composition comprising the multispecific molecule of any one of claims 1 to 10 and a pharmaceutically acceptable diluent or carrier.

## Patentansprüche

1. Multispezifisches Molekül, umfassend eine erste Antigenbindungsdomäne und eine zweite Antigenbindungsdomäne, wobei die zweite Antigenbindungsdomäne ein Immuneffektorzellantigen bindet, bei dem es sich um ein auf einer T-Zelle exprimiertes Antigen handelt, wobei das auf einer T-Zelle exprimierte Antigen CD3 ist, und wobei die erste Antigenbindungsdomäne eine Anti-CLL-1-Bindungsdomäne ist, umfassend:
eine komplementaritätsbestimmende Region 1 der schweren Kette (HC CDR1) von SEQ ID NO:322,
eine komplementaritätsbestimmende Region 2 der schweren Kette (HC CDR2) von SEQ ID NO:336,
eine komplementaritätsbestimmende Region 3 der schweren Kette (HC CDR3) von SEQ ID NO:350 und
eine komplementaritätsbestimmende Region 1 der leichten Kette (LC CDR1) von SEQ ID NO:364,
eine komplementaritätsbestimmende Region 2 der leichten Kette (LC CDR2) von SEQ ID NO:378 und
eine komplementaritätsbestimmende Region 3 der leichten Kette (LC CDR3) von SEQ ID NO:392;
eine komplementaritätsbestimmende Region 1 der schweren Kette (HC CDR1) von SEQ ID NO:319,
eine komplementaritätsbestimmende Region 2 der schweren Kette (HC CDR2) von SEQ ID NO:333,
eine komplementaritätsbestimmende Region 3 der schweren Kette (HC CDR3) von SEQ ID NO:347 und
eine komplementaritätsbestimmende Region 1 der leichten Kette (LC CDR1) von SEQ ID NO:361,
eine komplementaritätsbestimmende Region 2 der leichten Kette (LC CDR2) von SEQ ID NO:375 und
eine komplementaritätsbestimmende Region 3 der leichten Kette (LC CDR3) von SEQ ID NO:389;
eine komplementaritätsbestimmende Region 1 der schweren Kette (HC CDR1) von SEQ ID NO:323,
eine komplementaritätsbestimmende Region 2 der schweren Kette (HC CDR2) von SEQ ID NO:337,
eine komplementaritätsbestimmende Region 3 der schweren Kette (HC CDR3) von SEQ ID NO:351 und
eine komplementaritätsbestimmende Region 1 der leichten Kette (LC CDR1) von SEQ ID NO:365,
eine komplementaritätsbestimmende Region 2 der leichten Kette (LC CDR2) von SEQ ID NO:379 und
eine komplementaritätsbestimmende Region 3 der leichten Kette (LC CDR3) von SEQ ID NO:393;
eine komplementaritätsbestimmende Region 1 der schweren Kette (HC CDR1) von SEQ ID NO:324,
eine komplementaritätsbestimmende Region 2 der schweren Kette (HC CDR2) von SEQ ID NO:338,
eine komplementaritätsbestimmende Region 3 der schweren Kette (HC CDR3) von SEQ ID NO:352 und
eine komplementaritätsbestimmende Region 1 der leichten Kette (LC CDR1) von SEQ ID NO:366,
eine komplementaritätsbestimmende Region 2 der leichten Kette (LC CDR2) von SEQ ID NO:380 und
eine komplementaritätsbestimmende Region 3 der leichten Kette (LC CDR3) von SEQ ID NO:394;
eine komplementaritätsbestimmende Region 1 der schweren Kette (HC CDR1) von SEQ ID NO:325,
eine komplementaritätsbestimmende Region 2 der schweren Kette (HC CDR2) von SEQ ID NO:339,
eine komplementaritätsbestimmende Region 3 der schweren Kette (HC CDR3) von SEQ ID NO:353 und
eine komplementaritätsbestimmende Region 1 der leichten Kette (LC CDR1) von SEQ ID NO:367,
eine komplementaritätsbestimmende Region 2 der leichten Kette (LC CDR2) von SEQ ID NO:381 und
eine komplementaritätsbestimmende Region 3 der leichten Kette (LC CDR3) von SEQ ID NO:395;
eine komplementaritätsbestimmende Region 1 der schweren Kette (HC CDR1) von SEQ ID NO:314,
eine komplementaritätsbestimmende Region 2 der schweren Kette (HC CDR2) von SEQ ID NO:328,
eine komplementaritätsbestimmende Region 3 der schweren Kette (HC CDR3) von SEQ ID NO:342 und
eine komplementaritätsbestimmende Region 1 der leichten Kette (LC CDR1) von SEQ ID NO:356,
eine komplementaritätsbestimmende Region 2 der leichten Kette (LC CDR2) von SEQ ID NO:370 und
eine komplementaritätsbestimmende Region 3 der leichten Kette (LC CDR3) von SEQ ID NO:384;
eine komplementaritätsbestimmende Region 1 der schweren Kette (HC CDR1) von SEQ ID NO:315,
eine komplementaritätsbestimmende Region 2 der schweren Kette (HC CDR2) von SEQ ID NO:329,
eine komplementaritätsbestimmende Region 3 der schweren Kette (HC CDR3) von SEQ ID NO:343 und
eine komplementaritätsbestimmende Region 1 der leichten Kette (LC CDR1) von SEQ ID NO:357,
eine komplementaritätsbestimmende Region 2 der leichten Kette (LC CDR2) von SEQ ID NO:371 und
eine komplementaritätsbestimmende Region 3 der leichten Kette (LC CDR3) von SEQ ID NO:385;
eine komplementaritätsbestimmende Region 1 der schweren Kette (HC CDR1) von SEQ ID NO:316,
eine komplementaritätsbestimmende Region 2 der schweren Kette (HC CDR2) von SEQ ID NO:330,
eine komplementaritätsbestimmende Region 3 der schweren Kette (HC CDR3) von SEQ ID NO:344 und
eine komplementaritätsbestimmende Region 1 der leichten Kette (LC CDR1) von SEQ ID NO:358,
eine komplementaritätsbestimmende Region 2 der leichten Kette (LC CDR2) von SEQ ID NO:372 und
eine komplementaritätsbestimmende Region 3 der leichten Kette (LC CDR3) von SEQ ID NO:386;
eine komplementaritätsbestimmende Region 1 der schweren Kette (HC CDR1) von SEQ ID NO:317,
eine komplementaritätsbestimmende Region 2 der schweren Kette (HC CDR2) von SEQ ID NO:331,
eine komplementaritätsbestimmende Region 3 der schweren Kette (HC CDR3) von SEQ ID NO:345 und
eine komplementaritätsbestimmende Region 1 der leichten Kette (LC CDR1) von SEQ ID NO:359,
eine komplementaritätsbestimmende Region 2 der leichten Kette (LC CDR2) von SEQ ID NO:373 und
eine komplementaritätsbestimmende Region 3 der leichten Kette (LC CDR3) von SEQ ID NO:387;
eine komplementaritätsbestimmende Region 1 der schweren Kette (HC CDR1) von SEQ ID NO:318,
eine komplementaritätsbestimmende Region 2 der schweren Kette (HC CDR2) von SEQ ID NO:332,
eine komplementaritätsbestimmende Region 3 der schweren Kette (HC CDR3) von SEQ ID NO:346 und
eine komplementaritätsbestimmende Region 1 der leichten Kette (LC CDR1) von SEQ ID NO:360,
eine komplementaritätsbestimmende Region 2 der leichten Kette (LC CDR2) von SEQ ID NO:374 und
eine komplementaritätsbestimmende Region 3 der leichten Kette (LC CDR3) von SEQ ID NO:388;
eine komplementaritätsbestimmende Region 1 der schweren Kette (HC CDR1) von SEQ ID NO:320,
eine komplementaritätsbestimmende Region 2 der schweren Kette (HC CDR2) der SEQ ID NO:334,
eine komplementaritätsbestimmende Region 3 der schweren Kette (HC CDR3) von SEQ ID NO:348 und
eine komplementaritätsbestimmende Region 1 der leichten Kette (LC CDR1) von SEQ ID NO:362,
eine komplementaritätsbestimmende Region 2 der leichten Kette (LC CDR2) von SEQ ID NO:376 und
eine komplementaritätsbestimmende Region 3 der leichten Kette (LC CDR3) von SEQ ID NO:390;
eine komplementaritätsbestimmende Region 1 der schweren Kette (HC CDR1) von SEQ ID NO:321,
eine komplementaritätsbestimmende Region 2 der schweren Kette (HC CDR2) von SEQ ID NO:335,
eine komplementaritätsbestimmende Region 3 der schweren Kette (HC CDR3) von SEQ ID NO:349 und
eine komplementaritätsbestimmende Region 1 der leichten Kette (LC CDR1) von SEQ ID NO:363,
eine komplementaritätsbestimmende Region 2 der leichten Kette (LC CDR2) von SEQ ID NO:377 und
eine komplementaritätsbestimmende Region 3 der leichten Kette (LC CDR3) von SEQ ID NO:391:
eine komplementaritätsbestimmende Region 1 der schweren Kette (HC CDR1) von SEQ ID NO:326,
eine komplementaritätsbestimmende Region 2 der schweren Kette (HC CDR2) von SEQ ID NO:340,
eine komplementaritätsbestimmende Region 3 der schweren Kette (HC CDR3) von SEQ ID NO:354 und
eine komplementaritätsbestimmende Region 1 der leichten Kette (LC CDR1) von SEQ ID NO:368,
eine komplementaritätsbestimmende Region 2 der leichten Kette (LC CDR2) von SEQ ID NO:382 und
eine komplementaritätsbestimmende Region 3 der leichten Kette (LC CDR3) von SEQ ID NO:396;
oder
eine komplementaritätsbestimmende Region 1 der schweren Kette (HC CDR1) von SEQ ID NO:327,
eine komplementaritätsbestimmende Region 2 der schweren Kette (HC CDR2) von SEQ ID NO:341,
eine komplementaritätsbestimmende Region 3 der schweren Kette (HC CDR3) von SEQ ID NO:355 und
eine komplementaritätsbestimmende Region 1 der leichten Kette (LC CDR1) von SEQ ID NO:369,
eine komplementaritätsbestimmende Region 2 der leichten Kette (LC CDR2) von SEQ ID NO:383 und
eine komplementaritätsbestimmende Region 3 der leichten Kette (LC CDR3) von SEQ ID NO:397.

2. Multispezifisches Molekül nach Anspruch 1, wobei die Anti-CLL-1-Bindungsdomäne eine Aminosäuresequenz einer variablen Region der schweren Kette (VH) und eine Aminosäuresequenz einer variablen Region der leichten Kette (VL) umfasst, ausgewählt aus der Gruppe bestehend aus:
VH: SEQ ID NO:73 und VL: SEQ ID NO:86,
VH: SEQ ID NO:70 und VL: SEQ ID NO:83,
VH: SEQ ID NO:74 und VL: SEQ ID NO:87,
VH: SEQ ID NO:75 und VL: SEQ ID NO:88,
VH: SEQ ID NO:76 und VL: SEQ ID NO:89,
VH: SEQ ID NO:65 und VL: SEQ ID NO:78,
VH: SEQ ID NO:66 und VL: SEQ ID NO:79,
VH: SEQ ID NO:67 und VL: SEQ ID NO:80,
VH: SEQ ID NO:68 und VL: SEQ ID NO:81,
VH: SEQ ID NO:69 und VL: SEQ ID NO:82,
VH: SEQ ID NO:71 und VL: SEQ ID NO:84,
VH: SEQ ID NO:72 und VL: SEQ ID NO:85,
VH: SEQ ID NO:77 und VL: SEQ ID NO:90, und
VH: SEQ ID NO:195 und VL: SEQ ID NO:196.

3. Multispezifisches Molekül nach Anspruch 1 oder 2, wobei die Anti-CLL-1-Bindungsdomäne eine Aminosäuresequenz eines scFv umfasst, ausgewählt aus der Gruppe bestehend aus:
SEQ ID NO:47,
SEQ ID NO:44,
SEQ ID NO:48,
SEQ ID NO:49,
SEQ ID NO:50,
SEQ ID NO:39,
SEQ ID NO:40,
SEQ ID NO:41,
SEQ ID NO:42,
SEQ ID NO:43,
SEQ ID NO:45,
SEQ ID NO:46 und
SEQ ID NO:51.

4. Multispezifisches Molekül nach einem der Ansprüche 1 bis 3, wobei die zweite Antigenbindungsdomäne ein Immuneffektorzellantigen bindet, bei dem es sich um ein auf einer T-Zelle exprimiertes Antigen handelt, wobei das auf einer T-Zelle exprimierte Antigen CD3 ist und wobei die Anti-CD3-Antigenbindungsdomäne umfasst:
eine komplementaritätsbestimmende Region 1 der schweren Kette (HC CDR1) von SEQ ID NO:1700,
eine komplementaritätsbestimmende Region 2 der schweren Kette (HC CDR2) von SEQ ID NO:1738,
eine komplementaritätsbestimmende Region 3 der schweren Kette (HC CDR3) von SEQ ID NO:1776; und
eine komplementaritätsbestimmende Region 1 der leichten Kette (LC CDR1) von SEQ ID NO:1701,
eine komplementaritätsbestimmende Region 2 der leichten Kette (LC CDR2) von SEQ ID NO:1739 und
eine komplementaritätsbestimmende Region 3 der leichten Kette (LC CDR3) von SEQ ID NO:1777;
eine komplementaritätsbestimmende Region 1 der schweren Kette (HC CDR1) von SEQ ID NO:1702,
eine komplementaritätsbestimmende Region 2 der schweren Kette (HC CDR2) von SEQ ID NO:1740,
eine komplementaritätsbestimmende Region 3 der schweren Kette (HC CDR3) von SEQ ID NO:1778; und
eine komplementaritätsbestimmende Region 1 der leichten Kette (LC CDR1) von SEQ ID NO:1703,
eine komplementaritätsbestimmende Region 2 der leichten Kette (LC CDR2) von SEQ ID NO:1741 und
eine komplementaritätsbestimmende Region 3 der leichten Kette (LC CDR3) von SEQ ID NO:1779;
eine komplementaritätsbestimmende Region 1 der schweren Kette (HC CDR1) von SEQ ID NO:1704,
eine komplementaritätsbestimmende Region 2 der schweren Kette (HC CDR2) von SEQ ID NO:1742,
eine komplementaritätsbestimmende Region 3 der schweren Kette (HC CDR3) von SEQ ID NO:1780; und
eine komplementaritätsbestimmende Region 1 der leichten Kette (LC CDR1) von SEQ ID NO:1705,
eine komplementaritätsbestimmende Region 2 der leichten Kette (LC CDR2) von SEQ ID NO:1743 und
eine komplementaritätsbestimmende Region 3 der leichten Kette (LC CDR3) von SEQ ID NO:1781;
eine komplementaritätsbestimmende Region 1 der schweren Kette (HC CDR1) von SEQ ID NO:1706,
eine komplementaritätsbestimmende Region 2 der schweren Kette (HC CDR2) von SEQ ID NO:1744,
eine komplementaritätsbestimmende Region 3 der schweren Kette (HC CDR3) von SEQ ID NO:1782; und
eine komplementaritätsbestimmende Region 1 der leichten Kette (LC CDR1) von SEQ ID NO:1707,
eine komplementaritätsbestimmende Region 2 der leichten Kette (LC CDR2) von SEQ ID NO:1745 und
eine komplementaritätsbestimmende Region 3 der leichten Kette (LC CDR3) von SEQ ID NO:1783;
eine komplementaritätsbestimmende Region 1 der schweren Kette (HC CDR1) von SEQ ID NO:1708,
eine komplementaritätsbestimmende Region 2 der schweren Kette (HC CDR2) von SEQ ID NO:1746,
eine komplementaritätsbestimmende Region 3 der schweren Kette (HC CDR3) von SEQ ID NO:1784 und
eine komplementaritätsbestimmende Region 1 der leichten Kette (LC CDR1) von SEQ ID NO:1709,
eine komplementaritätsbestimmende Region 2 der leichten Kette (LC CDR2) von SEQ ID NO:1747 und
eine komplementaritätsbestimmende Region 3 der leichten Kette (LC CDR3) von SEQ ID NO:1785;
eine komplementaritätsbestimmende Region 1 der schweren Kette (HC CDR1) von SEQ ID NO:1710,
eine komplementaritätsbestimmende Region 2 der schweren Kette (HC CDR2) von SEQ ID NO:1748,
eine komplementaritätsbestimmende Region 3 der schweren Kette (HC CDR3) von SEQ ID NO:1786; und
eine komplementaritätsbestimmende Region 1 der leichten Kette (LC CDR1) von SEQ ID NO:1711,
eine komplementaritätsbestimmende Region 2 der leichten Kette (LC CDR2) von SEQ ID NO:1749 und
eine komplementaritätsbestimmende Region 3 der leichten Kette (LC CDR3) von SEQ ID NO:1787;
eine komplementaritätsbestimmende Region 1 der schweren Kette (HC CDR1) von SEQ ID NO:1712,
eine komplementaritätsbestimmende Region 2 der schweren Kette (HC CDR2) von SEQ ID NO:1750,
eine komplementaritätsbestimmende Region 3 der schweren Kette (HC CDR3) von SEQ ID NO:1788; und
eine komplementaritätsbestimmende Region 1 der leichten Kette (LC CDR1) von SEQ ID NO:1713,
eine komplementaritätsbestimmende Region 2 der leichten Kette (LC CDR2) von SEQ ID NO:1751 und
eine komplementaritätsbestimmende Region 3 der leichten Kette (LC CDR3) von SEQ ID NO:1789;
eine komplementaritätsbestimmende Region 1 der schweren Kette (HC CDR1) von SEQ ID NO:1714,
eine komplementaritätsbestimmende Region 2 der schweren Kette (HC CDR2) von SEQ ID NO:1752,
eine komplementaritätsbestimmende Region 3 der schweren Kette (HC CDR3) von SEQ ID NO:1790; und
eine komplementaritätsbestimmende Region 1 der leichten Kette (LC CDR1) von SEQ ID NO:1715,
eine komplementaritätsbestimmende Region 2 der leichten Kette (LC CDR2) von SEQ ID NO:1753 und
eine komplementaritätsbestimmende Region 3 der leichten Kette (LC CDR3) von SEQ ID NO:1791;
eine komplementaritätsbestimmende Region 1 der schweren Kette (HC CDR1) von SEQ ID NO:1716,
eine komplementaritätsbestimmende Region 2 der schweren Kette (HC CDR2) von SEQ ID NO:1754,
eine komplementaritätsbestimmende Region 3 der schweren Kette (HC CDR3) von SEQ ID NO:1792; und
eine komplementaritätsbestimmende Region 1 der leichten Kette (LC CDR1) von SEQ ID NO:1717,
eine komplementaritätsbestimmende Region 2 der leichten Kette (LC CDR2) von SEQ ID NO:1755 und
eine komplementaritätsbestimmende Region 3 der leichten Kette (LC CDR3) von SEQ ID NO:1793;
eine komplementaritätsbestimmende Region 1 der schweren Kette (HC CDR1) von SEQ ID NO:1718,
eine komplementaritätsbestimmende Region 2 der schweren Kette (HC CDR2) von SEQ ID NO:1756,
eine komplementaritätsbestimmende Region 3 der schweren Kette (HC CDR3) von SEQ ID NO:1794; und
eine komplementaritätsbestimmende Region 1 der leichten Kette (LC CDR1) von SEQ ID NO:1719,
eine komplementaritätsbestimmende Region 2 der leichten Kette (LC CDR2) von SEQ ID NO:1757 und
eine komplementaritätsbestimmende Region 3 der leichten Kette (LC CDR3) von SEQ ID NO:1795;
eine komplementaritätsbestimmende Region 1 der schweren Kette (HC CDR1) von SEQ ID NO:1720,
eine komplementaritätsbestimmende Region 2 der schweren Kette (HC CDR2) von SEQ ID NO:1758,
eine komplementaritätsbestimmende Region 3 der schweren Kette (HC CDR3) von SEQ ID NO:1796; und
eine komplementaritätsbestimmende Region 1 der leichten Kette (LC CDR1) von SEQ ID NO: 1721,
eine komplementaritätsbestimmende Region 2 der leichten Kette (LC CDR2) der SEQ ID NO: 1759 und
eine komplementaritätsbestimmende Region 3 der leichten Kette (LC CDR3) von SEQ ID NO: 1797;
eine komplementaritätsbestimmende Region 1 der schweren Kette (HC CDR1) von SEQ ID NO: 1722,
eine komplementaritätsbestimmende Region 2 der schweren Kette (HC CDR2) von SEQ ID NO: 1760,
eine komplementaritätsbestimmende Region 3 der schweren Kette (HC CDR3) von SEQ ID NO: 1798; und
eine komplementaritätsbestimmende Region 1 der leichten Kette (LC CDR1) von SEQ ID NO: 1723,
eine komplementaritätsbestimmende Region 2 der leichten Kette (LC CDR2) von SEQ ID NO: 1761 und
eine komplementaritätsbestimmende Region 3 der leichten Kette (LC CDR3) von SEQ ID NO: 1799;
eine komplementaritätsbestimmende Region 1 der schweren Kette (HC CDR1) von SEQ ID NO: 1724,
eine komplementaritätsbestimmende Region 2 der schweren Kette (HC CDR2) von SEQ ID NO: 1762,
eine komplementaritätsbestimmende Region 3 der schweren Kette (HC CDR3) von SEQ ID NO: 1800; und
eine komplementaritätsbestimmende Region 1 der leichten Kette (LC CDR1) von SEQ ID NO: 1725,
eine komplementaritätsbestimmende Region 2 der leichten Kette (LC CDR2) von SEQ ID NO: 1763 und
eine komplementaritätsbestimmende Region 3 der leichten Kette (LC CDR3) von SEQ ID NO: 1801;
eine komplementaritätsbestimmende Region 1 der schweren Kette (HC CDR1) von SEQ ID NO: 1726,
eine komplementaritätsbestimmende Region 2 der schweren Kette (HC CDR2) von SEQ ID NO: 1764,
eine komplementaritätsbestimmende Region 3 der schweren Kette (HC CDR3) von SEQ ID NO: 1802; und
eine komplementaritätsbestimmende Region 1 der leichten Kette (LC CDR1) von SEQ ID NO: 1727,
eine komplementaritätsbestimmende Region 2 der leichten Kette (LC CDR2) von SEQ ID NO: 1765 und
eine komplementaritätsbestimmende Region 3 der leichten Kette (LC CDR3) von SEQ ID NO: 1803;
eine komplementaritätsbestimmende Region 1 der schweren Kette (HC CDR1) von SEQ ID NO: 1728,
eine komplementaritätsbestimmende Region 2 der schweren Kette (HC CDR2) von SEQ ID NO: 1766,
eine komplementaritätsbestimmende Region 3 der schweren Kette (HC CDR3) von SEQ ID NO: 1804; und
eine komplementaritätsbestimmende Region 1 der leichten Kette (LC CDR1) von SEQ ID NO: 1729,
eine komplementaritätsbestimmende Region 2 der leichten Kette (LC CDR2) von SEQ ID NO: 1767 und
eine komplementaritätsbestimmende Region 3 der leichten Kette (LC CDR3) von SEQ ID NO: 1805;
eine komplementaritätsbestimmende Region 1 der schweren Kette (HC CDR1) von SEQ ID NO: 1730,
eine komplementaritätsbestimmende Region 2 der schweren Kette (HC CDR2) von SEQ ID NO: 1768,
eine komplementaritätsbestimmende Region 3 der schweren Kette (HC CDR3) von SEQ ID NO: 1806; und
eine komplementaritätsbestimmende Region 1 der leichten Kette (LC CDR1) von SEQ ID NO: 1731,
eine komplementaritätsbestimmende Region 2 der leichten Kette (LC CDR2) von SEQ ID NO: 1769 und
eine komplementaritätsbestimmende Region 3 der leichten Kette (LC CDR3) von SEQ ID NO: 1807;
eine komplementaritätsbestimmende Region 1 der schweren Kette (HC CDR1) von SEQ ID NO: 1732,
eine komplementaritätsbestimmende Region 2 der schweren Kette (HC CDR2) von SEQ ID NO: 1770,
eine komplementaritätsbestimmende Region 3 der schweren Kette (HC CDR3) von SEQ ID NO: 1808; und
eine komplementaritätsbestimmende Region 1 der leichten Kette (LC CDR1) von SEQ ID NO: 1733,
eine komplementaritätsbestimmende Region 2 der leichten Kette (LC CDR2) von SEQ ID NO: 1771 und
eine komplementaritätsbestimmende Region 3 der leichten Kette (LC CDR3) von SEQ ID NO: 1809;
eine komplementaritätsbestimmende Region 1 der schweren Kette (HC CDR1) von SEQ ID NO: 1734,
eine komplementaritätsbestimmende Region 2 der schweren Kette (HC CDR2) von SEQ ID NO: 1772,
eine komplementaritätsbestimmende Region 3 der schweren Kette (HC CDR3) von SEQ ID NO: 1810; und
eine komplementaritätsbestimmende Region 1 der leichten Kette (LC CDR1) von SEQ ID NO: 1735,
eine komplementaritätsbestimmende Region 2 der leichten Kette (LC CDR2) von SEQ ID NO: 1773 und
eine komplementaritätsbestimmende Region 3 der leichten Kette (LC CDR3) von SEQ ID NO: 1811;
oder
eine komplementaritätsbestimmende Region 1 der schweren Kette (HC CDR1) von SEQ ID NO: 1736,
eine komplementaritätsbestimmende Region 2 der schweren Kette (HC CDR2) von SEQ ID NO: 1774,
eine komplementaritätsbestimmende Region 3 der schweren Kette (HC CDR3) von SEQ ID NO: 1812 und
eine komplementaritätsbestimmende Region 1 der leichten Kette (LC CDR1) von SEQ ID NO: 1737,
eine komplementaritätsbestimmende Region 2 der leichten Kette (LC CDR2) von SEQ ID NO: 1775 und
eine komplementaritätsbestimmende Region 3 der leichten Kette (LC CDR3) von SEQ ID NO: 1813.

5. Multispezifisches Molekül nach Anspruch 4, wobei die Anti-CD3-Antigenbindungsdomäne eine Aminosäuresequenz einer variablen Region der schweren Kette (VH) und eine Aminosäuresequenz der variablen Region der leichten Kette (VL) umfasst, die ausgewählt ist aus der Gruppe bestehend aus:
VH: SEQ ID NO: 1200 und VL: SEQ ID NO: 1201,
VH: SEQ ID NO: 1202 und VL: SEQ ID NO: 1203,
VH: SEQ ID NO: 1204 und VL: SEQ ID NO: 1205,
VH: SEQ ID NO: 1206 und VL: SEQ ID NO: 1207,
VH: SEQ ID NO: 1208 und VL: SEQ ID NO: 1209,
VH: SEQ ID NO: 1210 und VL: SEQ ID NO: 1211,
VH: SEQ ID NO: 1212 und VL: SEQ ID NO: 1213,
VH: SEQ ID NO: 1214 und VL: SEQ ID NO: 1215,
VH: SEQ ID NO: 1216 und VL: SEQ ID NO: 1217,
VH: SEQ ID NO: 1218 und VL: SEQ ID NO: 1219,
VH: SEQ ID NO: 1220 und VL: SEQ ID NO: 1221,
VH: SEQ ID NO: 1222 und VL: SEQ ID NO: 1223,
VH: SEQ ID NO: 1224 und VL: SEQ ID NO: 1225,
VH: SEQ ID NO: 1226 und VL: SEQ ID NO: 1227,
VH: SEQ ID NO: 1230 und VL: SEQ ID NO: 1231,
VH: SEQ ID NO: 1232 und VL: SEQ ID NO: 1233,
VH: SEQ ID NO: 1234 und VL: SEQ ID NO: 1235 und
VH: SEQ ID NO: 1236 und VL: SEQ ID NO: 1237.

6. Multispezifisches Molekül nach einem der Ansprüche 1 bis 5, wobei die zweite Antigenbindungsdomäne CD3 bindet und eine VL-Sequenz von SEQ ID NO: 1209 und eine VH-Sequenz von SEQ ID NO: 1236 umfasst; und wobei die Anti-CLL-1-Bindungsdomäne umfasst:
eine VL-Sequenz von SEQ ID NO: 88 und eine VH-Sequenz von SEQ ID NO: 75,
eine VL-Sequenz von SEQ ID NO: 89 und eine VH-Sequenz von SEQ ID NO: 76,
eine VL-Sequenz von SEQ ID NO: 87 und eine VH-Sequenz von SEQ ID NO: 74,
eine VL-Sequenz von SEQ ID NO: 85 und eine VH-Sequenz von SEQ ID NO: 72,
eine VL-Sequenz von SEQ ID NO: 86 und eine VH-Sequenz von SEQ ID NO: 73,
eine VL-Sequenz von SEQ ID NO: 83 und eine VH-Sequenz von SEQ ID NO: 70,
eine VL-Sequenz von SEQ ID NO: 90 und eine VH-Sequenz von SEQ ID NO: 77,
eine VL-Sequenz von SEQ ID NO: 79 und eine VH-Sequenz von SEQ ID NO: 66 oder
eine VL-Sequenz von SEQ ID NO: 84 und eine VH-Sequenz von SEQ ID NO: 71.

7. Multispezifisches Molekül nach einem der Ansprüche 1 bis 6, wobei die zweite Antigenbindungsdomäne CD3 bindet und SEQ ID NO: 506 umfasst; und wobei die Anti-CLL-1-Bindungsdomäne umfasst:
SEQ ID NO: 49,
SEQ ID NO: 50,
SEQ ID NO: 48,
SEQ ID NO: 46,
SEQ ID NO: 47,
SEQ ID NO: 44,
SEQ ID NO: 51,
SEQ ID NO: 40 oder
SEQ ID NO: 45.

8. Multispezifisches Molekül nach einem der Ansprüche 1 bis 7, wobei das Polypeptid, das die zweite Antigenbindungsdomäne umfasst, CD3 bindet und SEQ ID NO: 507 umfasst; und wobei das Polypeptid, das die Anti-CLL-1-Bindungsdomäne umfasst, umfasst:
SEQ ID NO: 512,
SEQ ID NO: 517,
SEQ ID NO: 522,
SEQ ID NO: 527,
SEQ ID NO: 532,
SEQ ID NO: 537,
SEQ ID NO: 542,
SEQ ID NO: 547 oder
SEQ ID NO: 552.

9. Multispezifisches Molekül nach einem der Ansprüche 1 bis 8, wobei die Polypeptide, die die CDR-Sequenzen der schweren Kette der ersten und/oder zweiten Antigenbindungsdomäne umfassen, zudem eine CH3-Domäne und gegebenenfalls eine CH2-Domäne umfassen.

10. Multispezifisches Molekül nach Anspruch 9, wobei die Polypeptide, die die CDR-Sequenzen der schweren Kette der ersten und zweiten Antigenbindungsdomäne umfassen, zudem eine CH3-Domäne umfassen, wobei beide der CH3-Domänen eine oder mehrere Modifikationen umfassen, um die Heterodimerisierung zu verbessern, wobei solche Modifikationen die Einführung eines Knopfes in eine erste CH3-Domäne und eines Lochs in eine zweite CH3-Domäne oder umgekehrt umfassen, so dass die Heterodimerisierung des Polypeptids, das die erste CH3-Domäne umfasst, und des Polypeptids, das die zweite CH3-Domäne umfasst, gegenüber Polypeptiden, die unveränderte CH3-Domänen umfassen, bevorzugt ist.

11. Isolierte Nukleinsäure, die das multispezifische Molekül nach einem der Ansprüche 1 bis 10 codiert.

12. Isolierte Nukleinsäure nach Anspruch 11, umfassend:
SEQ ID NO: 508 und SEQ ID NO: 509,
SEQ ID NO: 513 und SEQ ID NO: 514,
SEQ ID NO: 518 und SEQ ID NO: 519,
SEQ ID NO: 523 und SEQ ID NO: 524,
SEQ ID NO: 528 und SEQ ID NO: 529,
SEQ ID NO: 533 und SEQ ID NO: 534,
SEQ ID NO: 538 und SEQ ID NO: 539,
SEQ ID NO: 543 und SEQ ID NO: 544,
SEQ ID NO: 548 und SEQ ID NO: 549,
SEQ ID NO: 553 und SEQ ID NO: 554,
SEQ ID NO: 515,
SEQ ID NO: 520,
SEQ ID NO: 525,
SEQ ID NO: 530,
SEQ ID NO: 535,
SEQ ID NO: 540,
SEQ ID NO: 545,
SEQ ID NO: 550,
SEQ ID NO: 555,
SEQ ID NO: 516,
SEQ ID NO: 521,
SEQ ID NO: 526,
SEQ ID NO: 531,
SEQ ID NO: 536,
SEQ ID NO: 541,
SEQ ID NO: 546,
SEQ ID NO: 551 oder
SEQ ID NO: 556.

13. Vektor, umfassend die isolierte Nukleinsäure nach Anspruch 11 oder 12.

14. Multispezifisches Molekül nach einem der Ansprüche 1 bis 10 zur Verwendung als Medikament oder zur Verwendung in einer Therapie oder zur Verwendung bei der Behandlung eines Patienten mit hämatologischem Krebs oder zur Verwendung bei der Behandlung eines Patienten mit akuter myeloischer Leukämie (AML).

15. Pharmazeutische Zusammensetzung, umfassend das multispezifische Molekül nach einem der Ansprüche 1 bis 10 und ein pharmazeutisch verträgliches Verdünnungsmittel oder einen pharmazeutisch verträglichen Träger.

## Revendications

1. Molécule multispécifique comprenant un premier domaine de liaison d'antigène et un deuxième domaine de liaison d'antigène, dans laquelle le deuxième domaine de liaison d'antigène se lie à un antigène de cellule effectrice immunitaire qui est un antigène exprimé sur un lymphocyte T, l'antigène exprimé sur un lymphocyte T étant CD3, et dans laquelle le premier domaine de liaison d'antigène est un domaine de liaison anti-CLL-1 comprenant :
une région déterminant la complémentarité 1 de chaîne lourde (HC CDR1) de SEQ ID NO : 322, une région déterminant la complémentarité 2 de chaîne lourde (HC CDR2) de SEQ ID NO : 336, une région déterminant la complémentarité 3 de chaîne lourde (HC CDR3) de SEQ ID NO : 350 et une région déterminant la complémentarité 1 de chaîne légère (LC CDR1) de SEQ ID NO : 364, une région déterminant la complémentarité 2 de chaîne légère (LC CDR2) de SEQ ID NO : 378, et une région déterminant la complémentarité 3 de chaîne légère (LC CDR3) de SEQ ID NO : 392 ;
une région déterminant la complémentarité 1 de chaîne lourde (HC CDR1) de SEQ ID NO : 319, une région déterminant la complémentarité 2 de chaîne lourde (HC CDR2) de SEQ ID NO : 333, une région déterminant la complémentarité 3 de chaîne lourde (HC CDR3) de SEQ ID NO : 347 et une région déterminant la complémentarité 1 de chaîne légère (LC CDR1) de SEQ ID NO : 361, une région déterminant la complémentarité 2 de chaîne légère (LC CDR2) de SEQ ID NO : 375, et une région déterminant la complémentarité 3 de chaîne légère (LC CDR3) de SEQ ID NO : 389 ;
une région déterminant la complémentarité 1 de chaîne lourde (HC CDR1) de SEQ ID NO : 323, une région déterminant la complémentarité 2 de chaîne lourde (HC CDR2) de SEQ ID NO : 337, une région déterminant la complémentarité 3 de chaîne lourde (HC CDR3) de SEQ ID NO : 351 et une région déterminant la complémentarité 1 de chaîne légère (LC CDR1) de SEQ ID NO : 365, une région déterminant la complémentarité 2 de chaîne légère (LC CDR2) de SEQ ID NO : 379, et une région déterminant la complémentarité 3 de chaîne légère (LC CDR3) de SEQ ID NO : 393 ;
une région déterminant la complémentarité 1 de chaîne lourde (HC CDR1) de SEQ ID NO : 324, une région déterminant la complémentarité 2 de chaîne lourde (HC CDR2) de SEQ ID NO : 338, une région déterminant la complémentarité 3 de chaîne lourde (HC CDR3) de SEQ ID NO : 352 et une région déterminant la complémentarité 1 de chaîne légère (LC CDR1) de SEQ ID NO : 366, une région déterminant la complémentarité 2 de chaîne légère (LC CDR2) de SEQ ID NO : 380, et une région déterminant la complémentarité 3 de chaîne légère (LC CDR3) de SEQ ID NO : 394 ;
une région déterminant la complémentarité 1 de chaîne lourde (HC CDR1) de SEQ ID NO : 325, une région déterminant la complémentarité 2 de chaîne lourde (HC CDR2) de SEQ ID NO : 339, une région déterminant la complémentarité 3 de chaîne lourde (HC CDR3) de SEQ ID NO : 353 et une région déterminant la complémentarité 1 de chaîne légère (LC CDR1) de SEQ ID NO : 367, une région déterminant la complémentarité 2 de chaîne légère (LC CDR2) de SEQ ID NO : 381, et une région déterminant la complémentarité 3 de chaîne légère (LC CDR3) de SEQ ID NO : 395 ;
une région déterminant la complémentarité 1 de chaîne lourde (HC CDR1) de SEQ ID NO : 314, une région déterminant la complémentarité 2 de chaîne lourde (HC CDR2) de SEQ ID NO : 328, une région déterminant la complémentarité 3 de chaîne lourde (HC CDR3) de SEQ ID NO : 342 et une région déterminant la complémentarité 1 de chaîne légère (LC CDR1) de SEQ ID NO : 356, une région déterminant la complémentarité 2 de chaîne légère (LC CDR2) de SEQ ID NO : 370, et une région déterminant la complémentarité 3 de chaîne légère (LC CDR3) de SEQ ID NO : 384 ;
une région déterminant la complémentarité 1 de chaîne lourde (HC CDR1) de SEQ ID NO : 315, une région déterminant la complémentarité 2 de chaîne lourde (HC CDR2) de SEQ ID NO : 329, une région déterminant la complémentarité 3 de chaîne lourde (HC CDR3) de SEQ ID NO : 343 et une région déterminant la complémentarité 1 de chaîne légère (LC CDR1) de SEQ ID NO : 357, une région déterminant la complémentarité 2 de chaîne légère (LC CDR2) de SEQ ID NO : 371, et une région déterminant la complémentarité 3 de chaîne légère (LC CDR3) de SEQ ID NO : 385 ;
une région déterminant la complémentarité 1 de chaîne lourde (HC CDR1) de SEQ ID NO : 316, une région déterminant la complémentarité 2 de chaîne lourde (HC CDR2) de SEQ ID NO : 330, une région déterminant la complémentarité 3 de chaîne lourde (HC CDR3) de SEQ ID NO : 344 et une région déterminant la complémentarité 1 de chaîne légère (LC CDR1) de SEQ ID NO : 358, une région déterminant la complémentarité 2 de chaîne légère (LC CDR2) de SEQ ID NO : 372, et une région déterminant la complémentarité 3 de chaîne légère (LC CDR3) de SEQ ID NO : 386 ;
une région déterminant la complémentarité 1 de chaîne lourde (HC CDR1) de SEQ ID NO : 317, une région déterminant la complémentarité 2 de chaîne lourde (HC CDR2) de SEQ ID NO : 331, une région déterminant la complémentarité 3 de chaîne lourde (HC CDR3) de SEQ ID NO : 345 et une région déterminant la complémentarité 1 de chaîne légère (LC CDR1) de SEQ ID NO : 359, une région déterminant la complémentarité 2 de chaîne légère (LC CDR2) de SEQ ID NO : 373, et une région déterminant la complémentarité 3 de chaîne légère (LC CDR3) de SEQ ID NO : 387 ;
une région déterminant la complémentarité 1 de chaîne lourde (HC CDR1) de SEQ ID NO : 318, une région déterminant la complémentarité 2 de chaîne lourde (HC CDR2) de SEQ ID NO : 332, une région déterminant la complémentarité 3 de chaîne lourde (HC CDR3) de SEQ ID NO : 346 et une région déterminant la complémentarité 1 de chaîne légère (LC CDR1) de SEQ ID NO : 360, une région déterminant la complémentarité 2 de chaîne légère (LC CDR2) de SEQ ID NO : 374, et une région déterminant la complémentarité 3 de chaîne légère (LC CDR3) de SEQ ID NO : 388 ;
une région déterminant la complémentarité 1 de chaîne lourde (HC CDR1) de SEQ ID NO : 320, une région déterminant la complémentarité 2 de chaîne lourde (HC CDR2) de SEQ ID NO : 334, une région déterminant la complémentarité 3 de chaîne lourde (HC CDR3) de SEQ ID NO : 348 et une région déterminant la complémentarité 1 de chaîne légère (LC CDR1) de SEQ ID NO : 362, une région déterminant la complémentarité 2 de chaîne légère (LC CDR2) de SEQ ID NO : 376, et une région déterminant la complémentarité 3 de chaîne légère (LC CDR3) de SEQ ID NO : 390 ;
une région déterminant la complémentarité 1 de chaîne lourde (HC CDR1) de SEQ ID NO : 321, une région déterminant la complémentarité 2 de chaîne lourde (HC CDR2) de SEQ ID NO : 335, une région déterminant la complémentarité 3 de chaîne lourde (HC CDR3) de SEQ ID NO : 349 et une région déterminant la complémentarité 1 de chaîne légère (LC CDR1) de SEQ ID NO : 363, une région déterminant la complémentarité 2 de chaîne légère (LC CDR2) de SEQ ID NO : 377, et une région déterminant la complémentarité 3 de chaîne légère (LC CDR3) de SEQ ID NO : 391 ;
une région déterminant la complémentarité 1 de chaîne lourde (HC CDR1) de SEQ ID NO : 326, une région déterminant la complémentarité 2 de chaîne lourde (HC CDR2) de SEQ ID NO : 340, une région déterminant la complémentarité 3 de chaîne lourde (HC CDR3) de SEQ ID NO : 354 et une région déterminant la complémentarité 1 de chaîne légère (LC CDR1) de SEQ ID NO : 368, une région déterminant la complémentarité 2 de chaîne légère (LC CDR2) de SEQ ID NO : 382, et une région déterminant la complémentarité 3 de chaîne légère (LC CDR3) de SEQ ID NO : 396 ;
ou
une région déterminant la complémentarité 1 de chaîne lourde (HC CDR1) de SEQ ID NO : 327, une région déterminant la complémentarité 2 de chaîne lourde (HC CDR2) de SEQ ID NO : 341, une région déterminant la complémentarité 3 de chaîne lourde (HC CDR3) de SEQ ID NO : 355 et une région déterminant la complémentarité 1 de chaîne légère (LC CDR1) de SEQ ID NO : 369, une région déterminant la complémentarité 2 de chaîne légère (LC CDR2) de SEQ ID NO : 383, et une région déterminant la complémentarité 3 de chaîne légère (LC CDR3) de SEQ ID NO : 397.

2. Molécule multispécifique selon la revendication 1, dans laquelle le domaine de liaison anti-CLL-1 comprend une séquence d'acides aminés d'une région variable de chaîne lourde (VH) et une séquence d'acides aminés d'une région variable de chaîne légère (VL) choisies dans le groupe constitué de :
VH : SEQ ID NO : 73 et VL : SEQ ID NO : 86,
VH : SEQ ID NO : 70 et VL : SEQ ID NO : 83,
VH : SEQ ID NO : 74 et VL : SEQ ID NO : 87,
VH : SEQ ID NO : 75 et VL : SEQ ID NO : 88,
VH : SEQ ID NO : 76 et VL : SEQ ID NO : 89,
VH : SEQ ID NO : 65 et VL : SEQ ID NO : 78,
VH : SEQ ID NO : 66 et VL : SEQ ID NO : 79,
VH : SEQ ID NO : 67 et VL : SEQ ID NO : 80,
VH : SEQ ID NO : 68 et VL : SEQ ID NO : 81,
VH : SEQ ID NO : 69 et VL : SEQ ID NO : 82,
VH : SEQ ID NO : 71 et VL : SEQ ID NO : 84,
VH : SEQ ID NO : 72 et VL : SEQ ID NO : 85,
VH : SEQ ID NO : 77 et VL : SEQ ID NO : 90, et
VH : SEQ ID NO : 195 et VL : SEQ ID NO : 196.

3. Molécule multispécifique selon la revendication 1 ou 2, dans laquelle le domaine de liaison anti-CLL-1 comprend une séquence d'acides aminés d'un scFv choisi dans le groupe constitué de :
SEQ ID NO : 47,
SEQ ID NO : 44,
SEQ ID NO : 48,
SEQ ID NO : 49,
SEQ ID NO : 50,
SEQ ID NO : 39,
SEQ ID NO : 40,
SEQ ID NO : 41,
SEQ ID NO : 42,
SEQ ID NO : 43,
SEQ ID NO : 45,
SEQ ID NO : 46, et
SEQ ID NO : 51.

4. Molécule multispécifique selon l'une quelconque des revendications 1 à 3, dans laquelle le deuxième domaine de liaison d'antigène se lie à un antigène de cellule effectrice immunitaire qui est un antigène exprimé sur un lymphocyte T, l'antigène exprimé sur un lymphocyte T étant CD3, et le domaine de liaison d'antigène anti-CD3 comprenant :
une région déterminant la complémentarité 1 de chaîne lourde (HC CDR1) de SEQ ID NO : 1700, une région déterminant la complémentarité 2 de chaîne lourde (HC CDR2) de SEQ ID NO : 1738, une région déterminant la complémentarité 3 de chaîne lourde (HC CDR3) de SEQ ID NO : 1776 ; et
une région déterminant la complémentarité 1 de chaîne légère (LC CDR1) de SEQ ID NO : 1701, une région déterminant la complémentarité 2 de chaîne légère (LC CDR2) de SEQ ID NO : 1739, et une région déterminant la complémentarité 3 de chaîne légère (LC CDR3) de SEQ ID NO : 1777 ;
une région déterminant la complémentarité 1 de chaîne lourde (HC CDR1) de SEQ ID NO : 1702, une région déterminant la complémentarité 2 de chaîne lourde (HC CDR2) de SEQ ID NO : 1740, une région déterminant la complémentarité 3 de chaîne lourde (HC CDR3) de SEQ ID NO : 1778 ; et
une région déterminant la complémentarité 1 de chaîne légère (LC CDR1) de SEQ ID NO : 1703, une région déterminant la complémentarité 2 de chaîne légère (LC CDR2) de SEQ ID NO : 1741, et une région déterminant la complémentarité 3 de chaîne légère (LC CDR3) de SEQ ID NO : 1779 ;
une région déterminant la complémentarité 1 de chaîne lourde (HC CDR1) de SEQ ID NO : 1704, une région déterminant la complémentarité 2 de chaîne lourde (HC CDR2) de SEQ ID NO : 1742, une région déterminant la complémentarité 3 de chaîne lourde (HC CDR3) de SEQ ID NO : 1780 ; et
une région déterminant la complémentarité 1 de chaîne légère (LC CDR1) de SEQ ID NO : 1705, une région déterminant la complémentarité 2 de chaîne légère (LC CDR2) de SEQ ID NO : 1743, et une région déterminant la complémentarité 3 de chaîne légère (LC CDR3) de SEQ ID NO : 1781 ;
une région déterminant la complémentarité 1 de chaîne lourde (HC CDR1) de SEQ ID NO : 1706, une région déterminant la complémentarité 2 de chaîne lourde (HC CDR2) de SEQ ID NO : 1744, une région déterminant la complémentarité 3 de chaîne lourde (HC CDR3) de SEQ ID NO : 1782 ; et
une région déterminant la complémentarité 1 de chaîne légère (LC CDR1) de SEQ ID NO : 1707, une région déterminant la complémentarité 2 de chaîne légère (LC CDR2) de SEQ ID NO : 1745, et une région déterminant la complémentarité 3 de chaîne légère (LC CDR3) de SEQ ID NO : 1783 ;
une région déterminant la complémentarité 1 de chaîne lourde (HC CDR1) de SEQ ID NO : 1708, une région déterminant la complémentarité 2 de chaîne lourde (HC CDR2) de SEQ ID NO : 1746, une région déterminant la complémentarité 3 de chaîne lourde (HC CDR3) de SEQ ID NO : 1784 ; et
une région déterminant la complémentarité 1 de chaîne légère (LC CDR1) de SEQ ID NO : 1709, une région déterminant la complémentarité 2 de chaîne légère (LC CDR2) de SEQ ID NO : 1747, et une région déterminant la complémentarité 3 de chaîne légère (LC CDR3) de SEQ ID NO : 1785 ;
une région déterminant la complémentarité 1 de chaîne lourde (HC CDR1) de SEQ ID NO : 1710, une région déterminant la complémentarité 2 de chaîne lourde (HC CDR2) de SEQ ID NO : 1748, une région déterminant la complémentarité 3 de chaîne lourde (HC CDR3) de SEQ ID NO : 1786 ; et
une région déterminant la complémentarité 1 de chaîne légère (LC CDR1) de SEQ ID NO : 1711, une région déterminant la complémentarité 2 de chaîne légère (LC CDR2) de SEQ ID NO : 1749, et une région déterminant la complémentarité 3 de chaîne légère (LC CDR3) de SEQ ID NO : 1787 ;
une région déterminant la complémentarité 1 de chaîne lourde (HC CDR1) de SEQ ID NO : 1712, une région déterminant la complémentarité 2 de chaîne lourde (HC CDR2) de SEQ ID NO : 1750, une région déterminant la complémentarité 3 de chaîne lourde (HC CDR3) de SEQ ID NO : 1788 ; et
une région déterminant la complémentarité 1 de chaîne légère (LC CDR1) de SEQ ID NO : 1713, une région déterminant la complémentarité 2 de chaîne légère (LC CDR2) de SEQ ID NO : 1751, et une région déterminant la complémentarité 3 de chaîne légère (LC CDR3) de SEQ ID NO : 1789 ;
une région déterminant la complémentarité 1 de chaîne lourde (HC CDR1) de SEQ ID NO : 1714, une région déterminant la complémentarité 2 de chaîne lourde (HC CDR2) de SEQ ID NO : 1752, une région déterminant la complémentarité 3 de chaîne lourde (HC CDR3) de SEQ ID NO : 1790 ; et
une région déterminant la complémentarité 1 de chaîne légère (LC CDR1) de SEQ ID NO : 1715, une région déterminant la complémentarité 2 de chaîne légère (LC CDR2) de SEQ ID NO : 1753, et une région déterminant la complémentarité 3 de chaîne légère (LC CDR3) de SEQ ID NO : 1791 ;
une région déterminant la complémentarité 1 de chaîne lourde (HC CDR1) de SEQ ID NO : 1716, une région déterminant la complémentarité 2 de chaîne lourde (HC CDR2) de SEQ ID NO : 1754, une région déterminant la complémentarité 3 de chaîne lourde (HC CDR3) de SEQ ID NO : 1792 ; et
une région déterminant la complémentarité 1 de chaîne légère (LC CDR1) de SEQ ID NO : 1717, une région déterminant la complémentarité 2 de chaîne légère (LC CDR2) de SEQ ID NO : 1755, et une région déterminant la complémentarité 3 de chaîne légère (LC CDR3) de SEQ ID NO : 1793 ;
une région déterminant la complémentarité 1 de chaîne lourde (HC CDR1) de SEQ ID NO : 1718, une région déterminant la complémentarité 2 de chaîne lourde (HC CDR2) de SEQ ID NO : 1756, une région déterminant la complémentarité 3 de chaîne lourde (HC CDR3) de SEQ ID NO : 1794 ; et
une région déterminant la complémentarité 1 de chaîne légère (LC CDR1) de SEQ ID NO : 1719, une région déterminant la complémentarité 2 de chaîne légère (LC CDR2) de SEQ ID NO : 1757, et une région déterminant la complémentarité 3 de chaîne légère (LC CDR3) de SEQ ID NO : 1795 ;
une région déterminant la complémentarité 1 de chaîne lourde (HC CDR1) de SEQ ID NO : 1720, une région déterminant la complémentarité 2 de chaîne lourde (HC CDR2) de SEQ ID NO : 1758, une région déterminant la complémentarité 3 de chaîne lourde (HC CDR3) de SEQ ID NO : 1796 ; et
une région déterminant la complémentarité 1 de chaîne légère (LC CDR1) de SEQ ID NO : 1721, une région déterminant la complémentarité 2 de chaîne légère (LC CDR2) de SEQ ID NO : 1759, et une région déterminant la complémentarité 3 de chaîne légère (LC CDR3) de SEQ ID NO : 1797 ;
une région déterminant la complémentarité 1 de chaîne lourde (HC CDR1) de SEQ ID NO : 1722, une région déterminant la complémentarité 2 de chaîne lourde (HC CDR2) de SEQ ID NO : 1760, une région déterminant la complémentarité 3 de chaîne lourde (HC CDR3) de SEQ ID NO : 1798 ; et
une région déterminant la complémentarité 1 de chaîne légère (LC CDR1) de SEQ ID NO : 1723, une région déterminant la complémentarité 2 de chaîne légère (LC CDR2) de SEQ ID NO : 1761, et une région déterminant la complémentarité 3 de chaîne légère (LC CDR3) de SEQ ID NO : 1799 ;
une région déterminant la complémentarité 1 de chaîne lourde (HC CDR1) de SEQ ID NO : 1724, une région déterminant la complémentarité 2 de chaîne lourde (HC CDR2) de SEQ ID NO : 1762, une région déterminant la complémentarité 3 de chaîne lourde (HC CDR3) de SEQ ID NO : 1800 ; et
une région déterminant la complémentarité 1 de chaîne légère (LC CDR1) de SEQ ID NO : 1725, une région déterminant la complémentarité 2 de chaîne légère (LC CDR2) de SEQ ID NO : 1763, et une région déterminant la complémentarité 3 de chaîne légère (LC CDR3) de SEQ ID NO : 1801 ;
une région déterminant la complémentarité 1 de chaîne lourde (HC CDR1) de SEQ ID NO : 1726, une région déterminant la complémentarité 2 de chaîne lourde (HC CDR2) de SEQ ID NO : 1764, une région déterminant la complémentarité 3 de chaîne lourde (HC CDR3) de SEQ ID NO : 1802 ; et
une région déterminant la complémentarité 1 de chaîne légère (LC CDR1) de SEQ ID NO : 1727, une région déterminant la complémentarité 2 de chaîne légère (LC CDR2) de SEQ ID NO : 1765, et une région déterminant la complémentarité 3 de chaîne légère (LC CDR3) de SEQ ID NO : 1803 ;
une région déterminant la complémentarité 1 de chaîne lourde (HC CDR1) de SEQ ID NO : 1728, une région déterminant la complémentarité 2 de chaîne lourde (HC CDR2) de SEQ ID NO : 1766, une région déterminant la complémentarité 3 de chaîne lourde (HC CDR3) de SEQ ID NO : 1804 ; et
une région déterminant la complémentarité 1 de chaîne légère (LC CDR1) de SEQ ID NO : 1729, une région déterminant la complémentarité 2 de chaîne légère (LC CDR2) de SEQ ID NO : 1767, et une région déterminant la complémentarité 3 de chaîne légère (LC CDR3) de SEQ ID NO : 1805 ;
une région déterminant la complémentarité 1 de chaîne lourde (HC CDR1) de SEQ ID NO : 1730, une région déterminant la complémentarité 2 de chaîne lourde (HC CDR2) de SEQ ID NO : 1768, une région déterminant la complémentarité 3 de chaîne lourde (HC CDR3) de SEQ ID NO : 1806 ; et
une région déterminant la complémentarité 1 de chaîne légère (LC CDR1) de SEQ ID NO : 1731, une région déterminant la complémentarité 2 de chaîne légère (LC CDR2) de SEQ ID NO : 1769, et une région déterminant la complémentarité 3 de chaîne légère (LC CDR3) de SEQ ID NO : 1807 ;
une région déterminant la complémentarité 1 de chaîne lourde (HC CDR1) de SEQ ID NO : 1732, une région déterminant la complémentarité 2 de chaîne lourde (HC CDR2) de SEQ ID NO : 1770, une région déterminant la complémentarité 3 de chaîne lourde (HC CDR3) de SEQ ID NO : 1808 ; et
une région déterminant la complémentarité 1 de chaîne légère (LC CDR1) de SEQ ID NO : 1733, une région déterminant la complémentarité 2 de chaîne légère (LC CDR2) de SEQ ID NO : 1771, et une région déterminant la complémentarité 3 de chaîne légère (LC CDR3) de SEQ ID NO : 1809 ;
une région déterminant la complémentarité 1 de chaîne lourde (HC CDR1) de SEQ ID NO : 1734, une région déterminant la complémentarité 2 de chaîne lourde (HC CDR2) de SEQ ID NO : 1772, une région déterminant la complémentarité 3 de chaîne lourde (HC CDR3) de SEQ ID NO : 1810 ; et
une région déterminant la complémentarité 1 de chaîne légère (LC CDR1) de SEQ ID NO : 1735, une région déterminant la complémentarité 2 de chaîne légère (LC CDR2) de SEQ ID NO : 1773, et une région déterminant la complémentarité 3 de chaîne légère (LC CDR3) de SEQ ID NO : 1811 ;
ou
une région déterminant la complémentarité 1 de chaîne lourde (HC CDR1) de SEQ ID NO : 1736, une région déterminant la complémentarité 2 de chaîne lourde (HC CDR2) de SEQ ID NO : 1774, une région déterminant la complémentarité 3 de chaîne lourde (HC CDR3) de SEQ ID NO : 1812 et une région déterminant la complémentarité 1 de chaîne légère (LC CDR1) de SEQ ID NO : 1737, une région déterminant la complémentarité 2 de chaîne légère (LC CDR2) de SEQ ID NO : 1775, et une région déterminant la complémentarité 3 de chaîne légère (LC CDR3) de SEQ ID NO : 1813.

5. Molécule multispécifique selon la revendication 4, dans laquelle le domaine de liaison d'antigène anti-CD3 comprend une séquence d'acides aminés d'une région variable de chaîne lourde (VH) et une séquence d'acides aminés de la région variable de chaîne légère (VL) choisies dans le groupe constitué de :
VH : SEQ ID NO : 1200 et VL : SEQ ID NO : 1201,
VH : SEQ ID NO : 1202 et VL : SEQ ID NO : 1203,
VH : SEQ ID NO : 1204 et VL : SEQ ID NO : 1205,
VH : SEQ ID NO : 1206 et VL : SEQ ID NO : 1207,
VH : SEQ ID NO : 1208 et VL : SEQ ID NO : 1209,
VH : SEQ ID NO : 1210 et VL : SEQ ID NO : 1211,
VH : SEQ ID NO : 1212 et VL : SEQ ID NO : 1213,
VH : SEQ ID NO : 1214 et VL : SEQ ID NO : 1215,
VH : SEQ ID NO : 1216 et VL : SEQ ID NO : 1217,
VH : SEQ ID NO : 1218 et VL : SEQ ID NO : 1219,
VH : SEQ ID NO : 1220 et VL : SEQ ID NO : 1221,
VH : SEQ ID NO : 1222 et VL : SEQ ID NO : 1223,
VH : SEQ ID NO : 1224 et VL : SEQ ID NO : 1225,
VH : SEQ ID NO : 1226 et VL : SEQ ID NO : 1227,
VH : SEQ ID NO : 1230 et VL : SEQ ID NO : 1231,
VH : SEQ ID NO : 1232 et VL : SEQ ID NO : 1233,
VH : SEQ ID NO : 1234 et VL : SEQ ID NO : 1235, et
VH : SEQ ID NO : 1236 et VL : SEQ ID NO : 1237.

6. Molécule multispécifique selon l'une quelconque des revendications 1 à 5, dans laquelle le deuxième domaine de liaison d'antigène se lie à CD3 et comprend une séquence VL de SEQ ID NO : 1209 et une séquence VH de SEQ ID NO : 1236 ; et dans laquelle le domaine de liaison anti-CLL-1 comprend :
une séquence VL de SEQ ID NO : 88 et une séquence VH de SEQ ID NO : 75,
une séquence VL de SEQ ID NO : 89 et une séquence VH de SEQ ID NO : 76,
une séquence VL de SEQ ID NO : 87 et une séquence VH de SEQ ID NO : 74,
une séquence VL de SEQ ID NO : 85 et une séquence VH de SEQ ID NO : 72,
une séquence VL de SEQ ID NO : 86 et une séquence VH de SEQ ID NO : 73,
une séquence VL de SEQ ID NO : 83 et une séquence VH de SEQ ID NO : 70,
une séquence VL de SEQ ID NO : 90 et une séquence VH de SEQ ID NO : 77,
une séquence VL de SEQ ID NO : 79 et une séquence VH de SEQ ID NO : 66, ou
une séquence VL de SEQ ID NO : 84 et une séquence VH de SEQ ID NO : 71.

7. Molécule multispécifique selon l'une quelconque des revendications 1 à 6, dans laquelle le deuxième domaine de liaison d'antigène se lie à CD3 et comprend SEQ ID NO : 506 ; et dans laquelle le domaine de liaison anti-CLL-1 comprend :
SEQ ID NO : 49,
SEQ ID NO : 50,
SEQ ID NO : 48,
SEQ ID NO : 46,
SEQ ID NO : 47,
SEQ ID NO : 44,
SEQ ID NO : 51,
SEQ ID NO : 40, ou
SEQ ID NO : 45.

8. Molécule multispécifique selon l'une quelconque des revendications 1 à 7, dans laquelle le polypeptide comprenant le deuxième domaine de liaison d'antigène se lie à CD3 et comprend SEQ ID NO : 507 ; et dans laquelle le polypeptide comprenant le domaine de liaison anti-CLL-1 comprend :
SEQ ID NO : 512,
SEQ ID NO : 517,
SEQ ID NO : 522,
SEQ ID NO : 527,
SEQ ID NO : 532,
SEQ ID NO : 537,
SEQ ID NO : 542,
SEQ ID NO : 547, ou
SEQ ID NO : 552.

9. Molécule multispécifique selon l'une quelconque des revendications 1 à 8, dans laquelle les polypeptides comprenant les séquences de CDR de chaîne lourde du premier et/ou deuxième domaine de liaison d'antigène comprennent en outre un domaine CH3, et facultativement un domaine CH2.

10. Molécule multispécifique selon la revendication 9, dans laquelle les polypeptides comprenant les séquences de CDR de chaîne lourde des premier et deuxième domaines de liaison d'antigène comprennent en outre un domaine CH3, dans laquelle lesdits deux domaines CH3 comprennent une ou plusieurs modifications pour augmenter l'hétérodimérisation, dans laquelle de telles modifications comprennent l'introduction d'une protubérance dans un premier domaine CH3 et d'un trou dans un deuxième domaine CH3, ou inversement, de sorte que l'hétérodimérisation du polypeptide comprenant le premier domaine CH3 et le polypeptide comprenant le deuxième domaine CH3 est favorisée par rapport à des polypeptides comprenant des domaines CH3 non modifiés.

11. Acide nucléique isolé codant pour la molécule multispécifique selon l'une quelconque des revendications 1 à 10.

12. Acide nucléique isolé selon la revendication 11 comprenant :
SEQ ID NO : 508 et SEQ ID NO : 509,
SEQ ID NO : 513 et SEQ ID NO : 514,
SEQ ID NO : 518 et SEQ ID NO : 519,
SEQ ID NO : 523 et SEQ ID NO : 524,
SEQ ID NO : 528 et SEQ ID NO : 529,
SEQ ID NO : 533 et SEQ ID NO : 534,
SEQ ID NO : 538 et SEQ ID NO : 539,
SEQ ID NO : 543 et SEQ ID NO : 544,
SEQ ID NO : 548 et SEQ ID NO : 549,
SEQ ID NO : 553 et SEQ ID NO : 554,
SEQ ID NO : 515,
SEQ ID NO : 520,
SEQ ID NO : 525,
SEQ ID NO : 530,
SEQ ID NO : 535,
SEQ ID NO : 540,
SEQ ID NO : 545,
SEQ ID NO : 550,
SEQ ID NO : 555,
SEQ ID NO : 516,
SEQ ID NO : 521,
SEQ ID NO : 526,
SEQ ID NO : 531,
SEQ ID NO : 536,
SEQ ID NO : 541,
SEQ ID NO : 546,
SEQ ID NO : 551, ou
SEQ ID NO : 556.

13. Vecteur comprenant l'acide nucléique isolé selon la revendication 11 ou 12.

14. Molécule multispécifique selon l'une quelconque des revendications 1 à 10 pour utilisation en tant que médicament, ou pour utilisation dans une thérapie, ou pour utilisation dans le traitement d'un sujet ayant un cancer hématologique ou pour utilisation dans le traitement d'un sujet ayant une leucémie myéloïde aiguë (LMA) .

15. Composition pharmaceutique comprenant la molécule multispécifique selon l'une quelconque des revendications 1 à 10 et un diluant ou véhicule pharmaceutiquement acceptable.
